# EUROPEAN PATENT APPLICATION

(11) **EP 4 578 856 A1**
(43) Date of publication of application: **02.07.2025**
(21) Application number: 23872591.5
(22) Date of filing: 29.09.2023
(51) Int. Cl.: C07D 471/04, A61K 31/496, A61K 31/506, A61P 43/00

(54) **HETEROCYCLIC COMPOUND**

(30) Priority: 30.09.2022 JP 2022159058
(71) Applicant: Ubience Inc., Tokyo 103-0012 (JP)
(72) Inventor: IWATA, Yasuhiro, Tokyo 103-0012 (JP); TAKAHASHI, Nobuyuki, Tokyo 103-0012 (JP)
(74) Representative: Vossius & Partner Patentanwälte Rechtsanwälte mbB
(86) International application number: PCT/JP2023/035592
(87) International publication number: WO 2024/071371

(57) **Abstract**

The present disclosure provides heterocyclic compounds.

The present disclosure relates to a compound in which a ligand of a specific E3 ligase and an intracellular target protein ligand are linked via a linker. More specifically, the present disclosure relates to a compound that induces the degradation of a target protein using a ligand of an inhibitor of apoptosis protein (IAP) that is an E3 ligase. The compound of the disclosure exhibits an effect of inducing protein degradation by using a ligand of an inhibitor of apoptosis protein (IAP). Therefore, the compound of the disclosure is useful as a therapeutic agent and/or a prophylactic agent.

## Description

### [Technical Field]

The present disclosure relates to a compound in which a ligand of a specific E3 ligase and an intracellular target protein ligand are linked via a linker. More specifically, the present disclosure relates to a compound that induces the degradation of a target protein using a ligand of an inhibitor of apoptosis protein (IAP) that is an E3 ligase.

### [Background Art]

Development of compounds that induce ubiquitination of target proteins by an E3 ligase (specifically, an inhibitor of apoptosis protein (IAP)) and degradation by proteasome has been attempted for the purpose of treatment by reducing disease-related proteins. For example, compounds containing MV1, MeBS, Nutlin-3, IκBα peptide, HIF-1α peptide, or VHL ligand as a ligand that binds to an E3 ligase, where the target protein is CRABP2, RIPK2, ERα, TACC3, AR, ERRα, FRS2α, PI3K or the like have been proposed.

### [Summary of the Invention]

### [Means for solving the problem]

The present disclosure provides a compound that induces the degradation of a target protein.

The present inventors have conducted intensive studies in an attempt and found that the compounds represented by the following Formula A and related structural formulas thereof, or pharmaceutically acceptable salts thereof (hereinafter sometimes referred to as "the compound(s) of the disclosure") can solve the above-mentioned problems, which resulted in the completion of the technical matters of the present disclosure. Accordingly, the present disclosure is as follows.

### (Item 1)

A compound represented by Formula (A) Z-L-X or an isomer thereof, or a salt thereof,
wherein
Z is a ligand moiety capable of specifically binding to an intracellular protein,
L is a linker moiety or a single bond, and
X is the following formula 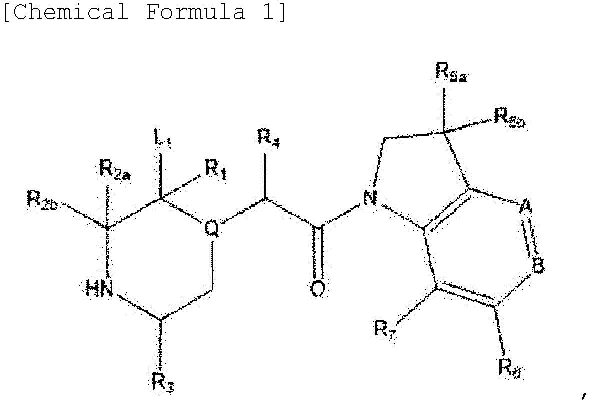 wherein
   A is N or CR₈,
   B is N, CR₉, or CL₂,
   Q is N or CR₁₀,
   R₁, and L₁ not used for bonding to L, are each independently selected from hydrogen, C₁₋₆ alkyl, C₂₋₆ alkenyl, C₂₋₆ alkynyl, - C(=O)NH_{(2-q)}(C₁₋₆ alkyl)_{q}, -(CH₂)ₛ-(3- to 12-membered heterocyclyl), -(CH₂)ₛ-C₃₋₁₂ carbocyclyl, -C(=O)-(3- to 12-membered heterocyclyl), and -C(=O)-C₃₋₁₂ carbocyclyl, or R₁, and L₁ not used for bonding to L, together with the carbon atom to which they are attached, can join to form a 3-to 10-membered saturated carbocyclyl or heterocyclyl group, wherein the C₁₋₆ alkyl, C₂₋₆ alkenyl, C₂₋₆ alkynyl, heterocyclyl and carbocyclyl groups are optionally substituted with one or more R^{b} groups;
   R₂ₐ and R_{2b} are each independently selected from hydrogen, C₁₋₆ alkyl, C₂₋₆ alkenyl, C₂₋₆ alkynyl, -C(=O)NH_{(2-q)}(C₁₋₆ alkyl)_{q}, - (CH₂)ₛ-(3- to 12-membered heterocyclyl), and -(CH₂)ₛ-C₃₋₁₂ carbocyclyl,
   or R₂ₐ and R_{2b}, together with the carbon atom to which they are attached, can join to form a 3- to 10-membered saturated carbocyclyl or heterocyclyl group, wherein the C₁₋₆ alkyl, C₂₋₆ alkenyl, C₂₋₆ alkynyl, heterocyclyl and carbocyclyl groups are optionally substituted with one or more R^{b} groups;
   R³ is selected from C₁₋₄ alkyl, C₂₋₄ alkenyl and - (CH₂)ₛ-C₃₋₈ cycloalkyl, wherein the C₁₋₄ alkyl, C₂₋₄ alkenyl, and C₃₋₈ cycloalkyl are optionally substituted with one or more R^{a} groups;
   R^{a} is selected from halogen, -OH and -O-C₁₋₆ alkyl;
   R₄ is selected from hydrogen, C₁₋₆ alkyl, C₂₋₆ alkenyl, C₂₋₆ alkynyl, -(CH₂)ₛ-C₃₋₈ cycloalkyl and -(CH₂)ₛ-C₃₋₈ cycloalkenyl, wherein the C₁₋₆ alkyl, C₂₋₆ alkenyl, C₂₋₆ alkynyl, C₃₋₈ cycloalkyl and C₃₋₈ cycloalkenyl are optionally substituted with one or more R^{a} groups;
   R₅ₐ and R_{5b} are each independently selected from hydrogen, C₁₋₈ alkyl, C₂₋₈ alkenyl, C₂₋₈ alkynyl, -Y-C₃₋₁₂ carbocyclyl, -W-(3- to 12-membered heterocyclyl), -(CR^{x}R^{y})ₛ-O-R^{z}, -O-(CR^{x}R^{y})ₙ-OR^{z}, - (CH₂)ₛ-CN, -S(O)_{q}-R^{x}, -C(=O)R^{x}, -C(=S)R^{x}, -C(=N)R^{x}, -(CRₓR_{y})ₛ-C(=O)OR^{z}, -(CR^{x}R^{y})ₛ-O-C(=O)-R^{z}, -(CRₓR_{y})ₛ-C(=O)NR^{x}R^{y}, -(CH₂)ₛ-NR^{x}C(=O)R^{y}, -(CH₂)ₛ-OC(=O)NR^{x}R^{y}, -(CH₂)ₛ-NR^{x}C(=O)OR^{y}, -(CH₂)ₛ-NR^{x}R^{y}, -NR^{×}-(CH₂)ₛ-R^{z}, -(CR^{x}R^{y})ₛ-C(=S)NR^{z}, -(CR^{x}R^{y})ₛ-C(=N)NR^{z}, - (CH₂)ₛ-O-C(=O)-C₁₋₄ alkyl-NR^{x}R^{y}, -(CH₂)ₛ-NR^{x}-(CH₂)ₙ-O-C(=O)-R^{z}, - (CH₂)ₛ-NR^{x}-(CH₂)ₛ-SO₂-R^{y}, -(CH₂)ₛ-NH-SO₂-NR^{x}R^{y} and - (CH₂)ₛ-SO₂NR^{x}R^{y} groups,
   or R₅ₐ and R_{5b}, together with the carbon atom to which they are attached, can join to form a 3- to 6-membered carbocyclyl or 4-to 6-membered heterocyclyl group,
   wherein the C₁₋₈ alkyl, C₂₋₈ alkenyl and C₂₋₈ alkynyl groups are optionally substituted with one or more R^{b} groups, and wherein the carbocyclyl group and heterocyclyl group are optionally substituted with one or more R^{b} groups;
   R₆ is selected from hydrogen, halogen, C₁₋₈ alkyl, C₂₋₈ alkenyl, C₂₋₈ alkynyl, -Y-C₃₋₁₂ carbocyclyl, -W- (3- to 12-membered heterocyclyl), -(CR^{x}R^{y})ₛ-O-R^{z}, -O-(CR^{x}R^{y})ₙ-OR^{z}, =O, =S, nitro, Si(R^{x})₄, -(CH₂)ₛ-CN, -S(O)_{q}-(CR^{x}R^{y})ₛ-R^{z}, -C(=O)R^{x}, -C(=S)R^{x}, - C(=N)R^{x}, -(CR^{x}R^{y})ₛ-C(=O)OR^{z}, - (CR^{x}R^{y})ₛ-O-C(=O)-R^{z}, -(CR^{x}R^{y})ₛ-C(=O)NR^{x}R^{y}, -(CH₂)ₛ-NR^{x}C(=O)R^{y}, -(CH₂)ₛ-OC(=O)NR^{x}R^{y}, -(CH₂)ₛ-NR^{x}C(=O)OR^{y}, -(CH₂)ₛ-NR^{x}R^{y}, -NR^{x}-(CH₂)ₛ-R^{z}, -(CRₓR_{y})ₛ-C(=S)NR^{z}, - (CR^{x}R^{y})ₛ-C(=N)NR^{x}, -S(O)(=NR^{x})R^{y}, -(CH₂)ₛ-O-C(=O)-C₁₋₄ alkyl-NR^{x}R^{y}, -(CH₂)ₛ-NR^{x}-(CH₂)ₙ-O-C(=O)-R^{z}, -(CH₂)ₛ-NR^{x}-(CH₂)ₛ-SO₂-R^{y}, -(CH₂)ₛ-NH-SO₂-NR^{x}R^{y} and -(CH₂)ₛ-SO₂NR^{x}R^{y} groups, -P(=O)(R^{x})₂, -Y-C₅₋₁₀ aryl, -Y-(5- to 10-membered heteroaryl), an optionally fused 5-to 10-membered carbocyclyl group, an optionally fused 5- to 10-membered heterocyclyl group, and 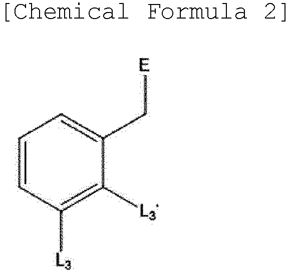
   wherein E indicates a bonding point,
   L₃ and L₃', together with the carbon atoms to which they are attached, can join to form a 3- to 6-membered carbocyclyl or 4-to 6-membered heterocyclyl group connected to a linker, wherein the C₁₋₈ alkyl, C₂₋₈ alkenyl and C₂₋₈ alkynyl groups, carbocyclyl group and heterocyclyl group, and 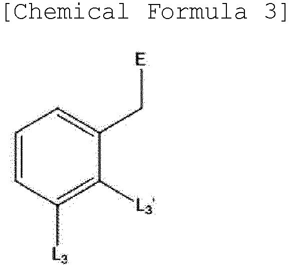
   wherein E indicates a bonding point, are optionally substituted with one or more R^{b} groups;
   R₇, R₈ and R₉ are each independently selected from hydrogen, halogen, C₁₋₈ alkyl, C₂₋₈ alkenyl, C₂₋₈ alkynyl, -Y-C₃₋₁₂ carbocyclyl, -W-(3- to 12-membered heterocyclyl), -(CR^{x}R^{y})ₛ-OR^{z}, -O-(CR^{x}R^{y})ₙ-OR^{z}, =O, =S, nitro, Si(R^{x})₄, -(CH₂)ₛ-CN, -S(O)_{q}-(CR^{x}R^{y})ₛ-R^{z}, -C(=O)R^{x}, -C(=S)R^{x}, -C(=N)R^{x}, -(CR^{x}R^{y})ₛ-C(=O)OR^{z}, - (CR^{x}R^{y})ₛ-O-C(=O)-R^{z}, -(CR^{x}R^{y})ₛ-C(=O)NR^{x}R^{y}, -(CH₂)ₛ-NR^{x}C(=O)R^{y}, - (CH₂)ₛ-OC(=O)NR^{x}R^{y}, -(CH₂)ₛ-NR^{x}C(=O)OR^{y}, -(CH₂)ₛ-NR^{x}R^{y}, -NR^{x}-(CH₂)ₛ-R^{z}, -(CR^{x}R^{y})ₛ-C(=S)NR^{z}, -(CR^{x}R^{y})ₛ-C(=N)NR^{x}, -S(O)(=NR^{x})R^{y}, -(CH₂)ₛ-O-C(=O)-C₁₋₄ alkyl-NR^{x}R^{y}, -(CH₂)ₛ-NR^{x}-(CH₂)ₙ-O-C(=O)-R^{z}, -(CH₂)ₛ-NR^{x}-(CH₂)ₛ-SO₂-R^{y}, -(CH₂)ₛ-NH-SO₂-NR^{x}R^{y} and -(CH₂)ₛ-SO₂NR^{x}R^{y} groups, -P(=O)(R^{x})₂ and -Y-C₅₋₁₀ aryl, wherein the C₁₋₈ alkyl, C₂₋₈ alkenyl and C₂₋₈ alkynyl groups are optionally substituted with one or more R^{b} groups, and wherein the carbocyclyl and heterocyclyl groups are optionally substituted with one or more (e.g., 1, 2 or 3) R^{b} group;
   each R^{b} is independently selected from halogen, C₁₋₆ alkyl, C₂₋₆ alkenyl, C₂₋₆ alkynyl, -(CH₂)ₛ-C₃₋₈ cycloalkyl, -(CH₂)ₛ-C₃₋₈ cycloalkenyl, -(CH₂)ₛ-phenyl, -(CH₂)ₛ-(5- to 10-membered heteroaryl), -(CH₂)ₛ-(4- to 7-membered saturated heterocyclyl), -(CR^{x}R^{y})ₛ-O-R^{z}, -O-(CR^{x}R^{y})ₙ-OR^{z}, haloC₁₋₆ alkyl, haloC₁₋₆ alkoxy, hydroxy, hydroxyC₁₋₆ alkyl, =O, =S, nitro, Si(Rx)₄, -(CH₂)ₛ-CN, - S(O)_{q}-R^{x}, -C(=O)R^{x}, -(CR^{x}R^{y})ₛ-C(=O)OR^{z}, -(CRₓR_{y})ₛ-O-C(=O)-R^{z}, - (CR^{x}R^{y})ₛ-C(=O)NR^{x}R^{y}, -(CH₂)ₛ-NR^{x}C(=O)R^{y}, -(CH₂)ₛ-OC(=O)NR^{x}R^{y}, - (CH₂)ₛ-NR^{x}C(=O)OR^{y}-(CH₂)ₛ-NR^{x}R^{y}, -NR^{x}-(CH₂)ₛ-R^{z}, -(CH₂)ₛ-O-C(=O)-C₁₋₄ alkyl-NR^{x}R^{y}, -(CH₂)ₛ-NR^{x}-(CH₂)ₙ-O-C(=O)-R^{z}, -(CH₂)ₛ-NR^{x}-(CH₂)ₛ-SO₂-R^{y}, -(CH₂)ₛ-NH-SO₂-NR^{x}R^{y} and -(CH₂)ₛ-SO₂NR^{x}R^{y} groups and - P(=O)(R^{x})₂, wherein the C₁₋₆ alkyl, C₂₋₆ alkenyl, C₂₋₆ alkynyl, C₃₋₈ cycloalkyl, C₃₋₈ cycloalkenyl and heterocyclyl groups, phenyl and heteroaryl are optionally substituted with one or more R^{x} groups;
   R^{x}, R^{y} and R^{z} are each independently selected from halogen, hydrogen, C₁₋₆ alkyl, C₂₋₆ alkenyl, C₂₋₆ alkynyl, -(CH₂)ₛ-C₃₋₈ cycloalkyl, -(CH₂)ₛ-C₃₋₈ cycloalkenyl, -(CH₂)ₛ-phenyl, -(CH₂)ₛ-(4-to 7-membered saturated heterocyclyl), hydroxyC₁₋₆ alkyl optionally substituted with one or more halo, -C(=O)OC₁₋₆ alkyl, hydroxy, C₁₋₆ alkoxy, haloC₁₋₆ alkyl, -(CH₂)ₙ-O-C₁₋₆ alkyl, - C(=O)-(CH₂)ₙ-C₁₋₆ alkoxy, -C(=O)-C₁₋₆ alkyl, -(CH₂)ₛ-CN, C₁₋₆ alkyl-N(H)_{2-q}(C₁₋₆ alkyl)_{q}, -N(H)_{2-q}(C₁₋₆ alkyl)_{q}, -C(=O)-N(H)_{2-q}(C₁₋₆ alkyl)_{q}, -(CH₂)ₛ-NH-SO₂-N(H)_{2-q}(C₁₋₆ alkyl)_{q}, -(CH₂)ₛ-N(C₁₋₄ alkyl)-SO₂-N(H)_{2-q}(C₁₋₆ alkyl)_{q} and -(CH₂)ₛ-O-C(=O)-C₁₋₄ alkyl-N(H)_{2-q}(C₁₋₆ alkyl) _{q}, and when attached to nitrogen, carbon, silicon or phosphorus atom, R^{x} and R^{y}, together with the atom(s) to which they are attached, may join to form a 3- to 7-membered ring optionally containing one or two additional heteroatoms selected from O, N, S and oxidized forms of N and S;
   Y and W are each independently selected from a bond, -(CR^{x}R^{y})ₘ-, -C(=CR^{x})-, -C(=O)-, -NR^{x}, -C(=O)NR^{x}-, -NR^{x}C(=O)-, -(CR^{x}R^{y})_{q}-O-, - O-(CR^{x}R^{y})_{q}-, -S(O)₂-NH, NH-S(O)₂- and -S(O)_{q}-;
   R₁₀ is selected from hydrogen, C₁₋₆ alkyl, C₂₋₆ alkenyl, C₂₋₆ alkynyl, -C(=O)NH_{(2-q)}(C₁₋₆ alkyl)_{q}, -(CH₂)ₛ-(3- to 12-membered heterocyclyl), -(CH₂)ₛ-C₃₋₁₂ carbocyclyl, -C(=O)-(3- to 12-membered heterocyclyl), and -C(=O)-C₃₋₁₂ carbocyclyl, wherein the C₁₋₆ alkyl, C₂₋₆ alkenyl, C₂₋₆ alkynyl, heterocyclyl and carbocyclyl groups are optionally substituted with one or more R^{b} groups;
   s independently represents an integer of 0 to 4;
   n independently represents an integer of 1 to 4;
   q independently represents an integer of 0 to 2;
   m represents an integer of 1 to 2, and
   X is bonded to L at any one of L₁ to L₃ and L₃' .

### (Item 2)

The compound or the isomer thereof or the salt thereof of item 1, wherein the intracellular protein in Z is a serin/threonine kinase, a receptor tyrosine kinase, a non-receptor tyrosine kinase, or a non-kinase.

### (Item 3)

The compound or the isomer thereof or the salt thereof of item 1, which has an inhibitory activity or binding activity against the intracellular protein in Z.

### (Item 4)

The compound or the isomer thereof or the salt thereof of the preceding item, wherein the intracellular protein in Z is a serin/threonine kinase, and the serin/threonine kinase is selected from the group consisting of AGC group, CAMK group, CK1 group, CMGC group, STE group, TKL group, and other serinthreonine kinases.

### (Item 5)

The compound or the isomer thereof or the salt thereof of the preceding item, wherein the serin/threonine kinase is ROCK2.

### (Item 6)

The compound or the isomer thereof or the salt thereof of the preceding item, wherein the intracellular protein in Z is a receptor tyrosine kinase, and is selected from the group consisting of epidermal growth factor receptor family, insulin receptor family, platelet-derived growth factor family, vascular endothelial growth factor receptor family, fibroblast growth factor receptor family, CCK family, nerve growth factor receptor family, hepatocyte growth factor receptor family, Eph receptor family, AXL receptor family, TIE receptor family, RYK receptor family, DDR receptor family, RET receptor family, ROS receptor family, LTK receptor family, ROR receptor family, MuSK receptor family, LMR receptor family, and other receptor tyrosine kinases.

### (Item 7)

The compound or the isomer thereof or the salt thereof of the preceding item, wherein the receptor tyrosine kinase is ALK.

### (Item 8)

The compound or the isomer thereof or the salt thereof of the preceding item, wherein the intracellular protein in Z is a non-receptor tyrosine kinase, and is selected from the group consisting of ABL family, ACK family, CSK family, FAK family, FES family, FRK family, JAK family, SRC family, TEC family and SYK family.

### (Item 9)

The compound or the isomer thereof or the salt thereof of the preceding item, wherein the non-receptor tyrosine kinase is FAK.

### (Item 10)

The compound or the isomer thereof or the salt thereof of the preceding item, wherein X is connected to L at either L₁, L₂, L₃ or L₃'.

### (Item 11)

The compound or the isomer thereof or the salt thereof of the preceding item, wherein Q is N.

### (Item 12)

The compound or the isomer thereof or the salt thereof of the preceding item, wherein Q is CH.

### (Item 13)

The compound or the isomer thereof or the salt thereof of the preceding item, wherein A is N.

### (Item 14)

The compound or the isomer thereof or the salt thereof of the preceding item, wherein A is CH.

### (Item 15)

The compound or the isomer thereof or the salt thereof of the preceding item, wherein B is N.

### (Item 16)

The compound or the isomer thereof or the salt thereof of the preceding item, wherein B is CH.

### (Item 17)

The compound or the isomer thereof or the salt thereof of the preceding item, wherein
R₁, and L₁ not used for bonding to L, are each independently selected from hydrogen, C₁₋₆ alkyl, C₂₋₆ alkenyl, C₂₋₆ alkynyl, - C(=O)NH_{(2-q)}(C₁₋₆ alkyl)_{q}, -(CH₂)ₛ-(3- to 7-membered heterocyclyl), -(CH₂)ₛ-C₃₋₇ carbocyclyl, -C(=O)-(3- to 7-membered heterocyclyl), and -C(=O)-C₃₋₇ carbocyclyl, or R₁, and L₁ not used for bonding to L, together with the carbon atom to which they are attached, can join to form a 3- to 6-membered saturated carbocyclyl or 4- to 6-membered heterocyclyl group, wherein the C₁₋₆ alkyl, C₂₋₆ alkenyl, C₂₋₆ alkynyl, heterocyclyl and carbocyclyl groups are optionally substituted with one or more R^{b} groups,
each R^{b} is independently selected from halogen, C₁₋₄ alkyl, C₃₋₄ alkynyl, -(CH₂)_{q}-C₃₋₆ cycloalkyl, -(CH₂)_{q}-phenyl, -(CH₂)_{q}-(5- to 6-membered heteroaryl), -(CH₂)_{q}-O-C₁₋₄ alkyl, haloC₁₋₄ alkyl, haloC₁₋₄ alkoxy, hydroxy, hydroxyC₁₋₄ alkyl, =O, -S(O)_{q}-C₁₋₄ alkyl, -C(=O)C₁₋₄ alkyl, -(CH₂)_{q}-C(=O)NR^{x}R^{y}, -(CH₂)_{q}-NR^{x}C(=O)C₁₋₄ alkyl, -(CH₂)ₛ-NR^{x}R^{y}, -NR^{x}-(CH₂)_{q}-C₁₋₄ alkyl, -(CH₂)_{q}-NR^{x}-(CH₂)_{q}-SO₂-C₁₋₄ alkyl, and -(CH₂)_{q}-SO₂NR^{x}R^{y} groups, wherein the cycloalkyl, phenyl and heteroaryl groups are optionally substituted with one or more R^{x} groups, and
R^{x} and R^{y} are each independently selected from halogen, hydrogen, C₁₋₄ alkyl, and C₁₋₄ alkoxy, and when attached to nitrogen or carbon, R^{x} and R^{y}, together with the atom(s) to which they are attached, may join to form a 3- to 7-membered ring optionally containing one additional heteroatom selected from O and N.

### (Item 18)

The compound or the isomer thereof or the salt thereof of the preceding item, wherein
R₁, and L₁ not used for bonding to L, are each independently selected from hydrogen, C₁₋₆ alkyl, C₂₋₆ alkenyl, C₂₋₆ alkynyl, - C(=O)NH_{(2-q)}(C₁₋₆ alkyl)_{q}, -(CH₂)ₛ-(3- to 7-membered heterocyclyl), -(CH₂)ₛ-C₃₋₇ carbocyclyl, -C(=O)-(3- to 7-membered heterocyclyl), and -C(=O)-C₃₋₇ carbocyclyl, wherein the C₁₋₆ alkyl, C₂₋₆ alkenyl, C₂₋₆ alkynyl, heterocyclyl and carbocyclyl groups are optionally substituted with one or more R^{b} groups,
each R^{b} is independently selected from fluorine, methyl, C₃₋₆ cycloalkyl, phenyl, 5- to 6-membered heteroaryl, -O-C₁₋₂ alkyl, fluoromethyl, fluoromethoxy, hydroxy, =O, -S(O)₂-C₁₋₄ alkyl, - C(=O)C₁₋₄ alkyl, -C(=O)NR^{x}R^{y}, -NR^{x}C(=O)C₁₋₄ alkyl, and -NR^{x}R^{y} groups, wherein the cycloalkyl, phenyl and heteroaryl groups are optionally substituted with one or more R^{x} groups, and R^{x} and R^{y} are each independently selected from chlorine, fluorine, hydrogen, C₁₋₄ alkyl, and C₁₋₄ alkoxy, and when attached to nitrogen or carbon, R^{x} and R^{y}, together with the atom(s) to which they are attached, may join to form a 3- to 7-membered ring optionally containing one additional heteroatom selected from O and N.

### (Item 19)

The compound or the isomer thereof or the salt thereof of the preceding item, wherein R₂ₐ and R_{2b} are each independently selected from hydrogen, C₁₋₃ alkyl, C₂₋₃ alkenyl and C₂₋₃ alkynyl, or R₂ₐ and R_{2b}, together with the carbon atom to which they are attached, can join to form a 3- to 4-membered saturated carbocyclyl group.

### (Item 20)

The compound or the isomer thereof or the salt thereof of the preceding item, wherein R₂ₐ and R_{2b} are hydrogen.

### (Item 21)

The compound or the isomer thereof or the salt thereof of the preceding item, wherein R³ is C₁₋₄ alkyl, wherein the C₁₋₄ alkyl is optionally substituted with one or more R^{a} groups.

### (Item 22)

The compound or the isomer thereof or the salt thereof of the preceding item, wherein R³ is methyl, wherein the methyl is optionally substituted with one or more fluorine.

### (Item 23)

The compound or the isomer thereof or the salt thereof of the preceding item, wherein R₄ is hydrogen.

### (Item 24)

The compound or the isomer thereof or the salt thereof of the preceding item, wherein
R₅ₐ and R_{5b} are each independently selected from hydrogen, C₁₋₈ alkyl, C₂₋₈ alkenyl, C₂₋₈ alkynyl, -Y-C₃₋₆ carbocyclyl, -W-(3- to 6-membered heterocyclyl), -(CR^{x}R^{y})ₛ-O-R^{z}, -O-(CR^{x}R^{y})ₙ-OR^{z}, - (CH₂)ₛ-CN and -C(=O)R^{x}, or R₅ₐ and R_{5b}, together with the carbon atom to which they are attached, can join to form a 3- to 5-membered carbocyclyl or 5- to 6-membered heterocyclyl group, wherein the C₁₋₈ alkyl, C₂₋₈ alkenyl and C₂₋₈ alkynyl groups are optionally substituted with one or more R^{b} groups, and each R^{b} is independently selected from fluorine, C₁₋₄ alkyl, C₃₋₄ alkynyl, -C₃₋₆ cycloalkyl, -O-C₁₋₄ alkyl, fluoroC₁₋₄ alkyl, haloC₁₋₄ alkoxy, hydroxy, hydroxyC₁₋₄ alkyl, -C(=O)NR^{x}R^{y}, - NR^{x}C(=O)C₁₋₄ alkyl, and -NR^{x}R^{y} groups.

### (Item 25)

The compound or the isomer thereof or the salt thereof of the preceding item, wherein R₅ₐ and R_{5b} are each independently hydrogen or methyl, or R₅ₐ and R_{5b}, together with the carbon atom to which they are attached, can join to form a cyclopropyl group.

### (Item 26)

The compound or the isomer thereof or the salt thereof of the preceding item, wherein
R₆ is selected from hydrogen, halogen, C₁₋₄ alkyl, C₂₋₅ alkenyl, C₂₋₅ alkynyl, -(CR^{x}R^{y})-C₃₋₇ carbocyclyl, -(CR^{x}R^{y})-(3- to 7-membered heterocyclyl), -(CR^{x}R^{y})-O-R^{z}, -O-(CR^{x}R^{y})ₙ-OR^{z}, -(CH₂)ₛ-CN, -S(O)₂-(CH₂)ₛ-R^{z}, -C(=O)R^{x}, -(CH₂)ₛ-NR^{x}R^{y}, -NR^{x}-(CH₂)ₛ-R^{z}, - (CH₂)ₙ-C₅₋₁₀ aryl, -(CH₂)ₙ-(5- to 10-membered heteroaryl), an optionally fused 5- to 7-membered carbocyclyl group, an optionally fused 5- to 7-membered heterocyclyl group, and 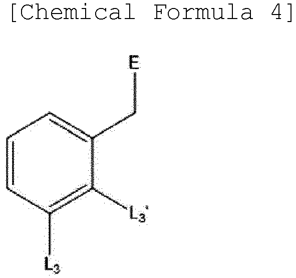
wherein E indicates a bonding point,
L₃ and L₃', together with the carbon atoms to which they are attached, can join to form a 3- to 6-membered carbocyclyl or 4-to 6-membered heterocyclyl group that can be connected to a linker, wherein the C₁₋₄ alkyl, C₂₋₅ alkenyl and C₂₋₅ alkynyl groups, carbocyclyl group, heterocyclyl group, aryl group, heteroaryl group, and 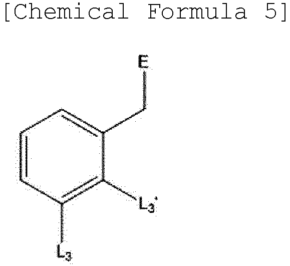
wherein E indicates a bonding point, are optionally substituted with one or more R^{b} groups,
each R^{b} is independently selected from halogen, C₁₋₄ alkyl, C₃₋₄ alkynyl, -(CH₂)_{q}-C₃₋₆ cycloalkyl, -(CH₂)_{q}-phenyl, -(CH₂)_{q}-(5- to 6-membered heteroaryl), -(CH₂)_{q}-O-C₁₋₄ alkyl, haloC₁₋₄ alkyl, haloC₁₋₄ alkoxy, hydroxy, hydroxyC₁₋₄ alkyl, =O, -S(O)_{q}-C₁₋₄ alkyl, -C(=O)C₁₋₄ alkyl, -(CH₂)_{q}-C(=O)NR^{x}R^{y}, -(CH₂)_{q}-NR^{x}C(=O)C₁₋₄ alkyl, -(CH₂)ₛ-NR^{x}R^{y}, -NR^{x}- (CH₂)_{q}-C₁₋₄ alkyl, -(CH₂)_{q}-NR^{x}-(CH₂)_{q}-SO₂-C₁₋₄ alkyl, and -(CH₂)_{q}-SO₂NR^{x}R^{y} groups, wherein the cycloalkyl, phenyl and heteroaryl groups are optionally substituted with one or more R^{x} groups,
the CR^{x}R^{y} is CH₂ or CF₂, and the other R^{x}, R^{y} and R^{z} are each independently selected from hydrogen, C₁₋₄ alkyl, C₃₋₄ alkynyl, fluoroC₁₋₄ alkyl, and C₁₋₄ alkoxy, and
the R^{x} group as a substituent of R^{b} is selected from halogen, hydrogen, C₁₋₄ alkyl, and C₁₋₄ alkoxy.

### (Item 27)

The compound or the isomer thereof or the salt thereof of the preceding item, wherein
R₆ is selected from -(CR^{x}R^{y})-H, -(CR^{x}R^{y})-F, -(CR^{x}R^{y})-C₁₋₂ alkyl, - (CR^{x}R^{y})-O-phenyl, -S(O)₂-CH₂-R^{z}, -C(=O)C₁₋₄ alkyl, -C (=O) -phenyl, -C (=O) - (5-6-membered heteroaryl), -CH₂-phenyl, -CH₂-(5-6-membered heteroaryl), and 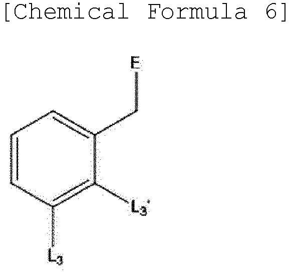
wherein E indicates a bonding point,
L₃ and L₃', together with the carbon atoms to which they are attached, can join to form a 3- to 6-membered carbocyclyl or 4-to 6-membered heterocyclyl group that can be connected to a linker, wherein the C₁₋₂ alkyl, C₁₋₄ alkyl and phenyl groups, heteroaryl group, and 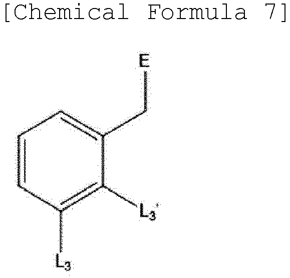
wherein E indicates a bonding point, are optionally substituted with one or more C₁₋₃ alkyl groups, fluorine, chlorine, or -O-C₁₋₃ alkyl groups, and
the CR^{x}R^{y} is CH₂ or CF₂, and the other R^{x}, R^{y} and R^{z} are each independently selected from hydrogen, C₁₋₄ alkyl, C₃₋₄ alkynyl, fluoroC₁₋₄ alkyl, and C₁₋₄ alkoxy.

### (Item 28)

The compound or the isomer thereof or the salt thereof of the preceding item, wherein
R₇ is selected from hydrogen, halogen, C₁₋₃ alkyl, C₂₋₃ alkenyl, C₂₋₃ alkynyl and C₃₋₄ carbocyclyl, wherein the C₁₋₃ alkyl, C₂₋₃ alkenyl, C₂₋₃ alkynyl and C₃₋₄ carbocyclyl are optionally substituted with one or more R^{b} groups,
each R^{b} is independently selected from fluorine, -O-C₁₋₄ alkyl, fluoroC₁₋₄ alkoxy, hydroxy, and -NR^{x}R^{y} groups, and
R^{x} and R^{y} are each independently selected from hydrogen, and C₁₋₄ alkyl, and when attached to nitrogen, R^{x} and R^{y}, together with the atom(s) to which they are attached, may join to form a 4-to 6-membered ring optionally containing one additional heteroatom selected from O and N.

### (Item 29)

The compound or the isomer thereof or the salt thereof of the preceding item, wherein R₇ is hydrogen or fluorine.

### (Item 30)

The compound or the isomer thereof or the salt thereof of the preceding item, wherein R₈ is hydrogen.

### (Item 31)

The compound of any one of the preceding items, wherein
R₉ is selected from hydrogen, halogen, C₁₋₄ alkyl, C₂₋₄ alkenyl, C₂₋₄ alkynyl, -Y-C₃₋₆ carbocyclyl, -W-(4- to 6-membered heterocyclyl), -(CR^{x}R^{y})ₛ-O-R^{z}, -O-(CR^{x}R^{y})ₙ-OR^{z}, =O, -(CH₂)ₛ-CN, - C(=O)R^{x}, -(CR^{x}R^{y})ₛ-C(=O)NR^{x}R^{y}, -(CH₂)ₛ-NR^{x}C(=O)R^{y}, -(CH₂)ₛ-NR^{x}R^{y}, - (CH₂)ₛ-O-C(=O)-C₁₋₄ alkyl-NR^{x}R^{y}, -(CH₂)ₛ-NR^{x}-(CH₂)ₛ-SO₂-R^{y}, -(CH₂)ₛ-NH-SO₂-NR^{x}R^{y}, -(CH₂)ₛ-SO₂NR^{x}R^{y}, and -Y-C₅₋₁₀ aryl, wherein the C₁₋₄ alkyl, C₂₋₄ alkenyl, C₂₋₄ alkynyl, carbocyclyl and heterocyclyl groups are optionally substituted with one or more R^{b} groups, each R^{b} is independently selected from fluorine, C₁₋₄ alkyl, C₃₋₄ alkynyl, C₃₋₆ cycloalkyl, phenyl, 5- to 6-membered heteroaryl, - O-C₁₋₄ alkyl, fluoroC₁₋₄ alkyl, fluoroC₁₋₄ alkoxy, hydroxy, hydroxyC₁₋₄ alkyl, =O, -C(=O)C₁₋₄ alkyl, -C(=O)NR^{x}R^{y}, -NR^{x}C(=O)C₁₋₄ alkyl, and -NR^{x}R^{y} groups, wherein the cycloalkyl, phenyl and heteroaryl groups are optionally substituted with one or more R^{x} groups, and
R^{x}, R^{y} and R^{z} are each independently selected from chlorine, fluorine, hydrogen, C₁₋₄ alkyl, and C₁₋₄ alkoxy, and when attached to nitrogen or carbon, R^{x} and R^{y}, together with the atom(s) to which they are attached, may join to form a 3- to 7-membered ring optionally containing one additional heteroatom selected from O and N.

### (Item 32)

The compound of any one of the preceding items, wherein
R₉ is selected from hydrogen, C₁₋₄ alkyl, C₂₋₄ alkenyl, C₂₋₄ alkynyl, -CH₂-C₃₋₆ carbocyclyl, -CH₂-(4- to 6-membered heterocyclyl), -CH₂-O-R^{z}-C(=O)R^{x}, and -CH₂-NR^{x}R^{y}, wherein the C₁₋₄ alkyl, C₂₋₄ alkenyl, C₂₋₄ alkynyl, carbocyclyl and heterocyclyl groups are optionally substituted with one or more R^{b} groups, each R^{b} is independently selected from fluorine, C₁₋₄ alkyl, C₃₋₄ alkynyl, phenyl, 5- to 6-membered heteroaryl, -O-C₁₋₄ alkyl, fluoroC₁₋₄ alkyl, fluoroC₁₋₄ alkoxy, hydroxy, and -NR^{x}R^{y} groups, wherein the phenyl and heteroaryl groups are optionally substituted with one or more R^{x} groups, and
R^{x}, R^{y} and R^{z} are each independently selected from chlorine, fluorine, hydrogen, C₁₋₄ alkyl, and C₁₋₄ alkoxy, and when attached to nitrogen or carbon, R^{x} and R^{y}, together with the atom(s) to which they are attached, may join to form a 3- to 7-membered ring optionally containing one additional heteroatom selected from O and N.

### (Item 33)

The compound of the preceding item, wherein R₁₀ is hydrogen or methyl.

### (Item 34)

The compound of any one of the preceding items, which is represented by the following structure:

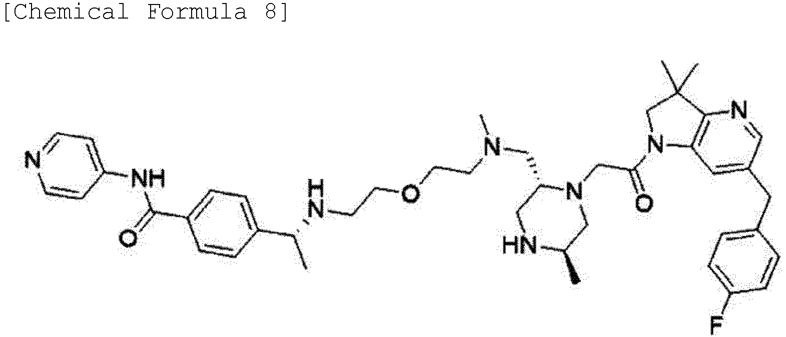

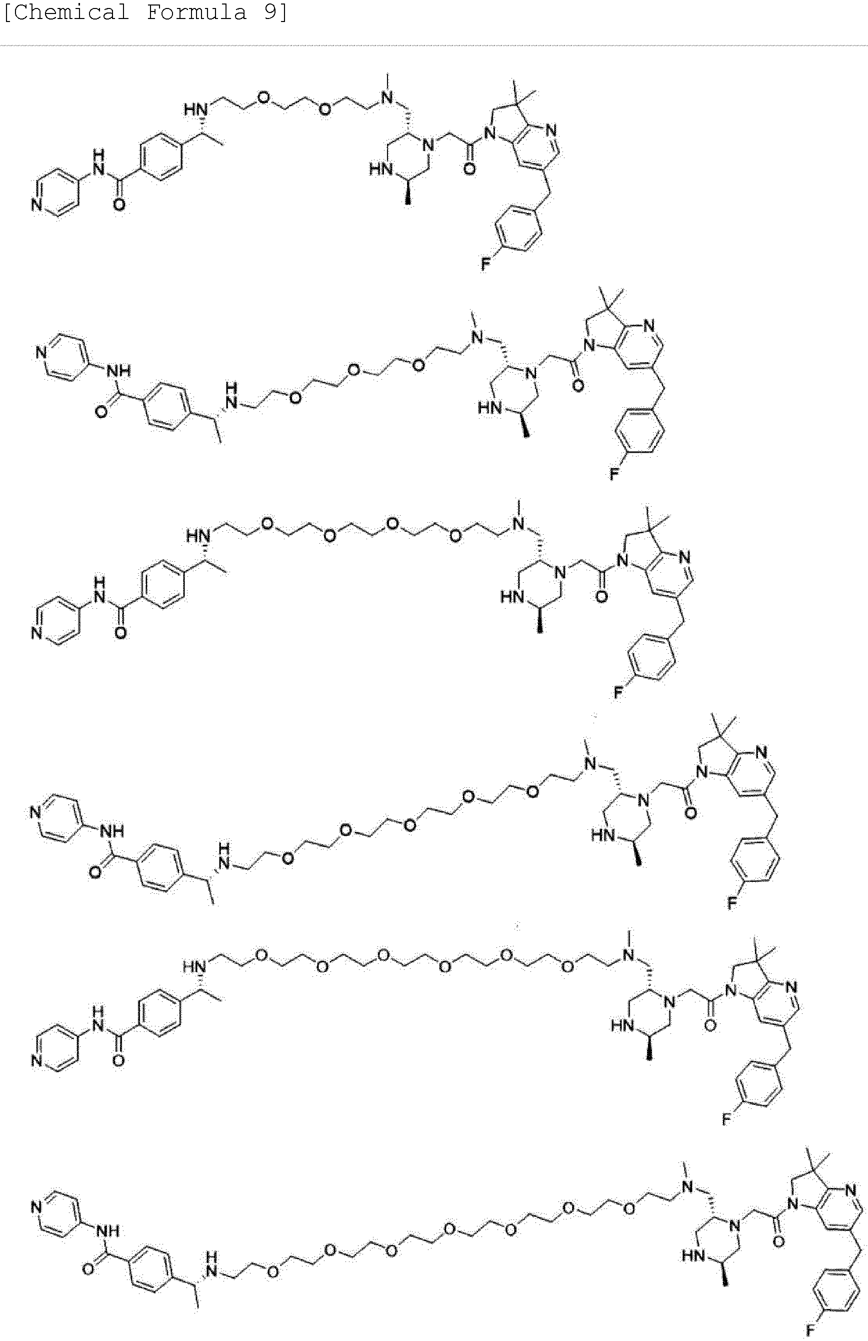

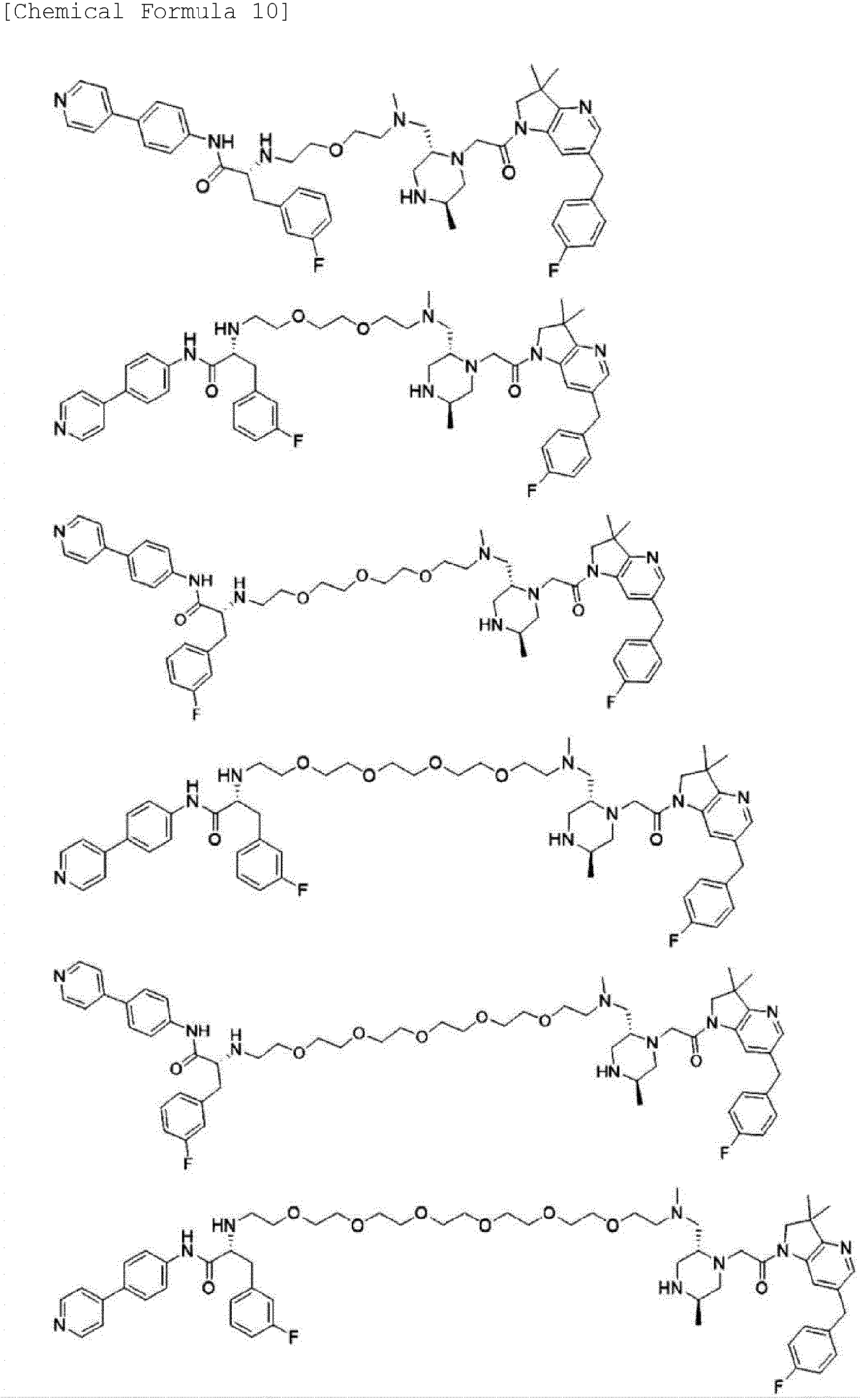

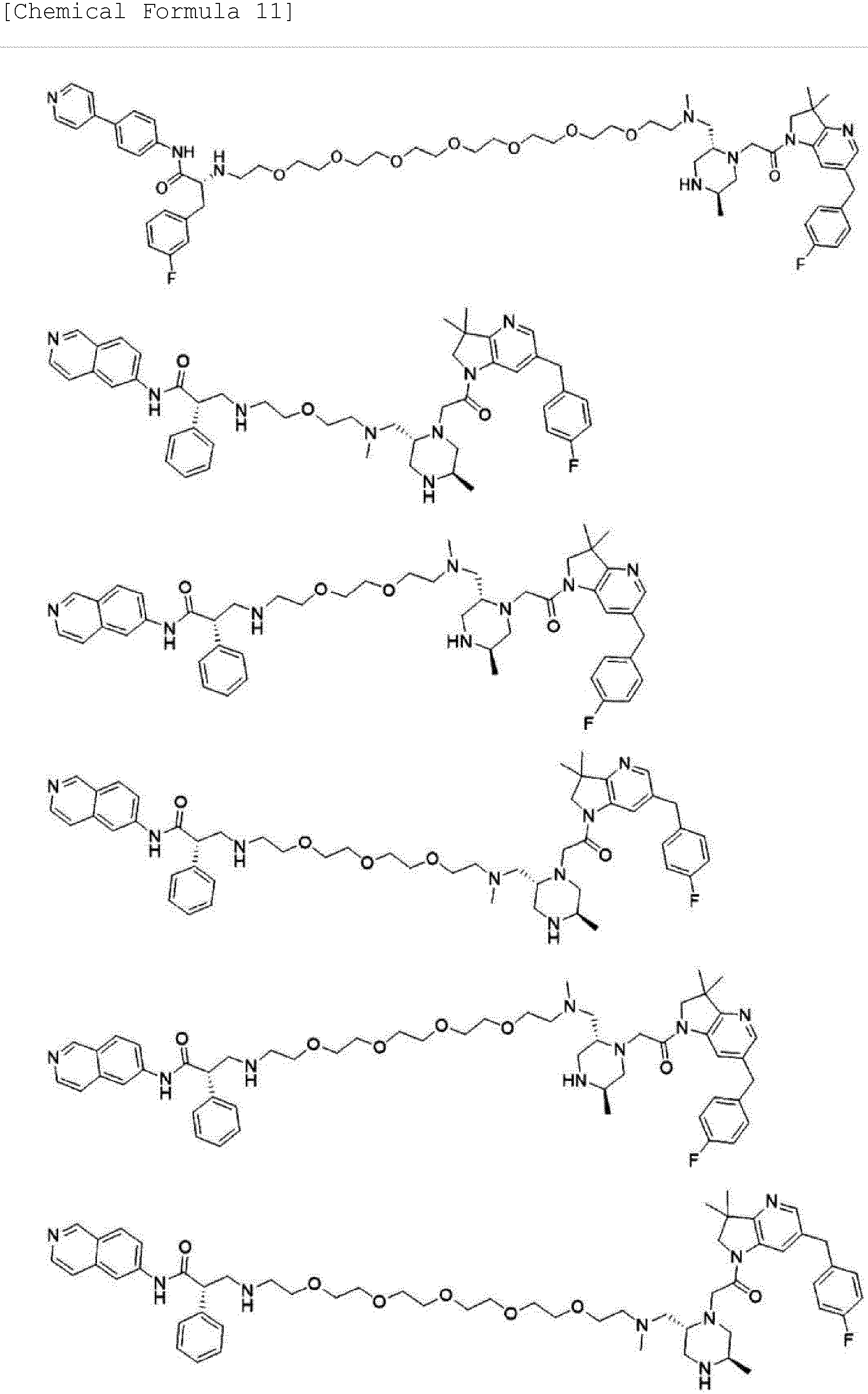

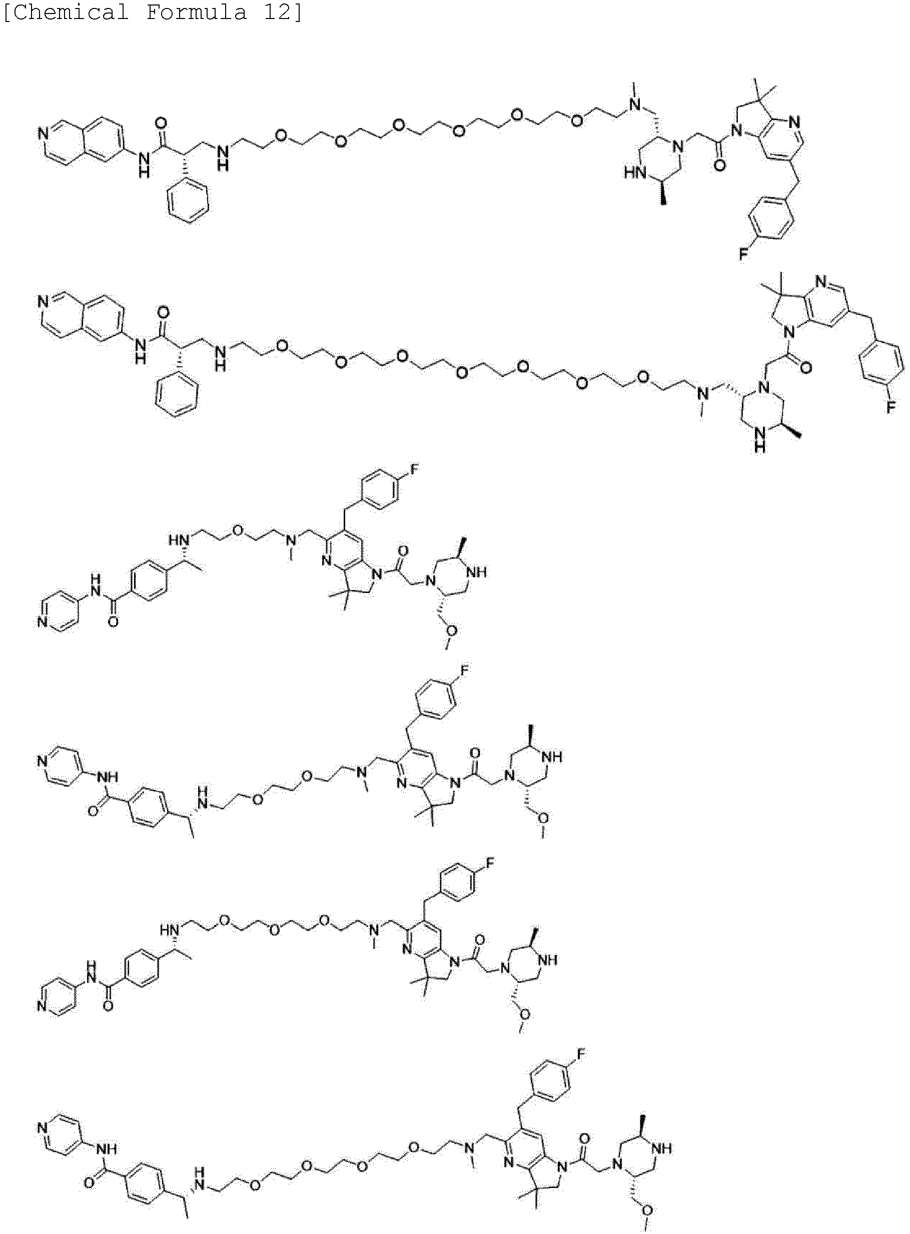

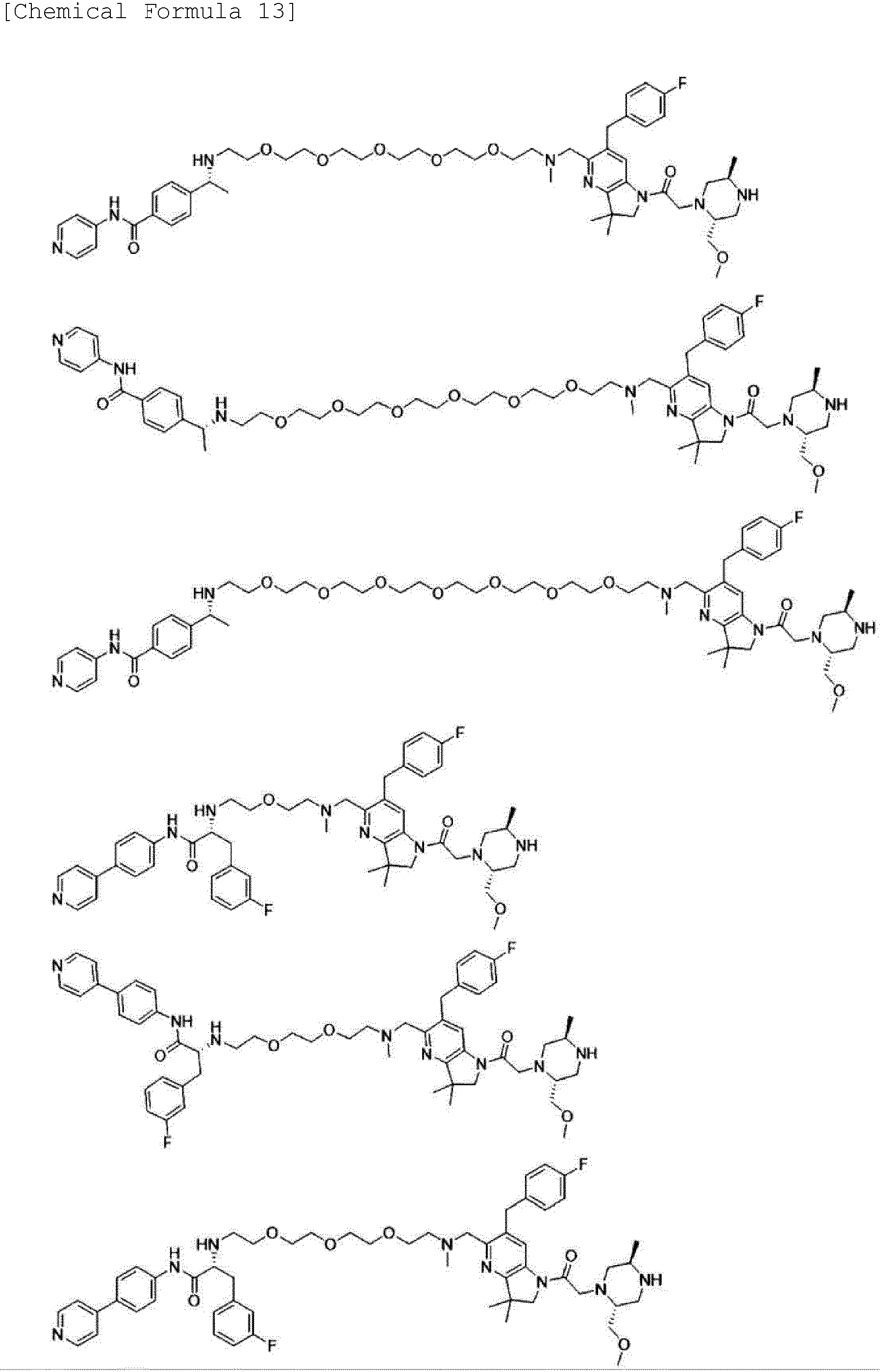

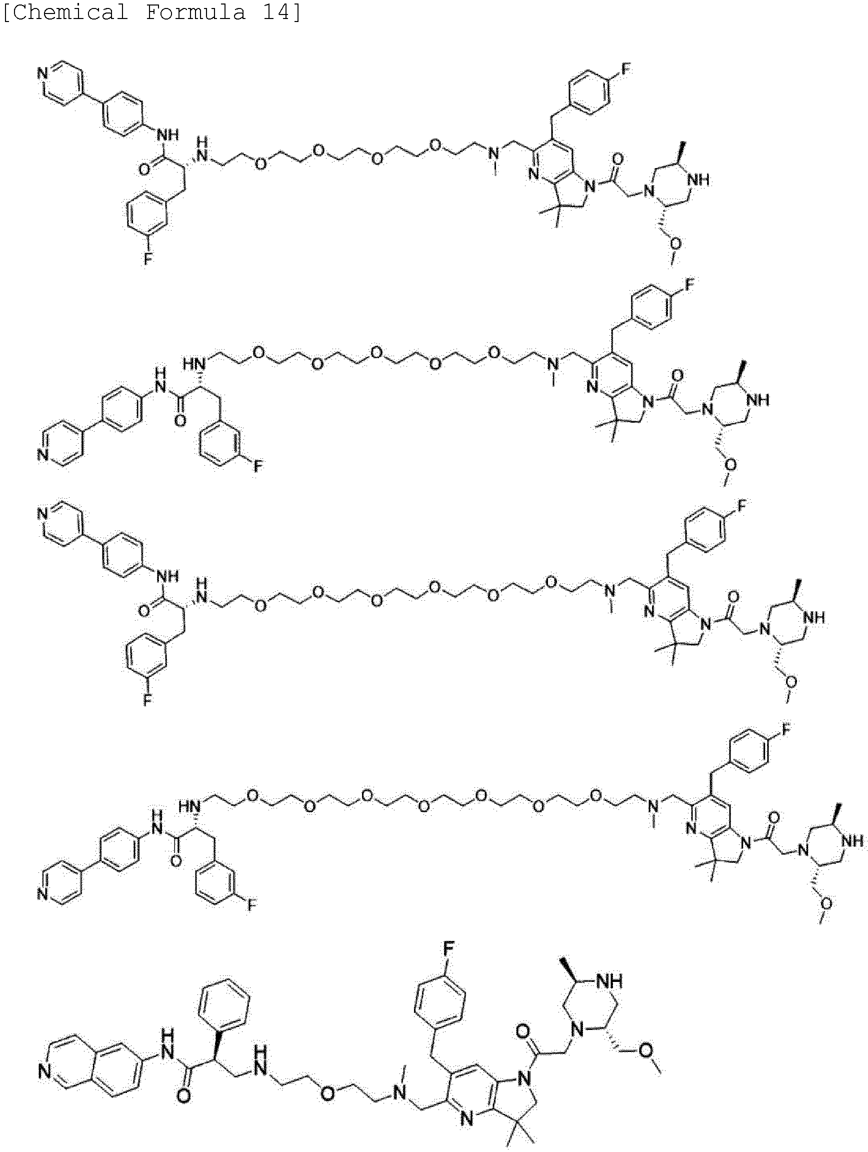

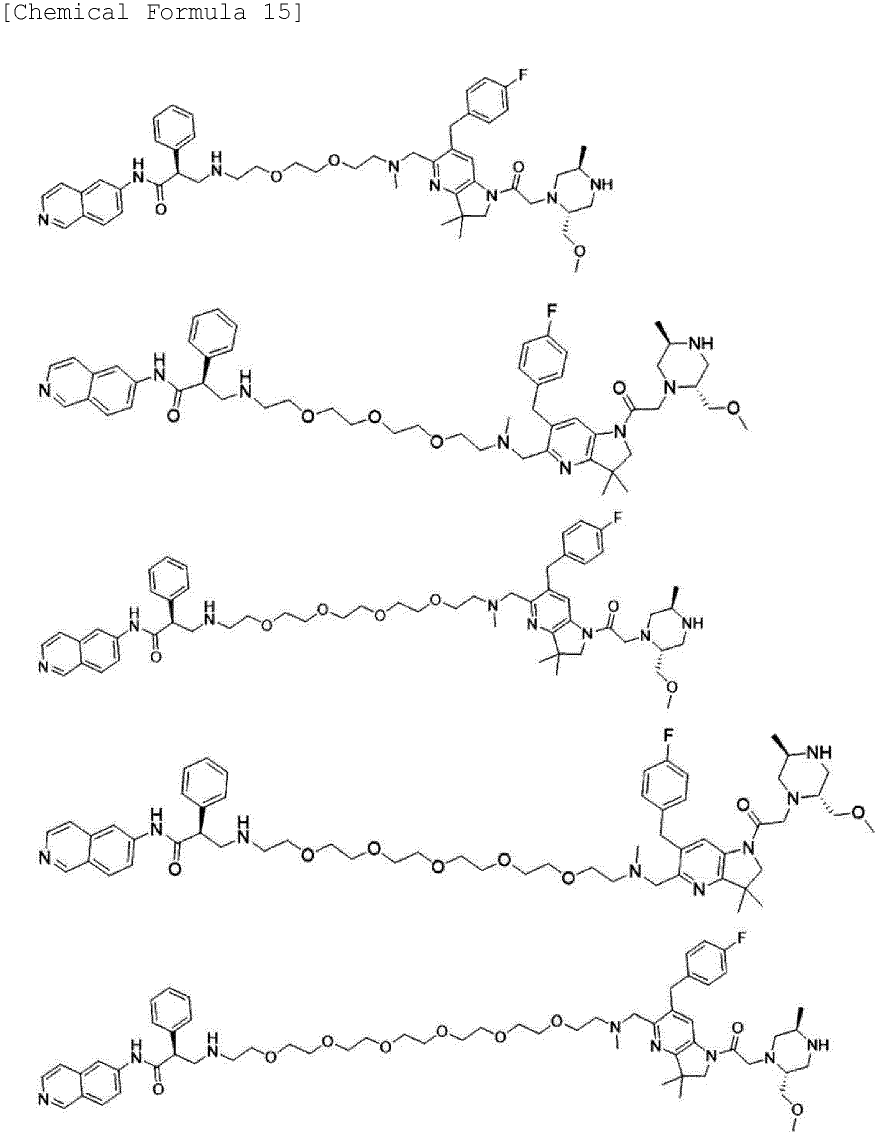

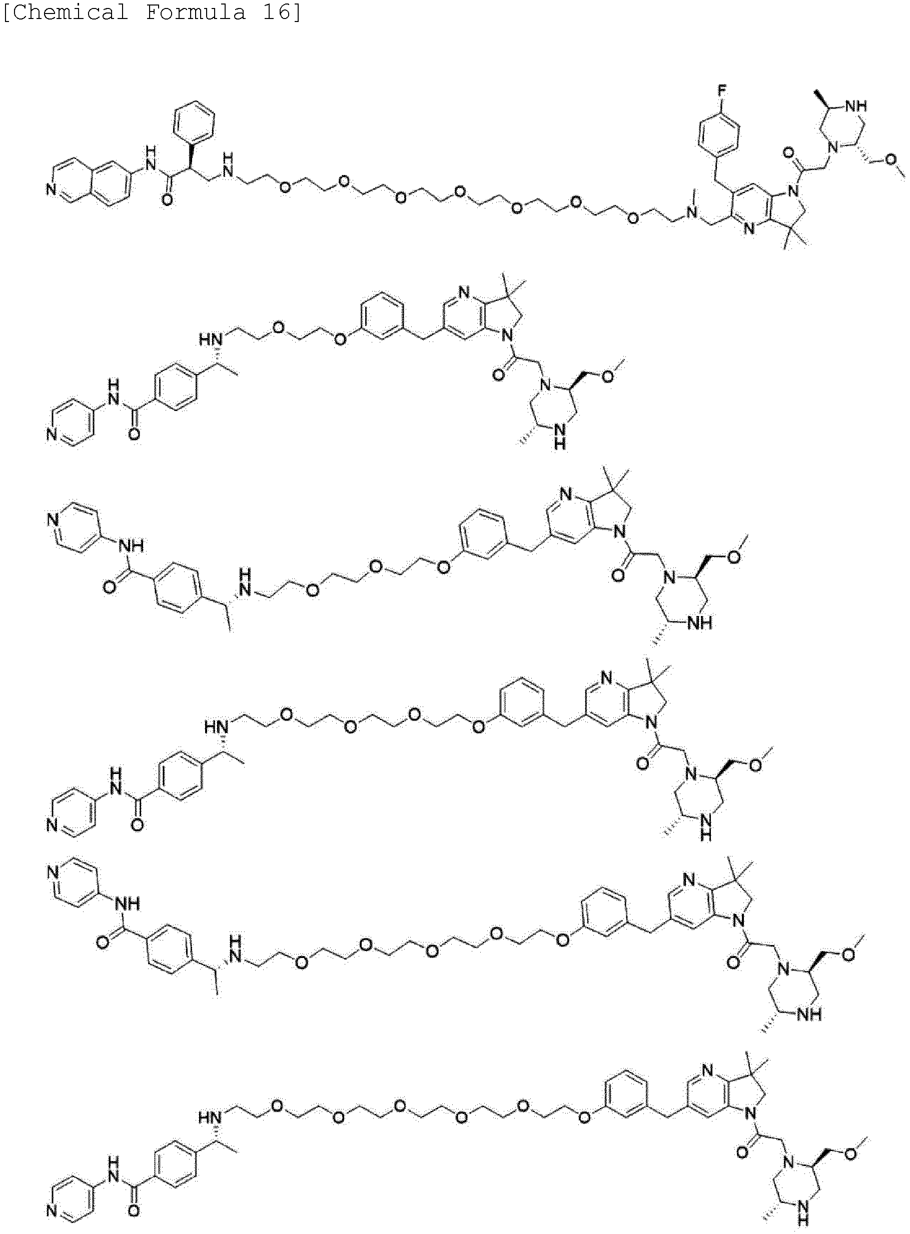

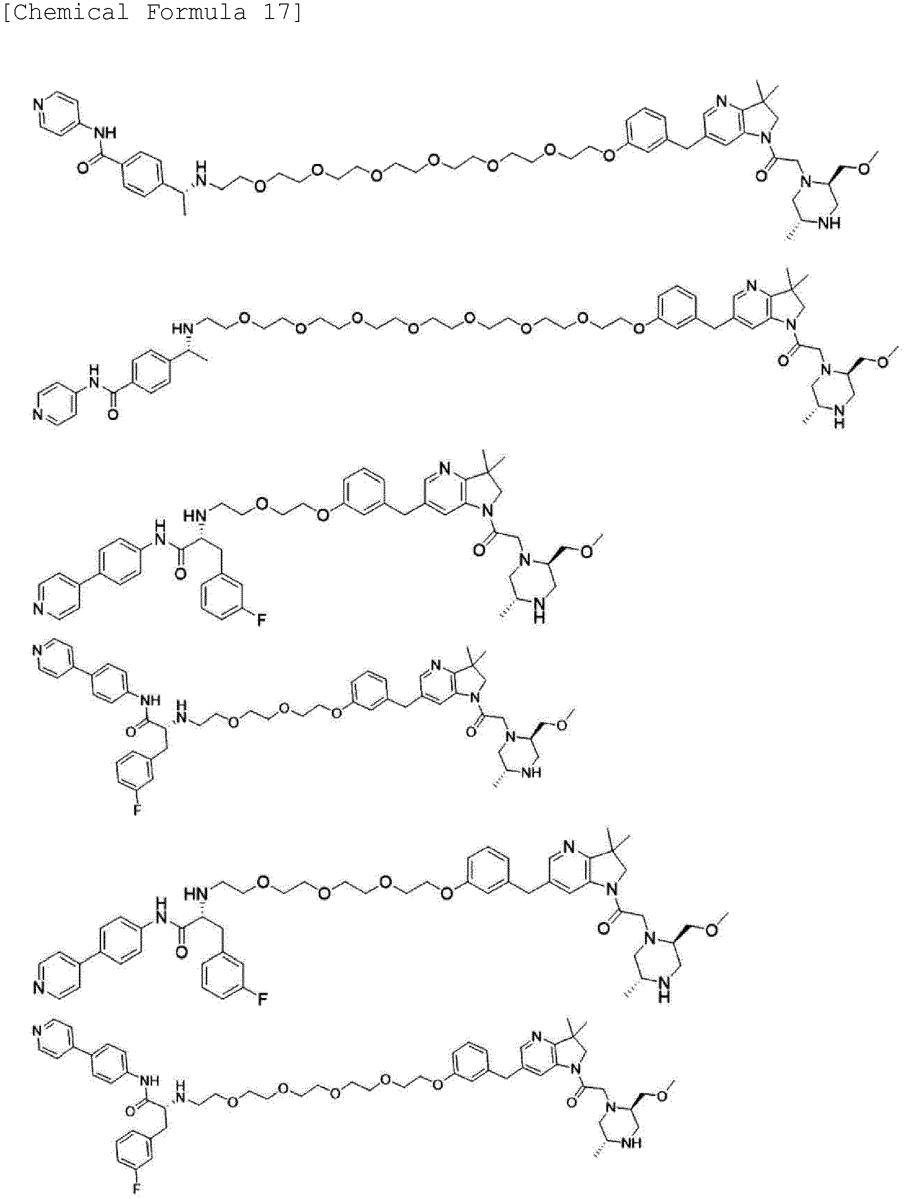

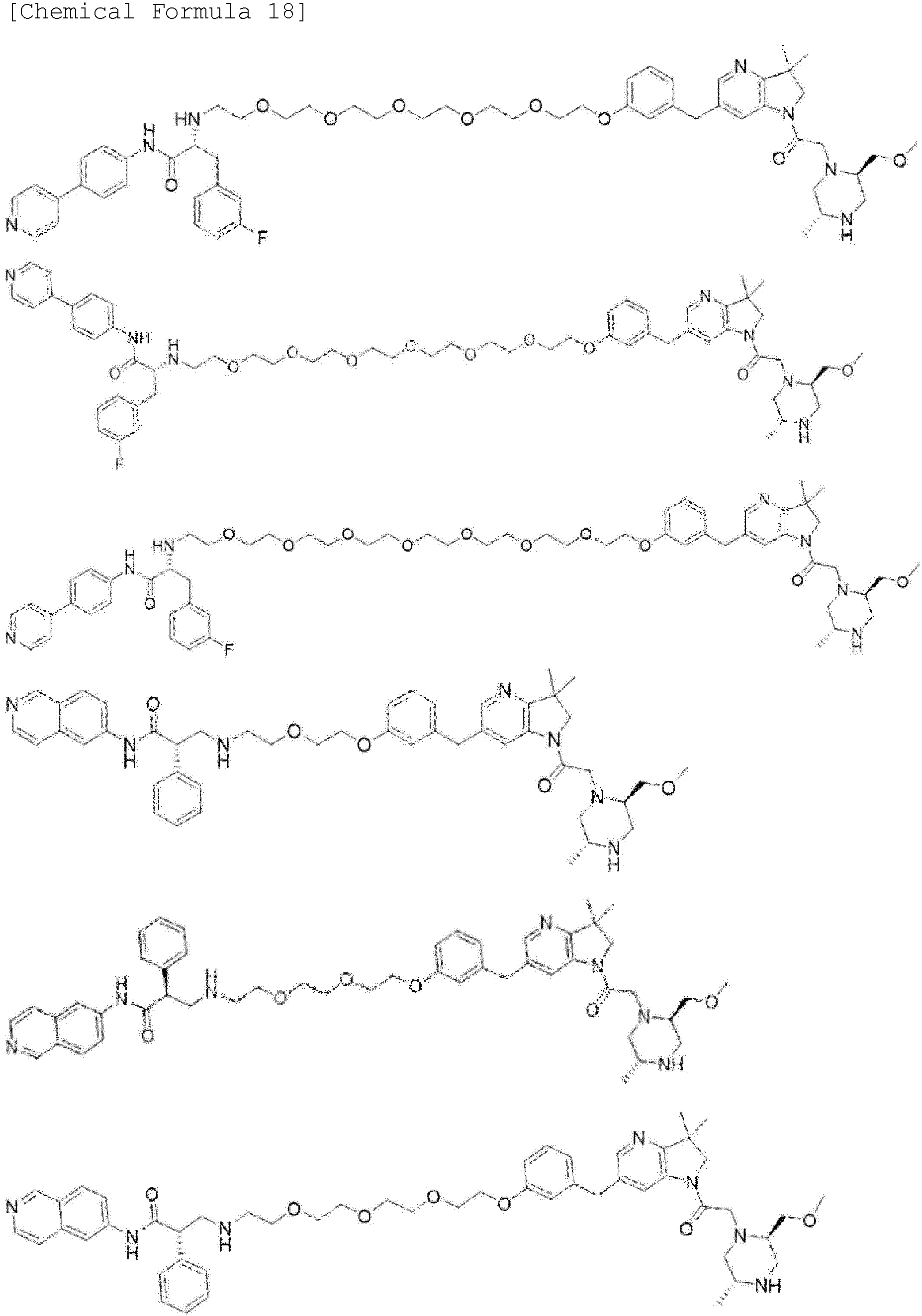

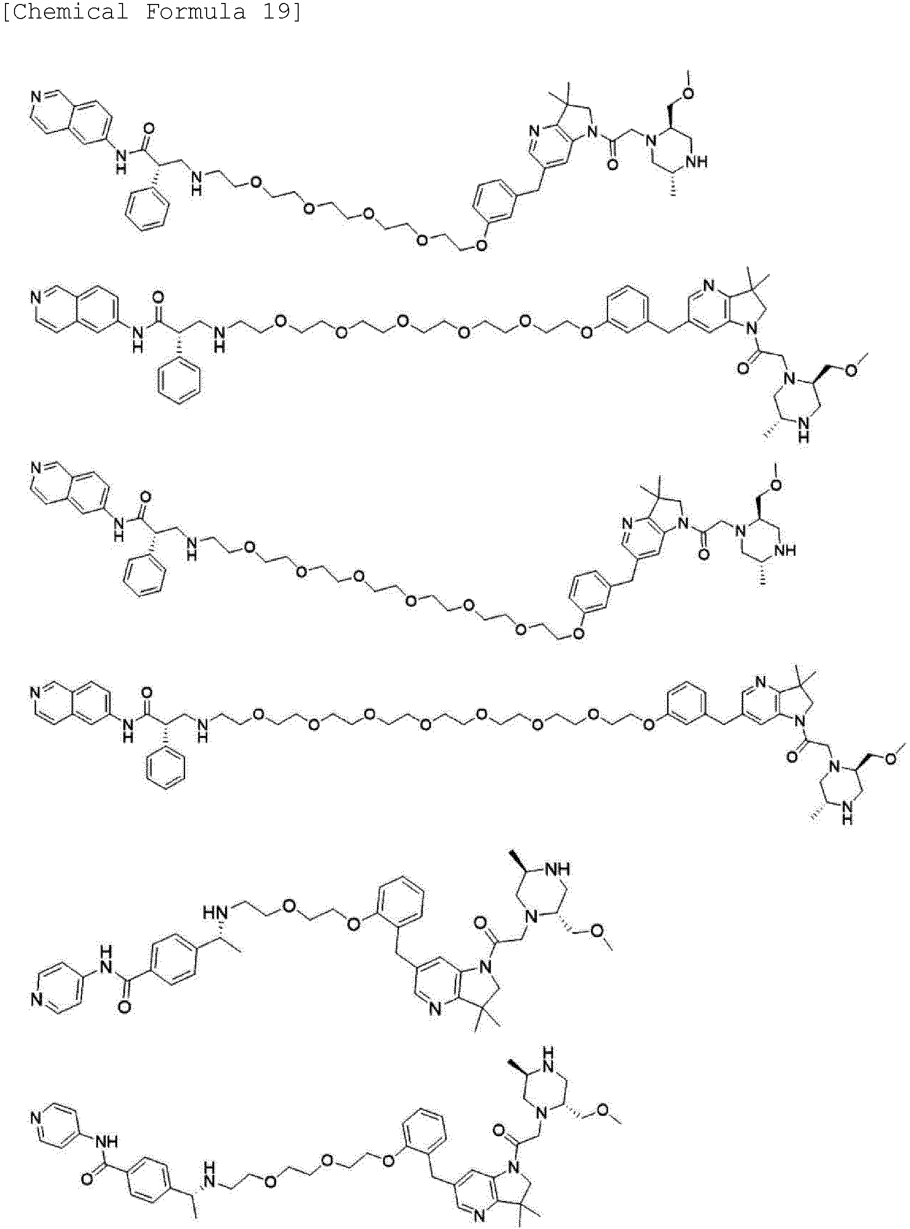

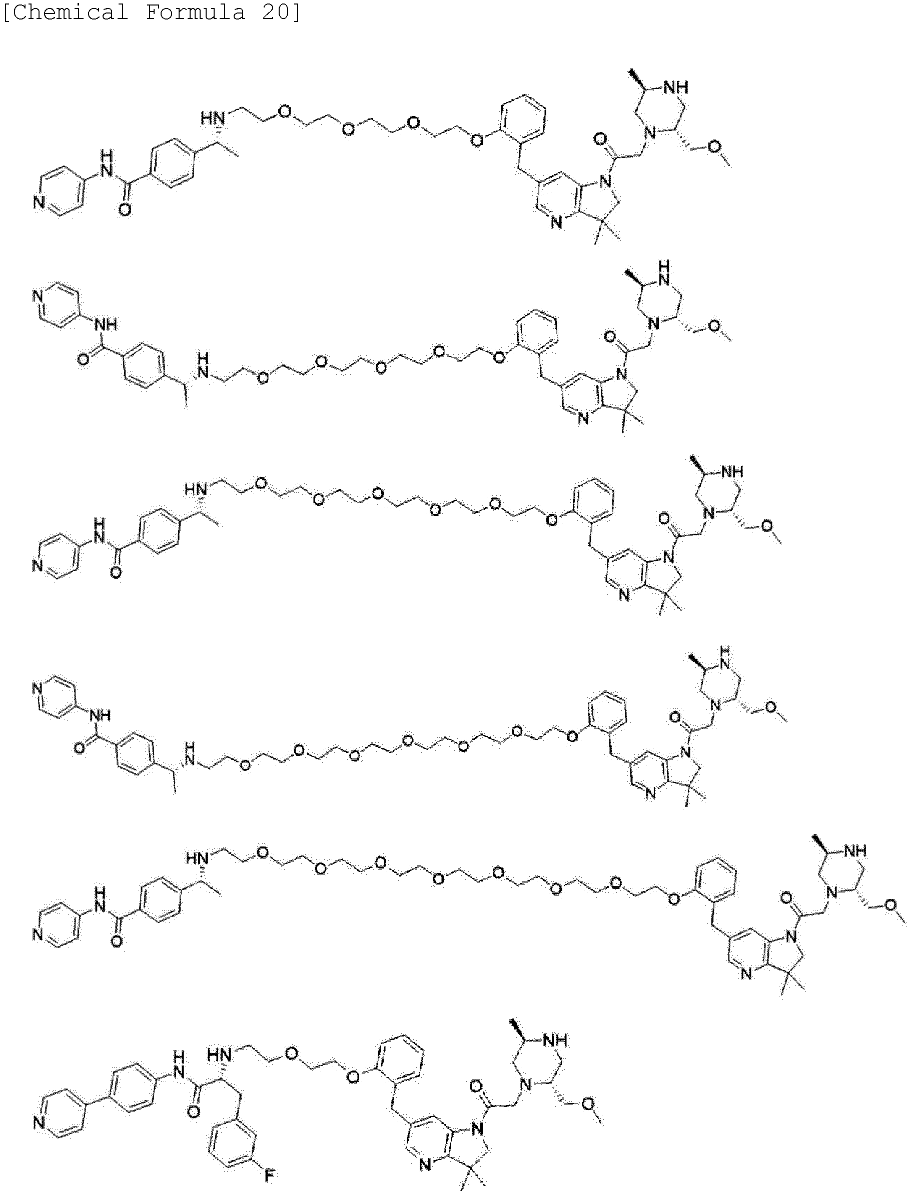

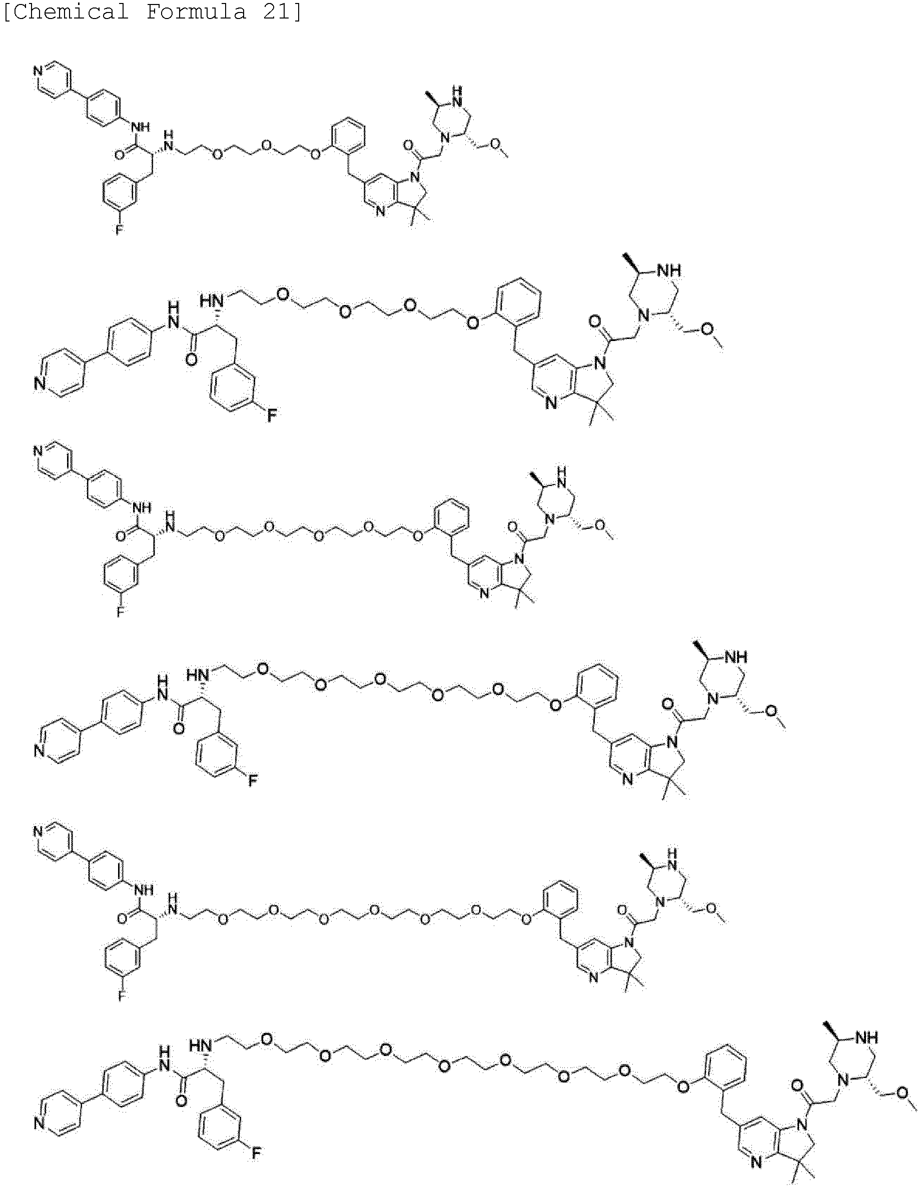

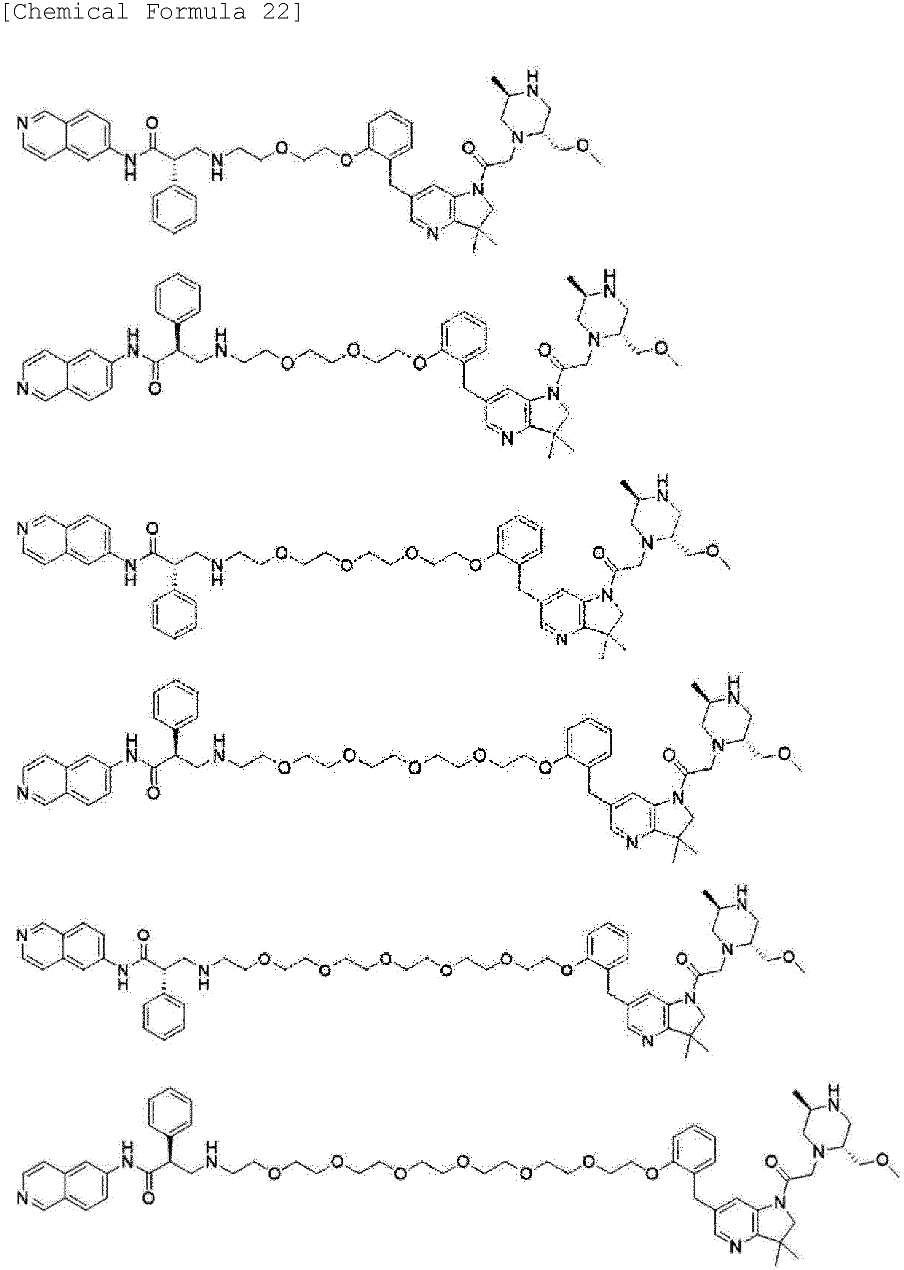

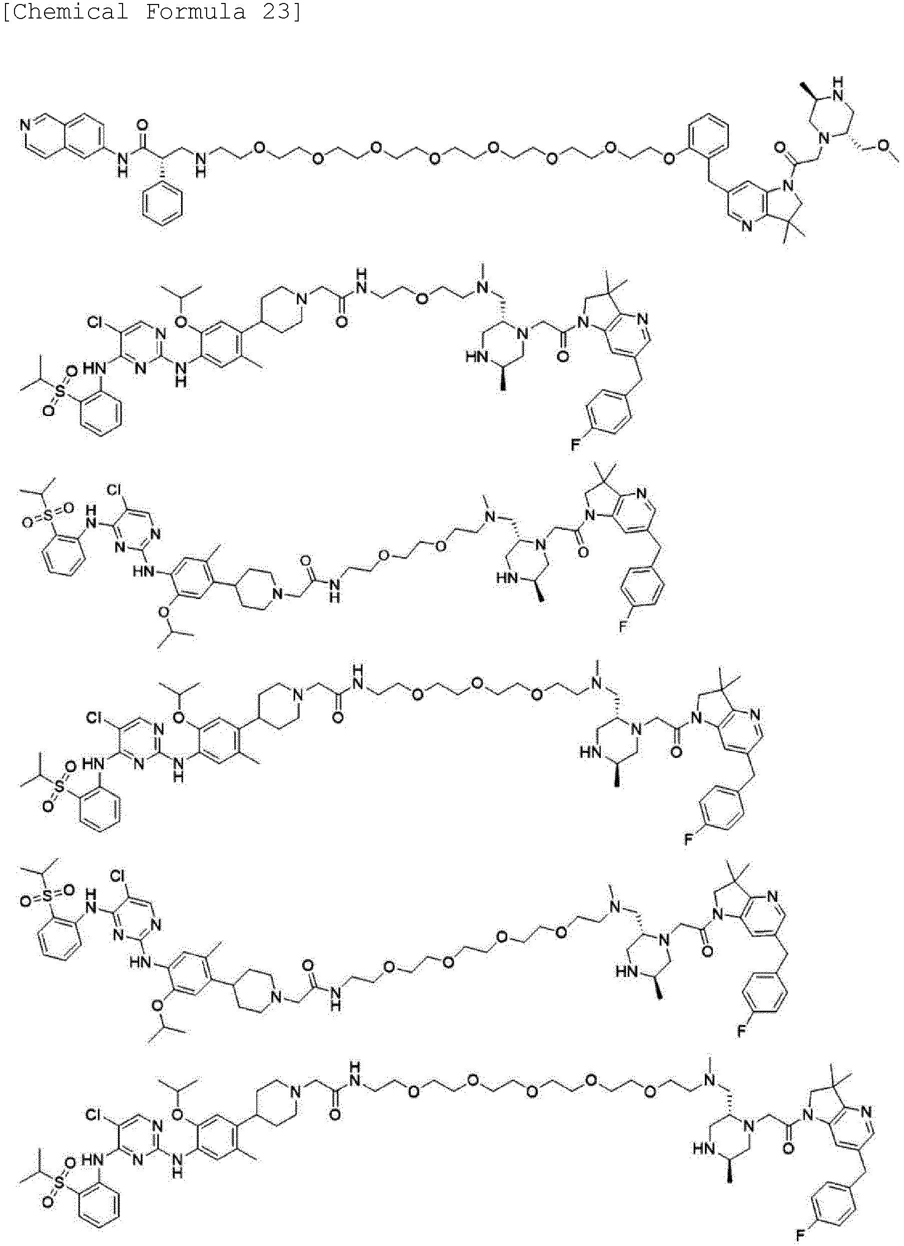

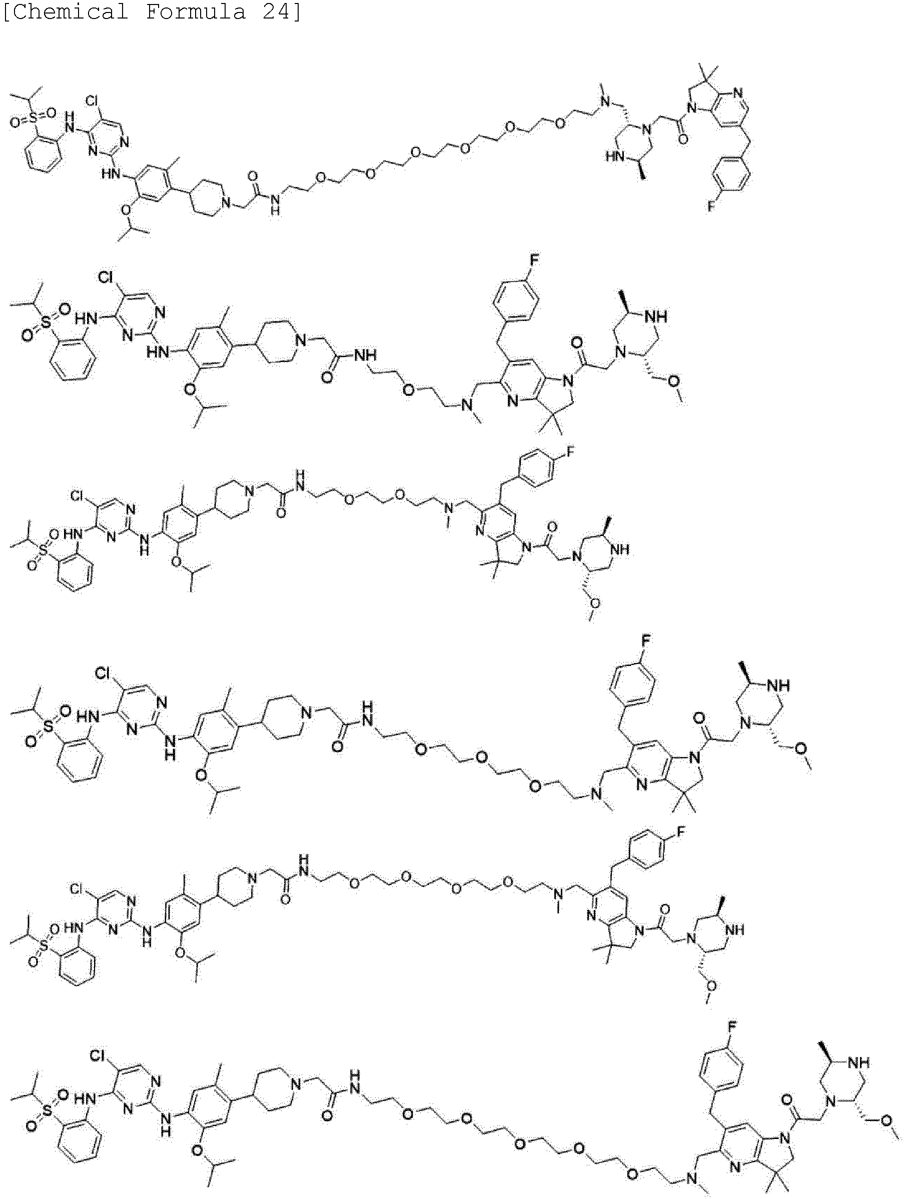

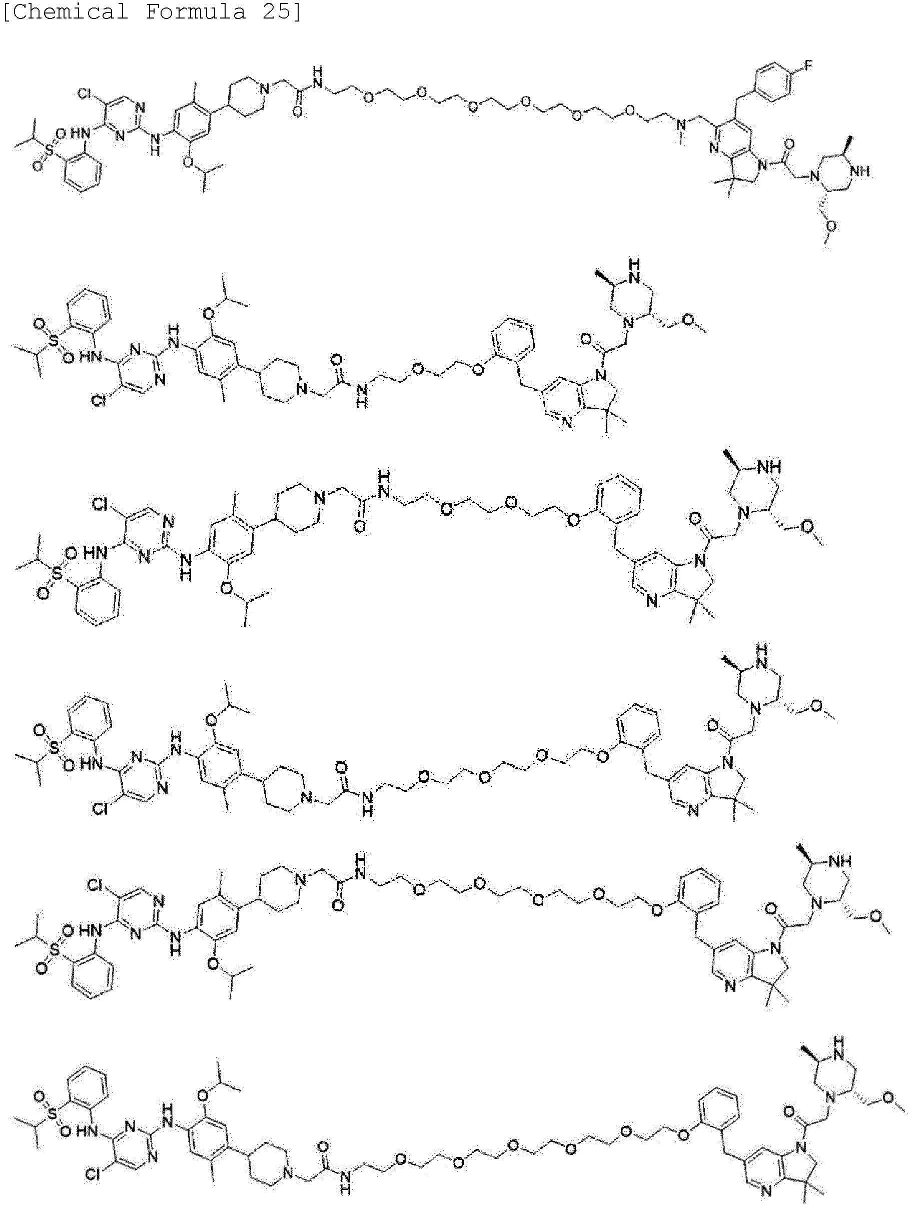

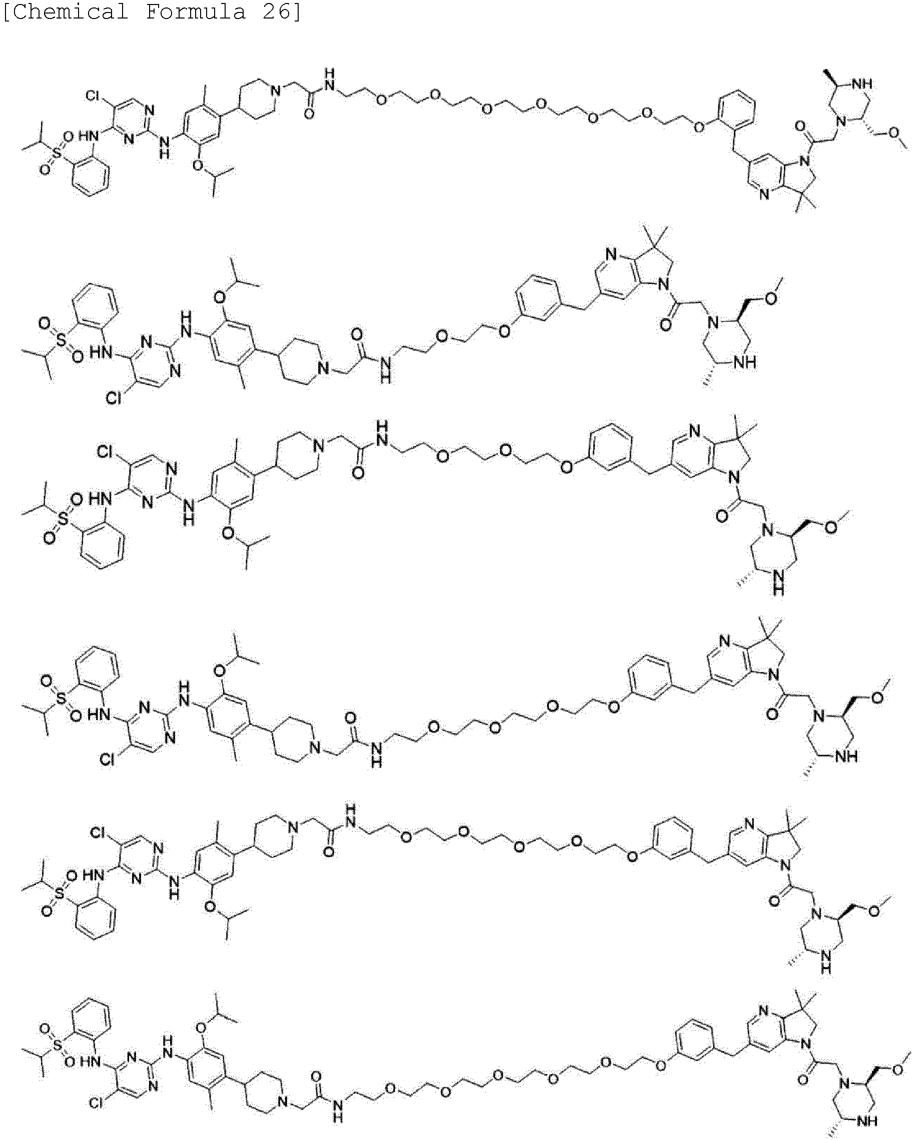

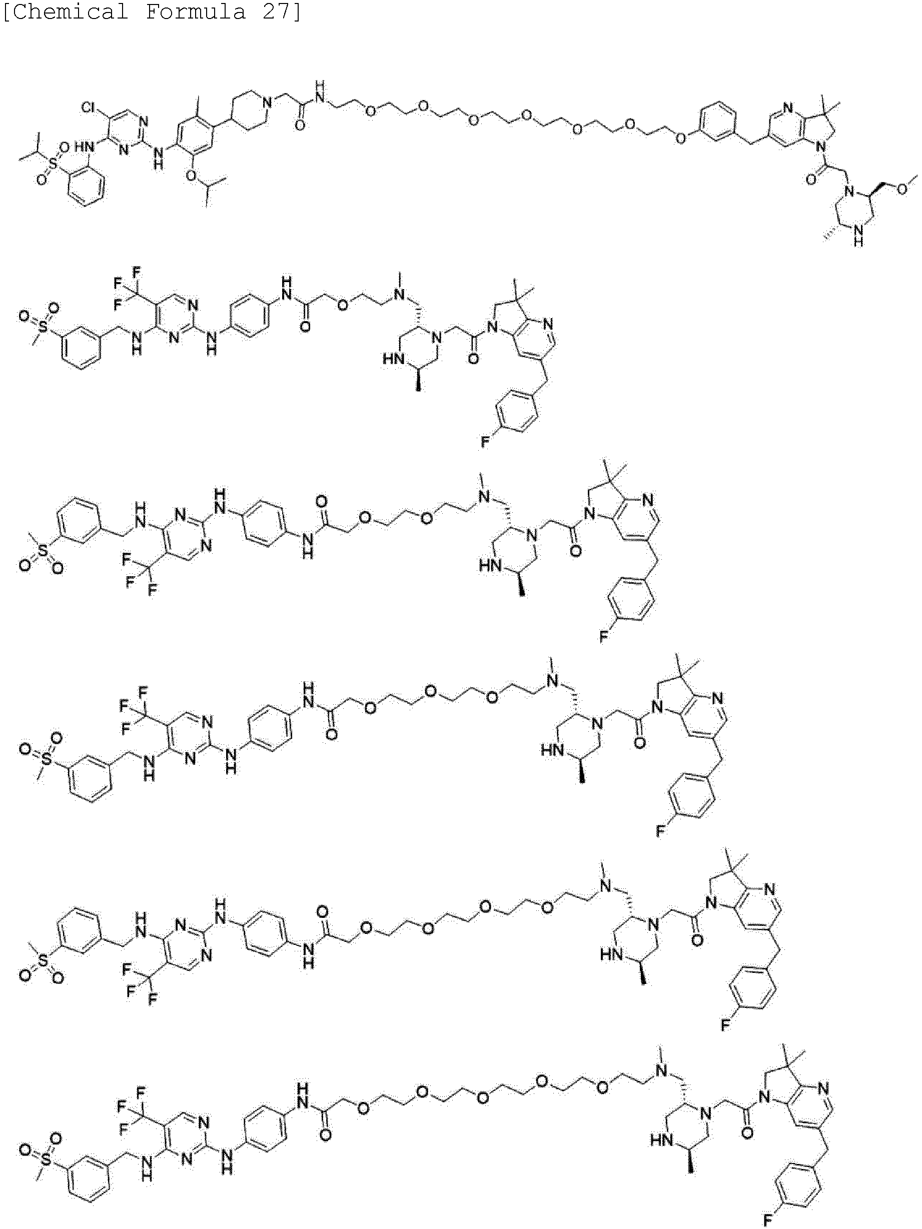

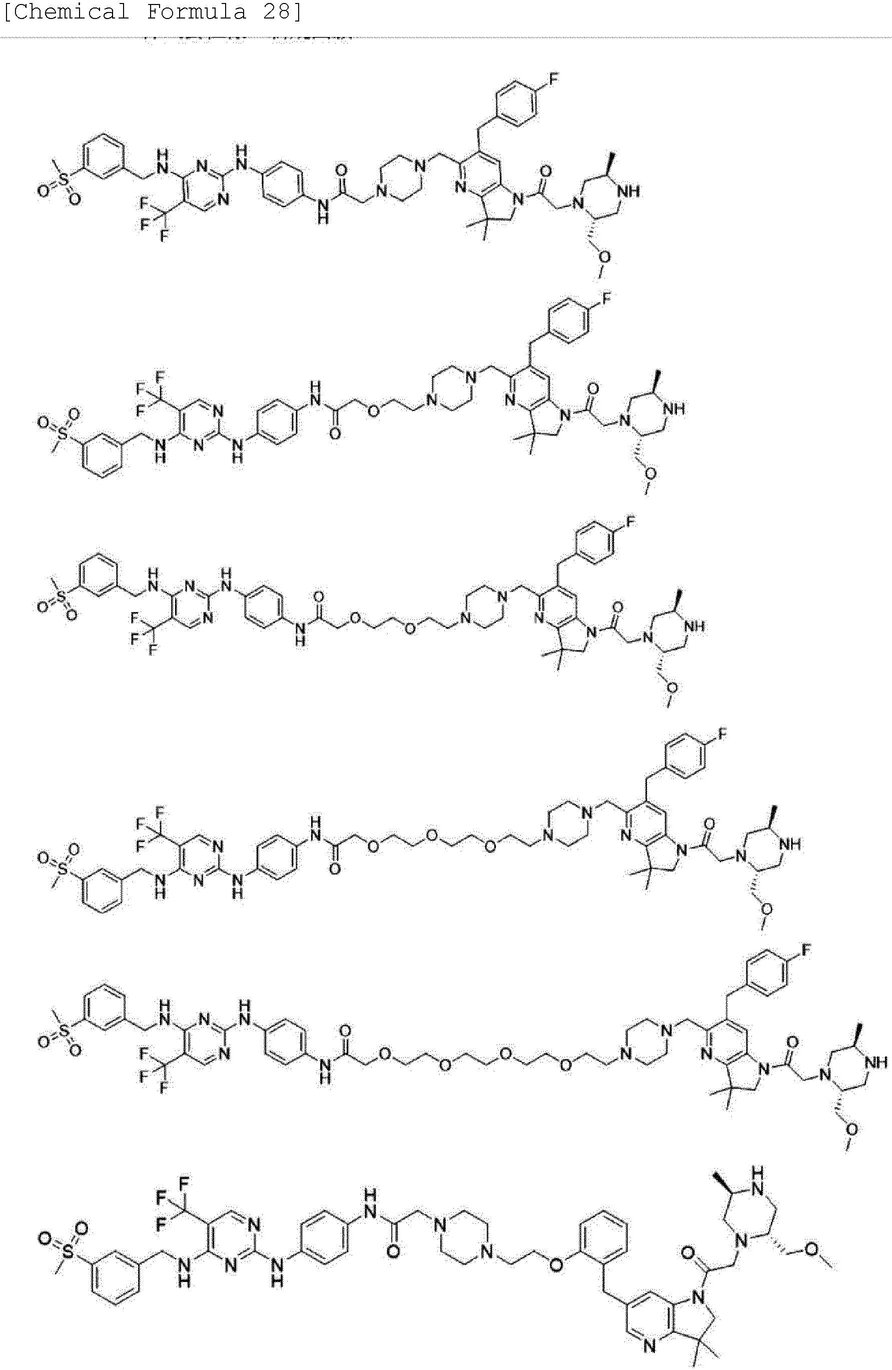

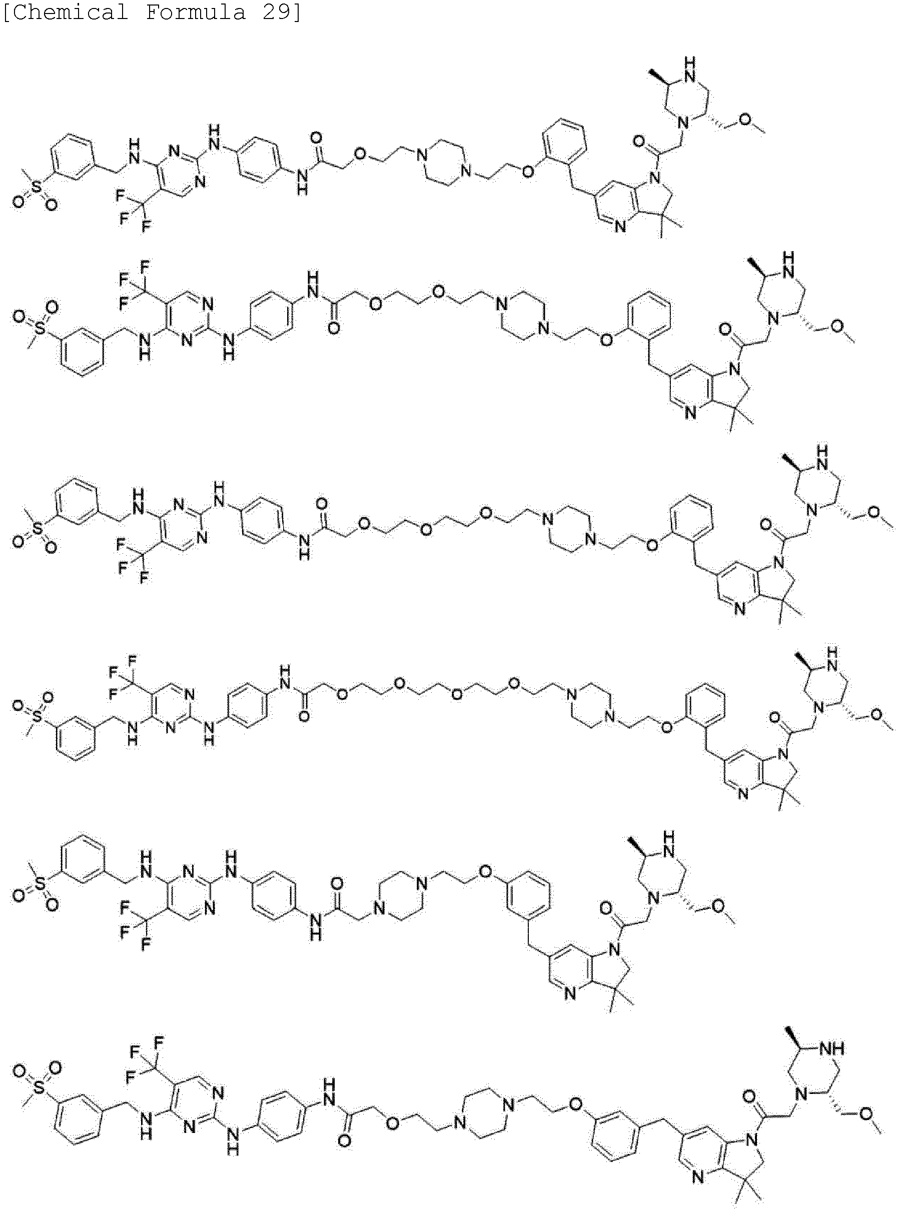

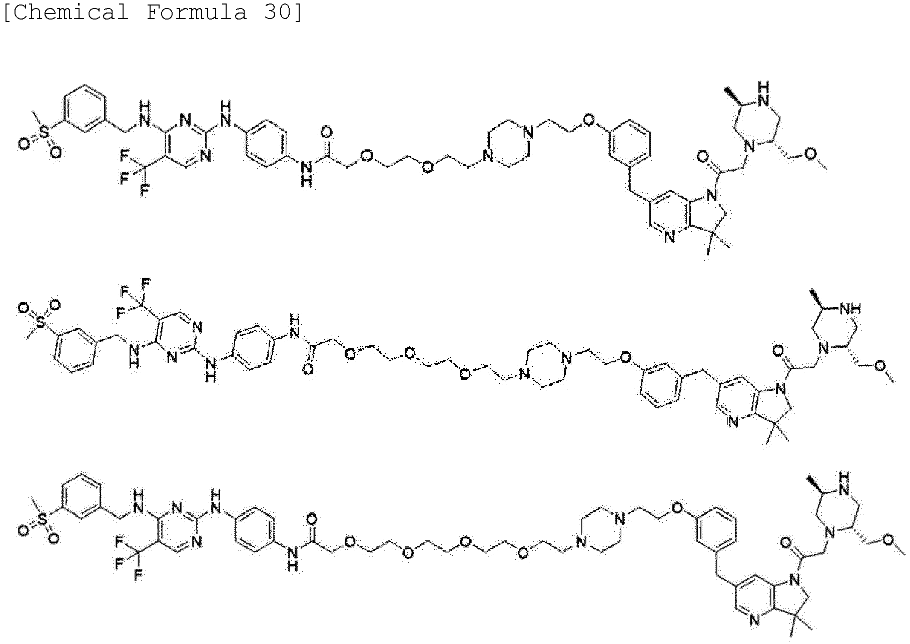

### (Item 35)

A pharmaceutical composition comprising the compound or the isomer thereof or the salt thereof of any one of the preceding items.

### (Item 36)

A target protein modulator comprising the compound or the isomer thereof or the salt thereof of any one of the preceding items.

### (Item 37)

A process for producing a compound of Formula (I) as defined in any one of the preceding items, comprising:
(a) reacting a compound of Formula (III) 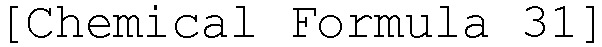 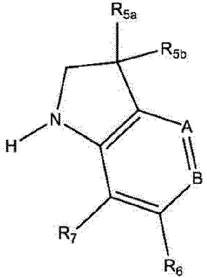
   wherein A, B, R₅ₐ, R_{5b}, R₆, and R₇ are as defined in any of the preceding items,
   with a compound of Formula (IV): 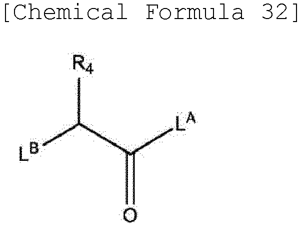
   wherein R₄ is as defined in any of the preceding items, and L^{A} and L^{B} each independently represent a leaving group,
   followed by a subsequent reaction with a compound of Formula (V): 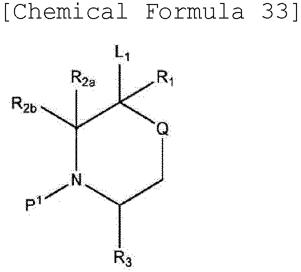
   wherein R₁, R₂ₐ, R_{2b}, and R³ are as defined in any of the preceding items, L₁ is not used for bonding to L, and P¹ represents hydrogen or a protecting group, and
   followed by a deprotection reaction suitable for removing the protecting group P¹,
   to produce X of the preceding item; and/or
(b) (i) reacting Z of the preceding item and L of the preceding item to produce Z-L, and
   reacting the Z-L with X to produce the compound of Formula (I), (ii) reacting X of the preceding item and L of the preceding item to produce X-L, and
   reacting the X-L with Z to produce the compound of Formula (I); and/or
   (iii) reacting Z of the preceding item and La, which is a part of L of the preceding item, to produce Z-La,
   reacting X of the preceding item and Lb, which is a remaining part of the L, to produce X-Lb, and
   reacting the Z-La with the X-Lb to produce the compound of Formula (I).

In the present disclosure, the above-described one or more characteristics intend that, in addition to the explicitly shown combination, further combinations may be provided. Further embodiment and advantage of the present disclosure can be recognized by those skilled in the art by reading and understanding the following detailed explanation as needed.

### [Effect of the Invention]

The present disclosure provides a compound in which a ligand of a specific E3 ligase and an intracellular target protein ligand are linked via a linker. The compound of the disclosure exhibits an effect of inducing protein degradation by using a ligand of an inhibitor of apoptosis protein (IAP). Therefore, the compound of the disclosure is useful as a therapeutic agent and/or a prophylactic agent.

### [Description of Embodiments]

The present disclosure is described hereinafter in more detail.

Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. The terms used herein should also be understood as being used in the meaning that is commonly used in the art, unless specifically noted otherwise. Thus, unless defined otherwise, all terminologies and scientific technical terms that are used herein have the same meaning as the general understanding of those skilled in the art to which the present disclosure pertains. In case of a contradiction, the present specification (including the definitions) takes precedence.

### (Definition)

The terms and the general technology used in the present disclosure are first described.

As used herein, the term "group" refers to a monovalent group, unless especially noted otherwise. Examples of a group that is not a monovalent group include alkylene group (divalent) and the like. The term "group" may also be abbreviated in the following description of substituents or the like.

As used herein, the number of substituents when a group is defined as "optionally substituted" or "substituted" is not particularly limited as long as it is substitutable and is one or more. The description for each group is also applicable when the substituent is a part of or a substituent on another substituent, unless specifically noted otherwise.

As used herein, "maximum substitutable number" is the maximum number of substituents that a group can have. The number can vary for each group. For example, the number is 3 for a methyl group, 5 for an ethyl group, 7 for a benzyl group, and 11 for a naphthalenyl ethyl group.

For a group that is modified by "optionally substituted" or "substituted" herein, any portion of the group can be substituted. For example, "optionally substituted arylalkyl" and "substituted arylalkyl" can have the aryl moiety substituted, the alkyl moiety substituted, or both the aryl moiety and the alkyl moiety substituted.

As used herein, the substituent used when "optionally substituted" can be one or more of the same or different substituents selected from any one of the following substituent groups I to III. While the types of atoms within a substituent associated with attachment are not particularly limited by the type of substituent, if the atom to which a substituent is attached is an oxygen atom, a nitrogen atom, or a sulfur atom, the atom is limited to and selected from those with an attachment point in the following substituents that is a carbon atom.

Substituent group I consists of halogen, hydroxy, oxo, carboxy, amino, imino, hydroxyamino, hydroxyimino, formyl, formyloxy, carbamoyl, sulfamoyl, sulfanyl, sulfino, sulfo, thioformyl, thiocarboxy, dithiocarboxy, thiocarbamoyl, cyano, nitro, nitroso, azide, hydrazino, ureide, amidino, guanidino, unsubstituted or substituted alkyl, unsubstituted or substituted alkenyl, unsubstituted or substituted alkynyl, unsubstituted or substituted aryl, unsubstituted or substituted cycloalkyl, unsubstituted or substituted heteroaryl, unsubstituted or substituted heterocycloalkyl, unsubstituted or substituted alkyloxy, unsubstituted or substituted alkenyloxy, unsubstituted or substituted alkynyloxy, unsubstituted or substituted aryl-L_{X}-oxy, unsubstituted or substituted cycloalkyl-L_{X}-oxy, unsubstituted or substituted heteroaryl-L_{X}-oxy, unsubstituted or substituted heterocycloalkyl-L_{X}-oxy, unsubstituted or substituted alkyloxyalkyl, unsubstituted or substituted alkenyloxyalkyl, unsubstituted or substituted alkynyloxyalkyl, unsubstituted or substituted aryloxyalkyl, unsubstituted or substituted cycloalkyloxyalkyl, unsubstituted or substituted heteroaryloxyalkyl, unsubstituted or substituted heterocycloalkyloxyalkyl, unsubstituted or substituted alkyloxyalkyloxy, unsubstituted or substituted alkenyloxyalkyloxy, unsubstituted or substituted alkynyloxyalkyloxy, unsubstituted or substituted aryloxyalkyloxy, unsubstituted or substituted cycloalkyloxyalkyloxy, unsubstituted or substituted heteroaryloxyalkyloxy, unsubstituted or substituted heterocycloalkyloxyalkyloxy, unsubstituted or substituted alkylcarbonyl, unsubstituted or substituted alkenylcarbonyl, unsubstituted or substituted alkynylcarbonyl, unsubstituted or substituted aryl-L_{X}-carbonyl, unsubstituted or substituted cycloalkyl-L_{X}-carbonyl, unsubstituted or substituted heteroaryl-L_{X}-carbonyl, unsubstituted or substituted heterocycloalkyl-L_{X}-carbonyl, unsubstituted or substituted alkylcarbonyloxy, unsubstituted or substituted alkenylcarbonyloxy, unsubstituted or substituted alkynylcarbonyloxy, unsubstituted or substituted aryl-L_{X}-carbonyloxy, unsubstituted or substituted cycloalkyl-L_{X}-carbonyloxy, unsubstituted or substituted heteroaryl-L_{X}-carbonyloxy, unsubstituted or substituted heterocycloalkyl-L_{X}-carbonyloxy, unsubstituted or substituted alkylcarbonylamino, unsubstituted or substituted alkenylcarbonylamino, unsubstituted or substituted alkynylcarbonylamino, unsubstituted or substituted aryl-L_{X}-carbonylamino, unsubstituted or substituted cycloalkyl-L_{X}-carbonylamino, unsubstituted or substituted heteroaryl-L_{X}-carbonylamino, unsubstituted or substituted heterocycloalkyl-L_{X}-carbonylamino, unsubstituted or substituted alkylcarbonylthio, unsubstituted or substituted alkenylcarbonylthio, unsubstituted or substituted alkynylcarbonylthio, unsubstituted or substituted aryl-L_{X}-carbonylthio, unsubstituted or substituted cycloalkyl-L_{X}-carbonylthio, unsubstituted or substituted heteroaryl-L_{X}-carbonylthio, unsubstituted or substituted heterocycloalkyl-L_{X}-carbonylthio, unsubstituted or substituted alkylcarbonylimino, unsubstituted or substituted alkenylcarbonylimino, unsubstituted or substituted alkynylcarbonylimino, unsubstituted or substituted aryl-L_{X}-carbonylimino, unsubstituted or substituted cycloalkyl-L_{X}-carbonylimino, unsubstituted or substituted heteroaryl-L_{X}-carbonylimino, unsubstituted or substituted heterocycloalkyl-L_{X}-carbonylimino, unsubstituted or substituted alkylthio, unsubstituted or substituted alkenylthio, unsubstituted or substituted alkynylthio, unsubstituted or substituted aryl-L_{X}-thio, unsubstituted or substituted cycloalkyl-L_{X}-thio, unsubstituted or substituted heteroaryl-L_{X}-thio, unsubstituted or substituted heterocycloalkyl-L_{X}-thio, unsubstituted or substituted alkylamino, unsubstituted or substituted alkenylamino, unsubstituted or substituted alkynylamino, unsubstituted or substituted alkynylamino, unsubstituted or substituted aryl-L_{X}-amino, unsubstituted or substituted cycloalkyl-L_{X}-amino, unsubstituted or substituted heteroaryl-L_{X}-amino, unsubstituted or substituted heterocycloalkyl-L_{X}-amino, unsubstituted or substituted alkylsulfonyl, unsubstituted or substituted alkenylsulfonyl, unsubstituted or substituted alkynylsulfonyl, unsubstituted or substituted aryl-L_{X}-sulfonyl, unsubstituted or substituted cycloalkyl-L_{X}-sulfonyl, unsubstituted or substituted heteroaryl-L_{X}-sulfonyl, unsubstituted or substituted heterocycloalkyl-L_{X}-sulfonyl, unsubstituted or substituted alkylsulfonylamino, unsubstituted or substituted alkenylsulfonylamino, unsubstituted or substituted alkynylsulfonylamino, unsubstituted or substituted aryl-L_{X}-sulfonylamino, unsubstituted or substituted cycloalkyl-L_{X}-sulfonylamino, unsubstituted or substituted heteroaryl-L_{X}-sulfonylamino, unsubstituted or substituted heterocycloalkyl-L_{X}-sulfonylamino, unsubstituted or substituted alkylimino, unsubstituted or substituted alkenylimino, unsubstituted or substituted alkynylimino, unsubstituted or substituted aryl-L_{X}-imino, unsubstituted or substituted cycloalkyl-L_{X}-imino, unsubstituted or substituted heteroaryl-L_{X}-imino, unsubstituted or substituted heterocycloalkyl-L_{X}-imino, unsubstituted or substituted alkyloxyimino, unsubstituted or substituted alkenyloxyimino, unsubstituted or substituted alkynyloxyimino, unsubstituted or substituted aryl-L_{X}-oxyimino, unsubstituted or substituted cycloalkyl-L_{X}-oxyimino, unsubstituted or substituted heteroaryl-L_{X}-oxyimino, unsubstituted or substituted heterocycloalkyl-L_{X}-oxyimino, unsubstituted or substituted alkyloxycarbonyl, unsubstituted or substituted alkenyloxycarbonyl, unsubstituted or substituted alkynyloxycarbonyl, unsubstituted or substituted aryl-L_{X}-oxycarbonyl, unsubstituted or substituted cycloalkyl-L_{X}-oxycarbonyl, unsubstituted or substituted heteroaryl-L_{X}-oxycarbonyl, unsubstituted or substituted heterocycloalkyl-L_{X}-oxycarbonyl, unsubstituted or substituted alkyloxycarbonylamino, unsubstituted or substituted alkenyloxycarbonylamino, unsubstituted or substituted alkynyloxycarbonylamino, unsubstituted or substituted aryl-L_{X}-oxycarbonylamino, unsubstituted or substituted cycloalkyl-L_{X}-oxycarbonylamino, unsubstituted or substituted heteroaryl-L_{X}-oxycarbonylamino, unsubstituted or substituted heterocycloalkyl-L_{X}-oxycarbonylamino, unsubstituted or substituted alkylsulfanyl, unsubstituted or substituted alkenylsulfanyl, unsubstituted or substituted alkynylsulfanyl, unsubstituted or substituted aryl-L_{X}-sulfanyl, unsubstituted or substituted cycloalkyl-L_{X}-sulfanyl, unsubstituted or substituted heteroaryl-L_{X}-sulfanyl, unsubstituted or substituted heterocycloalkyl-L_{X}-sulfanyl, unsubstituted or substituted alkylsulfinyl, unsubstituted or substituted alkenylsulfinyl, unsubstituted or substituted alkynylsulfinyl, unsubstituted or substituted aryl-L_{X}-sulfinyl, unsubstituted or substituted cycloalkyl-L_{X}-sulfinyl, unsubstituted or substituted heteroaryl-L_{X}-sulfinyl, unsubstituted or substituted heterocycloalkyl-L_{X}-sulfinyl, unsubstituted or substituted alkylcarbamoyl, unsubstituted or substituted alkenylcarbamoyl, unsubstituted or substituted alkynylcarbamoyl, unsubstituted or substituted aryl-L_{X}-carbamoyl, unsubstituted or substituted cycloalkyl-L_{X}-carbamoyl, unsubstituted or substituted heteroaryl-L_{X}-carbamoyl, unsubstituted or substituted heterocycloalkyl-L_{X}-carbamoyl, unsubstituted or substituted alkylsulfamoyl, unsubstituted or substituted alkenylsulfamoyl, unsubstituted or substituted alkynylsulfamoyl, unsubstituted or substituted aryl-L_{X}-sulfamoyl, unsubstituted or substituted cycloalkyl-L_{X}-sulfamoyl, unsubstituted or substituted heteroaryl-L_{X}-sulfamoyl, and unsubstituted or substituted heterocycloalkyl-L_{X}-sulfamoyl, wherein L_{X} is a single bond or unsubstituted or substituted alkylene,
wherein the substituted alkyl, substituted alkenyl, substituted alkynyl, substituted aryl, substituted cycloalkyl, substituted heteroaryl, substituted heterocycloalkyl, and substituted alkylene moieties (fully or partially) in the substituent group each independently have one to the maximum substitutable number of the same or different substituents selected from the group consisting of halogen, hydroxy, oxo, carboxy, amino, imino, hydroxyamino, hydroxyimino, formyl, formyloxy, carbamoyl, sulfamoyl, sulfanyl, sulfino, sulfo, thioformyl, thiocarboxy, dithiocarboxy, thiocarbamoyl, cyano, nitro, nitroso, azide, hydrazino, ureide, amidino, guanidino, alkyl, alkenyl, alkynyl, aryl, cycloalkyl, heteroaryl, heterocycloalkyl, alkyloxy, alkenyloxy, alkynyloxy, aryl-L_{X}-oxy, cycloalkyl-L_{X}-oxy, heteroaryl-L_{X}-oxy, heterocycloalkyl-L_{X}-oxy, alkyloxyalkyl, alkenyloxyalkyl, alkynyloxyalkyl, aryloxyalkyl, cycloalkyloxyalkyl, heteroaryloxyalkyl, heterocycloalkyloxyalkyl, alkyloxyalkyloxy, alkenyloxyalkyloxy, alkynyloxyalkyloxy, aryloxyalkyloxy, cycloalkyloxyalkyloxy, heteroaryloxyalkyloxy, heterocycloalkyloxyalkyloxy, alkylcarbonyl, alkenylcarbonyl, alkynylcarbonyl, aryl-L_{X}-carbonyl, cycloalkyl-L_{X}-carbonyl, heteroaryl-L_{X}-carbonyl, heterocycloalkyl-L_{X}-carbonyl, alkylcarbonyloxy, alkenylcarbonyloxy, alkynylcarbonyloxy, aryl-L_{X}-carbonyloxy, cycloalkyl-L_{X}-carbonyloxy, heteroaryl-L_{X}-carbonyloxy, heterocycloalkyl-L_{X}-carbonyloxy, alkylcarbonylamino, alkenylcarbonylamino, alkynylcarbonylamino, aryl-L_{X}-carbonylamino, cycloalkyl-L_{X}-carbonylamino, heteroaryl-L_{X}-carbonylamino, heterocycloalkyl-L_{X}-carbonylamino, alkylcarbonylthio, alkenylcarbonylthio, alkynylcarbonylthio, aryl-L_{X}-carbonylthio, cycloalkyl-L_{X}-carbonylthio, heteroaryl-L_{X}-carbonylthio, heterocycloalkyl-L_{X}-carbonylthio, alkylcarbonylimino, alkenylcarbonylimino, alkynylcarbonylimino, aryl-L_{X}-carbonylimino, cycloalkyl-L_{X}-carbonylimino, heteroaryl-L_{X}-carbonylimino, heterocycloalkyl-_{X}-carbonylimino, alkylthio, alkenylthio, alkynylthio, aryl-L_{X}-thio, cycloalkyl-L_{X}-thio, heteroaryl-L_{X}-thio, heterocycloalkyl-L_{X}-thio, alkylamino, alkenylamino, alkynylamino, alkynylamino, aryl-L_{X}-amino, cycloalkyl-L_{X}-amino, heteroaryl-L_{X}-amino, heterocycloalkyl-L_{X}-amino, alkylsulfonyl, alkenylsulfonyl, alkynylsulfonyl, aryl-L_{X}-sulfonyl, cycloalkyl-L_{X}-sulfonyl, heteroaryl-L_{X}-sulfonyl, heterocycloalkyl-L_{X}-sulfonyl, alkylsulfonylamino, alkenylsulfonylamino, alkynylsulfonylamino, aryl-L_{X}-sulfonylamino, cycloalkyl-L_{X}-sulfonylamino, heteroaryl-L_{X}-sulfonylamino, heterocycloalkyl-L_{X}-sulfonylamino, alkylimino, alkenylimino, alkynylimino, aryl-L_{X}-imino, cycloalkyl-L_{X}-imino, heteroaryl-L_{X}-imino, heterocycloalkyl-L_{X}-imino, alkyloxyimino, alkenyloxyimino, alkynyloxyimino, aryl-L_{X}-oxyimino, cycloalkyl-L_{X}-oxyimino, heteroaryl-L_{X}-oxyimino, heterocycloalkyl-L_{X}-oxyimino, alkyloxycarbonyl, alkenyloxycarbonyl, alkynyloxycarbonyl, aryl-L_{X}-oxycarbonyl, cycloalkyl-L_{X}-oxycarbonyl, heteroaryl-L_{X}-oxycarbonyl, heterocycloalkyl-L_{X}-oxycarbonyl, alkyloxycarbonylamino, alkenyloxycarbonylamino, alkynyloxycarbonylamino, aryl-L_{X}-oxycarbonylamino, cycloalkyl-L_{X}-oxycarbonylamino, heteroaryl-L_{X}-oxycarbonylamino, heterocycloalkyl-L_{X}-oxycarbonylamino, alkylsulfanyl, alkenylsulfanyl, alkynylsulfanyl, aryl-L_{X}-sulfanyl, cycloalkyl-L_{X}-sulfanyl, heteroaryl-L_{X}-sulfanyl, heterocycloalkyl-L_{X}-sulfanyl, alkylsulfinyl, alkenylsulfinyl, alkynylsulfinyl, aryl-L_{X}-sulfinyl, cycloalkyl-L_{X}-sulfinyl, heteroaryl-L_{X}-sulfinyl, heterocycloalkyl-L_{X}-sulfinyl, alkylcarbamoyl, alkenylcarbamoyl, alkynylcarbamoyl, aryl-L_{X}-carbamoyl, cycloalkyl-L_{X}-carbamoyl, heteroaryl-L_{X}-carbamoyl, heterocycloalkyl-L_{X}-carbamoyl, alkylsulfamoyl, alkenylsulfamoyl, alkynylsulfamoyl, aryl-L_{X}-sulfamoyl, cycloalkyl-L_{X}-sulfamoyl, heteroaryl-L_{X}-sulfamoyl, and heterocycloalkyl-L_{X}-sulfamoyl.

Substituent group II consists of halogen, hydroxy, oxo, carboxy, amino, imino, hydroxyamino, hydroxyimino, formyl, formyloxy, carbamoyl, sulfamoyl, sulfanyl, sulfino, sulfo, thioformyl, thiocarboxy, dithiocarboxy, thiocarbamoyl, cyano, nitro, nitroso, azide, hydrazino, ureide, amidino, guanidino, unsubstituted or substituted C₁₋₁₂ alkyl, unsubstituted or substituted C₂₋₁₂ alkenyl, unsubstituted or substituted C₂₋₁₂ alkynyl, unsubstituted or substituted C₆₋₁₀ aryl, unsubstituted or substituted C₃₋₁₀ cycloalkyl, unsubstituted or substituted 5-to 10-membered heteroaryl, unsubstituted or substituted 5- to 10-membered heterocycloalkyl, unsubstituted or substituted C₁₋₁₂ alkyloxy, unsubstituted or substituted C₂₋₁₂ alkenyloxy, unsubstituted or substituted C₂₋₁₂ alkynyloxy, unsubstituted or substituted C₆₋₁₀ aryl-L_{X}-oxy, unsubstituted or substituted C₃₋₁₀ cycloalkyl-L_{X}-oxy, unsubstituted or substituted 5- to 10-membered heteroaryl-L_{X}-oxy, unsubstituted or substituted 5- to 10-membered heterocycloalkyl-L_{X}-oxy, unsubstituted or substituted C₁₋₁₂ alkyloxy C₁₋₁₂ alkyl, unsubstituted or substituted C₂₋₁₂ alkenyloxy C₁₋₁₂ alkyl, unsubstituted or substituted C₂₋₁₂ alkynyloxy C₁₋₁₂ alkyl, unsubstituted or substituted C₆₋₁₀ aryloxy C₁₋₁₂ alkyl, unsubstituted or substituted C₃₋₁₀ cycloalkyloxy C₁₋₁₂ alkyl, unsubstituted or substituted 5- to 10-membered heteroaryloxy C₁₋₁₂ alkyl, unsubstituted or substituted 5- to 10-membered heterocycloalkyloxy C₁₋₁₂ alkyl, unsubstituted or substituted C₁₋₁₂ alkyloxy C₁₋₁₂ alkyloxy, unsubstituted or substituted C₂₋₁₂ alkenyloxy C₁₋₁₂ alkyloxy, unsubstituted or substituted C₂₋₁₂ alkynyloxy C₁₋₁₂ alkyloxy, unsubstituted or substituted C₆₋₁₀ aryloxy C₁₋₁₂ alkyloxy, unsubstituted or substituted C₃₋₁₀ cycloalkyloxy C₁₋₁₂ alkyloxy, unsubstituted or substituted 5- to 10-membered heteroaryloxy C₁₋₁₂ alkyloxy, unsubstituted or substituted 5- to 10-membered heterocycloalkyloxy C₁₋₁₂ alkyloxy, unsubstituted or substituted C₁₋₁₂ alkylcarbonyl, unsubstituted or substituted C₂₋₁₂ alkenylcarbonyl, unsubstituted or substituted C₂₋₁₂ alkynylcarbonyl, unsubstituted or substituted C₆₋₁₀ aryl-L_{X}-carbonyl, unsubstituted or substituted C₃₋₁₀ cycloalkyl-L_{X}-carbonyl, unsubstituted or substituted 5- to 10-membered heteroaryl-L_{X}-carbonyl, unsubstituted or substituted 5- to 10-membered heterocycloalkyl-L_{X}-carbonyl, unsubstituted or substituted C₁₋₁₂ alkylcarbonyloxy, unsubstituted or substituted C₂₋₁₂ alkenylcarbonyloxy, unsubstituted or substituted C₂₋₁₂ alkynylcarbonyloxy, unsubstituted or substituted C₆₋₁₀ aryl-L_{X}-carbonyloxy, unsubstituted or substituted C₃₋₁₀ cycloalkyl-L_{X}-carbonyloxy, unsubstituted or substituted 5- to 10-membered heteroaryl-L_{X}-carbonyloxy, unsubstituted or substituted 5- to 10-membered heterocycloalkyl-L_{X}-carbonyloxy, unsubstituted or substituted C₁₋₁₂ alkylcarbonylamino, unsubstituted or substituted C₂₋₁₂ alkenylcarbonylamino, unsubstituted or substituted C₂₋₁₂ alkynylcarbonylamino, unsubstituted or substituted C₆₋₁₀ aryl-L_{X}-carbonylamino, unsubstituted or substituted C₃₋₁₀ cycloalkyl-L_{X}-carbonylamino, unsubstituted or substituted 5- to 10-membered heteroaryl-L_{X}-carbonylamino, unsubstituted or substituted 5- to 10-membered heterocycloalkyl-L_{X}-carbonylamino, unsubstituted or substituted C₁₋₁₂ alkylcarbonylthio, unsubstituted or substituted C₂₋₁₂ alkenylcarbonylthio, unsubstituted or substituted C₂₋₁₂ alkynylcarbonylthio, unsubstituted or substituted C₆₋₁₀ aryl-L_{X}-carbonylthio, unsubstituted or substituted C₃₋₁₀ cycloalkyl-L_{X}-carbonylthio, unsubstituted or substituted 5- to 10-membered heteroaryl-L_{X}-carbonylthio, unsubstituted or substituted 5- to 10-membered heterocycloalkyl-L_{X}-carbonylthio, unsubstituted or substituted C₁₋₁₂ alkylcarbonylimino, unsubstituted or substituted C₂₋₁₂ alkenylcarbonylimino, unsubstituted or substituted C₂₋₁₂ alkynylcarbonylimino, unsubstituted or substituted C₆₋₁₀ aryl-L_{X}-carbonylimino, unsubstituted or substituted C₃₋₁₀ cycloalkyl-L_{X}-carbonylimino, unsubstituted or substituted 5- to 10-membered heteroaryl-L_{X}-carbonylimino, unsubstituted or substituted 5- to 10-membered heterocycloalkyl-L_{X}-carbonylimino, unsubstituted or substituted C₁₋₁₂ alkylthio, unsubstituted or substituted C₂₋₁₂ alkenylthio, unsubstituted or substituted C₂₋₁₂ alkynylthio, unsubstituted or substituted C₆₋₁₀ aryl-L_{X}-thio, unsubstituted or substituted C₃₋₁₀ cycloalkyl-L_{X}-thio, unsubstituted or substituted 5- to 10-membered heteroaryl-L_{X}-thio, unsubstituted or substituted 5- to 10-membered heterocycloalkyl-L_{X}-thio, unsubstituted or substituted C₁₋₁₂ alkylamino, unsubstituted or substituted C₂₋₁₂ alkenylamino, unsubstituted or substituted C₂₋₁₂ alkynylamino, unsubstituted or substituted C₂₋₁₂ alkynylamino, unsubstituted or substituted C₆₋₁₀ aryl-L_{X}-amino, unsubstituted or substituted C₃₋₁₀ cycloalkyl-L_{X}-amino, unsubstituted or substituted 5- to 10-membered heteroaryl-L_{X}-amino, unsubstituted or substituted 5-to 10-membered heterocycloalkyl-L_{X}-amino, unsubstituted or substituted C₁₋₁₂ alkylsulfonyl, unsubstituted or substituted C₂₋₁₂ alkenylsulfonyl, unsubstituted or substituted C₂₋₁₂ alkynylsulfonyl, unsubstituted or substituted C₆₋₁₀ aryl-L_{X}-sulfonyl, unsubstituted or substituted C₃₋₁₀ cycloalkyl-L_{X}-sulfonyl, unsubstituted or substituted 5- to 10-membered heteroaryl-L_{X}-sulfonyl, unsubstituted or substituted 5- to 10-membered heterocycloalkyl-L_{X}-sulfonyl, unsubstituted or substituted C₁₋₁₂ alkylsulfonylamino, unsubstituted or substituted C₂₋₁₂ alkenylsulfonylamino, unsubstituted or substituted C₂₋₁₂ alkynylsulfonylamino, unsubstituted or substituted C₆₋₁₀ aryl-L_{X}-sulfonylamino, unsubstituted or substituted C₃₋₁₀ cycloalkyl-L_{X}-sulfonylamino, unsubstituted or substituted 5- to 10-membered heteroaryl-L_{X}-sulfonylamino, unsubstituted or substituted 5- to 10-membered heterocycloalkyl-L_{X}-sulfonylamino, unsubstituted or substituted C₁₋₁₂ alkylimino, unsubstituted or substituted C₂₋₁₂ alkenylimino, unsubstituted or substituted C₂₋₁₂ alkynylimino, unsubstituted or substituted C₆₋₁₀ aryl-L_{X}-imino, unsubstituted or substituted C₃₋₁₀ cycloalkyl-L_{X}-imino, unsubstituted or substituted 5- to 10-membered heteroaryl-L_{X}-imino, unsubstituted or substituted 5-to 10-membered heterocycloalkyl-L_{X}-imino, unsubstituted or substituted C₁₋₁₂ alkyloxyimino, unsubstituted or substituted C₂₋₁₂ alkenyloxyimino, unsubstituted or substituted C₂₋₁₂ alkynyloxyimino, unsubstituted or substituted C₆₋₁₀ aryl-L_{X}-oxyimino, unsubstituted or substituted C₃₋₁₀ cycloalkyl-L_{X}-oxyimino, unsubstituted or substituted 5- to 10-membered heteroaryl-L_{X}-oxyimino, unsubstituted or substituted 5- to 10-membered heterocycloalkyl-L_{X}-oxyimino, unsubstituted or substituted C₁₋₁₂ alkyloxycarbonyl, unsubstituted or substituted C₂₋₁₂ alkenyloxycarbonyl, unsubstituted or substituted C₂₋₁₂ alkynyloxycarbonyl, unsubstituted or substituted C₆₋₁₀ aryl-L_{X}-oxycarbonyl, unsubstituted or substituted C₃₋₁₀ cycloalkyl-L_{X}-oxycarbonyl, unsubstituted or substituted 5- to 10-membered heteroaryl-L_{X}-oxycarbonyl, unsubstituted or substituted 5- to 10-membered heterocycloalkyl-L_{X}-oxycarbonyl, unsubstituted or substituted C₁₋₁₂ alkyloxycarbonylamino, unsubstituted or substituted C₂₋₁₂ alkenyloxycarbonylamino, unsubstituted or substituted C₂₋₁₂ alkynyloxycarbonylamino, unsubstituted or substituted C₆₋₁₀ aryl-L_{X}-oxycarbonylamino, unsubstituted or substituted C₃₋₁₀ cycloalkyl-L_{X}-oxycarbonylamino, unsubstituted or substituted 5- to 10-membered heteroaryl-L_{X}-oxycarbonylamino, unsubstituted or substituted 5- to 10-membered heterocycloalkyl-L_{X}-oxycarbonylamino, unsubstituted or substituted C₁₋₁₂ alkylsulfanyl, unsubstituted or substituted C₂-₁₂ alkenylsulfanyl, unsubstituted or substituted C₂₋₁₂ alkynylsulfanyl, unsubstituted or substituted C₆₋₁₀ aryl-L_{X}-sulfanyl, unsubstituted or substituted C₃₋₁₀ cycloalkyl-L_{X}-sulfanyl, unsubstituted or substituted 5- to 10-membered heteroaryl-L_{X}-sulfanyl, unsubstituted or substituted 5- to 10-membered heterocycloalkyl-L_{X}-sulfanyl, unsubstituted or substituted C₁₋₁₂ alkylsulfinyl, unsubstituted or substituted C₂-₁₂ alkenylsulfinyl, unsubstituted or substituted C₂₋₁₂ alkynylsulfinyl, unsubstituted or substituted C₆₋₁₀ aryl-L_{X}-sulfinyl, unsubstituted or substituted C₃₋₁₀ cycloalkyl-L_{X}-sulfinyl, unsubstituted or substituted 5- to 10-membered heteroaryl-L_{X}-sulfinyl, unsubstituted or substituted 5- to 10-membered heterocycloalkyl-L_{X}-sulfinyl, unsubstituted or substituted C₁₋₁₂ alkylcarbamoyl, unsubstituted or substituted C₂₋₁₂ alkenylcarbamoyl, unsubstituted or substituted C₂₋₁₂ alkynylcarbamoyl, unsubstituted or substituted C₆₋₁₀ aryl-L_{X}-carbamoyl, unsubstituted or substituted C₃₋₁₀ cycloalkyl-L_{X}-carbamoyl, unsubstituted or substituted 5- to 10-membered heteroaryl-L_{X}-carbamoyl, unsubstituted or substituted 5- to 10-membered heterocycloalkyl-L_{X}-carbamoyl, unsubstituted or substituted C₁₋₁₂ alkylsulfamoyl, unsubstituted or substituted C₂₋₁₂ alkenylsulfamoyl, unsubstituted or substituted C₂₋₁₂ alkynylsulfamoyl, unsubstituted or substituted C₆₋₁₀ aryl-L_{X}-sulfamoyl, unsubstituted or substituted C₃₋₁₀ cycloalkyl-L_{X}-sulfamoyl, unsubstituted or substituted 5- to 10-membered heteroaryl-L_{X}-sulfamoyl, and unsubstituted or substituted 5- to 10-membered heterocycloalkyl-L_{X}-sulfamoyl, wherein L_{X} is a single bond or unsubstituted or substituted C₁₋₁₂ alkylene, wherein the substituted C₁₋₁₂ alkyl, substituted C₂₋₁₂ alkenyl, substituted C₂₋₁₂ alkynyl, substituted C₆₋₁₀ aryl, substituted C₃-₁₀ cycloalkyl, substituted 5- to 10-membered heteroaryl, substituted 5- to 10-membered heterocycloalkyl, and substituted alkylene moieties (fully or partially) in the substituent group each independently have one to the maximum substitutable number of the same or different substituents selected from the group consisting of halogen, hydroxy, oxo, carboxy, amino, imino, hydroxyamino, hydroxyimino, formyl, formyloxy, carbamoyl, sulfamoyl, sulfanyl, sulfino, sulfo, thioformyl, thiocarboxy, dithiocarboxy, thiocarbamoyl, cyano, nitro, nitroso, azide, hydrazino, ureide, amidino, guanidino, C₁₋₁₂ alkyl, C₁₋₁₂ haloalkyl, C₂₋₁₂ alkenyl, C₂₋₁₂ alkynyl, C₆₋₁₀ aryl, C₃₋₁₀ cycloalkyl, 5- to 10-membered heteroaryl, 5- to 10-membered heterocycloalkyl, C₁₋₁₂ alkyloxy, C₁₋₁₂ haloalkyloxy, C₂₋₁₂ alkenyloxy, C₂₋₁₂ alkynyloxy, C₆₋₁₀ aryl-L_{X}-oxy, C₃₋₁₀ cycloalkyl-L_{X}-oxy, 5- to 10-membered heteroaryl-L_{X}-oxy, 5- to 10-membered heterocycloalkyl-L_{X}-oxy, C₁₋₁₂ alkyloxyalkyl, C₂₋₁₂ alkenyloxyalkyl, C₂₋₁₂ alkynyloxyalkyl, C₆₋₁₀ aryloxyalkyl, C₃₋₁₀ cycloalkyloxyalkyl, 5- to 10-membered heteroaryloxyalkyl, 5- to 10-membered heterocycloalkyloxyalkyl, C₁₋₁₂ alkyloxyalkyloxy, C₂₋₁₂ alkenyloxyalkyloxy, C₂₋₁₂ alkynyloxyalkyloxy, C₆₋₁₀ aryloxyalkyloxy, C₃₋₁₀ cycloalkyloxyalkyloxy, 5- to 10-membered heteroaryloxyalkyloxy, 5- to 10-membered heterocycloalkyloxyalkyloxy, C₁₋₁₂ alkylcarbonyl, C₂₋₁₂ alkenylcarbonyl, C₂₋₁₂ alkynylcarbonyl, C₆₋₁₀ aryl-L_{X}-carbonyl, C₃₋₁₀ cycloalkyl-L_{X}-carbonyl, 5- to 10-membered heteroaryl-L_{X}-carbonyl, 5- to 10-membered heterocycloalkyl-L_{X}-carbonyl, C₁₋₁₂ alkylcarbonyloxy, C₂₋₁₂ alkenylcarbonyloxy, C₂₋₁₂ alkynylcarbonyloxy, C₆₋₁₀ aryl-L_{X}-carbonyloxy, C₃₋₁₀ cycloalkyl-L_{X}-carbonyloxy, 5- to 10-membered heteroaryl-L_{X}-carbonyloxy, 5- to 10-membered heterocycloalkyl-L_{X}-carbonyloxy, C₁₋₁₂ alkylcarbonylamino, C₂₋₁₂ alkenylcarbonylamino, C₂₋₁₂ alkynylcarbonylamino, C₆₋₁₀ aryl-L_{X}-carbonylamino, C₃₋₁₀ cycloalkyl-L_{X}-carbonylamino, 5- to 10-membered heteroaryl-L_{X}-carbonylamino, 5- to 10-membered heterocycloalkyl-L_{X}-carbonylamino, C₁₋₁₂ alkylcarbonylthio, C₂₋₁₂ alkenylcarbonylthio, C₂₋₁₂ alkynylcarbonylthio, C₆₋₁₀ aryl-L_{X}-carbonylthio, C₃₋₁₀ cycloalkyl-L_{X}-carbonylthio, 5- to 10-membered heteroaryl-L_{X}-carbonylthio, 5- to 10-membered heterocycloalkyl-L_{X}-carbonylthio, C₁₋₁₂ alkylcarbonylimino, C₂₋₁₂ alkenylcarbonylimino, C₂₋₁₂ alkynylcarbonylimino, C₆₋₁₀ aryl-L_{X}-carbonylimino, C₃₋₁₀ cycloalkyl-L_{X}-carbonylimino, 5- to 10-membered heteroaryl-L_{X}-carbonylimino, 5- to 10-membered heterocycloalkyl-L_{X}-carbonylimino, C₁₋₁₂ alkylthio, C₂₋₁₂ **alkenylthio,** C₂₋₁₂ alkynylthio, C₆₋₁₀ aryl-L_{X}-thio, C₃₋₁₀ cycloalkyl-L_{X}-thio, 5- to 10-membered heteroaryl-L_{X}-thio, 5- to 10-membered heterocycloalkyl-L_{X}-thio, C₁₋₁₂ alkylamino, C₂₋₁₂ alkenylamino, C₂₋₁₂ alkynylamino, C₂₋₁₂ alkynylamino, C₆₋₁₀ aryl-L_{X}-amino, C₃₋₁₀ cycloalkyl-L_{X}-amino, 5- to 10-membered heteroaryl-L_{X}-amino, 5- to 10-membered heterocycloalkyl-L_{X}-amino, C₁₋₁₂ alkylsulfonyl, C₂₋₁₂ alkenylsulfonyl, C₂₋₁₂ alkynylsulfonyl, C₆₋₁₀ aryl-L_{X}-sulfonyl, C₃₋₁₀ cycloalkyl-L_{X}-sulfonyl, 5- to 10-membered heteroaryl-L_{X}-sulfonyl, 5- to 10-membered heterocycloalkyl-L_{X}-sulfonyl, C₁₋₁₂ alkylsulfonylamino, C₂₋₁₂ alkenylsulfonylamino, C₂₋₁₂ alkynylsulfonylamino, C₆₋₁₀ aryl-L_{X}-sulfonylamino, C₃₋₁₀ cycloalkyl-L_{X}-sulfonylamino, 5- to 10-membered heteroaryl-L_{X}-sulfonylamino, 5- to 10-membered heterocycloalkyl-L_{X}-sulfonylamino, C₁₋₁₂ alkylimino, C₂₋₁₂ alkenylimino, C₂₋₁₂ alkynylimino, C₆₋₁₀ aryl-L_{X}-imino, C₃₋₁₀ cycloalkyl-L_{X}-imino, 5- to 10-membered heteroaryl-L_{X}-imino, 5- to 10-membered heterocycloalkyl-L_{X}-imino, C₁₋₁₂ alkyloxyimino, C₂₋₁₂ alkenyloxyimino, C₂₋₁₂ alkynyloxyimino, C₆₋₁₀ aryl-L_{X}-oxyimino, C₃₋₁₀ cycloalkyl-L_{X}-oxyimino, 5- to 10-membered heteroaryl-L_{X}-oxyimino, 5- to 10-membered heterocycloalkyl-L_{X}-oxyimino, C₁₋₁₂ alkyloxycarbonyl, C₂₋₁₂ alkenyloxycarbonyl, C₂₋₁₂ alkynyloxycarbonyl, C₆₋₁₀ aryl-L_{X}-oxycarbonyl, C₃₋₁₀ cycloalkyl-L_{X}-oxycarbonyl, 5- to 10-membered heteroaryl-L_{X}-oxycarbonyl, 5- to 10-membered heterocycloalkyl-L_{X}-oxycarbonyl, C₁₋₁₂ alkyloxycarbonylamino, C₂₋₁₂ alkenyloxycarbonylamino, C₂₋₁₂ alkynyloxycarbonylamino, C₆₋₁₀ aryl-L_{X}-oxycarbonylamino, C₃₋₁₀ cycloalkyl-L_{X}-oxycarbonylamino, 5- to 10-membered heteroaryl-L_{X}-oxycarbonylamino, 5- to 10-membered heterocycloalkyl-L_{X}-oxycarbonylamino, C₁₋₁₂ alkylsulfanyl, C₂₋₁₂ alkenylsulfanyl, C₂₋₁₂ alkynylsulfanyl, C₆₋₁₀ aryl-L_{X}-sulfanyl, C₃₋₁₀ cycloalkyl-L_{X}-sulfanyl, 5- to 10-membered heteroaryl-L_{X}-sulfanyl, 5- to 10-membered heterocycloalkyl-L_{X}-sulfanyl, C₁₋₁₂ alkylsulfinyl, C₂₋₁₂ alkenylsulfinyl, C₂₋₁₂ alkynylsulfinyl, C₆₋₁₀ aryl-L_{X}-sulfinyl, C₃₋₁₀ cycloalkyl-L_{X}-sulfinyl, 5- to 10-membered heteroaryl-L_{X}-sulfinyl, 5- to 10-membered heterocycloalkyl-L_{X}-sulfinyl, C₁₋₁₂ alkylcarbamoyl, C₂₋₁₂ alkenylcarbamoyl, C₂₋₁₂ alkynylcarbamoyl, C₆₋₁₀ aryl-_{X}-carbamoyl, C₃₋₁₀ cycloalkyl-L_{X}-carbamoyl, 5- to 10-membered heteroaryl-L_{X}-carbamoyl, 5- to 10-membered heterocycloalkyl-_{X}-carbamoyl, C₁₋₁₂ alkylsulfamoyl, C₂₋₁₂ alkenylsulfamoyl, C₂₋₁₂ alkynylsulfamoyl, C₆₋₁₀ aryl-L_{X}-sulfamoyl, C₃₋₁₀ cycloalkyl-L_{X}-sulfamoyl, 5- to 10-membered heteroaryl-L_{X}-sulfamoyl, and 5- to 10-membered heterocycloalkyl-L_{X}-sulfamoyl.

Substituent group III consists of halogen, hydroxy, carboxy, amino, carbamoyl, nitro, guanidino, alkyl, alkenyl, alkynyl, aryl, cycloalkyl, heteroaryl, alkyloxy, alkylcarbonyl, cycloalkylcarbonyl, alkylcarbonylamino, cycloalkylcarbonylamino, alkylamino, cycloalkylalkylamino, alkyloxycarbonyl, and trialkylsilyloxy, and these groups of the substituent group are optionally substituted with one to the maximum substitutable number of the same or different substituents selected from the group consisting of halogen, hydroxy, carboxy, amino, carbamoyl, nitro, alkyl, haloalkyl, alkyloxy, haloalkyloxy, and alkyloxycarbonyl.

Substituent group III is preferably substituent group III', which consists of halogen, hydroxy, carboxy, amino, carbamoyl, nitro, guanidino, C₁₋₁₂ alkyl, C₁₋₁₂ alkenyl, C₁₋₁₂ alkynyl, C₆₋₁₀ aryl, C₃₋₁₀ cycloalkyl, 5- to 10-membered heteroaryl, C₁₋₁₂ alkyloxy, C₁₋₁₂ alkylcarbonyl, C₃₋₁₀ cycloalkylcarbonyl, C₁₋₁₂ alkylcarbonylamino, C₃₋₁₀ cycloalkylcarbonylamino, C₁₋₁₂ alkylamino, C₃₋₁₀ cycloalkyl C₁₋₆ alkylamino, C₁₋₁₂ alkyloxycarbonyl, and tri-C₁₋₆ alkylsilyloxy, wherein these groups of the substituent group are optionally substituted with one to the maximum substitutable number of the same or different substituents selected from the group consisting of halogen, hydroxy, carboxy, amino, carbamoyl, nitro, C₁₋₁₂ alkyl, C₁₋₁₂ haloalkyl, C₁₋₁₂ alkyloxy, C₁₋₁₂ haloalkyloxy, and C₁₋₁₂ alkyloxycarbonyl.

As used herein, examples of substituents used when "optionally substituted" include substituent group α and substituent group β. Substituent group α can be substituent group α1, substituent group α2, substituent group α3 or substituent group α4. Substituent group β can be substituent group β1, substituent group β2, substituent group β3 or substituent group β4. Substituents used when "optionally substituted" can be selected from substituent group α1, and substitution can be performed with 1 to 5 of the same or different substituents. While the types of atoms within a substituent associated with attachment are not particularly limited by the type of substituent, if the atom to which a substituent is attached is an oxygen atom, a nitrogen atom, or a sulfur atom, the substituent is limited to those with an attaching atom that is a carbon atom from the following substituents.

Substituent group α1 includes
1) a halogen atom
2) a hydroxyl group
3) a carboxyl group
4) a cyano group
5) C₁₋₆ alkyl
6) C₂₋₆ alkenyl
7) C₂₋₆ alkynyl
8) C₁₋₆ alkoxy
9) C₁₋₆ alkylthio
10) C₁₋₆ alkylcarbonyl
11) C₁₋₆ alkylsulfonyl
   (however, each substituent from 5) to 11) is optionally substituted with 1 to 5 of the same or different substituents selected from substituent group β1)
12) a C₃₋₁₀ carbocyclyl group
13) C₃₋₁₀ carbocyclyloxy
14) C₆₋₁₀ aryloxy
15) 5-membered or 6-membered heteroaryloxy
16) 4- to 10-membered heterocyclyloxy
17) C₃₋₁₀ carbocyclylthio
18) C₆₋₁₀ arylthio
19) 5-membered or 6-membered heteroarylthio
20) 4- to 10-membered heterocyclylthio
21) C₆₋₁₀ aryl
22) 5-membered or 6-membered heteroaryl
23) 4- to 10-membered heterocyclyl
24) C₃₋₁₀ carbocyclylcarbonyl
25) C₆₋₁₀ arylcarbonyl
26) 5-membered or 6-membered heteroarylcarbonyl
27) 4- to 10-membered heterocyclylcarbonyl
28) C₃₋₁₀ carbocyclylsulfonyl
29) C₆₋₁₀ arylsulfonyl
30) 5-membered or 6-membered heteroarylsulfonyl
31) 4- to 10-membered heterocyclylsulfonyl (however, each substituent from 12) to 31) is optionally substituted with 1 to 5 substituents in substituent group β1 or 5) C₁₋₆ alkyl)
32) -NR^{10a}R^{11a}
33) -SO₂-NR^{10b}R^{11b}
34) -NR^{10c}-_{C}(=O)R^{11c}
35) -NR^{10d}-C(=O)OR^{11d}
36) -NR^{12a}-C(=O)NR^{10e}R^{11e}
37) -NR¹⁰ⁱ-SO₂-R¹¹ⁱ
38) -NR^{12c}-SO₂-NR^{10j}R^{11j}
39) -C (=O)OR^{10k}
40) -C (=O)NR^{10l}R^{11k}
41) -C (=O)NR^{1m}OR^{11l}
42) -C (=O)NR^{12d}-NR¹⁰ⁿR^{11m}
43) -C (=NR^{13a})R^{10s}
44) -C (=NR^{13c})NR^{10t}R^{11q}
45) -C (=NR^{13d})NR^{12f}-NR^{10u}R^{11r}
46) -NR^{17c}-C(=NR13k)R^{17d}
47) -NR^{12g}-C(=NR^{13e})-NR^{10v}R^{11s}
48) -NR¹⁴-C(=NR^{13f})-NR^{12h}-NR^{10w}R^{11t}
49) -OC (=O)R^{10x}
50) -OC (=O)OR^{10y}
51) -OC (=O) NR^{10z}R^{11u}
52) -NR¹²ⁱ-NR^{10z2}R^{11v}
53) -NR^{10z3}OR^{11w}, and
54) a protecting group, and
substituent group β1 is a group consisting of
1) a halogen atom,
2) a hydroxyl group,
3) a carboxyl group,
4) a cyano group,
5) a C₃₋₁₀ carbocyclyl group,
6) C₁₋₆ alkoxy,
7) C₃₋₁₀ carbocyclyloxy,
8) C₁₋₆ alkylthio,
9) 5-membered or 6-membered heteroarylthio,
10) C₆₋₁₀ aryl,
11) 5-membered or 6-membered heteroaryl,
12) 4- to 10-membered heterocyclyl,
13) C₁₋₆ alkylcarbonyl,
14) C₃₋₁₀ carbocyclylcarbonyl,
15) C₆₋₁₀ arylcarbonyl,
16) 5-membered or 6-membered heteroarylcarbonyl,
17) 4- to 10-membered heterocyclylcarbonyl,
18) -NR^{15a}R^{16a},
19) -SO₂-NR^{15b}R^{16b},
20) -NR^{15c}-C(=O)R^{16c}
21) -NR^{17a}-C(=O)NR^{15d}R^{16d},
22) -C(=O)NR^{15e}R^{16e},
23) -C(=NR^{13g})R^{15f},
24) -C(=NR^{13h})NR^{15g}R^{16f}
25) -NR^{16g}-C(=NR¹³ⁱ)R^{15h}
26) -NR^{17b}-C(=NR^{13j})-NR¹⁵ⁱR^{16h}, and
27) a protecting group
   (however, each substituent from 5) to 17) in substituent group β1 is optionally substituted with 1 to 5 substituents selected from the group consisting of a halogen atom, a hydroxyl group, a cyano group, a carboxyl group, and -NR^{18a}R^{18b}),
wherein
R^{13a}, R13a2, R^{13c}, R13c2, R^{13d}, R13d2, R^{13e}, R^{13f}, R^{13g}, R^{13g2}, R^{13h}, R^{13h2}, R¹³ⁱ, R^{13j}, and R^{13k} are each independently the same or different a hydrogen atom, a hydroxyl group, C₁₋₆ alkyl, C₁₋₆ alkoxy, or C₁₋₆ alkoxycarbonyl,
R^{10a}, R^{10b}, R^{10c}, R^{10d}, R^{10e}, R¹⁰ⁱ, R^{10j}, R^{10k}, R101, R10m, R¹⁰ⁿ, R^{10s}, R^{10s2}, R^{10t}, R^{10t2}, R^{10u}, R10u2, R^{10v}, R^{10w}, R^{10x}, R^{10y}, R^{10z1}, R^{10z2}, R^{10z3}, R^{11a}, R^{11b}, R^{11c}, R^{11d}, R^{11e}, R¹¹ⁱ, R^{11j}, R^{11k}, R111, R^{11m}, R^{11q}, R^{11q2}, R^{11r}, R^{11r2}, R^{11s}, R^{11t}, R^{11u}, R^{11v}, R^{11w}, R12a, R^{12c}, R^{12d}, R^{12f}, R^{12f2}, R^{12g}, R^{12h}, R121, R14, R^{15a}, R^{15b}, R^{15c}, R^{15d}, R^{15e}, R^{15f}, R^{15f2}, R^{15g}, R^{15g2}, R^{15h}, R151, R16a, R^{16b}, R^{16c}, R^{16d}, R^{16e}, R^{16f}, R^{16f2}, R^{16g}, R^{16h}, R^{17a}, R^{17b}, R^{17c}, and R^{17d} are each independently the same or different a hydrogen atom, C₁₋₆ alkyl (the C₁₋₆ alkyl is optionally substituted with 1 to 3 of the same or different substituents selected from a hydroxyl group, a cyano group, C₁₋₆ alkoxy, and -NR^{18a}R^{18b}), or C₁₋₆ alkoxycarbonyl, and
R^{18a} and R^{18b} are each independently the same or different a hydrogen atom or C₁₋₆ alkyl.

In an exemplary embodiment, hydrogen of any hydroxyl group and amino group in substituent groups α1 and β1 is optionally substituted with a protecting group.

Examples of preferred substituents used when "optionally substituted" herein include the following.

Substituent group α2 preferably includes
1) a halogen atom
2) a hydroxyl group
3) a carboxyl group
4) a cyano group
5) C₁₋₆ alkyl
6) C₁₋₆ alkoxy
7) C₁₋₆ alkylthio
8) C₁₋₆ alkylcarbonyl
   (however, each substituent from 5) to 8) is optionally substituted with 1 to 5 same or different substituents selected from substituent group β2)
9) a C₃₋₁₀ carbocyclyl group
10) C₃₋₁₀ carbocyclyloxy
11) C₆₋₁₀ aryloxy
12) 5-membered or 6-membered heteroaryloxy
13) 4- to 10-membered heterocyclyloxy
14) C₃₋₁₀ carbocyclylthio
15) C₆₋₁₀ arylthio
16) 5-membered or 6-membered heteroarylthio
17) 4- to 10-membered heterocyclylthio
18) C₆₋₁₀ aryl
19) 5-membered or 6-membered heteroaryl
20) 4- to 10-membered heterocyclyl
21) C₃₋₁₀ carbocyclylcarbonyl
22) C₆₋₁₀ arylcarbonyl
23) 5-membered or 6-membered heteroarylcarbonyl
24) 4- to 10-membered heterocyclylcarbonyl
   (however, each substituent from 9) to 24) is optionally substituted with 1 to 5 substituents in substituent group β2 or 1) C₁₋₆ alkyl)
25) -NR^{10a}R^{11a}
26) -SO₂-NR^{10b}R^{11b}
27) -NR^{10c}-C(=O)R^{11c}
28) -NR^{12a}-C(=O)NR^{10d}R^{11d}
29) -NR^{10e}-SO₂-R^{11e}
30) -NR^{12b}-SO₂-NR^{10f}R^{11f}
31) -C(=O)NR^{10g}R^{11g}
32) -C(=NR^{13a})R^{10h}
33) -C(=NR^{13b})NR¹⁰ⁱR^{11h}
34) -NR^{11f2}-C(=NR^{13c})R^{10g2}, and
35) -NR^{12e}-C(=NR^{13d})-NR^{10j}R¹¹ⁱ, and
substituent group β2 is preferably a group consisting of
1) a halogen atom
2) a hydroxyl group
3) a cyano group
4) a C₃₋₁₀ carbocyclyl group
5) C₁₋₆ alkoxy
6) C₁₋₆ alkylthio
7) 5-membered or 6-membered heteroarylthio
8) 5-membered or 6-membered heteroaryl
9) 4- to 10-membered heterocyclyl
10) C₁₋₆ alkylcarbonyl
11) C₃₋₁₀ carbocyclylcarbonyl
12) C₆₋₁₀ arylcarbonyl
13) 5-membered or 6-membered heteroarylcarbonyl
14) 4- to 10-membered heterocyclylcarbonyl
15) -NR^{15a}R^{16a}
16) -NR^{15b}-C(=O)R^{16b}
17) -NR^{17a}-C(=O)NR^{15c}R^{16c}
18) -C(=O)NR^{15d}R^{16d}
19) -C(=NR^{13e})R^{15e}
20) -C(=NR^{13f})NR^{15f}R^{16e}
21) -NR^{16f}-C(=NR^{13g})R^{15g}
22) -NR^{17b}-C(=NR^{13h})-NR^{15h}R^{16g}
23) -C(=N-OR^{13e2})R^{15e2}, and
24) -C(=N-OR^{13f2})NR^{15f2}R^{16e2}
   (however, each substituent from 4) to 14) in substituent group β2 is optionally substituted with 1 to 5 substituents selected from the group consisting of a halogen atom, a hydroxyl group, a cyano group, a carboxyl group, and --NR^{18a}R^{18b})
wherein
R13a, R13a2, R^{13b}, R13b2, R^{13c}, R^{13d}, R^{13e}, R13e2, R^{13f}, R^{13f2}, R^{13g}, and R^{13h} are each independently the same or different a hydrogen atom, a hydroxyl group, C₁₋₆ alkyl, C₁₋₆ alkoxy, or C₁₋₆ alkoxycarbonyl,
R^{10a}, R^{10b}, R^{10c}, R^{10d}, R^{10e}, R^{10f}, R^{10g}, R^{10g2}, R^{10h}, R10h2, R101, R¹⁰ⁱ², R^{10j}, R^{11a}, R^{11b}, R^{11c}, R^{11d}, R^{11e}, R^{11f}, R^{11f2}, R^{11g}, R^{11h}, R^{11h2}, R111, R12a, R^{12b}, R^{12c}, R15a, R^{15b}, R^{15c}, R^{15d}, R^{15e}, R15e2, R^{15f}, R^{15f2}, R^{15g}, R^{15h}, R16a, R^{16b}, R^{16c}, R^{16d}, R^{16e}, R^{16e2}, R^{16f}, R^{16g}, R^{17a}, and R^{17b} are each independently the same or different a hydrogen atom, C₁₋₆ alkyl (the C₁₋₆ alkyl is optionally substituted with 1 to 3 same or different substituents selected from a hydroxyl group, a cyano group, C₁₋₆ alkoxy, and -NR^{18a}R^{18b}), or C₁₋₆ alkoxycarbonyl, and
R^{18a}, and R^{18b} are each independently the same or different a hydrogen atom or C₁₋₆ alkyl.

In an exemplary embodiment, hydrogen of any hydroxy group and amino group in substituent groups α2 and β2 is optionally substituted with a protecting group.

Examples of more preferred substituents used when "optionally substituted" herein include the following substituents.

Substituent group α3 more preferably includes
1) a halogen atom
2) a hydroxyl group
3) a cyano group
4) C₁₋₆ alkyl
5) C₁₋₆ alkoxy
6) C₁₋₆ alkylthio
7) C₁₋₆ alkylcarbonyl
   (however, each substituent from 4) to 7) is optionally substituted with 1 to 5 same or different substituents selected from substituent group β3)
8) a C₃₋₁₀ carbocyclyl group
9) 5-membered or 6-membered heteroaryloxy
10) 4- to 10-membered heterocyclyloxy
11) 5-membered or 6-membered heteroarylthio
12) 4- to 10-membered heterocyclylthio
13) C₆₋₁₀ aryl
14) 5-membered or 6-membered heteroaryl
15) 4- to 10-membered heterocyclyl
   (however, each substituent from 8) to 15) is optionally substituted with 1 to 5 substituents in substituent group β3 or 1) C₁₋₆ alkyl)
16) -NR^{10a}R^{11a}
17) -NR^{11b}-C(=O)R^{10b}
18) -NR^{12a}-C(=O)NR^{10c}R^{11c}
19) -C(=O)NR^{10d}R^{11d}
20) -C(=NR^{13a})R^{10e}
21) -C (=NR13b) NR^{10f}R^{11e}
22) -NR^{11f}-C(=NR^{13c})R^{10g}, and
23) -NR^{12b}-C(=NR^{13d})-NR^{10h}R^{11g}, and
substituent group β3 is more preferably
1) a halogen atom,
2) a hydroxyl group,
3) a cyano group,
4) -NR^{15a}R^{16a},
5) -NR^{15b}-C(=O)R^{16b},
6) -NR^{17a}-C(=O)NR^{15c}R^{16c},
7) -C(=O)NR^{15d}R^{16d},
8) -C (=NR^{13e})R^{15e},
9) -C(=NR^{13f})NR^{15f}R^{16e},
10) -NR^{16f}-C(=NR^{13g})R^{15g}, and
11) -NR^{17b}-C(=NR^{13h})-NR^{15h}R^{16g},
wherein
R13a, R13a2, R^{13b}, R13b2, R^{13c}, R^{13d}, R^{13e}, R13e2, R^{13f}, R^{13f2}, R^{13g}, and R^{13h} are each independently the same or different a hydrogen atom, a hydroxyl group, C₁₋₆ alkyl, C₁₋₆ alkoxy, or C₁₋₆ alkoxycarbonyl,
R^{10a}, R^{10b}, R^{10c}, R^{10d}, R^{10e}, R^{10f}, R10e2, R^{10f2}, R^{10 g}, R^{10h}, R^{11a}, R^{11b}, R^{11c}, R^{11d}, R^{11e}, R^{11e2}, R^{11f}, R^{11g}, R12a, R^{12b}, R15a, R^{15b}, R^{15c}, R15d, R^{15e}, R15e2, R^{15f}, R^{15f2}, R^{15g}, R^{15h}, R16a, R^{16b}, R^{16c}, R^{16d}, R^{16e}, R^{16e2}, R^{16f}, R^{16g}, R^{17a}, and R^{17b} are each independently the same or different a hydrogen atom, C₁₋₆ alkyl (the C₁₋₆ alkyl is optionally substituted with 1 to 3 same or different substituents selected from a hydroxyl group, a cyano group, C₁₋₆ alkoxy, and -NR^{18a}R^{18b}), or C₁₋₆ alkoxycarbonyl, and
R^{18a}, and R^{18b} are each independently the same or different a hydrogen atom or C₁₋₆ alkyl.

In an exemplary embodiment, hydrogen of any hydroxyl group and amino group in substituent groups α3 and β3 is optionally substituted with a protecting group.

Examples of still more preferred substituents used when "optionally substituted" herein include the following substituents.

Substituent group α4 still more preferably includes
1) a halogen atom
2) C₁₋₆ alkyl
3) C₁₋₆ alkylcarbonyl
   (however, each substituent from 2) to 3) is optionally substituted with 1 to 5 same or different substituents selected from substituent group β4)
4) a C₃₋₁₀ carbocyclyl group
5) C₆₋₁₀ aryl
6) 5-membered or 6-membered heteroaryl
7) 4- to 10-membered heterocyclyl
   (however, each substituent from 4) to 7) is optionally substituted with 1 to 5 substituents in substituent group β4 or 2) C₁₋₆ alkyl)
8) -NR^{10a}R^{11a}
9) -C(=O)NR^{10d}R^{11d}, and
10) -NR^{12b}-C(=NR^{13d})-NR^{10h}R^{11g}, and
substituent group β4 is still more preferably
1) a halogen atom,
2) -NR^{15a}R^{16a},
3) -NR^{15b}-C(=O)R^{16b},
4) -C(=O)NR^{15d}R^{16d}, and
5) -NR^{17b}-C(=NR^{13h})-NR^{15h}R^{16g},
wherein
R^{13d}, and R^{13h} are each independently the same or different a hydrogen atom, a hydroxyl group, C₁₋₆ alkyl, C₁₋₆ alkoxy, or C₁₋₆ alkoxycarbonyl, and
R^{10a}, R^{10d}, R^{10h}, R^{11a}, R^{11d}, R^{11g}, R^{12b}, R15a, R^{15b}, R^{15d}, R^{15h}, R^{16a}, R^{16b}, R^{16d}, R^{16g}, and R^{17b} are each independently the same or different a hydrogen atom, C₁₋₆ alkyl (the C₁₋₆ alkyl is optionally substituted with 1 to 3 same or different substituents selected from C₁₋₆ alkoxy, and -NR^{18a}R^{18b}), or C₁₋₆ alkoxycarbonyl.

In an exemplary embodiment, hydrogen of any hydroxyl group and amino group in substituent groups α4 and β4 is optionally substituted with a protecting group.

In an exemplary embodiment, a hydroxyl group in the aforementioned substituent group (e.g., α (α1 or the like) β (β1 or the like), or I to III) is also optionally substituted with a protecting group. In an exemplary embodiment, an amino group in the aforementioned substituent groups (e.g., α (α1 or the like) β (β1 or the like), or I to III) is also optionally protected with a nitrogen protecting group.

As used herein, "C₁₋₆" means that the number of carbon atoms is 1 to 6. The same applies to other numbers, for example, "C₁₋₄" means that the number of carbon atoms is 1 to 4, and "C₁₋₃" means that the number of carbon atoms is 1 to 3. A description with a limitation in the number of carbons herein is only a preferred numerical range, and it is intended so that groups with a substituent with a number of carbons other than the number of carbons specified in the present disclosure are also within the scope of the present disclosure.

As used herein, substituent names ending in the suffix "ene" refer to a biradical resulting from the removal of an additional hydrogen atom from a monoradical group as defined herein. Thus, for example, a monoradical alkyl as defined herein becomes a biradical alkylene by removal of an additional hydrogen atom. Similarly, alkenyl becomes alkenylene, alkynyl becomes alkynylene, heteroalkyl becomes heteroalkylene, heteroalkenyl becomes heteroalkenylene, heteroalkynyl becomes heteroalkynylene, carbocyclyl becomes carbocyclylene, heterocyclyl becomes heterocyclylene, aryl becomes arylene, and heteroaryl becomes heteroarylene.

As used herein, "hydrocarbon group" is also referred to as a hydrocarbyl group, referring to a group generated by removing at least one hydrogen from "hydrocarbon" comprising at least one carbon and at least one hydrogen.

As used herein, "functional group" refers to any group conferring some type of functionality, encompassing a carboxyl group, nitrile group, carbonyl group, hydroxyl group, amino group, imino group, nitro group, halogen group, as well as alkyl group, and more broadly acid anhydrides and groups formed by a bond such as an ester bond, amide bond, or ether bond.

As used herein, "heteroatom" refers to atoms other than carbon atoms and hydrogen atoms such as oxygen atoms, nitrogen atoms, and sulfur atoms. A group comprising a heteroatom is also known as a hetero... group (e.g., heteroaryl group (means that an aryl group comprises at least a heteroatom), a heterocyclic... group (e.g., a heterocyclic group (means that a cyclic group (carbon ring group) comprises at least one heteroatom)) or the like.

As used herein, "halogen atom" is an atom belonging to the halogen group, referring to a fluorine atom, chlorine atom, bromine atom, iodine atom, or the like. The halogen atom is preferably a fluorine atom or chlorine atom. The halogen atom is more preferably a fluorine atom. A "halogen atom" is also referred to as "halogen" or "halo".

As used herein, "hydroxyl group" is a monovalent group of -OH. This group is also referred to as a "hydroxy group" or "hydroxy".

As used herein, "carboxyl group" is a monovalent group of -COOH. This group is also referred to as a "carboxy group", "carboxy", or "carboxyl".

As used herein, "cyano group" is a monovalent group of - CN.

As used herein, "amino" is a monovalent group of -NH₂. This group is also referred to as an "amino group".

As used herein, "imino" is a monovalent group of the formula =N, or a divalent group of the formula -N-.

As used herein, "alkyl" refers to a linear or branched saturated aliphatic hydrocarbon group. "C₁₋₁₂ alkyl" is an alkyl group with 1 to 12 carbon atoms. Examples thereof include, but are not limited to, C₁₋₆ alkyl, heptyl, iso-heptyl, octyl, iso-octyl, nonyl, iso-nonyl, decyl, iso-decyl, undecyl, iso-undecyl, dodecyl, iso-dodecyl, and the like. "C₁₋₆ alkyl" is an alkyl group with 1 to 6 carbon atoms, which is preferably "C₁₋₄ alkyl", more preferably "C₁₋₃ alkyl", and still more preferably "C₁₋₂ alkyl". Specific examples of "C₁₋₄ alkyl" include methyl, ethyl, propyl, isopropyl, n-butyl, isobutyl, tert-butyl, sec-butyl, and the like. Specific examples of "C₁₋₆ alkyl" include, but are not limited to, C₁₋₄ alkyl, n-pentyl, isopentyl, neopentyl, tert-pentyl, 1,2-dimethylpropyl, n-hexyl, and the like.

As used herein, "alkenyl" refers to a linear or branched unsaturated aliphatic hydrocarbon group comprising at least one carbon-carbon double bond. "C₂₋₁₂ alkenyl" is an alkenyl group with 2 to 12 carbon atoms. Examples thereof include, but are not limited to, heptenyl, isoheptenyl, octenyl, isooctenyl, nonenyl, isononenyl, decenyl, isodecenyl, undecenyl, isoundecenyl, dodecenyl, isododecenyl, and the like. "C₂₋₆ alkenyl" is an alkenyl group with 2 to 6 carbon atoms. Preferred examples thereof include "C₂₋₄ alkenyl". Specific examples of "C₂₋₆ alkenyl" include, but are not limited to, vinyl, 1-propenyl, 2-propenyl, 1-butenyl, 2-butenyl, 3-butenyl, 2-methyl-1-propenyl, 2-methyl-2-propenyl, and the like.

As used herein, "alkynyl" refers to a linear or branched unsaturated aliphatic hydrocarbon group comprising at least one carbon-carbon triple bond. "C₂₋₁₂ alkynyl" is an alkynyl group with 2 to 12 carbon atoms. Examples thereof include, but are not limited to, heptynyl, isoheptynyl, octynyl, isooctynyl, nonynyl, isononynyl, decynyl, isodecynyl, undecynyl, isoundecynyl, dodecynyl, isododecynyl, and the like. "C₂₋₆ alkynyl" is an alkynyl group with 2 to 6 carbon atoms. Preferred examples thereof include "C₂₋₄ alkynyl". Specific examples of "C₂₋₆ alkynyl" include, but are not limited to, ethynyl, 1-propynyl, 2-propynyl, 1-butynyl, 1-methyl-2-propynyl, 3-butynyl, 1-pentynyl, 1-hexynyl, and the like.

As used herein, "aryl" refers to a monovalent group of a monocyclic or bicyclic aromatic hydrocarbon ring. "C₅₋₁₀ aryl" refers to an aryl group with 5 to 10 carbon atoms. Examples of "aryl" include, but are not limited to, C₆ aryl, C₁₀ aryl, and the like. Specific examples of C₆ aryl include, but are not limited to, phenyl and the like. Specific examples of C₁₀ aryl include, but are not limited to, 1-naphthyl, 2-naphthyl, and the like.

An aryl group as a substituent or a portion thereof may be fused to a carbocyclyl group. For example, a phenyl group may be fused to a cyclohexane ring to form a 1,2,3,4-tetrahydronaphthalenyl group. In such a case, one of the possible carbon atoms on a benzene ring attaches to the backbone, or to a group near the backbone, or to its atom. An aryl group encompasses 5,6,7,8-tetrahydronaphthalen-1-yl and 5,6,7,8-tetrahydronaphthalen-2-yl.

As used herein, "arylalkyl" refers to alkyl substituted with at least one aryl. "C₆₋₁₀ aryl C₁₋₆ alkyl" refers to C₁₋₆ alkyl substituted with at least one C₆₋₁₀ aryl. Specific examples of C₆₋₁₀ aryl C₁₋₆ alkyl include, but are not limited to, benzyl (phenyl-CH₂-), phenethyl (phenyl-CH₂CH₂-), naphthalen-1-ylmethyl, naphthalen-2-ylmethyl, 2-(naphthalen-1-yl)ethyl, 2-(naphthalen-2-yl)ethyl, and the like.

As used herein, "(optionally substituted amino)-arylalkyl" refers to arylalkyl substituted with an optionally substituted amino group, wherein the alkyl group, the aryl group, or both is substituted with an amino group. An amino group of such an arylalkyl group may be unsubstituted, or substituted with 1, 2, or 3 substituents, such as optionally substituted alkyl (e.g., unsubstituted C₁₋₆ alkyl, C₃₋₆ cycloalkyl-C₁₋₆ alkyl, C₃₋₆ cycloalkylcarbonyl, or the like). Examples of (optionally substituted amino)-C₆₋₁₀ aryl C₁₋₆ alkyl include, but are not limited to, (di(alkyl)amino)benzyl, ((cycloalkylalkyl)amino)benzyl, ((cycloalkylcarbonyl)amino)benzyl, ((carbamoylalkyl)carbonylamino)benzyl, ((carboxyalkyl)carbonyl)aminobenzyl, (di(alkyl) amino)naphthalenylmethyl, ((cycloalkylalkyl)amino)naphthalenylmethyl, ((cycloalkylcarbonyl)amino)naphthalenylmethyl, ((carbamoylalkyl)carbonylamino)naphthalenylmethyl, ((carboxyalkyl)carbonyl)aminonaphthalenylmethyl, and the like.

As used herein, the aryl moiety of "arylthio" has the same meaning as the aryl described above. Preferred examples of "C₆₋₁₀ arylthio" include "C₆ or C₁₀ arylthio". Specific examples of "C₆₋₁₀ aryloxy" include, but are not limited to, phenylthio, 1-naphthylthio, 2-naphthylthio, and the like.

As used herein, "aryl sulfonyl" refers to sulfonyl substituted with the "aryl" described above. "C₆₋₁₀ aryl sulfonyl" is preferably "C₆ or C₁₀ aryl sulfonyl". Specific examples of "C₆₋₁₀ aryl sulfonyl" include, but are not limited to, phenylsulfonyl, 1-naphthylsulfonyl, 2-naphthylsulfonyl, and the like.

As used herein, "heteroaryl" refers to a monovalent group of a monocyclic or bicyclic aromatic heterocycle comprising 1 to 4 same or different heteroatoms selected from the group consisting of an oxygen atom, nitrogen atom, and sulfur atom.

As used herein, "5-membered or 6-membered heteroaryl" refers to a monovalent group of a monocyclic aromatic heterocycle composed of 5 to 6 atoms comprising 1 to 4 same or different heteroatoms selected from the group consisting of an oxygen atom, nitrogen atom, and sulfur atom. Specific examples of "5-membered or 6-membered heteroaryl" include, but are not limited to, pyrrolyl, furyl, thienyl, pyrazolyl, imidazolyl, oxazolyl, isoxazolyl, oxadiazolyl, thiazolyl, isothiazolyl, thiadiazolyl, tetrazolyl, pyridyl, furyl, pyridazinyl, pyrimidinyl, pyrazinyl and the like.

As used herein, "5- to 10-membered heteroaryl" refers to a monovalent group of a monocyclic or bicyclic aromatic heterocycle composed of 5 to 10 atoms comprising 1 to 4 same or different heteroatoms selected from the group consisting of an oxygen atom, nitrogen atom, and sulfur atom. Specific examples of "5- to 10-membered heteroaryl" include, but are not limited to, 5-membered or 6-membered heteroaryl, quinolyl, isoquinolyl, naphthyridinyl, quinoxalinyl, cinnolinyl, quinazolinyl, phthalazinyl, imidazopyridyl, imidazothiazolyl, imidazooxazolyl, benzothiazolyl, benzoxazolyl, benzoimidazolyl, indolyl, isoindolyl, indazolyl, pyrrolopyridyl, thienopyridyl, furopyridyl, benzothiadiazolyl, benzoxadiazolyl, pyridopyrimidinyl, benzofuryl, benzothienyl, benzo[1,3]dioxole, thienofuryl, chromenyl, chromanyl, coumarinyl, quinolonyl, and the like.

As used herein, "heteroarylalkyl" refers to alkyl substituted with at least one heteroaryl. "5- to 10-membered heteroaryl C₁₋₆ alkyl" refers to C₁₋₆ alkyl substituted with at least one 5- to 10-membered heteroaryl. Specific examples of 5- to 10-membered heteroaryl C₁₋₆ alkyl include, but are not limited to, pyridin-2-ylmethyl, pyridin-4-ylmethyl, 2-(quinolin-8-yl)ethyl, 2-(quinolin-5-yl)ethyl, 2-(quinoxalin-5-yl)ethyl, 2-(1H-indol-3-yl)ethyl, and the like.

As used herein, "carbocyclyl" refers to a monovalent group from monocyclic, bicyclic or tricyclic non-aromatic hydrocarbon ring, including those that have a partially unsaturated bond, those that have a partially crosslinked structure, those that are partially spiro, and those having 1, 2, or more carbonyl structures. "Carbocyclyl" includes cycloalkyl, cycloalkenyl, and cycloalkynyl. "C₃₋₂₀ carbocyclyl" is preferably "C₃₋₁₀ carbocyclyl", more preferably "C₃₋₆ carbocyclyl". Specific examples of "C₃₋₂₀ carbocyclyl" include, but are not limited to, cyclopropyl, cyclobutyl, cyclopentyl, cyclohexyl, cycloheptyl, cyclooctyl, cyclohexadinyl, cycloheptadinyl, cyclooctadinyl, adamantyl or norbornyl and the like.

Carbocyclyl may be a fused ring formed by non-aryl ring and aryl and/or heteroaryl ring. For example, carbocyclyl include cycloalkyl fused with C₆₋₁₀ aryl or 5-membered or 6-membered heteroaryl. Examples of fused carbocyclyl include monovalent groups formed by removing one hydrogen atom from 1,2,3,4-tetrahydronaphthalene, indane, 1,2,3,4-tetrahydroanthracene, and 5,6,7,8-tetrahydroquinoline, and the like, and specific examples thereof include 1,2,3,4-tetrahydronaphthalen-1-yl, 1,2,3,4-tetrahydronaphthalen-2-yl, indan-1-yl, indan-2-yl, 5,6,7,8-tetrahydroquinolin-5-yl, 5,6,7,8-tetrahydroquinolin-6-yl and the like. In such fused carbocyclyl, any one of the possible ring-constituting atoms on the non-aryl ring attaches to the backbone.

As used herein, "C₃₋₁₀ carbocyclyl" refers to a substituent with C₃₋₁₀ carbocyclyl" that is a monovalent group among the "C₃₋₂₀ carbocyclyl" described above.

As used herein, "carbocyclyloxy" refers to a (carbocyclyl)-O- group, and the carbocyclyl moiety has the same meaning as the carbocyclyl group. "C₃₋₆ carbocyclyloxy" refers to a (C₃₋₆ carbocyclyl)-O- group, and the C₃₋₆ carbocyclyl moiety has the same meaning as the C₃₋₆ carbocyclyl. "C₃₋₆ carbocyclyloxy" is preferably "C₃₋₅ carbocyclyloxy". Specific examples of "C₃₋₆ carbocyclyloxy" include, but are not limited to, cyclopropyloxy, cyclobutyloxy, cyclopentyloxy, cyclohexyloxy and the like.

As used herein, "carbocyclylcarbonyl" refers to carbonyl substituted with the "carbocyclyl" described above. "C₃₋₁₀ carbocyclylcarbonyl" is preferably "C₃₋₆ carbocyclylcarbonyl". Specific examples of "C₃₋₁₀ carbocyclylcarbonyl" include, but are not limited to, cyclopropylcarbonyl, cyclobutylcarbonyl, cyclopentylcarbonyl, cyclohexylcarbonyl and the like.

As used herein, "carbocyclylthio" refers to a (carbocyclyl)-S- group, and the carbocyclyl moiety is as defined above. "C₃₋₁₀ carbocyclylthio" is preferably "C₃₋₆ carbocyclylthio". Specific examples of "C₃₋₆ carbocyclylthio" include, but are not limited to, cyclopropylthio, cyclobutylthio, cyclopentylthio, cyclohexylthio and the like.

As used herein, "carbocyclylsulfonyl" refers to a sulfonyl group substituted with the "carbocyclyl" described above. "C₃₋₁₀ carbocyclylsulfonyl" is preferably "C₃₋₆ carbocyclylsulfonyl". Specific examples of "C₃₋₁₀ carbocyclylsulfonyl" include, but are not limited to, cyclopropylsulfonyl, cyclobutylsulfonyl, cyclopentylsulfonyl, cyclohexylsulfonyl and the like.

As used herein, "cycloalkyl" refers to a non-aromatic saturated hydrocarbon ring group, including those that have a partially crosslinked structure, those that are partially spiro, those having 1, 2, or more carbonyl structures. "C₃₋₂₀ cycloalkyl" refers to monocyclic or bicyclic cycloalkyl with 3 to 20 carbon atoms. "C₃₋₆ cycloalkyl" refers to monocyclic cycloalkyl with 3 to 6 carbon atoms. Specific examples of C₃₋₆ cycloalkyl include, but are not limited to, cyclopropyl, cyclobutyl, cyclopentyl, cyclohexyl, and the like.

A cycloalkyl group can be fused to aryl and/or heteroaryl ring as a substituent or a portion thereof. For example, a cyclohexyl group can be fused to a benzene ring to form a 1,2,3,4-tetrahydronaphthalenyl group. In such a case, one of the possible carbon atoms on the cyclohexane ring attaches to the backbone, to a group near the backbone, or to its atom. A cycloalkyl group encompasses 1,2,3,4-tetrahydronaphthalen-1-yl, 1,2,3,4-tetrahydronaphthalen-2-yl, indan-1-yl, indan-2-yl, 5,6,7,8-tetrahydroquinolin-5-yl, and 5,6,7,8-tetrahydroquinolin-6-yl.

As used herein, "cycloalkylalkyl" refers to alkyl substituted with at least one cycloalkyl. "C₃₋₆ cycloalkyl C₁₋₆ alkyl" refers to C₁₋₆ alkyl substituted with at least one C₃₋₆ cycloalkyl. Specific examples of C₃₋₆ cycloalkyl C₁₋₆ alkyl include, but are not limited to, cyclopropylmethyl, cyclobutylmethyl, cyclopentylmethyl, cyclohexylmethyl, 2-cyclopropylethyl, 2-cyclobutylethyl, 2-cyclopentylethyl, 2-cyclohexylethyl, 3-cyclopropylpropyl, 3-cyclobutylpropyl, 3-cyclopentylpropyl, 3-cyclohexylpropyl, and the like.

As used herein, "heterocycloalkyl" refers to a non-aromatic saturated or partially unsaturated heterocycle comprised of 3 or more atoms, comprising 1, 2, or more same or different heteroatoms selected from the group consisting of an oxygen atom, nitrogen atom, and sulfur atom, including those that have a partially crosslinked structure and those that are partially spiro. "Heterocycloalkyl" encompasses "heterocyclyl". Heterocycloalkyl can have a structure where a non-aromatic heterocycle is fused to an aryl ring and/or heteroaryl ring.

As used herein, "heterocyclyl" refers to a monocyclic or bicyclic non-aromatic heterocycle comprised of 3 or more atoms, comprising 1, 2, or more same or different heteroatoms selected from the group consisting of an oxygen atom, nitrogen atom, and sulfur atom, including saturated heterocyclyl, those that have a partially unsaturated attachment, those that have a partially crosslinked structure, and those that are partially spiro. Heterocyclyl can form a fused ring with aryl or heteroaryl. For example, heterocyclyl fused to C₆₋₁₀ aryl or 5-membered or 6-membered heteroaryl is also encompassed by a heterocycle. 1, 2, or more carbonyl, thiocarbonyl, sulfinyl, or sulfonyl can be comprised to constitute the heterocyclyl. For example, lactam, thiolactam, lactone, thiolactone, cyclic imide, cyclic carbamate, cyclic thiocarbamate, and other cyclic groups are also encompassed by the heterocyclyl. In this regard, an oxygen atom of carbonyl, sulfinyl, and sulfonyl and a sulfur atom of thiocarbonyl are not included in the number of members of the ring (ring size) or the number of heteroatoms constituting the ring.

As used herein, "4- to 10-membered heterocyclyl" refers to a substituent with "4- to 10-membered heterocyclyl" that is a monovalent group among the "heterocyclyl" described above.

As used herein, the heterocyclyl moiety of "heterocyclyloxy" has the same meaning as the "heterocyclyl" described above. "4- to 10-membered heterocyclyloxy" is preferably "4- to 6-membered heterocyclyloxy". Specific examples of "4- to 10-membered heterocyclyloxy" include, but are not limited to, tetrahydrofuranyloxy, tetrahydropyranyloxy, azetidinyloxy, pyrrolidinyloxy, piperidinyloxy and the like.

As used herein, the heterocyclyl moiety of "heterocyclylthio" has the same meaning as the "heterocyclyl" described above. "4- to 10-membered heterocyclylthio" is preferably "4- to 6-membered heterocyclylthio". Specific examples of "4- to 10-membered heterocyclylthio" include, but are not limited to, tetrahydropyranylthio, piperidinylthio and the like.

As used herein, "heterocyclylcarbonyl" refers to a carbonyl group substituted with the "heterocyclyl" described above. "4- to 10-membered heterocyclylcarbonyl" is preferably "4- to 6-membered heterocyclylcarbonyl". Specific examples of "4- to 10-membered heterocyclylcarbonyl" include, but are not limited to, azetidinylcarbonyl, pyrrolidinylcarbonyl, piperidinylcarbonyl, morpholinylcarbonyl and the like.

As used herein, "heterocyclylsulfonyl" refers to a sulfonyl group substituted with the "heterocyclyl" described above. "4- to 10-membered heterocyclylsulfonyl" is preferably "4- to 6-membered heterocyclylsulfonyl". Specific examples of "4- to 10-membered heterocyclylsulfonyl" include, but are not limited to, azetidinylsulfonyl, pyrrolidinylsulfonyl, piperidinylsulfonyl, morpholinylsulfonyl and the like.

As used herein, "5- to 6-membered heterocycloalkyl" refers to heterocycloalkyl comprised of 5 to 6 cyclic atoms, comprising 1, 2, or more same or different heteroatoms selected from the group consisting of an oxygen atom, nitrogen atom, and sulfur atom.

As used herein, "heterocycloalkylalkyl" refers to alkyl substituted with at least one heterocycloalkyl.

As used herein, "alkylcarbonyl" is a monovalent group of -C(=O)-alkyl. Preferred examples of alkylcarbonyl include C₁₋₆ alkylcarbonyl. Specific examples of C₁₋₆ alkylcarbonyl include, but are not limited to, acetyl (CH₃C(=O)-), n-propanoyl (CH₃CH₂C(=O)-), n-butanoyl (CH₃CH₂CH₂C(=O)-), n-pentanoyl (CH₃(CH₂)₃C(=O)-), n-hexanoyl (CH₃(CH₂)₄C(=O)-), n-heptanoyl (CH₃(CH₂)₅C(=O)-), and the like.

As used herein, "alkoxy" is a monovalent group of -O-alkyl. Preferred examples of alkoxy include C₁₋₆ alkoxy (i.e., C₁₋₆ alkyl-O-), C₁₋₄ alkoxy (i.e., C₁₋₄ alkyl-O-), and the like. Specific examples of C₁₋₄ alkoxy include methoxy (CH₃O-), ethoxy (CH₃CH₂O-), n-propoxy (CH₃(CH₂)₂O-), isopropoxy ((CH₃)₂CHO-), n-butoxy (CH₃(CH₂)₃O-), isobutoxy ((CH₃)₂CHCH₂O-), tert-butoxy ((CH₃)₃CO-), sec-butoxy (CH₃CH₂CH(CH₃)O-), and the like. Specific examples of C₁₋₆ alkoxy include, but are not limited to, C₁₋₄ alkoxy, n-pentyloxy (CH₃(CH₂)₄O-), isopentyloxy ((CH₃)₂CHCH₂CH₂O-), neopentyloxy ((CH₃)₃CCH₂O-), tert-pentyloxy (CH₃CH₂C(CH₃)₂O-), 1,2-dimethylpropoxy (CH₃CH(CH₃)CH(CH₃)O-), and the like.

As used herein, "alkoxycarbonyl" is a monovalent group of -C(=O)-O-alkyl. Examples of alkoxycarbonyl include, but are not limited to, C₁₋₆ alkoxycarbonyl, preferably C₁₋₄ alkoxycarbonyl. Specific examples of C₁₋₄ alkoxycarbonyl include methoxycarbonyl, ethoxycarbonyl, n-propoxycarbonyl, isopropoxycarbonyl, n-butoxycarbonyl, sec-butoxycarbonyl, tert-butoxycarbonyl, isobutoxycarbonyl, and the like. Specific examples of C₁₋₆ alkoxycarbonyl include, but are not limited to, C₁₋₄ alkoxycarbonyl, n-pentyloxycarbonyl, isopentyloxycarbonyl, neopentyloxycarbonyl, tert-pentyloxycarbonyl, 1,2-dimethylpropyloxycarbonyl, n-hexyloxycarbonyl, and the like.

As used herein, "alkoxycarbonylamino" is a monovalent group of -NH-C(=O)-O-alkyl. Examples of alkoxycarbonylamino include, but are not limited to, C₁₋₆ alkoxycarbonylamino, preferably C₁₋₄ alkoxycarbonylamino. Specific examples of C₁₋₄ alkoxycarbonylamino include methoxycarbonylamino, ethoxycarbonylamino, n-propoxycarbonylamino, isopropoxycarbonylamino, n-butoxycarbonylamino, sec-butoxycarbonylamino, tert-butoxycarbonylamino, isobutoxycarbonylamino, and the like. Specific examples of C₁₋₆ alkoxycarbonylamino include, but are not limited to, C₁₋₄ alkoxycarbonylamino, n-pentyloxycarbonylamino, isopentyloxycarbonylamino, neopentyloxycarbonylamino, tert-pentyloxycarbonylamino, 1,2-dimethylpropyloxycarbonylamino, n-hexyloxycarbonylamino, and the like.

As used herein, "haloalkyl" is a monovalent group of halogenated alkyl, having one or more hydrogen on an alkyl group substituted with halogen. The term "perhaloalkyl" refers to haloalkyl with all hydrogen on the alkyl group substituted with halogen. For example, perfluoroethyl is -CF₂CF₃, and perchloro-n-propyl is -CCl₂CCl₂CCl₃. Examples of haloalkyl include C₁₋₆ haloalkyl, C₁₋₄ haloalkyl, C₁₋₃ haloalkyl, and the like. The term "fluoroalkyl" refers to an alkyl group in which one or more hydrogens is replaced with a fluoro group. Specific examples of C₁₋₃ alkyl include, but are not limited to, fluoromethyl, chloromethyl, bromomethyl, difluoromethyl, dichloromethyl, dibromomethyl, trifluoromethyl, trichloromethyl, tribromomethyl, fluorochloromethyl, difluorochloromethyl, fluorodichloromethyl, fluoroethyl, chloroethyl, bromoethyl, trifluoroethyl, trichloroethyl, tribromoethyl, perfluoroethyl, perchloroethyl, perbromoethyl, perfluoropropyl, perchloropropyl, perbromopropyl, perfluoroisopropyl, perchloroisopropyl, perbromoisopropyl, and the like. Specific examples of C₁₋₄ alkyl include, but are not limited to, C₁₋₃ haloalkyl, perfluorobutyl, perchlorobutyl, perbromobutyl, perfluoroisobutyl, perfluoro-t-butyl, and the like. Specific examples of C₁₋₆ alkyl include, but are not limited to, C₁₋₄ haloalkyl, perfluoro-n-pentyl, perfluoroisopentyl, perfluoroneopentyl, perfluoro-tert-pentyl, perfluoro-1,2-dimethylpropyl, and the like.

As used herein, "haloalkoxy" as well as "haloalkyloxy" is a monovalent group of -O-haloalkyl with one or more hydrogen on the alkyl group substituted with halogen. The term "perhaloalkoxy" refers to haloalkoxy with all hydrogen on the alkyl group substituted with halogen. For example, perfluoroethoxy is -OCF₂CF₃, and perchloro-n-propoxy is - OCCl₂CCl₂CCl₃. Preferred examples of haloalkoxy include C₁₋₆ haloalkoxy, C₁₋₄ haloalkoxy, C₁₋₃ haloalkoxy, and the like. Specific examples of C₁₋₃ alkoxy include, but are not limited to, fluoromethoxy, chloromethoxy, bromomethoxy, difluoromethoxy, dichloromethoxy, dibromomethoxy, trifluoromethoxy, trichloromethoxy, tribromomethoxy, fluorochloromethoxy, difluorochloromethoxy, fluorodichloromethoxy, fluoroethoxy, chloroethoxy, bromoethoxy, trifluoroethoxy, trichloroethoxy, tribromoethoxy, perfluoroethoxy, perchloroethoxy, perbromoethoxy, perfluoropropoxy, perchloropropoxy, perbromopropoxy, perfluoroisopropoxy, perchloroisopropoxy, perbromoisopropoxy, and the like. Specific examples of C₁₋₄ alkoxy include, but are not limited to, C₁₋₃ haloalkoxy, perfluorobutoxy, perchlorobutoxy, perbromobutoxy, perfluoroisobutoxy, perfluoro-t-butoxy, and the like. Specific examples of C₁₋₆ alkoxy include, but are not limited to, C₁₋₄ haloalkoxy, perfluoro-n-pentyloxy, perfluoroisopentyloxy, perfluoroneopentyloxy, perfluoro-tert-pentyloxy, perfluoro-1,2-dimethylpropoxy, and the like.

As used herein, "alkylsulfonyl" refers to a sulfonyl group substituted with the "alkyl" described above. "C₁₋₆ alkylsulfonyl" is preferably "C₁₋₄ alkylsulfonyl". Specific examples of "C₁₋₆ alkylsulfonyl" include, but are not limited to, methylsulfonyl, propionylsulfonyl, butyrylsulfonyl, and the like.

As used herein, the alkyl moiety of "alkylthio" has the same meaning as the alkyl described above. Examples of "C₁₋₆ alkylthio" include "C₁₋₄ alkylthio", and preferred examples thereof include "C₁₋₃ alkylthio". Specific examples of "C₁₋₆ alkylthio" include, but are not limited to, methylthio, ethylthio, propylthio, butylthio, isopropylthio, isobutylthio, tert-butylthio, sec-butylthio, isopentylthio, neopentylthio, tert-pentylthio, 1,2-dimethylpropylthio, and the like.

As used herein, "arylcarbonyl" is a monovalent group of - C(=O)-aryl. Preferred examples of arylcarbonyl include C₆₋₁₀ arylcarbonyl. Specific examples of C₆₋₁₀ arylcarbonyl include, but are not limited to, benzoyl (i.e., phenyl-C(=O)-), 1-naphthylcarbonyl, 2-naphthylcarbonyl, and the like.

As used herein, the aryl moiety of "aryloxy" has the same meaning as the aryl described above. Preferred examples of "C₆₋₁₀ aryloxy" include "C₆ or C₁₀ aryloxy". Specific examples of "C₆₋₁₀ aryloxy group" include, but are not limited to, a phenoxy group, 1-naphthyloxy group, 2-naphthyloxy group, and the like.

As used herein, "heteroarylcarbonyl" is a monovalent group of -C(=O)-heteroaryl.

As used herein, "heteroarylcarbonyl group" refers to a carbonyl group substituted with the "heteroaryl" described above. Specific examples of "5-membered or 6-membered heteroarylcarbonyl group" include, but are not limited to, a pyrazolylcarbonyl group, triazolylcarbonyl group, thiazolylcarbonyl group, thiadiazolylcarbonyl group, pyridylcarbonyl group, pyridazinylcarbonyl group, and the like.

As used herein, the heteroaryl moiety of the "heteroaryloxy group" has the same meaning as the "heteroaryl" described above. The 5-membered or 6-membered heteroaryl moiety of the "5-membered or 6-membered heteroaryloxy group" has the same meaning as the "5-membered heteroaryl" or "6-membered heteroaryl", respectively. Specific examples of "5-membered or 6-membered heteroaryloxy group" include, but are not limited to, a pyrazolyloxy group, triazolyloxy group, thiazolyloxy group, thiadiazolyloxy group, pyridyloxy group, pyridazinyloxy group, and the like.

As used herein, the heteroaryl moiety of the "heteroarylthio group" has the same meaning as the "heteroaryl" described above. The 5-membered or 6-membered heteroaryl moiety of the "5-membered or 6-membered heteroarylthio group" has the same meaning as the "5-membered heteroaryl" or "6-membered heteroaryl", respectively. Specific examples of "5-membered or 6-membered heteroarylthio group" include, but are not limited to, a pyrazolylthio group, triazolylthio group, thiazolylthio group, thiadiazolylthio group, pyridylthio group, pyridazinylthio group, and the like.

As used herein, the heteroaryl moiety of the "heteroarylsulfonyl group" has the same meaning as the "heteroaryl" described above. "5-membered or 6-membered heteroarylsulfonyl group" refers to a sulfonyl group substituted with "5-membered or 6-membered heteroaryl". Specific examples of "5-membered or 6-membered heteroarylsulfonyl group" include, but are not limited to, a pyrazolylsulfonyl group, triazolylsulfonyl group, thiazolylsulfonyl group, thiadiazolylsulfonyl group, pyridylsulfonyl group, pyridazinylsulfonyl group, and the like.

As used herein, "acyl" refers to is a monovalent group of -C(=O)-R_{acyl}, wherein R_{acyl} is hydrogen, optionally substituted alkyl, optionally substituted alkenyl, optionally substituted alkynyl, optionally substituted aryl, optionally substituted heteroaryl, or optionally substituted heterocycloalkyl. Specific examples of acyl, but are not limited to, formyl, and the exemplified groups of alkylcarbonyl, arylcarbonyl, and heteroarylcarbonyl, and the like.

As used herein, "optionally substituted carbonyl" group refers to a monovalent group of -C(=O)- (hydrogen or any group selected from a substituent group described herein). Examples of "optionally substituted carbonyl" group include, but are not limited to, formyl, and optionally substituted carbamoyl, alkylcarbonyl, alkoxycarbonyl, alkenylcarbonyl, alkenyloxycarbonyl, alkynylcarbonyl, alkynyloxycarbonyl, arylcarbonyl, aryloxycarbonyl, cycloalkylcarbonyl, cycloalkyloxycarbonyl, heteroarylcarbonyl, heteroaryloxycarbonyl, heterocycloalkylcarbonyl, heterocycloalkyloxycarbonyl, and the like. A carbonyl group substituted with hydrogen is a formyl group. A carbonyl group substituted with amino is a carbamoyl group.

As used herein, "optionally substituted oxy" group refers to a monovalent group of -O- (hydrogen or any group selected from a substituent group described herein). Examples of "optionally substituted oxy" group include, but are not limited to, hydroxy, and optionally substituted alkyloxy, alkenyloxy, alkynyloxy, aryloxy, heteroaryloxy, heterocycloalkyloxy, alkylcarbonyloxy, alkenylcarbonyloxy, alkynylcarbonyloxy, arylcarbonyloxy, heteroarylcarbonyloxy, heterocycloalkylcarbonyloxy, and the like. An oxy group substituted with hydrogen is a hydroxy group.

As used herein, "carbamoyl" is a monovalent group of - C(=O)-NH₂.

As used herein, the phrase "a substituent-substituted group" means that the group is substituted with at least one substituent. For example, "hydroxy-substituted C₁₋₆ alkyl" refers to C₁₋₆ alkyl that has at least one hydroxy substitution.

As used herein, "carbamoyl-substituted C₁₋₆ alkyl" is C₁₋₆ alkyl substituted with at least one -C(=O)-NH₂ group. Examples of "carbamoyl-substituted C₁₋₆ alkyl" include, but are not limited to, carbamoyl-substituted C₁₋₄ alkyl. Specific examples of "carbamoyl-substituted C₁₋₄ alkyl" include, but are not limited to, 2-amino-2-oxoethyl (i.e., H₂NC(=O)-CH₂- or carbamoylmethyl), 3-amino-3-oxopropyl (i.e., H₂NC(=O)-CH₂CH₂- or carbamoylethyl), 4-amino-4-oxobutyl (i.e., H₂NC(=O)-(CH₂)₃- or carbamoylpropyl), 5-amino-5-oxopentyl (i.e., H₂NC(=O)-(CH₂)₄- or carbamoylbutyl), and the like. Specific examples of "carbamoyl-substituted C₁₋₆ alkyl" include, but are not limited to, carbamoyl-substituted C₁₋₄ alkyl, 6-amino-6-oxohexyl (i.e., H₂NC(=O)-(CH₂)₅- or carbamoylpentyl), 7-amino-7-oxoheptyl (i.e., H₂NC(=O)-(CH₂)₆- or carbamoylhexyl), and the like.

As used herein, "arylalkyl substituted with alkyl-substituted amino" is arylalkyl substituted with at least one alkyl-substituted amino. Specific examples of C₁₋₆ alkyl-substituted amino include, but are not limited to, -NH(CH₃), - N(CH₃)₂, -NH(CH₂CH₃), -N(CH₂CH₃)₂, -NH((CH₂)₂CH₃), -N((CH₂)₂CH₃)₂, -NH(CH(CH₃)₂), -N(CH(CH₃)₂)₂, -NH((CH₂)₃CH₃), -N((CH₂)₃CH₃)₂, - NH(CH₂CH(CH₃)₂), -N(CH₂CH(CH₃)₂)₂, -NH((CH₂)₄CH₃), -N((CH₂)₄CH₃)₂, -NH((CH₂)₅CH₃), -N((CH₂)₅CH₃)₂ and the like. As used herein, the "alkyl-substituted amino" has the same meaning as "alkylamino".

As used herein, "carboxy-substituted alkyl" is alkyl substituted with at least one -COOH group. Examples of "carboxy-substituted C₁₋₆ alkyl" include, but are not limited to, "carboxy-substituted C₁₋₄ alkyl" and the like. Specific examples of "carboxy-substituted C₁₋₄ alkyl" include, but are not limited to, carboxymethyl, 2-carboxyethyl, 3-carboxypropyl, 4-carboxybutyl, and the like. Specific examples of "carboxy-substituted C₁₋₆ alkyl" include, but are not limited to, carboxy-substituted C₁₋₄ alkyl, 5-carboxypentyl, 6-carboxyhexyl, and the like.

A "protecting group" refers to a group of atoms that blocks, reduces, or prevents reactivity of a functional group when attached to a reactive functional group in a molecule. The compound of the present disclosure can have a substitution with a protecting group when appropriate or needed at any of R₁ to R₄ or any position of a substituent thereof or other substituents or the like. Compounds comprising such a protecting group are also within the scope of the present disclosure. Typically, a protecting group can be selectively removed during a synthesis process if desired. Examples of protecting groups are found in Greene and Wuts, Protective Groups in Organic Chemistry, 5th Edition, 2014, John Wiley & Sons, NY and Harrison et al., Compendium of Synthetic Organic Methods, Vol. 1 to 8, John Wiley & Sons, NY, or the like. As used herein, a "protecting group" may correspond to the definitions of 1) to 53) of substituent α and 1) to 26) of substituent β. In that case, in the substituent group α1, "54) protecting group" may be described as "54) a protecting group other than 1) to 53)," and in the substituent group β1, "27) protecting group" may be described as "a protecting group other than 1) to 26)". Representative examples of nitrogen protecting groups include, but are not limited to, formyl, acetyl, trifluoroacetyl, benzyl and substituted benzyl, benzyloxycarbonyl ("CBZ"), tert-butoxycarbonyl ("Boc"), trimethylsilyl ("TMS"), 2-trimethylsilylethanesulfonyl ("TES"), trityl and substituted trityl group, allyloxycarbonyl, 9-fluorenylmethyloxycarbonyl ("FMOC"), nitro-veratryloxycarbonyl ("NVOC"), 2-nitrobenzenesulfonyl, 2,4-dinitrobenzenesulfonyl, the group represented by "Protect" herein, and the like. Representative examples of hydroxyl protecting groups include, but are not limited to, groups that acylate (esterify) or alkylate a hydroxyl group, such as benzyl and trityl ether, as well as alkyl ether, tetrahydropyranyl ether, trialkylsilyl ether (e.g., TMS, triethylsilyl, t-butyldimethylsilyl (TBDMS), and triisopropylsilyl (TIPS)), alkyldiarylsilyl ether (e.g., t-butyldiphenylsilyl (TBDPS)), triarylsilyl ether (e.g., triphenylsilyl), glycol ether (e.g., ethylene glycol ether, propylene glycol ether, and the like), and allyl ether.

An amino group of the compound of the present disclosure (e.g., amino group of the backbone, amino group as a substituent, amino group in a substituent of said compound, or the like) can be protected with a nitrogen protecting group or a group represented by "Protect". An amino group in a substituent listed in a substituent group can be further protected with a nitrogen protecting group or a group represented by "Protect". A protected substituent can also be used as a substituent.

A hydroxy group of the compound of the present disclosure (e.g., a hydroxy group as a substituent, a hydroxy group in a substituent of said compound, a hydroxy group in a substituent group described above, or the like) can also be protected with a protecting group of a hydroxy group. A hydroxy group in a substituent listed in a substituent group can be further protected with a hydroxyl protecting group described herein. A protected substituent can also be used as a substituent.

### (Biotechnology, Infection)

As used herein, "serin/threonine kinase" refers to a protein kinase that has the function of phosphorylating specific parts of a protein to change its function, and phosphorylate the serine or threonine residues of the protein.

As used herein, "receptor tyrosine kinase" refers to a well-known family of membrane receptors that phosphorylate tyrosine residues. Many play important roles during development or cell division. Receptor tyrosine kinases have an extracellular ligand-binding domain, a transmembrane domain, and an intracellular catalytic domain. The extracellular domain binds cytokines, growth factors, or other ligands, and usually consists of one or more identifiable structural motifs, including a cysteine-rich region, a fibronectin III-like domain, an immunoglobulin-like domain, an EGF-like domain, a cadherin-like domain, a kringle-like domain, a factor VIII-like domain, a glycine-rich region, a leucine-rich region, an acidic region, and a discoidin-like domain. Activation of the intracellular kinase domain is achieved by ligand binding to the extracellular domain, which induces receptor dimerization. The thus activated receptor can autophosphorylate tyrosine residues outside the catalytic domain to promote stabilization of the active receptor conformation. The phosphorylated residues then also serve as binding sites for proteins that transduce signals in cells.

As used herein, "non-receptor tyrosine kinase" refers to a well-known family of tyrosine kinases that do not have a transmembrane region that phosphorylates tyrosine residues.

### (Pharmaceutical)

As used herein, "pharmaceutically acceptable salt" refers to an acid addition salt or base addition salt which is pharmaceutically acceptable for use. Specific examples of "pharmaceutically acceptable salts" include, but are not limited to, acid addition salts such as acetate, propionate, butyrate, formate, trifluoroacetate, maleate, fumarate, tartrate, citrate, stearate, succinate, ethylsuccinate, malonate, lactobionate, gluconate, glucoheptonate, benzoate, methanesulfonate, benzenesulfonate, para-toluenesulfonate (tosylate), laurylsulfate, malate, ascorbate, mandelate, saccharinate, xinafoate, pamoate, cinnamate, adipate, cysteine salt, N-acetyl cysteine salt, hydrochloride, hydrobromide, phosphate, sulfate, hydroiodide, nicotinate, oxalate, picrate, thiocyanate, undecanoate, acrylic acid polymer salt, and carboxyvinyl polymer; inorganic base addition salts such as lithium salt, sodium salt, potassium salt, and calcium salt; organic base addition salts such as morpholine and piperidine; amino acid addition salts such as aspartic acid and glutamic acid; and the like.

The phrase "compound or an isomer thereof or a salt thereof" refers to a compound, an isomer of the compound, a salt of the compound, or a salt of the isomer.

The term "target protein modulator" refers to a substance capable of modulating the activity of a target protein. Target protein modulators include degradation inducers, inhibitors and agonists of target proteins, and the like, and can bring about changes in the function or expression level of the protein.

The term "treat" includes preventive and/or therapeutic treatment. The term "preventive and/or therapeutic" treatment includes administration, to a host, of one or more compounds or pharmaceutical compositions of the present disclosure, which is approved in the art. When administered prior to a clinical diagnosis of an undesirable condition (e.g., disease, disorder or symptom of a host animal, or other undesirable conditions), treatment is preventive (i.e., for protection of the host from developing the undesirable condition), whereas when administered after a diagnosis of an undesirable condition, treatment is therapeutic (i.e., intended for ameliorating, recovering from, or stabilizing an existing undesirable condition or side effect thereof).

The term "prodrug" is intended to encompass compounds that are converted into the therapy activation agent (e.g., the compound of Formula A) of the disclosure under physiological conditions. A general method for preparing a prodrug is a method of including one or more selected portions for exposing a desired molecule by hydrolysis under physiological conditions. In another embodiment, a prodrug is converted by enzymatic activity of a host animal. For example, esters and carbonate (e.g., alcohol or carboxylic acid ester or carbonate) are preferred prodrugs of the present disclosure. In a specific embodiment, some of the formulations presented in above table or all of the compound of Formula A can be replaced with a suitable corresponding prodrug, wherein, for example, hydroxyl in the parent compound is given as an ester or carbonate, or carboxylic acid in the parent compound is given as an ester.

### (Pharmaceutical composition)

The composition and method of the present disclosure can be utilized for treating an individual in need thereof. In a specific embodiment, an individual is a mammal such as a human or non-human mammal. If administered to an animal such as a human, the composition or compound is preferably administered as a pharmaceutical composition preferably comprising, for example, the compound of the disclosure and a pharmaceutically acceptable carrier. Pharmaceutically acceptable carriers are well known in the art. Examples thereof include aqueous solutions such as water and buffered saline, and other solvents and vehicles such as glycol, glycerol, oil such as olive oil, and organic esters for injection. In a preferred embodiment, an aqueous solution does not, or substantially does not, contain a pyrogen if such a pharmaceutical composition is for administration to a human, especially for administration through an invasive route (e.g., route such as injection or implantation that avoids transport or diffusion through the epithelial barrier). An excipient can be selected, for example, to achieve delayed release of an agent or to selectively target one or more cells, tissues, or organs. A pharmaceutical composition can be a unit dose, such as a tablet, capsule (including sprinkle capsule and gelatin capsule), granule, lyophilized form for reconstitution, powder, liquid agent, syrup, suppository, injection, or the like. A composition can also be in a transdermal delivery system such as a skin patch. A composition can also be a suitable liquid agent for topical administration, such as an eye drop.

A pharmaceutically acceptable carrier can comprise a physiologically acceptable agent, which has an effect of, for example, stabilizing, increasing the solubility, or increasing the absorption of a compound such as the compound of the disclosure. Examples of such a physiologically acceptable agent include carbohydrates such as glucose, sucrose, and dextran, antioxidants such as ascorbic acid and glutathione, chelating agents, low molecular weight proteins, other stabilizers, excipients, and the like. Selection of a pharmaceutically acceptable carrier including physiologically acceptable carrier is dependent on, for example, the route of administration of a composition. A preparation or pharmaceutical composition can be a self-emulsifying drug delivery system or self-microemulsifying drug delivery system. A pharmaceutical composition (preparation) can also be a liposome or other polymer matrix, and a compound of the disclosure can be incorporated therein. A liposome such as a liposome comprising a phospholipid or another lipid is a non-toxic, physiologically acceptable, and metabolizable carrier that is relatively easy to prepare and administer.

The phrase "pharmaceutically acceptable" refers to a compound, material, composition, and/or dosage form that is suitable for use in contact with human or animal tissue without excessive toxicity, stimulation, allergic reaction, other problems, or complication, with a reasonable risk-reward ratio, within the scope of a sound medical judgment, upon use herein.

As used herein, the phrase "pharmaceutically acceptable carrier" refers to a pharmaceutically acceptable material, composition, or vehicle such as a liquid or solid filler, diluent, excipient, solvent, or capsule material. Each carrier must be "acceptable" in terms of being compatible with other ingredients of a formulation and unharmful to a patient. Some examples of materials that can act as a pharmaceutically acceptable carrier include the following: (1) saccharide such as lactose, glucose, and sucrose; (2) starch such as corn starch and potato starch; (3) cellulose and derivatives thereof such as sodium carboxymethylcellulose, ethyl cellulose, and cellulose acetate; (4) powder tragacanth; (5) malt; (6) gelatin; (7) talc; (8) excipients such as cocoa butter and suppository wax; (9) oil such as peanut oil, cottonseed oil, safflower oil, sesame oil, olive oil, corn oil, and soybean oil; (10) glycol such as propylene glycol; (11) polyol such as glycerin, sorbitol, mannitol, and polyethylene glycol; (12) esters such as ethyl oleate and ethyl laurate; (13) agar; (14) buffer such as magnesium hydroxide and aluminum hydroxide; (15) alginic acid; (16) pyrogen-free water; (17) isotonic saline; (18) Ringer's solution; (19) ethyl alcohol; (20) phosphate buffer; and (21) other nontoxic compatible substances that are used in pharmaceutical formulations.

A pharmaceutical composition (preparation) can be administered to a subject through any of several routes of administration including, for example, oral administration (e.g., oral medicine, tablet, capsule (including sprinkle capsule and gelatin capsule), bolus, powder, granule, or paste agent for application on the tongue in an aqueous or non-aqueous liquid agent of suspension); absorption through an oral mucous membrane (e.g., sublingual administration); rectal, through the anus, or vaginal administration (e.g., as a pessary, cream, foam, or the like); parenteral administration (including intramuscular, intravenous, subcutaneous, and intraspinal cavity administration as, for example, sterilized liquid agent or suspension); intranasal administration; intraperitoneal administration; subcutaneous administration; transdermal administration (e.g., as a patch applied to the skin); topical administration (e.g., cream, ointment, or spray applied to the skin, and eye drop); and the like. A compound can also be formulated for inhalation. In a specific embodiment, a compound only needs to be dissolved or suspended in sterilized water. Details for suitable routes of administration and composition that are suited thereto can be found in, for example, US Patent No. 6,110,973, US Patent No. 5,731,000, US Patent No. 5,541,231, US Patent No. 5,427,798, US Patent No. 5,358,970, and US Patent No. 4,172,896, and patents cited therein.

A formulation can be provided in a convenient unit dosage form and prepared by any method that is well known in the pharmaceutical field. The amount of active ingredient that can form a unit dose in combination with a carrier varies depending on the host being treated or specific dosing mode. The amount of active ingredient that can form a unit dose in combination with a carrier is generally an amount of compound that results in a therapeutic effect. In general, such an amount is in the range of about 1 percent to about 99 percent active ingredient, preferably about 5 percent to about 70 percent active ingredient, and most preferably about 10 percent to about 30 percent active ingredient with respect to 100 percent.

A method of preparing such formulations or compositions comprises associating an active compound such as the compound of the disclosure with a carrier and optionally one or more secondary ingredients. In general, a formulation is prepared by associating the compound of the disclosure with a liquid carrier or a finely divided solid carrier or both in a uniform and dense manner and then optionally molding a product.

The formulation of the present disclosure that is suitable for oral administration can be in a form of a capsule (including sprinkle capsule and gelatin capsule), cachet, pill, tablet, lozenge (flavored base, generally using sucrose and acacia or tragacanth), lyophilized formulation, powder, granule, liquid agent or suspension in an aqueous or non-aqueous liquid, oil-in-water or water-in-oil liquid emulsion, elixir or syrup, pastille (using an inert base such as gelatin or glycerin, or sucrose and acacia), and/or mouthwash. Each of them contains a determined amount of the compound of the disclosure as an active ingredient. The composition or compound can also be administered as a bolus, lozenge, or paste.

To prepare a solid dosage form for oral administration (capsule (including sprinkle capsule and gelatin capsule), tablet, pill, sugar-coated tablet, powder, granule, or the like), an active ingredient is mixed with one or more pharmaceutically acceptable carriers such as sodium citrate or dicalcium phosphate, and/or one of the following: (1) a filler or bulking agent such as starch, lactose, sucrose, glucose, mannitol, and/or silicic acid; (2) binding agent, such as carboxymethyl cellulose, alginate, gelatin, polyvinylpyrrolidone, sucrose, and/or acacia; (3) moisturizing agent such as glycerol; (4) disintegrant such as agar, calcium carbonate, potato starch or tapioca starch, alginic acid, specific silicate, and sodium carbonate; (5) dissolution retardant such as paraffin; (6) absorption promoting agent such as a quaternary ammonium compound; (7) humectant such as cetyl alcohol and glycerol monostearate; (8) absorbent such as kaolin or bentonite clay; (9) lubricant such as talc, calcium stearate, magnesium stearate, solid polyethylene glycol, sodium lauryl sulfate, or a mixture thereof; (10) complexing agent such as modified or unmodified cyclodextrin; and (11) colorant. For a capsule (including sprinkle capsule and gelatin capsule), tablet, and pill, a pharmaceutical composition can also comprise a buffer. A same type of solid composition can also use an excipient such as lactose or milk sugar, high molecular weight polyethylene glycol, and the like as a filler in a soft or hard filled gelatin capsule.

A tablet can be prepared by compressing or molding the active ingredient together with one or more optional secondary ingredients. A compressed tablet can be prepared by using a binding agent (e.g., gelatin or hydroxypropyl methylcellulose), lubricant, inert diluent, preservative, disintegrant (e.g., sodium carboxymethyl starch or crosslinked sodium carboxymethylcellulose), surface activator, or dispersant. A molded tablet can be prepared by molding a mixture of a powder compound moisturized with an inert liquid diluent in a suitable instrument.

A tablet or other solid dosage form of a pharmaceutical composition such as a sugar-coated tablet, capsule (including sprinkle capsule and gelatin capsule), pill, or granule can be optionally notched or prepared using a coating or shell such as an enteric coating or other coating that is known in pharmaceutical product formulation technologies. The tablet or solid dosage form can also be formulated to provide sustained release or controlled release of the active ingredient by using, for example, hydroxypropyl methylcellulose, other polymer matrix, liposome, and/or microsphere, which contains the active ingredient, at different ratios to provide a desired release profile. The tablet or solid dosage form can be sterilized, for example, by filtration through a bacteria retaining filter or by incorporating a sterilizing agent in a form of a sterilized solid composition that can be dissolved in sterilized water or another medium for sterilized injection immediately prior to use. These compositions can also be a composition, which optionally comprises an emulsifying agent and releases an active ingredient(s) only at, or preferentially at, a specific part of a digestive tract, optionally in a delayed manner. Examples of embedded composition that can be used include polymeric substances and wax. An active ingredient can be in a microcapsule form by using one or more types of excipients described above when suitable.

Examples of liquid dosage forms that are useful for oral administration include a pharmaceutically acceptable emulsion, lyophilized form for reconstitution, microemulsion, liquid agent, suspension, syrup, and elixir. In addition to the active ingredient, a liquid dosage form can comprise an inert diluent that is commonly used in the art such as water or another solvent, cyclodextrin or a derivative thereof, solubilizing agent or emulsifying agent such as ethyl alcohol, isopropyl alcohol, ethyl carbonate, ethyl acetate, benzyl alcohol, benzyl benzoate, propylene glycol, 1,3-butylene glycol, oil (especially, cottonseed oil, peanut oil, corn oil, germ oil, olive oil, castor oil, or sesame oil), glycerol, tetrahydrofuryl alcohol, polyethylene glycol, sorbitan fatty acid ester, a mixture thereof, or the like.

In addition to an inert diluent, an oral composition can also comprise an adjuvant such as a humectant, emulsifying agent, suspending agent, sweetener, flavoring agent, colorant, fragrance, preservative, and the like.

In addition to an active compound, a suspension can comprise a suspending agent such as ethoxylated isostearyl alcohol, polyoxyethylene sorbitol, sorbitan ester, microcrystalline cellulose, aluminum metahydroxide, bentonite, agar, tragacanth, mixture thereof, or the like.

A formulation of a pharmaceutical composition for rectal, vaginal, or urethral administration can be given as a suppository, which can be prepared by mixing one or more active compounds with one or more suitable non-stimulatory excipients or carriers, including for example, cocoa butter, polyethylene glycol, suppository wax, salicylate, or the like. The formulation is a solid at room temperature, but a liquid at body temperature, so that the formulation melts in the rectum or vaginal cavity and releases an active compound.

A formulation of a pharmaceutical composition for oral administration can be given as a mouthwash, oral spray, or oral ointment.

Instead of or in addition, the composition can be formulated for delivery via a catheter, stent, wire, or another intracavity device. Delivery via such a device can be particularly useful for delivery to the bladder, urethra, urinary tract, rectum, or intestine.

A formulation that is suitable for transvaginal administration includes a pessary, tampon, cream, gel, paste, foam, or spray formulation, which comprises a carrier that is known to be suitable in the art.

A dosage form for topical or transdermal administration includes powder, spray agent, ointment, paste, cream, lotion, gel, liquid agent, patch, and inhalant. An active compound can be mixed with a pharmaceutically acceptable carrier and any preservative, buffer, or aerosol agent that may be needed under sterilized conditions.

In addition to an active compound, an ointment, paste, cream, and gel can contain an excipient such as an animal or vegetable oil and fat, wax, paraffin, starch, tragacanth, cellulose derivative, polyethylene glycol, silicone, bentonite, silicic acid, talc, zinc oxide, mixture thereof, or the like.

In addition to an active compound, powder and spray can contain an excipient such as lactose, talc, silicic acid, aluminum hydroxide, calcium silicate, polyamide powder, mixture thereof, or the like. A spray can further contain conventional aerosol agent such as chlorofluorohydrocarbon or volatile unsubstituted hydrocarbon such as butane or propane.

A transdermal patch has given an advantage of providing controlled delivery of the compound of the disclosure to the body. Such a dosage form can be prepared by dissolving or dispersing an active compound in a suitable medium. The flux of a compound crossing the skin can be increased by using an absorption enhancing agent. Such a flux rate can be controlled either by providing a rate controlling membrane or by dispersing a compound within a polymer matrix or gel.

An ophthalmic formulation, ophthalmic ointment, powder, liquid agent, and the like are also envisioned to be within the scope of the present disclosure. Exemplary ophthalmic formulations are described in US Patent Application Publication Nos. 2005/0080056, 2005/0059744, 2005/0031697, and 2005/004074, and US Patent No. 6,583,124 (content of which is incorporated herein by reference). When desirable, a liquid ophthalmic formulation has the same property as a lachrymal fluid, aqueous humor, or vitreous humor, or is compatible with such fluids. The preferred route of administration is administration to the affected part (e.g., topical administration such as eye drops or administration through an implant).

As used herein, the phrases "parenteral administration" and "parenterally administered" refer to an administration mode other than enteral and topical administration and generally to injections. Examples thereof include, but are not limited to, intravenous, intramuscular, intraarterial, intraspinal cavity, intracapsular, intraorbital, intracardial, intradermal, intraperitoneal, transtracheal, subcutaneous, subepidermal, intraarticular, subcapsular, subarachnoidal, intraspinal, and intrasternum injection and infusion. A pharmaceutical composition that is suitable for parenteral administration comprises one or more active compounds in combination with one or more pharmaceutically acceptable, sterile, isotonic, aqueous or non-aqueous liquid agent, dispersion, suspension, or emulsion, or sterile powder that can be reconstituted in a sterile injection liquid agent or dispersion immediately prior to use, which can comprise an antioxidant, buffer, bacteriostatic agent, solute for isotonizing the target recipient blood and formulation, or suspending agent or thickening agent.

Examples of suitable aqueous and non-aqueous carriers that can be used in the pharmaceutical composition of the present disclosure include water, ethanol, polyol (e.g., glycerol, propylene glycol, polyethylene glycol, and the like), suitable mixtures thereof, vegetable oil such as olive oil, organic ester for injection such as ethyl oleate, and the like. A suitable fluidity can be maintained, for example, by using a coating material such as lecithin, by maintaining a required particle size for a dispersion, and by using a surfactant.

These compositions can also comprise an adjuvant such as a preservative, humectant, emulsifying agent, or dispersant. Various antimicrobial agents and antifungal agents such as paraben, chlorobutanol, and phenol sorbic acid can be included to ensure prevention of microbial action. It is also desirable to include an isotonizing agent such as a saccharide or sodium chloride in the composition. In addition, long-term absorption in a form of a pharmaceutical product for injection can be induced by including an agent for delaying absorption, such as aluminum monostearate or gelatin.

In some cases, it is desirable to delay absorption of a drug from subcutaneous injection or intramuscular injection in order to prolong the effect of the drug. This can be accomplished by using a liquid suspension of a crystalline or amorous material with poor water solubility. Accordingly, the drug absorption rate can be dependent on the dissolution rate thereof, and the rate can be dependent on the crystal size and crystalline form. Delayed absorption of a drug form that is parenterally administered can be accomplished by dissolving or suspending a drug in an oil vehicle.

A depot form of injection is prepared by forming a microcapsule matrix of a target compound in a biodegradable polymer (e.g., polylactide-polyglycolide or the like). The drug release rate can be controlled in accordance with the drug to polymer ratio and the property of the specific polymer used. Examples of other biodegradable polymers include poly(orthoester) and poly(anhydride). A formulation for depot injection is prepared by encapsulating a drug within a liposome or microemulsion that is compatible with body tissue.

For use in the method of the present disclosure, the active compound itself can be given, or 0.1 to 99.5% (more preferably 0.5 to 90%) of active ingredient can be given as a pharmaceutical composition in combination with a pharmaceutically acceptable carrier.

A method of introduction can also provide a drug with a refillable device or a biodegradable device. In recent years, various sustained release polymer devices have been developed and tested in vivo for controlled delivery of a drug including proteinaceous biological formulations. An implant for sustained release of a compound at a specific target site can be formed by using various biocompatible polymers (including hydrogel) including both a biodegradable polymer and a non-biodegradable polymer.

An amount of active ingredient that is effective to achieve a desired therapeutic response for a specific patient, composition, and mode of administration without toxicity to the specific patient can be obtained by changing the actual dosage level of the active ingredient in a pharmaceutical composition.

The selected dosage level is dependent on various factors including the activity of the specific compound or combination of compounds, ester, salt, or amide thereof used, route of administration, dosing time, discharge rate of the specific compound(s) used, period of continued treatment, other drugs, compound, and/or substance used in combination with the specific compound(s) used, age, sex, body weight, condition, overall health condition, and medical record of patient receiving treatment, and same type of factors that are well-known in the medical field.

Those skilled in the art who are physicians or veterinarians can readily determine and prescribe a therapeutically effective amount of the pharmaceutical composition that is required. For example, a physician or veterinarian can start the dose of a pharmaceutical composition or compound from a level that is lower than the level required to achieve a desired therapeutic effect and gradually increase the dosage until the desired effect is achieved. "Therapeutically effective amount" refers to a concentration of a compound that is sufficient to result in manifestation of the desired therapeutic effect. It is generally understood that the effective amount of compound varies depending on the body weight, sex, age, and medical record of the subject. Examples of other factors affecting the effective amount include, but are not limited to, the severity of the condition of a patient, disorder being treated, stability of compound, and, if desirable, another type of therapeutic agent co-administered with the compound of the disclosure. A greater total dose can be delivered by multiple administrations of the agent. A method of determining the potency and dosage is known to those skilled in the art (Isselbacher et al., (1996) Harrison's Principles of Internal Medicine, 13th edition, pages 1814 to 1882, which is incorporated herein by reference).

A suitable daily dose of an active compound used in the composition and method of the present disclosure is an amount of the compound, which is the lowest dose that is effective to generate a therapeutic effect. Such an effective dose is generally dependent on the factors described above.

When desirable, the effective daily dose of the active compound can be optionally administered in a unit dosage form divided into 1, 2, 3, 4, 5, 6, or more doses administered separately at a suitable interval throughout a day. In a specific embodiment of the present disclosure, an active compound can be administered two or three times daily. In a preferred embodiment, an active compound is administered once daily.

A patient who receives such a treatment can be any animal requiring the treatment, including primates, especially humans, and other mammals such as horses, cows, pigs, and sheep, and poultry and all pets.

In a specific embodiment, the compound of the disclosure can be used alone or administered concomitantly with another type of therapeutic agent. As used herein, the phrase "administered concomitantly" refers to any form of administration of two or more different therapeutic compounds, which administers a second compound while a previously administered therapeutic compound is still effective in the body (e.g., two compounds are simultaneously effective in the body of a patient, and this can include a synergistic effect of the two compounds). For example, different therapeutic compounds can be administered in the same formulation or separate formulations, simultaneously or sequentially. In a specific embodiment, different therapeutic compounds can be administered within 1 hour, within 12 hours, within 24 hours, within 36 hours, within 48 hours, within 72 hours, or within one week of each other. Thus, an individual receiving such treatment can benefit from a combined effect of different therapeutic compounds.

In a specific embodiment, concomitant administration of the compound of the disclosure and one or more additional therapeutic agent(s) (i.e., one or more additional chemotherapeutic agent(s)) provides improved potency compared to administration of each of the compound of the disclosure (e.g., compound of Formula A) and one or more additional therapeutic agent(s) individually. In such a specific embodiment, concomitant administration provides a synergistic effect, wherein the synergistic effect refers the sum of the effects of each individual administration of the compound of the disclosure and one or more additional therapeutic agents(s).

In one embodiment, the compound of the disclosure can be administered directly, or as a formulation, medicament, or a pharmaceutical composition using a suitable dosage form, by oral or parenteral administration. Specific examples of such dosage forms include, but are not limited to, tablets, capsules, powdered agents, granules, liquid agents, suspension, injection agents, patch-on agents, poultice, and the like. These formulations can be manufactured by a known method using an additive that is commonly used as a pharmaceutical additive.

As these additives, an excipient, disintegrant, binding agent, fluidizer, lubricant, coating agent, solubilizing agent, solubilizing promotor, thickener, dispersant, stabilizer, sweetener, flavoring agent, or the like can be used depending on the objective. Specific examples of these additives include, but are not limited to, lactose, mannitol, crystalline cellulose, low-substituted hydroxypropyl cellulose, corn starch, partially pregelatinized starch, carmellose calcium, croscarmellose sodium, hydroxypropyl cellulose, hydroxypropyl methylcellulose, polyvinyl alcohol, magnesium stearate, sodium stearyl fumarate, polyethylene glycol, propylene glycol, titanium oxide, talc, and the like.

The dosage of the compound of the disclosure is appropriately selected depending on the subject to be administered, route of administration, target disease, disorder or symptom, age or body weight of subject, combinations thereof, or the like. For example, the dosage is 0.01 mg as the lower limit (preferably 100 mg) and 10000 mg as the upper limit (preferably 6000 mg) per day for adults for oral administration. This amount can be administered once daily, or divided into several doses.

The timing of dosing of the compound of the disclosure and therapeutic agents thereof is not limited. The compound and therapeutic agent can be administered concurrently or sequentially to a subject being administered therewith. The compound of the disclosure and therapeutic agent thereof can be formulated as a combined agent. The dosage of the therapeutic agent can be appropriately selected based on the clinically used dose. The ratio of the compound of the disclosure and therapeutic agent thereof can be appropriately selected depending on the subject to be administered, route of administration, target disease, disorder or symptom, age or body weight of subject, combinations thereof, or the like.

In one embodiment of the present disclosure, the compound of the disclosure can be combined and administered concurrently or at different times upon use of a pharmaceutical composition. Such a pharmaceutical composition is also within the scope of the present disclosure.

Such a medicament, formulation, or pharmaceutical composition can be manufactured by mixing the compound of the disclosure and/or an addition agent (e.g., antimicrobial agent, antiviral agent (e.g., ribavirin, amantadine, or the like), sedative (e.g., ketamine, midazolam, or the like) or the like) with any suitable component, together or separately, as a combined agent, or as separate agents using any technology that is known in the art. An appropriate formulation such as a tablet, capsule, powder, granule, liquid agent, suspension, injection, patch, or poultice can be formulated by using any technology that is known in the art. If the compound of the disclosure and/or an addition agent (e.g., antimicrobial agent, antiviral agent (e.g., ribavirin, amantadine, or the like), sedative (e.g., ketamine, midazolam, or the like) or the like) are prepared as separate agents, they can be provided as a kit of two agents. The kit can provide one of the components as a single agent, with instructions (package insert or the like) instructing to combine and administer the other component (for the compound of the disclosure, the additional agent; for the addition agent (e.g., antimicrobial agent, antiviral agent (e.g., ribavirin, amantadine, or the like), sedative (e.g., ketamine, midazolam, or the like) or the like), the compound of the disclosure) concurrently or at different times.

If the compound of the disclosure is used as an active ingredient of a medicament, the compound is intended for use in not just humans, but also other animals other than humans (cat, dog, cow, horse, bat, fox, mongoose, raccoon, and the like).

### (Medicament)

In one aspect, the present disclosure provides a medicament (composition) for modulating the activity of a target protein. Such a medicament or pharmaceutical composition can be used in the preparation of individual single unit dosage forms. The pharmaceutical composition and dosage form of the present disclosure comprise the compound of the disclosure or a pharmaceutically acceptable salt, solvate, or stereoisomer thereof, and optionally an additional active substance. The pharmaceutical composition and dosage form of the present disclosure can further comprise one or more carriers, excipients, or diluents.

The pharmaceutical composition and dosage form of the present disclosure can also comprise one or more additional active substances or components. As a result, the pharmaceutical composition and dosage form of the present disclosure can comprise the compound of the disclosure or a pharmaceutically acceptable salt, solvate, or stereoisomer thereof, and at least one second active substance. An example of any second active substance is disclosed herein.

In one embodiment, the compound of the disclosure can be administered directly or as a formulation, medicament, or pharmaceutical composition using a suitable dosage form, through oral administration or parenteral administration (e.g., transmucosal (e.g., sublingual, transnasal, transvaginal, intracystic, transrectal, transpreputial, intraocular, cheek, or intra-aural), non-parenteral (e.g., subcutaneous, intravenous, bolus injection, intramuscular, or intraarterial), topical (e.g., eye drop or other ophthalmic preparation), transdermal, or transepidermal administration). Specific examples of such dosage forms include, but are not limited to, tablet, caplet, capsule such as a soft gelatin capsule with elasticity, cachet, troche, powder, lozenge, granule, liquid agent, dispersion, suppository, aerosol (e.g., transnasal aerosol or inhalant), gel, suspension (e.g., aqueous or non-aqueous liquid suspension, oil-in-water liquid emulsion, or water-in-oil liquid emulsion), elixir, injection, patch, poultice, and the like. These formulations can be manufactured by a known method using an additive that is commonly used as a pharmaceutical additive. The present disclosure can also be a liquid dosage form suitable for oral or transmucosal administration to a patient including elixir, a liquid dosage form suitable for parenteral administration to a patient, an ophthalmic preparation suitable for eye drops or other topical administration, an aseptic solid (e.g., crystalline or amorphous solid) that can be reconstituted to provide a liquid dosage form suitable for parenteral administration to a patient, or the like. The present disclosure can be administered as a single unit dosage form.

As the additive that can be used in the present disclosure, an excipient, disintegrant, binding agent, fluidizer, lubricant, coating agent, solubilizing agent, solubilizing promotor, thickener, dispersant, stabilizer, sweetener, flavoring agent, or the like can be used depending on the objective. Specific examples of these additives include, but are not limited to, lactose, mannitol, crystalline cellulose, low-substituted hydroxypropyl cellulose, corn starch, partially pregelatinized starch, carmellose calcium, croscarmellose sodium, hydroxypropyl cellulose, hydroxypropyl methylcellulose, polyvinyl alcohol, magnesium stearate, sodium stearyl fumarate, polyethylene glycol, propylene glycol, titanium oxide, talc, and the like.

The composition, shape, and type of the composition and dosage form of the present disclosure typically vary depending on the application thereof. For example, a dosage form used in quick therapy of a disease, disorder or symptom can contain a large amount of one or more active ingredients, which is an amount greater than the amount of dosage form used in chronic therapy of the disease, disorder or symptom. Likewise, a parenteral dosage form can comprise one or more active ingredients at a smaller amount, and the content of the active ingredient is less than the oral dosage form used in therapy of the disease, disorder or symptom. These and other methods with specific dosage forms that are different from each other encompassed by the present disclosure are obvious to those skilled in the art. See, for example, Remington's Pharmaceutical Sciences, 18th Ed., Mack Publishing, Easton, PA (1990).

In one embodiment, the pharmaceutical composition of the present disclosure that is suitable for oral administration can be presented as individual dosage form such as, but not limited to, tablet (e.g., chewable tablet), caplet, capsule, and liquid agent (e.g., flavored syrup). A typical oral dosage form of the present disclosure is prepared by forming a complete mixture combining at least one excipient and an active ingredient in accordance with normal drug formulation technology. An excipient can be in various forms in accordance with the form of a preparation that is desirable for administration. Non-limiting examples of excipients suitable for use in oral liquid or aerosol dosage form include water, glycol, oil, alcohol, flavoring agent, preservative, and colorant. Non-limiting examples of excipients suitable for use in a solid oral dosage form (e.g., powder, tablet, capsule, or caplet) include starch, saccharide, microcrystalline cellulose, diluent, granulating agent, lubricant, binding agent, and disintegrant.

Non-limiting examples of excipients that can be used in the oral dosage form of the present disclosure include binding agent, filler, disintegrant, and lubricant. Non-limiting examples of binding agents suitable for use in a pharmaceutical composition and dosage form include corn starch, potato starch, other starches, gelatin, natural and synthetic rubber such as acacia gum, sodium alginate, alginic acid, other alginate, tragacanth powder, guar gum, cellulose, and derivatives thereof (e.g., ethyl cellulose, cellulose acetate, carboxymethylcellulose calcium, and sodium carboxymethylcellulose), polyvinylpyrrolidone, methylcellulose, pregelatinized starch, hydroxypropyl methylcellulose, microcrystalline cellulose, and mixtures thereof.

Non-limiting examples of fillers suitable for use in the pharmaceutical composition and dosage form disclosed herein include talc, calcium carbonate (e.g., granule or powder), microcrystalline cellulose, powdered cellulose, dextran, kaolin, mannitol, silicic acid, sorbitol, starch, pregelatinized starch, and mixtures thereof. The binding agent or filter in the pharmaceutical composition of the present disclosure is typically present at about 50 to about 99% by mass of the pharmaceutical composition of dosage form.

Non-limiting examples of disintegrants that can be used in the pharmaceutical composition and dosage form of the present disclosure include agar, alginic acid, calcium carbonate, microcrystalline cellulose, sodium croscarmellose, crospovidone, polacrilin potassium, sodium carboxymethyl starch, potato or tapioca starch, other starches, pregelatinized starch, other starches, clay, other algin, other cellulose, gum, and mixtures thereof.

Non-limiting examples of lubricants that can be used in the pharmaceutical composition and dosage form of the present disclosure include calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (e.g., peanut oil, cottonseed oil, sunflower oil, sesame oil, olive oil, corn oil, and soybean oil), zinc stearate, ethyl oleate, ethyl laurate, agar, and mixtures thereof. Additional examples of lubricants include Syloid silica gel, coagulated aerosol of synthetic silica, CAB-O-SIL (pyrogenic silicon dioxide product, sold by Cabot (Boston, MA)), and mixtures thereof. A lubricant, if any, is typically used at an amount of about less than 1% by mass of the pharmaceutical composition of dosage form into which it is incorporated.

An effective ingredient or active ingredient of the present disclosure can be administered through release controlling means or through a delivery device or dosage form that is well known to those skilled in the art. Non-limiting examples of release controlling means or delivery devices include those disclosed in US Patent Nos. 3,845,770, 3,916,899, 3,536,809, 3,598,123, 4,008,719, 5,674,533, 5,059,595, 5,591,767, 5,120,548, 5,073,543, 5,639,476, 5,354,556, and 5,733,566, which are incorporated herein by reference. It is possible to provide delayed release or release control of one or more active ingredients using, for example, hydropropylmethyl cellulose, other polymer matrices, gel, permeable membrane, osmotic pressure system, multilayer coating, microparticles, liposome, microsphere or combination thereof in order to provide a desirable release profile at various ratios using such a dosage form. A suitable release control formulation that is known to those skilled in the art, including those disclosed herein, can be readily selected for use with the effective ingredient or active ingredient of the present disclosure. Thus, the present disclosure is not limited thereto, but encompasses unit dosage forms that are suitable for oral administration such as, without limitation, tablets, capsules, gelcaps, and caplets, which are prepared to be compatible with release control.

A parenteral dosage form can be administered to a patient through various routes that include, but are not limited to, subcutaneous, intravenous (including bolus injection), intramuscular, and intraarterial administration. Since such administration typically circumvents the natural defense of a patient against contaminants, a parenteral dosage form is preferably aseptic or sterilizable prior to administration to the patient. Non-limiting examples of the parental dosage form include a liquid agent that can be directly injected, a dried product which is readily dissolved or suspended in a pharmaceutically acceptable vehicle for injection, and suspension and emulsion that can be directly injected. A suitable vehicle that can be used for providing the parenteral dosage form of the present disclosure is well known to those skilled in the art. Non-limiting examples of the suitable vehicle include injection water (US Pharmacopoeia); aqueous vehicle such as, without limitation, sodium chloride solution for injection, Ringer's injection solution, dextrose injection solution, dextrose and sodium chloride injection solution, and Ringer's lactate injection solution; water miscible vehicles such as, without limitation, ethyl alcohol, polyethylene glycol, and polypropylene glycol; and non-water-miscible vehicles such as, without limitation, corn oil, cottonseed oil, peanut oil, sesame oil, ethyl oleate, isopropyl myristate, and benzyl benzoate.

A drug can be topically applied to the skin, appendage thereof, or various mucous membranes. Routes that can be used include sublingual, transnasal, transvaginal, intracystic, transrectal, transpreputial, intraocular, cheek, or intra-aural administration. Many dosage forms have been developed to deliver an active ingredient to a site of application to induce a topical action. Non-limiting examples of the topical and mucosal dosage form of the present disclosure include ointment, cream, gel, paste, powder, lotion, spray, liniment, poultice, aerosol, liquid agent, emulsion, suspension, eye drops, other ophthalmic preparations, and other dosage forms known to those skilled in the art. See Remington's Pharmaceutical Sciences, 18th Ed., Mack Publishing, Easton, PA (1990), Introduction to Pharmaceutical Dosage Forms, Lea & Febiger, Philadelphia (1985), and the like. A dosage form suitable for treatment of the mucosal tissue within the oral cavity can be formulated as a gargle or oral gel.

A suitable excipient (e.g., carrier or diluent) and other materials that can be used for providing the topical mucosal dosage form encompassed by the present disclosure are well known to those skilled in the medicinal field, and are determined in accordance with the specific tissue to which a given pharmaceutical composition or dosage form is applied. Non-limiting examples of typical excipients for forming a liquid agent, emulsion, or gel include water, acetone, ethanol, ethylene glycol, propylene glycol, butane-1,3-diol, isopropyl myristate, isopropyl palmitate, mineral oil, and mixture thereof, which are non-toxic and pharmaceutically acceptable.

A humectant such as an occlusive, moisturizer, mitigant, or a protein rejuvenator can also be added to a pharmaceutical composition or dosage form if desirable.

An occlusive is a substance that physically blocks the loss of moisture within the stratum corneum. Non-limiting examples of the occlusive include petroleum jelly, lanolin, mineral oil, silicone such as dimethicone, zinc oxide, and combinations thereof. An occlusive is preferably petroleum jelly or lanolin, and more preferably petroleum jelly with a minimum concentration of 5%.

A moisturizing agent is a substance that draws water to the skin and theoretically improve the hydration of the stratum corneum upon application the skin. However, water that is drawn to the skin is not from the atmosphere, but is water from another cell. With this type of moisturizing agent, evaporation from the skin can continue and actually exacerbate dryness. Non-limiting examples of the moisturizing agent include glycerin, sorbitol, urea, alpha hydroxyl acid, saccharide, and combinations thereof. Preferred examples of the moisturizing agent include alpha hydroxyl acid such as glycolic acid, lactic acid, malic acid, citric acid, tartaric acid, and the like.

A mitigant is a substance that provides smoothness to the skin by filling the space between skin layers with oil droplets, which generally does not result in blockage unless thickly applied. When used with an emulsifying agent, they can help retain oil and moisture within the stratum corneum. Vitamin E is a general additive that appears to have no effect except as a mitigant. Other vitamins such as Vitamin A and Vitamin D are also added, but the effect thereof is questionable. Non-limiting examples of the mitigant include mineral oil, lanolin, fatty acid, cholesterol, squalene, structured lipids, and combinations thereof.

The timing of administration of the compound of the disclosure and therapeutic agent thereof is not limited. They can be administered simultaneously or at an interval (e.g., different times) to a subject. The compound of the disclosure and therapeutic agent thereof can be prepared as a combined agent. The dosage of such a therapeutic agent can be appropriately selected based on clinically used doses. The ratio of the compound of the disclosure and therapeutic agent thereof can be appropriately selected depending on the subject to be administered, route of administration, target disease, disorder or symptom, age or body weight of subject, combinations thereof, or the like.

In one embodiment of the present disclosure, the compound of the disclosure, when using a pharmaceutical composition, can be combined at the same time or different time for administration. Such a pharmaceutical composition is also within the scope of the present disclosure.

Such a medicament, formulation, and pharmaceutical composition can be manufactured by mixing the compound of the disclosure and/or additional agent (e.g., antimicrobial drug, antiviral agent (e.g., ribavirin, amantadine, or the like), sedative (e.g., ketamine, midazolam, etc.), or the like) with any appropriate component, together or separately as a combined agent or separate agents, by using any technology known in the art, and can be formulated using any known technology in the art as an appropriate formulation such as a tablet, capsule, powder, granule, liquid agent, suspension, injection, patch, or poultice. If the compound of the disclosure and/or additional agent (e.g., antimicrobial drug, antiviral agent (e.g., ribavirin, amantadine, or the like), sedative (e.g., ketamine, midazolam, etc.), or the like) are prepared as separate agents, they can be provided as a kit of two agents. One component can be provided as a single agent, with an instruction (package insert or the like) instructing to combine another component (for the compound of the disclosure, additional agent, and for an additional agent (e.g., antimicrobial drug, antiviral agent (e.g., ribavirin, amantadine, or the like), sedative (e.g., ketamine, midazolam, etc.), or the like), the compound of the disclosure) at the same time or different times for administration.

If the compound of the disclosure is used as the active ingredient of a medicament, this can be intended for use in not only humans, but also other animals besides humans (cats, dogs, cows, horses, bats, foxes, mongooses, raccoons, and the like).

The toxicity and therapeutic efficacy of the compound of the disclosure can be determined by a standard pharmaceutical procedure in cultured cells or experimental animals by measuring, for example, LD₅₀ (lethal dosage for 50% of the population) and ED₅₀ (dosage that is therapeutically effective for 50% of the population). The ratio of doses between a toxic effect and therapeutic effect is a therapeutic index, which can be represented as LD₅₀/ED₅₀.

A compound exhibiting a high therapeutic index is preferable. While a compound exhibiting a toxic side effect can be used, a delivery system for targeting such a compound to an affected site of tissue must be carefully designed in order to minimize the potential damage to uninfected cells and therefore to reduce side effects.

Data obtained from a cell culture assay or animal test can be used for a prescription within the range of doses for use in humans. Such a dose of a compound is preferably within the range of a circulating concentration including ED₅₀ that involves hardly any or no toxicity. A dose can be varied within this range in accordance with the dosage form used and route of administration utilized. For a compound used in the method of the present disclosure, a therapeutically sufficient dosage can be first estimated from a cell culture assay. A dose that can materialize a range of circulating plasma concentration including IC₅₀ (i.e., concentration of test compound materializing half of maximum inhibition of symptom) determined in cell culture can be prescribed in an animal model. The dose that is useful in humans can be more accurately determined using such information. The plasma level can be measured by, for example, high performance liquid chromatography.

The compound of the disclosure can be administered orally and at a single dose of about 0.10 to about 150 mg/day in terms of the amount of active ingredient, or the same amount divided into multiple daily doses. The dose can be similarly computed for parenteral, topical, transmucosal (e.g., transnasal, transrectal, or transdermal), or topical administration. The amount of a pharmaceutical composition administered in the method of the present disclosure is determined in accordance with the target of treatment, severity of the disorder or symptom thereof, administration method, dosing frequency, and judgment of a physician. The dosing frequency is within the range from dosing nearly every hour to monthly dosing. In a specific embodiment, dosing is 8 times daily to once every other day, or 1 to 3 times a day. In a specific embodiment, the pharmaceutical composition of the present disclosure is administered chronically, e.g., every day.

As used herein, "or" is used when "at least one or more" of the listed matters in the sentence can be employed. When explicitly described herein as "within the range of two values", the range also includes the two values themselves.

Reference literatures such as scientific literatures, patents, and patent applications cited herein are incorporated herein by reference to the same extent that the entirety of each document is specifically described.

### (Preferred embodiments)

The preferred embodiments of the present disclosure are described hereinafter. It is understood that the embodiments provided hereinafter are provided for the better understanding of the present disclosure, so that the scope of the present disclosure should not be limited by the following descriptions. Thus, it is apparent that those skilled in the art can refer to the descriptions herein to make appropriate modifications within the scope of the present disclosure. It is also understood that the following embodiments of the present disclosure can be used individually or as a combination.

### (Compound and composition of the present disclosure)

In one aspect, the compound of the present disclosure, a compound represented by the following Formula (A) Z-L-X or an isomer thereof, or a salt thereof,
wherein
Z is a ligand moiety capable of specifically binding to an intracellular protein,
L is a linker moiety or a single bond, and
X is the following formula 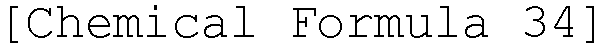 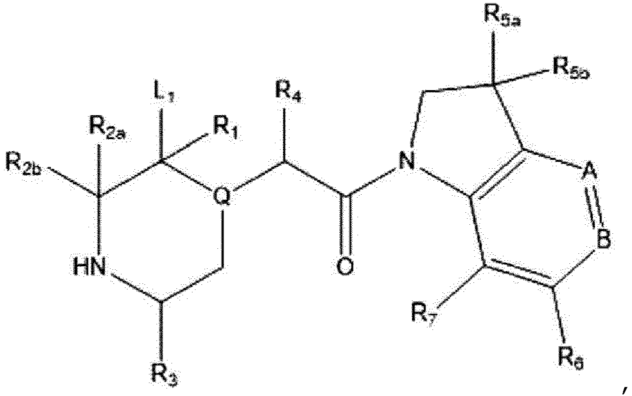 wherein
   A is N or CR₈,
   B is N, CR₉, or CL₂,
   Q is N or CR₁₀,
   R₁, and L₁ not used for bonding to L, are each independently selected from hydrogen, optionally substituted alkyl,
   optionally substituted alkenyl, optionally substituted alkynyl,
   optionally substituted heterocyclyl, and optionally substituted carbocyclyl,
   or R₁, and L₁ not used for bonding to L, together with the carbon atom to which they are attached, can join to form an optionally substituted carbocyclyl group or an optionally substituted heterocyclyl group;
   R₂ₐ and R_{2b} are each independently selected from hydrogen, optionally substituted alkyl, optionally substituted alkenyl, optionally substituted alkynyl, optionally substituted heterocyclyl, and optionally substituted carbocyclyl;
   or R₂ₐ and R_{2b}, together with the carbon atom to which they are attached, can join to form an optionally substituted carbocyclyl group or an optionally substituted heterocyclyl group;
   R³ is selected from optionally substituted alkyl, optionally substituted alkenyl, and optionally substituted carbocyclyl;
   R₄ is selected from hydrogen, optionally substituted alkyl, optionally substituted alkenyl, optionally substituted alkynyl, optionally substituted heterocyclyl, and optionally substituted carbocyclyl;
   R₅ₐ and R₅ are each independently selected from hydrogen, halogen, optionally substituted alkyl, optionally substituted heteroalkyl, optionally substituted alkenyl, optionally substituted alkynyl, optionally substituted heterocyclyl, and optionally substituted carbocyclyl,
   or R₅ₐ and R_{5b}, together with the carbon atom to which they are attached, can join to form an optionally substituted carbocyclyl group or an optionally substituted heterocyclyl group;
   R₆ is selected from hydrogen, halogen, =O, =S, nitro, optionally substituted alkyl, optionally substituted heteroalkyl, optionally substituted alkenyl, optionally substituted alkynyl, optionally substituted carbonyl, optionally substituted amino, optionally substituted hydroxy, optionally substituted heterocyclyl, optionally substituted carbocyclyl, optionally substituted aryl, and optionally substituted heteroaryl, and 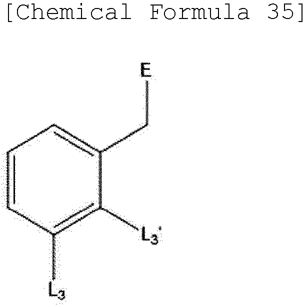
   wherein E indicates a bonding point,
   L₃ and L₃', together with the carbon atoms to which they are attached, can join to form an optionally substituted carbocyclyl or heterocyclyl group connected to a linker;
   R₇, R₈ and R₉ are each independently selected from hydrogen, halogen, =O, =S, nitro, optionally substituted alkyl, optionally substituted alkenyl, optionally substituted alkynyl, optionally substituted heterocyclyl, optionally substituted carbocyclyl, optionally substituted aryl, and optionally substituted heteroaryl;
   R₁₀ is selected from hydrogen, optionally substituted alkyl, optionally substituted alkenyl, optionally substituted alkynyl, optionally substituted heterocyclyl, and optionally substituted carbocyclyl; and
   X is bonded to L at any one of L₁ to L₃ and L₃'.

The compound of the present disclosure has a structure in which X capable of specifically binding to an E3 ligase (specifically, an inhibitor of apoptosis protein (IAP)) and Z capable of specifically binding to a target protein are linked via a linker (L). By binding to both the IAP and the target protein in cells, the compound can induce ubiquitination of the target protein by the IAP and modulation of its activity by the proteasome. Therefore, the Z moiety itself does not need to have a "functional activity" such as an inhibitory activity or agonist activity of the target protein, but as long as it has a "binding activity" that can specifically bind to the target protein, the activity modulation of the compound of the present disclosure can be achieved. For this reason, the Z moiety may be capable of binding to any other site in addition to the active site of a target protein such as an enzyme.

As an example, when the compound of the present disclosure is used as an anticancer agent, by administering it to a patient with breast cancer, cervical cancer, ovarian cancer, or the like, it can control protein modulatory activity in cancer tissue, thereby suppressing the proliferation of cancer cells and inducing the self-destruction of cancer cells, and can be used as a preventive and therapeutic agent for breast cancer, etc.

In one embodiment, the alkyl, alkenyl, alkynyl, carbocyclyl, and heterocyclyl in R₁, L₁ not used for bonding to L, R₂ₐ, R_{2b}, R₃, R₄, R₅ₐ, R_{5b} and R₁₀ are each independently optionally substituted with one to the maximum substitutable number of the same or different substituents selected from substituent group I,
the alkyl, alkenyl, alkynyl, carbocyclyl, and heterocyclyl, aryl and heteroaryl in R₆, R₇, R₈ and R₉ are each independently optionally substituted with one to the maximum substitutable number of the same or different substituents selected from substituent group I, and
the carbocyclyl and heterocyclyl groups in L₃ and L₃' are each independently optionally substituted with one to the maximum substitutable number of the same or different substituents selected from substituent group I.

In one embodiment, R₁, L₁ not used for bonding to L, R₂ₐ, R_{2b}, R₃, R₄, R₅ₐ, R_{5b} and R₁₀ are each independently hydrogen, optionally substituted alkyl, optionally substituted alkenyl, optionally substituted alkynyl, optionally substituted carbonylalkyl, optionally substituted carbamoylalkyl, optionally substituted carbocyclyl, optionally substituted heterocyclyl, optionally substituted carbonylcarbocyclyl, optionally substituted alkylcarbonyl, optionally substituted alkoxycarbonyl, carbamoyl, optionally substituted alkoxy, optionally substituted amino, optionally substituted alkylcarbamoyl, optionally substituted alkoxycarbamoyl, optionally substituted arylcarbamoyl, optionally substituted heteroarylcarbamoyl, optionally substituted cycloalkylcarbamoyl, or optionally substituted heterocycloalkylcarbamoyl, or R₁, and L₁ not used for bonding to L, together with the carbon atom to which they are attached, can join to form an optionally substituted carbocyclyl group or an optionally substituted heterocyclyl group, R₂ₐ and R_{2b}, together with the carbon atom to which they are attached, can join to form an optionally substituted carbocyclyl group or an optionally substituted heterocyclyl group, and R₅ₐ and R_{5b}, together with the carbon atom to which they are attached, can join to form an optionally substituted carbocyclyl group or an optionally substituted heterocyclyl group, wherein the aforementioned groups in R₁, L₁ not used for bonding to L, R₂ₐ, R_{2b}, R₃, R₄, R₅ₐ, R_{5b} and R₁₀ are optionally substituted with one to the maximum substitutable number of the same or different substituents selected from substituent group II.

In one embodiment, R₆ is selected from hydrogen, halogen, optionally substituted alkyl, optionally substituted heteroalkyl, optionally substituted cyanoalkyl, optionally substituted alkoxyalkyl, optionally substituted carbonyl, optionally substituted alkylamino, optionally substituted aminoalkyl, optionally substituted alkoxy, optionally substituted alkylcarbonyl, optionally substituted alkenyl, optionally substituted alkynyl, optionally substituted heterocyclylalkyl, optionally substituted carbocyclylalkyl, optionally substituted arylalkyl, optionally substituted heteroarylalkyl, and 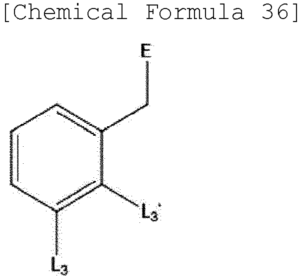
wherein E indicates a bonding point, and
L₃ and L₃', together with the carbon atoms to which they are attached, can join to form optionally substituted carbocyclyl or heterocyclyl connected to a linker, wherein the aforementioned groups in R₆ are optionally substituted with one to the maximum substitutable number of the same or different substituents selected from substituent group II.

In one embodiment, R₇, R₈ and R₉ are hydrogen, halogen, optionally substituted alkyl, optionally substituted alkenyl, optionally substituted alkynyl, optionally substituted alkoxyalkyl, optionally substituted alkoxy, optionally substituted cyanoalkyl, optionally substituted carboxyalkyl, optionally substituted carboxy, optionally substituted carbonyl, optionally substituted carbamoylalkyl, optionally substituted carbamoyl, optionally substituted alkylamino, optionally substituted carbocyclyl, optionally substituted heterocyclyl, optionally substituted carbocyclylalkyl, optionally substituted heterocyclylalkyl, optionally substituted alkoxycarbonyl, carbamoyl, optionally substituted alkoxy, optionally substituted amino, optionally substituted alkylcarbamoyl, optionally substituted alkoxycarbamoyl, optionally substituted arylcarbamoyl, optionally substituted heteroarylcarbamoyl, optionally substituted cycloalkylcarbamoyl, or optionally substituted heterocycloalkylcarbamoyl, wherein the aforementioned groups in R₇, R₈ and R₉ are optionally substituted with one to the maximum substitutable number of the same or different substituents selected from substituent group II.

In one embodiment, R₁₀ is hydrogen, optionally substituted alkyl, optionally substituted alkenyl, optionally substituted alkynyl, optionally substituted alkylcarbamoyl, optionally substituted carbamoyl, optionally substituted carbocyclylalkyl, optionally substituted heterocyclylalkyl, optionally substituted heterocyclylcarbonyl, or optionally substituted carbocyclylcarbonyl, wherein the aforementioned groups in R₁₀ are optionally substituted with one to the maximum substitutable number of the same or different substituents selected from substituent group II.

In one embodiment, R₁, L₁ not used for bonding to L, R₂ₐ, R_{2b}, R₃, R₄, R₅ₐ, R_{5b} and R₁₀ are each independently hydrogen, optionally substituted C₁₋₆ alkyl, optionally substituted C₂₋₆ alkenyl, optionally substituted C₂₋₆ alkynyl, optionally substituted C₂₋₆ alkylcarbamoyl, optionally substituted 3- to 12-membered heterocyclylalkyl, optionally substituted C₃₋₁₂ carbocyclylalkyl, optionally substituted 3- to 12-membered heterocyclylcarbonyl, or optionally substituted C₃₋₁₂ carbocyclylcarbonyl, or R₁, and L₁ not used for bonding to L, together with the carbon atom to which they are attached, can join to form an optionally substituted 3- to 10-membered carbocyclyl group or an optionally substituted 3- to 10-membered heterocyclyl group, wherein the aforementioned groups in R₁, and L₁ not used for bonding to L, are optionally substituted with one to the maximum substitutable number of the same or different substituents selected from substituent group III.

In one embodiment, R₂ₐ and R_{2b} are each independently hydrogen, optionally substituted C₁₋₆ alkyl, optionally substituted C₂₋₆ alkenyl, optionally substituted C₂₋₆ alkynyl, optionally substituted C₂₋₆ alkylcarbamoyl, optionally substituted 3- to 12-membered heterocyclylalkyl, and optionally substituted C₃₋₁₂ carbocyclylalkyl, or R₂ₐ and R_{2b}, together with the carbon atom to which they are attached, can join to form an optionally substituted 3- to 10-membered carbocyclyl group or an optionally substituted 3- to 10-membered heterocyclyl group, wherein the aforementioned groups in R₂ₐ and R_{2b} are optionally substituted with one to the maximum substitutable number of the same or different substituents selected from substituent group III.

In one embodiment, R³ is hydrogen, optionally substituted hydroxy, or optionally substituted hydroxyC₁₋₆ alkyl, wherein the aforementioned groups in R₃ are optionally substituted with one to the maximum substitutable number of the same or different substituents selected from substituent group III.

In one embodiment, R₄ is hydrogen, optionally substituted C₁₋₆ alkyl, optionally substituted C₂₋₆ alkenyl, optionally substituted C₂₋₆ alkynyl, optionally substituted C₃₋₈ cycloalkyl alkyl, or optionally substituted C₃₋₈ cycloalkenyl alkyl, wherein the aforementioned groups in R₄ are optionally substituted with one to the maximum substitutable number of the same or different substituents selected from substituent group III.

In one embodiment, R₅ₐ and R_{5b} are each independently hydrogen, optionally substituted C₁₋₈ alkyl, optionally substituted C₂₋₈ alkenyl, optionally substituted C₂₋₈ alkynyl, optionally substituted C₁₋₈ alkoxy, optionally substituted cyanoC₁₋₈ alkyl, optionally substituted carbonylC₁₋₈ alkyl, optionally substituted C₁₋₈ alkylcarbonyloxy, optionally substituted carboxyC₁₋₈ alkyl, optionally substituted hydroxyC₁₋₈ alkyl, optionally substituted aminoC₁₋₈ alkyl, optionally substituted C₁₋₈ alkylamino, optionally substituted iminoC₁₋₈ alkyl, optionally substituted C₁₋₈ alkylimino, or C₁₋₈ alkylcarbonylamino, or R₅ₐ and R_{5b}, together with the carbon atom to which they are attached, can join to form an optionally substituted 3- to 6-membered carbocyclyl group or an optionally substituted 4- to 6-membered heterocyclyl group, wherein the aforementioned groups in R₅ₐ and R_{5b} are optionally substituted with one to the maximum substitutable number of the same or different substituents selected from substituent group III.

In one embodiment, R₆ is hydrogen, halogen, optionally substituted C₁₋₈ alkyl, optionally substituted C₂₋₈ alkenyl, optionally substituted C₂₋₈ alkynyl, optionally substituted C₁₋₈ alkoxy, optionally substituted cyanoC₁₋₈ alkyl, optionally substituted carbonylC₁₋₈ alkyl, optionally substituted C₁₋₈ alkylcarbonyloxy, optionally substituted carboxyC₁₋₈ alkyl, optionally substituted hydroxyC₁₋₈ alkyl, optionally substituted aminoC₁₋₈ alkyl, optionally substituted C₁₋₈ alkylamino, optionally substituted iminoC₁₋₈ alkyl, optionally substituted C₁₋₈ alkylimino, C₁₋₈ alkylcarbonylamino, or 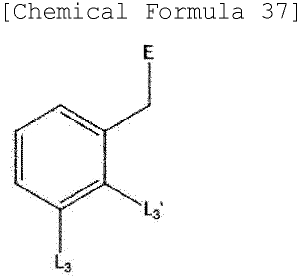
wherein E indicates a bonding point, and
L₃ and L₃', together with the carbon atoms to which they are attached, can join to form an optionally substituted 3- to 6-membered carbocyclyl or 4- to 6-membered heterocyclyl group connected to a linker, wherein the aforementioned groups in R₆ are optionally substituted with one to the maximum substitutable number of the same or different substituents selected from substituent group III.

In one embodiment, R₇, R₈ and R₉ are each independently hydrogen, halogen, optionally substituted C₁₋₈ alkyl, optionally substituted C₂₋₈ alkenyl, optionally substituted C₂₋₈ alkynyl, optionally substituted C₁₋₈ alkoxy, optionally substituted cyanoC₁₋₈ alkyl, optionally substituted carbonylC₁₋₈ alkyl, optionally substituted C₁₋₈ alkylcarbonyloxy, optionally substituted carboxyC₁₋₈ alkyl, optionally substituted hydroxyC₁₋₈ alkyl, optionally substituted aminoC₁₋₈ alkyl, optionally substituted C₁₋₈ alkylamino, optionally substituted iminoC₁₋₈ alkyl, optionally substituted C₁₋₈ alkylimino, or C₁₋₈ alkylcarbonylamino, or R₇, R₈ and R₉, together with the carbon atoms to which they are attached, can join to form an optionally substituted 3- to 6-membered carbocyclyl group or an optionally substituted 4- to 6-membered heterocyclyl group, wherein the aforementioned groups in R₇, R₈ and R₉ are optionally substituted with one to the maximum substitutable number of the same or different substituents selected from substituent group III.

In one embodiment, R₁₀ is hydrogen, optionally substituted C₁₋₆ alkyl, optionally substituted C₂₋₆ alkenyl, optionally substituted C₂₋₆ alkynyl, optionally substituted C₁₋₆ alkylcarbamoyl, optionally substituted 3- to 12-membered heterocyclylalkyl, optionally substituted C₃₋₁₂ carbocyclylalkyl, optionally substituted 3- to 12-membered heterocyclylcarbonyl, or optionally substituted C₃₋₁₂ carbocyclylcarbonyl, wherein the aforementioned groups in R₄ are optionally substituted with one to the maximum substitutable number of the same or different substituents selected from substituent group III.

In one aspect, the compound of the present disclosure is a compound represented by the following Formula (A) Z-L-X or an isomer thereof, or a salt thereof,
wherein
Z is a ligand moiety capable of specifically binding to an intracellular protein,
L is a linker moiety or a single bond, and
X is the following formula 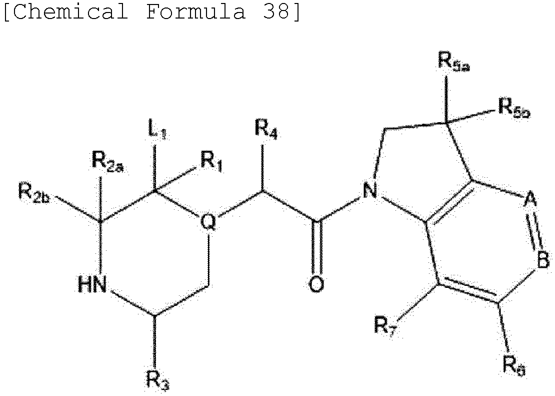 wherein
   A is N or CR₈,
   B is N, CR₉, or CL₂,
   Q is N or CR₁₀,
   R₁, and L₁ not used for bonding to L, are each independently selected from hydrogen, C₁₋₆ alkyl, C₂₋₆ alkenyl, C₂₋₆ alkynyl, - C(=O)NH_{(2-q)}(C₁₋₆ alkyl)_{q}, -(CH₂)ₛ-(3- to 12-membered heterocyclyl), -(CH₂)ₛ-C₃₋₁₂ carbocyclyl, -C(=O)-(3- to 12-membered heterocyclyl), and -C(=O)-C₃₋₁₂ carbocyclyl, or R₁, and L₁ not used for bonding to L, together with the carbon atom to which they are attached, can join to form a 3-to 10-membered saturated carbocyclyl or heterocyclyl group, wherein the C₁₋₆ alkyl, C₂₋₆ alkenyl, C₂₋₆ alkynyl, heterocyclyl and carbocyclyl groups are optionally substituted with one or more R^{b} groups;
   R₂ₐ and R_{2b} are each independently selected from hydrogen, C₁₋₆ alkyl, C₂₋₆ alkenyl, C₂₋₆ alkynyl, -C(=O) NH_{(2-q)}(C₁₋₆ alkyl)_{q}, - (CH₂)ₛ- (3- to 12-membered heterocyclyl), and -(CH₂)ₛ-C₃₋₁₂ carbocyclyl,
   or R₂ₐ and R_{2b}, together with the carbon atom to which they are attached, can join to form a 3- to 10-membered saturated carbocyclyl or heterocyclyl group, wherein the C₁₋₆ alkyl, C₂₋₆ alkenyl, C₂₋₆ alkynyl, heterocyclyl and carbocyclyl groups are optionally substituted with one or more R^{b} groups;
   R³ is selected from C₁₋₄ alkyl, C₂₋₄ alkenyl and -(CH₂)ₛ-C₃₋₈ cycloalkyl, wherein the C₁₋₄ alkyl, C₂₋₄ alkenyl, and C₃₋₈ cycloalkyl are optionally substituted with one or more R^{a} groups;
   R^{a} is selected from halogen, -OH and -O-C₁₋₆ alkyl;
   R₄ is selected from hydrogen, C₁₋₆ alkyl, C₂₋₆ alkenyl, C₂₋₆ alkynyl, -(CH₂)ₛ-C₃₋₈ cycloalkyl and - (CH₂)ₛ-C₃₋₈ cycloalkenyl, wherein the C₁₋₆ alkyl, C₂₋₆ alkenyl, C₂₋₆ alkynyl, C₃₋₈ cycloalkyl and C₃₋₈ cycloalkenyl are optionally substituted with one or more R^{a} groups;
   R₅ₐ and R_{5b} are each independently selected from hydrogen, C₁₋₈ alkyl, C₂₋₈ alkenyl, C₂₋₈ alkynyl, -Y-C₃₋₁₂ carbocyclyl, -W-(3- to 12-membered heterocyclyl), -(CR^{x}R^{y})ₛ-O-R^{z}, -O-(CR^{x}R^{y})ₙ-OR^{z}, - (CH₂)ₛ-CN, -S(O)_{q}-R^{x}, -C(=O)R^{x}, -C(=S)R^{x}, -C(=N)R^{x}, -(CRₓR_{y})ₛ-C(=O)OR^{z}, -(CR^{x}R^{y})ₛ-O-C(=O)-R^{z}, -(CR^{x}R^{y})ₛ-C(=O)NR^{x}R^{y}, -(CH₂)ₛ-NR^{x}C(=O)R^{y}, -(CH₂)ₛ-OC(=O)NR^{x}R^{y}, -(CH₂)ₛ-NR^{x}C(=O)OR^{y}, -(CH₂)ₛ-NR^{x}R^{y}, -NR^{x}-(CH₂)ₛ-R^{z}, -(CR^{x}R^{y})ₛ-C(=S)NR^{z}, -(CR^{x}R^{y})ₛ-C(=N)NR^{z}, - (CH₂)ₛ-O-C(=O)-C₁₋₄ alkyl-NR^{x}R^{y}, -(CH₂)ₛ-NR^{x}-(CH₂)ₙ-O-C(=O)-R^{z}, - (CH₂)ₑ-NR^{x}-(CH₂)ₛ-SO₂-R^{y}, -(CH₂)ₛ-NH-SO₂-NR^{x}R^{y} and - (CH₂)ₛ-SO₂NR^{x}R^{y} groups,
   or R₅ₐ and R_{5b}, together with the carbon atom to which they are attached, can join to form a 3- to 6-membered carbocyclyl or 4-to 6-membered heterocyclyl group,
   wherein the C₁₋₈ alkyl, C₂₋₈ alkenyl and C₂₋₈ alkynyl groups are optionally substituted with one or more R^{b} groups, and wherein the carbocyclyl group and heterocyclyl group are optionally substituted with one or more R^{b} groups;
   R₆ is selected from hydrogen, halogen, C₁₋₈ alkyl, C₂₋₈ alkenyl, C₂₋₈ alkynyl, -Y-C₃₋₁₂ carbocyclyl, -W-(3- to 12-membered heterocyclyl), -(CR^{x}R^{y})ₛ-O-R^{z}, -O-(CR^{x}R^{y})ₙ-OR^{z}, =O, =S, nitro, Si(R^{x})₄, -(CH₂)ₛ-CN, -S(O)_{q}-(CR^{x}R^{y})ₛ-R^{z}, -C(=O)R^{x}, -C(=S)R^{x}, - C(=N)R^{x}, -(CR^{x}R^{y})ₛ-C(=O)OR^{z}, - (CR^{x}R^{y})ₛ-O-C(=O) -R^{z}, -(CR^{x}R^{y})ₛ-C(=O)NR^{x}R^{y}, -(CH₂)ₛ-NR^{x}C(=O)R^{y}, -(CH₂)ₛ-OC(=O)NR^{x}R^{y}, -(CH₂)ₛ-NR^{x}C(=O)OR^{y}, - (CH₂)ₛ-NR^{x}R^{y}, -NR^{x}-(CH₂)ₛ-R^{z}, - (CR^{x}R^{y})ₛ-C(=S)NR^{z}, - (CR^{x}R^{y})ₛ-C(=N)NR^{x}, -S(O)(=NR^{x})R^{y}, -(CH₂)ₛ-O-C(=O)-C₁₋₄ alkyl-NR^{x}R^{y}, -(CH₂)ₛ-NR^{x}-(CH₂)ₙ-O-C(=O)-R^{z}, -(CH₂)ₛ-NR^{x}-(CH₂)ₛ-SO₂-R^{y}, -(CH₂)ₛ-NH-SO₂-NR^{x}R^{y} and -(CH₂)ₛ-SO₂NR^{x}R^{y} groups, -P(=O)(R^{x})₂, -Y-C₅₋₁₀ aryl, -Y-(5- to 10-membered heteroaryl), an optionally fused 5-to 10-membered carbocyclyl group, an optionally fused 5- to 10-membered heterocyclyl group, and 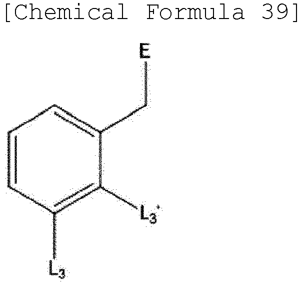
   wherein E indicates a bonding point,
   L₃ and L₃', together with the carbon atoms to which they are attached, can join to form a 3- to 6-membered carbocyclyl or 4-to 6-membered heterocyclyl group connected to a linker, wherein the C₁₋₈ alkyl, C₂₋₈ alkenyl and C₂₋₈ alkynyl groups, carbocyclyl group and heterocyclyl group, and 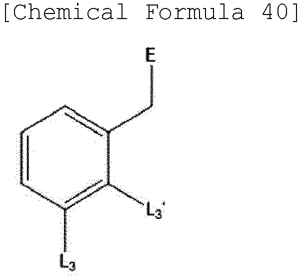
   wherein E indicates a bonding point, are optionally substituted with one or more R^{b} groups;
   R₇, R₈ and R₉ are each independently selected from hydrogen, halogen, C₁₋₈ alkyl, C₂₋₈ alkenyl, C₂₋₈ alkynyl, -Y-C₃₋₁₂ carbocyclyl, -W-(3- to 12-membered heterocyclyl), -(CR^{x}R^{y})ₛ-O-R^{z}, -O-(CR^{x}R^{y})ₙ-OR^{z}, =O, =S, nitro, Si(R^{x})₄, - (CH₂)ₛ-CN, -S(O)_{q}-(CR^{x}R^{y})ₛ-R^{z}, -C(=O)R^{x}, -C(=S)R^{x}, -C (=N) R^{x}, - (CR^{x}R^{y})ₛ-C(=O) OR^{z}, - (CR^{x}R^{y})ₛ-O-C(=O)-R^{z}, -(CR^{x}R^{y})ₛ-C(=O)NR^{x}R^{y}, -(CH₂)ₛ-NR^{x}C(=O)R^{y}, - (CH₂)ₛ-OC(=O)NR^{x}R^{y}, -(CH₂)ₛ-NR^{x}C(=O)OR^{y}, -(CH₂)ₛ-NR^{x}R^{y}, -NR^{x}-(CH₂)ₛ-R^{z}, -(CR^{x}R^{y})ₛ-C(=S)NR^{z}, -(CR^{x}R^{y})ₛ-C(=N)NR^{x}, -S(O)(=NR^{x})R^{y}, -(CH₂)ₛ-O-C(=O)-C₁₋₄alkyl-NR^{x}R^{y}, -(CH₂)ₛ-NR^{x}-(CH₂)ₙ-O-C(=O)-R^{z}, -(CH₂)ₛ-NR^{x}-(CH₂)ₛ-SO₂-R^{y}, -(CH₂)ₛ-NH-SO₂-NR^{x}R^{y} and -(CH₂)ₛ-SO₂NR^{x}R^{y} groups, -P (=O)(R^{x})₂ and -Y-C₅₋₁₀ aryl, wherein the C₁₋₈ alkyl, C₂₋₈ alkenyl and C₂₋₈ alkynyl groups are optionally substituted with one or more R^{b} groups, and wherein the carbocyclyl and heterocyclyl groups are optionally substituted with one or more (e.g., 1, 2 or 3) R^{b} group;
   each R^{b} is independently selected from halogen, C₁₋₆ alkyl, C₂₋₆ alkenyl, C₂₋₆ alkynyl, - (CH₂)ₛ-C₃₋₈ cycloalkyl, - (CH₂)ₛ-C₃₋₈ cycloalkenyl, -(CH₂)ₛ-phenyl, -(CH₂)ₛ-(5- to 10-membered heteroaryl), -(CH₂)ₛ-(4- to 7-membered saturated heterocyclyl), - (CR^{x}R^{y})ₛ-O-R^{z}, -O-(CR^{x}R^{y})ₙ-OR^{z}, haloC₁₋₆ alkyl, haloC₁₋₆ alkoxy, hydroxy, hydroxyC₁₋₆ alkyl, =O, =S, nitro, Si(Rx)₄, - (CH₂)ₛ-CN, - S (O)_{q}-R^{x}, -C(=O)R^{x}, - (CR^{x}R^{y})ₛ-C(=O) OR^{z}, - (CR^{x}R^{y})ₛ-O-C(=O)-R^{z}, - (CR^{x}R^{y})ₛ-C(=O)NR^{x}R^{y}, - (CH₂)ₛ-NR^{x}C(=O)R^{y}, - (CH₂)ₛ-OC(=O)NR^{x}R^{y},-(CH₂)ₛ-NR^{x}C(=O)OR^{y}-(CH₂)ₛ-NR^{x}R^{y}, -NR^{×}-(CH₂)ₛ-R^{z}, - (CH₂)ₑ-O-C(=O)-C₁-₄ alkyl-NR^{x}R^{y}, - (CH₂)ₛ-NR^{x}-(CH₂)ₙ-O-C(=O) -R^{z}, - (CH₂)ₛ-NR^{x}-(CH₂)ₛ-SO₂-R^{Y}, - (CH₂)ₑ-NH-SO₂-NR^{×}R^{y} and - (CH₂)ₛ-SO₂NR^{×}R^{y} groups and - P(=O)(R^{×})₂, wherein the C₁₋₆ alkyl, C₂₋₆ alkenyl, C₂₋₆ alkynyl, C₃₋₈ cycloalkyl, C₃₋₈ cycloalkenyl and heterocyclyl groups, phenyl and heteroaryl are optionally substituted with one or more R^{x} groups;
   R^{×}, R^{y} and R^{z} are each independently selected from halogen, hydrogen, C₁₋₆ alkyl, C₂₋₆ alkenyl, C₂₋₆ alkynyl, - (CH₂)ₛ-C₃₋₈ cycloalkyl, - (CH₂)ₛ-C₃₋₈ cycloalkenyl, - (CH₂)ₛ-phenyl, - (CH₂)ₛ-(4-to 7-membered saturated heterocyclyl), hydroxyC₁₋₆ alkyl optionally substituted with one or more halo, -C(=O)OC₁₋₆ alkyl, hydroxy, C₁₋₆ alkoxy, haloC₁₋₆ alkyl, - (CH₂)ₙ-O-C₁₋₆ alkyl, - C (=O)- (CH₂)ₙ-C₁₋₆ alkoxy, -C (=O)-C₁₋₆ alkyl, - (CH₂)ₛ-CN, C₁₋₆ alkyl-N (H) _{2-q}(C₁₋₆ alkyl) _{q}, -N (H) _{2-q}(C₁₋₆ alkyl) _{q}, -C(=O)-N(H)_{2-q}(C₁₋₆ alkyl)_{q}, -(CH₂)ₛ-NH-SO₂-N(H)_{2-q}(C₁₋₆ alkyl)_{q}, -(CH₂)ₛ-N(C₁₋₄ alkyl) -SO₂-N(H) _{2-q}(C₁₋₆ alkyl)_{q} and -(CH₂)ₛ-O-C(=O)-C₁₋₄ alkyl-N(H)_{2-q}(C₁₋₆ alkyl) _{q}, and when attached to nitrogen, carbon, silicon or phosphorus atom, R^{x} and R^{y}, together with the atom(s) to which they are attached, may join to form a 3- to 7-membered ring optionally containing one or two additional heteroatoms selected from O, N, S and oxidized forms of N and S;
   Y and W are each independently selected from a bond, -(CR^{x}R^{y})ₘ-, -C(=CR^{x})-, -C(=O)-, -NR^{x}, -C(=O)NR^{x}-, -NR^{×}C(=O)-, -(CR^{x}R^{y})_{q}-O-, - O- (CR^{x}R^{y})_{q}-, -S (O)₂-NH, NH-S(O)₂- and -S(O)_{q}-;
   R₁₀ is selected from hydrogen, C₁₋₆ alkyl, C₂₋₆ alkenyl, C₂₋₆ alkynyl, -C (=O) NH_{(2-q)} (C₁₋₆ alkyl) _{q}, - (CH₂)ₛ-(3- to 12-membered heterocyclyl), - (CH₂)ₛ-C₃₋₁₂ carbocyclyl, -C (=O) - (3- to 12-membered heterocyclyl), and -C(=O)-C₃₋₁₂ carbocyclyl, wherein the C₁₋₆ alkyl, C₂₋₆ alkenyl, C₂₋₆ alkynyl, heterocyclyl and carbocyclyl groups are optionally substituted with one or more R^{b} groups;
   s independently represents an integer of 0 to 4;
   n independently represents an integer of 1 to 4;
   q independently represents an integer of 0 to 2;
   m represents an integer of 1 to 2, and
   X is bonded to L at any one of L₁ to L₃ and L₃'.

The embodiments of each group in X in the above Formula (A) are as follows.

In one embodiment, R₁, and L₁ not used for bonding to L, are each independently selected from hydrogen, C₁₋₆ alkyl, C₂₋₆ alkenyl, C₂₋₆ alkynyl, -C(=O)NH_{(2-q)}(C₁₋₆ alkyl) q, -(CH₂)ₛ-(3- to 7-membered heterocyclyl), -(CH₂)ₛ-C₃₋₇ carbocyclyl, -C(=O)-(3-to 7-membered heterocyclyl), and -C(=O)-C₃₋₇ carbocyclyl, or R₁, and L₁ not used for bonding to L, together with the carbon atom to which they are attached, can join to form a 3- to 6-membered saturated carbocyclyl or 4- to 6-membered heterocyclyl group, wherein the C₁₋₆ alkyl, C₂₋₆ alkenyl, C₂₋₆ alkynyl, heterocyclyl and carbocyclyl groups are optionally substituted with one or more R^{b} groups,
each R^{b} is independently selected from halogen, C₁₋₄ alkyl, C₃₋₄ alkynyl, -(CH₂)_{q}-C₃₋₆ cycloalkyl, -(CH₂)_{q}-phenyl, -(CH₂)_{q}-(5- to 6-membered heteroaryl), -(CH₂)_{q}-O-C₁₋₄ alkyl, haloC₁₋₄ alkyl, haloC₁₋₄ alkoxy, hydroxy, hydroxyC₁₋₄ alkyl, =O, -S(O)_{q}-C₁₋₄ alkyl, -C(=O)C₁₋₄ alkyl, -(CH₂)_{q}-C(=O)NR^{x}R^{y}, -(CH₂)_{q}-NR^{x}C(=O)C₁₋₄ alkyl, -(CH₂)ₛ-NR^{×}R^{y}, -NR^{×}-(CH₂)_{q}-C₁₋₄ alkyl, -(CH₂)_{q}-NR^{x}- (CH₂)_{q}-SO₂-C₁₋₄ alkyl, and -(CH₂)_{q}-SO₂NR^{x}R^{y} groups, wherein the cycloalkyl, phenyl and heteroaryl groups are optionally substituted with one or more R^{x} groups, and
R^{x} and R^{y} are each independently selected from halogen, hydrogen, C₁₋₄ alkyl, and C₁₋₄ alkoxy, and when attached to nitrogen or carbon, R^{x} and R^{y}, together with the atom(s) to which they are attached, may join to form a 3- to 7-membered ring optionally containing one additional heteroatom selected from O and N.

In one embodiment, R₁, and L₁ not used for bonding to L, are each independently selected from hydrogen, C₁₋₆ alkyl, C₂₋₆ alkenyl, C₂₋₆ alkynyl, -C(=O)NH_{(2-q)}(C₁₋₆ alkyl)_{q}, -(CH₂)ₛ-(3- to 7-membered heterocyclyl), -(CH₂)ₛ-C₃₋₇ carbocyclyl, -C(=O)-(3-to 7-membered heterocyclyl), and -C(=O)-C₃₋₇ carbocyclyl, wherein the C₁₋₆ alkyl, C₂₋₆ alkenyl, C₂₋₆ alkynyl, heterocyclyl and carbocyclyl groups are optionally substituted with one or more R^{b} groups,
each R^{b} is independently selected from fluorine, methyl, C₃₋₆ cycloalkyl, phenyl, 5- to 6-membered heteroaryl, -O-C₁₋₂ alkyl, fluoromethyl, fluoromethoxy, hydroxy, =O, -S(O)₂-C₁₋₄ alkyl, - C(=O)C₁₋₄ alkyl, -C(=O)NR^{x}R^{y}, -NR^{×}C(=O)C₁₋₄ alkyl, and -NR^{x}R^{y} groups, wherein the cycloalkyl, phenyl and heteroaryl groups are optionally substituted with one or more R^{x} groups, and R^{x} and R^{y} are each independently selected from chlorine, fluorine, hydrogen, C₁₋₄ alkyl, and C₁₋₄ alkoxy, and when attached to nitrogen or carbon, R^{x} and R^{y}, together with the atom(s) to which they are attached, may join to form a 3- to 7-membered ring optionally containing one additional heteroatom selected from O and N.

In one embodiment, R₁, and L₁ not used for bonding to L, are each independently hydrogen, or C₁₋₆ alkyl (preferably methyl), wherein the C₁₋₆ alkyl is optionally substituted with one or more R^{b} groups.

In one embodiment, R₁, and L₁ not used for bonding to L, are each independently hydrogen, or C₁₋₆ alkyl (preferably methyl), wherein the C₁₋₆ alkyl is optionally substituted with one or more -O-C₁₋₂ alkyl (preferably methoxy).

In one embodiment, one of R₁, and L₁ not used for bonding to L, is hydrogen, and the other is C₁₋₆ alkyl (preferably methyl) optionally substituted with one or more -O-C₁₋₂ alkyl (preferably methoxy).

In one embodiment, R₂ₐ and R_{2b} are each independently selected from hydrogen, C₁₋₃ alkyl, C₂₋₃ alkenyl and C₂₋₃ alkynyl, or R₂ₐ and R_{2b}, together with the carbon atom to which they are attached, can join to form a 3- to 4-membered saturated carbocyclyl group.

In one embodiment, R₂ₐ and R_{2b} are hydrogen.

In one embodiment, R³ is C₁₋₄ alkyl (preferably methyl) optionally substituted with one or more R^{a} group.

In one embodiment, R³ is C₁₋₄ alkyl (preferably methyl) optionally substituted with one or more halogen (preferably fluorine).

In one embodiment, R³ is methyl optionally substituted with one or more fluorine.

In one embodiment, R₄ is hydrogen.

In one embodiment, R₅ₐ and R_{5b} are each independently selected from hydrogen, C₁₋₈ alkyl, C₂₋₈ alkenyl, C₂₋₈ alkynyl, - Y-C₃₋₆ carbocyclyl, -W-(3- to 6-membered heterocyclyl), - (CR^{x}R^{y})ₛ-O-R^{z}, -O-(CR^{x}R^{y})ₙ-OR^{z}, -(CH₂)ₛ-CN and -C(=O)R^{x}, or R₅ₐ and R_{5b}, together with the carbon atom to which they are attached, can join to form a 3- to 5-membered carbocyclyl or 5- to 6-membered heterocyclyl group, wherein the C₁₋₈ alkyl, C₂₋₈ alkenyl and C₂₋₈ alkynyl groups are optionally substituted with one or more R^{b} groups, and each R^{b} is independently selected from fluorine, C₁₋₄ alkyl, C₃₋₄ alkynyl, -C₃₋₆ cycloalkyl, -O-C₁₋₄ alkyl, fluoroC₁₋₄ alkyl, haloC₁₋₄ alkoxy, hydroxy, hydroxyC₁₋₄ alkyl, -C(=O)NR^{x}R^{y}, -NR^{×}C(=O)C₁₋₄ alkyl, and -NR^{x}R^{y} groups.

In one embodiment, R₅ₐ and R_{5b} are each independently hydrogen or methyl, or R₅ₐ and R_{5b}, together with the carbon atom to which they are attached, can join to form a cyclopropyl group.

In one embodiment, R₅ₐ and R_{5b} are each independently C₁₋₄ alkyl (preferably methyl).

In one embodiment, R₆ is selected from hydrogen, halogen, C₁₋₄ alkyl, C₂₋₅ alkenyl, C₂₋₅ alkynyl, -(CR^{x}R^{y})-C₃₋₇ carbocyclyl, -(CR^{x}R^{y})-(3- to 7-membered heterocyclyl), -(CR^{x}R^{y})-O-R^{z}, -O-(CR^{x}R^{y})ₙ-OR^{z}, - (CH₂)ₛ-CN, -S(O)₂-(CH₂)ₛ-R^{z}, -C(=O)R^{x}, - (CH₂)ₛ-NR^{x}R^{y}, -NR^{x}- (CH₂)ₛ-R^{z}, - (CH₂)ₙ-C₅₋₁₀ aryl, - (CH₂)ₙ-(5- to 10-membered heteroaryl), an optionally fused 5- to 7-membered carbocyclyl group, an optionally fused 5- to 7-membered heterocyclyl group, and 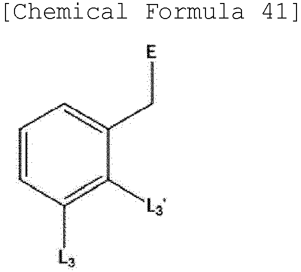
wherein E indicates a bonding point,
L₃ and L₃', together with the carbon atoms to which they are attached, can join to form a 3- to 6-membered carbocyclyl or 4-to 6-membered heterocyclyl group that can be connected to a linker, wherein the C₁₋₄ alkyl, C₂₋₅ alkenyl and C₂₋₅ alkynyl groups, carbocyclyl group, heterocyclyl group, aryl group, heteroaryl group, and 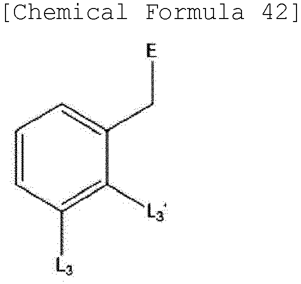
wherein E indicates a bonding point, are optionally substituted with one or more R^{b} groups,
each R^{b} is independently selected from halogen, C₁₋₄ alkyl, C₃₋₄ alkynyl, - (CH₂)_{q}-C₃₋₆ cycloalkyl, - (CH₂)_{q}-phenyl, - (CH₂)_{q}-(5- to 6-membered heteroaryl), - (CH₂)_{q}-O-C₁₋₄ alkyl, haloC₁₋₄ alkyl, haloC₁₋₄ alkoxy, hydroxy, hydroxyC₁₋₄ alkyl, =O, -S (O)_{q}-C₁₋₄ alkyl, -C (=O) C₁₋₄ alkyl, - (CH₂)_{q}-C(=O)NR^{x}R^{y}, - (CH₂)_{q}-NR^{x}C(=O) C₁₋₄ alkyl, - (CH₂)ₛ-NR^{x}R^{y}, -NR^{x}-(CH₂)_{q}-C₁₋₄ alkyl, -(CH₂)_{q}-NR^{x}-(CH₂)_{q}-SO₂-C₁₋₄ alkyl, and - (CH₂)_{q}-SO₂NR^{×}R^{y} groups, wherein the cycloalkyl, phenyl and heteroaryl groups are optionally substituted with one or more R^{x} groups,
the CR^{x}R^{y} is CH₂ or CF₂, and the other R^{×}, R^{y} and R^{z} are each independently selected from hydrogen, C₁₋₄ alkyl, C₃₋₄ alkynyl, fluoroC₁₋₄ alkyl, and C₁₋₄ alkoxy, and
the R^{x} group as a substituent of R^{b} is selected from halogen, hydrogen, C₁₋₄ alkyl, and C₁₋₄ alkoxy.

In one embodiment, R₆ is selected from -(CR^{x}R^{y})-H, - (CR^{x}R^{y})-F, -(CR^{x}R^{y})-C₁₋₂ alkyl, -(CR^{x}R^{y})-O-phenyl, -S(O)₂-CH₂-R^{z}, - C(=O)C₁₋₄ alkyl, -C(=O)-phenyl, -C(=O)-(5-6-membered heteroaryl), -CH₂-phenyl, -CH₂-(5-6-membered heteroaryl), and 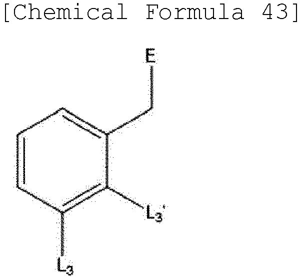
wherein E indicates a bonding point,
L₃ and L₃', together with the carbon atoms to which they are attached, can join to form a 3- to 6-membered carbocyclyl or 4-to 6-membered heterocyclyl group that can be connected to a linker,
wherein the C₁₋₂ alkyl, C₁₋₄ alkyl and phenyl groups, heteroaryl group, and 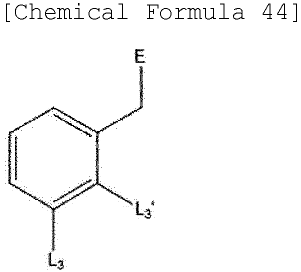
wherein E indicates a bonding point, are optionally substituted with one or more C₁₋₃ alkyl groups, fluorine, chlorine, or -O-C₁₋₃ alkyl groups, and
the CR^{x}R^{y} is CH₂ or CF₂, and the other R^{×}, R^{y} and R^{z} are each independently selected from hydrogen, C₁₋₄ alkyl, C₃₋₄ alkynyl, fluoroC₁₋₄ alkyl, and C₁₋₄ alkoxy.

In one embodiment, R₆ is -CH₂-phenyl, or 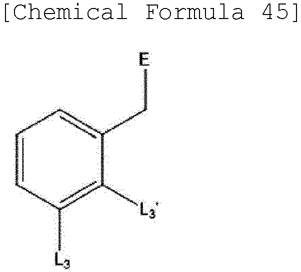
wherein E indicates a bonding point,
each of which is optionally substituted with one or more R^{b} groups.

In one embodiment, R₆ is -CH₂-phenyl, or 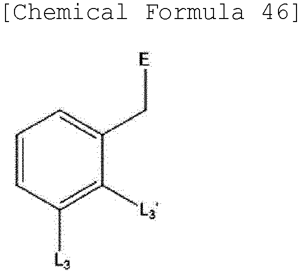
wherein E indicates a bonding point,
each of which is optionally substituted with one or more halogen atoms (preferably a fluorine atom).

In one embodiment, R₇ is selected from hydrogen, halogen, C₁₋₃ alkyl, C₂₋₃ alkenyl, C₂₋₃ alkynyl and -C₃₋₄ carbocyclyl, wherein the C₁₋₃ alkyl, C₂₋₃ alkenyl, C₂₋₃ alkynyl and C₃₋₄ carbocyclyl are optionally substituted with one or more R^{b} groups,
each R^{b} is independently selected from fluorine, -O-C₁₋₄ alkyl, fluoroC₁₋₄ alkoxy, hydroxy, and -NR^{x}R^{y} groups, and R^{x} and R^{y} are each independently selected from hydrogen, and C₁₋₄ alkyl, and when attached to nitrogen, R^{x} and R^{y}, together with the atom(s) to which they are attached, may join to form a 4-to 6-membered ring optionally containing one additional heteroatom selected from O and N.

In one embodiment, R₇ is hydrogen or fluorine.

In one embodiment, R₇ is hydrogen.

In one embodiment, R₈ is hydrogen.

In one embodiment, R₉ is selected from hydrogen, halogen, C₁₋₄ alkyl, C₂₋₄ alkenyl, C₂₋₄ alkynyl, -Y-C₃₋₆ carbocyclyl, -W-(4-to 6-membered heterocyclyl), -(CR^{x}R^{y})ₛ-O-R^{z}, -O-(CR^{x}R^{y})ₙ-OR^{z}, =O, -(CH₂)ₛ-CN, -C(=O)R^{x}, -(CR^{x}R^{y})ₛ-C(=O)NR^{x}R^{y}, -(CH₂)ₛ-NR^{x}C(=O)R^{y}, - (CH₂)ₛ-NR^{x}R^{y}, -(CH₂)ₛ-O-C(=O) -C₁₋₄ alkyl-NR^{x}R^{y}, -(CH₂)ₛ-NR^{x}-(CH₂)ₛ-SO₂-R^{y}, -(CH₂)ₛ-NH-SO₂-NR^{×}R^{y}, -(CH₂)ₛ-SO₂NR^{x}R^{y}, and -Y-C₅₋₁₀ aryl, wherein the C₁₋₄ alkyl, C₂₋₄ alkenyl, C₂₋₄ alkynyl, carbocyclyl and heterocyclyl groups are optionally substituted with one or more R^{b} groups,
each R^{b} is independently selected from fluorine, C₁₋₄ alkyl, C₃₋₄ alkynyl, C₃₋₆ cycloalkyl, phenyl, 5- to 6-membered heteroaryl, - O-C₁₋₄ alkyl, fluoroC₁₋₄ alkyl, fluoroC₁₋₄ alkoxy, hydroxy, hydroxyC₁₋₄ alkyl, =O, -C(=O)C₁₋₄ alkyl, -C(=O)NR^{x}R^{y}, -NR^{X}C(=O)C₁₋₄ alkyl, and -NR^{x}R^{y} groups, wherein the cycloalkyl, phenyl and heteroaryl groups are optionally substituted with one or more R^{x} groups, and
R^{×}, R^{y} and R^{z} are each independently selected from chlorine, fluorine, hydrogen, C₁₋₄ alkyl, and C₁₋₄ alkoxy, and when attached to nitrogen or carbon, R^{x} and R^{y}, together with the atom(s) to which they are attached, may join to form a 3- to 7-membered ring optionally containing one additional heteroatom selected from O and N.

In one embodiment, R₉ is selected from hydrogen, C₁₋₄ alkyl, C₂₋₄ alkenyl, C₂₋₄ alkynyl, -CH₂-C₃₋₆ carbocyclyl, -CH₂-(4-to 6-membered heterocyclyl), -CH₂-O-R^{z}-C(=O)R^{X}, and -CH₂-NR^{x}R^{y}, wherein the C₁₋₄ alkyl, C₂₋₄ alkenyl, C₂₋₄ alkynyl, carbocyclyl and heterocyclyl groups are optionally substituted with one or more R^{b} groups,
each R^{b} is independently selected from fluorine, C₁₋₄ alkyl, C₃₋₄ alkynyl, phenyl, 5- to 6-membered heteroaryl, -O-C₁₋₄ alkyl, fluoroC₁₋₄ alkyl, fluoroC₁₋₄ alkoxy, hydroxy, and -NR^{x}R^{y} groups, wherein the phenyl and heteroaryl groups are optionally substituted with one or more R^{x} groups, and
R^{×}, R^{y} and R^{z} are each independently selected from chlorine, fluorine, hydrogen, C₁₋₄ alkyl, and C₁₋₄ alkoxy, and when attached to nitrogen or carbon, R^{x} and R^{y}, together with the atom(s) to which they are attached, may join to form a 3- to 7-membered ring optionally containing one additional heteroatom selected from O and N.

In one embodiment, R₉ is hydrogen.

In one embodiment, R₁₀ is hydrogen, or methyl.

In one embodiment, each R^{b} is independently selected from halogen, C₁₋₆ alkyl, C₂₋₆ alkenyl, C₂₋₆ alkynyl, -(CH₂)ₛ-C₃₋₈ cycloalkyl, -(CH₂)ₛ-C₃₋₈ cycloalkenyl, -(CH₂)ₛ-phenyl, -(CH₂)ₛ-(4-to 7-membered saturated heterocyclyl), -(CR^{x}R^{y})ₛ-O-R^{z}, -O-(CR^{x}R^{y})ₙ-OR^{z}, haloC₁₋₆ alkyl, haloC₁₋₆ alkoxy, hydroxyC₁₋₆ alkyl, =O, =S, nitro, Si(Rx)₄, -(CH₂)ₛ-CN, -S(O)_{q}-R^{x}, -C(=O)R^{x}, - (CR^{x}R^{y})ₛ-C(=O) OR^{z}, - (CR^{x}R^{y})ₛ-O-C(=O) -R^{z}, - (CR^{x}R^{y})ₛ-C(=O)NR^{x}R^{y}, - (CH₂)ₛ-NR^{x}C(=O)R^{y}, - (CH₂)ₛ-OC(=O)NR^{x}R^{y}, - (CH₂)ₛ-NR^{x}C(=O) OR^{y}-(CH₂)ₛ-NR^{x}R^{y}, -NR^{×}-(CH₂)ₛ-R^{z}, - (CH₂)ₛ-O-C(=O)-C₁₋₄ alkyl-NR^{x}R^{y}, - (CH₂)ₛ-NR^{×}-(CH₂)ₙ-O-C(=O)-R^{z}, - (CH₂)ₛ-NR^{x}-(CH₂)ₛ-SO₂-R^{y}, - (CH₂)ₛ-NH-SO₂-NR^{x}R^{y} and - (CH₂)ₛ-SO₂NR^{x}R^{y} groups and -P(=O)(R^{x})₂, wherein the C₁₋₆ alkyl, C₂₋₆ alkenyl, C₂₋₆ alkynyl, C₃₋₈ cycloalkyl, C₃₋₈ cycloalkenyl and heterocyclyl groups are optionally substituted with one or more R^{x} groups.

In one embodiment, R^{×}, R^{y} and R^{z} are each independently selected from halogen, hydrogen, C₁₋₆ alkyl, C₂₋₆ alkenyl, C₂₋₆ alkynyl, - (CH₂)ₛ-C₃₋₈ cycloalkyl, - (CH₂)ₛ-C₃₋₈ cycloalkenyl, - (CH₂)ₛ-phenyl, -(CH₂)ₛ-(4- to 7-membered saturated heterocyclyl), hydroxyC₁₋₆ alkyl optionally substituted with one or more halo, -C (=O) OC₁₋₆ alkyl, hydroxy, C₁₋₆ alkoxy, haloC₁₋₆ alkyl, - (CH₂)ₙ-O-C₁₋₆ alkyl, -C (=O) - (CH₂)ₙ-C₁₋₆ alkoxy, -C(=O)-C₁₋₆ alkyl, - (CH₂)ₛ-CN, C₁₋₆ alkyl-N(H) _{2-q}(C₁₋₆ alkyl) _{q}, -N(H)_{2-q}(C₁-₆ alkyl) _{q}, -C (=O) -N (H) _{2-q}(C₁₋₆ alkyl) _{q}, - (CH₂)ₛ-NH-SO₂-N(H) _{2-q}(C₁₋₆ alkyl) _{q}, - (CH₂)ₛ-N(C₁₋₄ alkyl) -SO₂-N(H) _{2-q}(C₁₋₆ alkyl) _{q} and - (CH₂)ₛ-O-C (=O) -C₁₋₄ alkyl-N (H) _{2-q}(C₁₋₆ alkyl) _{q}, and when attached to nitrogen, carbon, silicon or phosphorus atom, R^{x} and R^{y}, together with the atom(s) to which they are attached, may join to form a 3- to 7-membered ring optionally containing one or two additional heteroatoms selected from O, N, S and oxidized forms of N and S.

In one embodiment, Y and W are each independently a bond, - (CR^{×}R^{y})ₘ-, -C (=CR^{x})-, -C(=O)-, -NR^{x}, -C (=O) NR^{×}-, -NR^{×}C(=O) -, - (CR^{x}R^{y})_{q}-O-, -O- (CR^{x}R^{y})_{q}-, -S (O)₂-NH, NH-S(O)₂- or -S (O)_{q}-.

One embodiment of X in the above Formula (A) is as follows:
A is N or CR₈ (preferably N),
B is N, CR₉, or CL₂ (preferably CR₉, or CL₂),
Q is N,
R₁, and L₁ not used for bonding to L, are each independently hydrogen, or C₁₋₆ alkyl (preferably methyl), wherein the C₁₋₆ alkyl is optionally substituted with one or more -O-C₁₋₂ alkyl (preferably methoxy),
R₂ₐ and R_{2b} are hydrogen,
R³ is C₁₋₄ alkyl (preferably methyl),
R₄ is hydrogen,
R₅ₐ and R_{5b} are each independently C₁₋₄ alkyl (preferably methyl),
R₆ is -CH₂-phenyl, or 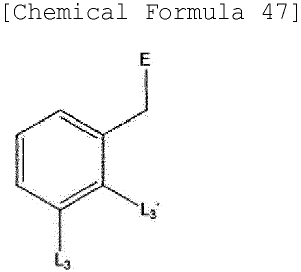
wherein E indicates a bonding point,
each of which is optionally substituted with one or more halogen atoms (preferably a fluorine atom),
R₇ is hydrogen,
R₉ is hydrogen, and
X is bonded to L at any one of L₁ to L₃ and L₃'.

Examples of the case where X is bonded to L at any one of L₁ to L₃ and L₃' include the following.

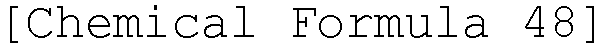

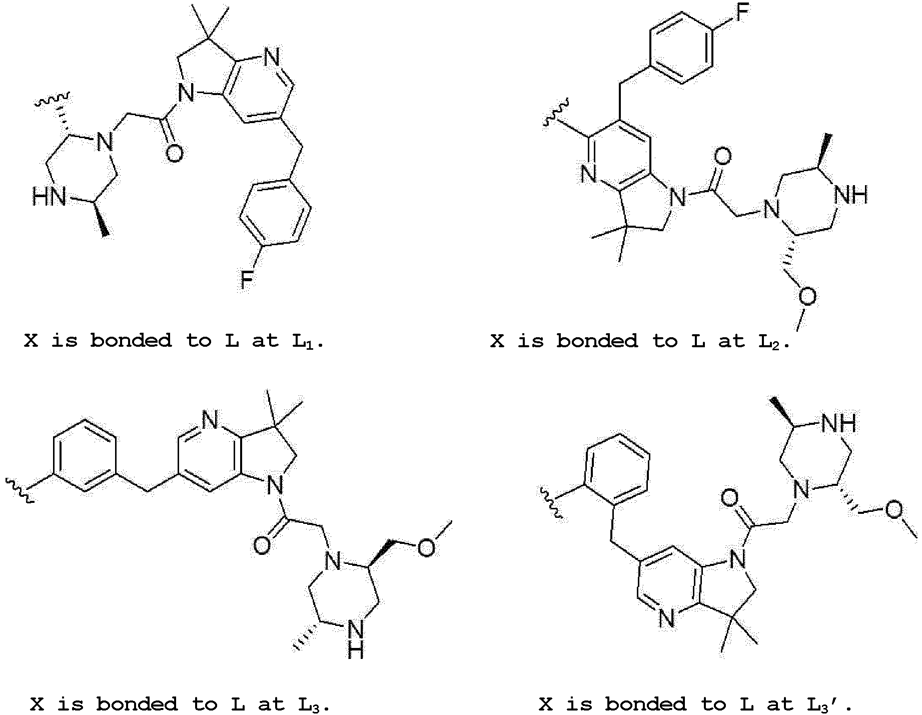

In one embodiment, the compound of Formula (A) includes the following compounds:

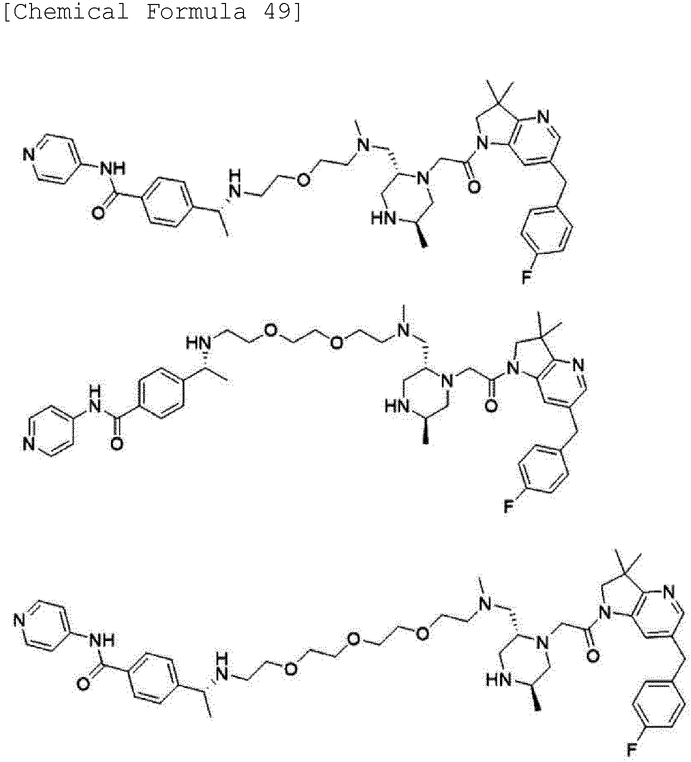

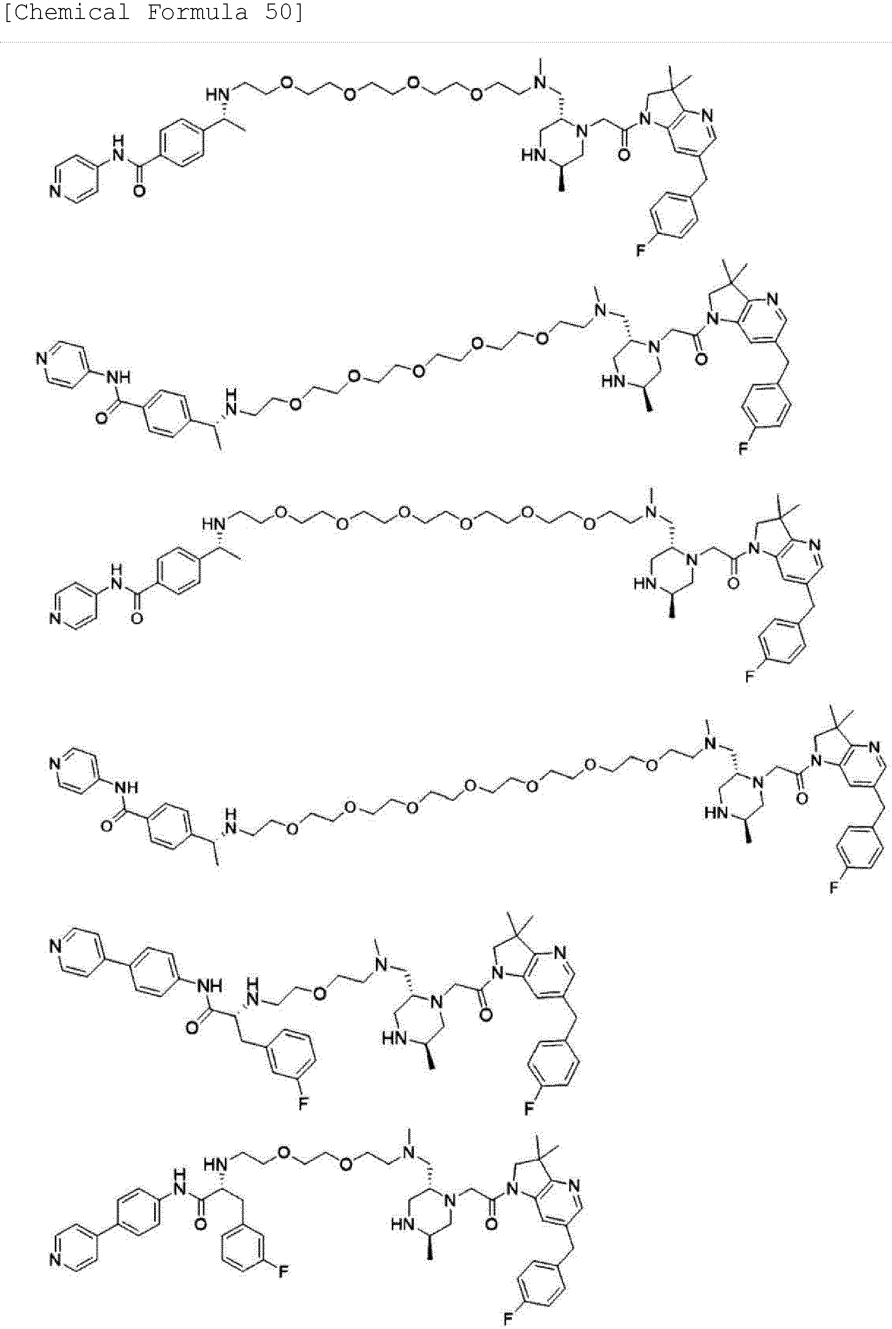

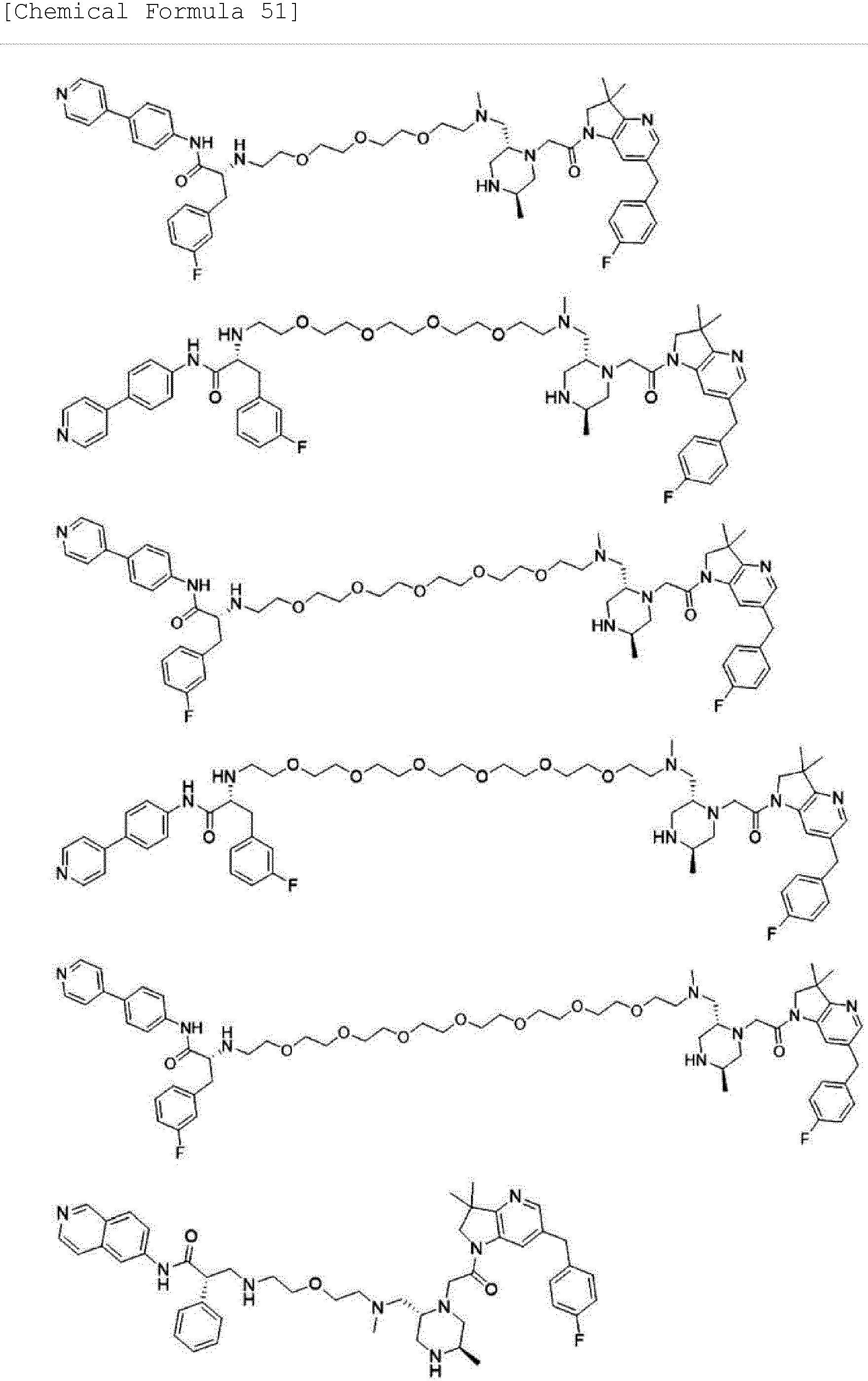

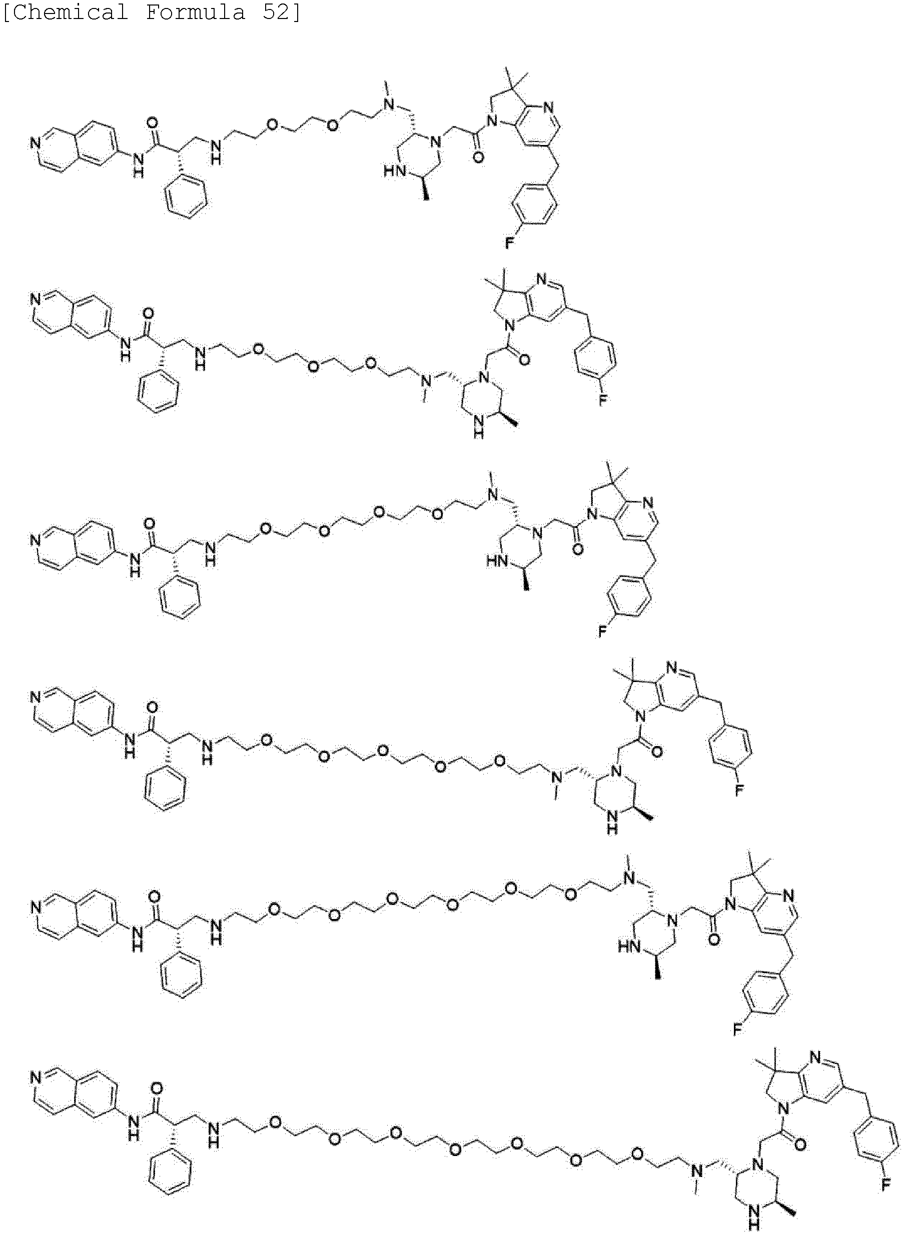

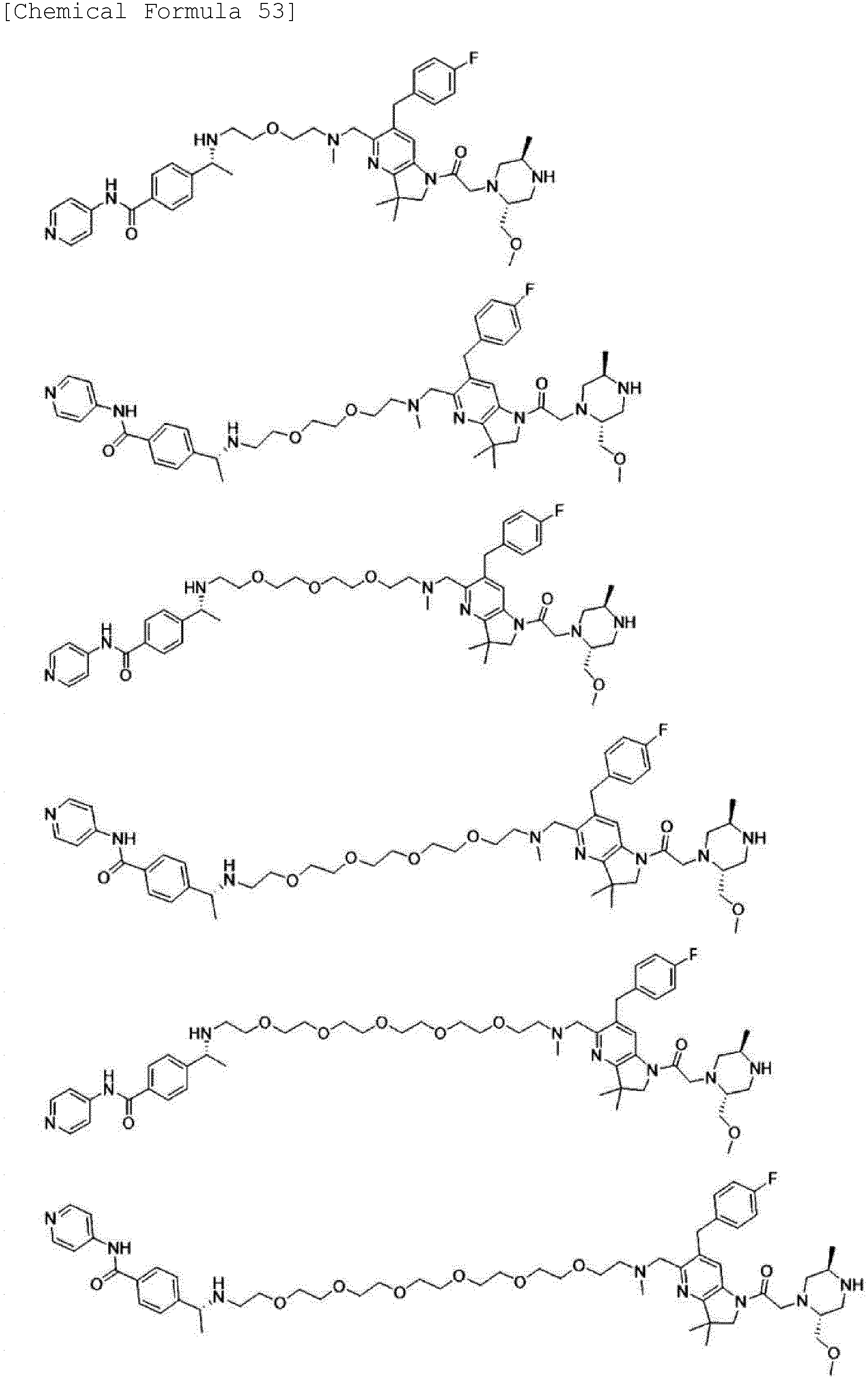

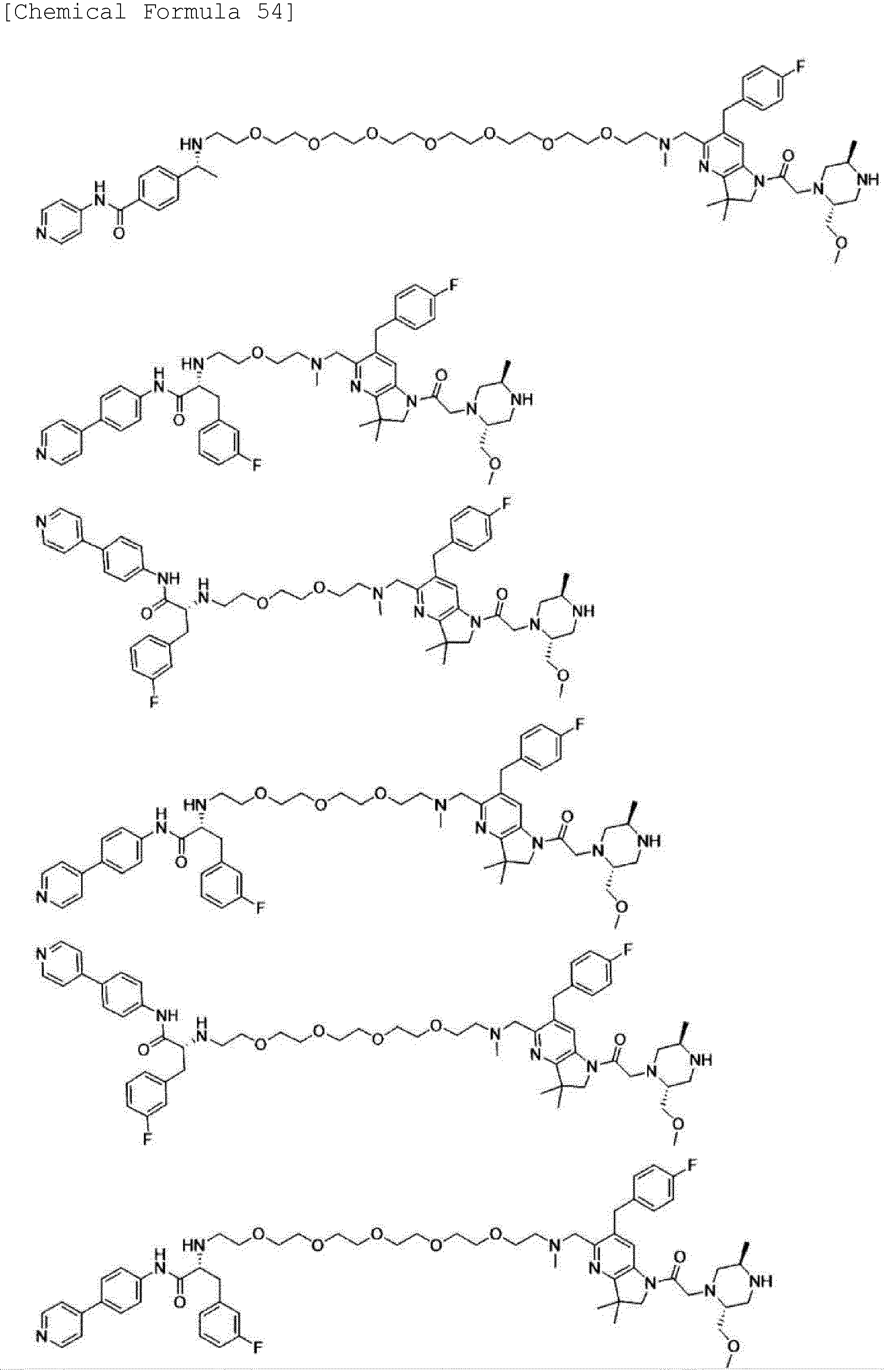

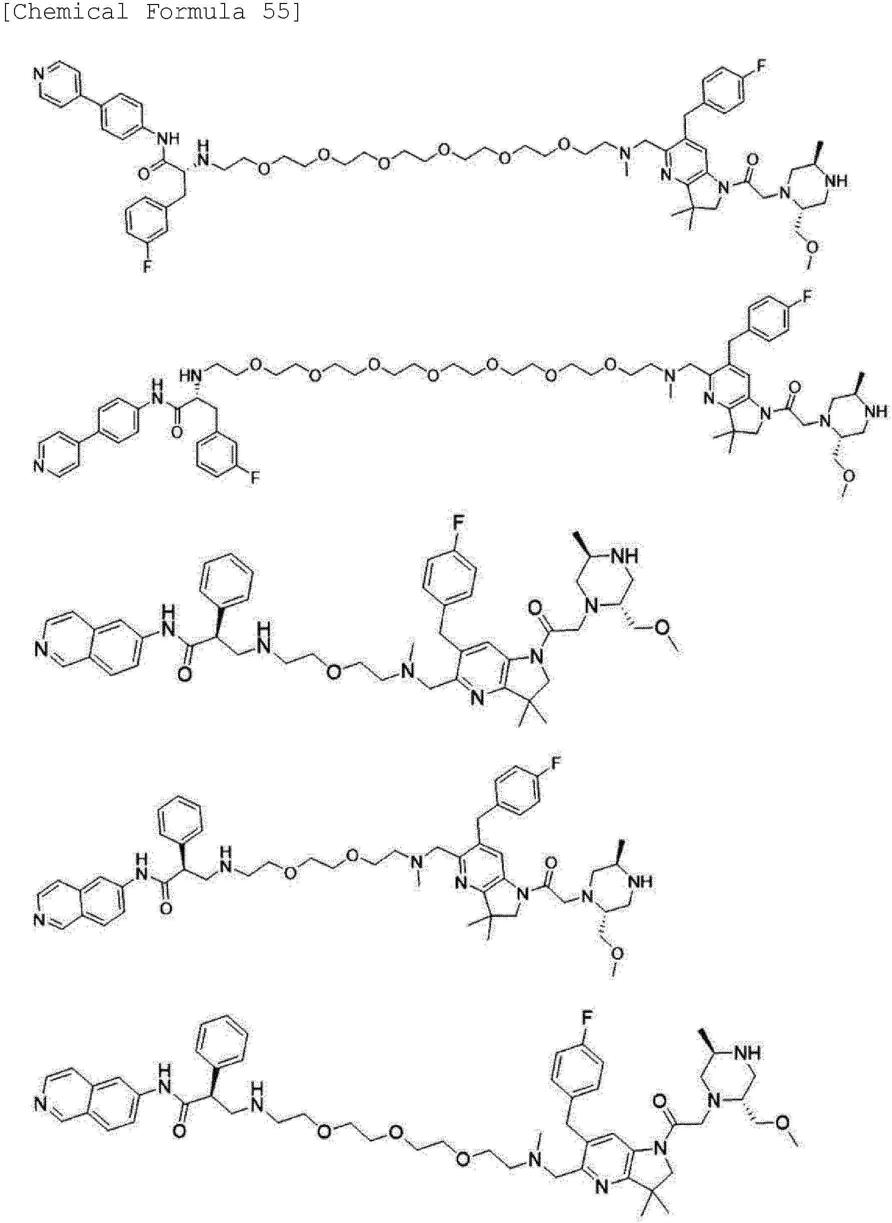

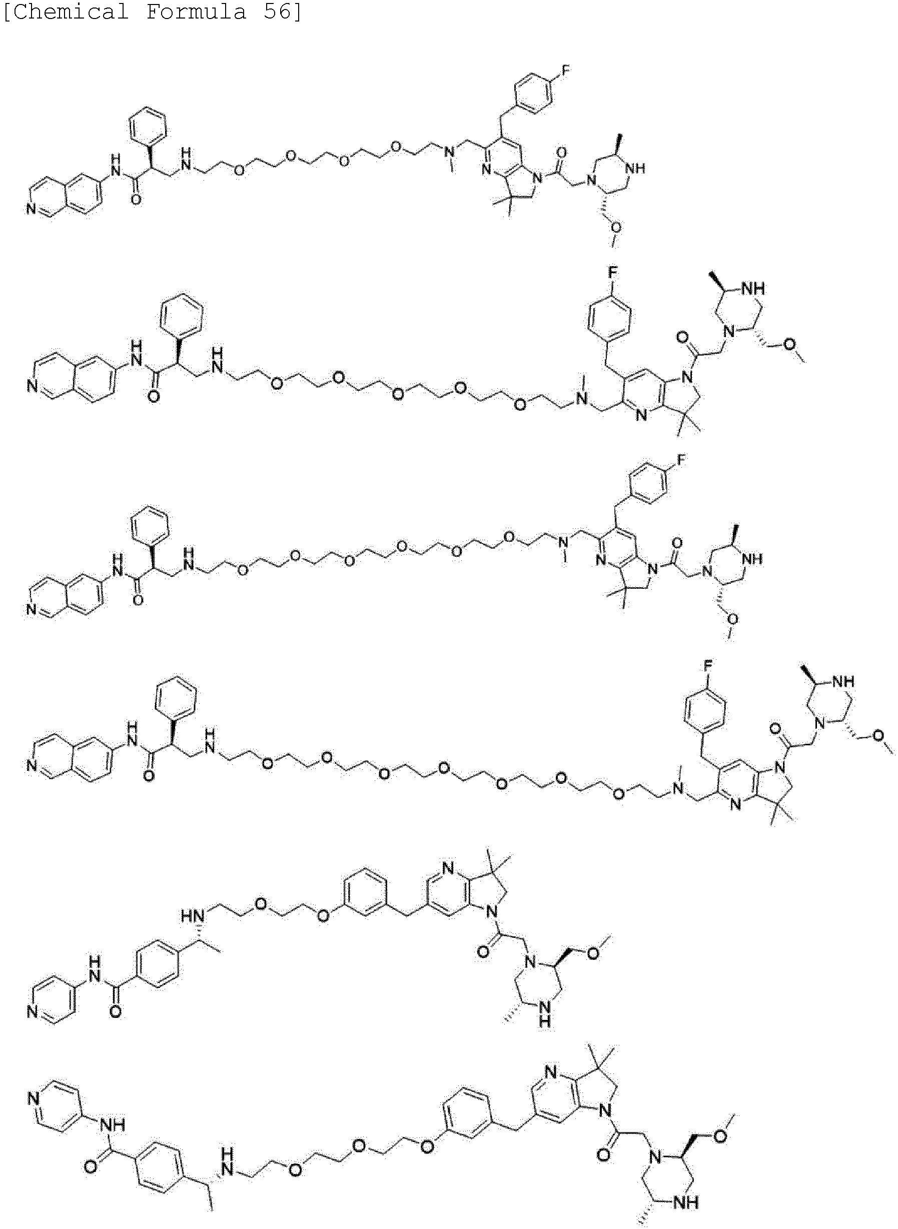

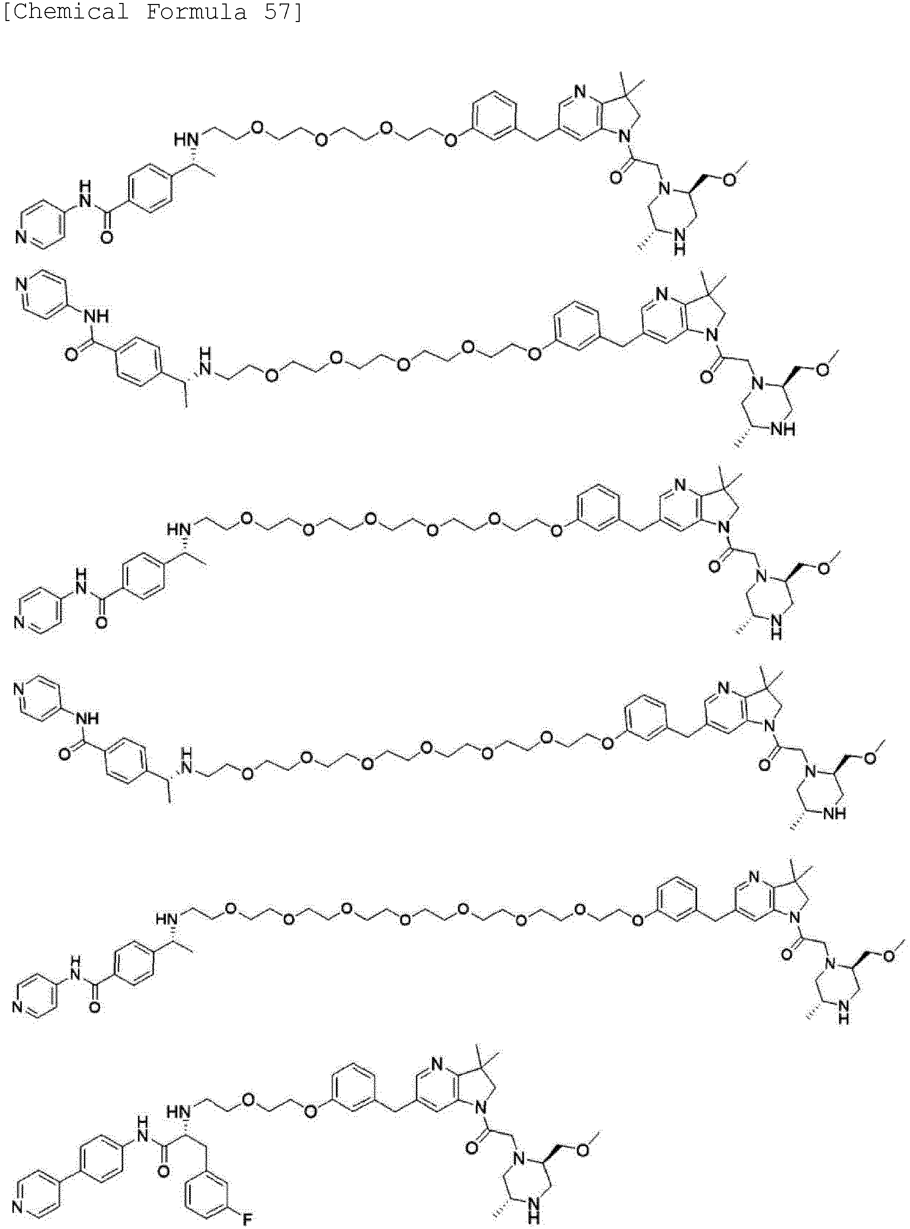

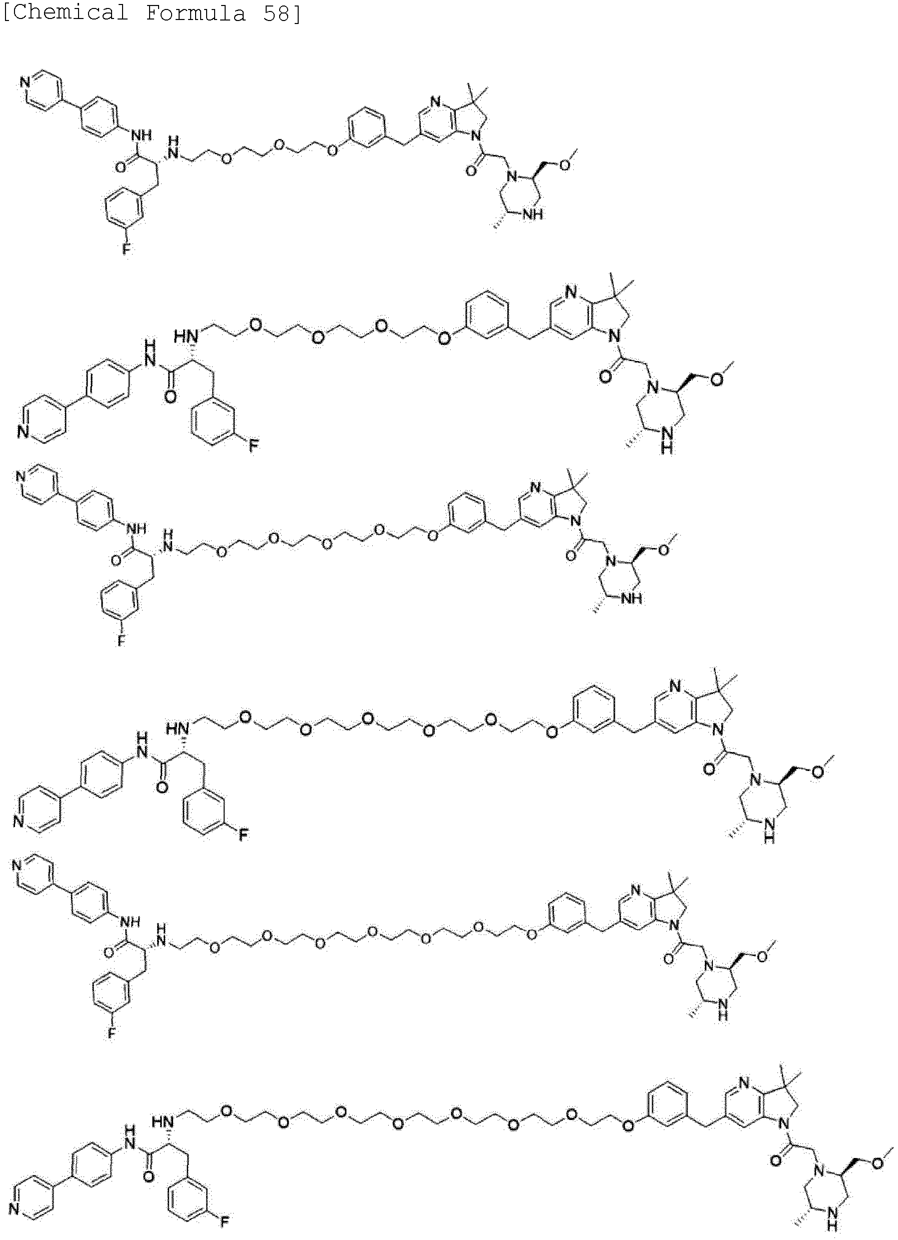

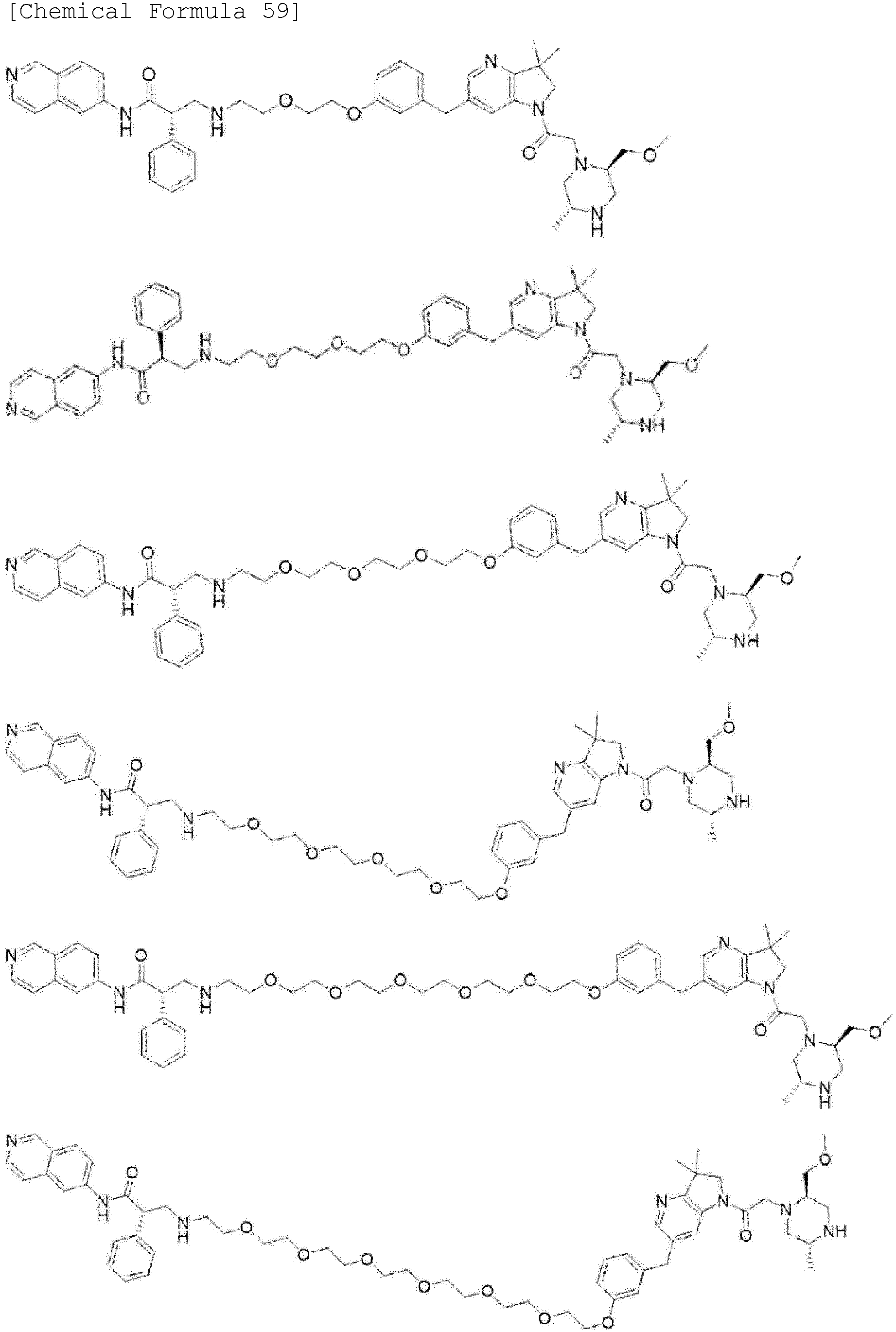

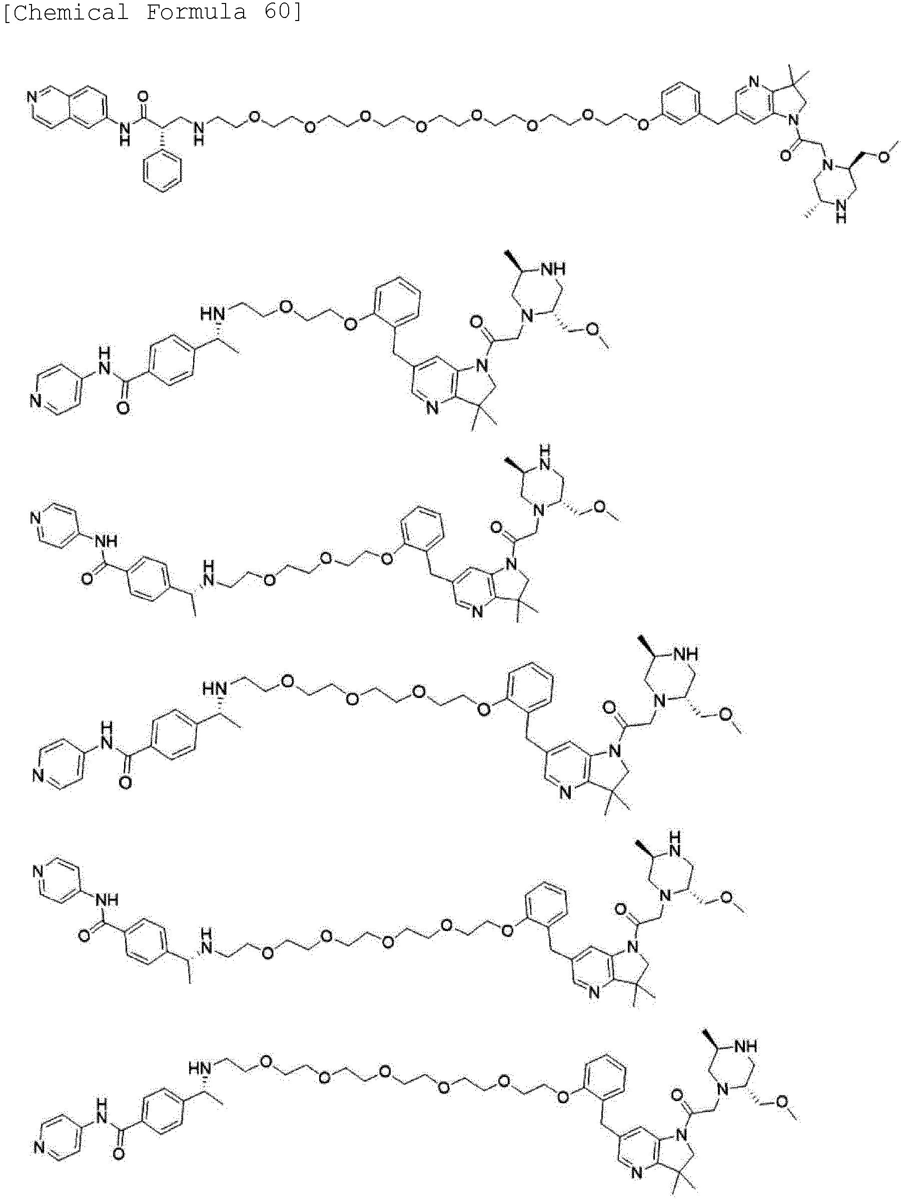

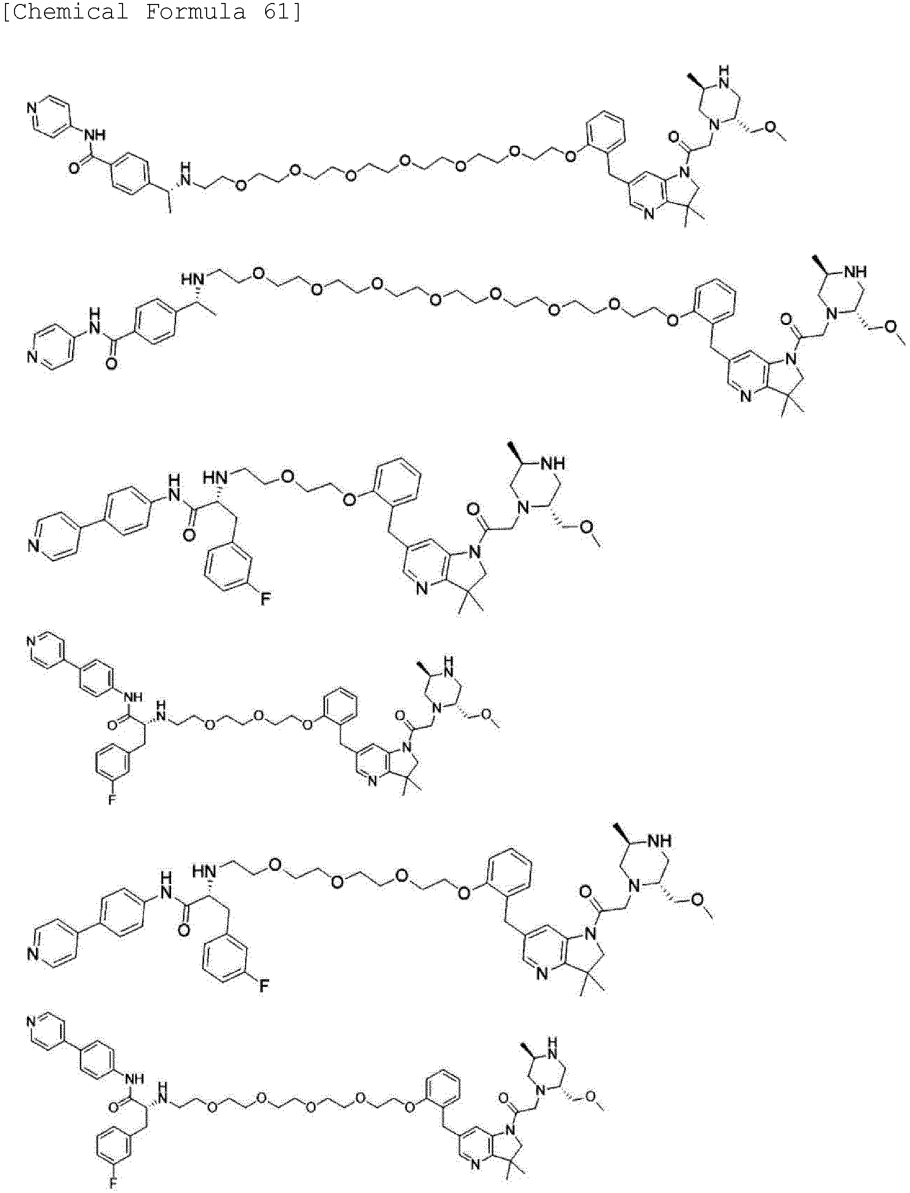

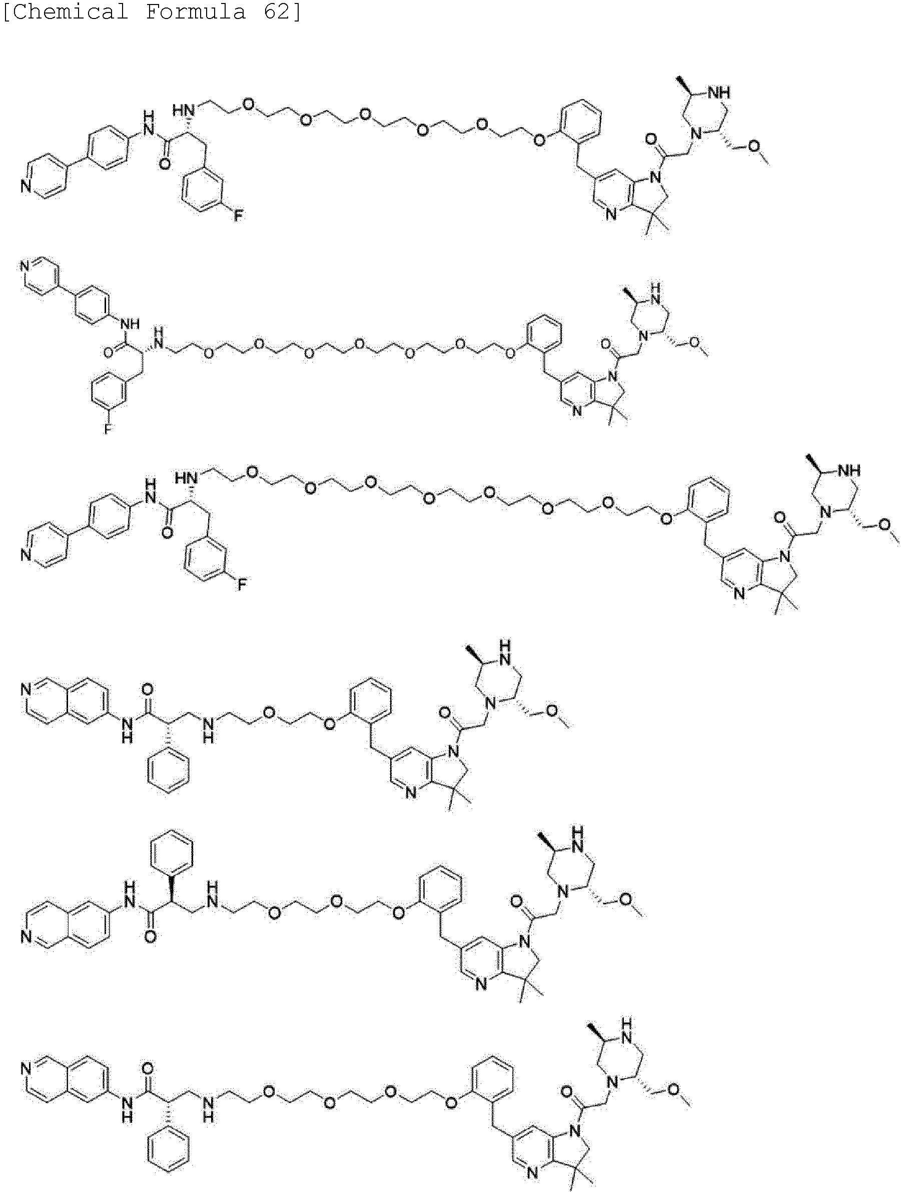

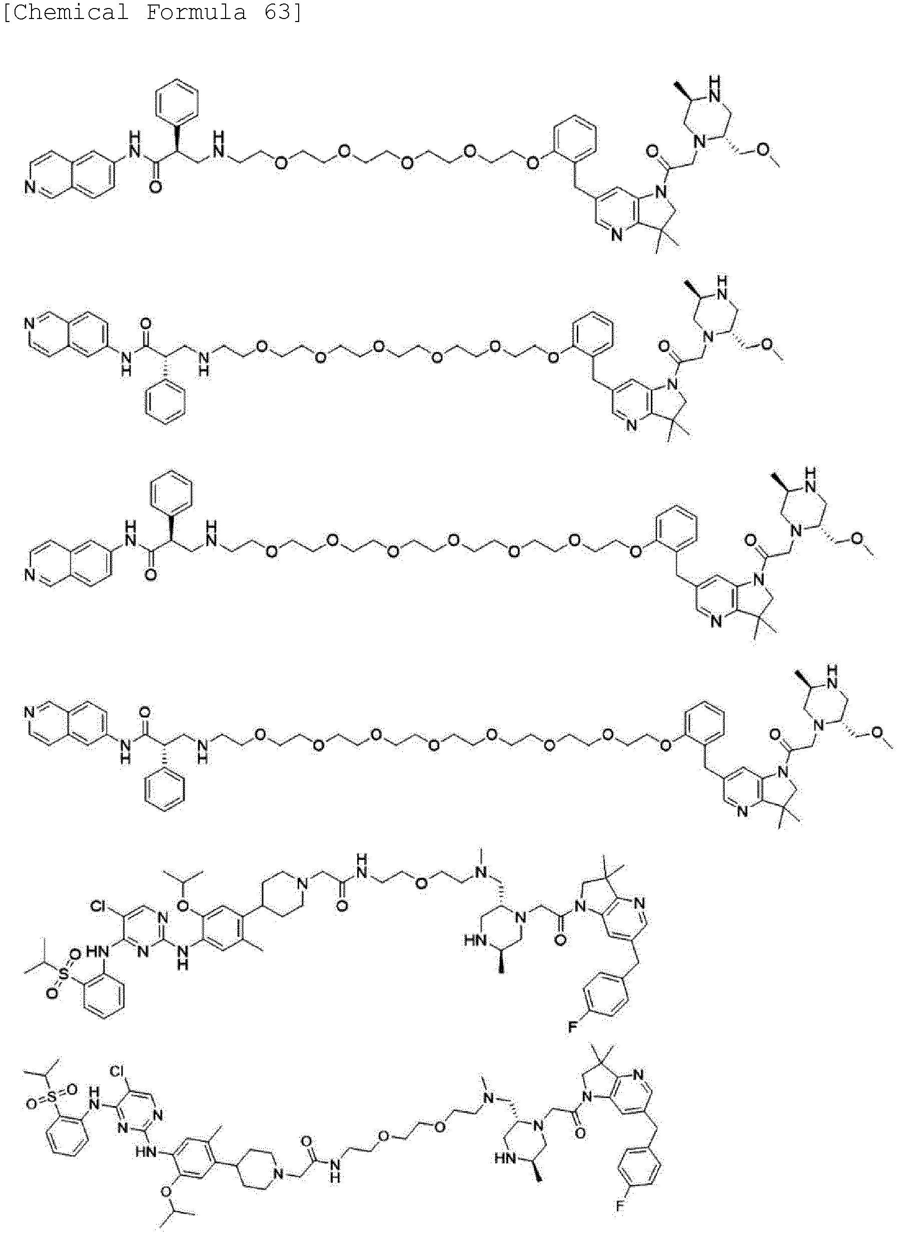

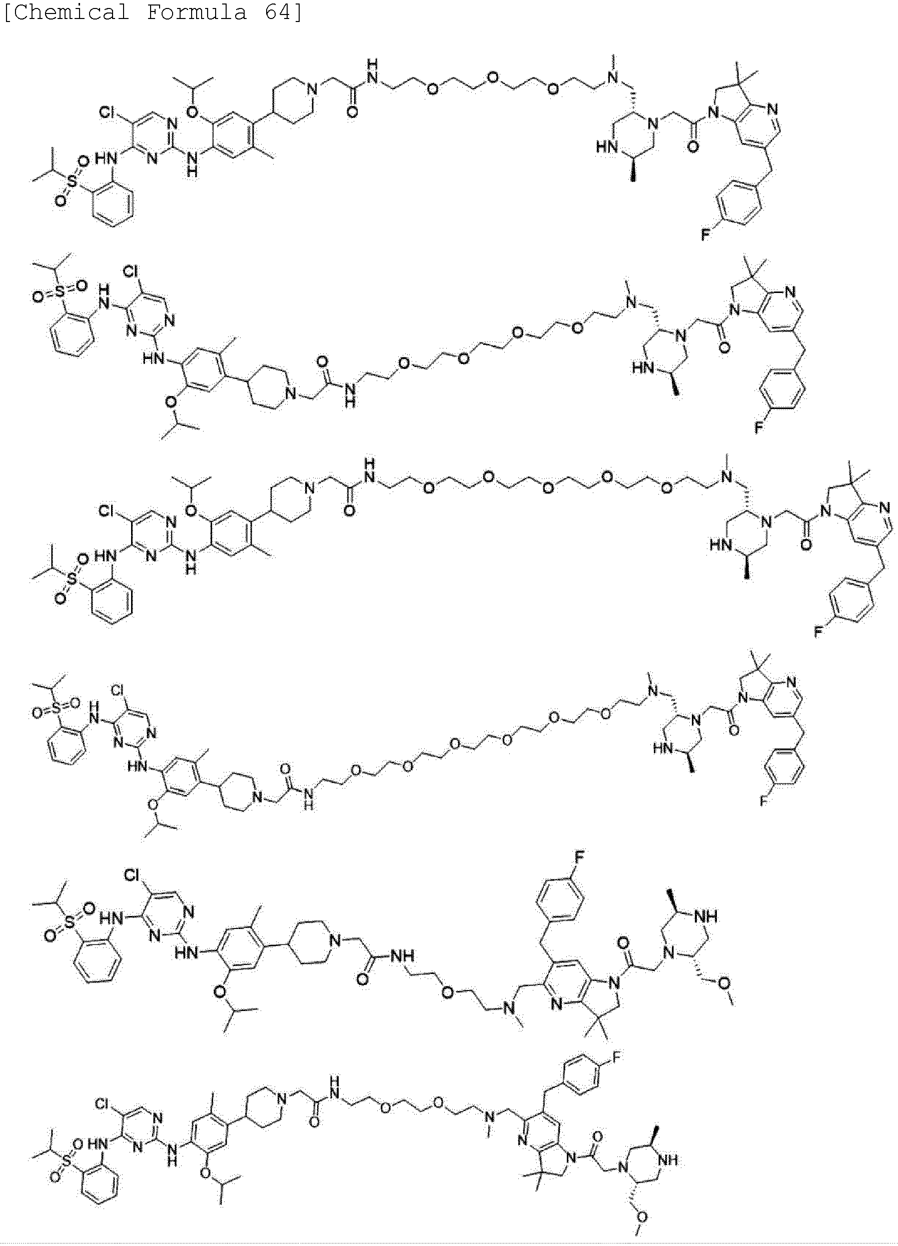

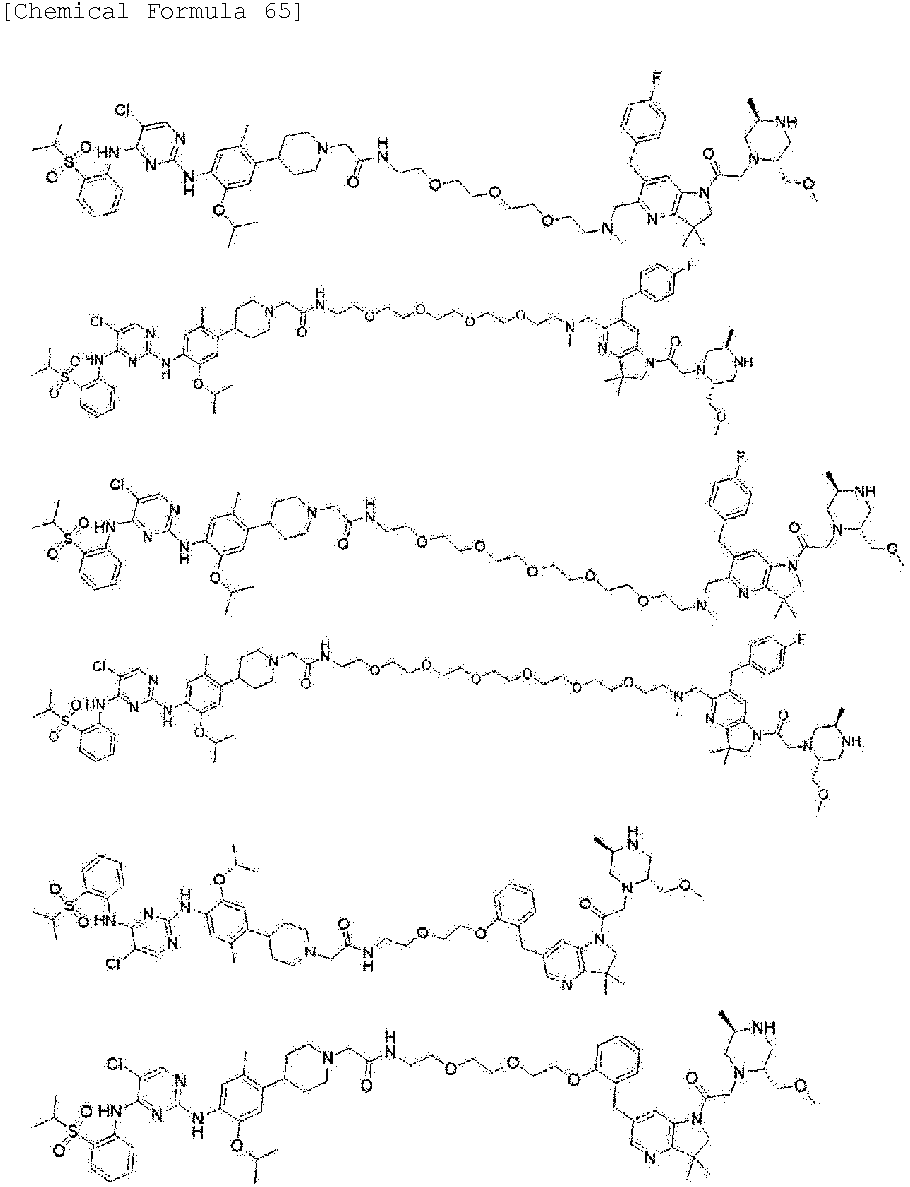

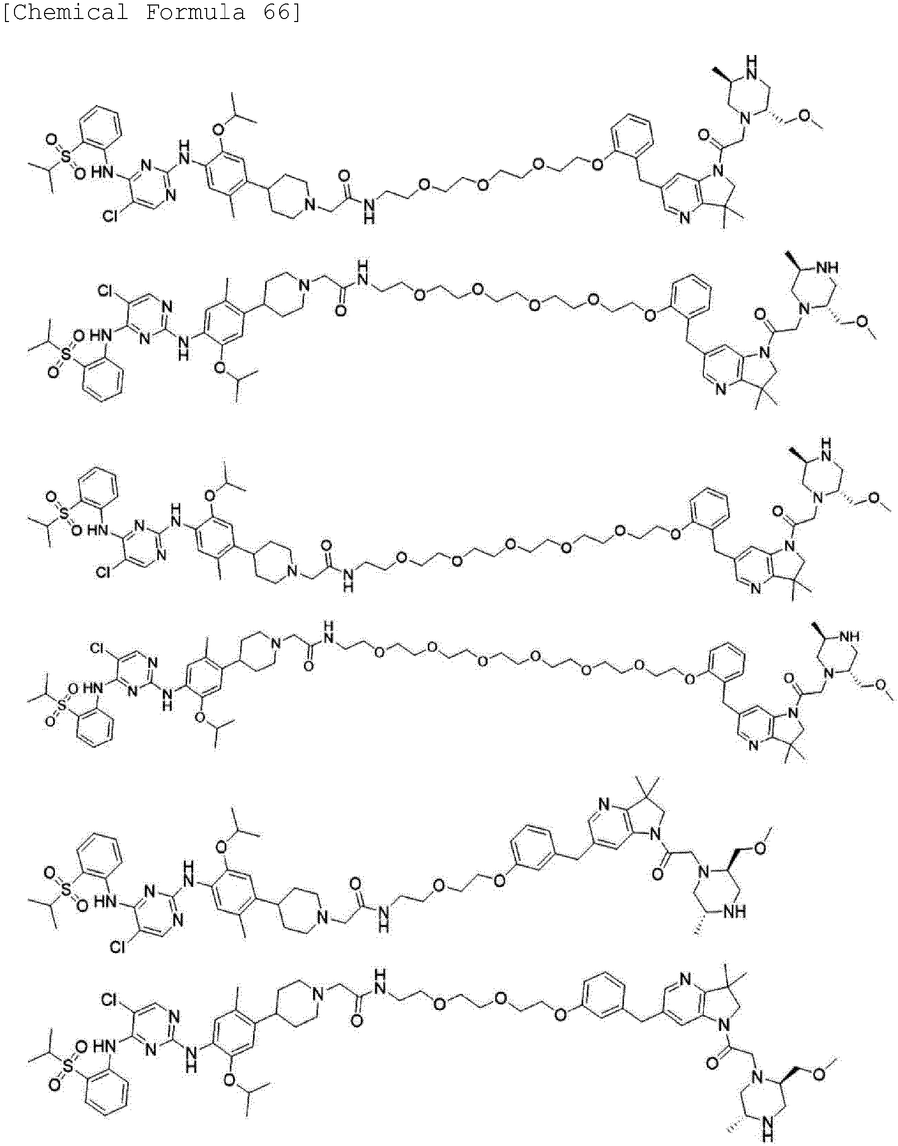

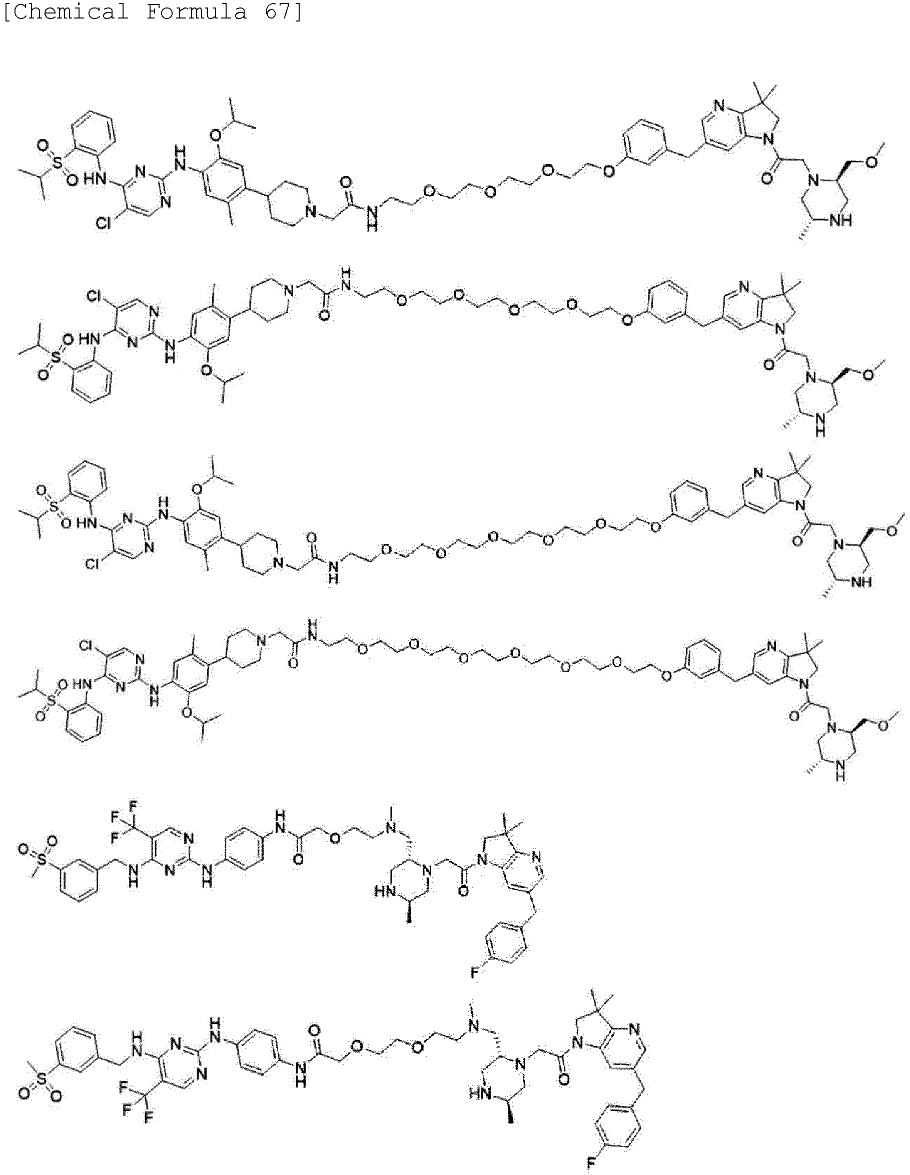

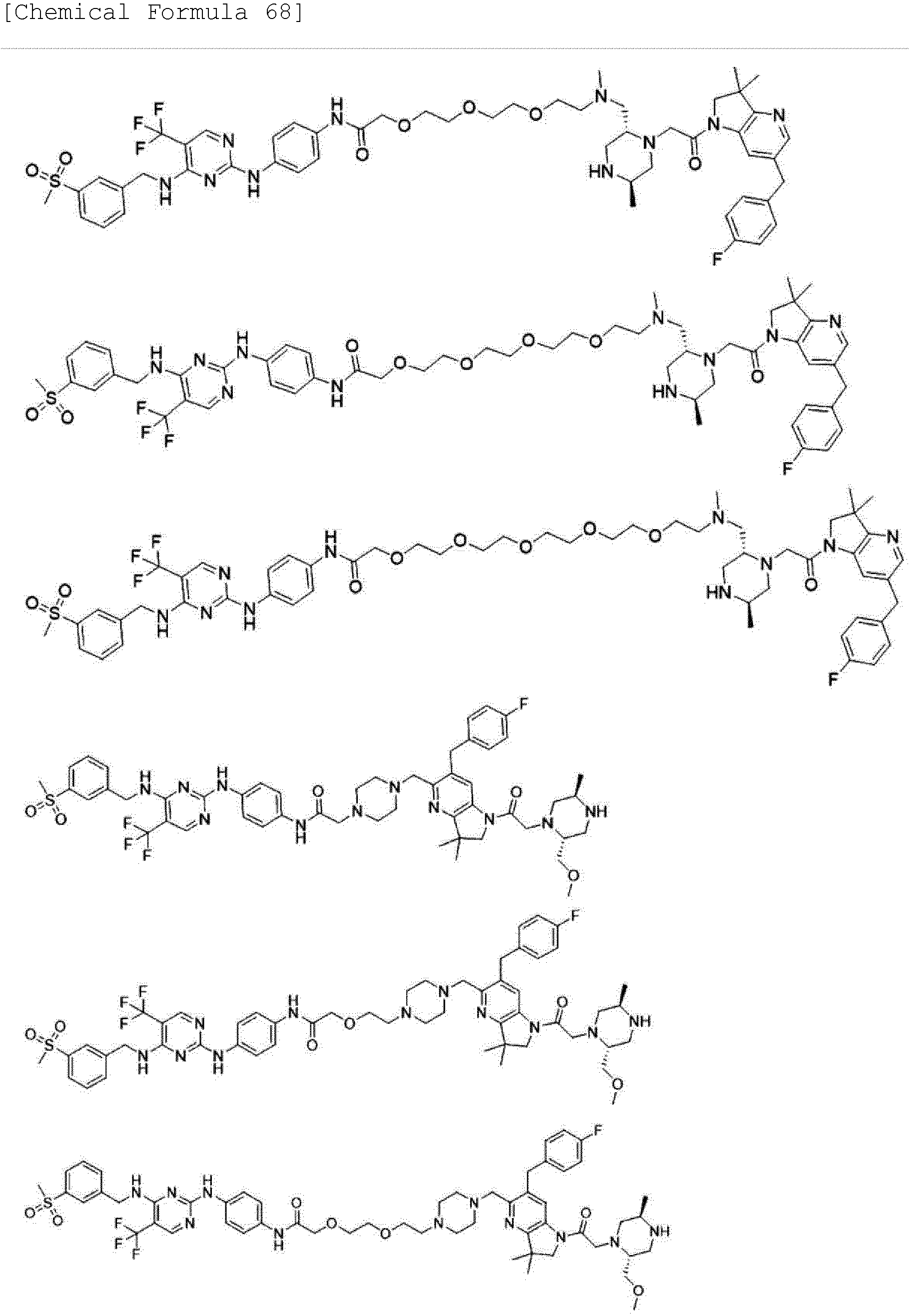

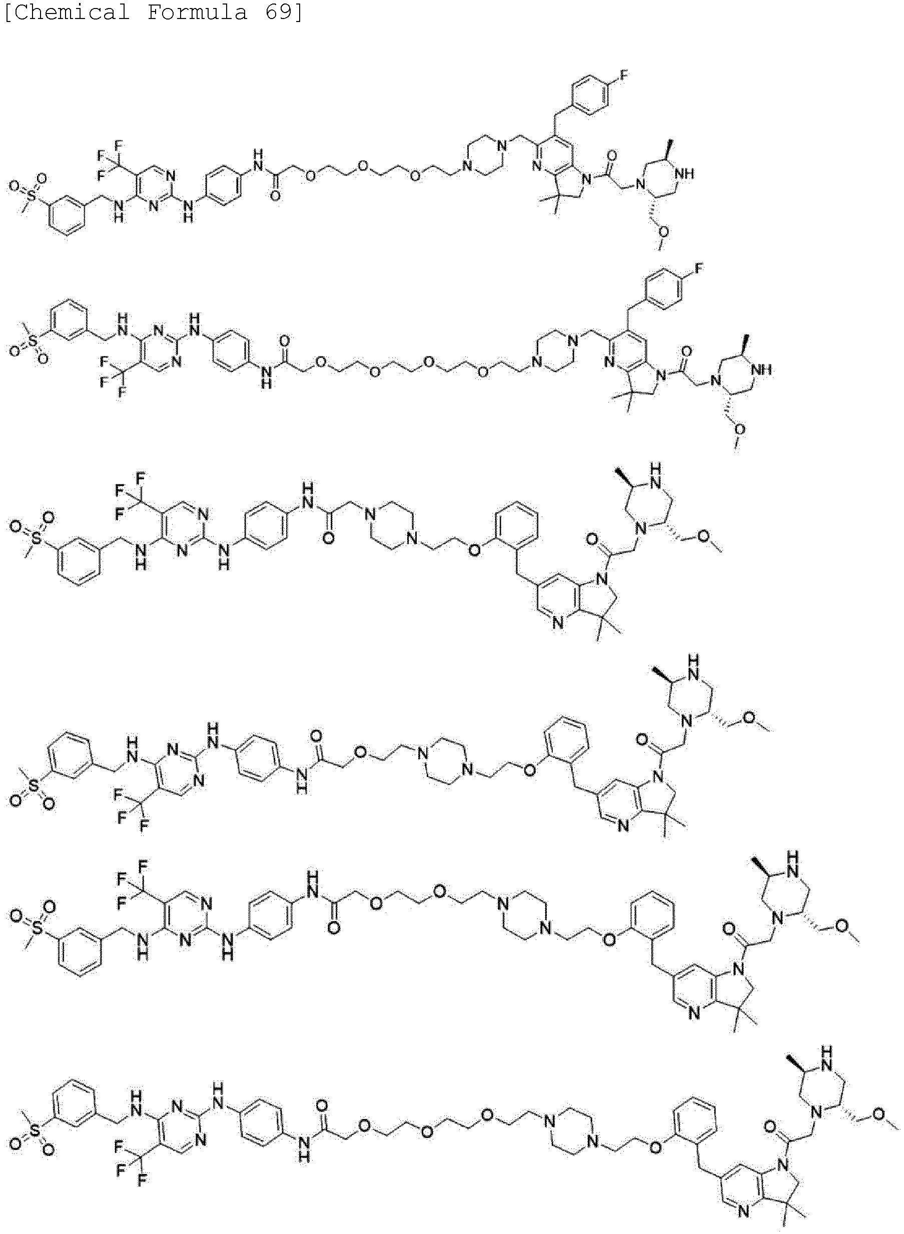

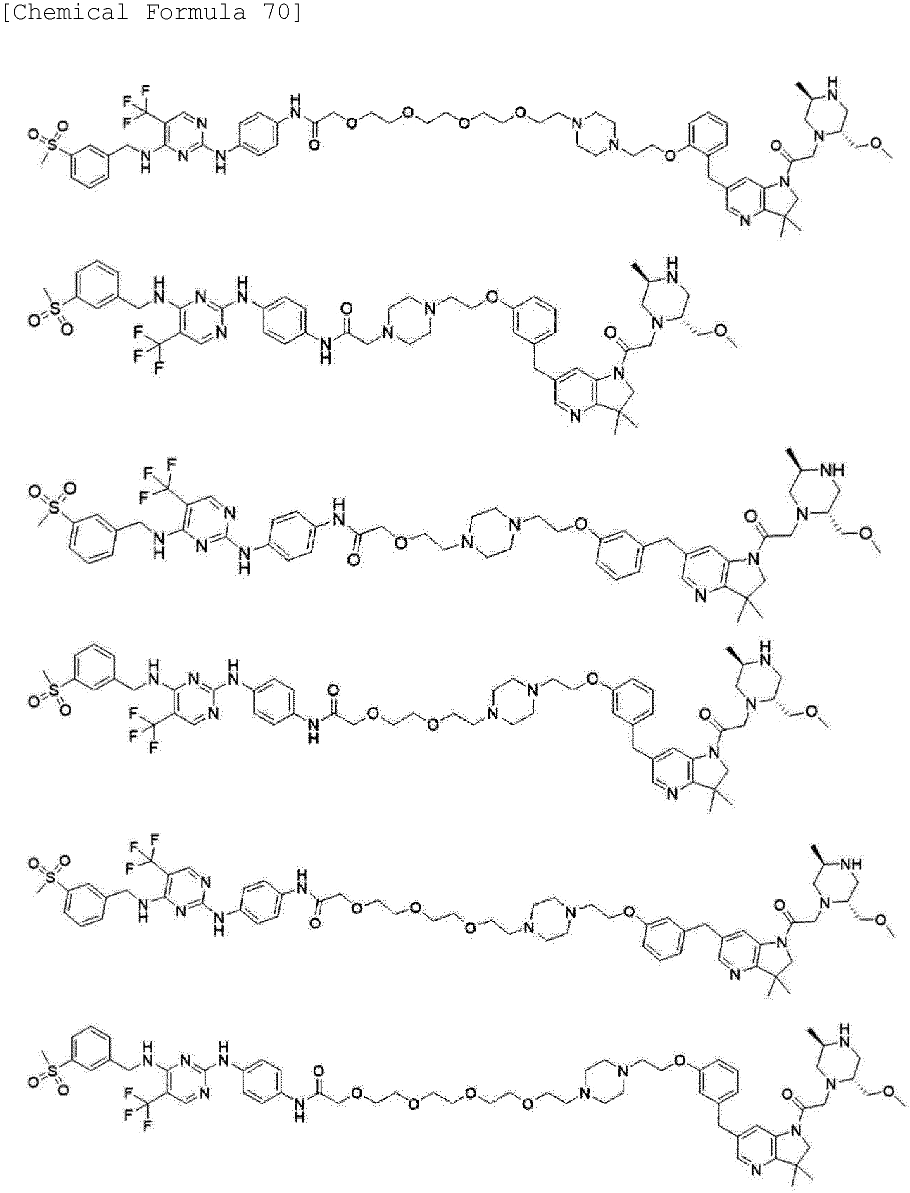

In one embodiment, the compounds of the present disclosure exclude the compounds of the following formula: 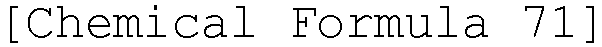 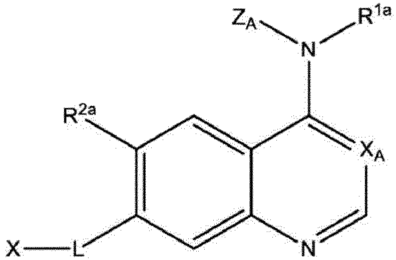 wherein
X_{A} represents N or CH,
L is a linker, X is as described above and is bonded to L at L1,
R^{1a} is H, -SO₂C₁₋₄ alkyl, -COC₁₋₄ alkyl, or C₁₋₄ alkyl,
R^{2b} is -SR^{aa}, -SOR^{aa}, -SO₂R^{aa}, -SO₂NH₂, or -SO₂NR^{ab}R^{ac},
wherein R^{aa} is C₁₋₆ alkyl, haloC₁₋₆ alkyl, C₃₋₇ cycloalkyl, 4- to 7-membered heterocycloalkyl, aryl, or heteroaryl, wherein the C₁₋₆ alkyl is optionally substituted with one or two groups independently selected from the group consisting of cyano, hydroxyl, C₁₋₆ alkoxy, C₁₋₆ alkoxy C₂₋₆ alkoxy, -CO₂H, -CO₂C₁₋₄ alkyl, -SO₂C₁₋₄ alkyl, C₃₋₇ cycloalkyl, phenyl, 5- to 6-membered heteroaryl, 9- to 10-membered heteroaryl, 4- to 7-membered heterocycloalkyl, and (phenyl) (C₁₋₄ alkyl)amino-, (wherein the C₃₋₇ cycloalkyl, phenyl, (phenyl) (C₁₋₄ alkyl)amino-, 5- to 6-membered heteroaryl, 9- to 10-membered heteroaryl, or 4- to 7-membered heterocycloalkyl is optionally substituted with one to three groups independently selected from the group consisting of halogen, -CF₃, hydroxyl, amino, (C₁₋₄ alkyl)amino-, (C₁₋₄ alkyl) (C₁₋₄ alkyl) amino-, C₁₋₄ alkyl, phenylC₁₋₄ alkyl-, hydroxyC₁₋₄ alkyl, and C₁₋₄ alkoxy),
the C₃₋₇ cycloalkyl or 4- to 7-membered heterocycloalkyl is optionally substituted with one to three groups independently selected from the group consisting of halogen, -CF₃, hydroxyl, amino, (C₁₋₄ alkyl) amino, (C₁₋₄ alkyl) (C₁₋₄ alkyl)amino, C₁₋₄ alkyl, phenylC₁₋₄ alkyl, hydroxyC₁₋₄ alkyl, oxo, and C₁₋₄ alkoxy, and
the aryl or heteroaryl is optionally substituted with one to three groups independently selected from the group consisting of halogen, -CF₃, hydroxyl, amino, (C₁₋₄ alkyl) amino, (C₁₋₄ alkyl) (C₁₋₄ alkyl) amino, C₁₋₄ alkyl, phenylC₁₋₄ alkyl, hydroxyC₁₋₄ alkyl-, and C₁₋₄ alkoxy,
R^{ab} is C₁₋₆ alkyl or 4- to 7-membered heterocycloalkyl, wherein the C₁₋₆ alkyl is optionally substituted with one or two groups independently selected from the group consisting of hydroxyl, C₁₋₆ alkoxy, C₁₋₆ alkoxy C₁₋₆ alkoxy, -CO₂H, -CO₂C₁₋₄ alkyl, (C₁₋₄ alkyl) amino, (C₁₋₄ alkyl) (C₁₋₄ alkyl)amino, 5- to 6-membered heteroaryl, and 4- to 7-membered heterocycloalkyl (wherein the 5- to 6-membered heteroaryl or 4- to 7-membered heterocycloalkyl is optionally substituted with one to three groups independently selected from the group consisting of halogen, C₁₋₄ alkyl, hydroxyC₁₋₄ alkyl, and C₁₋₄ alkoxy), and the 4- to 7-membered heterocycloalkyl is optionally substituted with one to three groups independently selected from the group consisting of hydroxyl, amino, C₁₋₄ alkyl, C₁₋₄ alkoxycarbonyl, hydroxyC₁₋₄ alkyl, oxo, and C₁₋₄ alkoxy,
R^{ac} is H, C₁₋₄ alkoxy or C₁₋₆ alkyl,
or R^{ab} and R^{ac}, together with the nitrogen atom to which they are attached, join to form a 3- to 7-membered heterocycloalkyl group optionally containing one or two additional ring heteroatoms independently selected from nitrogen and oxygen, wherein the 3- to 7-membered heterocycloalkyl is optionally substituted with one to three groups independently selected from the group consisting of C₁₋₄ alkyl, hydroxy, -CO₂H, and - COC₁₋₄ alkyl, and
Z_{A} is phenyl or aryl C₁₋₄ alkyl-, wherein the aryl moiety of the phenyl group or aryl C₁₋₄ alkyl- group is substituted with R^{4a}, R^{5a}, R^{6a}, and R^{7a}, wherein
R^{4a} is H, halogen, cyano, C₁₋₄ alkyl, haloC₁₋₄ alkyl, C₁₋₄ alkoxy, phenoxy, phenylC₁₋₄ alkoxy, hydroxyl, hydroxyC₁₋₄ alkyl-, or aminocarbonyl, wherein the phenyl moiety of the phenoxy or phenylC₁₋₄ alkoxy is optionally substituted with 1 to 3 substituents independently selected from the group consisting of halogen, -CF₃, C₁₋₄ alkyl, and C₁₋₄ alkoxy, and R^{5a}, R^{6a}, and R^{7a} are each independently selected from the group consisting of H, hydroxyl, halogen, -CF₃, hydroxyC₁₋₄ alkyl, C₁₋₄ alkyl, and C₁₋₄ alkoxy, or
Z_{A} is phenyl or pyridyl substituted with R^{8a}, R^{9a}, and R^{10a}, wherein
R^{8a} and R^{9a} are attached to the adjacent atoms, and, together with the atoms to which they are attached, join to form a 5-membered ring containing 1, 2 or 3 heteroatoms independently selected from N, O, and S, wherein the 5-membered ring is substituted with R^{11a}, and
one of R^{10a} or R^{11a} is H, halogen, cyano, C₁₋₄ alkyl, haloC₁₋₄ alkyl, C₁₋₄ alkoxy, phenoxy, phenylC₁₋₄ alkoxy, hydroxyl, hydroxyC₁₋₄ alkyl-, or aminocarbonyl, wherein the phenyl moiety of the phenoxy or phenylC₁₋₄ alkoxy is optionally substituted with 1 to 3 substituents independently selected from the group consisting of halogen, -CF₃, C₁₋₄ alkyl, and C₁₋₄ alkoxy, and the other of R^{10a} or R^{11a} is H, hydroxyl, halogen, -CF₃, hydroxyC₁₋₄ alkyl, C₁₋₄ alkyl, or C₁₋₄ alkoxy, or Z_{A} is the formula: 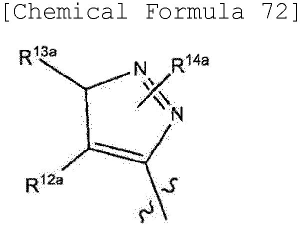
wherein 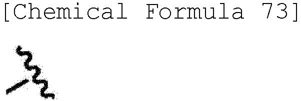
is a bonding point,
R^{12a} is H, methyl, or hydroxymethyl,
R^{13a} is methyl, trifluoromethyl, or hydroxymethyl, and
R^{14a} is H, OH, or C₁₋₃ alkyl, or
R^{12a} and R^{13a}, together with the atoms to which they are attached, join to form a 6-membered ring substituted with R^{15a} and R^{16a}, wherein the 6-membered ring optionally contains one nitrogen atom,
wherein R^{15a} and R^{16a} are each independently selected from the group consisting of H, halogen, cyano, C₁₋₄ alkyl, haloC₁₋₄ alkyl, C₁₋₄ alkoxy, phenoxy, phenylC₁₋₄ alkoxy, hydroxyl, hydroxyC₁₋₄ alkyl-, and aminocarbonyl, wherein the phenyl moiety of the phenoxy or phenylC₁₋₄ alkoxy is independently from the group consisting of halogen, -CF₃, C₁₋₄ alkyl, and C₁₋₄ alkoxy.

X of the present disclosure may have a substituent represented by any of the following.

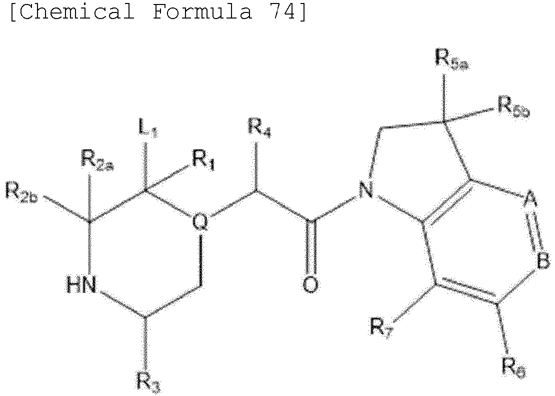

**[Table A-1]**

| | R₁ | L₁ | R₂ₐ, R_{2b} | R₃ | R₄ | R₅ₐ | R_{5b} | R₆ | R₇ | A | B | Q |
|---|---|---|---|---|---|---|---|---|---|---|---|---|
| 1 | hydrogen | hydrogen | hydrogen | methyl | hydro gen | hydroge n | hydrog on | -(CR^{x}p^{y})-H | hydroge n | CH | CH | N |
| 2 | C₁₋₆ alkyl | C₁₋₆ alkyl | | fluoromethy l | | methyl | methyl | -(CR^{x}R^{y})-F | fluorine | N | C(C₁₋₄ alkyl) | CH |
| 3 | C₂₋₆ alkenyl | C₂₋₆ alkenyl | | difluoromet hyl | | form cyclopropyl group together with the carbon atom to which they are attached | | -(CR^{x}p^{y})-C₁₋₂ alkyl | | | C(C₂₋₄ alkenyl) | |
| 4 | C₂₋₆ alkynyl | C₂₋₆ alkynyl | | trifluoromet hyl | | | | -(CR^{x}R^{y}}-O-phenyl | | | C(C₂₋₄ alkynyl) | |
| 5 | -C(=O)NH_{(2-q)}(C₁₋₆ alkyl)_{q} | -C(=O)NH_{(2-q)}(C₁₋₆ alkyl)_{q} | | | | | | -S(O)₂-CH₂-R^{z} | | | C(CH₂-C₃₋₆ carbocyclyl) | |
| 6 | -(CH₂)ₛ-(3- to -7 membered heterocyclyl) | -(CH₂)ₛ-(3- to 7-membered heterocyclyl) | | | | | | -C(=O)C₁₋₄ alkyl | | | C(CH₂-(4-to 6-membered heterocyclyl) | |
| 7 | -(CH₂)ₛ-C₃₋₇ carbocyclyl | -(CH₂)ₛ-C₃₋₇ carbocyclyl | | | | | | -C(=O)-phenyl | | | C(CH₂-O-R^{z}-C(=O)R^{x}) | |

**[Table A-2]**

| | | | | | | | | | | | | |
|---|---|---|---|---|---|---|---|---|---|---|---|---|
| 8 | -C(=O)-(3- to 7-membered heterocyclyl) | -C(=O)-(3- to 7-membered heterocyclyl) | | | | | | -C(=O)-(5-6-membered heteroaryl) | | | C(CH₂-NR^{x}R^{y}) | |
| 9 | -C(=O)-C₃₋₇ carbocyclyl | -C(=O)-C₃₋₇ carbocyclyl | | | | | | -CH₂-phenyl | | | N | |
| 10 | form 3- to 6-membered saturated carbocyclyl group together with the carbon atom to which they are attached | | | | | | | -CH₂-(5-6-membered heteroaryl) | | | CL | |
| 11 | form 4- to 6-membered heterocyclyl group together with the carbon atom to which they are attached | | | | | | | 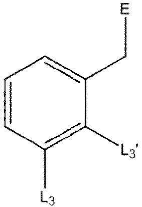 | | | | |
| 12 | | L | | | | | | | | | | |

**[Table B-1]**

| | R^{b} | R^{x} | R^{y} | R^{z} | L₃ | L₃' |
|---|---|---|---|---|---|---|
| 1 | - | - | - | - | - | - |
| 2 | fluorine | chlorine | chlorine | chlorine | L | L |
| 3 | methyl | fluorine | fluorine | fluorine | form 3- to 6-membered carbocyclyl group that can be connected to a linker, together with the carbon atoms to which they are attached | |
| 4 | C₃₋₆ cycloalkyl | hydrogen | hydrogen | hydrogen | form 4- to 6-membered heterocyclyl group that can be connected to a linker, together with the carbon atoms to which they are attached | |
| 5 | phenyl | C₁₋₄ alkyl | C₁₋₄ alkyl | C₁₋₄ alkyl | | |
| 6 | 5- to 6-membered heteroaryl | C₁₋₄ alkoxy | C₁₋₄ alkoxy | C₁₋₄ alkoxy | | |
| 7 | -O-C₁₋₂ alkyl | when attached to nitrogen or carbon, form 3- to 7-membered ring optionally containing one or one additional heteroatom selected from O, and N, together with the atom(s) to which they are attached | | | | |
| 8 | fluoromethyl | | | | | |
| 9 | fluoromethoxy | | | | | |
| 10 | hydroxy | | | | | |
| 11 | =O | | | | | |

**[Table B-2]**

| | | | | | | |
|---|---|---|---|---|---|---|
| 12 | -S(O)₂-C₁₋₄ alkyl | | | | | |
| 13 | -C(=O)C₁₋₄ alkyl | | | | | |
| 14 | -C(=O)NR^{x}R^{y} | | | | | |
| 15 | -NR^{x}C(=O)C₁₋₄ alkyl | | | | | |
| 16 | -NR^{x}R^{y} | | | | | |

In Table A and Table B, alkyl, alkenyl, alkynyl, carbocyclyl, heterocyclyl, aryl, heteroaryl, and 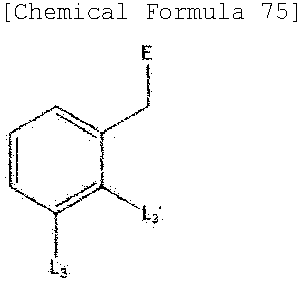 other than R^{b} are optionally substituted with one or more R^{b} groups. In addition, L in Table A and Table B means that X is bonded to the linker L at that point (present in the columns L₁, L₂, L₃ and L₃' in Tables A and B) .

(R₁, L₁, R₂ₐ and R_{2b}, R₃, R₄, R₅ₐ, R_{5b}, R₆, R₇, A, B, Q, R^{b}, R^{x}, R^{y}, R^{z}, L₃, L₃') = (R_{1(AX)}, L_{1(BX)}, R_{2(CX)}, R_{3(DX)}, R_{4(EX)}, R_{5a(FX)}, R_{5b(GX)}, R_{6(HX)}, R_{7(IX)}, A_{(JX)}, B_{(KX)}, Q_{(LX)}, R^{b}_{(MX)}, R^{x}_{(NX)}, R^{y}_{(OX)}, R^{z}_{(PX)}, L_{3(QX)}, L₃'_{(RX)}), which represents any combination of substituents.

Here, AX is 1 to 11, BX is 1 to 12, CX is 1, DX is 1 to 4, EX is 1, FX is 1 to 3, GX is 1 to 3, HX is 1 to 11, IX is 1 to 2, JX is 1 to 2, KX is 1 to 10, LX is 1 to 2, MX is 1 to 16, NX is 1 to 7, OX is 1 to 7, PX is 1 to 6, QX is 1 to 4, and RX is 1 to 4, each of which is an integer. For example, R_{1(AX)} refers to the AX-th substituent of R₁ in Table A above. Here, if the substituent is not substituted with R^{b} group, MX is 1; if R^{x} is not present, NX is 1; if R^{y} is not present, OX is 1; if R^{z} is not present, PX is 1; and if L₃ and L₃' are not present, QX and RX are 1; which indicate that each substituent is not present.

Here, there is only one linker L per compound.

In one aspect, a process for producing the compound of the present disclosure is provided.

The process comprises
(a) reacting a compound of Formula (III) 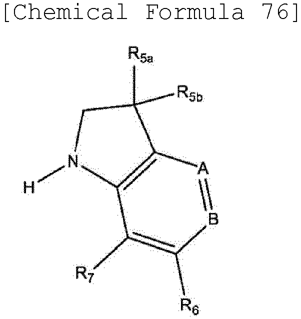
   wherein A, B, R₅ₐ, R_{5b}, R₆, and R₇ are as defined herein,
   with a compound of Formula (IV): 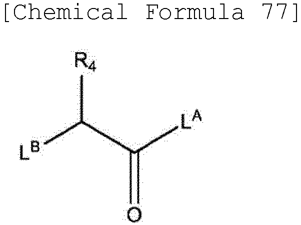
   wherein R₄ is as defined herein, and L^{A} and L^{B} each independently represent a leaving group,
   followed by a subsequent reaction with a compound of Formula (V): 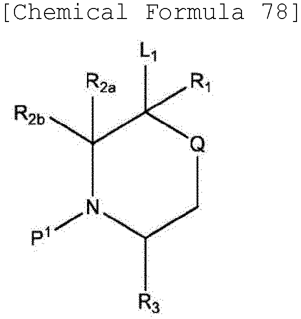
   wherein R₁, R₂ₐ, R_{2b}, and R³ are as defined herein, L₁ is not used for bonding to L, and P¹ represents hydrogen or a protecting group, and
   followed by a deprotection reaction suitable for removing the protecting group P¹,
   to produce X of the present disclosure; and/or
(b) (i) reacting Z of the present disclosure and L of the present disclosure to produce Z-L, and
   reacting the Z-L with X to produce the compound of Formula (I), (ii) reacting X of the present disclosure and L of the present disclosure to produce X-L, and
   reacting the X-L with Z to produce the compound of Formula (I); and/or
   (iii) reacting Z of the present disclosure and La, which is a part of L of the present disclosure, to produce Z-La,
   reacting X of the present disclosure and Lb, which is a remaining part of the L, to produce X-Lb, and
   reacting the Z-La with the X-Lb to produce the compound of Formula (I).

In cases other than Q=N, a person skilled in the art can produce the compounds of the present disclosure based on the common general knowledge in the art using an appropriate synthetic method (see Argiriadi, Maria A. et al., WO 2016198908 A1, Smith, Aaron C. et al., J. Org. Chem. (2016), 81(9);3509-3519, Thompson, Wayne J. et al., US 5756508 A).

In one aspect, the compound of the present disclosure is a compound represented by the following formula 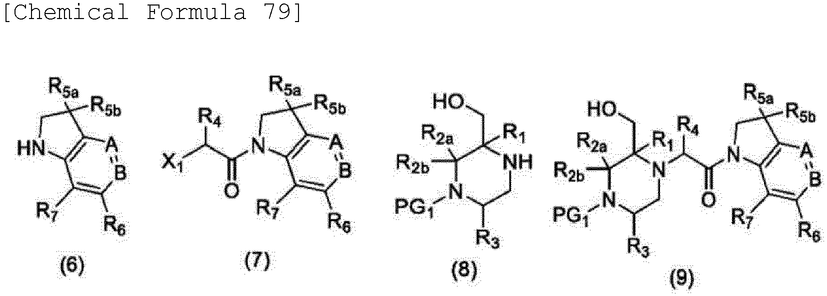 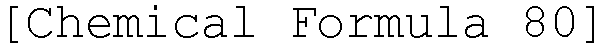 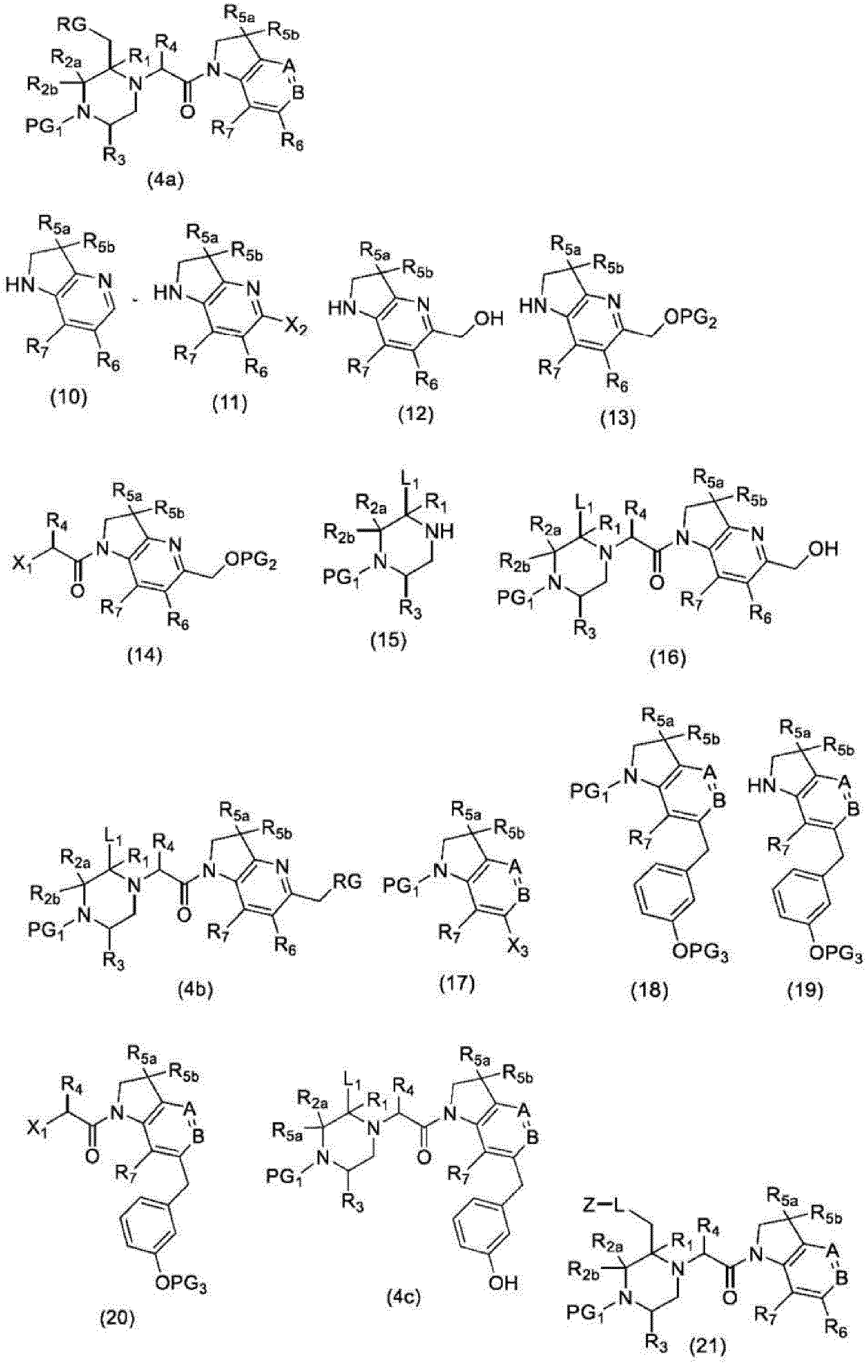 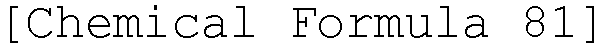 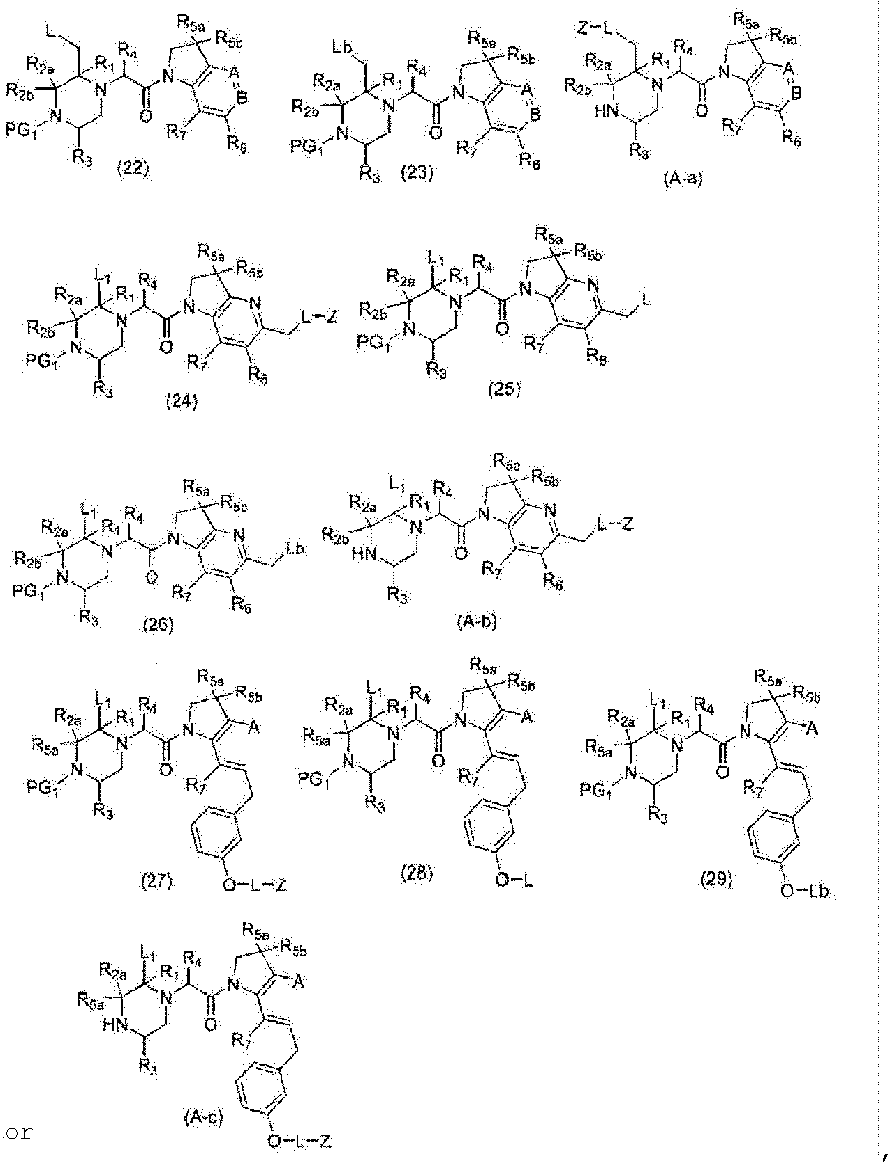
or an isomer thereof, or a salt thereof,
wherein
R₁, R₂ₐ, R_{2b}, R₃, R₄, R₅ₐ, R_{5b}, A, B, R₆, and R₇ are as defined above, X₁, X₂, and X₃ each independently represent a halogen atom or a leaving group, PG₁ represents a suitable protecting group such as a tert-butoxycarbonyl (Boc) group, PG₂ represents a suitable protecting group such as a tert-butyldiphenylsilyl (TBDPS) group, PG₃ represents a suitable protecting group such as a benzyl group, RG represents a halogen atom or a reactive functional group such as a leaving group, a hydroxyl group, an amino group, a carboxyl group, an amido group, a sulfonamido group or the like, L, La, and Lb each independently represent a linker, and Z represents a ligand moiety capable of specifically binding to an intracellular protein.

The compound of the disclosure is further described hereinafter.

While the compound of the disclosure can have enantiomers and stereoisomers such as tautomers and geometric isomers depending on the type of substituent, they are also encompassed by the present disclosure. Specifically, if there is one or more asymmetric carbon atoms in the compound of the disclosure, there is a diastereomer or enantiomer, and a mixture of such a diastereomer and enantiomer, and isolated diastereomers and enantiomers are also encompassed by the compound of the disclosure.

The present disclosure is also intended to encompass various hydrates, solvates, and crystalline polymorphisms.

Furthermore, the compound of the disclosure can be substituted with an isotope (e.g., ²H (or D), ³H (or T), ¹¹C, ¹³C, ¹⁴C, ¹³N, ¹⁵N, ¹⁵O, ³⁵S, ¹⁸F, ¹²⁵I, or the like). These compounds are also encompassed by the compound of the disclosure.

Furthermore, the scope of the present disclosure encompasses prodrugs of the compound of the disclosure. In the present disclosure, a prodrug refers to a derivative that yields a compound represented by Formula A or a related structural formula thereof by acid hydrolysis or enzymatic degradation in the body. For example, if a compound represented by Formula A or a related structural formula thereof has a functional group such as a hydroxyl group, amino group, or carboxyl group or other substituents, these groups can be modified by a conventional method to manufacture a prodrug. Prodrug technologies are described in, for example, C.G. Wermuth, "The Practice of Medicinal Chemistry", 4th Ed., Academic Press, (2015), Chapter 28.

Examples for compounds having a carboxy group include compound whose carboxyl group is modified to be an alkoxycarbonyl group, alkylthiocarbonyl group, or alkylaminocarbonyl group.

Examples of compounds having an amino group include compounds whose amino group is substituted with an alkanoyl group to be an alkanoylamino group, compounds substituted with an alkoxycarbonyl group to be an alkoxycarbonylamino group, compounds modified to have an alkanoyloxymethylamino group, and compounds modified to have hydroxylamine.

Examples for compounds having a hydroxyl group include compounds whose hydroxyl group is substituted with an alkanoyl group to be an alkanoyloxy group, phosphate ester, or alkanoyloxymethyloxy group.

Examples of the alkyl moiety of a group used for preparing a prodrug thereof include the alkyl group. The alkyl group is optionally substituted with, for example, an alkoxy group or the like. Preferred examples thereof include the following.

For compounds whose carboxyl group is modified to be an alkoxycarbonyl group, examples thereof include alkoxycarbonyl such as methoxycarbonyl and ethoxycarbonyl, and alkoxycarbonyl substituted with an alkoxy group such as methoxymethoxycarbonyl, ethoxymethoxycarbonyl, 2-methoxyethoxycarbonyl, and 2-methoxyethoxymethoxycarbonyl, or privaloyloxymethoxycarbonyl.

Z in the above Formula (A) is a ligand moiety capable of specifically binding to an intracellular protein, and has an inhibitory activity or binding activity against the intracellular protein.

The intracellular protein in Z in the above Formula (A) can be a kinase or a non-kinase. The abbreviations for kinase and non-kinase used in present disclosure are given below.

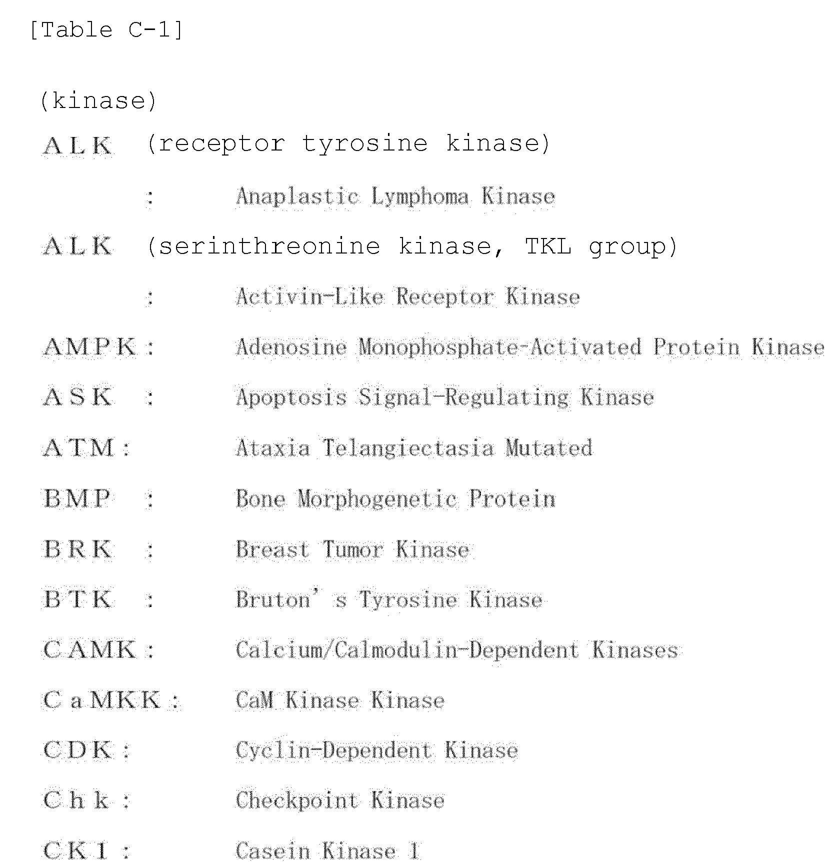

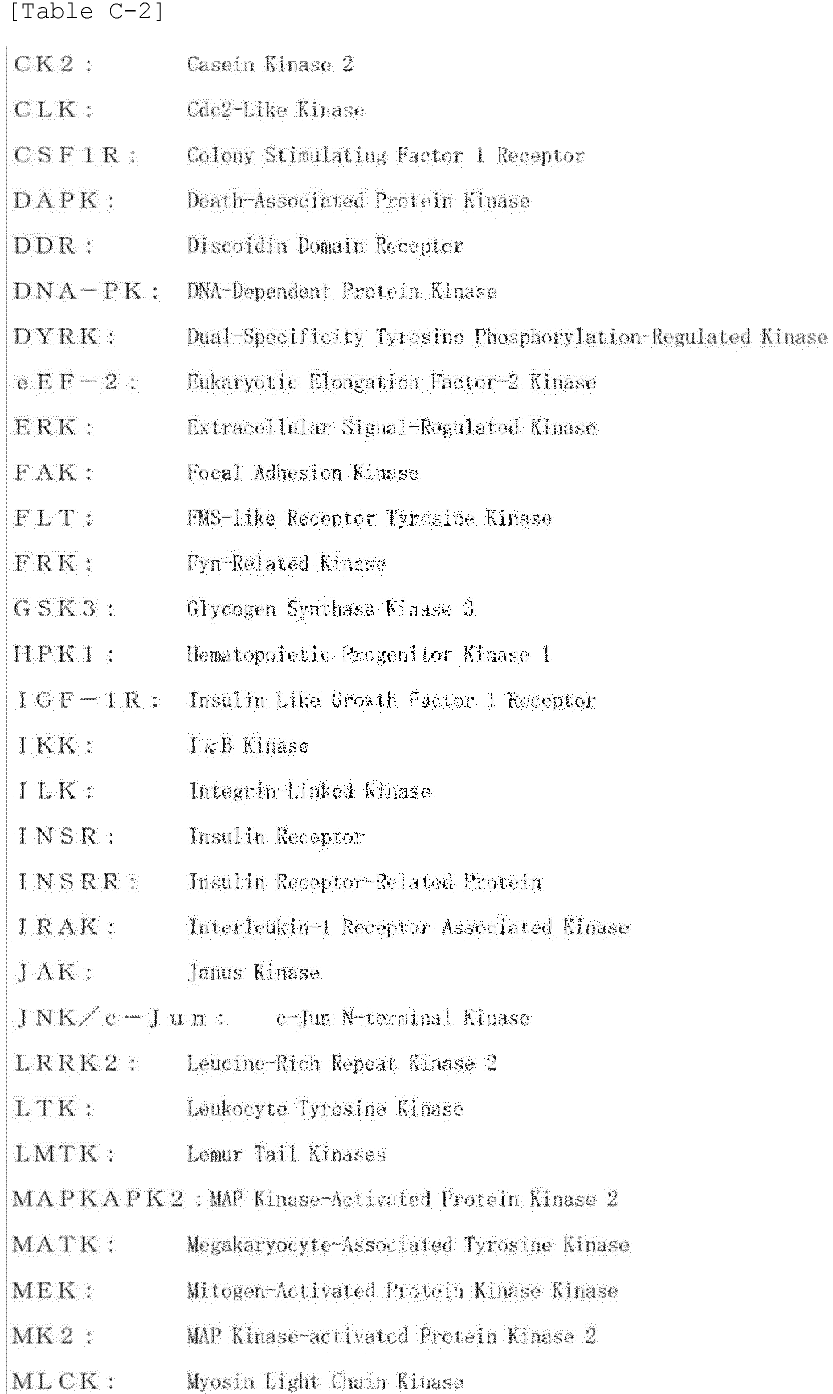

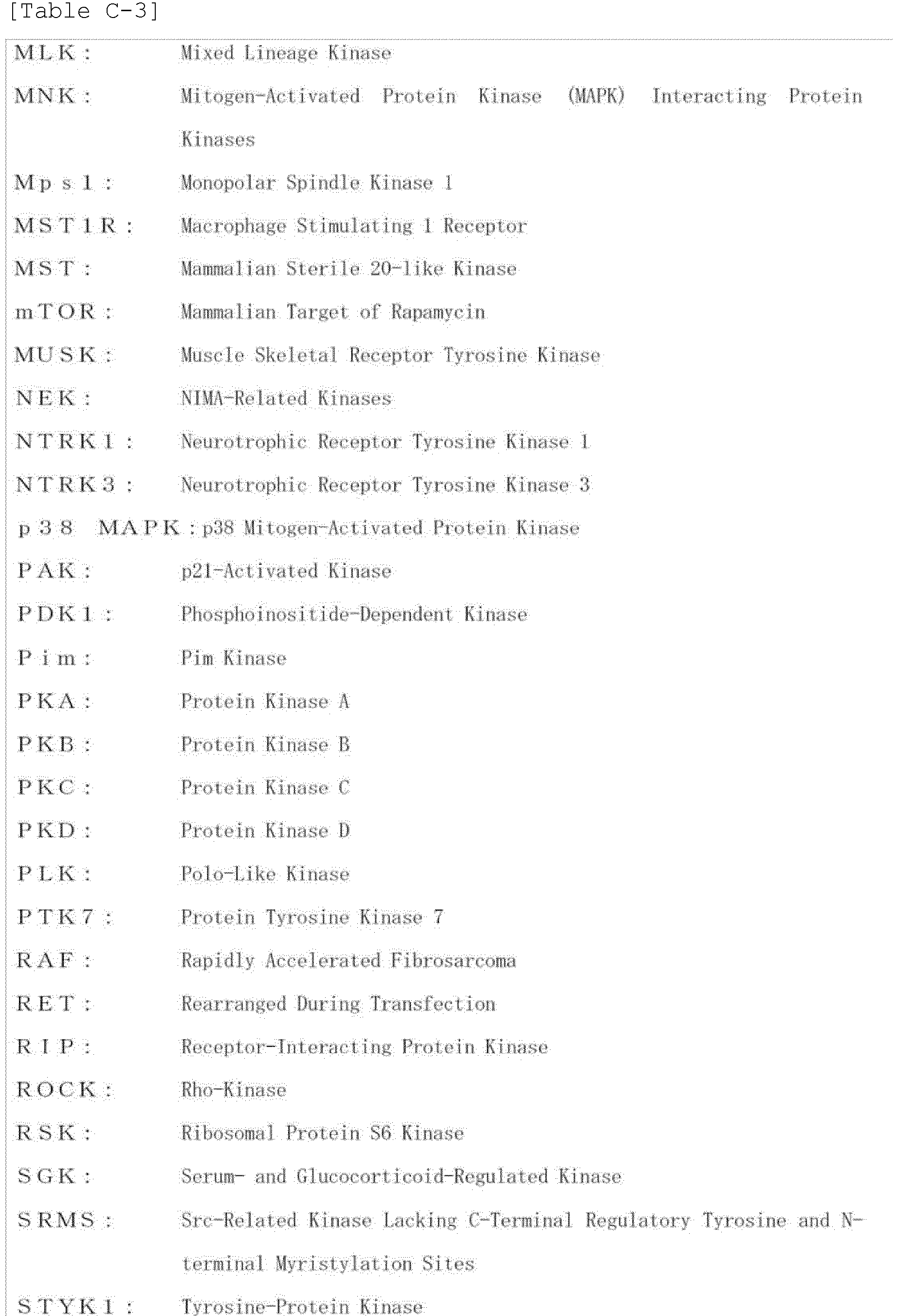

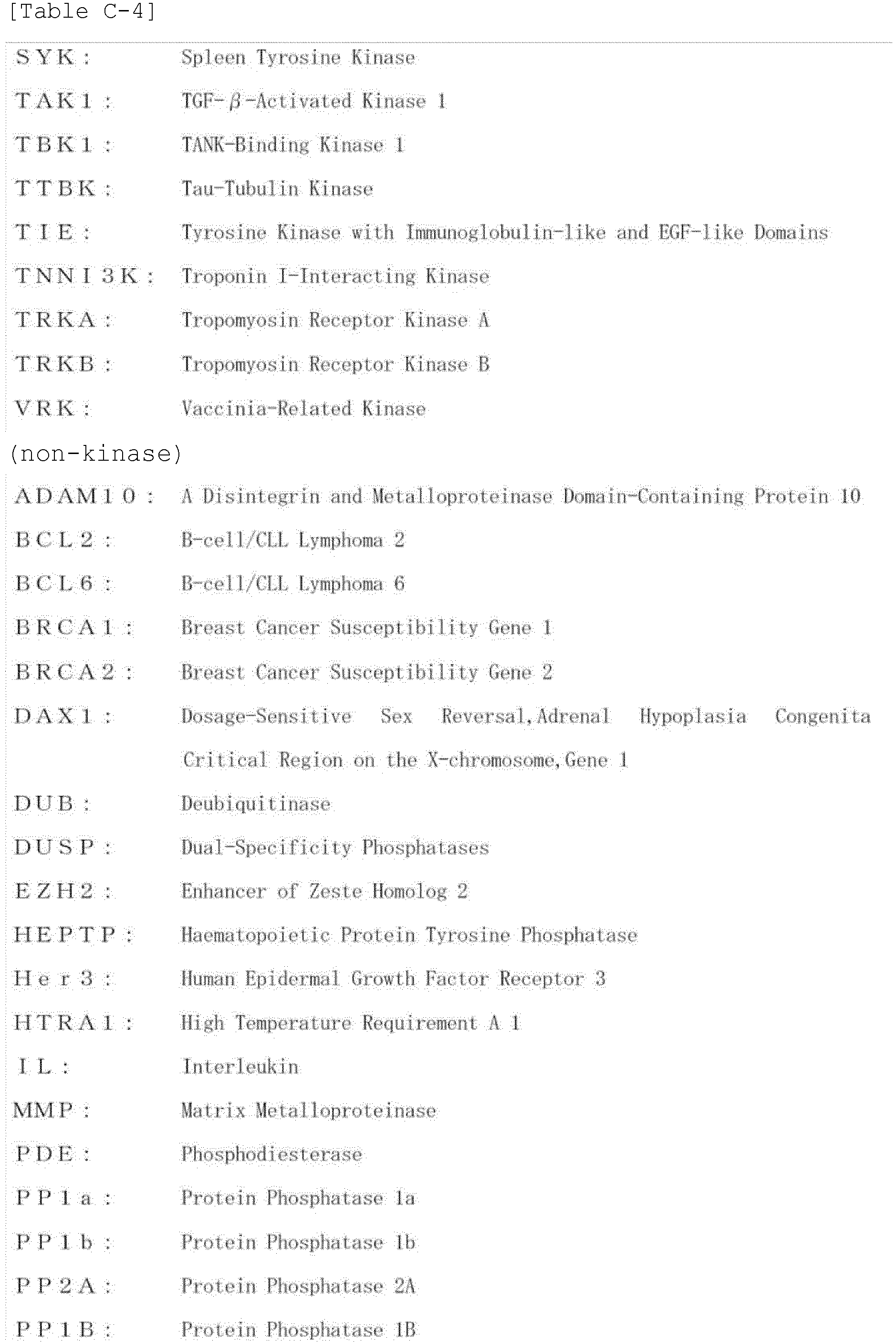

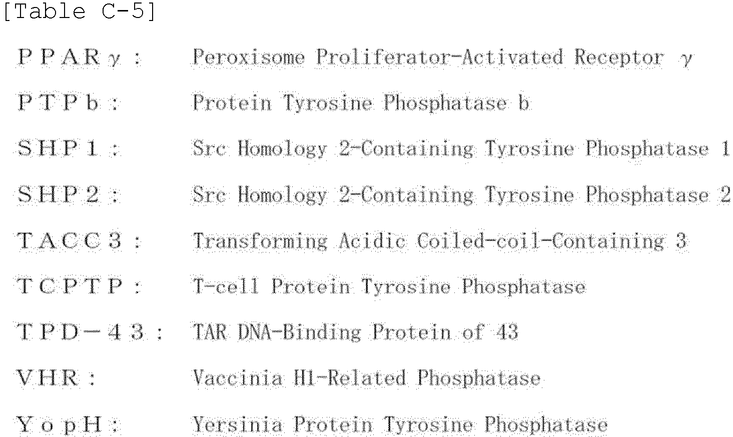

Serine/threonine kinases, receptor tyrosine kinases, and non-receptor tyrosine kinases that may be the subject of the present disclosure include, but are not limited to, Akt/PKB family (AKT1, AKT2, AKT3), PDK1, PKA, PKC, PKG, ROCK (ROCK1, ROCK2), RSK family (RSK1, RSK2, RSK3, RSK4), SGK family (SGK1, SGK2, SGK3), AMPK, CaMK family (CaMK1, CaMK2, CaMK4), Chk (ChK1, ChK2), DAPK family (DAPK1, DAPK2, DAPK3), MK2 (MAPKAP2, MAPKAP), MLCK (MYLK1, MYLK2, MYLK3, MYLK4), MNK, PI family (PIM1, PIM2), PKD, CK1, TTBK (TTBK1), VRK family (VRK1), CDK family (CDK1-9, CDK11-14, CDK16, CDK19), CK2, CLK family (CLK1, CLK2, CLK3, CLK4), ERK family (ERK1, ERK2, ERK5, ERK7, ERK8), GSK3, JNK/c-Jun, p38 MAPK, DYRK family (DYRK1, DYRK2, DYRK3), HIPK, ASK family (ASK1, ASK2, ASK3), MEK family (MEK1, MEK2), PAK family (PAK1, PAK2, PAK3, PAK4, PAK5, PAK6), MST family (MST1, MST2, MST4), HPK1, BMP family and other activin receptors (BMPR1A, BMPR1B, BMPR2, ALK1, ALK2, ALK3, ALK4, ALK5), LRRK2, RAF family (A-RAF, B-RAF, RAF1), RIP family (RIP1, RIP2, RIP3), TAK1, TGF-b receptor IRAK family (IRAK1, IRAK2, IRAK3, IRAK4), MLK family (MLK1, MLK2, MLK3, MLK4), LIMK family (LIMK1, LIMK2), Aurora Kinase, CaMKK, IKK (IKK-1, IKK-2), Mps1, PLK family (PKK1, PLK2, PLK3, PLK4, PLK5), ATM, DNA-PK, mTOR (mTORC1, mTORC2), ILK, NEK family (NEK2, 3, 6,7, 11), TNNI3K, Wee1, TBK1, MST1R, epidermal growth factor receptor family (EGFR, RBB1, ERBB2, ERBB3, ERBB4), insulin receptor family (INSR, IGF-1R, INSRR), platelet-derived growth factor family (PDGFRα, PDGFRβ, CSF1R, FLT1, FLT3, FLT4, KIT), vascular endothelial growth factor receptor family (VEGFR1, VEGF2, VEGF3, VEGFR4, soluble VEGFR1, soluble VEGFR2, soluble VEGFR3), fibroblast growth factor receptor family (FGFR1, FGFR2, FGFR3, FGFR4), CCK family, nerve growth factor receptor family (MET or c-MET, RON), hepatocyte growth factor receptor family, Eph receptor family (EPHA1, EPHA2, EPHA3, EPHA4, EPHA5, EPHA6, EPHA7, EPHA8, EPHA10, EPHB1, EPHB2, EPHB3, EPHB4, EPHB6), AXL receptor family (AXL, MER, TYRO3), TIE receptor family (TIE, TEK), RYK receptor family (RYK), DDR receptor family (DDR1, DDR2), RET receptor family, ROS receptor family (ROS1), LTK receptor family (LTK, ALK), ROR receptor family (ROR1, ROR2), MuSK receptor family (MUSK), LRM receptor family (LMTK2, LMTK3), TRKA, TRKB, PTK7, NTRK1, NTRA2, NTRK3, RYK, TYRP3, STYK1, ABL family (ABL1, ARG), ACK family (ACK1, TNK1), CSK family (CSK, MATK), FAK family (FAK, PYK2), FES family (FES, FER), FRK family (FRK, BRK, SRMS), JAK family (JAK1, JAK2, JAK3, TYRK2), SRC family (SRC, YES1, FYN, FGR, LYN, LCK, HCK, BLK), TEC family (TEC, BMX, BTK, ITK, TXK), SYK family (SYK, ZAP70).

Table 1 shows examples of the structures of ligands for typical kinases.

**[Table 1-1]**

| Typical target kinase | Example of ligand Compound name | Literature |
|---|---|---|
| Akt/PKB | 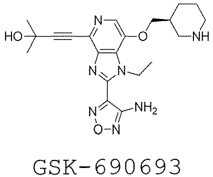 | Yang T. et al., eBioMedicine.47;114-127(2019) |
| PDK1 | 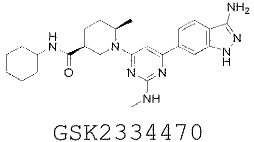 | Najafov A. et al., Biochem.J.433;357-369(2011) |
| PKA | 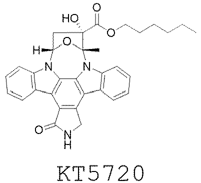 | Davies SP. et al., Biochem.J.351;95-105(2000) |
| PKC | 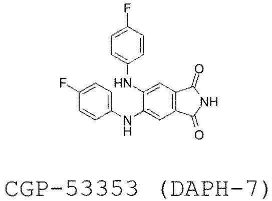 | Yamamoto M. et al., Am.J.Physiol.Cell Physiol.279;C587-C595(2000) |
| PKG | 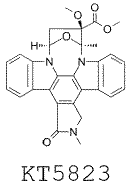 | Gadbois DM.et al., Pro.Natl.Acad.Sci.USA .89;8626-8630(1992) |
| ROCK1/2 | 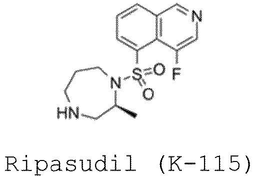 | Isobe T. et al., Curr.Eye Res.39;813-822(2014) |

**[Table 1-2]**

| | | |
|---|---|---|
| ROCK1/2 | 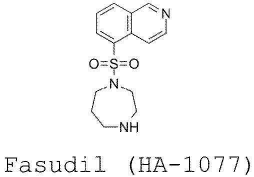 | Chen M. et al., Expert Opin.Investig.Drugs.22;537-550(2013) |
| RSK1/2/3 | 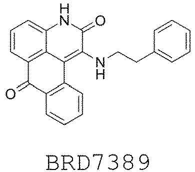 | Fomina-Yadlin D. et al., Proc.Natl.Acad.Sci.US A.107;15099-15104(2010) |
| GK | 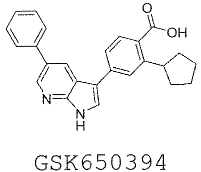 | Sherk AB.et al., Cancer Res.68;7475-7483(2008) |
| AMPK | 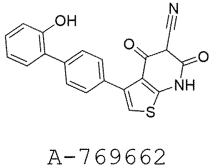 | Cool B. et al., Cell Metab.3;403-416(2006) |
| CaMK | 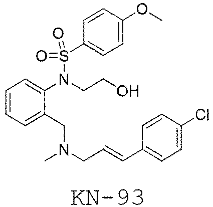 | Tombes RM. et al., Cell Growth Differ.6;1063-1070(1995) |
| Chk1 | 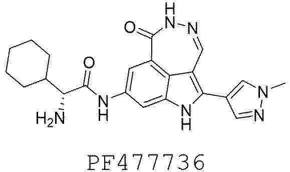 | Blasina A. et al., Mol.Cancer Ther.7;2394-2404(2008) |

**[Table 1-3]**

| | | |
|---|---|---|
| DAPK | 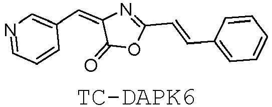 | Okamoto M. et al., J.Med.Chem.52;7323-7327(2009) |
| MK2 | 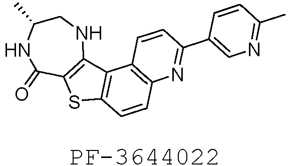 | Mourey R. et al., J Pharmacol.Exp.Ther.33 3;797-807 (2010) |
| MLCK | 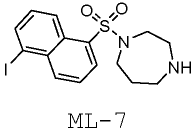 | Shi J. et al., Br.J.Pharmacol.152;12 2-131(2007) |
| MNK1 | 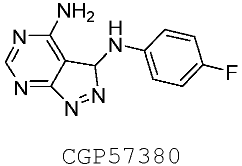 | Knauf U. et al., Mol.Cell.Biol.21;5500 -5511(2001) |
| PIM1 | 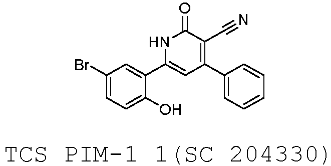 | Cheney IW. et al., Bioorg.Med.Chem.Lett. 17;1679-1683(2007) |
| PKD | 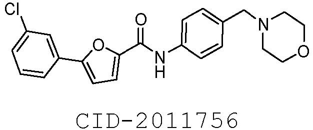 | CID 2011756 Sharlow ER. et al., PLoS One.6; e25134(2011) |

**[Table 1-4]**

| | | |
|---|---|---|
| CK1 | 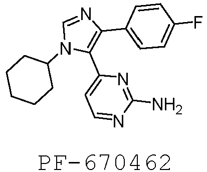 | Badura L. et al., J Pharmacol.Exp.Ther.32 2;730-738(2007) |
| TTBK1 | 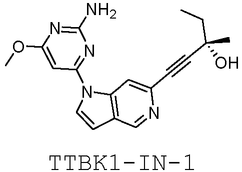 | Halkina T. et al., J.Med.Chem.64;6358-6380 (2021) |
| VRK1 | 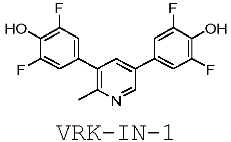 | Serafim RAM. et al., ACS Med.Chem.Lett.10; 1266-1271(2019) |
| CDK1/2/9 | 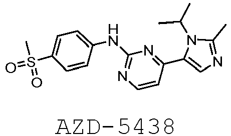 | Byth K. et al., Mol.Cancer Ther.8;1856-1866 (2009) |
| CK2 | 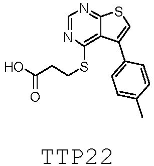 | Golub AG. et al., Eur.J.Med.Chem.46;870 -876(2011) |
| CLK1/4 | 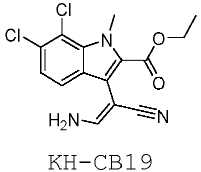 | Fedorov O. et al., Chem.Biol.18;67-76 (2011) |
| ERK | 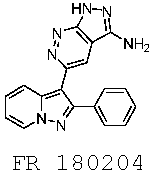 | Ohori M. et al., Biochem.Biophys.Res.C ommun.336;357-363(2005) |

**[Table 1-5]**

| | | |
|---|---|---|
| GSK3 | 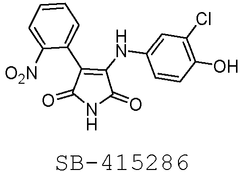 | Coghlan MP. et al., Chem.Biol. 7;793-803(2000) |
| JNK | 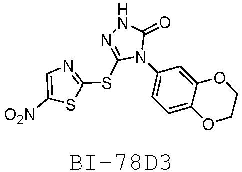 | Stebbins JL. et al., Proc.Natl.Acad Sci.USA.105;16809-16813(2008) |
| p38 MAPK | 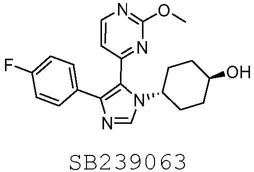 | Underwood DC. et al., J.Pharmacol.Exp.Ther. 293;281-288(2000) |
| DYRK3 | 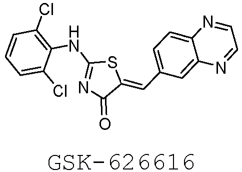 | Wippich F. et al., Cell.152;791-805(2013) |
| ASK1 | 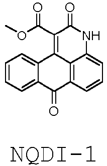 | Volynets GP. et al., J.Med.Chem.54;2680-2686(2011) |
| MEK1/2 | 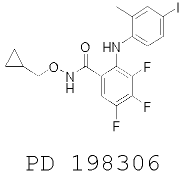 | Ciruela A. et al., Br.J.Pharmacol.138;75 1-756 (2003) |

**[Table 1-6]**

| | | |
|---|---|---|
| PAK1 | 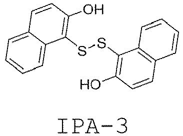 | Viaud J. et al., Mol.Cancer.Ther.8;255 9-2565(2009) |
| MST1/2 | 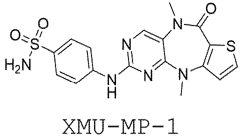 | Fan F. et al., Sci.Transl.Med.8;352r a108(2016) |
| BMP and ALK | 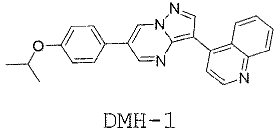 | Engers DW. et al., Bioorg.Med.Chem.Lett. 23;3248-3285(2013) |
| LRRK2 | 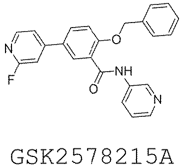 | Reith AD. et al., Bioorg.Med.Chem.Lett. 22;5625-5629(2012) |
| B-RAF | 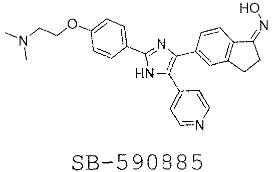 | King AJ. et al., Cancer.Res.66;11100-11105(2006) |
| RIP1 | 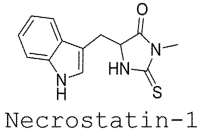 | Degterev A. et al., Nat.Chem.Biol.1;112-119(2005) |
| TAK1 | 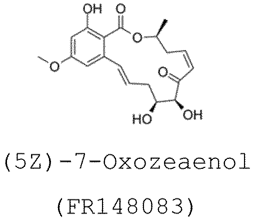 | Ninomiya-Tsuji J. et al., J.Biol.Chem.278;18485 -18490(2003) |

**[Table 1-7]**

| | | |
|---|---|---|
| TGF-b receptor | 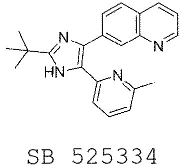 | Grygielko ET. et al., J.Pharmacol.Exp.Ther. 313;945-951(2005) |
| IRAK4 | 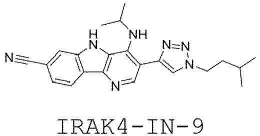 | Sabnis RW., ACS Med.Chem.Lett.13;336-337(2022) |
| MLK | 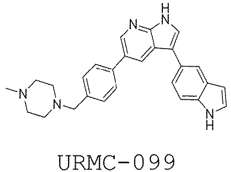 | Goodfellow VS. et al., J.Med.Chem.56;8032-8048(2013) |
| LIMK1/2 | 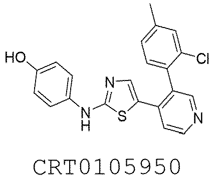 | Mardiovich K. et al., Oncotarget.6;38469-38486(2015) |
| Aurora Kinase | 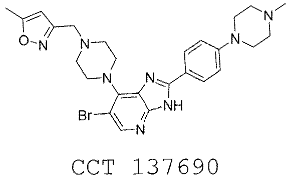 | Bavetsias V. et al., J.Med.Chem.53;5213-5228(2010) |
| CaMKK | 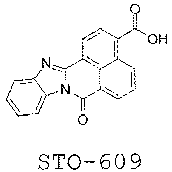 | Tokumitsu H. et al., J.Biol.Chem.277;15813 -15818(2002) |
| IKK-2 | 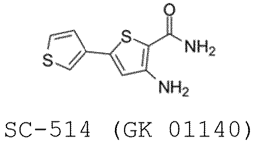 | Kishore N. et al., J.Biol.Chem.278;32861 -32871(2003) |

**[Table 1-8]**

| | | |
|---|---|---|
| Mps1 | 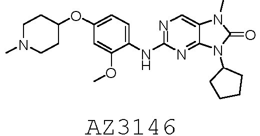 | Hewitt L. et al., J.Cell Biol.190;25-34(2010) |
| PLK1/3 | 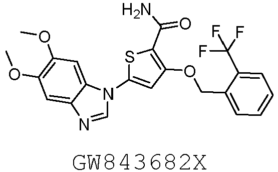 | Lansing TJ. et al., Mol.Cancer Ther.6;450-459(2007) |
| ATM | 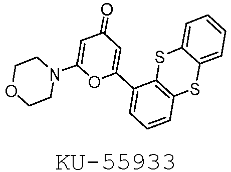 | Hickson I. et al., Cancer Res.64;9152-9159(2004) |
| DNA-PK | 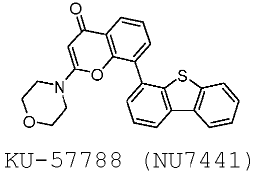 | Leahy JJJ.et al.,Bioorg.Med.Chem.L ett.14;6083-6087(2004) |
| mTORC1/2 | 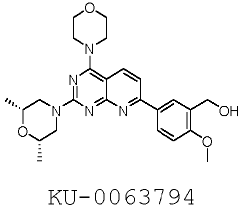 | Garcia-Martinez JM. et al., Biochem. J.421;29-42(2009) |
| ILK | 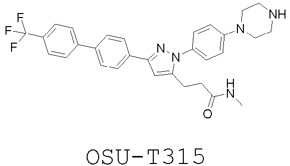 | Kee SL.et al.,J.Med.Chem.54;636 4-6374(2011) |

**[Table 1-9]**

| | | |
|---|---|---|
| NEK2 | 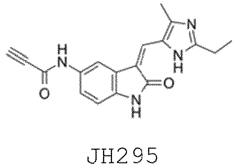 | Henise JC. et al., J.Med.Chem.54;4133-4146(2011) |
| TNNI3K | 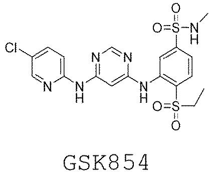 | Braian JP. et al., J.Med.Chem.61;3076-3088(2018) |
| Wee1 | 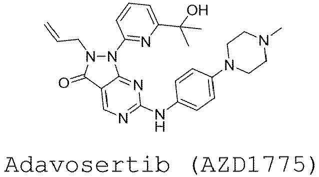 | Hirai H. et al., Mol.Cancer.Ther.8;299 2-3000(2009) |
| Epidermal growth factor receptor | 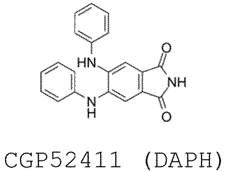 | Buchdunger E. et al.,Proc.Natl.Acad Sci.USA.91;2334-2338(1994) |
| IGF-1R | 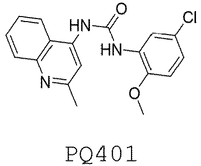 | Gable KL.et al., Mol.Cancer Ther.5;1079-1086 (2006) |
| PDGFRα/β | 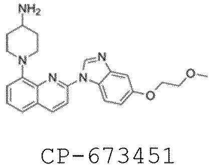 | Xi Y. et al., Onco Targets Ther.7;1215-1221(2014) |

**[Table 1-10]**

| | | |
|---|---|---|
| VEGFR1/2/ 3 | 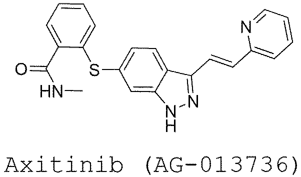 | Hu-Lowe DD. et al., Clin.Cancer Res.14;7272-7283(2008) |
| FGFR1 | 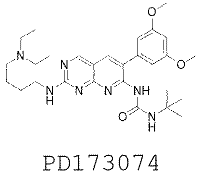 | Mohammadi M. et al., EMBO J.17;5896-5904 (1998) |
| c-MET/ALK | 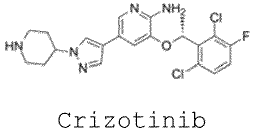 | Zou HY. et al., Cancer Res.67;4408-4417(2007) |
| EPHB4 | 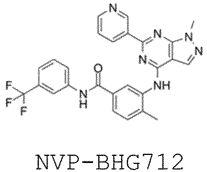 | Troster A. et al., ChemMedChem.13;1629-1633(2018) |
| DDR1 | 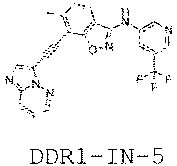 | Aleksandr MA, et al., Kinase inhibitors. WO2020079652A1. |
| VEGFR/PDG FR/Kit/RE T | 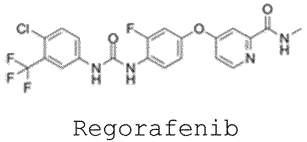 | Wilhelm SM. et al., Int.J.Cancer.129;245-255(2011) |
| ROS1 | 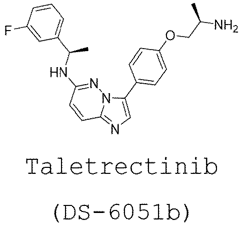 | Katayama R. et al., Nat.Commun.10;3604 (20 19) |

**[Table 1-11]**

| | | |
|---|---|---|
| LTK/ALK | 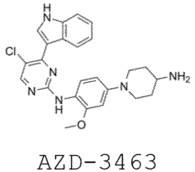 | Wang Y. et al., Sci.Rep.6;19423(2016) |
| TRKA | 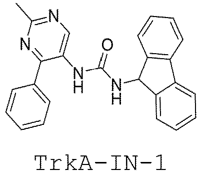 | Su HP. et al., Proc.Natl.Acad.Sci.US A.114;E297-E306(2017) |
| ABL | 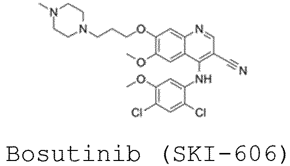 | Boschelli DH. et al., J.Med.Chem.44;3965-3977(2001) |
| ACK1 | 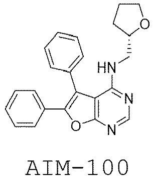 | Mahajan K. et al., Prostate.70;1274-1285(2010) |
| CSK | 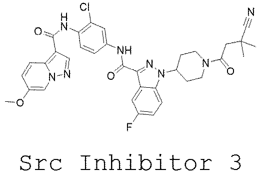 | O'Malley DP. et al., ACS Med.Chem.Lett. 10;1486-1491(2019) |
| FAK | 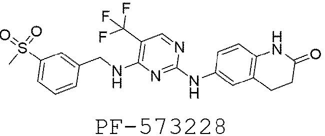 | Slack-Davis JK. et al., J.Biol.Chem. 282;14845-14852(2007) |

**[Table 1-12]**

| | | |
|---|---|---|
| FER | 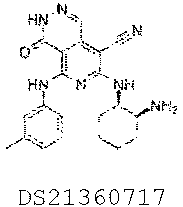 | Taniguchi T. et al., ACS Med.Chem.Lett. 10;737-742(2019) |
| BRK | 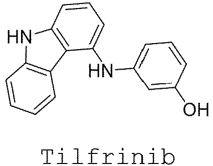 | Mahmoud KA. et al., Bioorg.Med.Chem.Lett. 24;1948-1951(2014) |
| JAK1/2/3 | 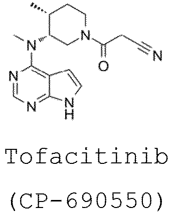 | Jiang JK. et al., J.Med.Chem.51;8012-8018 (2008) |
| SRC | 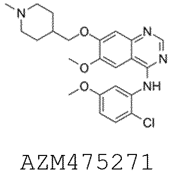 | Ple PA. et al., J.Med.Chem.47;871-887(2004) |
| BTK | 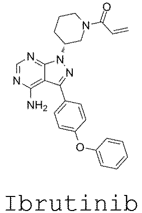 | Honigberg LA. et al., Proc.Natl.Acad Sci. USA.107;13075-13080(2010) |
| SYK | 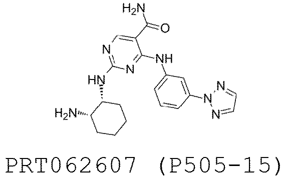 | Coffey G. et al., J.Pharmacol.Exp.Ther. 340;350-359(2012) |

Serin/threonine kinases that may be the subject of the present disclosure include, but are not limited to, Akt/PKB (AKT1, AKT2, AKT3), PDK1, PKA, PKC, PKG, ROCK (ROCK1, ROCK2), RSK (RSK1, RSK2, RSK3, RSK4), SGK (SGK1, SGK2, SGK3), AMPK, CaMK (CaMK1, CaMK2, CaMK4), Chk (ChK1, ChK2), DAPK (DAPK1, DAPK2, DAPK3), MK2 (MAPKAP2, MAPKAP), MLCK (MYLK1, MYLK2, MYLK3, MYLK4), MNK, PIM (PIM1, PIM2), PKD, CK1, TTBK (TTBK1), VRK (VRK1), CDK (CDK1-9, CDK11-14, CDK16, CDK19), CK2, CLK (CLK1, CLK2, CLK3, CLK4), ERK (ERK1, ERK2, ERK5, ERK7, ERK8), GSK3, JNK/c-Jun, p38 MAPK, DYRK (DYRK1, DYRK2, DYRK3), HIPK, ASK (ASK1), MEK (MEK1, MEK2), PAK (PAK1, PAK2, PAK3, PAK4, PAK5, PAK6), MST (MST1, MST2, MST4), HPK1, BMP and other activin receptors (BMPR1A, BMPR1B, BMPR2, ALK1, ALK2, ALK3, ALK4, ALK5), LRRK2, RAF (A-RAF, B-RAF, RAF1), RIP (RIP1, RIP2, RIP3), TAK1, TGF-b receptor IRAK (IRAK1, IRAK2, IRAK3, IRAK4), MLK (MLK1, MLK2, MLK3, MLK4), LIMK (LIMK1, LIMK2), Aurora Kinase, CaMKK, IKK (IKK-1, IKK-2), Mps1, PLK (PKK1, PLK2, PLK3, PLK4, PLK5), ATM, DNA-PK, mTOR (mTORC1, mTORC2), ILK, NEK (NEK2, 3, 6,7, 11), TNNI3K, Wee1, TBK1, and MST1R.

In one embodiment, serin/threonine kinases include, but are not limited to:
<AGC group>
   Akt/PKB (AKT1, AKT2, AKT3), PDK1, PKA, PKC, PKG, ROCK (ROCK1, ROCK2), RSK (RSK1, RSK2, RSK3, RSK4), SGK (SGK1, SGK2, SGK3)
<CAMK group>
   AMPK, CaMK (CaMK1, CaMK2, CaMK4), Chk (ChK1, ChK2), DAPK (DAPK1, DAPK2, DAPK3), MK2 (MAPKAP2, MAPKAP), MLCK (MYLK1, MYLK2, MYLK3, MYLK4), MNK, PIM (PIM1, PIM2), PKD
<CK1 group>
   CK1, TTBK (TTBK1), VRK (VRK1)
<CMGC group>
   CDK (CDK1-9, CDK11-14, CDK16, CDK19), CK2, CLK (CLK1, CLK2, CLK3, CLK4), ERK (ERK1, ERK2, ERK5, ERK7, ERK8), GSK3, JNK/c-Jun, p38 MAPK, DYRK (DYRK1, DYRK2, DYRK3), HIPK
<STE group>
   ASK (ASK1, ASK2, ASK3), MEK (MEK1, MEK2), PAK (PAK1, PAK2, PAK3, PAK4, PAK5, PAK6), MST (MST1, MST2, MST4), HPK1
<TKL group>
   BMP and other activin receptors (BMPR1A, BMPR1B, BMPR2, ALK1, ALK2, ALK3, ALK4, ALK5), LRRK2, RAF (A-RAF, B-RAF, RAF1), RIP (RIP1, RIP2, RIP3), TAK1, TGF-b receptor, IRAK (IRAK1, IRAK2, IRAK3, IRAK4), MLK (MLK1, MLK2, MLK3, MLK4), LIMK (LIMK1, LIMK2)
<Other serinthreonine kinases>
   Aurora Kinase, CaMKK, IKK (IKK-1, IKK-2), Mps1, PLK, ATM, DNA-PK, mTOR, ILK, NEK (NEK2, 3, 6,7, 11), TNNI3K, Wee1, TBK1, MST1R

Receptor tyrosine kinases that may be the subject of the present disclosure include, but are not limited to, epidermal growth factor receptor family (EGFR, RBB1, ERBB2, ERBB3, ERBB4), insulin receptor family (INSR, IGF-1R, INSRR), platelet-derived growth factor family (PDGFRα, PDGFRβ, CSF1R, FLT1, FLT3, FLT4, KIT), vascular endothelial growth factor receptor family (VEGFR1, VEGF2, VEGF3, VEGFR4, soluble VEGFR1, soluble VEGFR2, soluble VEGFR3), fibroblast growth factor receptor family (FGFR1, FGFR2, FGFR3, FGFR4), CCK family, nerve growth factor receptor family (MET or c-MET, RON), hepatocyte growth factor receptor family, Eph receptor family (EPHA1, EPHA2, EPHA3, EPHA4, EPHA5, EPHA6, EPHA7, EPHA8, EPHA10, EPHB1, EPHB2, EPHB3, EPHB4, EPHB6), AXL receptor family (AXL, MER, TYRO3), TIE receptor family (TIE, TEK), RYK receptor family (RYK), DDR receptor family (DDR1, DDR2), RET receptor family, ROS receptor family (ROS1), LTK receptor family (LTK, ALK), ROR receptor family (ROR1, ROR2), MuSK receptor family (MUSK), LRM receptor family (LMTK2, LMTK3) and other receptor tyrosine kinases (TRKA, TRKB, PTK7, NTRK1, NTRA2, NTRK3, RYK, TYRP3, STYK1).

Non-receptor tyrosine kinases that may be the subject of the present disclosure include, but are not limited to, ABL family (ABL1, ARG), ACK family (ACK1, TNK1), CSK family (CSK, MATK), FAK family (FAK, PYK2), FES family (FES, FER), FRK family (FRK, BRK, SRMS), JAK family (JAK1, JAK2, JAK3, TYRK2), SRC family (SRC, YES1, FYN, FGR, LYN, LCK, HCK, BLK), TEC family (TEC, BMX, BTK, ITK, TXK) and SYK family (SYK, ZAP70).

In one embodiment, protein kinases include, but are not limited to CDK2, CDK4, CDK6, CDK8, CDK9, CDK12, TRK, BTK, FAK, epidermal growth factor receptor family, TGF-b receptor ABL1, p38 MAPK, JAK2, c-MET, ALK, Wee1, Akt, MEK (MEK1, MEK2), Mps1, Aurora Kinase, SGK3, TBK1, FLT3, LRRK2, RIP1, RIP2 and HPK1.

In one embodiment, protein kinases include, but are not limited to FAK, ROCK (ROCK1, ROCK2), TGF-b receptor platelet-derived growth factor family, fibroblast growth factor receptor family, vascular endothelial growth factor receptor family, epidermal growth factor receptor family, SYK, PIM2, and ALK5.

Non-kinases that may be the subject of the present disclosure include, but are not limited to, muscarinic acetylcholine receptor, adenosine receptor, adrenalin receptor, GABA receptor, angiotensin receptor, cannabinoid receptor, cholestokinin receptor, dopamine receptor, orexin receptor, glucagon receptor, histamine receptor, olfactory receptor, opioid receptor, rhodopsin receptor, secretin receptor, serotonin receptor, somatostatin receptor, gastrin receptor, P2Y receptor, chemokine receptor, potassium channel, sodium channel, calcium channel, chlorine channel, cation channel, IL-2 receptor, IL-3 receptor, IL-4 receptor, IL-5 receptor, IL-6 receptor, IL-7 receptor, IL-9 receptor, IL-11 receptor, IL-12 receptor, IL-13 receptor, IL-15 receptor, granulocyte macrophage colony stimulating factor receptor, humans granulocyte colony stimulating factor receptor, erythropoietin receptor, thrombopoietin receptor, leukemia inhibitory factor receptor, oncostatin M receptor, ciliary neurotrophic factors receptor, growth hormone receptor, leptin receptor, interferon receptor, IL-10 receptor, tumor necrosis factor receptor, Fas ligand receptor, CD40 L receptor, inhibin receptor, IL-8 receptor, IL-16 receptor, eotaxin receptor, rantes receptor, IL-1 receptor, epidermal growth factor, insulin-like growth factor, transforming growth factor, basic fibroblast growth factor, nerve growth factor, brain-derived neurotrophic factor, vascular endothelial growth factor, granulocyte colony stimulating factor, granulocyte macrophage colony stimulating factor, platelet-derived growth factor, erythropoietin, thrombopoietin, hepatocyte growth factor, ligand-gated ion channel, voltage-gated ion channel, phosphorylation-gated ion channel, temperature-sensitive ion channel, mechano-sensitive ion channel, ion pump, ABC transporter, secondary active transporter, thyroid hormone receptor, retinoic acid receptor, peroxisome proliferator-activated receptor, Rev-ErbA, RAR-related orphan receptor, liver X receptor, farnesoid X receptor, vitamin D receptor, pregnane X receptor, constitutive androstane receptor, hepatocyte nuclear factor 4, retinoid X receptor, TXL/PNR, chicken ovalbumin upstream promoter, V-erbA-related protein, estrogen receptor, estrogen-related receptor, glucocorticoid receptor, mineralocorticoid receptor, progesterone receptor, androgen receptor, nerve growth factor IB, nuclear receptor-related 1 protein, neuron-derived orphan receptor-1, steroidogenic factor-1, liver receptor homolog-1, germ cell nuclear factor, DAX1, SHP, PPARγ, CD45, DUSP, HEPTP, LMPTPA, LMPTPB, PP1a, PP1b, PP2A, PP1B, PTPb, PTPMRG1, PTPMEG2, SHP1, SHP2, TCPTP, VHR, YopH, ADAM10, collagenase, MMP-2, MMP-8, MMP-9, MMP-12, MMP-13, caspase (1-12), cathepsin (B, C, F, H, K, L, O, S, V, W, X), HTRA1, histone acetyltransferase, histone deacetylase, bromo domain protein, lysine methyltransferase (DOT1 L, EZH2), lysine demethylase, methyl-lysine binding protein, signal transducer and activator of transcription, BAF, CDC20, MYC, PDE1, PDE2, PDE3, PDE4, PDE5, PDE6, PDE7, PDE8, PDE9, PDE10, PDE11, ubiquitin, ubiquitin-like protein, DUB, BCL2, BCL6, BRCA1, BRCA2, FANCA, FANCD2, FANCE, Her3, and heat shock protein, amyloid β, α-synuclein, tau protein, huntingtin protein, TPD-43, cellular retinoic acid-binding protein, TACC3, and parkin.

Table 2 shows examples of the structures of ligands for non-kinase proteins.

**[Table 2-1]**

| Typical target kinase | Example of ligand compound name | Literature |
|---|---|---|
| muscarinic acetylcholine receptor | 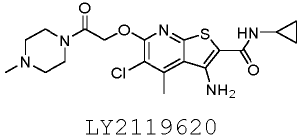 | Croy CH.et al., Mol. Pharmacol.86;106-1150(2014) |
| adenosine receptor | 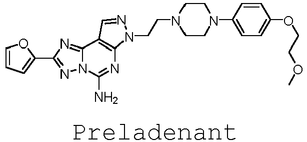 | Hodgson RA.et al., J Pharmacol.Exp. Ther.330;294-303(2009) |

**[Table 2-2]**

| | | |
|---|---|---|
| adrenalin receptor | 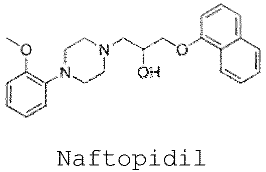 | Yamanaka N. et al., Hinyokika Kiyo.37;1759-1772(1991) |
| GABA receptor | 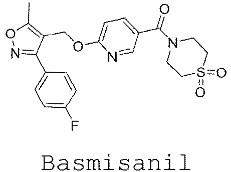 | Hipp JF.et al., Sci. Rep.11;7700(2021) |
| angiotensin receptor | 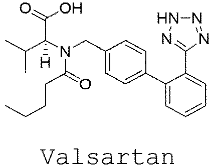 | Goossen LJ.et al., J.Org.Chem.72;7473-7476(2007) |
| cannabinoid receptor | 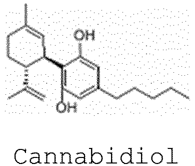 | Burstein S., Bioorg.Med.Chem.23;1 377-1385(2015) |
| gastrin receptor/ cholestokinin receptor | 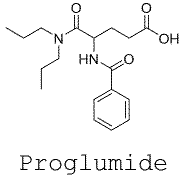 | Jensen RT.et al., Am J.Physiol.249; G214-G220(1985) |
| dopamine receptor | 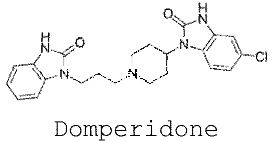 | Prakash A. et al., Drugs.56;429-445 (1998) |
| orexin receptor | 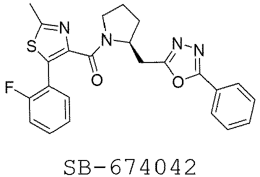 | Heifetz A. et al., Biochemistry.51;3178 -3197(2012) |

**[Table 2-3]**

| | | |
|---|---|---|
| histamine receptor | 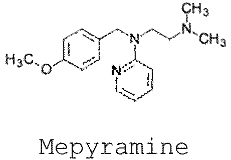 | Goot H. et al., Eur. J.Med.Chem.35;5-20(2000) |
| opioid receptor | 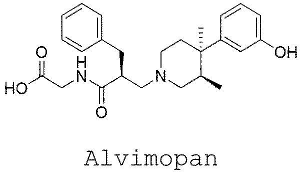 | Bourdonnec BL.et al. Bioorg.Med.Chem. Lett.18;2006-2012 (2008) |
| serotonin receptor | 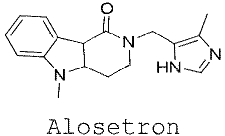 | Balfour JA.et al., Drugs.59;511-518(2000) |
| P2Y receptor | 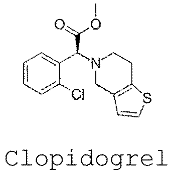 | Herbert JM.et al., Semin.Vasc.Med.3;113 -122(2003) |
| potassium channel | 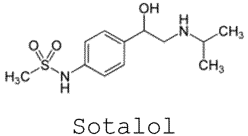 | Anderson JL.et al., Am. Heart J.137;388-409(1999) |
| sodium channel | 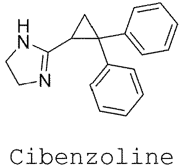 | Gholap AR et al., Synthetic Communications. 38;29 67-2982 (2008) |
| calcium channel | 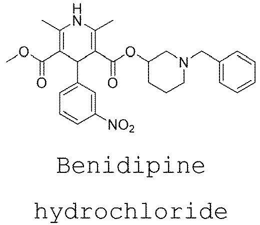 | Muto K.et al., Arzneimittelforschun g.38;1666-1670(1988) |

**[Table 2-4]**

| | | |
|---|---|---|
| chlorine channel | 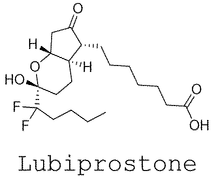 | McKeage K. et al., Drugs.66;873-879(2006) |
| cation channel (HCN channel) | 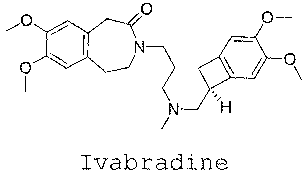 | Ragueneau I. et al., Clin.Pharmacol. Ther.64;192-203(1998) |
| thyroid hormone receptor | 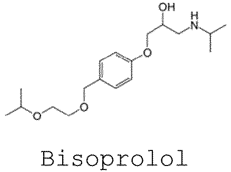 | Leopold G., J Cardiovasc.Pharmacol .8; S16-S20(1986) |
| retinoic acid receptor | 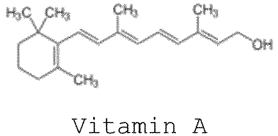 | Emerick RJ.et al., Biochem.J.102;606-611(1967) |
| peroxisome proliferator-activated receptor | 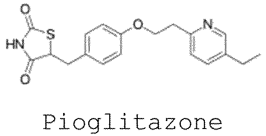 | ASohda T. et al., J. Med.Chem.35;2617-2626 (1992) |
| vitamin D receptor | 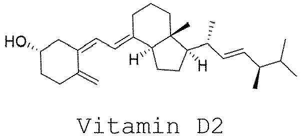 | Nigwekar SU.et al., Am.J. Kidney Dis.60;139-156(2012) |
| retinoid X receptor | 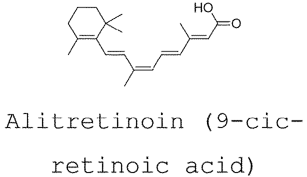 | Heyman RA.et al., Cell.68;397-406 (1992) |

**[Table 2-5]**

| | | |
|---|---|---|
| estrogen receptor | 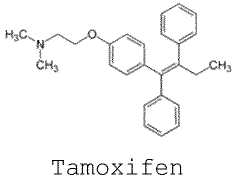 | Konstantions M. et al., Current Organic Chem.16;335-352(2012) |
| mineralocorticoid receptor | 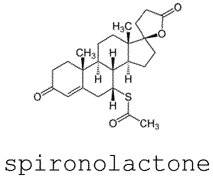 | Sorrentino R. et al., J.Cardiovasc. Pharmacol.36;230-235(2000) |
| progesterone receptor | 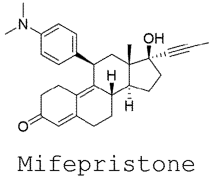 | Hazra B. et al., J.Indian Institute Sci. (2003) |
| androgen receptor | 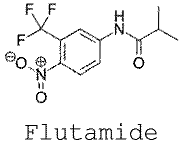 | Tucker H. et al., J. Med.Chem.31;954-959(1988) |
| ADAM10 | 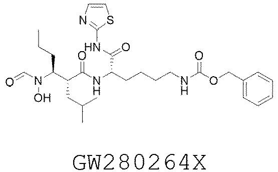 | Hundhausen C. et al., Blood.102;1186-1195(2003) |
| BET bromo domain | 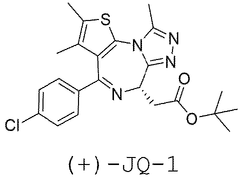 | Filippakopoulos P. et al., Nature.468;1067-1073(2010) |
| histone acetyltransferase | 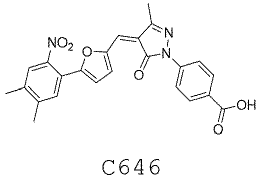 | Bowers EM.et al., Chem.Biol. 17;471-482(2010) |

**[Table 2-6]**

| | | |
|---|---|---|
| histone deacetylase | 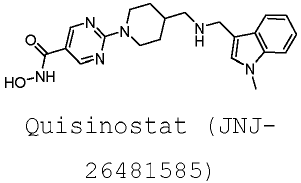 | Arts J. et al., Clin. Cancer Res.15;6841-6851(2009) |
| lysine methyltarans-ferase | 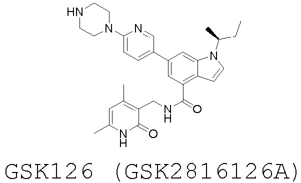 | McCabe MT et al., Nature.492;108-112(2012) |
| methyl-lysine binding protein | 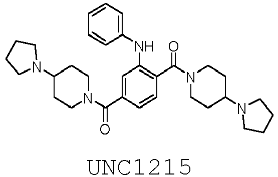 | James LI.et al., Nat.Chem.Biol.9;184-191(2013) |
| lysine demethylase | 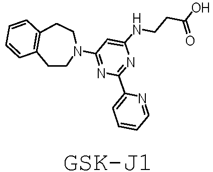 | Kruidenier L. et al., Nature.488;404-408(2012) |
| phosphodieste rase 4 (PDE4) | 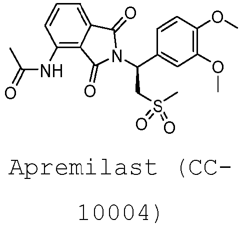 | Chen LG.et al., J.Chromatogr.Sci.54; 1336-1340(2016) |
| BCL2 | 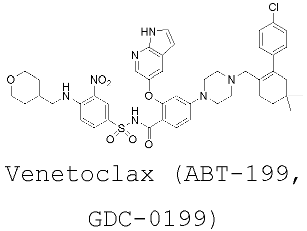 | Souers AJ.et al., Nat.Med.19;202-208 (2013) |

In one embodiment, non-kinases include, but are not limited to:
<G protein-coupled receptor (GPCR)>
   muscarinic acetylcholine receptor, adenosine receptor, adrenalin receptor, GABA receptor, angiotensin receptor, cannabinoid receptor, cholestokinin receptor, dopamine receptor, orexin receptor, glucagon receptor, histamine receptor, olfactory receptor, opioid receptor, rhodopsin receptor, secretin receptor, serotonin receptor, somatostatin receptor, gastrin receptor, P2Y receptor, chemokine receptor
<Ion channel>
   potassium channel, sodium channel, calcium channel, chlorine channel, cation channel
<Cytokine receptor>
   IL-2 receptor, IL-3 receptor, IL-4 receptor, IL-5 receptor, IL-6 receptor, IL-7 receptor, IL-9 receptor, IL-11 receptor, IL-12 receptor, IL-13 receptor, IL-15 receptor, granulocyte macrophage colony stimulating factor receptor, humans granulocyte colony stimulating factor receptor, erythropoietin receptor, thrombopoietin receptor, leukemia inhibitory factor receptor, oncostatin M receptor, ciliary neurotrophic factors receptor, growth hormone receptor, leptin receptor, interferon receptor, IL-10 receptor, tumor necrosis factor receptor, Fas ligand receptor, CD40 L receptor, inhibin receptor, IL-8 receptor, IL-16 receptor, eotaxin receptor, rantes receptor, IL-1 receptor
<Growth factor and its receptor>
   epidermal growth factor, insulin-like growth factor, transforming growth factor, basic fibroblast growth factor, nerve growth factor, brain-derived neurotrophic factor, vascular endothelial growth factor, granulocyte colony stimulating factor, granulocyte macrophage colony stimulating factor, platelet-derived growth factor, erythropoietin, thrombopoietin, hepatocyte growth factor
<Transporter>
   ligand-gated ion channel, voltage-gated ion channel, phosphorylation-gated ion channel, temperature-sensitive ion channel, mechano-sensitive ion channel, ion pump, ABC transporter, secondary active transporter
<Nuclear receptor and its ligand>
   thyroid hormone receptor, retinoic acid receptor, peroxisome proliferator-activated receptor, Rev-ErbA, RAR-related orphan receptor, liver X receptor, farnesoid X receptor, vitamin D receptor, pregnane X receptor, constitutive androstane receptor, hepatocyte nuclear factor 4, retinoid X receptor, TXL/PNR, chicken ovalbumin upstream promoter, V-erbA-related protein, estrogen receptor, estrogen-related receptor, glucocorticoid receptor, mineralocorticoid receptor, progesterone receptor, androgen receptor, nerve growth factor IB, nuclear receptor-related 1 protein, neuron-derived orphan receptor-1, steroidogenic factor-1, liver receptor homolog-1, germ cell nuclear factor, DAX1, SHP, PPARγ
<Phosphatase>
   CD45, DUSP, HEPTP, LMPTPA, LMPTPB, PP1a, PP1b, PP2A, PP1B, PTPb, PTPMRG1, PTPMEG2, SHP1, SHP2, TCPTP, VHR, YopH
<Protease>
   ADAM10, collagenase, MMP-2, MMP-8, MMP-9, MMP-12, MMP-13, caspase (1-12), cathepsin (B, C, F, H, K, L, O, S, V, W, X), HTRA1
<Epigenetic protein and enzyme>
   histone acetyltransferase, histone deacetylase, bromo domain protein, lysine methyltransferase (DOT1 L, EZH2), lysine demethylase, methyl-lysine binding protein, signal transducer and activator of transcription, BAF, CDC20, MYC
<Phosphodiesterase>
   PDE1, PDE2, PDE3, PDE4, PDE5, PDE6, PDE7, PDE8, PDE9, PDE10, PDE11
<Ubiquitin-related protein, central-related protein, and oncogene-related protein>
   ubiquitin, ubiquitin-like protein, DUB, BCL2, BRCA1, BRCA2, FANCA, FANCD2, FANCE, Her3, heat shock protein, amyloid β, α-synuclein, tau protein, huntingtin protein, TPD-43, cellular retinoic acid-binding protein, TACC3, parkin

In one embodiment, non-kinases include, but are not limited to estrogen receptor, androgen receptor histone acetyltransferase, bromo domain protein, lysine methyltransferase, phosphodiesterase, BCL2, BCL6, signal transducer and activator of transcription α-synuclein, tau protein, huntingtin protein, cellular retinoic acid-binding protein, TACC3, CDC20, and MYC.

In one embodiment, non-kinases include, but are not limited to chemokine receptor, sodium channel, epidermal growth factor, insulin-like growth factor, transforming growth factor, basic fibroblast growth factor, nerve growth factor, brain-derived neurotrophic factor, vascular endothelial growth factor, granulocyte colony stimulating factor, granulocyte macrophage colony stimulating factor, platelet-derived growth factor, hepatocyte growth factor, IL-2 receptor, IL-3 receptor, IL-4 receptor, IL-5 receptor, IL-6 receptor, IL-7 receptor, IL-9 receptor, IL-11 receptor, IL-12 receptor, IL-13 receptor, IL-15 receptor, granulocyte macrophage colony stimulating factor receptor, humans granulocyte colony stimulating factor receptor, leukemia inhibitory factor receptor, interferon receptor, IL-10 receptor, tumor necrosis factor receptor, Fas ligand receptor, CD40 L receptor, IL-8 receptor, rantes receptor, IL-1 receptor, estrogen receptor, androgen receptor, thyroid hormone receptor, caspase, histone acetyltransferase, histone deacetylase, bromo domain protein, lysine methyltransferase, lysine demethylase, methyl-lysine binding protein, PDE3, PDE4, PDE5, PDE11, ubiquitin, ubiquitin-like protein, DUB, BCL2, BCL6, BRCA1, BRCA2, FANCA, FANCD2, FANCE, Her3, and heat shock protein, PPARγ, signal transducer and activator of transcription, cellular retinoic acid-binding protein, BAF, CDC20, MYC, and TACC3.

In one embodiment, non-kinases include, but are not limited to chemokine receptor, vitamin D receptor, phosphodiesterase, and heat shock protein, and signal transducer and activator of transcription.

The embodiments of Z in the above Formula (A) are as follows.

One embodiment of the intracellular protein in Z may be a serin/threonine kinase, a receptor tyrosine kinase, or a non-receptor tyrosine kinase.

One embodiment of the intracellular protein in Z may be a serin/threonine kinase, and the serin/threonine kinase may be selected from the group consisting of AGC group, CAMK group, CK1 group, CMGC group, STE group, TKL group, and other serinthreonine kinases.

Here, the serin/threonine kinase may be AGC group, and the AGC group may be ROCK2.

Examples of a ligand moiety capable of specifically binding to ROCK2 include

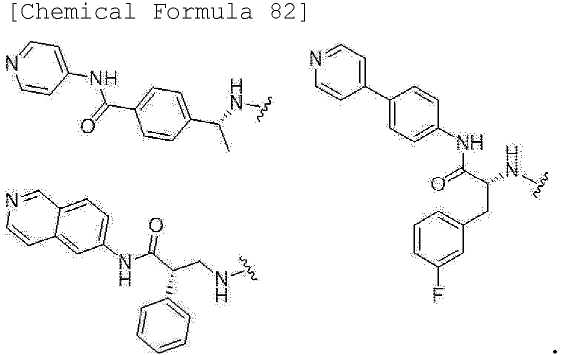

One embodiment of the intracellular protein in Z may be a receptor tyrosine kinase, and the receptor tyrosine kinase may be selected from the group consisting of epidermal growth factor receptor family, insulin receptor family, platelet-derived growth factor family, vascular endothelial growth factor receptor family, fibroblast growth factor receptor family, CCK family, nerve growth factor receptor family, hepatocyte growth factor receptor family, Eph receptor family, AXL receptor family, TIE receptor family, RYK receptor family, DDR receptor family, RET receptor family, ROS receptor family, LTK receptor family, ROR receptor family, MuSK receptor family, LMR receptor family, and other receptor tyrosine kinases.

Here, the receptor tyrosine kinase may be LTK receptor family, and the LTK receptor family may be ALK.

Examples of a ligand moiety capable of specifically binding to ALK include

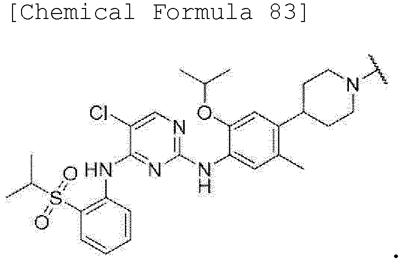

One embodiment of the intracellular protein in Z may be a non-receptor tyrosine kinase, and the non-receptor tyrosine kinase may be selected from the group consisting of ABL family, ACK family, CSK family, FAK family, FES family, FRK family, JAK family, SRC family, TEC family and SYK family.

Here, the non-receptor tyrosine kinase may be FAK family, and the FAK family may be FAK.

Examples of a ligand moiety capable of specifically binding to FAK include

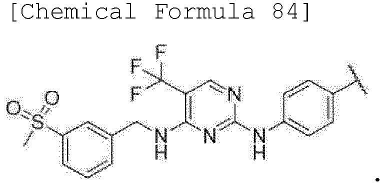

linker (L) is represented by Formula (L1):

-B-L¹-L²-L³-L⁴-L⁵-L⁶-L⁷-

wherein
each B independently represents a group represented by the following structure formula: 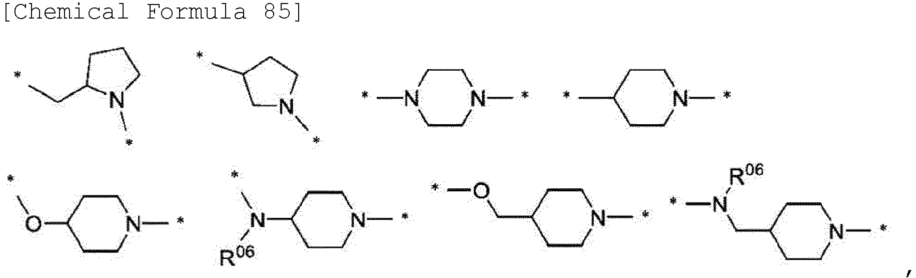
*-O-*, *-NR⁰⁶-* wherein R⁰⁶ is a hydrogen atom or a C₁₋₆ alkyl group, *-CO₂-*, *-CO-*, *-SO₂-*, an optionally substituted C₁₋₆ alkylene group, an optionally substituted C₂₋₆ alkenylene group, an optionally substituted C₂₋₆ alkynylene group, an optionally substituted C₃₋₁₀ cycloalkylene group, an optionally substituted C₆₋₁₄ arylene group, or a bond, and
L¹, L², L³, L⁴, L⁵, L⁶, and L⁷ each independently represent a group represented by the following structure formula: 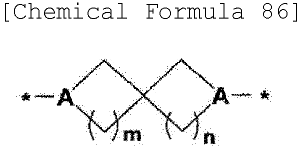
   wherein m represents an integer of 1 to 4, n represents an integer of 1 to 4, and each A independently represents N, CHCO or CHCH₂O,
   provided that cases where three or more of L¹ to L⁷ have this structure are excluded,
a bond, an oxygen atom, a sulfur atom, a C₁₋₆ alkylene group, a C₃₋₁₀ cycloalkylene group, a carbonyl group, an imino group optionally substituted with a C₁₋₆ alkyl group, an ethynylene group, a vinylene group optionally substituted with a C₁₋₆ alkyl group, a C₃₋₁₀ cycloalkenylene group, a phenylene group, a thiazolyldiyl group, a non-aromatic heterocyclic group optionally substituted with an optionally substituted C₁₋₆ alkyl group or a halogen atom, a piperazinediyl group optionally substituted with an optionally substituted C₁₋₆ alkyl group, a piperidinediyl group optionally substituted with an optionally substituted C₁₋₆ alkyl group or fluorine, a pyrrolidinediyl group optionally substituted with a fluorine atom, a morpholinediyl group optionally substituted with an optionally substituted C₁₋₆ alkyl group, an azetidinediyl group optionally substituted with a fluorine atom, formula -SO₂-, formula - CH₂CH₂O-, formula -OCH₂CH₂-, formula -COCH₂-, formula -CH₂CO-, formula -CO₂-, formula -OCO-, formula -COCHR¹⁰¹NR¹⁰²-, formula - OCH₂CHR¹⁰³NR¹⁰⁴-, formula -NR¹⁰⁵CHR¹⁰⁶CO-, formula -NR¹⁰⁷CO-, formula -CONR¹⁰⁸-, formula -SO₂NR¹⁰⁹-, formula -NR¹¹⁰SO₂-, or formula -NR¹¹¹CHR¹¹²CH₂O wherein, in the above formula, R¹⁰¹, R¹⁰³, R¹⁰⁶, and R¹¹² each independently represent a hydrogen atom, a C₁₋₆ alkyl group, a 3-guanidinopropyl group, a carbamoylmethyl group, a carboxymethyl group, a mercaptomethyl group, a 2-carbamoylethyl group, a 2-carboxyethyl group, an imidazol-4-ylmethyl group, a 4-aminobutyl group, a 2-methylthioethyl group, a benzyl group, a hydroxymethyl group, a 1-hydroxyethyl group, an indol-3-ylmethyl group, a 4-hydroxyphenylmethyl group, or a pyridylmethyl group, and R¹⁰², R¹⁰⁴, R¹⁰⁵, R¹⁰⁷, R¹⁰⁸, R¹⁰⁹, R¹¹⁰, and R¹¹¹ each independently represent a hydrogen atom or a C₁₋₆ alkyl group,
or join to form one bond. Here, * indicates a bonding point.

B is a group represented by the following structure formula: 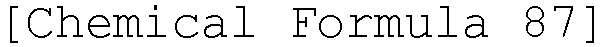 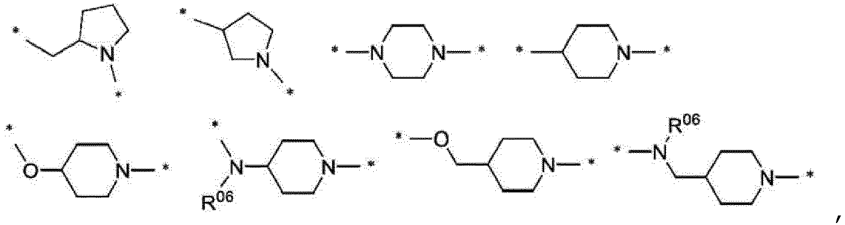 *-O-*, *-NR⁰⁶-* wherein R⁰⁶ is a hydrogen atom or a C1-6 alkyl group, *-CO-*, an optionally substituted C1-6 alkylene group, an optionally substituted C₆₋₁₄ arylene group, an optionally substituted C2-6 alkynylene group, or a bond.

Linker (L) is any group represented by the following structural formula: 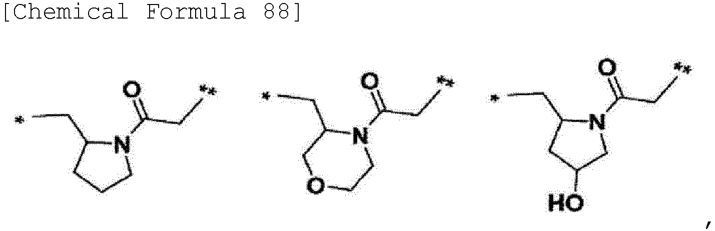 wherein * indicates a bond to X or Z, *-(CH₂CH₂O)n(CH₂)m(NRCO)s(CH₂)t-* (n is an integer of 1 to 5, m is 0, 1, or 2, s is 0 or 1, t is 0 or 1, and R is a hydrogen atom or a C₁₋₆ alkyl group), or bond, or

[Chemical Formula 89] **-N(R)-(CH2)ₘ-O(CH2)ₙ-O(CH2)ₒ-O(CH2)ₚ-O(CH2)_{q}-O(CH2)ᵣ-OCH2-, -O-(CH2)ₘ-O(CH2)ₙ-O(CH2)ₒ-O(CH2)ₚ-O(CH2)_{q}-O(CH2)ᵣ-OCH2-, -O-(CH2)ₘ-O(CH2)ₙ-O(CH2)ₒ-O(CH2)ₚ-O(CH2)_{q}-O(CH2)ᵣ-O-; -N(R)-(CH2)ₘ-O(CH2)ₙ-O(CH2)ₒ-O(CH2)ₚ-O(CH2)_{q}-O(CH2)ᵣ-O-;**

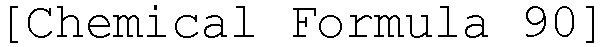

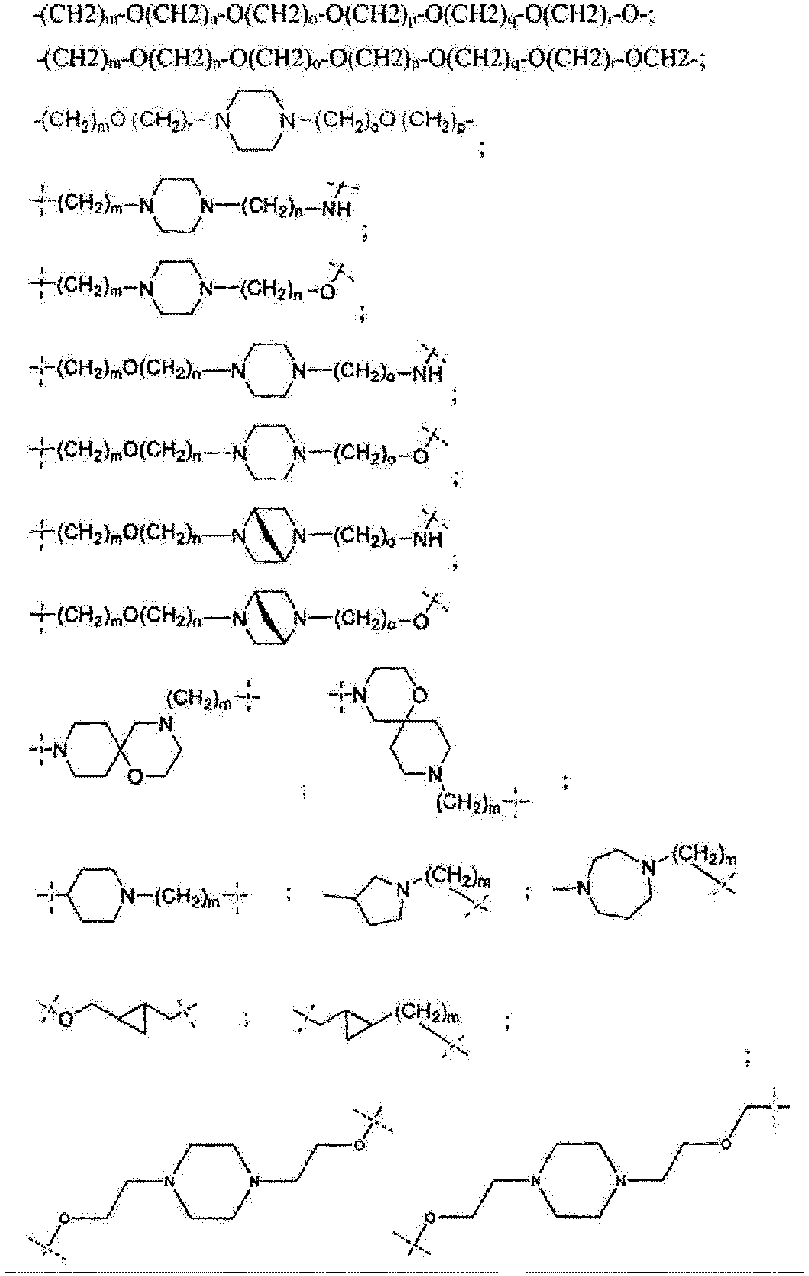

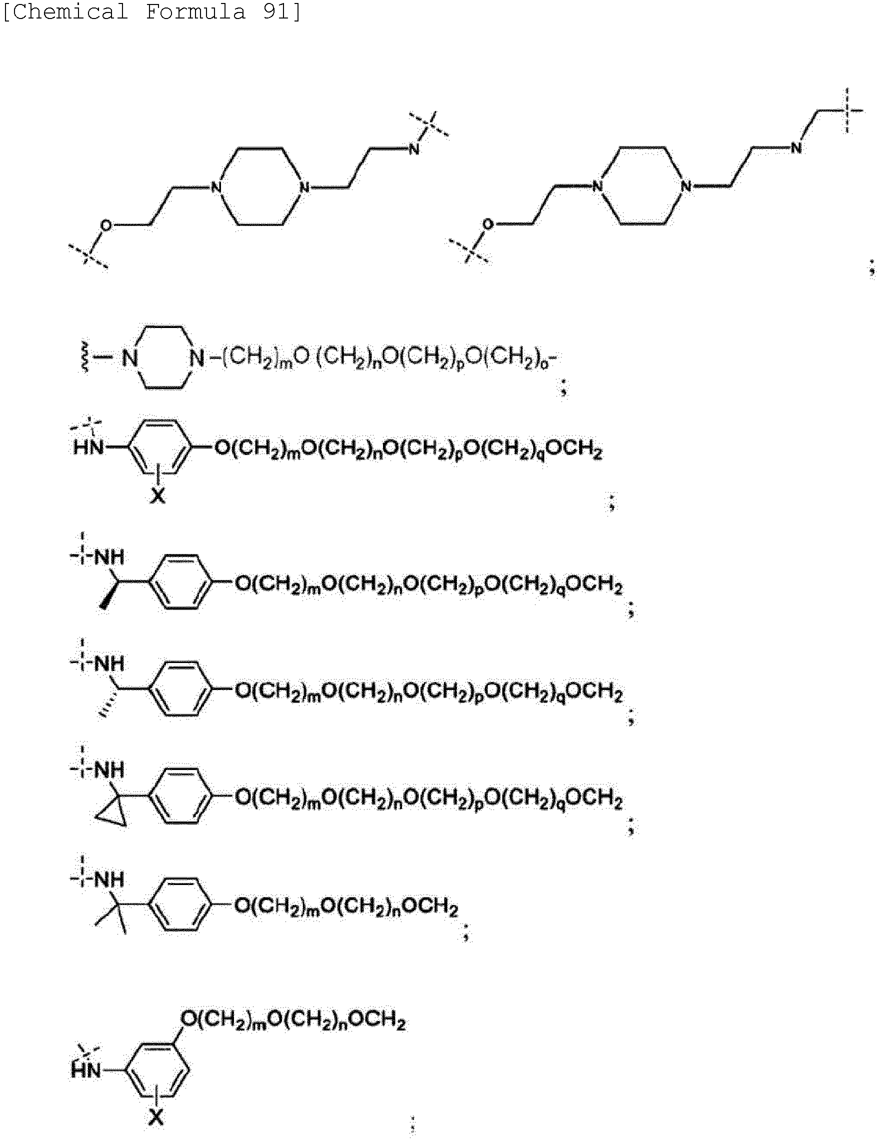

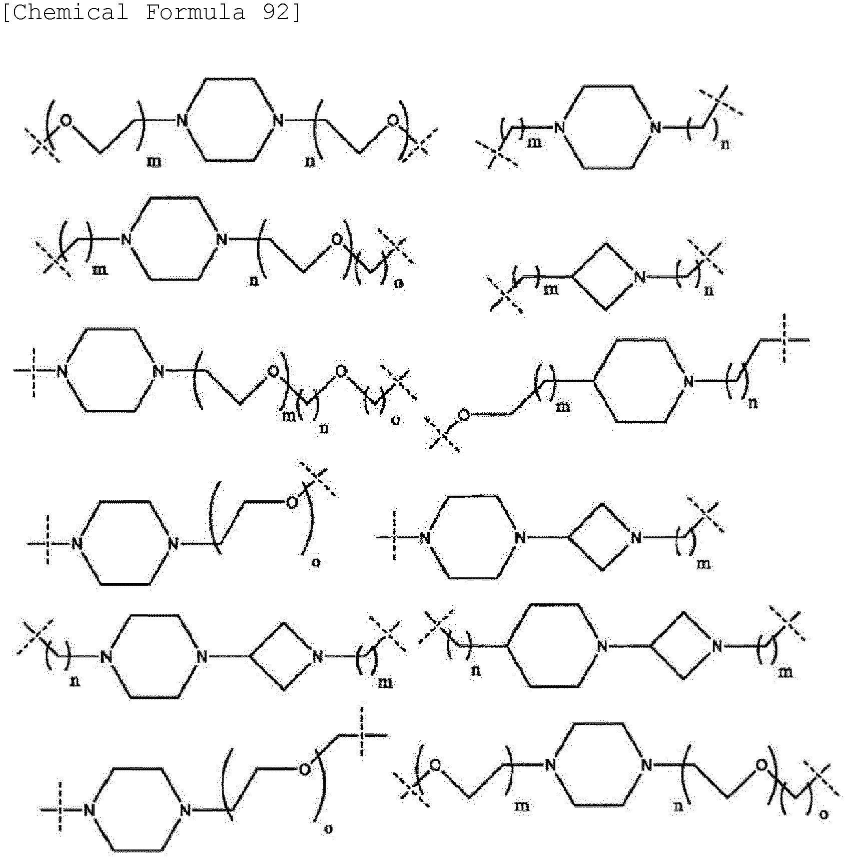

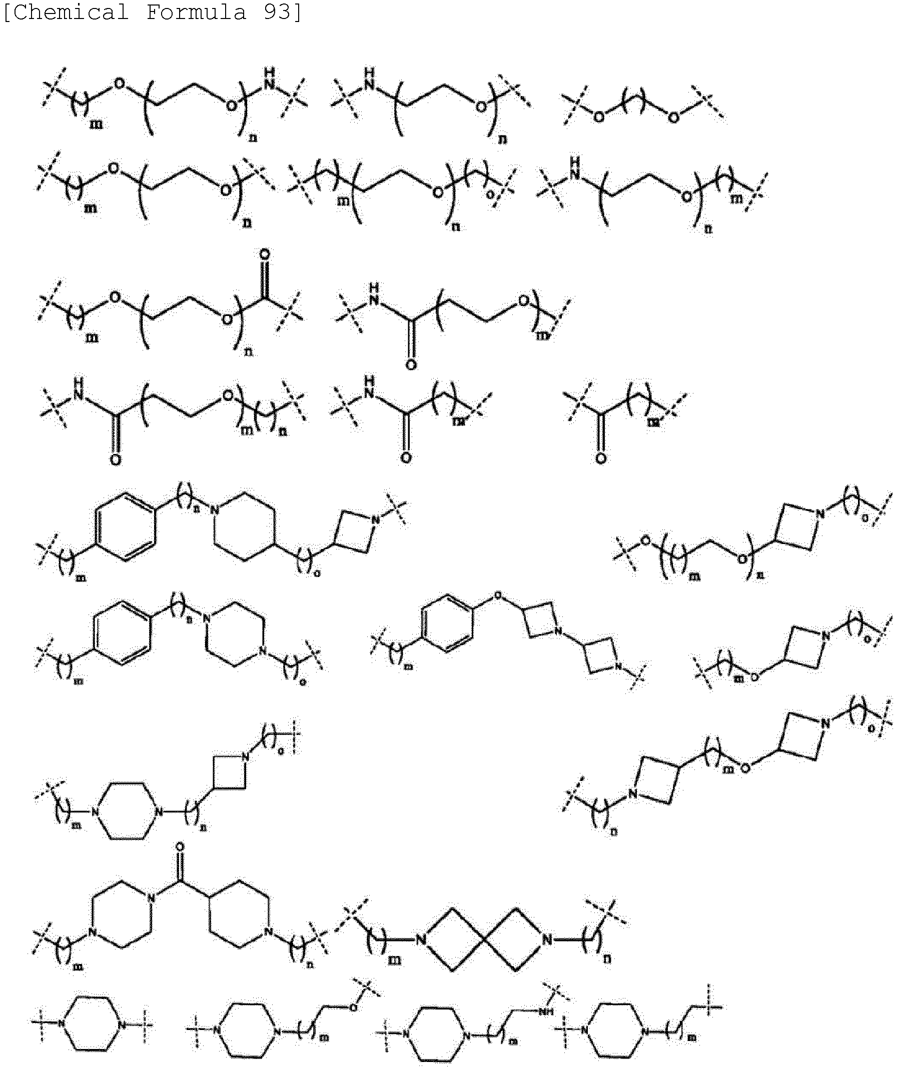

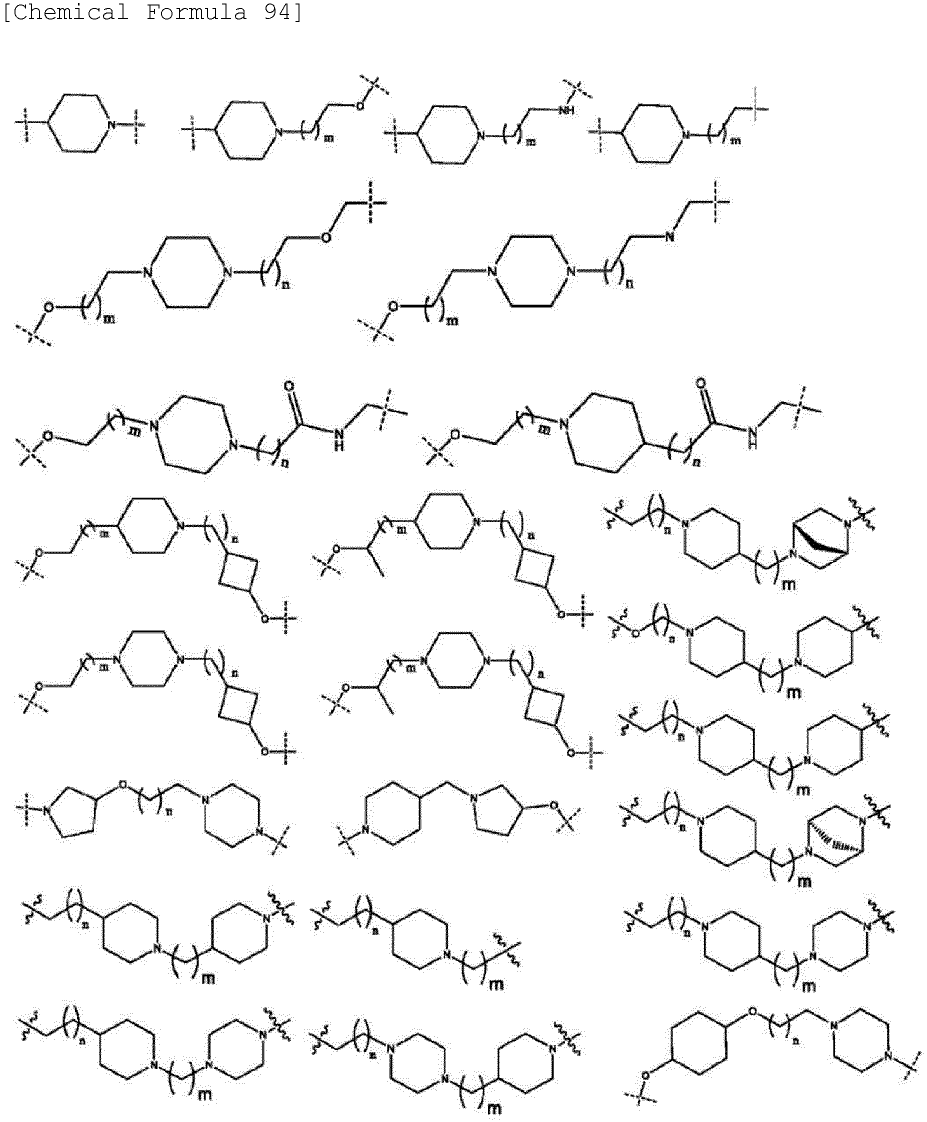

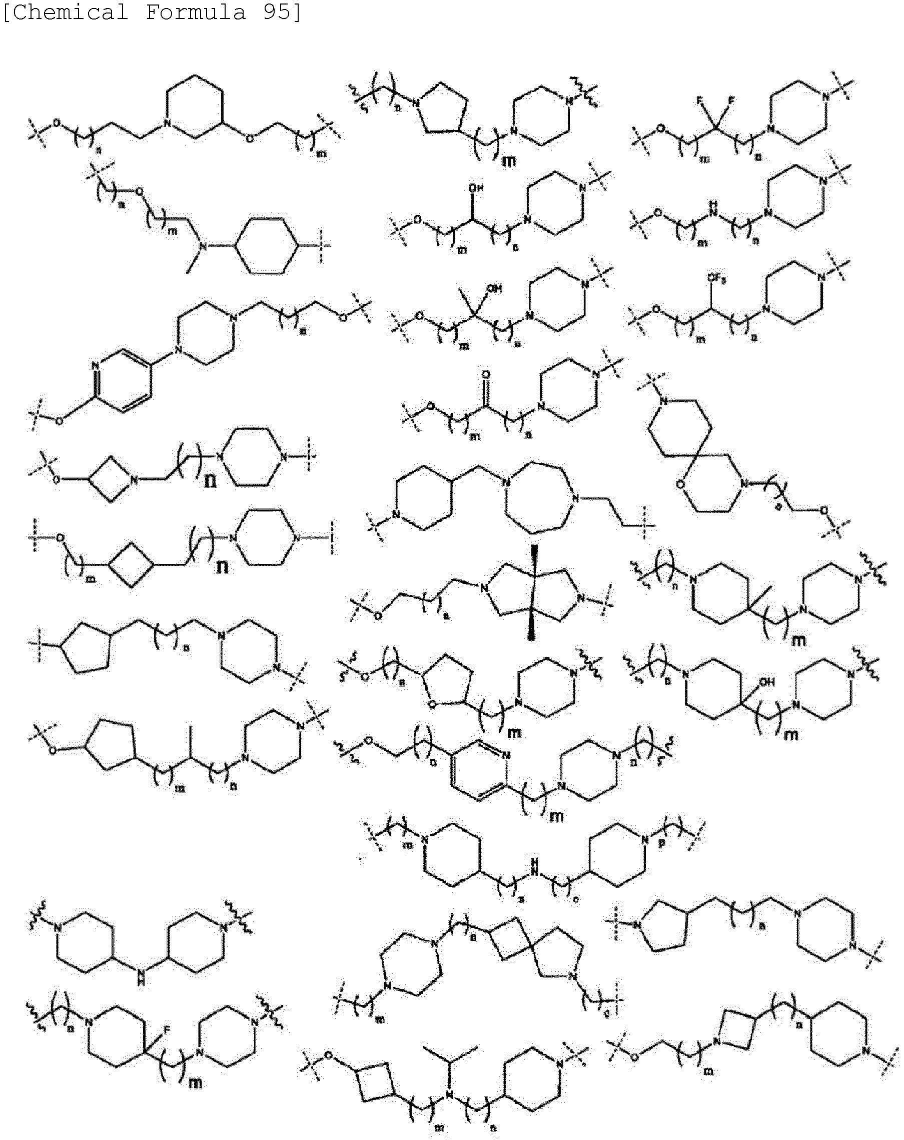

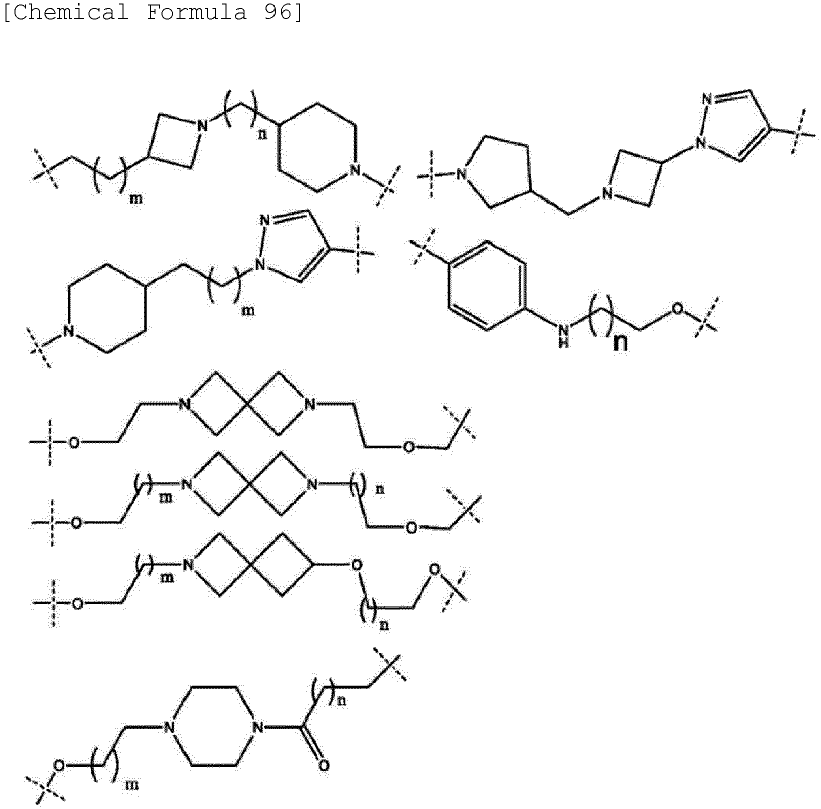

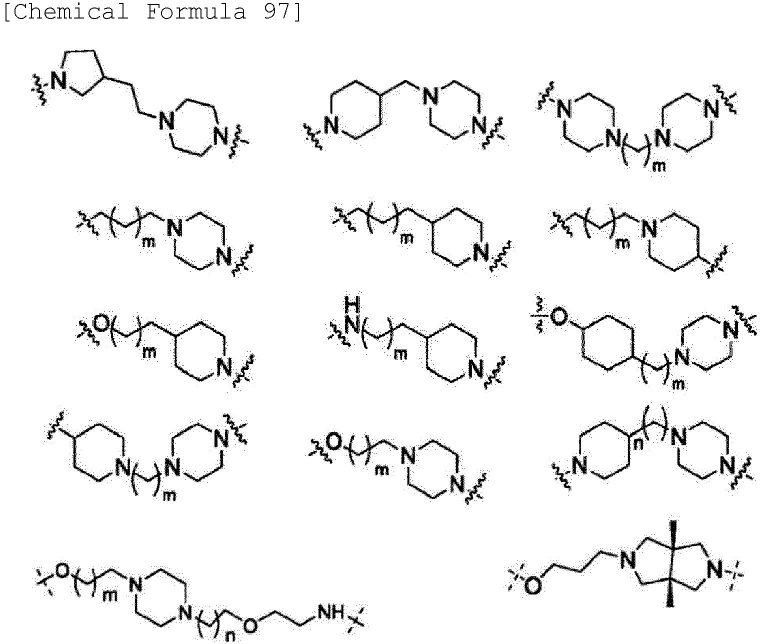

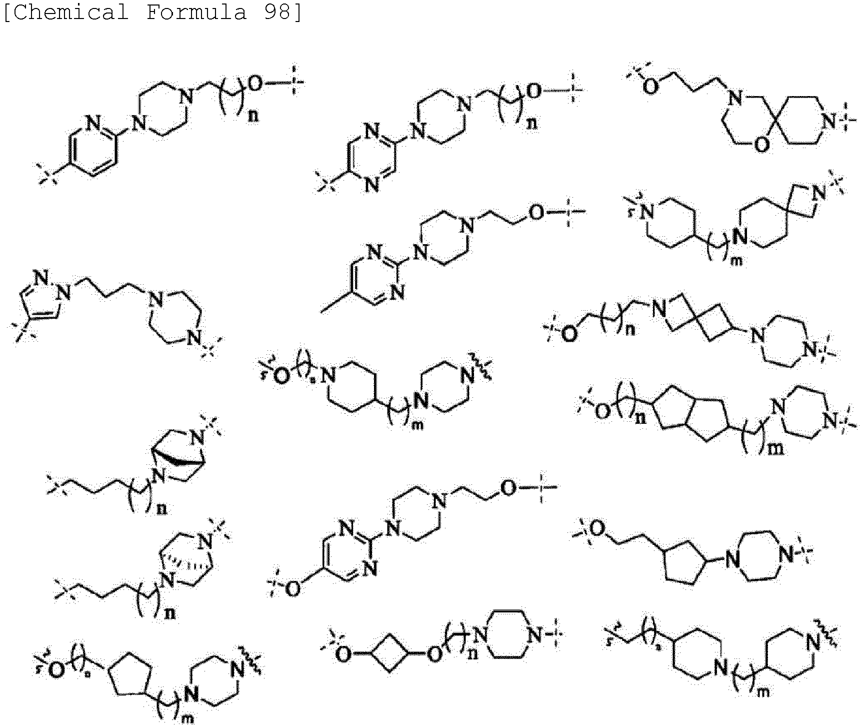

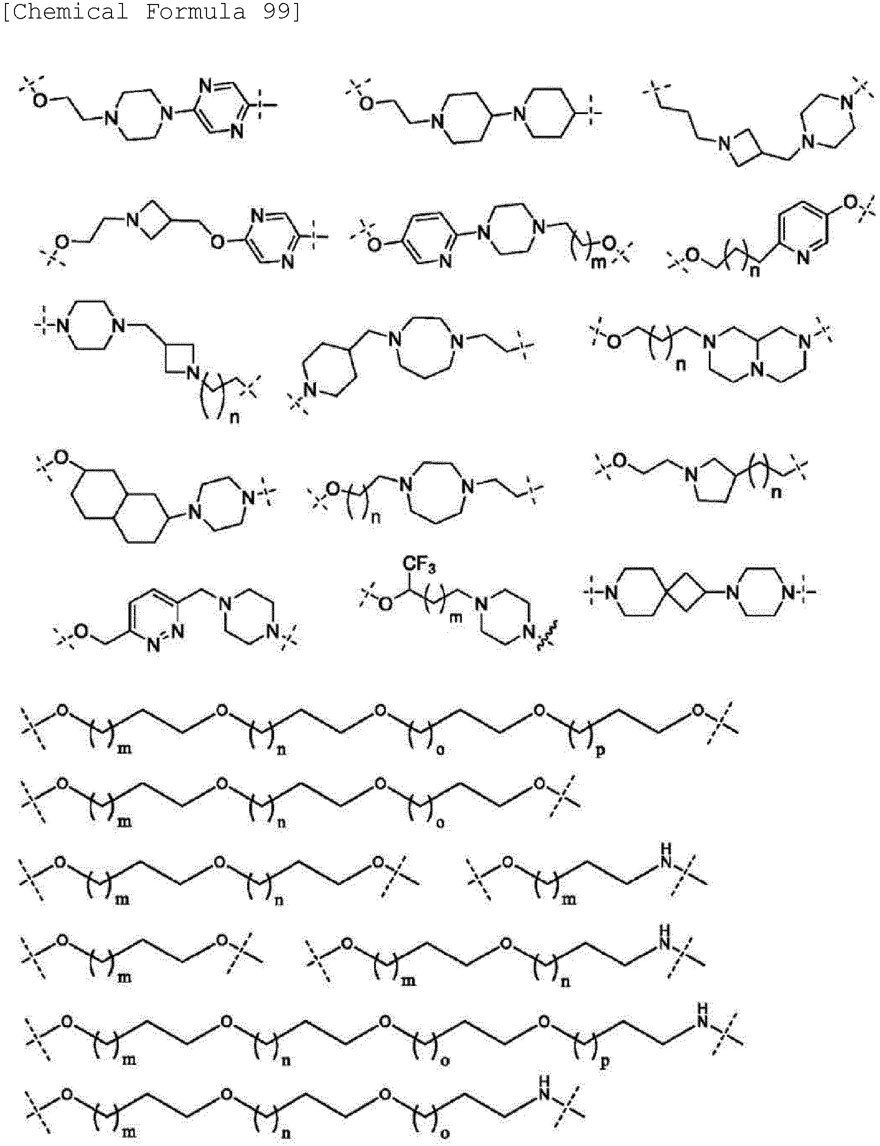

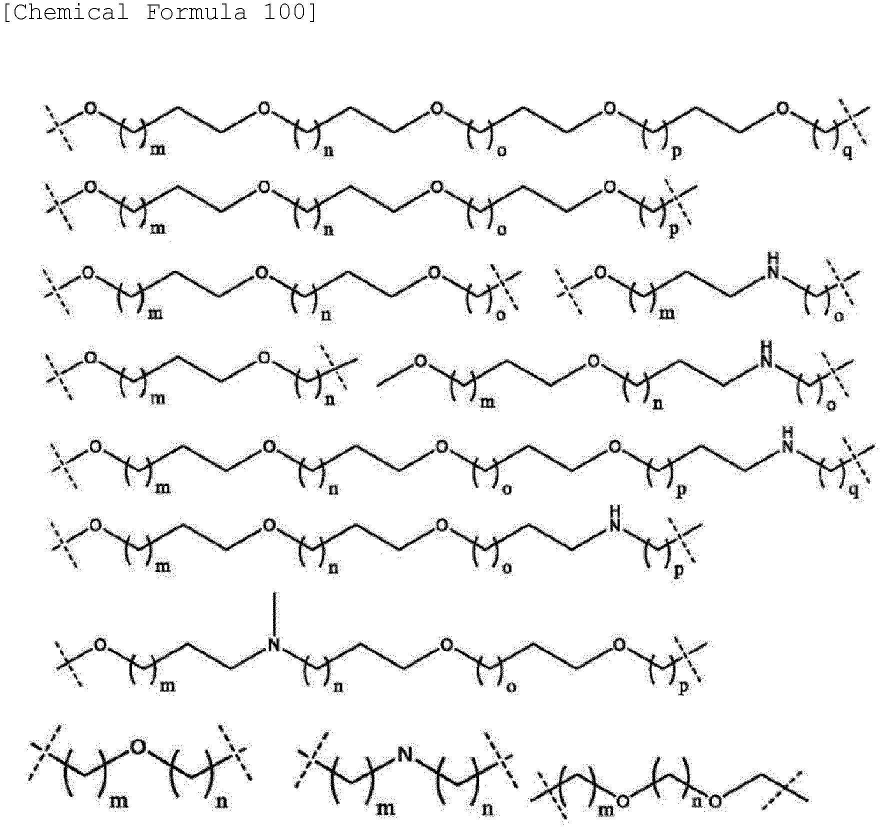

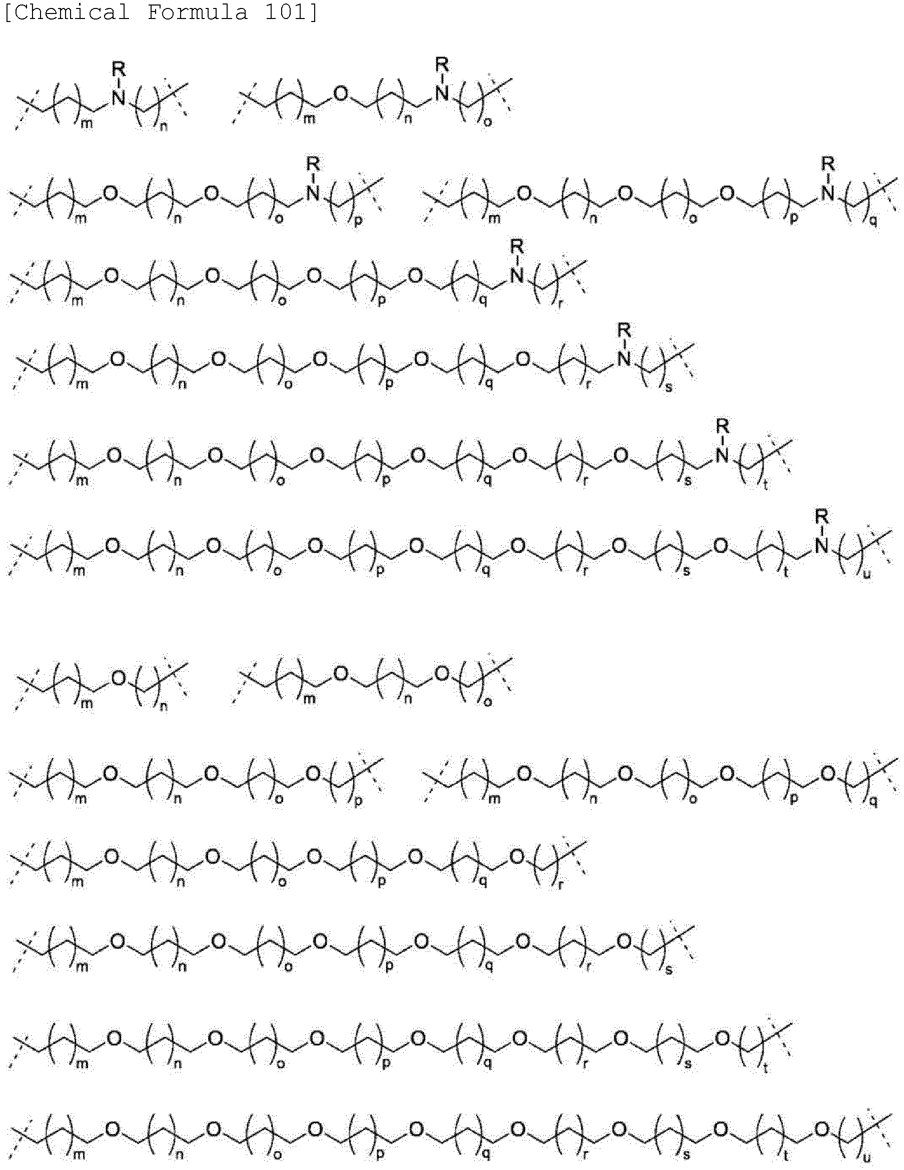

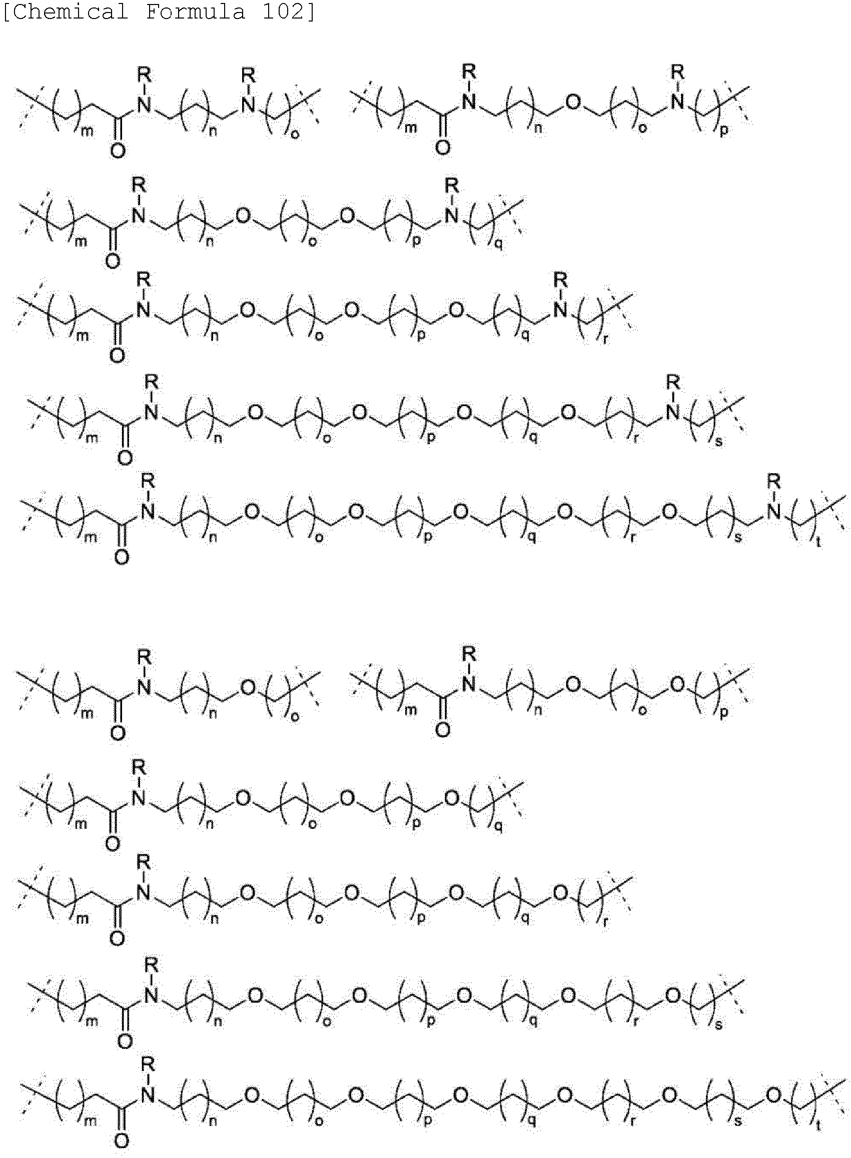

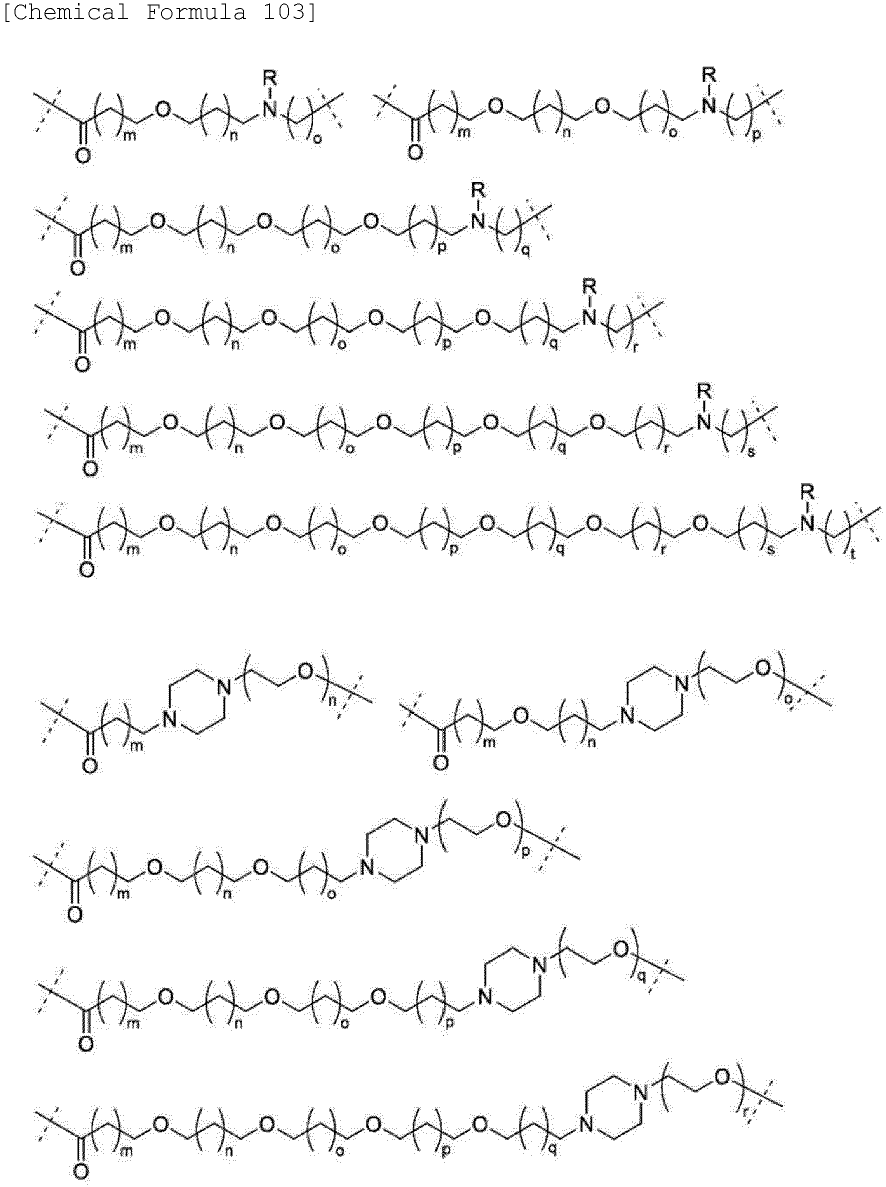

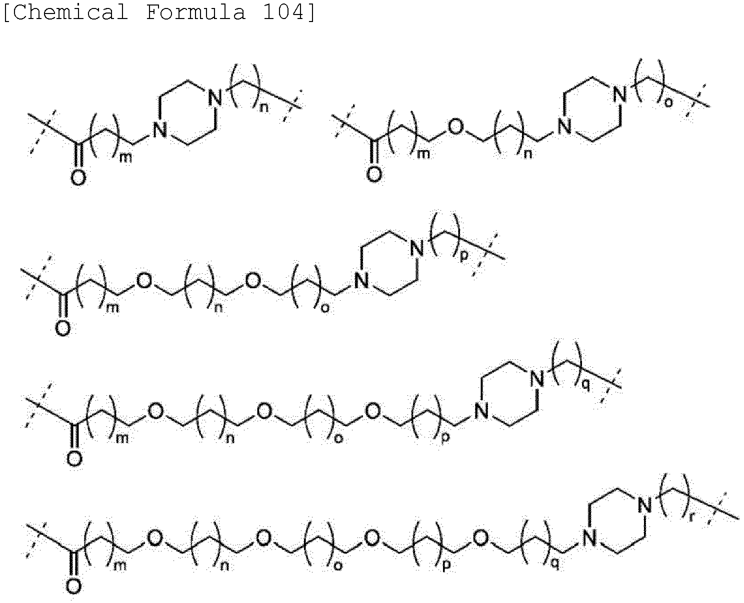

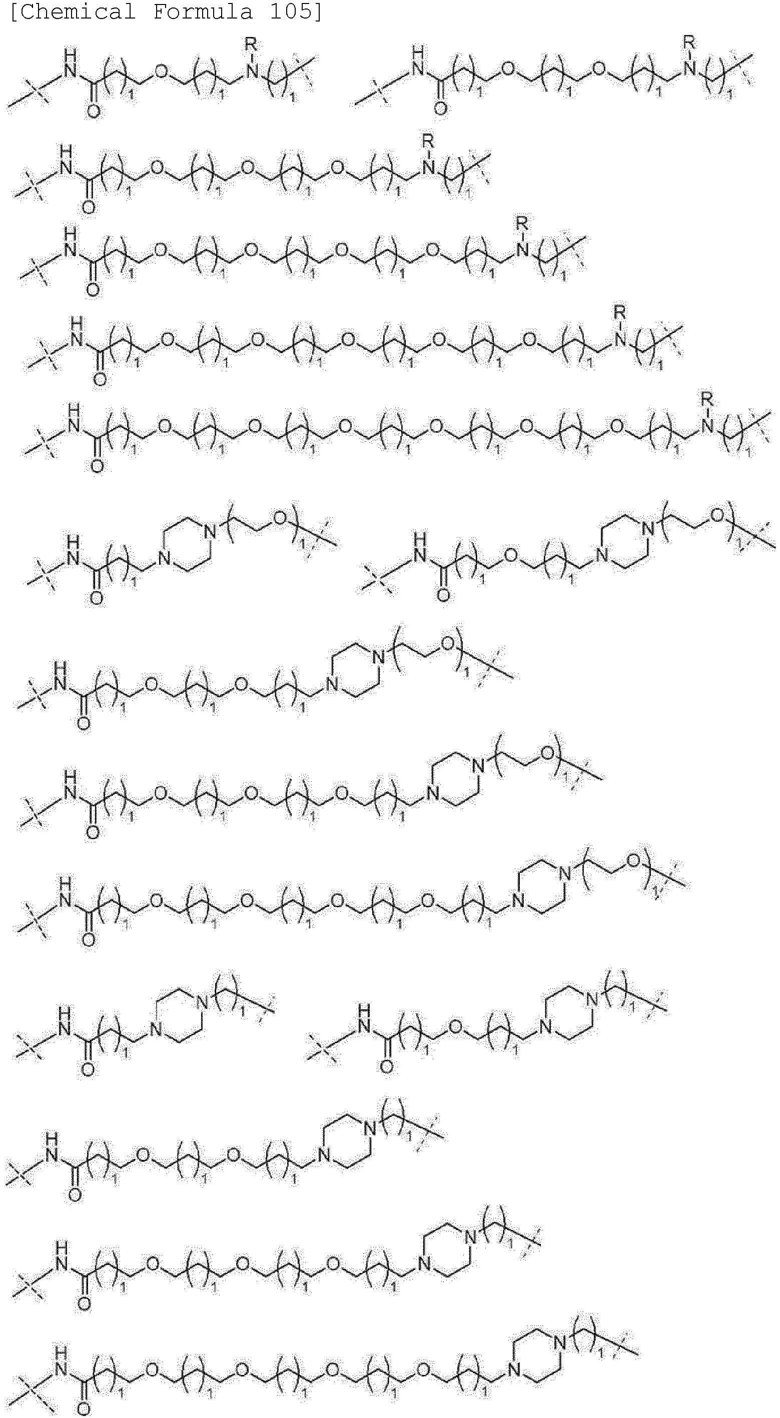

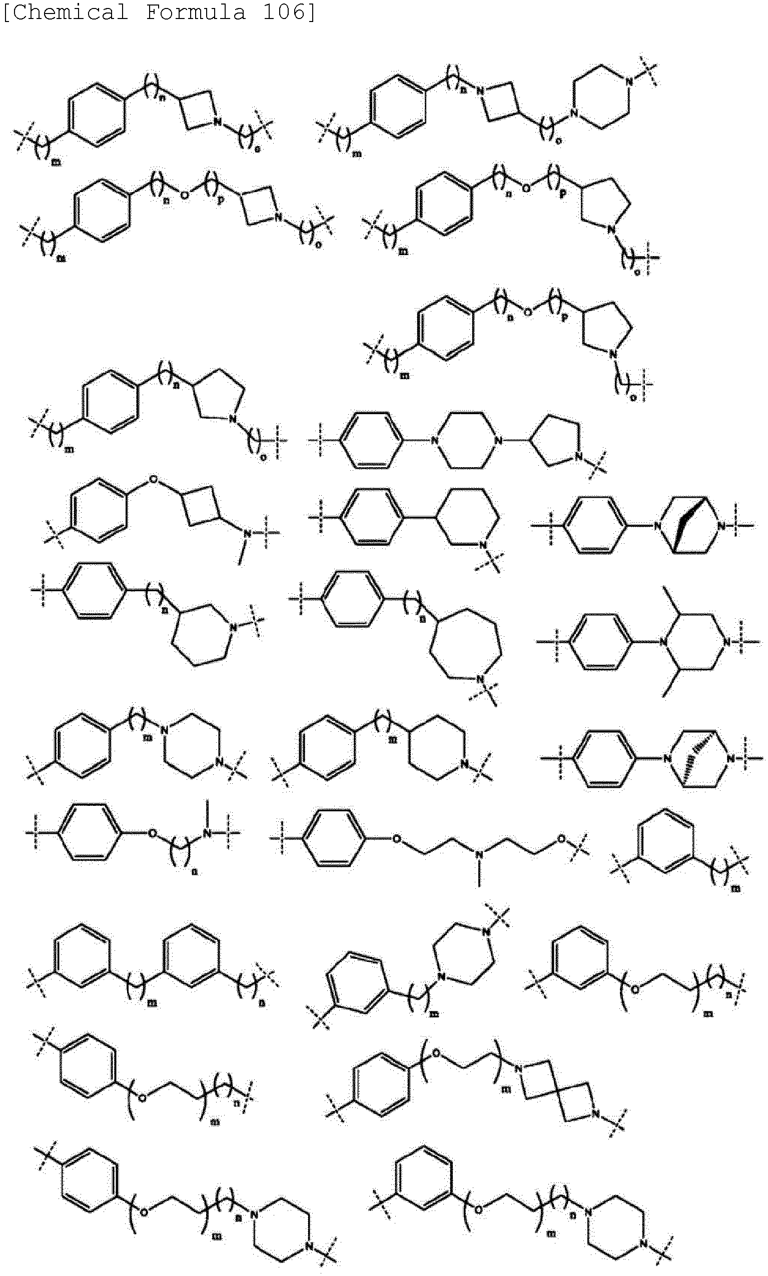

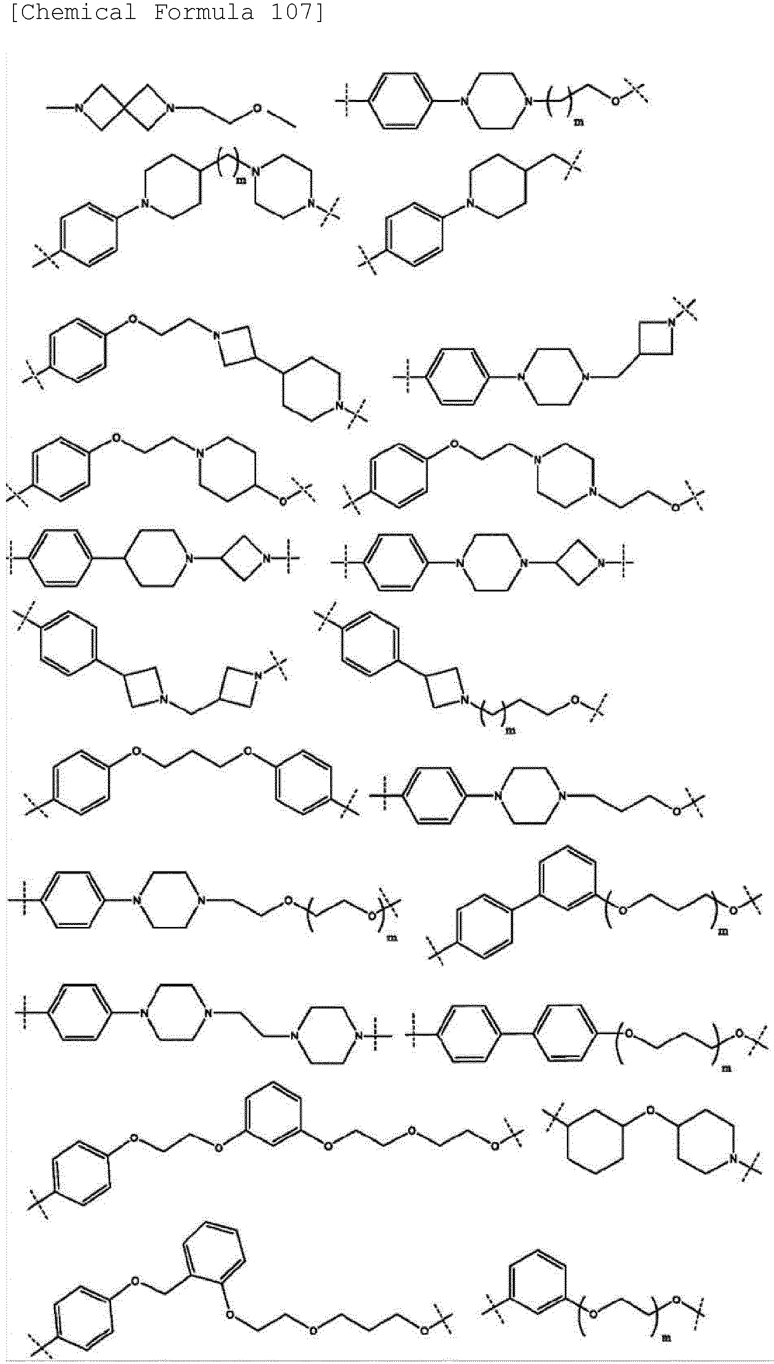

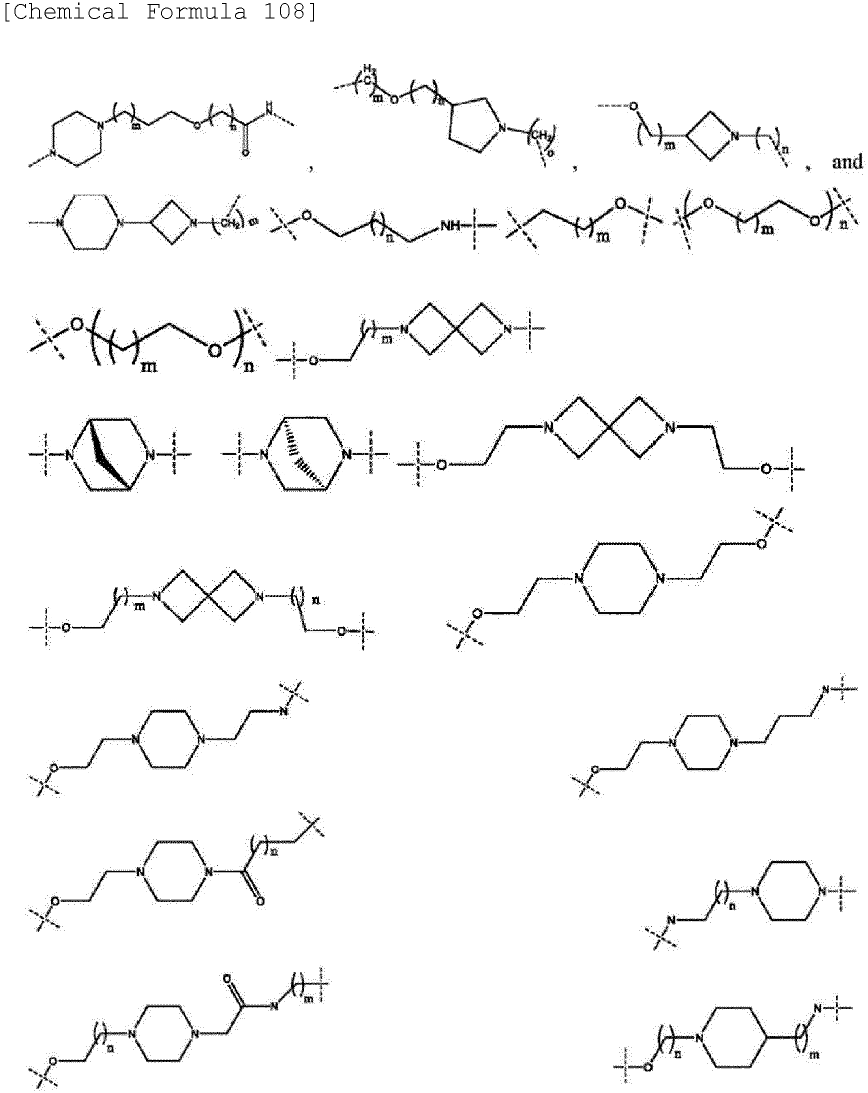

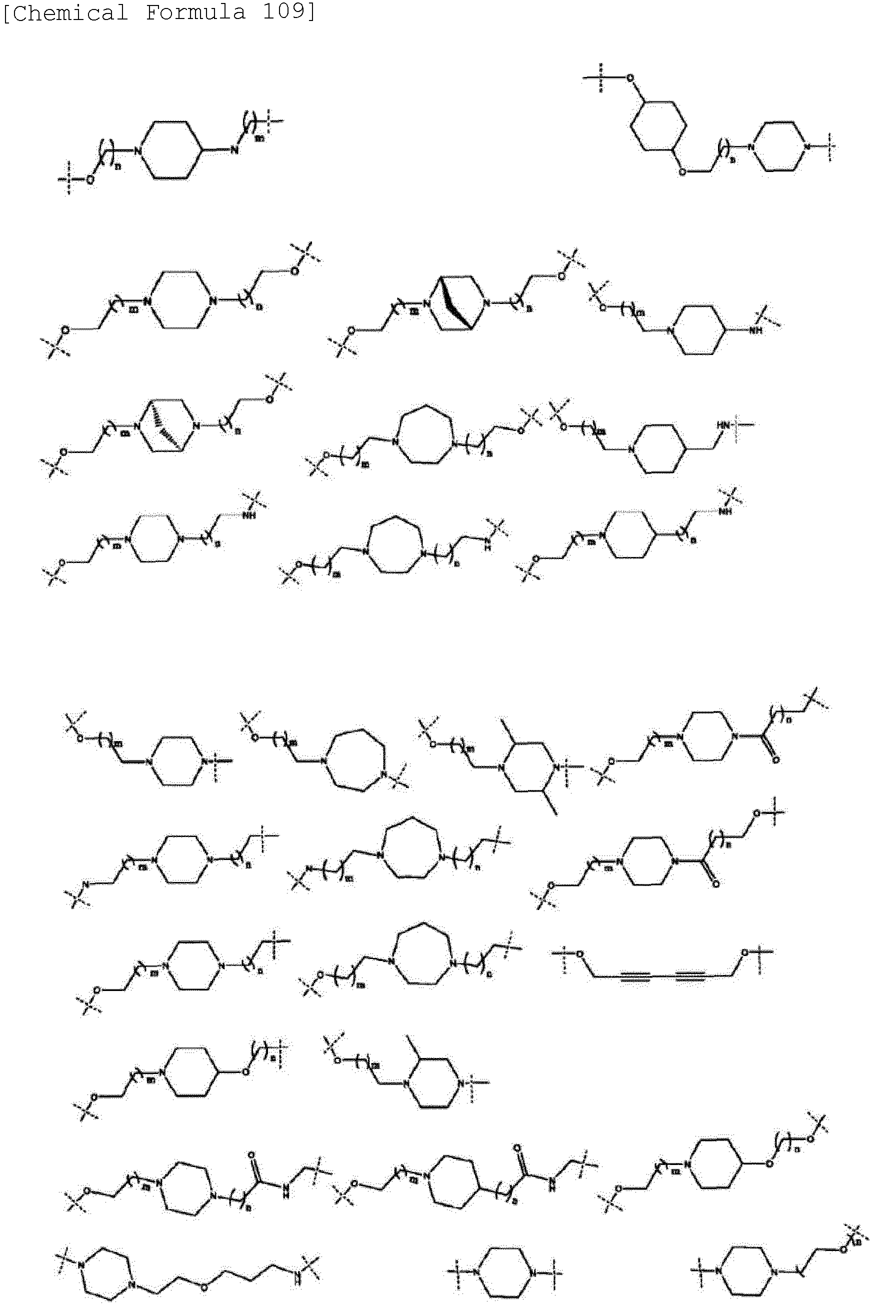

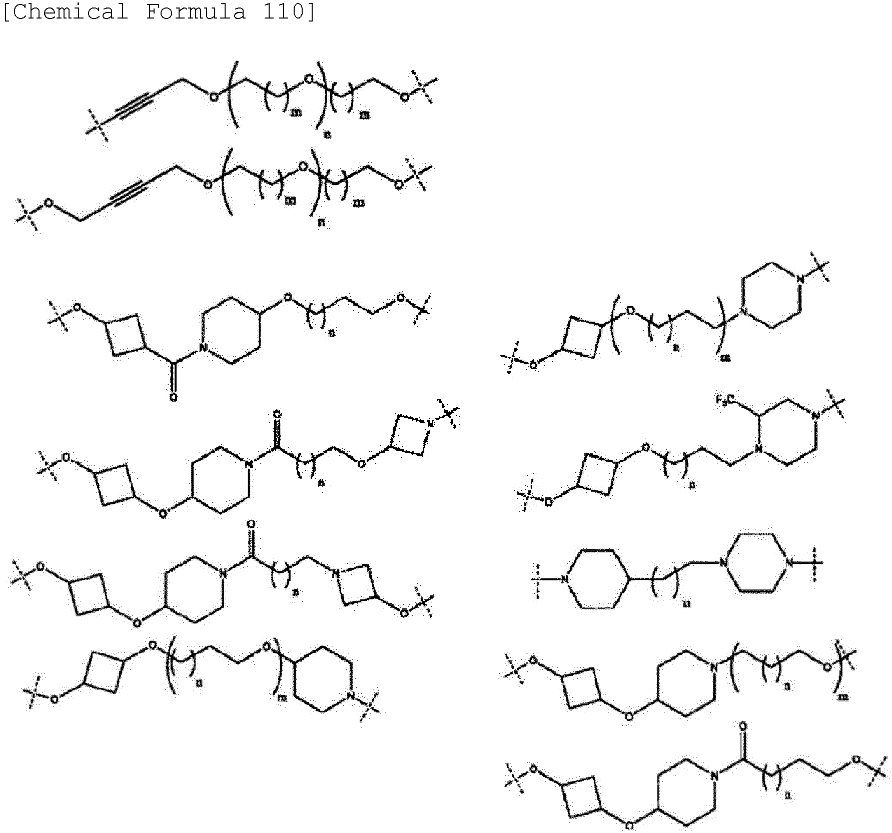

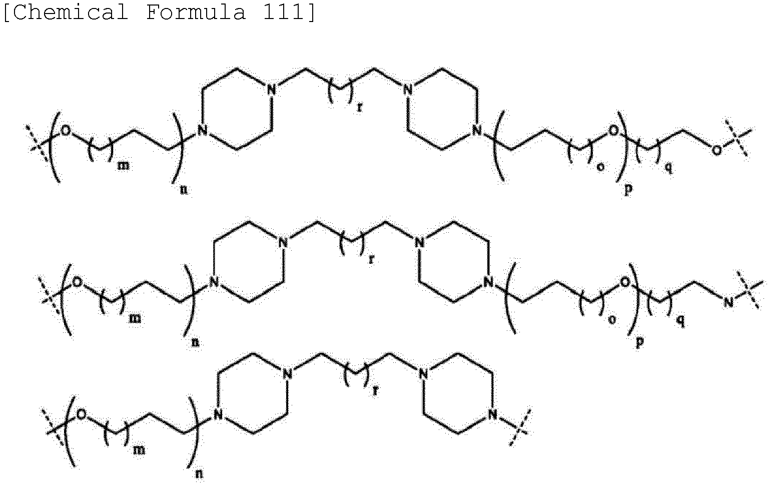

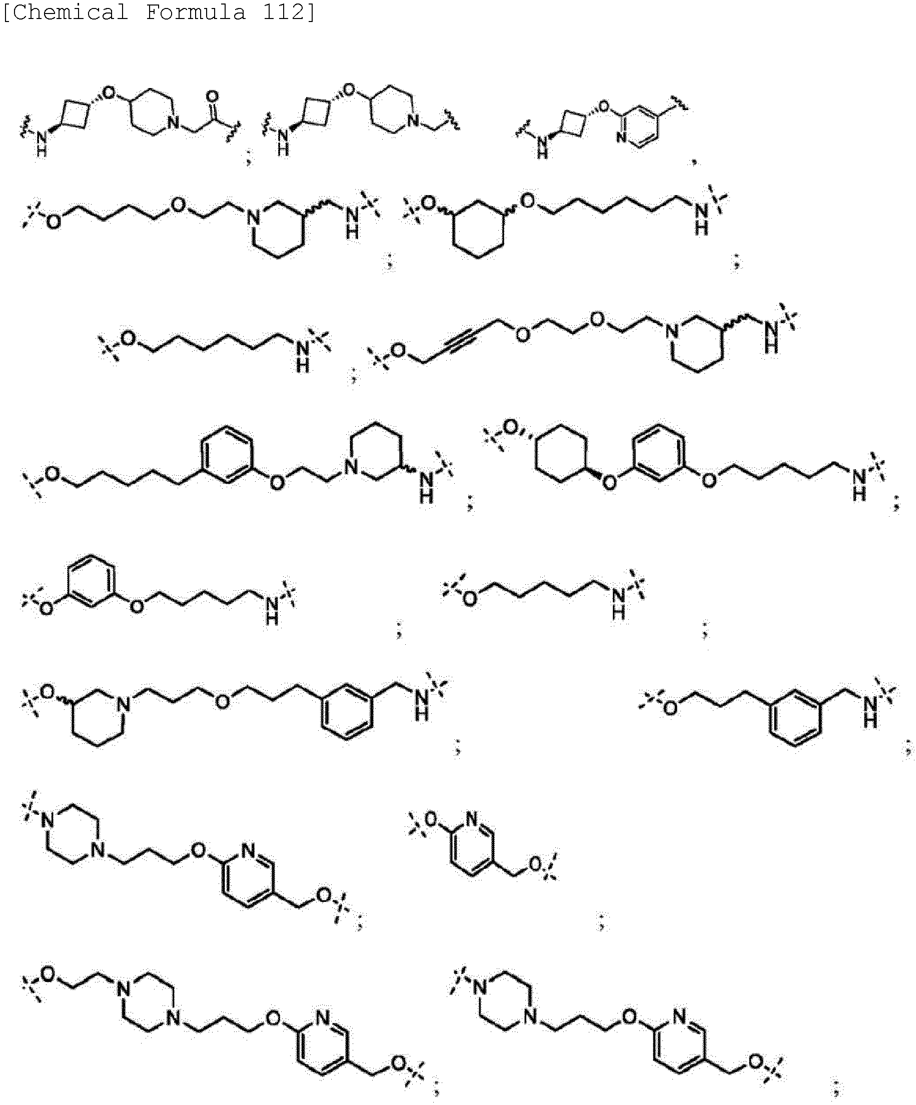

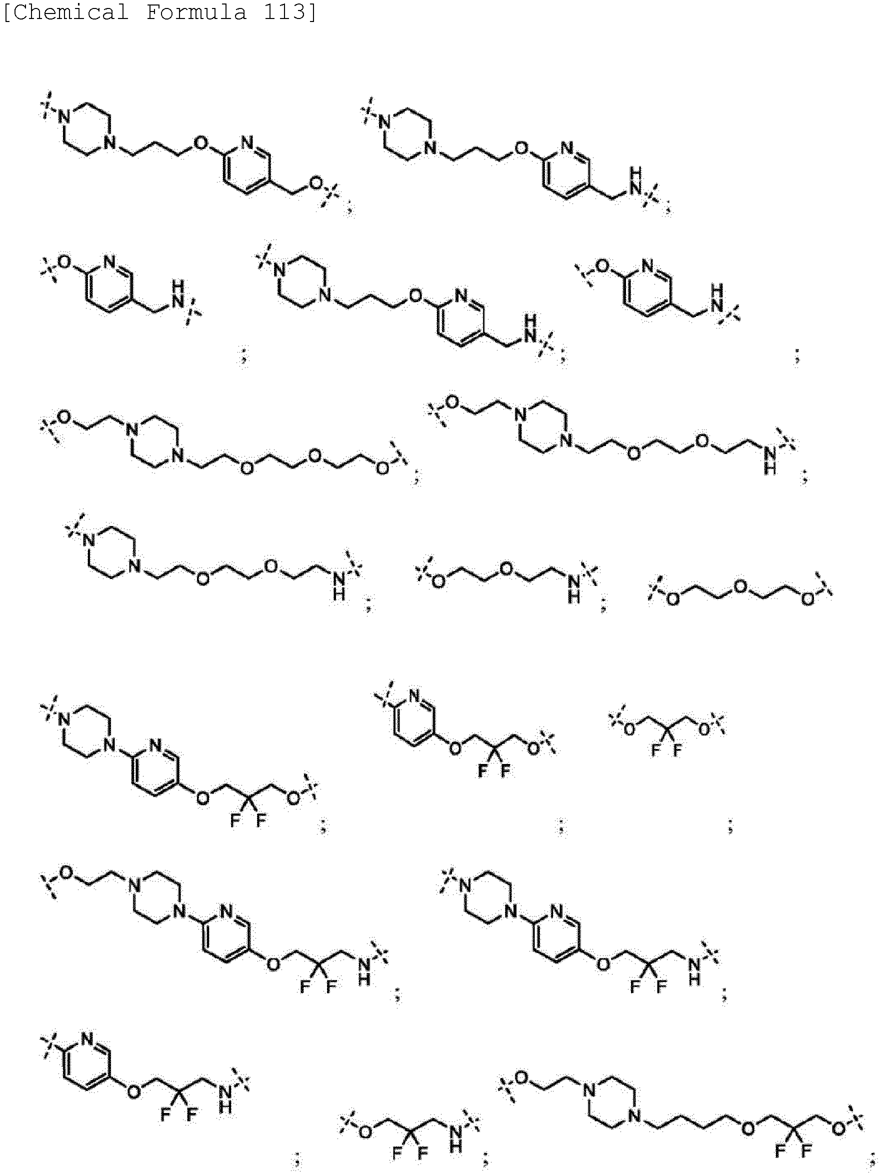

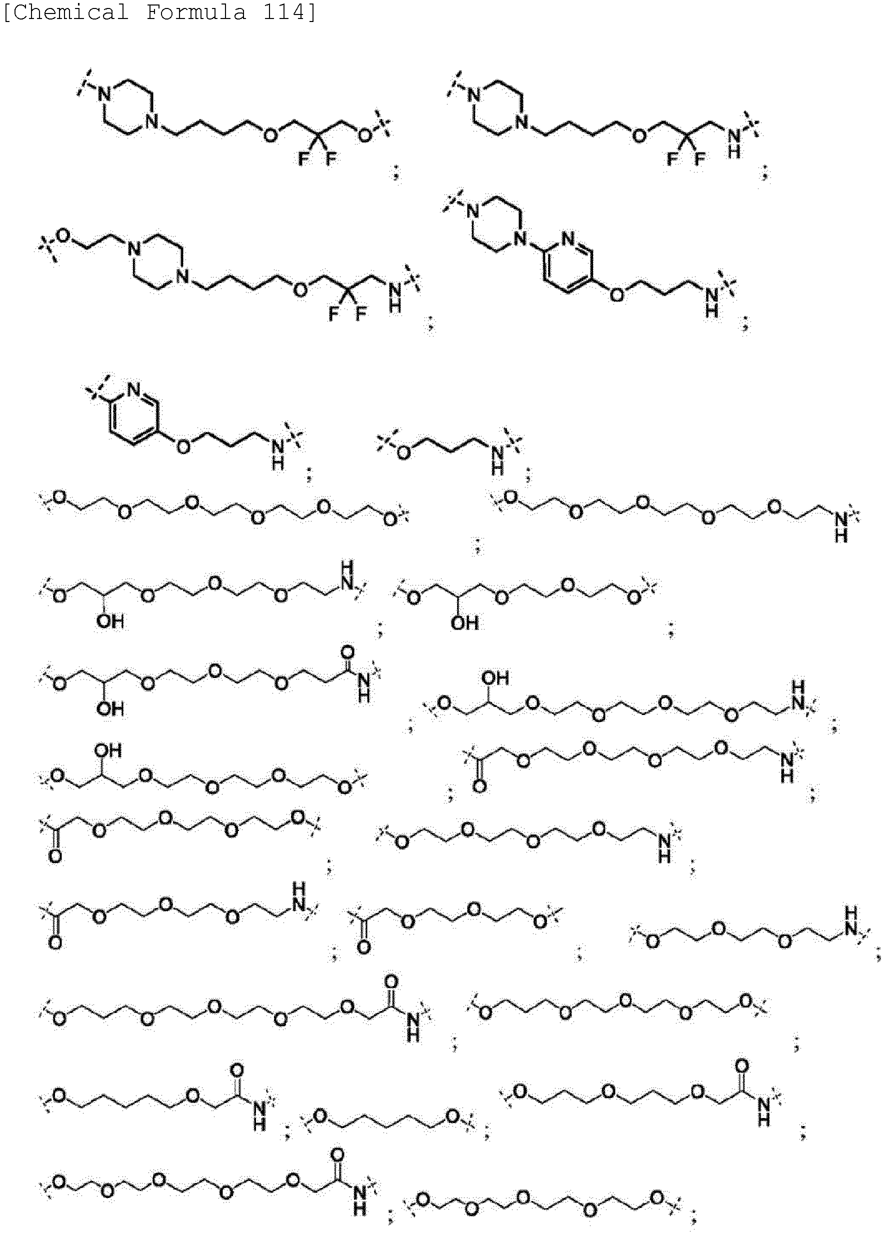

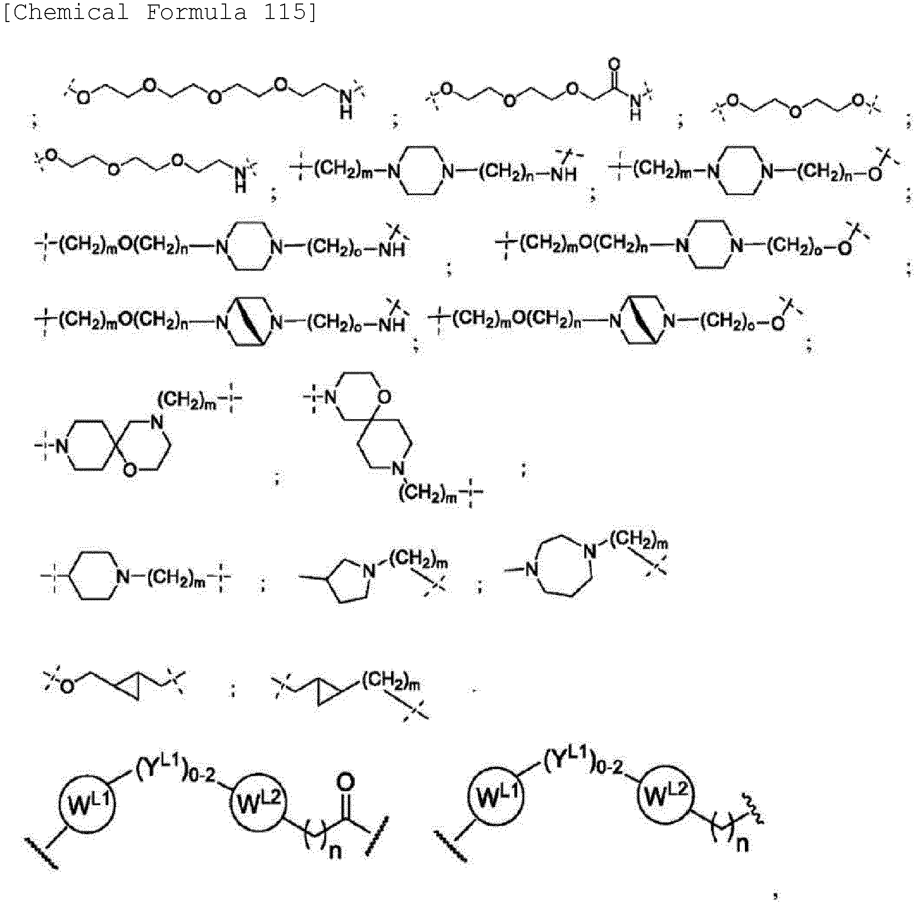

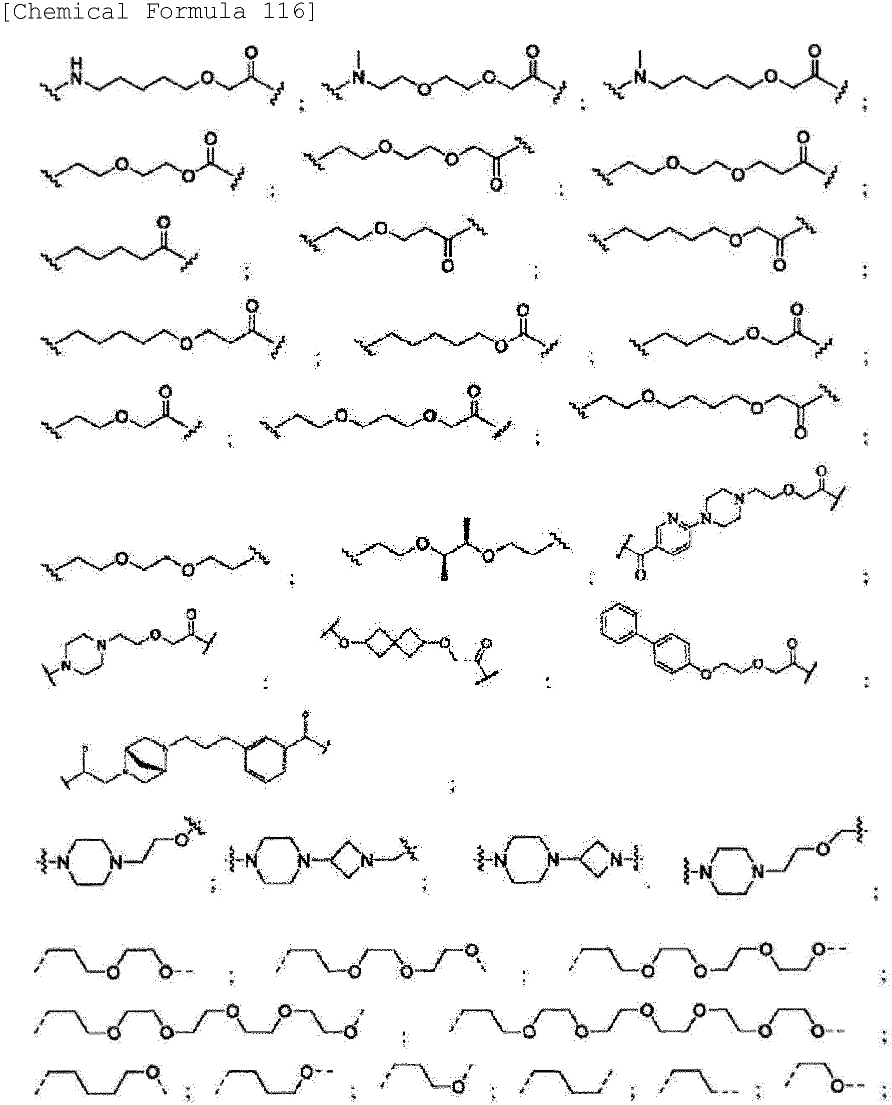

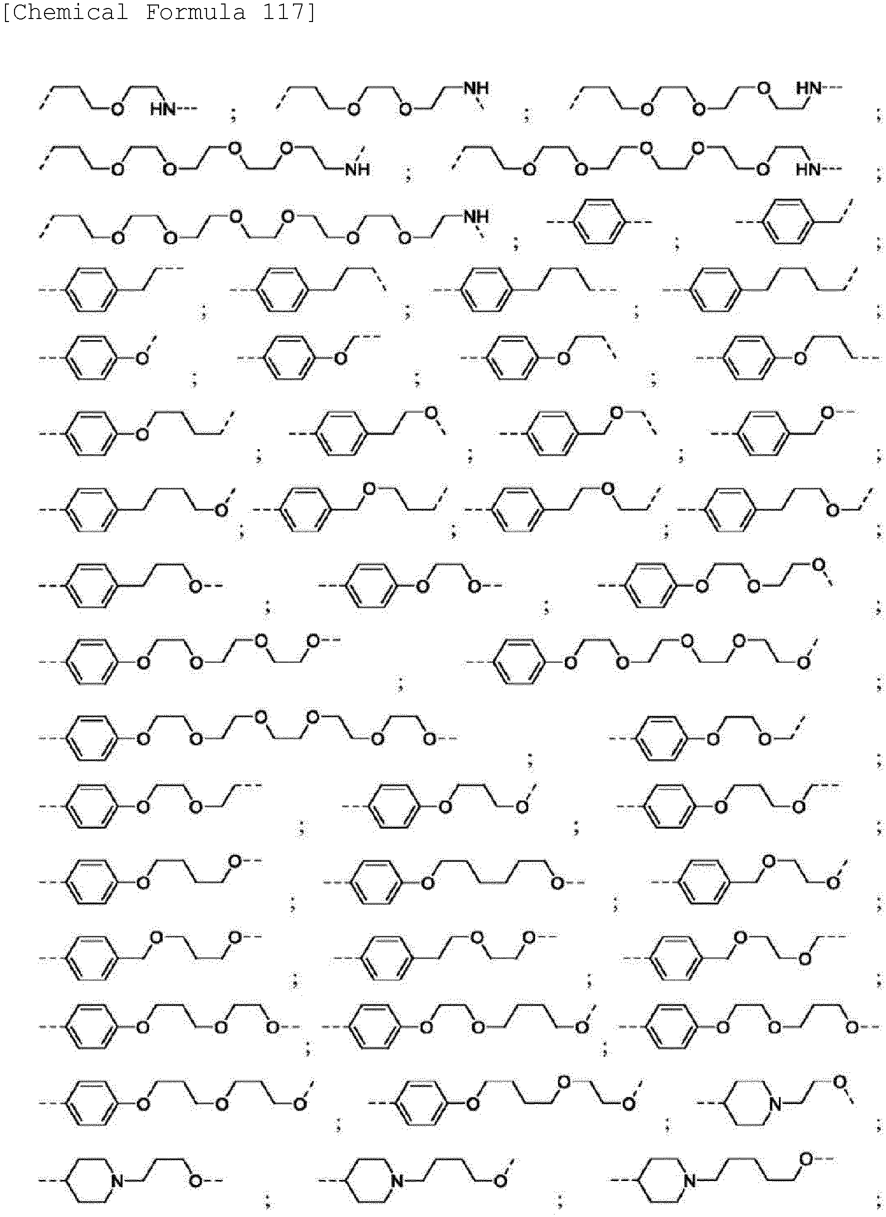

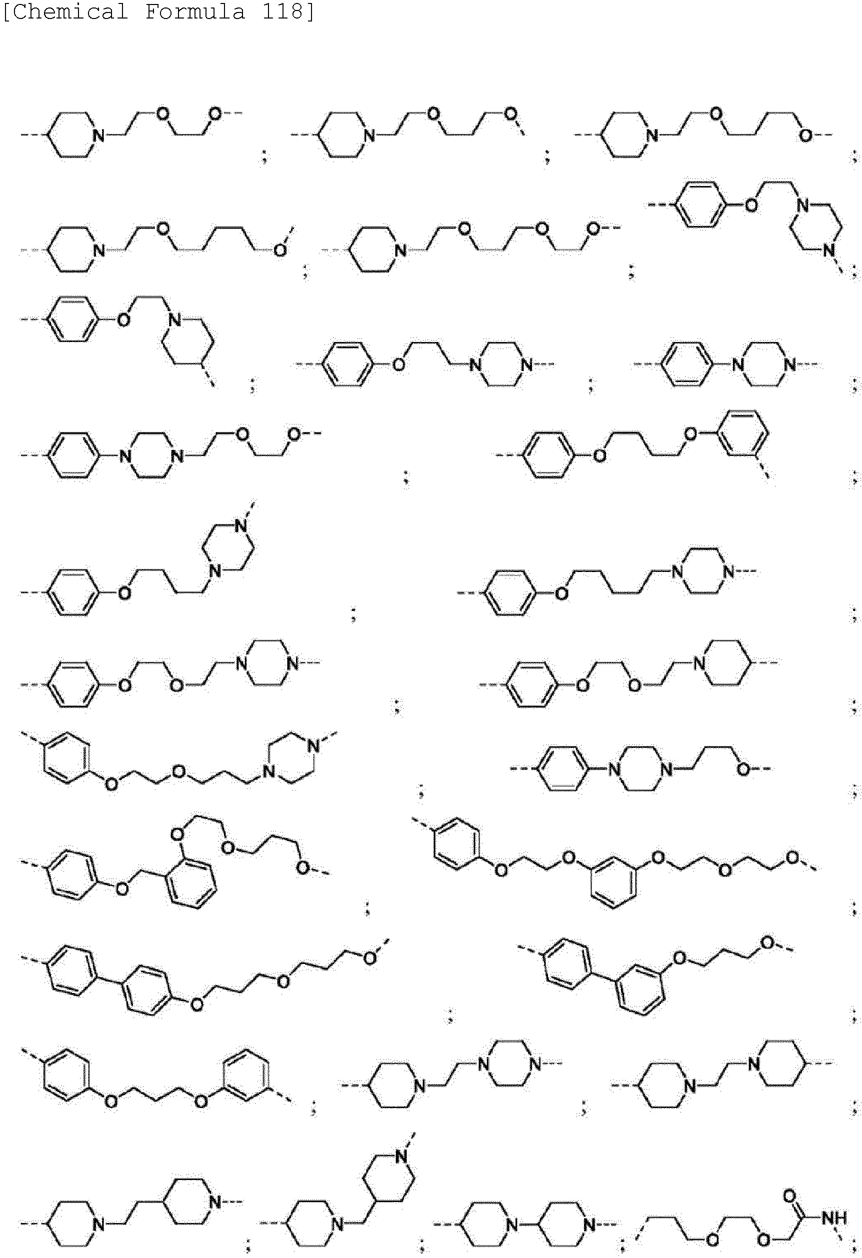

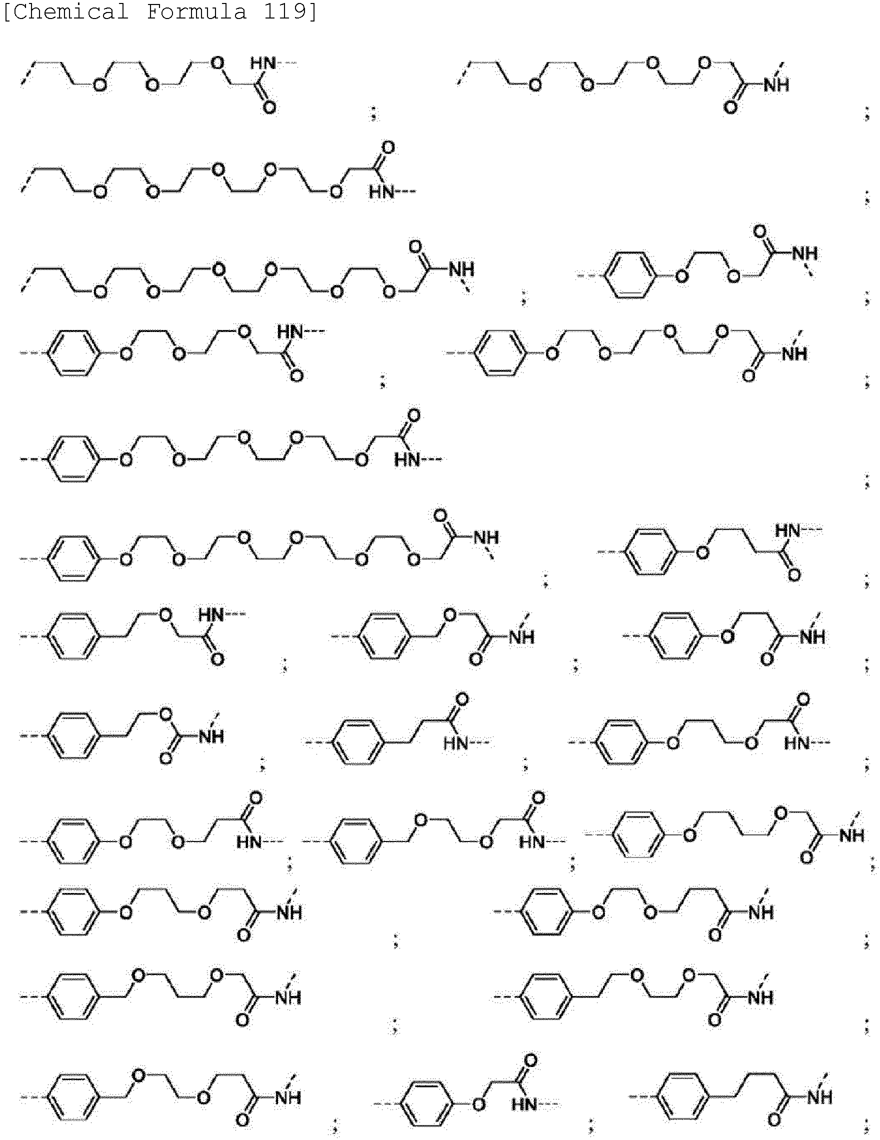

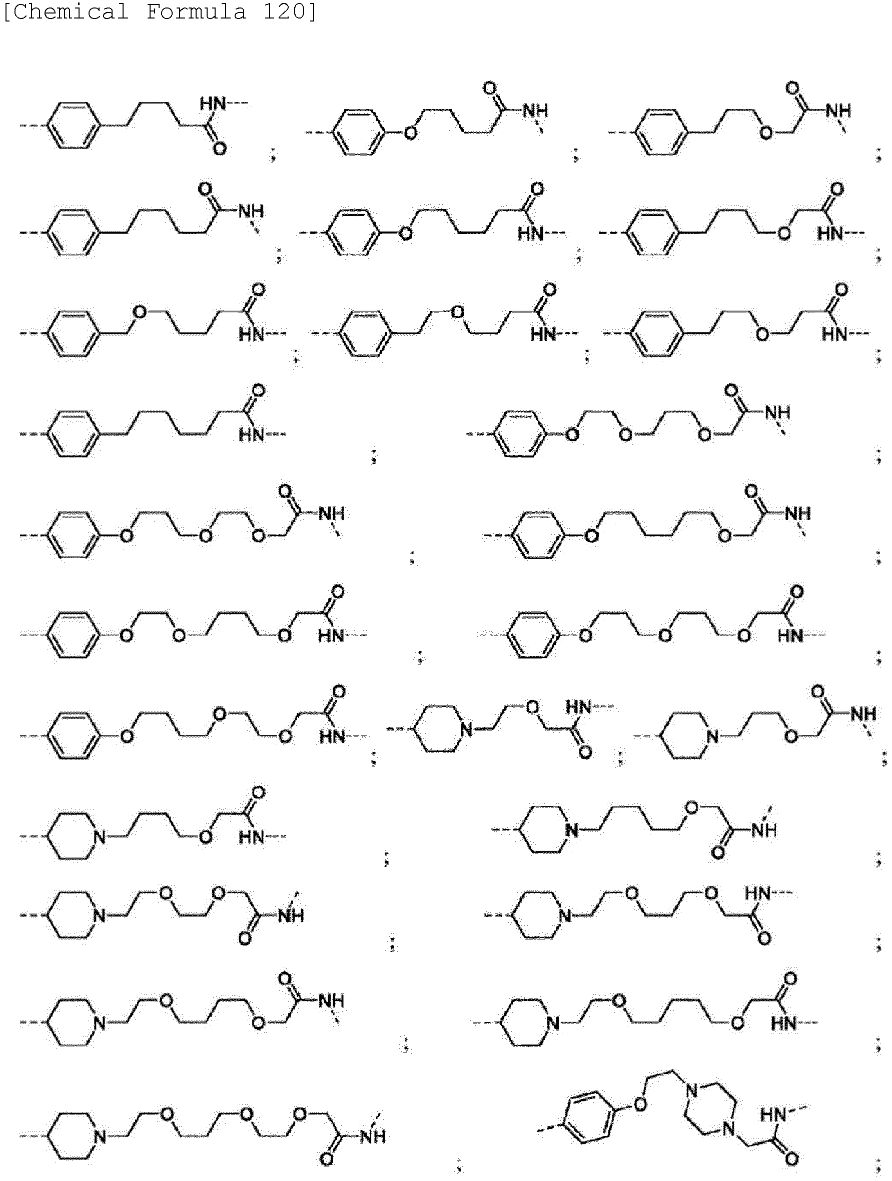

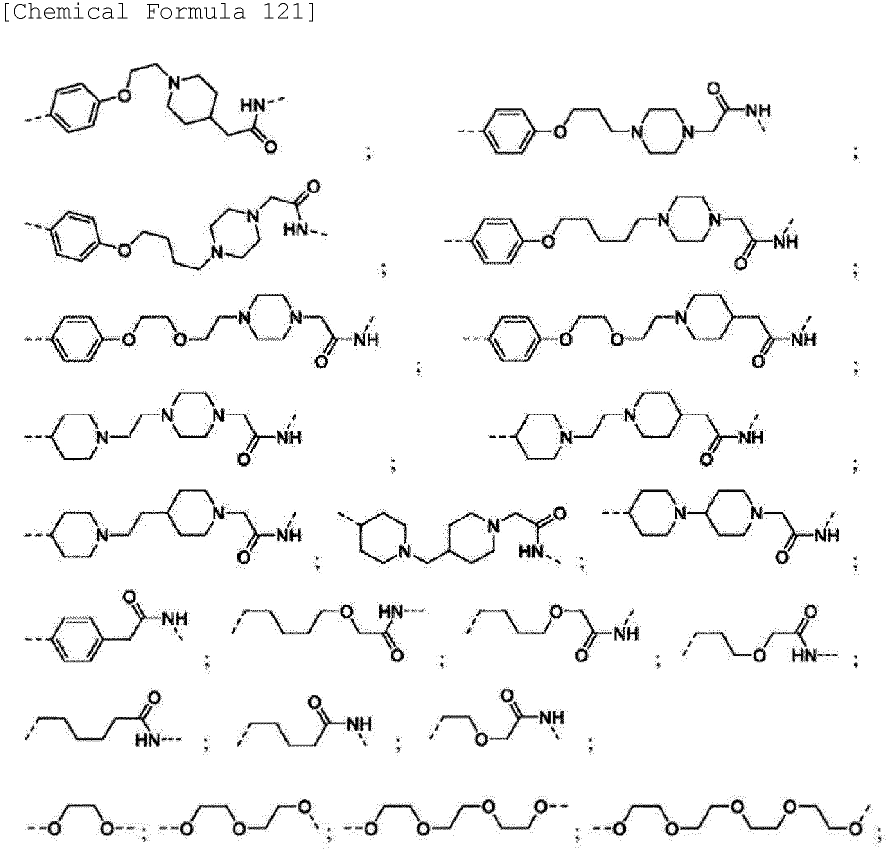

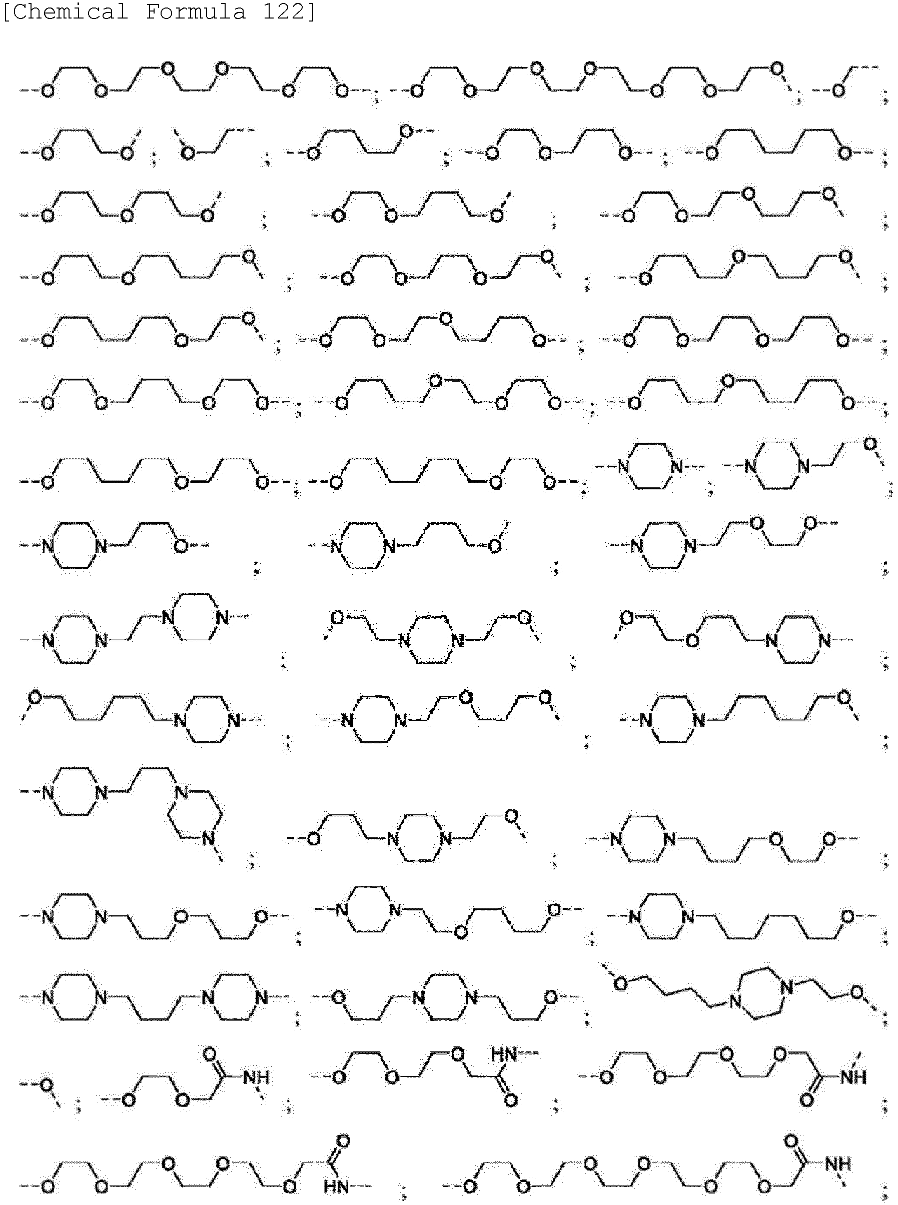

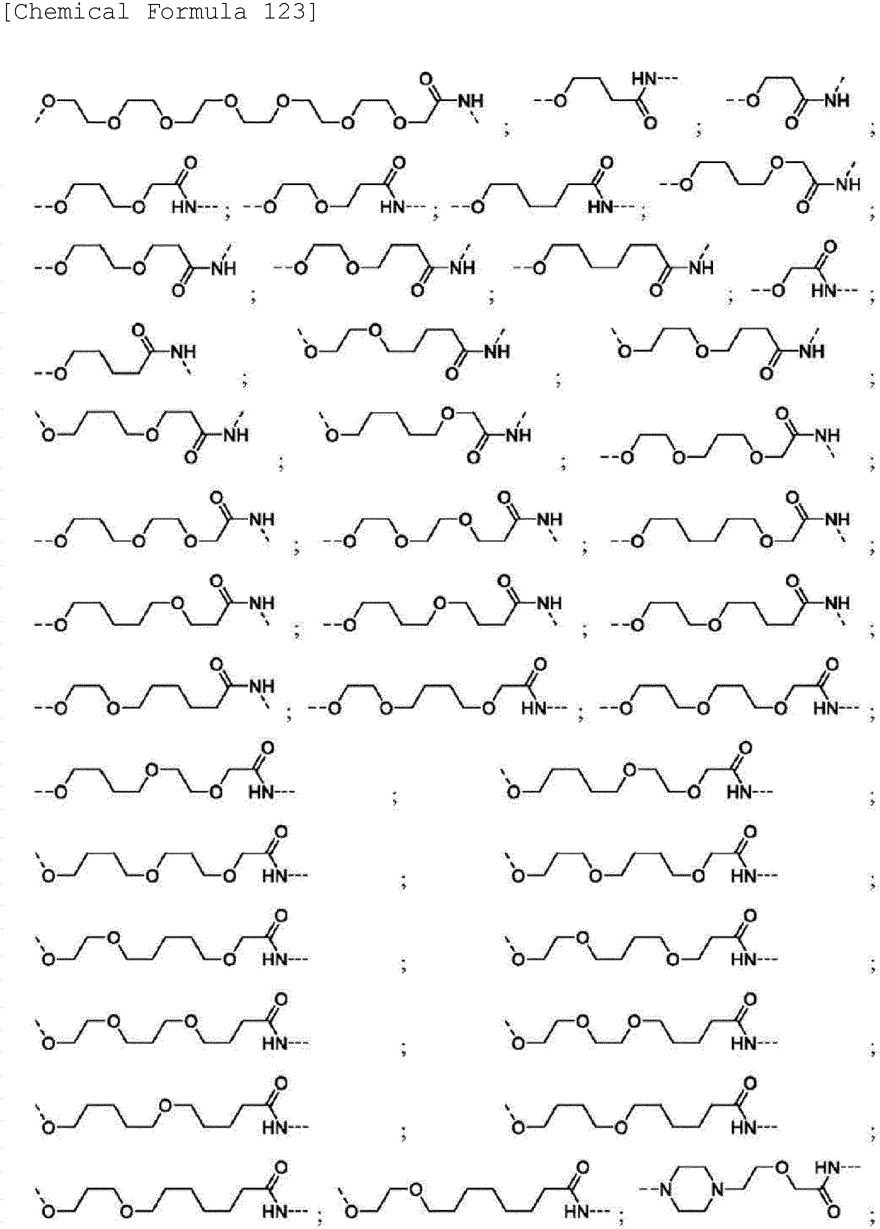

wherein 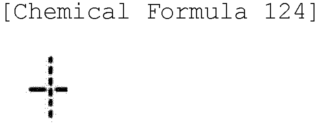 and 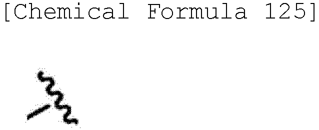 are each a bonding point,
m, n, o, p, q, r, s, t and u are each independently 0, 1, 2, 3, 4, 5, 6, and when the number is 0, then N-O or O-O bonds do not exist,
each R is independently hydrogen, methyl and ethyl at each occurrence,
X is H or F,
W^{L1} and W^{L1} are independently a 4- to 8-membered ring having 0 to 4 heteroatoms, a 4- to 8-membered ring optionally substituted with RQ wherein each RQ is independently H, halo, OH, CN, or CF3, optionally substituted linear or branched C₁₋₆ alkyl, or optionally substituted linear or branched C₁₋₆ alkoxy, or two RQ groups, together with the carbon atom(s) to which they are attached, may join to form a 4- to 8-membered ring having 0 to 4 heteroatoms, and
Y^{L1} is independently a bond, optionally substituted linear or branched C₁₋₆ alkyl in which one or more C is optionally replaced with O, or optionally substituted linear or branched C₁₋₆ alkoxy.

### (Medicament, therapeutic method, etc.)

### General description

In one embodiment, the compound of the disclosure can be administered directly, or as a formulation, medicament, or a pharmaceutical composition using a suitable dosage form, by oral or parenteral administration. Specific examples of such dosage forms include, but are not limited to, tablets, capsules, powdered agents, granules, liquid agents, suspension, injection agents, patch-on agents, poultice, and the like. These formulations can be manufactured by a known method using an additive that is commonly used as a pharmaceutical additive.

As these additives, an excipient, disintegrant, binding agent, fluidizer, lubricant, coating agent, solubilizing agent, solubilizing promotor, thickener, dispersant, stabilizer, sweetener, flavoring agent, or the like can be used depending on the objective. Specific examples of these additives include, but are not limited to, lactose, mannitol, crystalline cellulose, low-substituted hydroxypropyl cellulose, corn starch, partially pregelatinized starch, carmellose calcium, croscarmellose sodium, hydroxypropyl cellulose, hydroxypropyl methylcellulose, polyvinyl alcohol, magnesium stearate, sodium stearyl fumarate, polyethylene glycol, propylene glycol, titanium oxide, talc, and the like.

In one embodiment, the compound of the present disclosure is a compound having a target protein modulatory activity, and has an inhibitory activity or binding activity against a target protein.

The timing of dosing of the compound of the disclosure and therapeutic agents thereof is not limited. The compound and therapeutic agent can be administered concurrently or sequentially to a subject being administered therewith. The compound of the disclosure and therapeutic agent thereof can be formulated as a combined agent. The dosage of the therapeutic agent can be appropriately selected based on the clinically used dose. The ratio of the compound of the disclosure and therapeutic agent thereof can be appropriately selected depending on the subject to be administered, route of administration, target disease, disorder or symptom, combinations thereof, or the like.

In one embodiment of the present disclosure, the compound of the disclosure, when using a pharmaceutical composition, can be combined at the same time or different time for administration. Such a pharmaceutical composition is also within the scope of the present disclosure.

Such a medicament, formulation, and pharmaceutical composition can be manufactured by mixing the compound of the disclosure and/or additional agent (e.g., antimicrobial drug, antiviral agent (e.g., ribavirin, amantadine, or the like), sedative (e.g., ketamine, midazolam, etc.), or the like) with any appropriate component, together or separately as a combined agent or separate agents, by using any technology known in the art, and can be formulated using any known technology in the art as an appropriate formulation such as a tablet, capsule, powder, granule, liquid agent, suspension, injection, patch, or poultice. If the compound of the disclosure and/or additional agent (e.g., antimicrobial drug, antiviral agent (e.g., ribavirin, amantadine, or the like), sedative (e.g., ketamine, midazolam, etc.), or the like) are prepared as separate agents, they can be provided as a kit of two agents. One component can be provided as a single agent, with an instruction (package insert or the like) instructing to combine another component (for the compound of the disclosure, additional agent, and for an additional agent (e.g., antimicrobial drug, antiviral agent (e.g., ribavirin, amantadine, or the like), sedative (e.g., ketamine, midazolam, etc.), or the like), the compound of the disclosure) at the same time or different times for administration.

If the compound of the disclosure is used as the active ingredient of a medicament, this can be intended for use in not only humans, but also other animals besides humans (cats, dogs, cows, horses, bats, foxes, mongooses, raccoons, and the like).

### (Production method of the compound of the present disclosure)

Hereinafter, the method of manufacturing the compound of the disclosure is described with examples, but the present disclosure is not limited thereto.

The compound of the disclosure can be manufactured by, for example, the following manufacturing methods, but are not limited to such methods. These manufacturing methods can be appropriately improved upon based on the expertise of those skilled in the art of organic synthetic chemistry. Salts of the compounds used as a raw material can be used in the following manufacturing method, as long as the reaction is not affected.

The compound represented by Formula (A) of the present invention, for example, may have isomers depending on the type of the substituent. Here, the chemical structure of only one form of the isomer may be described, but the present invention also includes all isomers arising from the structure (geometric isomers, stereoisomers, tautomers, etc.), and all isomers alone or mixtures thereof are also included. In the present invention, the "hydrogen atom" includes ¹H and ²H(D), and deuterium converters in which any one or more ¹H in the compound represented by Formula (A) is converted to ²H(D) are also included in the compound represented by Formula (A).

The compounds represented by Formula (A) of the present invention and their pharmaceutically acceptable salts, crystal polymorphs, prodrugs, solvates, and isotope-containing derivatives can be produced, for example, by the following methods. In the production methods shown below, when the defined groups are changed under the conditions of the method to be carried out or are inappropriate for carrying out the method, the compounds can be easily produced by applying a method generally used in organic synthesis chemistry, for example, by the means described in [Organic Reactions, John Wiley & Sons, Inc., 1942-2021; Organic Syntheses, Organic Syntheses, Inc., 1921-2021;T.W.Greene, Greene's Protective Groups in Organic Synthesis 4th Edition, John Wiley & Sons, Inc., 2006] or the like or a method similar thereto. In addition, the order of reaction steps such as introduction of substituents and bond formation can be changed as necessary.

The abbreviations and symbols used in the following explanations, Reference Example, Example, etc. have the following meanings:

**[Table D]**

| | |
|---|---|
| ¹H NMR: | proton nuclear magnetic resonance |
| HPLC: | high-performance liquid chromatography |
| M: | mol concentration |
| MS: | mass spectrum |
| UPLC: | ultra-performance liquid chromatography |
| DCE: | 1,2-dichloroethane |
| DME: | 1,2-dimethoxyethane |
| DCM: | dichloromethane |
| DIPEA: | N,N-diisopropylethylamine |
| DMF: | N,N-dimethylformamide |
| DMSO: | dimethyl sulfoxide |
| EDC-HCl: | 1-ethyl-3-(3-dimethylaminopropyl)carbodiimide hydrochloride |
| HATU: | O-(7-azabenzotriazol-1-yl)-N,N,N',N'-tetramethyluroniumhexafluorophosphorate |
| NMP: | N-methylpyrrolidone |
| TBAF: | tetra-n-butylammonium fluoride |
| TEA: | triethylamine |
| THF: | tetrahydrofuran |
| LAH: | lithium aluminium hydride |

The compounds represented by Formula (A), or their pharmaceutically acceptable salts, crystal polymorphs, prodrugs, solvates, and isotope-containing derivatives can be produced by the following general approaches (Schemes 1-3), together with synthetic methods known in the art of organic chemistry, or modification and derivatization methods known to those skilled in the art.

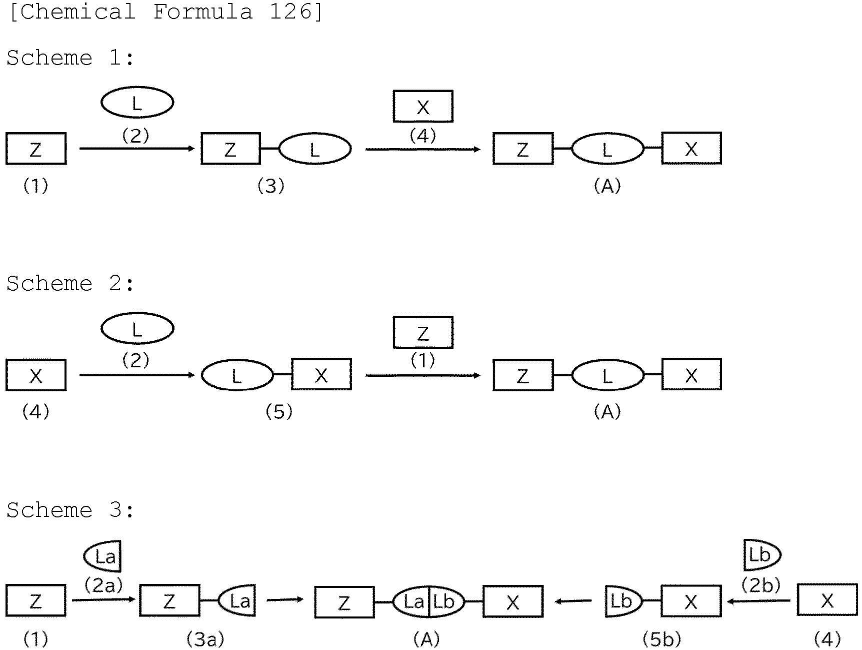

As shown in Scheme 1, Compound (3) can be produced by subjecting ligand Z (Compound (1)) or its reactive derivative that can bind specifically to an intracellular protein, and Compound (2) or its reactive derivative, which is linker L, to an alkylation reaction, Mitsunobu reaction, a reductive amination reaction, an amidation reaction, a coupling reaction using a transition metal catalyst, or the like. The compound represented by Formula A (Compound (A)) can be produced by subjecting Compound (3) or its reactive derivative and X represented by Formula (I) (Compound (4)) or its reactive derivative to an alkylation reaction, Mitsunobu reaction, a reductive amination reaction, an amidation reaction, a coupling reaction using a transition metal catalyst, or the like.

Alternatively, as shown in Scheme 2, Compound (5) can be produced by subjecting Compound (4) or its reactive derivative and Compound (2) or its reactive derivative to an alkylation reaction, Mitsunobu reaction, a reductive amination reaction, an amidation reaction, a coupling reaction using a transition metal catalyst, or the like. Compound (A) can be produced by subjecting Compound (5) or its reactive derivative and Compound (1) or its reactive derivative to an alkylation reaction, Mitsunobu reaction, a reductive amination reaction, an amidation reaction, a coupling reaction using a transition metal catalyst, or the like.

Alternatively, as shown in Scheme 3, Compound (3a) can be produced by subjecting Compound (1) or its reactive derivative, and Compound (2a) or its reactive derivative, which is a linker, to an alkylation reaction, Mitsunobu reaction, a reductive amination reaction, an amidation reaction, a coupling reaction using a transition metal catalyst, or the like. Compound (5b) can be produced by subjecting Compound (4) or its reactive derivative, and Compound (2b) or its reactive derivative, which is a linker, to an alkylation reaction, Mitsunobu reaction, a reductive amination reaction, an amidation reaction, a coupling reaction using a transition metal catalyst, or the like. Compound (A) can be produced by subjecting Compound (3a) and Compound (5b) or their reactive derivatives to an alkylation reaction, Mitsunobu reaction, a reductive amination reaction, an amidation reaction, a coupling reaction using a transition metal catalyst, or the like.

Compound (4a), which is one of the reactive derivatives of (X) constituting a part of Formula (A), can be produced, for example, according to Scheme 4.

Each symbol in the following schemes has the same meaning as in the above Formula (A) and Formula (I). X1, X2, and X3 represent a halogen atom or a leaving group, PG1 represents a suitable protecting group such as a tert-butoxycarbonyl (Boc) group, PG2 represents a suitable protecting group such as a tert-butyldiphenylsilyl (TBDPS) group, PG3 represents a suitable protecting group such as a benzyl group, and RG represents a halogen atom or a leaving group, or a reactive functional group such as a hydroxyl group, an amino group, a carboxyl group, an amido group, a sulfonamido group or the like.

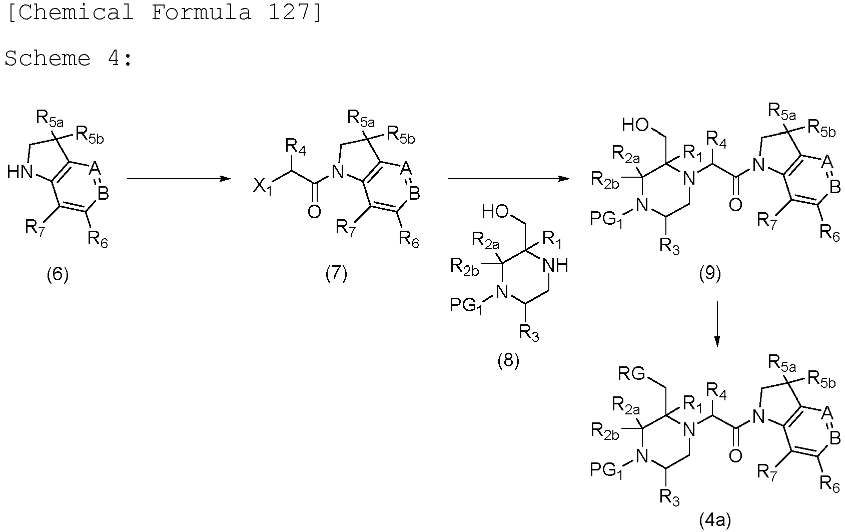

When X1 is a halogen, Compound (7) can usually be produced by reacting a halogenated haloacetic acid such as chloroacetyl chloride and Compound (6) in the presence of a suitable base such as TEA in a solvent such as THF or acetonitrile.

Compound (9) can usually be produced by reacting Compounds (7) and (8), and, for example, the reaction may be carried out in an acetonitrile solvent, in the presence of a suitable base such as potassium carbonate and a suitable additive such potassium iodide.

When RG is chlorine, Compound (4a) can be produced, for example, by reacting Compound (9) with methanesulfonyl chloride or the like in the presence of a suitable base such as TEA in a solvent such as THF or DCM.

Compound (4b), which is one of the reactive derivatives of (X) constituting a part of Formula (A), can be produced, for example, according to Scheme 5.

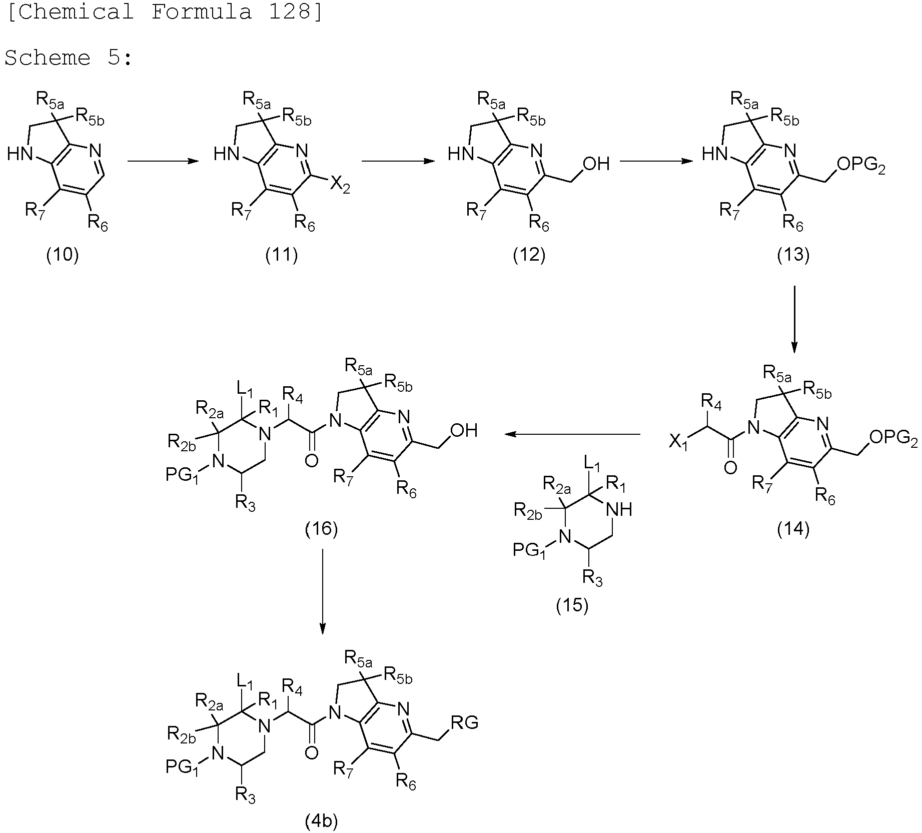

Compound (11) can usually be produced by reacting Compound (10) with a halogenating agent such as N-bromosuccinimide in a solvent such as DMF.

Compound (12) can be produced in a stepwise manner, for example, by subjecting Compound (11) to a halogen-metal exchange reaction with tert-butyllithium or the like, and reacting the resulting anion species with a suitable electrophile such as DMF to introduce a formyl group, and then treating the resulting compound with a reducing agent such as sodium borohydride. Alternatively, Compound (12) can be produced in a stepwise manner, for example, by treating Compound (11) with a transition metal catalyst such as a combination of palladium acetate and 1,1'-ferrocene bis(diphenylphosphine) under carbon monoxide atmosphere in an alcohol solvent such as methanol to introduce an ester group, and then treating the resulting compound with a reducing agent such as lithium borohydride or lithium triethylborohydride.

Compound (13) can be produced by reacting Compound (12) and a suitable protecting reagent such as tert-butylchlorodiphenylsilane in the presence of a suitable base such as TEA in a THF or DCM solvent.

When X1 is halogen, Compound (14) can be obtained by reacting a halogenated haloacetic acid such as chloroacetyl chloride and Compound (13) in the presence of a suitable base such as TEA in a solvent such as THF or acetonitrile.

Compound (16) can usually be produced by reacting Compounds (14) and (15), and then deprotecting the resulting compound. For example, Compounds (14) and (15) may be reacted in the presence of a suitable base such as potassium carbonate and a suitable additive such potassium iodide in an acetonitrile solvent, and, when PG2 is a TBDPS group, the resulting compound may be reacted with a suitable deprotecting agent such as TBAF in a THF solvent.

When RG is chlorine, Compound (4b) can be produced, for example, by reacting Compound (16) with methanesulfonyl chloride or the like in the presence of a suitable base such as TEA in a solvent such as THF or DCM.

Compound (4c), which is one of the reactive derivatives of (X) constituting a part of Formula (A), can be produced, for example, according to Scheme 6.

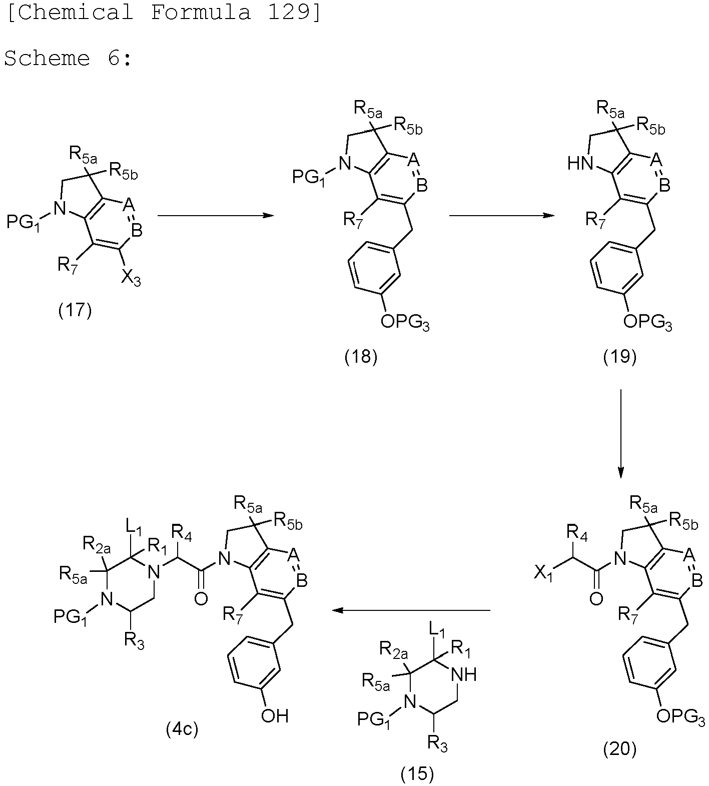

Compound (18) can be produced by reacting Compound (17) and a compound represented by PG3-O-C6H4-CH2-M in the presence of an organic transition metal catalyst such as a divalent or zerovalent palladium salt or complex in a solvent such as THF or DMF. Here, PG3-O-C6H4-CH2-M represents, for example, a benzyloxybenzylzinc halide or a benzyloxybenzyl boronate ester.

Compound (19) can usually be obtained by subjecting Compound (18) to a deprotection reaction, and, for example, when PG1 is Boc, Compound (19) can be produced by treating Compound (18) with an acid such as hydrochloric acid or TFA.

When X1 is halogen, Compound (20) can be obtained by reacting a halogenated haloacetic acid such as chloroacetyl chloride and Compound (18) in the presence of a suitable base such as TEA in a solvent such as THF or acetonitrile.

Compound (4c) can usually be obtained by reacting Compounds (15) and (20), and then deprotecting the resulting compound. For example, Compounds (15) and (20) may be reacted in the presence of a suitable base such as potassium carbonate and a suitable additive such potassium iodide in an acetonitrile solvent, and, when PG3 is a benzyl group, the resulting compound may be reacted under hydrogen atmosphere in the presence of a catalyst such as palladium on carbon in a solvent such as methanol.

When Compound (4a), which is one of the reactive derivatives of (X), is used, a compound of Formula (A-a) can be produced, for example, according to Scheme 7.

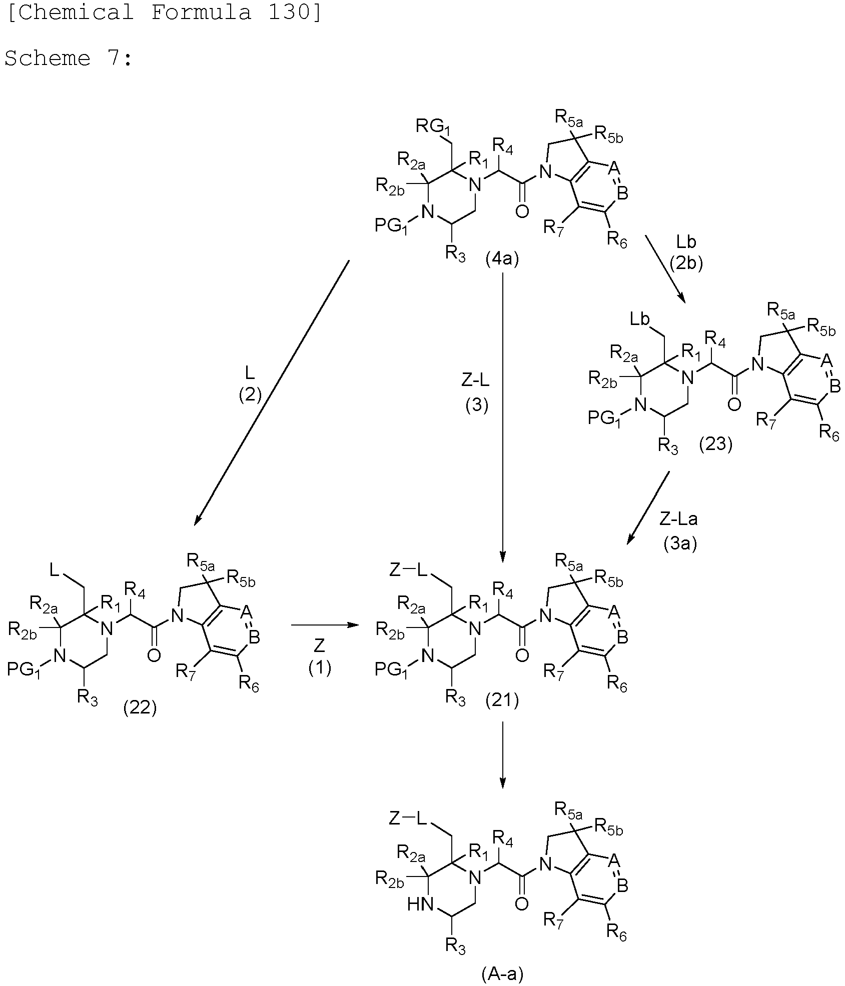

Compound (2), which is linker L constituting a part of Formula (A-a), and La (Compound (2a) and Lb (Compound (2b), which are a part of a linker, and Compound (1), which is ligand Z that can bind specifically to an intracellular protein, can be commercially available products, or can be produced by a method known per se or a method similar thereto.

Compound (21) can be produced by reacting Compound (3) or its reactive derivative, which is comprised of ligand Z that can bind specifically to an intracellular protein and linker L, with Compound (4a) under conditions for an alkylation reaction, Mitsunobu reaction, a reductive amination reaction, an amidation reaction, a coupling reaction using a transition metal catalyst, or the like.

Compound (21) can also be produced via Compound (22), which is comprised of Compound (4a) and Compound (2), which is linker L. For example, Compound (22) can be produced by reacting Compound (2) or its reactive derivative with Compound (4a) under conditions for an alkylation reaction, Mitsunobu reaction, a reductive amination reaction, an amidation reaction, a coupling reaction using a transition metal catalyst, or the like. Compound (21) can be produced by subjecting Compound (22) or its reactive derivative and Compound (1) or its reactive derivative to an alkylation reaction, Mitsunobu reaction, a reductive amination reaction, an amidation reaction, a coupling reaction using a transition metal catalyst, or the like.

Compound (21) can also be produced via Compound (23), which is comprised of Compound (4a) and Lb (Compound (2b)), which is a part of a linker. For example, Compound (23) can be produced by reacting Compound (2b) or its reactive derivative with Compound (4a) under conditions for an alkylation reaction, Mitsunobu reaction, a reductive amination reaction, an amidation reaction, a coupling reaction using a transition metal catalyst, or the like. Compound (21) can be produced by reacting Compound (3a), which is comprised of Compound (1) and La (Compound (2a)), which is a part of a linker, with Compound (23) under conditions for an alkylation reaction, Mitsunobu reaction, a reductive amination reaction, an amidation reaction, a coupling reaction using a transition metal catalyst, or the like.

Compound (A-a) can be produced by subjecting Compound (21) to a deprotection reaction, and, for example, when PG1 is Boc, Compound (A-a) can be produced by treating Compound (21) with an acid such as hydrochloric acid or TFA.

When Compound (4b), which is one of the reactive derivatives of (X), is used, a compound of Formula (A-b) can be produced, for example, according to Scheme 8.

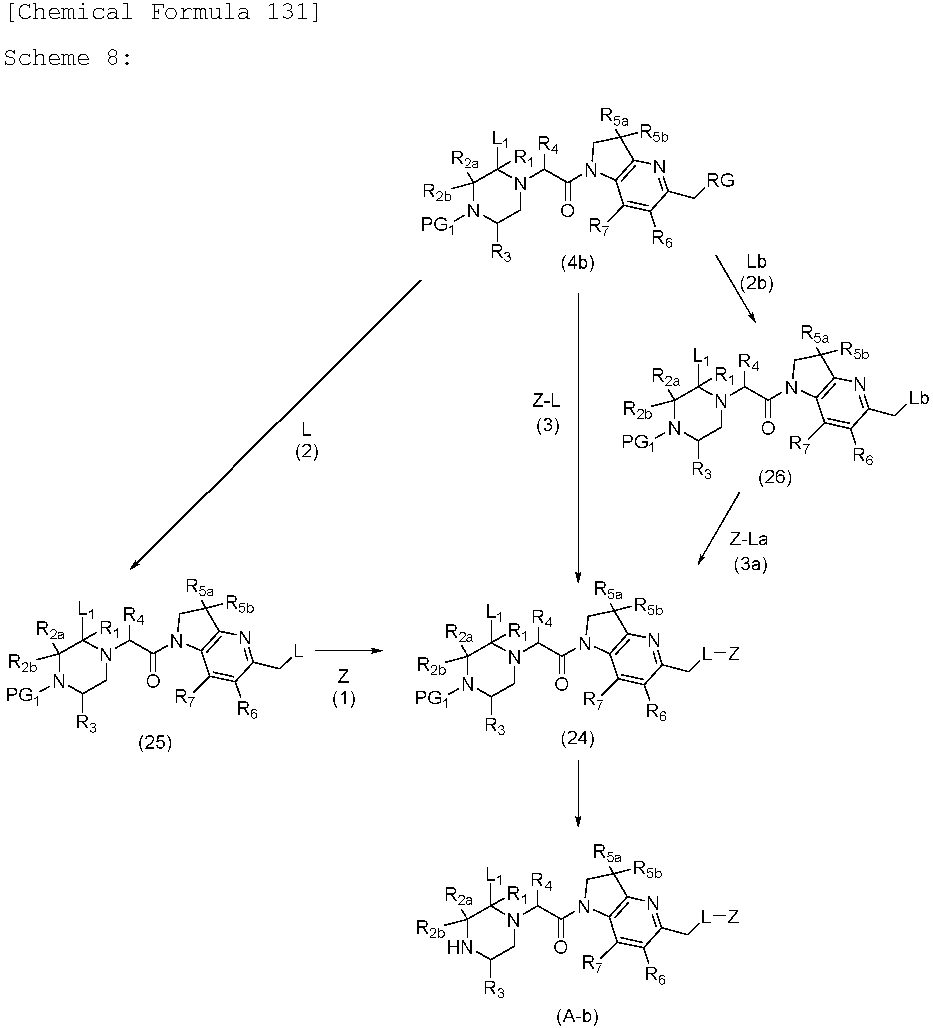

Compound (2), which is linker L constituting a part of Formula (A-b), and La (Compound (2a) and Lb (Compound (2b), which are a part of a linker, and Compound (1), which is ligand Z that can bind specifically to an intracellular protein, can be commercially available products, or can be produced by a method known per se or a method similar thereto.

Compound (24) can be produced by reacting Compound (3) or its reactive derivative, which is comprised of ligand Z that can bind specifically to an intracellular protein and linker L, with Compound (4b) under conditions for an alkylation reaction, Mitsunobu reaction, a reductive amination reaction, an amidation reaction, a coupling reaction using a transition metal catalyst, or the like.

Compound (24) can also be produced via Compound (25), which is comprised of Compound (4b) and Compound (2), which is linker L. For example, Compound (25) can be produced by reacting Compound (2) or its reactive derivative with Compound (4b) under conditions for an alkylation reaction, Mitsunobu reaction, a reductive amination reaction, an amidation reaction, a coupling reaction using a transition metal catalyst, or the like. Compound (24) can be produced by subjecting Compound (25) or its reactive derivative and Compound (1) or its reactive derivative to an alkylation reaction, Mitsunobu reaction, a reductive amination reaction, an amidation reaction, a coupling reaction using a transition metal catalyst, or the like.

Compound (24) can also be produced by via Compound (26), which is comprised of Compound (4b) and Lb (Compound (2b)), which is a part of a linker. For example, Compound (26) can be produced by reacting Compound (2b) or its reactive derivative with Compound (4b) under conditions for an alkylation reaction, Mitsunobu reaction, a reductive amination reaction, an amidation reaction, a coupling reaction using a transition metal catalyst, or the like. Compound (24) can be produced by reacting Compound (3a), which is comprised of Compound (1) and La (Compound (2a)), which is a part of a linker, with Compound (26) under conditions for an alkylation reaction, Mitsunobu reaction, a reductive amination reaction, an amidation reaction, a coupling reaction using a transition metal catalyst, or the like.

Compound (A-b) can be produced by subjecting Compound (24) to a deprotection reaction, and, for example, when PG1 is Boc, Compound (A-b) can be produced by treating Compound (24) with an acid such as hydrochloric acid or TFA.

When Compound (4c), which is one of the reactive derivatives of (X), is used, a compound of Formula (A-c) can be produced, for example, according to Scheme 9.

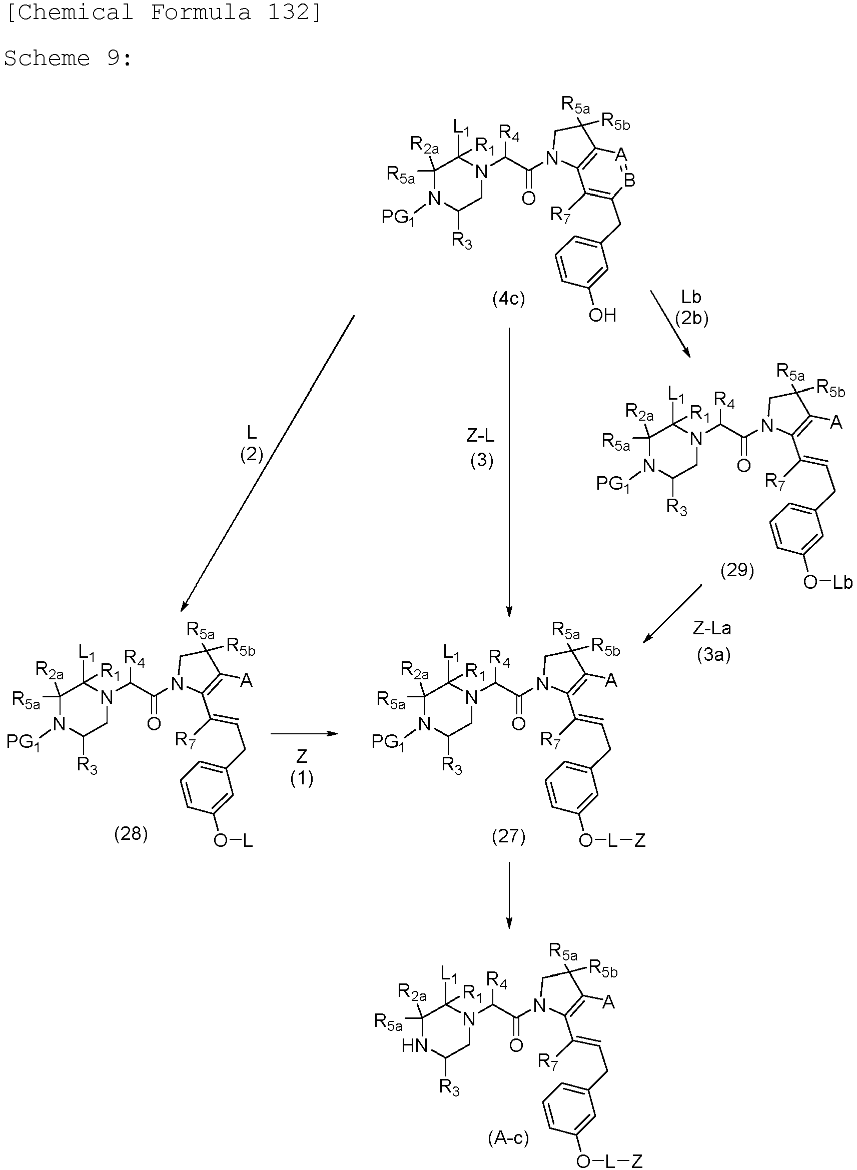

Compound (2), which is linker L constituting a part of Formula (A-c), and La (Compound (2a) and Lb (Compound (2b), which are a part of a linker, and Compound (1), which is ligand Z that can bind specifically to an intracellular protein, can be commercially available products, or can be produced by a method known per se or a method similar thereto.

Compound (27) can be produced by reacting Compound (3) or its reactive derivative, which is comprised of ligand Z that can bind specifically to an intracellular protein and linker L, with Compound (4c) under conditions for an alkylation reaction, Mitsunobu reaction, a reductive amination reaction, an amidation reaction, a coupling reaction using a transition metal catalyst, or the like.

Compound (27) can also be produced via Compound (28), which is comprised of Compound (4c) and Compound (2), which is linker L. For example, Compound (28) can be produced by reacting Compound (2) or its reactive derivative with Compound (4b) under conditions for an alkylation reaction, Mitsunobu reaction, a reductive amination reaction, an amidation reaction, a coupling reaction using a transition metal catalyst, or the like. Compound (27) can be produced by subjecting Compound (28) or its reactive derivative and Compound (1) or its reactive derivative to an alkylation reaction, Mitsunobu reaction, a reductive amination reaction, an amidation reaction, a coupling reaction using a transition metal catalyst, or the like.

Compound (27) can also be produced via Compound (29), which is comprised of Compound (4c) and Lb (Compound (2b)), which is a part of a linker. For example, Compound (29) can be produced by reacting Compound (2b) or its reactive derivative with Compound (4c) under conditions for an alkylation reaction, Mitsunobu reaction, a reductive amination reaction, an amidation reaction, a coupling reaction using a transition metal catalyst, or the like. Compound (27) can be produced by reacting Compound (3a), which is comprised of Compound (1) and La (Compound (2a)), which is a part of a linker, with Compound (29) under conditions for an alkylation reaction, Mitsunobu reaction, a reductive amination reaction, an amidation reaction, a coupling reaction using a transition metal catalyst, or the like.

Compound (A-c) can be produced by subjecting Compound (27) to a deprotection reaction, and, for example, when PG1 is Boc, Compound (A-c) can be produced by treating Compound (27) with an acid such as hydrochloric acid or TFA.

One embodiment of the combination of Z, L and X groups in Formula (A) includes the following.

The intracellular protein in Z may be a serin/threonine kinase, a receptor tyrosine kinase, or a non-receptor tyrosine kinase, may be AGC group, LTK receptor family, or FAK family, or may be ROCK2, ALK, or FAK. (Examples of such ligand moiety capable of specifically binding to an intracellular protein include the following: 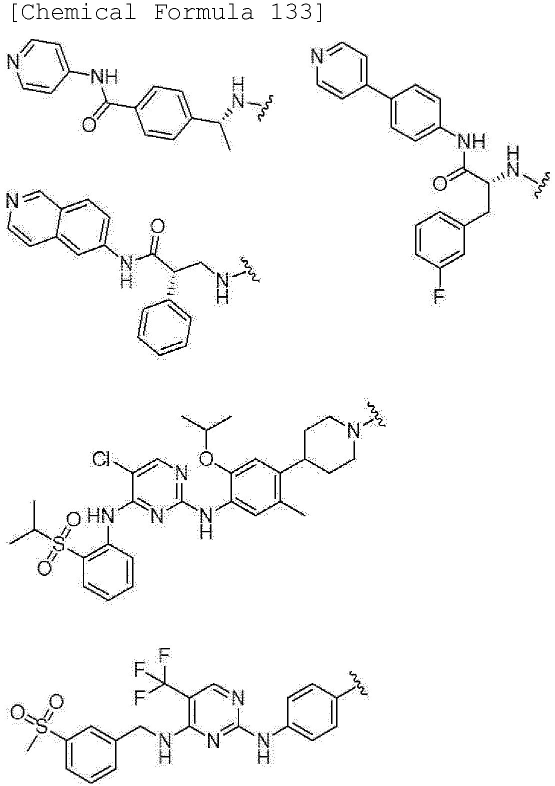 and the like);
L is selected from: 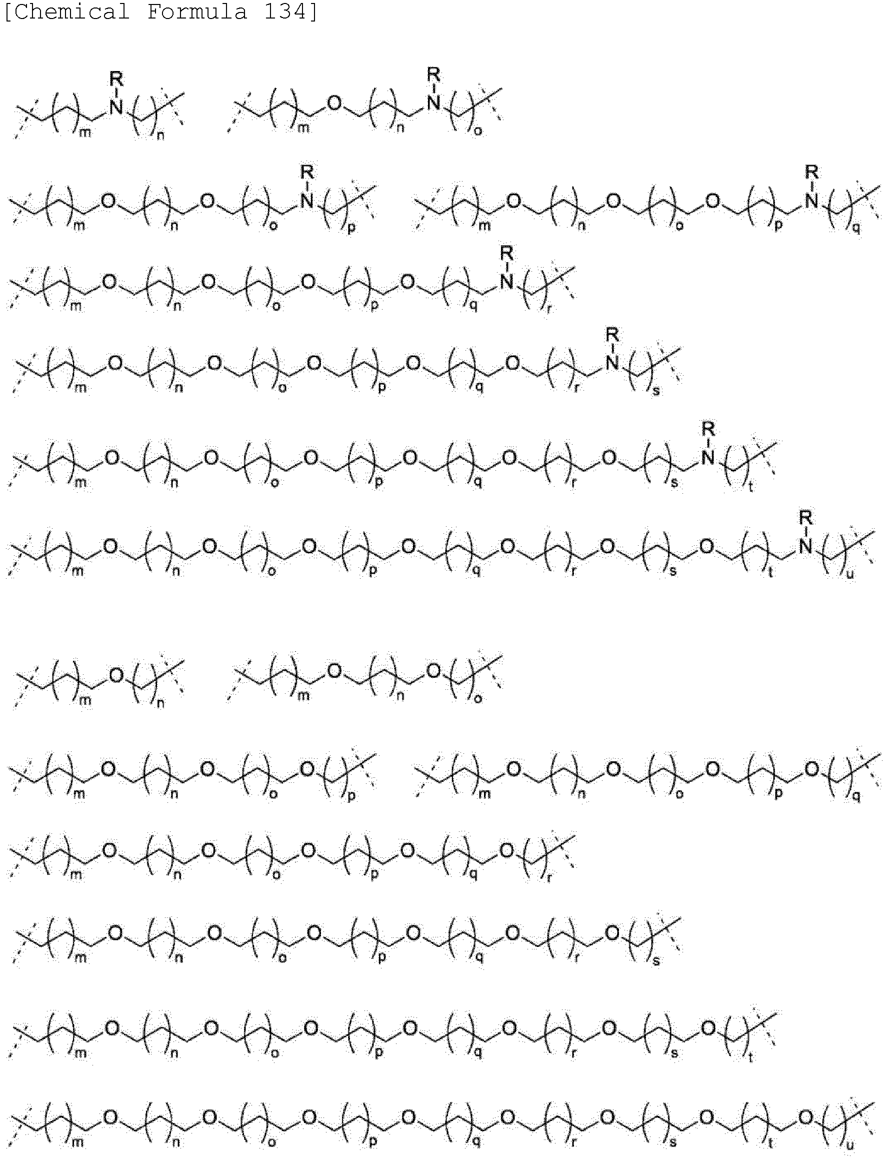 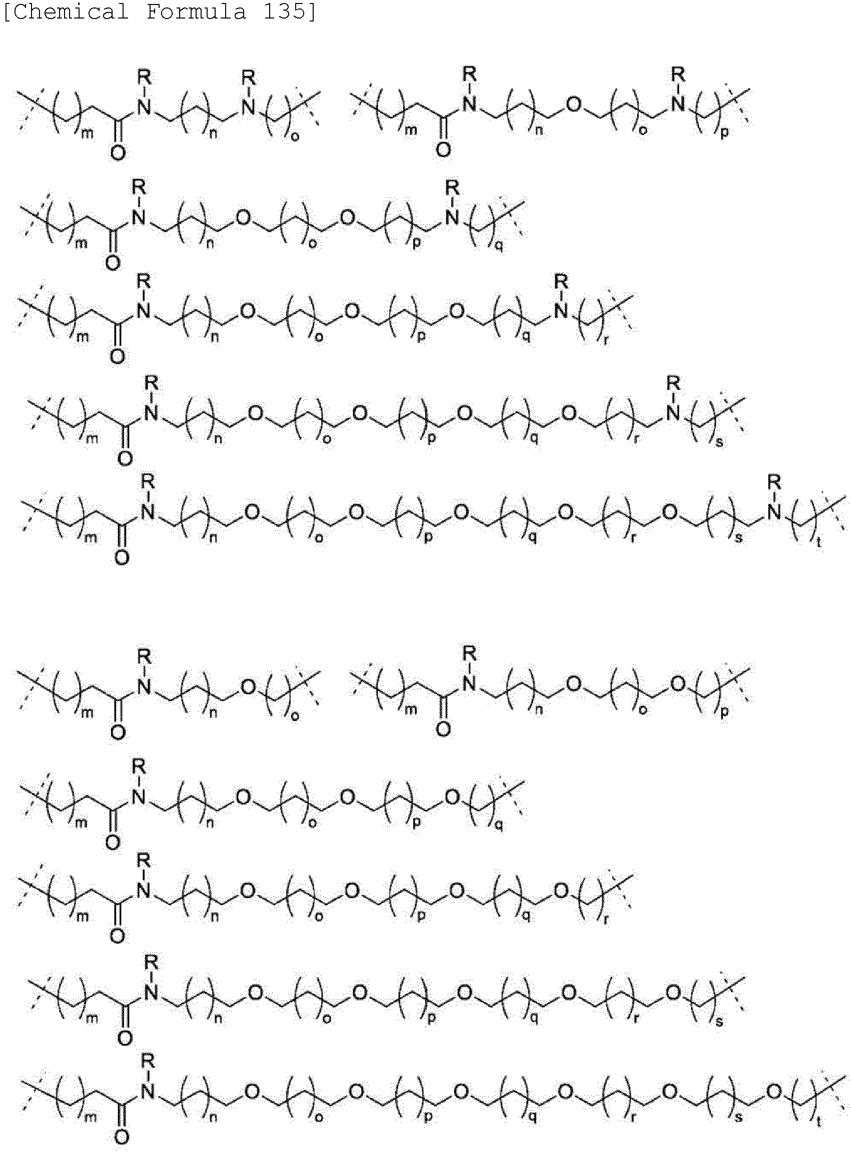 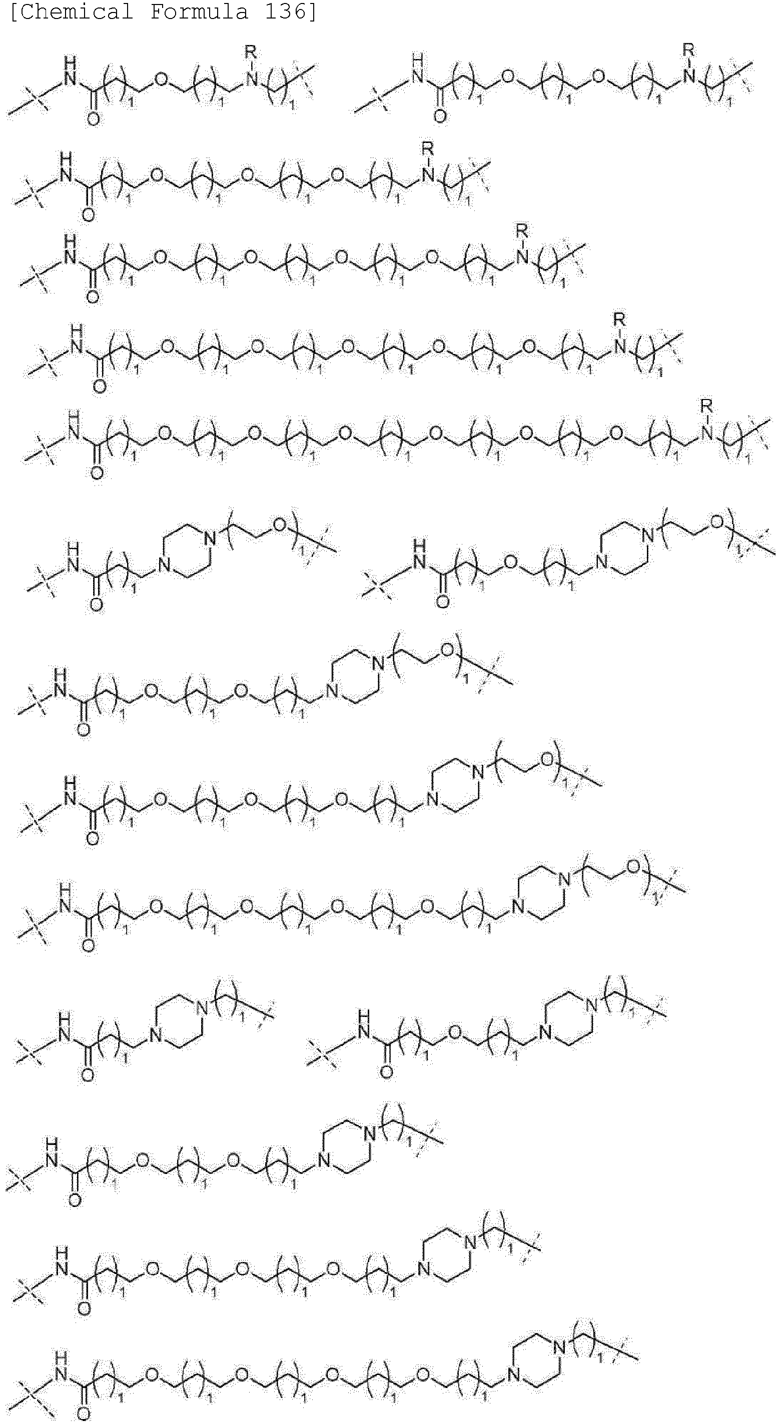
wherein each group is as defined above; and
in group X,
A is N or CR₈ (preferably N),
B is N, CR₉, or CL₂ (preferably CR₉, or CL₂),
Q is N,
R₁, and L₁ not used for bonding to L, are each independently hydrogen, or C₁₋₆ alkyl (preferably methyl), wherein the C₁₋₆ alkyl is optionally substituted with one or more -O-C₁₋₂ alkyl (preferably methoxy),
R₂ₐ and R_{2b} are hydrogen,
R³ is C₁₋₄ alkyl (preferably methyl),
R₄ is hydrogen,
R₅ₐ and R_{5b} are each independently C₁₋₄ alkyl (preferably methyl),
R₆ is -CH₂-phenyl, or 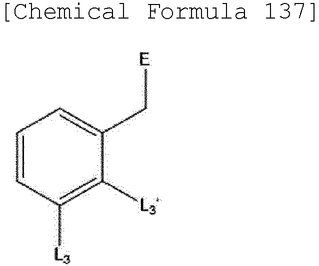
wherein E indicates a bonding point,
each of which is optionally substituted with one or more halogen atoms (preferably a fluorine atom),
R₇ is hydrogen,
R₉ is hydrogen, and
X is bonded to L at any one of L₁ to L₃ and L₃'.

(Examples of X being bonded to L at any one of L₁ to L₃ and L₃' include the following:

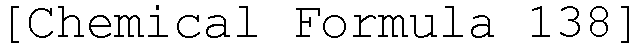

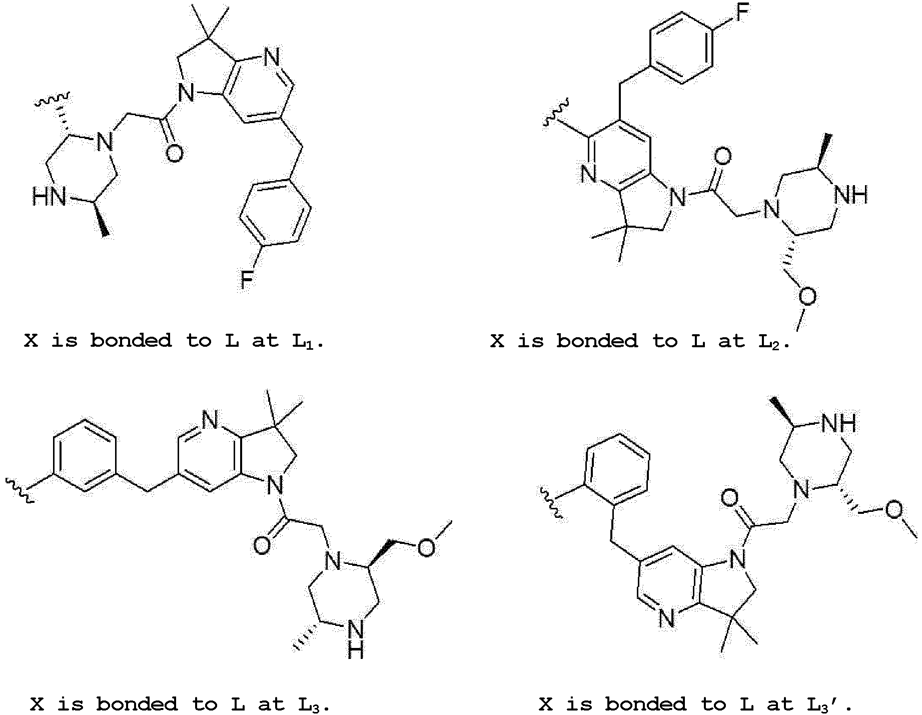

The present disclosure has been described while showing preferred embodiments to facilitate understanding. While the present disclosure is described hereinafter based on the Examples, the above descriptions and the following Examples are provided for the sole purpose of exemplification, not limitation of the present disclosure. Thus, the scope of the present disclosure is not limited to the embodiments and Examples that are specifically described herein and is limited only by the scope of claims.

### [Examples]

Compounds were identified by hydrogen nuclear magnetic resonance spectroscopy (¹H NMR) and mass spectroscopy (MS). ¹H NMR was measured at 400 MHz or 500 MHz, and exchangeable hydrogen may not be clearly observed depending on the compound and measurement conditions. MS was measured by LC/MS. The HPLC or UPLC analytical methods shown in Table 3 were performed to measure the retention times of the compounds.

**[Table 3-1]**

| Analytical method | Condition |
|---|---|
| A | column: AQUITY UPLC BEH C18 (2.1 x 100 mm, 1.7 µm) |
| | mobile phase A: 0.05% formic acid aqueous solution; mobile phase B: 0.05% formic acid-acetonitrile solution |
| | flow rate: 0.55 mL/min; column temperature: 50°C |
| | gradient (time (min)/mobile phase B percentage (%)): 0/3, 8.5/100, 9.0/100, 9.5/3, 10/3 |
| B | column: AQUITY UPLC BEH C18 (2.1 x 100 mm, 1.7 µm) |
| | mobile phase A: 0.05% TFA aqueous solution; mobile phase B: 0.05% TFA acetonitrile solution |
| | flow rate: 0.55 mL/min; column temperature: 50°C |
| | gradient (time (min)/mobile phase B percentage (%)): 0/3, 8.5/100, 9.0/100, 9.5/3, 10/3 |
| C | column: X-Bridge C18 (150 mm x 4.8, 3.5 µm) |
| | mobile phase A: 10 mM ammonium carbonate aqueous solution; mobile phase B: acetonitrile |
| | flow rate: 1.0 mL/min; column temperature: 25°C |
| | gradient (time (min)/mobile phase B percentage (%)): 0/5, 1/5, 3/15, 7/55, 11/98, 16/98, 16.1/5, 20/5 |
| D | column: AQUITY UPLC BEH C18, (2.1 x 100 mm, 1.7 µm) |
| | mobile phase A: 0.05% TFA aqueous solution; mobile phase B: 0.05% TFA acetonitrile solution |
| | flow rate: 0.3 mL/min; column temperature: 40°C |
| | gradient (time (min)/mobile phase B percentage (%)): 0/10, 7/98, 12/98, 12.01/10 |
| E | column: X-Select CSH C18 (150 mm x 4.8, 3.5 µm) |
| | mobile phase A: 10 mM ammonium carbonate aqueous solution; mobile phase B: acetonitrile |
| | flow rate: 1.0 mL/min; column temperature: 45°C |
| | gradient (time (min)/mobile phase B percentage (%)): 0/5, 15/95, 20/95, 20.1/5, 25/5 |
| F | column: AQUITY UPLC BEH C18, (2.1 x 100 mm, 1.7 µm) |
| | mobile phase A: 0.05% formic acid aqueous solution; mobile phase B: 0.05% formic acid acetonitrile solution |
| | flow rate: 0.55 mL/min; column temperature: 50°C |
| | gradient (time (min)/mobile phase B percentage (%)): 0/3, 0.50/3, 3/15, 5/100, 8/100, 8.10/3, 10/3 |

**[Table 3-2]**

| | |
|---|---|
| G | column: X-Bridge C18 (150 mm x 4.8, 3.5 µm) |
| | mobile phase A: 10 mM ammonium acetate aqueous solution; mobile phase B: acetonitrile |
| | flow rate: 1.0 mL/min; column temperature: 25°C |
| | gradient (time (min)/mobile phase B percentage (%)): 0/5, 1.5/5, 3/15, 7/55, 10/95, 14/95, 17/5, 20/5 |
| H | column: AQUITY UPLC BEH C18, (2.1 x 100 mm, 1.7 µm) |
| | mobile phase A: 0.05% TFA aqueous solution; mobile phase B: 0.05% TFA acetonitrile solution |
| | flow rate: 0.55 mL/min; column temperature: 50°C |
| | gradient (time (min)/mobile phase B percentage (%)): 0/1, 1/1, 8.5/100, 9/100, 9.511, 10/1 |
| I | column: X-Bridge C18 (150 x 4.6 mm, 3.5 µm) |
| | mobile phase A: 10 mM ammonium acetate aqueous solution; mobile phase B: acetonitrile |
| | flow rate: 1.0 mL/min; column temperature: 35°C |
| | gradient (time (min)/mobile phase B percentage (%)): 0/5, 1/5, 3/15, 7/55, 11/98, 16/98, 16.1/5, 20/5 |
| J | column: AQUITY UPLC BEH C18, (2.1 x 100 mm, 1.7 µm) |
| | mobile phase A: 0.05% formic acid aqueous solution; mobile phase B: 0.05% formic acid acetonitrile solution |
| | flow rate: 0.3 mL/min; column temperature: 40°C |
| | gradient (time (min)/mobile phase B percentage (%)): 0/10, 5/98, 8/98, 8.01/10 |
| K | column: X-Bridge C18 (4.6 x 150 mm, 3.5 µm) |
| | mobile phase A: 10 mM ammonium carbonate aqueous solution; mobile phase B: acetonitrile |
| | flow rate: 1.0 mL/min; column temperature: 25°C |
| | gradient (time (min)/mobile phase B percentage (%)): 0/5, 0.5/5, 1.4/15, 3.3/55, 5.2/98, 7.6/98, 7.7/5, 10/5 |
| L | column: X-Bridge C18 (4.6 x 150 mm, 3.5 µm) |
| | mobile phase A: 10 mM ammonium carbonate aqueous solution; mobile phase B: acetonitrile |
| | flow rate: 1.4 mL/min; column temperature: 25°C |
| | gradient (time (min)/mobile phase B percentage (%)): 0/5, 0.5/5, 1.4/15, 3.3/55, 5.2/98, 7.6/98, 7.7/5, 10/5 |

### Reference Example 1

### tert-Butyl (2R,5R)-5-(hydroxymethyl)-2-methylpiperazine-1-carboxylate

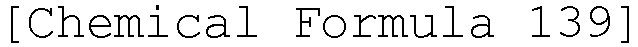

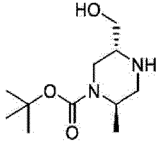

### (Step A) Methyl ((benzyloxy)carbonyl)-L-seryl-D-alaninate

To a DCM solution (1.8 L) of methyl D-alaninate (75 g, 0.54 mol) was added ((benzyloxy)carbonyl)-L-serin (0.13 kg, 0.54 mol) at room temperature, and then EDC-HCl (0.13 kg, 1.1 mol) was added. The reaction mixture was cooled to 0°C, DIPEA (0.25 L, 1.3 mol) was added, and the mixture was stirred at room temperature for 18 hr. The reaction solution was concentrated under reduced pressure, and the residue was added to saturated aqueous sodium carbonate solution and ethyl acetate, and the organic layer was separated. The organic layer was washed with 2M hydrochloric acid, and dried over anhydrous sodium sulfate, and the solvent was evaporated under reduced pressure to give the title compound (0.12 kg).
MS (m/z): 325.19 [M+H]⁺.

### (Step B) Methyl L-seryl-D-alaninate

To a methanol solution (0.40 L) of methyl ((benzyloxy)carbonyl)-L-seryl-D-alaninate (40 g, 0.12 mol) was added palladium-carbon (5.0 g) at room temperature, and the mixture was stirred under hydrogen atmosphere (30 psi) for 18 hr. The reaction solution was filtered through Celite, the Celite was washed with methanol, and the filtrate was concentrated under reduced pressure to give the title compound (23 g).
MS (m/z): 191.27 [M+H]⁺.

### (Step C) (3S,6R)-3-(Hydroxymethyl)-6-methylpiperazine-2,5-dione

A methanol solution (1.5 L) of methyl L-seryl-D-alaninate (68 g, 0.36 mol) was heated under reflux for 18 hr. The reaction solution was cooled, and the solvent was evaporated under reduced pressure to give the title compound (64 g).
¹H NMR (400 MHz, DMSO-d₆) δ 1.29 (d, J = 7.2 Hz, 3H), 3.51-3.54 (m, 1H), 3.68-3.79 (m, 3H), 5.12 (brs, 1H), 7.15 (s, 1H), 8.09 (s, 1H).

### (Step D) ((2R,5R)-5-Methylpiperazin-2-yl)methanol dihydrochloride

To a THF solution (0.45 L) of (3S,6R)-3-(hydroxymethyl)-6-methylpiperazine-2,5-dione (42 g, 0.27 mol) was added borane-THF complex (1M THF solution, 0.23 L, 0.23 mol) at room temperature, and the reaction solution was stirred at 70°C for 18 hr. The reaction solution was cooled to 0°C, methanol (0.45 L) and 5M hydrochloric acid (0.10 L) were added, and the mixture was stirred at 70°C for 2 hr. The precipitated solid was collected by filtration, washed with THF, and dried under reduced pressure to give the title compound (41 g).
¹H NMR (400 MHz, DMSO-d₆) δ 1.30 (d, J = 6.4 Hz, 3H), 3.05-3.09 (m, 2H), 3.42-3.71 (m, 6H), 5.57-5.68 (m, 1H), 9.30-10.35 (m, 2H).

### (Step E) tert-Butyl (6R,8aR)-6-methyl-3-oxotetrahydro-3H-oxazolo[3,4-a]pyrazine-7(1H)-carboxylate

To a methanol solution (0.50 L) of ((2R,5R)-5-methylpiperazin-2-yl)methanol dihydrochloride (50 g, 0.25 mol) was added TEA (0.11 L, 0.75 mol) under ice-cooled, and a methanol solution (50 mL) of di-tert-butyl dicarbonate (0.14 L, 0.60 mol) was then added dropwise. The mixture was stirred at 0°C for 1 hr, and then at 50°C for 18 hr, and the reaction solution was concentrated under reduced pressure to give the title compound (62 g).
MS (m/z): 201.13 [M-56+H]⁺.

### (Step F) tert-Butyl (2R,5R)-5-(hydroxymethyl)-2-methylpiperazine-1-carboxylate

To an ethanol solution (0.30 L) of tert-butyl (6R,8aR)-6-methyl-3-oxotetrahydro-3H-oxazolo[3,4-a]piperazine-7(1H)-carboxylate (31 g, 0.12 mol) was added an aqueous solution (0.30 L) of sodium hydroxide (28 g) at room temperature. The mixture was stirred at 100°C for 18 hr, 1M hydrochloric acid (0.25 L) was added, and the mixture was subjected to extraction with DCM. The organic layer was dried over anhydrous sodium sulfate, and the solvent was evaporated under reduced pressure to give the title compound (23 g).
¹H NMR (400 MHz, DMSO-d₆) δ 1.13 (d, J = 6.4 Hz, 3H), 1.38 (s, 9H), 2.32-2.38 (m, 2H), 2.72-2.77 (m, 1H), 2.88 (dd, J = 12.4, 4.4 Hz, 1H), 3.06 (dd, J = 13.6, 4.4 Hz, 1H), 3.27-3.32 (m, 1H), 3.38-3.44 (m, 1H), 3.56-3.59 (m, 1H), 3.92-3.95 (m, 1H), 4.47 (brs, 1H); MS (m/z): 231.20 [M+H]⁺.

### Reference Example 2

### tert-Butyl (2R,5R)-5-(methoxymethyl)-2-methylpiperazine-1-carboxylate

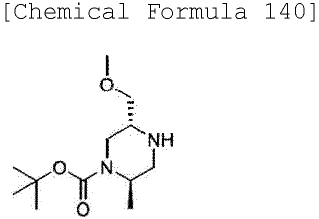

### (Step A) 1-Benzyl 4-(tert-butyl) (2R,5R)-2-(hydroxymethyl)-5-methylpiperazine-1,4-dicarboxylate

To a THF solution (0.40 L) of tert-butyl (2R,5R)-5-(hydroxymethyl)-2-methylpiperazine-1-carboxylate (the compound of Reference Example 1, 40 g, 0.17 mol) was added 1M aqueous sodium hydroxide solution (0.20 L), and carbobenzoxy chloride (25 mL,0.17 mol) was then added dropwise at 0°C. The reaction solution was stirred at room temperature for 6 hr, and diluted with ethyl acetate and water, and the organic layer was separated. The organic layer was dried over anhydrous sodium sulfate, and the solvent was evaporated under reduced pressure, and the obtained crude product was purified by column chromatography (silica gel, petroleum ether/ethyl acetate) to give the title compound (48 g).
¹H NMR (400 MHz, DMSO-d₆) δ 1.16-1.19 (m, 1H), 1.39 (s, 9H), 1.98 (s, 1H), 3.04-3.13 (m, 2H), 3.32-3.44 (m, 2H), 3.65-3.84 (m, 2H), 4.00-4.08 (m, 3H), 4.79-4.86 (m, 1H), 5.04-5.16 (m, 2H), 7.30-7.38 (m, 5H); MS (m/z): 265.14 [M-Boc+H]⁺.

### (Step B) 1-Benzyl 4-(tert-butyl) (2R,5R)-2-(methoxymethyl)-5-methylpiperazine-1,4-dicarboxylate

To a DCM solution (0.10 L) of 1-benzyl 4-(tert-butyl) (2R,5R)-2-(hydroxymethyl)-5-methylpiperazine-1,4-dicarboxylate (8.0 g, 22 mmol) was added proton sponge (24 g, 0.11 mol), and trimethyloxonium tetrafluoroborate (17 g, 0.11 mmol) was then added dropwise at 0°C. The mixture was stirred at room temperature for 4 hr, to the reaction mixture was added saturated aqueous ammonium chloride solution, and the mixture was subjected to extraction with DCM. The organic layer was washed successively with 1M hydrochloric acid and saturated aqueous sodium hydrogencarbonate solution, and dried over anhydrous sodium sulfate. The solvent was evaporated under reduced pressure, and the obtained crude product was purified by column chromatography (silica gel, petroleum ether/ethyl acetate) to give the title compound (5.8 g).
¹H NMR (400 MHz, CDCl₃) δ 1.23-1.28 (m, 1H), 1.46 (s, 9H), 2.04 (s, 1H), 3.14-3.55 (m, 7H), 3.74-3.84 (m, 1H), 3.92-3.94 (m, 1H), 4.10-4.18 (m, 1H), 4.20-4.70 (m, 2H), 5.00-5.30 (m, 2H), 7.26-7.36 (m, 5H); MS (m/z): 379.24 [M+H]⁺.

### (Step C) tert-Butyl (2R,5R)-5-(methoxymethyl)-2-methylpiperazine-1-carboxylate

To a methanol solution (0.30 L) of 1-benzyl 4-(tert-butyl) (2R,5R)-2-(methoxymethyl)-5-methylpiperazine-1,4-dicarboxylate (30 g, 79 mmol) was added palladium on carbon (13 g), and the mixture was stirred under hydrogen atmosphere at room temperature for 18 hr. The reaction solution was filtered through Celite, and the obtained filtrate was concentrated under reduced pressure to give the title compound (15 g).
¹H NMR (400 MHz, CDCl₃) δ 1.25 (d, J = 6.8 Hz, 3H), 1.46 (s, 9H), 2.50 (dd, J = 12.8, 3.2 Hz, 1H), 3.06-3.12 (m, 2H), 3.20-3.30 (m, 2H), 3.34-3.39 (m, 3H), 3.60-3.72 (m, 2H), 4.14-4.18 (m, 1H), one proton missing; GC-MS (m/z): 244.2 [M]⁺.

### Reference Example 3

### 6-(4-Fluorobenzyl)-3,3-dimethyl-2,3-dihydro-1H-pyrrolo[3,2-b]pyridine

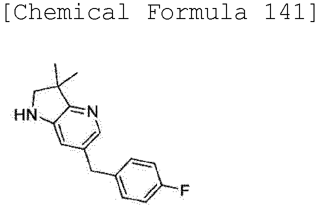

### (Step A) 5-Bromo-2-iodopyridin-3-amine

To an acetic acid solution (5.0 L) of 5-bromopyridin-3-amine (0.25 kg, 1.4 mol) was added N-iodosuccinimide (0.29 kg, 1.3 mol), and the mixture was stirred at room temperature for 48 hr. The reaction mixture was concentrated under reduced pressure, to the residue was added aqueous sodium hydrogencarbonate solution, and the mixture was subjected to extraction with ethyl acetate. The organic layer was dried over anhydrous sodium sulfate, and concentrated under reduced pressure. The obtained crude product was purified by column chromatography (silica gel, petroleum ether/ethyl acetate) to give the title compound (0.27 kg).
MS (m/z) : 300.83 [M(Br⁸¹)+H]⁺.

### (Step B) 5-Bromo-2-iodo-N-(2-methylallyl)pyridin-3-amine

To a THF solution (2.6 L) of 5-bromo-2-iodopyridin-3-amine (0.17 kg, 0.57 mol) was added potassium tert-butoxide (77 g, 0.68 mol) at 0°C, and the mixture was stirred for 30 min. Then, 3-bromo-2-methyl-1-propene (92 g, 0.68 mol) was added, and the mixture was stirred at 0°C for 1 hr, and then at room temperature for 16 hr. The reaction solution was diluted with ethyl acetate, and washed with water. The organic layer was dried over anhydrous sodium sulfate, and the solvent was evaporated under reduced pressure to give a crude product. This was purified by column chromatography (silica gel, petroleum ether/ethyl acetate) to give the title compound (0.12 kg).
MS (m/z): 353.06 [M+H]⁺.

### (Step C) 6-Bromo-3,3-dimethyl-2,3-dihydro-1H-pyrrolo[3,2-b]pyridine

To a DMSO/water mixed solution (0.70 L:0.15 L) of 5-bromo-2-iodo-N-(2-methylallyl)pyridin-3-amine (60 g, 0.20 mol), sodium formate (16 g, 0.24 mol), tetrabutylammonium chloride (67 g, 0.24 mol) and TEA (84 mL, 0.60 mol) was added palladium acetate (4.5 g, 20 mmol) at room temperature, and the reaction solution was stirred at 100°C for 18 hr. Then, the reaction solution was diluted with ice water, and subjected to extraction with ethyl acetate. The organic layer was washed with saturated brine, and dried over anhydrous sodium sulfate, and the solvent was evaporated under reduced pressure. The residue was purified by column chromatography (silica gel, petroleum ether/ethyl acetate) to give the title compound (13 g).
¹H NMR (400 MHz, DMSO-d₆) δ 1.20 (s, 6H), 3.29-3.32 (m, 2H), 6.05 (s, 1H), 6.87 (d, J = 2 Hz, 1H), 7.70 (d, J = 2 Hz, 1H); MS (m/z): 227.22 [M+H]⁺.

### (Step D) tert-Butyl 6-bromo-3,3-dimethyl-2,3-dihydro-1H-pyrrolo[3,2-b]pyridine-1-carboxylate

To a THF solution (0.50 L) of 6-bromo-3,3-dimethyl-2,3-dihydro-1H-pyrrolo[3,2-c]pyridine (25 g, 0.11 mol) were added potassium tert-butoxide (15 g, 0.13 mol) and di-tert-butyl dicarbonate (27 g, 0.12 mol), and the mixture was stirred at room temperature for 18 hr. To the reaction mixture was added ice water, the mixture was subjected to extraction with ethyl acetate, and the organic layer was washed with saturated brine, and dried over anhydrous sodium sulfate. The solvent was evaporated under reduced pressure, and the residue was purified by column chromatography (silica gel, petroleum ether/ethyl acetate) to give the title compound (29 g).
¹H NMR (400 MHz, DMSO-d₆) δ 1.32 (s, 6H), 1.51 (s, 9H), 3.72 (s, 2H), 7.57 (brs, 1H), 8.15 (s, 1H); MS (m/z): 327.36 [M+H]⁺.

### (Step E) tert-Butyl 6-(4-fluorobenzyl)-3,3-dimethyl-2,3-dihydro-1H-pyrrolo[3,2-b]pyridine-1-carboxylate

To a NMP/THF mixed solution (0.18 L:0.18 L) of tert-butyl 6-bromo-3,3-dimethyl-2,3-dihydro-1H-pyrrolo[3,2-c]pyridine-1-carboxylate (28 g, 86 mmol) were added lithium bromide (30 g, 0.34 mol) and [1,3-bis(2,6-diisopropylphenyl)imidazol-2-ylidene](3-chloropyridyl)palladium(II) dichloride (1.2 g, 1.7 mmol) at room temperature, and the mixture was stirred for 10 min. Then, 4-fluorobenzylzinc bromide (0.5M THF solution, 0.70 L, 0.35 mol) was added, and the mixture was stirred for 18 hr. To the reaction mixture was added 5% aqueous citric acid solution, and the mixture was subjected to extraction with ethyl acetate. The organic layer was dried over anhydrous sodium sulfate, and the solvent was evaporated under reduced pressure. The obtained residue was purified by column chromatography (silica gel, petroleum ether/ethyl acetate) to give the title compound (30 g).
¹H NMR (400 MHz, DMSO-d₆) δ 1.25 (s, 6H), 1.45 (s, 9H), 3.68 (s, 2H), 3.92 (s, 2H), 7.13 (t, J = 8.8 Hz, 2H), 7.27-7.30 (m, 2H), 7.74 (brs, 1H), 8.04 (s, 1H); MS (m/z): 357.21 [M+H]⁺.

### (Step F) 6-(4-Fluorobenzyl)-3,3-dimethyl-2,3-dihydro-1H-pyrrolo[3,2-b]pyridine

To a 1,4-dioxane solution (5.0 mL) of tert-butyl 6-(4-fluorobenzyl)-3,3-dimethyl-2,3-dihydro-1H-pyrrolo[3,2-b]pyridine-1-carboxylate (0.48 g, 1.3 mmol) was added 4M hydrogen chloride/1,4-dioxane solution (5.0 mL), and the mixture was stirred at room temperature for 18 hr. The reaction solution was diluted with ethyl acetate and water, and the aqueous layer was separated. Aqueous sodium carbonate solution was added until the pH of the aqueous layer became alkaline, and then the aqueous layer was subjected to extraction with DCM. The obtained organic layer was dried over anhydrous sodium sulfate, and the solvent was evaporated under reduced pressure to give the title compound (0.30 g).
S (m/z): 257.22 [M+H]⁺.

### Reference Example 4

### (6-(4-Fluorobenzyl)-3,3-dimethyl-2,3-dihydro-1H-pyrrolo[3,2-b]pyridin-5-yl)methanol

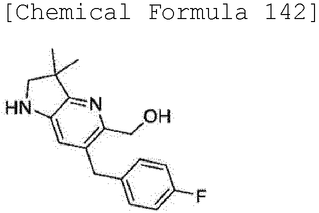

### (Step A) 5-Bromo-6-(4-fluorobenzyl)-3,3-dimethyl-2,3-dihydro-1H-pyrrolo[3,2-b]pyridine

To a DMF solution (0.60 L) of 6-(4-fluorobenzyl)-3,3-dimethyl-2,3-dihydro-1H-pyrrolo[3,2-b]pyridine (the compound of Reference Example 3, 59 g, 0.23 mol) was added N-bromosuccinimide (41 g, 0.23 mol) under ice-cooled, and the mixture was stirred at 0°C for 2 hr, and then at room temperature for 16 hr. To the reaction mixture was added ice water, and the resulting solid was collected by filtration, and dried under reduced pressure to give the title compound (37 g) as a crude product.
¹H NMR (400 MHz, CDCl₃) δ 1.34 (s, 6H), 3.37 (s, 2H), 3.65 (s, 1H), 3.94 (s, 2H), 6.51 (s, 1H), 6.97-7.01 (m, 2H), 7.14-7.18 (m, 2H); MS (m/z): 335.2 [M+H]⁺.

### (Step B) 6-(4-Fluorobenzyl)-3,3-dimethyl-2,3-dihydro-1H-pyrrolo[3,2-b]pyridine-5-carbaldehyde

To a THF solution (0.15 L) of 5-bromo-6-(4-fluorobenzyl)-3,3-dimethyl-2,3-dihydro-1H-pyrrolo[3,2-b]pyridine (15 g, 44 mmol) was added methyllithium (1.6M diethyl ether solution, 42 mL, 67 mmol) at -78°C, and the mixture was stirred for 30 min. Then, tert-butyllithium (1.7M pentane solution, 66 mL, 0.11 mol) was added, and the mixture was stirred for 30 min. Then, to the reaction solution was added dropwise DMF (15 mL), and the mixture was stirred for 30 min. To the reaction mixture was added saturated aqueous ammonium chloride solution, and the mixture was subjected to extraction with ethyl acetate. The organic layer was dried over anhydrous sodium sulfate, and the solvent was evaporated under reduced pressure to give the title compound (15 g).
¹H NMR (400 MHz, CDCl₃) δ 1.38 (s, 6H), 3.48 (s, 2H), 4.26-4.39 (m, 3H), 6.40 (s, 1H), 6.94-6.98 (m, 2 H), 7.14-7.18 (m, 2H), 10.00 (s, 1H). MS (m/z); 285.14 [M+H]⁺.

### (Step C) (6-(4-Fluorobenzyl)-3,3-dimethyl-2,3-dihydro-1H-pyrrolo[3,2-b]pyridin-5-yl)methanol

To a methanol solution (0.30 L) of 6-(4-fluorobenzyl)-3,3-dimethyl-2,3-dihydro-1H-pyrrolo[3,2-b]pyridine-5-carbaldehyde (29 g, 0.10 mol) was added sodium borohydride (4.5 g, 0.12 mol) at 0°C. The reaction solution was stirred at room temperature for 2 hr, ice water was added, and the mixture was subjected to extraction with ethyl acetate. The organic layer was dried over anhydrous sodium sulfate, and the solvent was evaporated under reduced pressure. The obtained crude product was purified by column chromatography (silica gel, petroleum ether/ethyl acetate) to give the title compound (17 g).
¹H NMR (400 MHz, DMSO-d₆) δ 1.20 (s, 6H), 3.20-3.21 (m, 2H), 3.92 (s, 2H), 4.46 (d, J = 5.2 Hz, 2H), 4.89-4.92 (m, 1H), 5.60 (brs, 1H), 6.45 (s, 1H), 7.08-7.13 (m, 2H), 7.21-7.25 (m, 2H);
MS (m/z): 287.29 [M+H]⁺.

### Reference Example 5

### (R)-4-(1-Aminoethyl)-N-(pyridin-4-yl)benzamide dihydrochloride

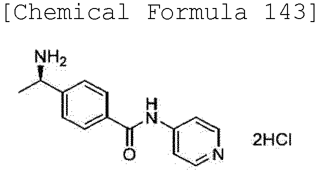

### (Step A) Methyl (R)-4-(1-((tert-butoxycarbonyl)amino)ethyl)benzoate

To a DCM solution (0.10 L) of methyl (R)-4-(1-aminoethyl)benzoate (5.0 g, 28 mmol) were added TEA (8.3 mL, 70 mmol) and di-tert-butyl dicarbonate (7.3 g, 33 mmol). The reaction solution was stirred at room temperature for 16 hr, diluted with water, and subjected to extraction with DCM. The organic layer was dried over anhydrous sodium sulfate, and the solvent was evaporated under reduced pressure to give the title compound (7.7 g).
¹H NMR (400 MHz, CDCl₃) δ 1.42-1.58 (m, 13H), 3.91 (s, 3H), 4.83 (brs, 1H), 7.35-7.37 (m, 2H), 7.99-8.02 (m, 2H); MS (m/z): 224.25 [M-56+H]⁺.

### (Step B) (R)-4-(1-((tert-Butoxycarbonyl)amino)ethyl)benzoic acid

To a THF/water mixed solution (35 mL:40 mL) of methyl (R)-4-(1-((tert-butoxycarbonyl)amino)ethyl)benzoate (7.7 g, 27 mmol) was added lithium hydroxide monohydrate (5.6 g, 0.14 mol) at room temperature, and the mixture was stirred for 16 hr. The reaction solution was concentrated under reduced pressure, to the residue was added saturated citric acid aqueous solution, and the precipitated solid was collected by filtration. The solid was washed with water, and dried under reduced pressure to give the title compound (6.7 g).
¹H NMR (400 MHz, DMSO-d₆) δ 1.42-1.59 (m, 13H), 4.86 (brs, 2H), 7.40 (d, J = 8 Hz, 2H), 8.06 (d, J = 8.4 Hz, 2H); MS (m/z): 266.36 [M+H] ⁺.

### (Step C) tert-Butyl (R)-(1-(4-(pyridin-4-ylcarbamoyl) phenyl) ethyl) carbamate

To a DMF solution (67 mL) of (R)-4-(1-((tert-butoxycarbonyl)amino)ethylbenzoic acid (6.7 g, 25 mmol) were added pyridin-4-amine (2.6 g, 28 mmol) and HATU (11 g, 28 mmol) at room temperature, and then DIPEA (11 mL, 60 mmol) was added. The reaction mixture was stirred for 18 hr, and diluted with water, and the precipitate was collected by filtration. The obtained solid was washed with water, and dried under reduced pressure to give the title compound (7.5 g).
¹H NMR (400 MHz, DMSO-d₆) δ 1.24-1.37 (m, 12H), 4.66-4.68 (m, 1H), 7.44-7.51 (m, 3H), 7.77 (d, J = 6.4 Hz, 2H), 7.90 (d, J = 8.0 Hz, 2H), 8.47 (d, J = 6.0 Hz, 2H), 10.52 (s, 1H); MS (m/z): 342.29 [M+H]⁺.

### (Step D) (R)-4-(1-Aminoethyl)-N-(pyridin-4-yl)benzamide dihydrochloride

To a 1,4-dioxane solution (38 mL) of tert-butyl (R)-(1-(4-(pyridin-4-ylcarbamoyl)phenyl)ethyl)carbamate (7.5 g, 22 mmol) was added 4M hydrogen chloride/1,4-dioxane solution (75 mL), and the mixture was stirred for 18 hr. The reaction solution was concentrated under reduced pressure, and the residue was washed with diethyl ether. The solid was collected by filtration, and dried under reduced pressure to give the title compound (6.1 g).
¹H NMR (400 MHz, DMSO-d₆) δ 1.55 (d, J = 6.8 Hz, 3H), 4.51-4.54 (m, 1H), 7.74 (d, J = 8.4 Hz, 2H), 8.17 (d, J = 8.4 Hz, 2H), 8.43 (d, J = 7.2 Hz, 2H), 8.72-8.77 (m, 5H), 11.85 (s, 1H), 15.29 (brs, 1H); MS (m/z): 242.30 [M+H]⁺.

### Reference Example 6

### (R)-2-Amino-3-(3-fluorophenyl)-N-(4-(pyridin-4-yl)phenyl)propenamide dihydrochloride

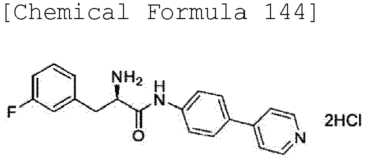

### (Step A) 4-(Pyridin-4-yl)aniline

To a DMF/water mixed solution (0.8 L:0.4 L) of 4-bromopyridine (20 g, 91 mmol) and 4-(4,4,5,5-tetramethyl-1,3,2-dioxaborolan-2-yl)aniline (18 g, 93 mmol) were added sodium carbonate (51 g, 0.46 mol) and 1,1'-bis(diphenylphosphino)ferrocene]palladium(II) dichloride dichloromethane adduct (3.7 g, 4.8 mmol) at room temperature. The reaction mixture was stirred at 130°C for 16 hr, and the reaction solution was diluted with water, and the precipitated solid was collected by filtration. The obtained solid was washed successively with water and petroleum ether, and dried under reduced pressure to give the title compound (14 g).
¹H NMR (400 MHz, DMSO-d₆) δ 3.88 (brs, 2H), 6.77 (d, J = 8.4 Hz, 2H), 6.48-7.50 (m, 4H), 8.57-8.58 (m, 2H); MS (m/z): 171.15 [M+H]⁺.

### (Step B) tert-Butyl (R)-(3-(3-fluorophenyl)-1-oxo-1-((4-(pyridin-4-yl)phenyl)amino)propan-2-yl)carbamate

To a DMF solution (0.25 L) of 4-(pyridin-4-yl)aniline (14 g, 78 mmol) were added DIPEA (25 mL, 0.18 mol), HATU (29 g, 77 mmol) and (R)-2-((tert-butoxycarbonyl)amino)-3-(3-fluorophenyl)propionic acid (20 g, 71 mmol) under ice-cooled. The mixture was stirred at room temperature for 16 hr, to the reaction mixture was added ice water, and the precipitate was collected by filtration. The obtained solid was dissolved in a methanol/DCM mixed solvent (10:90), and the solution was dried over anhydrous sodium sulfate. The solvent was evaporated under reduced pressure, and the obtained crude product was purified by column chromatography (petroleum ether/ethyl acetate) to give the title compound (16 g).
¹H NMR (400 MHz, DMSO-d₆) δ 1.44 (s, 9H), 3.10-3.25 (m, 2H), 4.46-4.48 (m, 1H), 5.05 (brs, 1H), 6.95-7.00 (m, 2H), 7.04-7.06 (m, 1H), 7.28-7.32 (m, 1H), 7.47 (d, J = 6 Hz, 2H), 7.52 -7.61 (m, 4H), 8.10 (brs, 1H), 8.63-8.64 (m, 2H); MS (m/z): 436.45 [M+H]⁺.

### (Step C) (R)-2-Amino-3-(3-fluorophenyl)-N-(4-(pyridin-4-yl)phenyl)propenamide dihydrochloride

To a DCM solution (0.20 L) of tert-butyl (R)-(3-(3-fluorophenyl)-1-oxo-1-((4-(pyridin-4-yl)phenyl)amino)propan-2-yl)carbamate (16 g, 38 mmol) was added 4M hydrogen chloride/1,4-dioxane solution (30 mL) under ice-cooled, and the mixture was stirred at room temperature for 16 hr. The reaction solution was concentrated under reduced pressure to give the title compound (13 g).
¹H NMR (500 MHz, DMSO-d₆) δ 3.12-3.17 (m, 1H), 3.27-3.32 (m, 1H), 4.39 (brs, 1H), 7.11 (dt, J₁ = 2.4 8.8 Hz, 1H), 7.34-7.40 (m, 1H), 7.84 (d, J = 8.4 Hz, 2H), 8.02-8.04 (m, 2H), 8.15-8.22 (m, 2H), 8.40-8.45 (brs, 3H), 8.84-8.85 (m, 2H), 11.35-11.38 (m, 1H). MS (m/z): 336.18 [M+H]⁺.

### Reference Example 7

### (S)-3-Amino-N-(isoquinolin-6-yl)-2-phenylpropanamide dihydrochloride

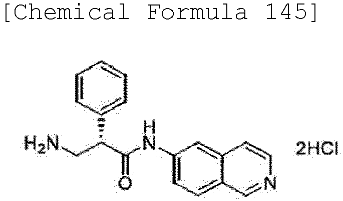

### (Step A) tert-Butyl (S)-(3-(isoquinolin-6-ylamino)-3-oxo-2-phenylpropyl) carbamate

To a DMF solution (0.10 L) of (S)-3-((tert-butoxycarbonyl)amino)-2-phenylpropionic acid (10 g, 39 mmol) were added DIPEA (17 mL, 98 mmol) and HATU (16 g, 43 mmol) under ice-cooled, and then isoquinolin-6-amine (6.2 g, 43 mmol) was added. The mixture was stirred at room temperature for 16 hr, and the reaction solution was diluted with ice water, and subjected to extraction with ethyl acetate. The organic layer was dried over anhydrous sodium sulfate, and the solvent was evaporated under reduced pressure to give the title compound (13 g).
¹H NMR (400 MHz, DMSO-d₆) δ 1.35 (s, 9H), 2.69-2.73 (m, 1H), 3.52-3.57 (m, 1H), 4.07-4.11 (m, 1H), 7.02-7.05 (m, 1H), 7.25-7.42 (m, 5H), 7.67-7.71 (m, 2H), 8.02-8.04 (m, 1H), 8.39-8.40 (m, 2H), 9.15 (s, 1H), 10.59 (s, 1H).

### (Step B) (S)-3-Amino-N-(isoquinolin-6-yl)-2-phenylpropanamide dihydrochloride

To a DCM solution (0.15 L) of tert-butyl (S)-(3-(isoquinolin-6-ylamino)-3-oxo-2-phenylpropyl)carbamate (13 g, 33 mmol) was added 4M hydrogen chloride/1,4-dioxane (27 mL), and the mixture was stirred at room temperature for 24 hr. The reaction solution was concentrated under reduced pressure to give the title compound (8.5 g).
¹H NMR (400 MHz, DMSO-d₆) δ 3.08-3.12 (m, 1H), 3.62-3.65 (m, 1H), 4.35-4.39 (m, 1H), 7.32-7.35 (m, 1H), 7.39-7.42 (m, 2H), 7.49-7.51 (m, 2H), 8.06 (dd, J = 9.2, 2.0 Hz, 1H), 8.15 (brs, 3H), 8.28 (d, J = 6.4 Hz, 1H), 8.40 (d, J = 9.2 Hz, 1H), 8.54 (d, J = 6.4 Hz, 1H), 8.68-8.69 (m, 1H), 11.52 (s, 1H); MS (m/z): 292.13 [M+H]⁺.

### Reference Example 8

### 2-(4-(4-((5-Chloro-4-((2-(isopropylsulfonyl)phenyl)amino)pyrimidin-2-yl)amino)-5-isopropoxy-2-methylphenyl)piperidin-1-yl)acetic acid

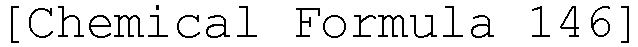

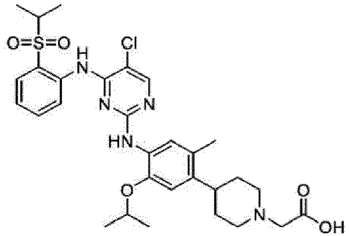

### (Step A) tert-Butyl 2-(4-(4-((5-chloro-4-((2-(isopropylsulfonyl)phenyl)amino)pyrimidin-2-yl)amino)-5-isopropoxy-2-methylphenyl)piperidin-1-yl) acetate

To a DCM solution (100 mL) of Ceritinib (10 g, 19 mmol) were added TEA (6.6 mL, 47 mmol) and tert-butyl 2-bromoacetate (5.5 g, 28 mmol) under ice-cooled, and the mixture was stirred at room temperature for 16 hr. The reaction solution was diluted with DCM, and washed with water. The organic layer was dried over anhydrous sodium sulfate, and the solvent was evaporated under reduced pressure to give the title compound (12 g).
¹H NMR (400 MHz, DMSO-d₆) δ 1.16 (d, J = 6.4 Hz, 6H), 1.22 (d, J = 6.0 Hz, 6H), 1.43 (s, 9H), 1.64 (d, J = 5.2 Hz, 4H), 2.11 (s, 3H), 2.27-2.33 (m, 2H), 2.56-2.61 (m, 1H), 2.94 (d, J = 11.2 Hz, 2H), 3.13 (s, 2H), 3.44 (t, J = 6.8 Hz, 1H), 4.58 (t, J = 6.0 Hz, 1H), 6.85 (s, 1H), 7.35 (t, J = 7.6 Hz, 1H), 7.50 (s, 1H), 7.60-7.64 (m, 1H), 7.83 (dd, J = 8.0, 1.6 Hz, 1H), 8.05 (s, 1H), 8.25 (s, 1H), 8.46 (d, J = 8.4 Hz, 1H), 9.46 (s, 1H); MS (m/z): 672.74 [M+H]⁺.

### (Step B) 2-(4-(4-((5-Chloro-4-((2-(isopropylsulfonyl)phenyl)amino)pyrimidin-2-yl)amino)-5-isopropoxy-2-methylphenyl)piperidin-1-yl)acetic acid

To tert-butyl 2-(4-(4-((5-chloro-4-((2-(isopropylsulfonyl)phenyl)amino)pyrimidin-2-yl)amino)-5-isopropoxy-2-methylphenyl)piperidin-1-yl)acetate (12 g, 17 mmol) was added TFA (110 mL), and the mixture was stirred at room temperature for 16 hr. The reaction solution was concentrated under reduced pressure, and the residue was diluted with DCM. The organic layer was washed with water and saturated aqueous sodium hydrogencarbonate solution, and dried over anhydrous sodium sulfate, and the solvent was evaporated to give the title compound (10 g).
¹H NMR (400 MHz, DMSO-d₆) δ 1.16 (d, J = 6.8 Hz, 6H), 1.23 (d, J = 6.0 Hz, 6H), 1.70 (d, J = 12.4 Hz, 2H), 1.87 (d, J = 13.2 Hz, 2H), 2.12 (s, 3H), 2.64 (t, J = 10.0 Hz, 2H), 2.73-2.75 (m, 1H), 3.21 (s, 3H), 3.23-3.27 (m, 4H), 4.56 (t, J = 6.4 Hz, 1H), 6.87 (s, 1H), 7.35 (t, J = 7.6 Hz, 1H), 7.51 (s, 1H), 7.61 (t, J = 7.2 Hz, 1H), 7.83 (d, J = 7.6 Hz, 1H), 8.07 (s, 1H), 8.24 (d, J = 7.2 Hz, 1H), 9.46 (brs, 1H); MS (m/z): 616.50 [M+H]⁺.

### Reference Example 9

### N²-(4-Aminophenyl)-N⁴-(3-(methylsulfonyl) benzyl) -5-N²-(4-aminophenyl)-N⁴-(3-(methylsulfonyl)benzyl)-5-(trifluoromethyl)pyrimidine-2,4-diamine

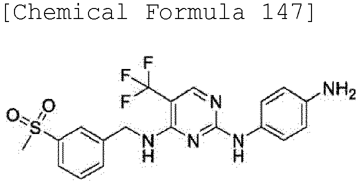

### (Step A) tert-Butyl (4-((4-chloro-5-(trifluoromethyl)pyrimidin-2-yl)amino)phenyl)carbamate

To a tert-butyl alcohol/DCE mixed solution (10 mL:10 mL) of 2,4-dichloro-5-(trifluoromethyl)pyrimidine (1.2 g, 5.8 mmol) was added zinc bromide (3.2 g, 14 mmol) under ice-cooled, and the mixture was stirred at room temperature for 1 hr. Then, tert-butyl (4-aminophenyl)carbamate (1.0 g, 4.8 mmol) and TEA (1.5 mL, 11 mmol) were added at 0°C, and the mixture was stirred at room temperature for 16 hr. The reaction mixture was concentrated under reduced pressure, and the obtained residue was purified by column chromatography (silica gel, petroleum ether/ethyl acetate) to give the title compound (1.2 g).
¹H NMR (400 MHz, CDCl₃) δ 1.52 (s, 9H), 6.48 (brs, 1H), 7.37-7.39 (m, 3H), 7.47-7.50 (m, 2H), 8.54 (brs, 1H); MS (m/z): 389.23 [M+H]⁺.

### (Step B) tert-Butyl (4-((4-((3-(methylsulfonyl)benzyl)amino)-5-(trifluoromethyl)pyrimidin-2-yl)amino)phenyl)carbamate

To a tert-butyl alcohol/DCE mixed solution (10 mL:10 mL) of tert-butyl (4-((4-chloro-5-(trifluoromethyl)pyrimidin-2-yl)amino)phenyl)carbamate (0.50 g, 1.3 mmol) were added DIPEA (1.1 mL, 6.4 mmol) and 3-methylsulfonylbenzylamine (0.60 g, 3.2 mmol) at room temperature, and the mixture was stirred at 80°C for 16 hr. The reaction mixture was concentrated under reduced pressure, and the obtained residue was triturated with a tert-butyl alcohol/DCE mixed solution (10 mL:10 mL) to give the title compound (0.45 g).
MS (m/z): 538.41 [M+H]⁺.

### (Step C) N²-(4-Aminophenyl)-N⁴-(3-(methylsulfonyl) benzyl) -5-(trifluoromethyl)pyrimidine-2,4-diamine

To a 1,4-dioxane solution (5.0 mL) of tert-butyl (4-((4-((3-(methylsulfonyl)benzyl)amino)-5-(trifluoromethyl)pyrimidin-2-yl)amino)phenyl)carbamate (0.45 g, 0.84 mmol) was added 4M hydrogen chloride/dioxane solution (30 mL), and the mixture was stirred at room temperature for 16 hr. To the reaction solution was added saturated aqueous sodium hydrogencarbonate solution, and the mixture was subjected to extraction with ethyl acetate. The organic layer was dried over anhydrous sodium sulfate, and the solvent was evaporated under reduced pressure to give the title compound (0.19 g).
MS (m/z): 438.32 [M+H]⁺.

### Reference Example 10

### (R)-4-(1-((2-(2-Methoxyethoxy)ethyl)amino)ethyl)-N-(pyridin-4-yl)benzamide

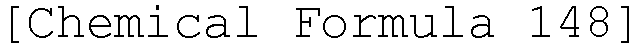

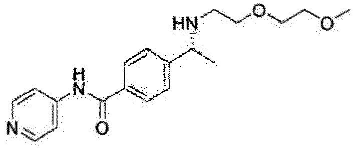

To an acetonitrile solution (5.0 mL) of (R)-4-(1-aminoethyl)-N-(pyridin-4-yl)benzamide dihydrochloride (the compound of Reference Example 5, 0.26 g, 0.83 mmol) were added potassium carbonate (0.23 g, 1.7 mmol) and potassium iodide (catalytic amount), followed by 1-bromo-2-(2-methoxyethoxy)ethane (0.15 g, 0.83 mmol) at room temperature. The reaction solution was stirred at 50°C for 24 hr, and diluted with ethyl acetate. The suspension was filtered to remove the inorganic salt, the filtrate was concentrated under reduced pressure, and the obtained crude product was purified by HPLC preparative chromatography (column: X-Bridge, eluent: 10 mM ammonium hydrogencarbonate aqueous solution/acetonitrile, gradient mode) to give the title compound (0.035 g).
¹H NMR (400 MHz, DMSO-d₆) δ 1.27 (d, J = 6.8 Hz, 3H), 2.12 (brs, 1H), 2.40-2.45 (m, 1H), 2.53-2.54 (m, 1H), 3.23 (s, 3H), 3.41-3.44 (m, 4H), 3.46-3.49 (m, 2H), 3.78-3.81 (m, 1H), 7.50 (d, J = 8.4 Hz, 2H), 7.78 (dd, J = 4.8, 1.6 Hz, 2H), 7.90 (d, J = 8.0 Hz, 2H), 8.47 (d, J = 6.0 Hz, 2H), 10.52 (s, 1H); MS (m/z): 344.3 [M+H]⁺.

### Reference Example 11

### (R)-4-(1-((3-(2-Methoxyethoxy)propyl)amino)ethyl)-N-(pyridin-4-yl)benzamide

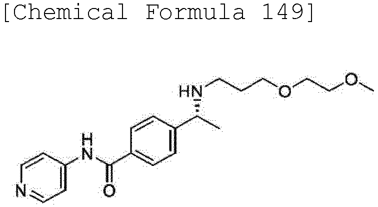

(R)-4-(1-Aminoethyl)-N-(pyridin-4-yl)benzamide dihydrochloride (the compound of Reference Example 5) was reacted in the same manner as in Reference Example 10 using 1-bromo-3-(2-methoxyethoxy)propane instead of 1-bromo-2-(2-methoxyethoxy)ethane to give the title compound (0.025 g).
¹H NMR (400 MHz, DMSO-d₆) δ 1.25 (d, J = 6.8 Hz, 3H), 1.57-1.63 (m, 2H), 2.27-2.34 (m, 1H), 2.37-2.43 (m, 1H), 3.21 (s, 3H), 3.38-3.48 (m, 7H), 3.73-3.78 (m, 1H), 7.49 (d, J = 8.0 Hz, 2H), 7.78 (dd, J = 4.8, 1.2 Hz, 2H), 7.89 (d, J = 8.0 Hz, 2H), 8.47 (d, J = 6.4 Hz, 2H), 10.51 (s, 1H); MS (m/z): 358.38 [M+H]⁺.

### Reference Example 12

### (R)-3-(3-Fluorophenyl)-2-((2-(2-methoxyethoxy)ethyl)amino)-N-(4-(pyridin-4-yl)phenyl)propanamide

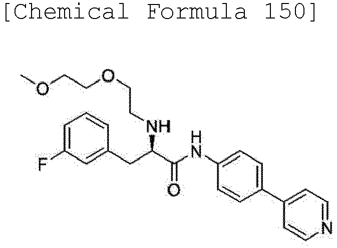

To an acetonitrile solution (10 mL) of (R)-2-amino-3-(3-fluorophenyl)-N-(4-(pyridin-4-yl)phenyl)propanamide dihydrochloride (the compound of Reference Example 6, 1.2 g, 3.0 mmol) were added 1-bromo-2-(2-methoxyethoxy)ethane (0.60 g, 3.3 mmol), potassium carbonate (1.2 g, 9.0 mmol) and potassium iodide (0.050 g) at room temperature. The reaction solution was stirred at 60°C for 48 hr, and the solvent was evaporated. The residue was purified by HPLC preparative chromatography (column: Inertsil ODS, eluent: 10 mM ammonium hydrogencarbonate aqueous solution/acetonitrile, gradient mode) to give the title compound (0.45 g).
¹H NMR (400 MHz, DMSO-d₆) δ 2.21 (brs, 1H), 2.60-2.67 (m, 2H), 2.87-2.89 (m, 1H), 2.95-2.96 (m, 1H), 3.22 (s, 3H), 3.39-3.50 (m, 7H), 7.01 (t, J = 8.8 Hz, 1H), 7.09 (t, J = 10 Hz, 2H), 7.27-7.33 (m, 1H), 7.68-7.80 (m, 6H), 8.6 (d, J = 4.8 Hz, 2H), 10.06 (s, 1H); MS (m/z): 438.34 [M+H]⁺.

### Reference Example 13

### (R)-3-(3-Fluorophenyl)-2-((3-(2-methoxyethoxy)propyl)amino)-N-(4-(pyridin-4-yl)phenyl)propanamide

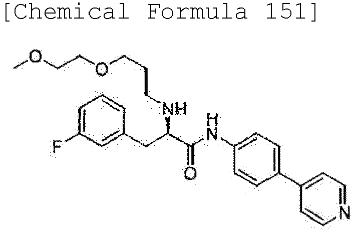

(R)-2-Amino-3-(3-fluorophenyl)-N-(4-(pyridin-4-yl)phenyl)propanamide dihydrochloride (the compound of Reference Example 6) was reacted in the same manner as in Reference Example 12 using 1-bromo-3-(2-methoxyethoxy)propane instead of 1-bromo-2-(2-methoxyethoxy)ethane to give the title compound (54 mg).
¹H NMR (400 MHz, DMSO-d₆) δ 1.60 (t, J = 6.4 Hz, 2H), 2.16 (brs, 1H), 2.44-2.47 (m, 1H), 2.56-2.58 (m, 1H), 2.84-2.87 (m, 1H), 2.91-2.96 (m, 1H), 3.20 (s, 3H), 3.36-3.47 (m, 7H), 7.00 (td, J = 8.4, 2.4 Hz, 1H), 7.09 (t, J = 8.8 Hz, 2H), 7.26-7.32 (m, 1H), 7.68 (dd, J = 4.8, 1.6 Hz, 2H), 7.71-7.79 (m, 4H), 8.60 (d, J = 4.8 Hz, 2H), 10.02 (s, 1H); MS (m/z): 452.33 [M+H]⁺.

### Reference Example 14

### (S)-N-(Isoquinolin-6-yl)-3-((2-(2-methoxyethoxy)ethyl)amino)-2-phenylpropanamide

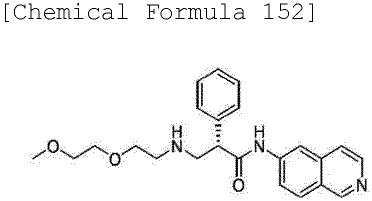

To an acetonitrile solution (3.0 mL) of (S)-3-amino-N-(isoquinolin-6-yl)-2-phenylpropanamide dihydrochloride (the compound of Reference Example 7, 0.38 g, 1.0 mmol) were added 1-bromo-2-(2-methoxyethoxy)ethane (0.28 g, 1.5 mmol), potassium carbonate (0.43 g, 3.1 mmol) and potassium iodide (0.010 g). The reaction solution was stirred at 60°C for 18 hr, and diluted with water, and the aqueous layer was subjected to extraction with a methanol/DCM mixed solvent (10:90). The organic layer was washed with saturated brine, and dried over anhydrous sodium sulfate, and the solvent was evaporated under reduced pressure. The obtained crude product was purified by HPLC preparative chromatography (column: X-Bridge, eluent: 10 mM ammonium hydrogencarbonate aqueous solution/acetonitrile, gradient mode) to give the title compound (0.50 g).
¹H NMR (400 MHz, DMSO-d₆) δ 2.32-2.33 (m, 1H), 2.67-2.72 (m, 2H), 2.81-2.85 (m, 1H), 3.17 (s, 3H), 3.26 (s, 1H), 3.35-3.38 (m, 2H), 3.43-3.48 (m, 4H), 3.94 (dd, J = 9.2, 5.2 Hz, 1H), 7.24-7.28 (m, 1H), 7.32-7.36 (m, 2H), 7.39-7.42 (m, 2H), 7.66-7.71 (m, 2H), 8.04 (d, J = 8.8 Hz, 1H), 8.38-8.41 (m, 2H), 9.15 (s, 1H), 10.64 (brs, 1H); MS (m/z): 394.42 [M+H]⁺.

### Reference Example 15

### (S)-N-(Isoquinolin-6-yl)-3-((3-(2-methoxyethoxy)propyl)amino)-2-phenylpropanamide

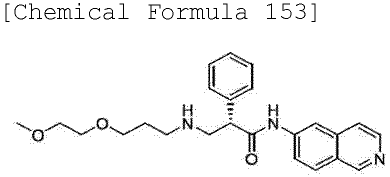

(S)-3-Amino-N-(isoquinolin-6-yl)-2-phenylpropanamide dihydrochloride (the compound of Reference Example 7) was reacted in the same manner as Reference Example 14 using 1-bromo-3-(2-methoxyethoxy)propane instead of 1-bromo-2-(2-methoxyethoxy)ethane in to give the title compound (0.085 g). ¹H NMR (400 MHz, DMSO-d₆) δ 1.59-1.64 (m, 3H), 2.61 (d, J = 7.6 Hz, 2H), 2.77 (dd, J = 11.6, 4.8 Hz, 1H), 3.18 (s, 3H), 3.24-3.29 (m, 1H), 3.37-3.40 (m, 6H), 3.91-3.94 (m, 1H), 7.24-7.27 (m, 1H), 7.34 (t, J = 7.6 Hz, 2H), 7.40-7.42 (m, 2H), 7.66-7.72 (m, 2H), 8.04 (d, J = 9.2 Hz, 1H), 8.40 (d, J = 6.0 Hz, 2H), 9.15 (s, 1H), 10.62 (brs, 1H); MS (m/z): 408.17 [M+H]⁺.

### Example 1

### 4-((R)-1-((2-(2-((((2R,5R)-1-(2-(6-(4-Fluorobenzyl)-3,3-dimethyl-2,3-dihydro-1H-pyrrolo[3,2-b]pyridin-1-yl)-2-oxoethyl)-5-methylpiperazin-2-yl)methyl)(methyl)amino)ethoxy)ethyl)amino)ethyl)-N-(pyridin-4-yl)benzamide

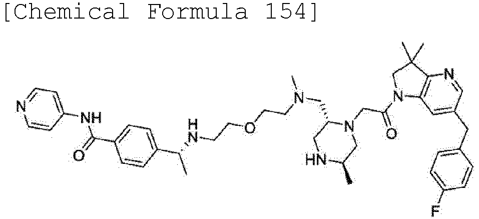

### (Step A) N-methyl-2-nitrobenzenesulfonamide

To a THF solution (0.50 L) of 2-nitrobenzenesulfonyl chloride (50 g, 0.23 mol) were added TEA (65 mL, 0.45 mol) and methylamine (2M THF solution, 0.14 L, 0.27 mol) under ice-cooled. The reaction solution was stirred at room temperature for 2 hr, and the solvent was evaporated under reduced pressure. To the residue was added ethyl acetate, and the mixture was washed with water and saturated brine. The organic layer was dried over anhydrous sodium sulfate, and the solvent was evaporated under reduced pressure to give the title compound (30 g).
MS (m/z): 215.04 [M-H]⁻.

### (Step B) 2-(2-Hydroxyethoxy)ethyl 4-methylbenzenesulfonate

To a THF/water mixed solution (60 mL:40 mL) of bis(2-hydroxyethyl)ether (10 g, 94 mmol) was added sodium hydroxide (5.7 g, 0.14 mol) under ice-cooled, and the mixture was stirred for 5 min. A THF solution (0.10 L) of p-toluenesulfonyl chloride (20 g, 0.10 mol) was added dropwise thereto, and the mixture was stirred at room temperature for 2 hr. The reaction solution was diluted with ethyl acetate, and washed with water. The organic layer was dried over anhydrous sodium sulfate, and the solvent was evaporated under reduced pressure. The obtained crude product was purified by column chromatography (silica gel, petroleum ether/ethyl acetate) to give the title compound (10 g).
¹H NMR (400 MHz, CDCl₃) δ 2.45 (s, 3H), 3.53 (t, J = 4.4 Hz, 2H), 3.66-3.70 (m, 4H), 4.20 (t, J = 3.6 Hz, 2H), 7.35 (d, J = 8.0 Hz, 2H), 7.81 (d, J = 8.4 Hz, 2H); MS (m/z): 261.06 [M+H]⁺.

### (Step C) N-(2-(2-Hydroxyethoxy)ethyl)-N-methyl-2-nitrobenzenesulfonamide

To an acetonitrile solution (20 mL) of N-methyl-2-nitrobenzenesulfonamide (3.4 g, 16 mmol) were added successively 2-(2-hydroxyethoxy)ethyl 4-methylbenzenesulfonate (4.5 g, 17 mmol), potassium carbonate (6.5 g, 47 mmol) and potassium iodide (catalytic amount). The reaction mixture was stirred at 70°C for 16 hr, diluted with ethyl acetate, and washed with water. The organic layer was dried over anhydrous sodium sulfate, and the solvent was evaporated under reduced pressure. The obtained crude product was purified by column chromatography (silica gel, petroleum ether/ethyl acetate) to give the title compound (4.0 g).
¹H NMR (400 MHz, DMSO-d₆) δ 2.88 (s, 3H), 3.36-3.41 (m, 4H), 3.44-3.48 (m, 2H), 3.56 (t, J = 5.6 Hz, 2H), 4.58 (t, J = 5.2 Hz, 1H), 7.82-7.91 (m, 2H), 7.97-8.03 (m, 2H). MS (m/z): 305.05 [M+H]⁺.

### (Step D) 2-(2-((N-Methyl-2-nitrophenyl)sulfonamido)ethoxy)ethyl methanesulfonate

To a DCM solution (50 mL) of N-(2-(2-hydroxyethoxy)ethyl)-N-methyl-2-nitrobenzenesulfonamide (4.8 g, 16 mmol) were added methanesulfonyl chloride (1.8 mL, 24 mmol) and TEA (6.6 mL, 47 mmol) under ice-cooled, and the reaction mixture was stirred at room temperature for 2 hr. The reaction solution was diluted with DCM, and washed with aqueous sodium hydrogencarbonate solution. The organic layer was dried over anhydrous sodium sulfate, and the solvent was evaporated under reduced pressure. The obtained crude product was purified by column chromatography (silica gel, petroleum ether/ethyl acetate) to give the title compound (5.0 g).
¹H NMR (400 MHz, CDCl₃) δ 2.98 (s, 3H), 3.06 (s, 3H), 3.47 (t, J = 5.2 Hz, 2H), 3.68-3.74 (m, 4H), 4.32-4.34 (m, 2H), 7.62-7.64 (m, 1H), 7.69-7.72 (m, 2H), 7.99-8.02 (m, 1H). MS (m/z): 383.27 [M+H]⁺.

### (Step E) (R)-4-(1-((2-(2-((N-Methyl-2-nitrophenyl)sulfonamido)ethoxy)ethyl)amino)ethyl)-N-(pyridin-4-yl)benzamide

To an acetonitrile solution (20 mL) of (R)-4-(1-aminoethyl)-N-(pyridin-4-yl)benzamide dihydrochloride (the compound of Reference Example 5, 1.2 g, 3.8 mmol) were added 2-(2-((N-methyl-2-nitrophenyl)sulfonamido)ethoxy)ethyl methanesulfonate (1.6 g, 4.2 mmol), potassium carbonate (2.6 g, 19 mmol) and potassium iodide (catalytic amount) at room temperature. The mixture was stirred at 70°C for 24 hr, and the reaction solution was diluted with ethyl acetate, and washed with water. The organic layer was dried over anhydrous sodium sulfate, and the solvent was evaporated under reduced pressure. The obtained crude product was purified by HPLC preparative chromatography (column: LUNA, eluent: 0.1% formic acid aqueous solution/acetonitrile, gradient mode) to give the title compound (0.30 g).
¹H NMR (400 MHz, DMSO-d₆) δ 1.25 (d, J = 6.8 Hz, 3H), 2.09 (brs, 1H), 2.37-2.41 (m, 1H), 2.45-2.48 (m, 1H), 2.89 (s, 3H), 3.36-3.42 (m, 4H), 3.52 (t, J = 5.6 Hz, 2H), 3.79 (d, J = 6.4 Hz, 1H), 7.49 (d, J = 8.4 Hz, 2H), 7.77 (dd, J = 4.8, 1.2 Hz, 2H), 7.82-7.86 (m, 2H), 7.87-7.91 (m, 2H), 7.96-8.01 (m, 2H), 8.22 (dd, J = 4.8, 1.6 Hz, 2H), 10.52 (s, 1H). MS (m/z): 528.42 [M+H]⁺.

### (Step F) tert-Butyl (R)-(2-(2-((N-methyl-2-nitrophenyl)sulfonamido)ethoxy)ethyl)(1-(4-(pyridin-4-ylcarbamoyl) phenyl) ethyl) carbamate

To a DCM solution (3.0 mL) of (R)-4-(1-((2-(2-((N-methyl-2-nitrophenyl)sulfonamido)ethoxy)ethyl)amino)ethyl)-N-(pyridin-4-yl)benzamide (0.30 g, 0.57 mmol) were added di-tert-butyl dicarbonate (0.15 mL, 0.68 mmol) and TEA (0.19 mL, 1.4 mmol) under ice-cooled. The mixture was stirred at room temperature for 16 hr, and the reaction solution was diluted with DCM, and washed with water. The organic layer was dried over anhydrous sodium sulfate, and the solvent was evaporated under reduced pressure to give the title compound (0.35 g).
MS (m/z): 628.88 [M+H].

### (Step G) tert-Butyl (R)-(2-(2-(methylamino)ethoxy)ethyl) (1-(4-(pyridin-4-ylcarbamoyl)phenyl)ethyl)carbamate

To a DMF solution (3.5 mL) of tert-butyl (R)-(2-(2-((N-methyl-2-nitrophenyl)sulfonamido)ethoxy)ethyl)(1-(4-(pyridin-4-ylcarbamoyl) phenyl) ethyl) carbamate (0.35 g, 0.56 mmol) were added thiophenol (0.080 mL, 0.84 mmol) and potassium carbonate (0.12 g, 0.84 mmol) under ice-cooled. The reaction solution was stirred at room temperature for 2 hr, and diluted with water and ethyl acetate, the aqueous layer was separated, and the organic layer was washed with 1M hydrochloric acid. The washing was alkalified with aqueous sodium hydroxide solution, and subjected to extraction with a methanol/DCM mixed solvent (10:90). The obtained organic layer was dried over anhydrous sodium sulfate, and the solvent was evaporated under reduced pressure to give the title compound (0.22 g).
MS (m/z): 443.54 [M+H]⁺.

### (Step H) 2-Chloro-1-(6-(4-fluorobenzyl)-3,3-dimethyl-2,3-dihydro-1H-pyrrolo[3,2-b]pyridin-1-yl)ethan-1-one

To a THF solution (5.0 mL) of 6-(4-fluorobenzyl)-3,3-dimethyl-2,3-dihydro-1H-pyrrolo[3,2-b]pyridine (the compound of Reference Example 3, 0.50 g, 1.2 mmol) were added successively TEA (0.34 g, 3.3 mmol) and chloroacetyl chloride (0.18 g, 1.6 mmol), and the mixture was stirred at room temperature for 4 hr. The reaction mixture was diluted with ethyl acetate, and washed with aqueous sodium hydrogencarbonate solution. The organic layer was dried over anhydrous sodium sulfate, and the solvent was evaporated under reduced pressure to give the title compound (0.40 g).
¹H NMR (500 MHz, DMSO-d6) δ 1.42 (s, 6H), 3.92-3.94 (m, 4H), 4.14 (s, 2H), 6.97 (t, J = 9.0 Hz, 2H), 7.05-7.08 (m, 1H), 7.14-7.17 (m, 1H), 8.12-8.13 (m, 1H), 8.20-8.21 (m, 1H); MS (m/z): 333.33 [M+H]⁺.

### (Step I) tert-Butyl (2R,5R)-4-(2-(6-(4-fluorobenzyl)-3,3-dimethyl-2,3-dihydro-1H-pyrrolo[3,2-c]pyridin-1-yl)-2-oxoethyl)-5-(hydroxymethyl)-2-methylpiperazine-1-carboxylate

To an acetonitrile solution (5.0 mL) of 2-chloro-1-(6-(4-fluorobenzyl)-3,3-dimethyl-2,3-dihydro-1H-pyrrolo[3,2-b]pyridin-1-yl)ethan-1-one (0.26 g, 0.78 mmol) were added tert-butyl (2R,5R)-5-(hydroxymethyl)-2-methylpiperazine-1-carboxylate (the compound of Reference Example 1, 0.19 g, 0.86 mmol), potassium carbonate (0.22 g, 1.6 mmol) and potassium iodide (catalytic amount), and the mixture was stirred at room temperature for 16 hr. The reaction solution was diluted with ethyl acetate, and filtered through Celite, and the filtrate was washed with water, and dried over anhydrous sodium sulfate. The solvent was evaporated under reduced pressure to give the title compound (0.40 g).
MS (m/z): 527.23 [M+H]⁺.

### (Step J) tert-Butyl (2R,5R)-5-(chloromethyl)-4-(2-(6-(4-fluorobenzyl)-3,3-dimethyl-2,3-dihydro-1H-pyrrolo[3,2-b]pyridin-1-yl)-2-oxoethyl)-2-methylpiperazine-1-carboxylate

To a DCM solution (10 mL) of tert-butyl (2R,5R)-4-(2-(6-(4-fluorobenzyl)-3,3-dimethyl-2,3-dihydro-1H-pyrrolo[3,2-c]pyridin-1-yl)-2-oxoethyl)-5-(hydroxymethyl)-2-methylpiperazine-1-carboxylate (0.40 g, 0.94 mmol) were added successively TEA (0.19 g, 1.9 mmol) and methanesulfonyl chloride (0.13 g, 1.1 mmol). The reaction solution was stirred at room temperature for 16 hr, diluted with a mixture of 10% methanol/DCM, and washed with water. The organic layer was dried over anhydrous sodium sulfate, and the solvent was evaporated under reduced pressure. The obtained residue was purified by column chromatography (silica gel, petroleum ether/ethyl acetate) to give the title compound (0.30 g).
¹H NMR (400 MHz, DMSO-d₆) δ 1.09-1.19 (m, 3H), 1.28 (s, 6H), 1.41 (s, 9H), 2.69-2.71 (m, 2H), 3.03-3.06 (m, 1H), 3.20-3.30 (m, 2H), 3.36-3.38 (m, 3H), 3.48-3.52 (m, 1H), 3.70-3.74 (m, 2H), 3.90-4.00 (m, 3H), 7.10-7.14 (m, 2H), 7.26-7.29 (m, 2H), 8.09 (s, 2H); MS (m/z): 545.55 [M+H]⁺.

### (Step K) tert-Butyl (2R,5S)-4-(2-(6-(4-fluorobenzyl)-3,3-dimethyl-2,3-dihydro-1H-pyrrolo[3,2-b]pyridin-1-yl)-2-oxoethyl)-2-methyl-5-(2,11,11-trimethyl-9-oxo-8-((R)-1-(4-(pyridin-4-ylcarbamoyl)phenyl)ethyl)-5,10-dioxa-2,8-diazadodecyl)piperazine-1-carboxylate

To an acetonitrile solution (3.0 mL) of tert-butyl (R)-(2-(2-(methylamino)ethoxy)ethyl) (1-(4-(pyridin-4-ylcarbamoyl) phenyl) ethyl) carbamate (0.12 g, 0.27 mmol) were added successively tert-butyl (2R,5R)-5-(chloromethyl)-4-(2-(6-(4-fluorobenzyl)-3,3-dimethyl-2,3-dihydro-1H-pyrrolo[3,2-b]pyridin-1-yl)-2-oxoethyl)-2-methylpiperazine-1-carboxylate (0.16 g, 0.28 mmol), potassium carbonate (0.11 g, 0.81 mmol) and potassium iodide (catalytic amount) at room temperature. The mixture was stirred at 70°C for 16 hr, diluted with ethyl acetate, and washed with water. The organic layer was dried over anhydrous sodium sulfate, and the solvent was evaporated under reduced pressure. The obtained crude product was purified by HPLC preparative chromatography (column: X-SELECT CSH C18, eluent: 10 mM ammonium hydrogencarbonate aqueous solution/acetonitrile, gradient mode) to give the title compound (0.060 g).
MS (m/z): 951.89 [M+H]⁺.

### (Step L) 4-((R)-1-((2-(2-((((2R,5R)-1-(2-(6-(4-Fluorobenzyl)-3,3-dimethyl-2,3-dihydro-1H-pyrrolo[3,2-b]pyridin-1-yl)-2-oxoethyl)-5-methylpiperazin-2-yl)methyl)(methyl)amino)ethoxy)ethyl)amino)ethyl)-N-(pyridin-4-yl)benzamide

To a DCM solution (1.0 mL) of tert-butyl (2R,5S)-4-(2-(6-(4-fluorobenzyl)-3,3-dimethyl-2,3-dihydro-1H-pyrrolo[3,2-b]pyridin-1-yl)-2-oxoethyl)-2-methyl-5-(2,11,11-trimethyl-9-oxo-8-((R)-1-(4-(pyridin-4-ylcarbamoyl)phenyl)ethyl)-5,10-dioxa-2,8-diazadodecyl)piperazine-1-carboxylate (0.060 g, 0.063 mmol) was added TFA (1.0 mL) under ice-cooled. The reaction solution was stirred at room temperature for 3 hr, aqueous sodium hydrogencarbonate solution was added, and the mixture was subjected to extraction with DCM. The organic layer was dried over anhydrous sodium sulfate, and the solvent was evaporated under reduced pressure to give the title compound (0.040 g).
¹H NMR (400 MHz, DMSO-d₆) δ 0.86 (d, J = 6.11 Hz, 3H), 1.25 (s, 6H), 1.98-2.01 (m, 1H), 2.10 (s, 3H), 2.24-2.28 (m, 4H), 2.30-2.35 (m, 5H), 2.42-2.44 (m, 2H), 2.62-2.67 (m, 4H), 2.78 (d, J = 11.6 Hz, 1H), 3.21-3.26 (m, 4H), 3.37 (brs, 1H), 3.74 (d, J = 6.4 Hz, 1H), 3.80 (t, J = 4.4 Hz, 2H), 3.90 (s, 2H), 3.97 (d, J = 10.8 Hz, 1H), 7.09 (t, J = 9.2 Hz, 2H), 7.26 (dd, J = 8.44, 5.50 Hz, 2H), 7.45 (d, J = 8.4 Hz, 2H), 7.77 (dd, J = 4.8, 1.2 Hz, 2H), 7.89 (d, J = 8.40 Hz, 2H), 8.07 (s, 2H), 8.47 (dd, J = 4.81, 1.60 Hz, 2H), 10.50 (s, 1H); MS (m/z):751.69 [M+H]⁺; retention time/analysis method: 3.12 min/D.

### Example 8

### (R)-2-((2-(2-((((2R,5R)-1-(2-(6-(4-Fluorobenzyl)-3,3-dimethyl-2,3-dihydro-1H-pyrrolo[3,2-b]pyridin-1-yl)-2-oxoethyl)-5-methylpiperazin-2-yl)methyl) (methyl)amino)ethoxy)ethyl)amino)-3-(3-fluorophenyl)-N-(4-(pyridin-4-yl)phenyl)propanamide

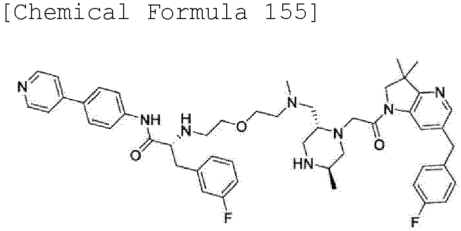

### (Step A) Methyl (R)-3-(3-fluorophenyl)-2-((2-(2-((N-methyl-2-nitrophenyl)sulfonamido)ethoxy)ethyl)amino)propanoate

To an acetonitrile solution (20 mL) of methyl (R)-2-amino-3-(3-fluorophenyl)propanoate hydrochloride (2.0 g, 8.6 mmol) were added 2-(2-((N-methyl-2-nitrophenyl) sulfonamido) ethoxy) ethyl methanesulfonate (the compound of Example 1, Step D, 3.6 g, 9.4 mmol), potassium carbonate (5.9 g, 43 mmol) and potassium iodide (0.050 g) at room temperature. The mixture was stirred at 70°C for 24 hr, diluted with ethyl acetate, and washed with water. The organic layer was dried over anhydrous sodium sulfate, and the solvent was evaporated under reduced pressure. The obtained crude product was purified by column chromatography (silica gel, petroleum ether/ethyl acetate) to give the title compound (1.0 g).
MS (m/z): 484.37 [M+H]⁺.

### (Step B) (R)-2-((tert-Butoxycarbonyl)(2-(2-((N-methyl-2-nitrophenyl)sulfonamido)ethoxy)ethyl)amino)-3-(3-fluorophenyl)propanoic acid

To a THF/water mixed solution (7.5 mL:7.5 mL) of methyl (R)-3-(3-fluorophenyl)-2-((2-(2-((N-methyl-2-nitrophenyl)sulfonamido)ethoxy)ethyl)amino)propanoate (1.7 g, 3.5 mmol) was added lithium hydroxide monohydrate (0.44 g, 11 mmol), and the mixture was stirred at room temperature for 16 hr. Then, to the reaction solution was added di-tert-butyl dicarbonate (1.2 g, 5.3 mmol), and the mixture was stirred for 18 hr. The reaction solution was diluted with aqueous citric acid solution, and subjected to extraction with ethyl acetate. The organic layer was dried over anhydrous sodium sulfate, and the solvent was evaporated under reduced pressure. The obtained crude product was purified by column chromatography (silica gel, DCM/methanol) to give the title compound (1.4 g). MS (m/z): 570.22 [M+H]⁺.

### (Step C) tert-Butyl (R)-(3-(3-fluorophenyl)-1-oxo-1-((4-(pyridin-4-yl)phenyl)amino)propan-2-yl) (2-(2-((N-methyl-2-nitrophenyl)sulfonamido)ethoxy)ethyl)carbamate

To a DMF solution (15 mL) of (R)-2-((tert-butoxycarbonyl)(2-(2-((N-methyl-2-nitrophenyl)sulfonamido)ethoxy)ethyl)amino)-3-(3-fluorophenyl)propanoic acid (1.4 g, 2.5 mmol) were added 4-(pyridin-4-yl)aniline (0.49 g, 2.7 mmol), HATU (1.1 g, 2.7 mmol) and DIPEA (1.2 mL, 6.2 mmol) at room temperature, and the mixture was stirred for 16 hr. The reaction solution was diluted with ethyl acetate, and washed with water. The organic layer was dried over anhydrous sodium sulfate, and the solvent was evaporated under reduced pressure. The obtained crude product was purified by column chromatography (silica gel, petroleum ether/ethyl acetate) to give the title compound (0.60 g).
¹H NMR (400 MHz, DMSO-d₆) δ 1.31-1.34 (m, 9H), 2.67-2.69 (m, 1H), 2.73 (s, 2H), 2.86-2.89 (m, 6H), 3.11 (t, J = 10.4 Hz, 1H), 3.41 (brs, 2H), 3.53-3.62 (m, 2H), 4.52 (brs, 1H), 4.84 (s, 1H), 7.08 (d, J = 7.6 Hz, 2H), 7.34 (brs, 1H), 7.65-7.75 (m, 3H), 7.78-7.88 (m, 3H), 7.94-7.99 (m, 2H), 8.60 (d, J = 6.0 Hz, 2H), 9.59 (brs, 1H), 10.02 (brs, 1H). MS (m/z): 722.44 [M+H]⁺.

### (Step D) tert-Butyl (R)-(3-(3-fluorophenyl)-1-oxo-1-((4-(pyridin-4-yl)phenyl)amino)propan-2-yl) (2-(2-(methylamino) ethoxy) ethyl) carbamate

To a DMF solution (3.5 mL) of tert-butyl (R)-(3-(3-fluorophenyl)-1-oxo-1-((4-(pyridin-4-yl)phenyl)amino)propan-2-yl) (2-(2-((N-methyl-2-nitrophenyl)sulfonamido)ethoxy)ethyl)carbamate (0.30 g, 0.42 mmol) were added thiophenol (0.060 mL, 0.62 mmol) and potassium carbonate (0.086 g, 0.62 mmol) under ice-cooled. The mixture was stirred at room temperature for 2 hr, and the reaction solution was diluted with water and ethyl acetate, and the aqueous layer was separated. The organic layer was washed with 1M hydrochloric acid, and the washing was alkalified with aqueous sodium hydroxide solution, and subjected to extraction with a methanol/DCM mixed solution (10:90). The organic layer was dried over anhydrous sodium sulfate, and the solvent was evaporated under reduced pressure to give the title compound (0.20 g).
MS (m/z): 537.50 [M+H]⁺.

### (Step E) tert-Butyl (2R,5S)-4-(2-(6-(4-fluorobenzyl)-3,3-dimethyl-2,3-dihydro-1H-pyrrolo[3,2-b]pyridin-1-yl)-2-oxoethyl)-5-(8-((R)-3-(3-fluorophenyl)-1-oxo-1-((4-(pyridin-4-yl)phenyl) amino)propan-2-yl)-2,11,11-trimethyl-9-oxo-5,10-dioxa-2,8-diazadodecyl)-2-methylpiperazine-1-carboxylate

To an acetonitrile solution (1.0 mL) of tert-butyl (R)-(3-(3-fluorophenyl)-1-oxo-1-((4-(pyridin-4-yl)phenyl)amino)propan-2-yl) (2-(2-((N-methyl-2-nitrophenyl)sulfonamido)ethoxy)ethyl)carbamate (0.10 g, 0.19 mmol) were added tert-butyl (2R,5R)-5-(chloromethyl)-4-(2-(6-(4-fluorobenzyl)-3,3-dimethyl-2,3-dihydro-1H-pyrrolo[3,2-b]pyridin-1-yl)-2-oxoethyl)-2-methylpiperazine-1-carboxylate (the compound of Example 1, Step J, 0.11 g, 2.0 mmol), potassium carbonate (0.080 g, 0.56 mmol) and potassium iodide (catalytic amount) at room temperature. The reaction solution was stirred at 70°C for 24 hr, diluted with ethyl acetate, and washed with water. The organic layer was dried over anhydrous sodium sulfate, and the solvent was evaporated under reduced pressure. The obtained crude product was purified by HPLC preparative chromatography (column: X-SELECT CSH C18, eluent: 10 mM ammonium hydrogencarbonate aqueous solution/acetonitrile, gradient mode) to give the title compound (0.060 g).
¹H NMR (400 MHz, DMSO-d₆) δ 1.08 (d, J = 6.4 Hz, 3H), 1.24 (d, J = 3.6 Hz, 6H), 1.30-1.35 (m, 18H), 2.16 (s, 3H), 2.32 (t, J = 1.6 Hz, 3H), 2.41 (t, J = 8.8 Hz, 3H), 2.62-2.67 (m, 1H), 2.74 (brs, 1H), 3.02 (d, J = 9.6 Hz, 2H), 3.12-3.17 (m, 2H), 3.44-3.53 (m, 7H), 3.81-3.85 (m, 2H), 3.89-4.00 (m, 2H), 4.08-4.09 (m, 2H), 7.06-7.11 (m, 5H), 7.23-7.26 (m, 2H), 7.33 (t, J = 6.8 Hz, 1H), 7.67 (d, J = 4.4 Hz, 2H), 7.71 (d, J = 8.4 Hz, 2H), 7.81 (t, J = 8.4 Hz, 2H), 8.07 (d, J = 4.8 Hz, 2H), 8.58 (d, J = 6.0 Hz, 2H), 9.59 (s, 1H). MS (m/z): 1045.75 [M+H]⁺.

### (Step F) (R)-2-((2-(2-((((2R,5R)-1-(2-(6-(4-Fluorobenzyl)-3,3-dimethyl-2,3-dihydro-1H-pyrrolo[3,2-b]pyridin-1-yl)-2-oxoethyl)-5-methylpiperazin-2-yl)methyl)(methyl)amino)ethoxy)ethyl)amino)-3-(3-fluorophenyl)-N-(4-(pyridin-4-yl)phenyl)propanamide

To a DCM solution (1.0 mL) of tert-butyl (2R,5S)-4-(2-(6-(4-fluorobenzyl)-3,3-dimethyl-2,3-dihydro-1H-pyrrolo[3,2-b]pyridin-1-yl)-2-oxoethyl)-5-(8-((R)-3-(3-fluorophenyl)-1-oxo-1-((4-(pyridin-4-yl)phenyl)amino)propan-2-yl)-2,11,11-trimethyl-9-oxo-5,10-dioxa-2,8-diazadodecyl)-2-methylpiperazine-1-carboxylate (0.050 g, 0.048 mmol) was added TFA (1.0 mL) under ice-cooled. The reaction solution was stirred at room temperature for 2 hr, and the volatiles were evaporated under reduced pressure. The residue was dissolved in methanol, and the solution was filtered through SCX column. The column was washed with methanol (2 mL), followed by 7M ammonia/methanol solution (5 mL) to eluate the target product. This eluate was concentrated under reduced pressure to give the title compound (0.019 g).
¹H NMR (500 MHz, DMSO-d₆) δ 0.92 (d, J = 5.0 Hz, 3H), 1.24 (d, J = 3.0 Hz, 8H), 2.01-2.05 (m, 2H), 2.07 (s, 3H), 2.30-2.37 (m, 2H), 2.40-2.49 (m, 2H), 2.51-2.57 (m, 2H), 2.63-2.64 (m, 2H), 2.84-2.85 (2H), 2.86-2.94 (m, 2H), 3.22-3.28 (m, 4H), 3.46 (brs, 2H), 3.77-3.79 (m, 2H), 3.88 (s, 3H), 7.07-7.11 (m, 5H), 7.23-7.30 (m, 3H), 7.67 (dd, J = 4.5, 1.5 Hz, 2H), 7.72 (d, J = 8.5 Hz, 2H), 7.78 (d, J = 9.0 Hz, 2H), 8.06 (d, J = 7.0 Hz, 2H), 8.59 (d, J = 5.0, 2.0 Hz, 2H), 10.04 (s, 1H). MS (m/z) :845.68 [M+H]⁺; retention time/analysis method: 3.55 min/D.

### Example 18

### (S)-1-((2R,5R)-1-(2-(6-(4-Fluorobenzyl)-3,3-dimethyl-2,3-dihydro-1H-pyrrolo[3,2-b]pyridin-1-yl)-2-oxoethyl)-5-methylpiperazin-2-yl)-N-(isoquinolin-6-yl)-2-methyl-19-phenyl-5,8,11,14-tetraoxa-2,17-diazaicosan-20-amide

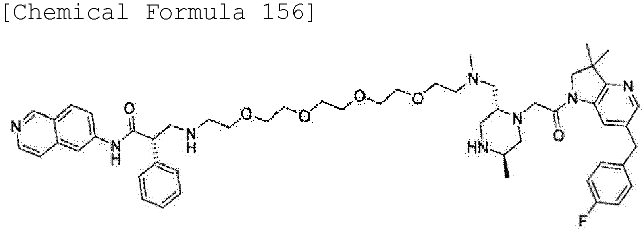

### (Step A) 14-Hydroxy-3,6,9,12-tetraoxatetradecyl 4-methylbenzenesulfonate

To a THF solution (0.20 L) of 3,6,9,12-tetraoxatetradecane-1,14-diol (6.5 g, 27 mmol) was added an aqueous solution (78 mL) of sodium hydroxide (1.6 g, 41 mmol) under ice-cooled, and then a THF solution (50 mL) of p-toluenesulfonyl chloride (5.2 g, 27 mmol) was added dropwise. The mixture was stirred at 0°C for 2 hr, and the reaction solution was diluted with ethyl acetate, and washed with water. The organic layer was dried over anhydrous sodium sulfate, and the solvent was evaporated under reduced pressure. The obtained crude product was purified by column chromatography (silica gel, DCM/methanol) to give the title compound (3.7 g).
MS (m/z): 393.22 [M+H]⁺.

### (Step B) N-(14-Hydroxy-3,6,9,12-tetraoxatetradecyl)-N-methyl-2-nitrobenzenesulfonamide

To an acetonitrile solution (20 mL) of N-methyl-2-nitrobenzenesulfonamide (4.0 g, 19 mmol) were added successively 14-hydroxy-3,6,9,12-tetraoxatetradecyl 4-methylbenzenesulfonate (7.4 g, 19 mmol), potassium carbonate (7.8 g, 57 mmol) and potassium iodide (catalytic amount). The mixture was stirred at 70°C for 16 hr, and the reaction solution was diluted with ethyl acetate, and washed with water. The organic layer was dried over anhydrous sodium sulfate, and the solvent was evaporated under reduced pressure. The obtained crude product was purified by column chromatography (silica gel, DCM/methanol) to give the title compound (5.2 g). ¹H NMR (400 MHz, DMSO-d₆) δ 2.90 (s, 3H), 3.36-3.42 (m, 4H), 3.46-3.50 (m, 14H), 3.56 (t, J = 5.6 Hz, 2H), 4.58 (t, J = 5.6 Hz, 1H), 7.82-7.91 (m, 2H), 7.97-8.02 (m, 2H). MS (m/z): 437.15 [M+H]⁺.

### (Step C) 2-((2-Nitrophenyl)sulfonyl)-5,8,11,14-tetraoxa-2-azahexadecan-16-yl methanesulfonate

To N-(14-hydroxy-3,6,9,12-tetraoxatetradecyl)-N-methyl-2-nitrobenzenesulfonamide (0.15 g, 0.34 mmol) in DCM (3.0 mL) were added TEA (0.27 mL, 2.1 mmol) and methanesulfonyl chloride (0.080 mL, 1.0 mmol) under ice-cooled. The mixture was stirred at room temperature for 3 hr, and the reaction solution was diluted with DCM, and washed with aqueous sodium hydrogencarbonate solution. The organic layer was dried over anhydrous sodium sulfate, and the solvent was evaporated under reduced pressure to give the title compound (0.24 g).
MS (m/z): 515.55 [M+H]⁺.

### (Step D) (S)-N-(Isoquinolin-6-yl)-2-((2-nitrophenyl)sulfonyl)-19-phenyl-5,8,11,14-tetraoxa-2,17-diazaicosan-20-amide

To an acetonitrile solution (5.0 mL) of (S)-3-amino-N-(isoquinolin-6-yl)-2-phenylpropanamide dihydrochloride (the compound of Reference Example 7, 0.4 g, 1.1 mmol) were added 2-((2-nitrophenyl)sulfonyl)-5,8,11,14-tetraoxa-2-azahexadecane-16-yl methanesulfonate (0.68 g, 1.3 mmol), potassium carbonate (0.76 g, 5.5 mmol) and potassium iodide (catalytic amount) at room temperature. The mixture was stirred at 70°C for 24 hr, and the reaction solution was diluted with water, and subjected to extraction with ethyl acetate. The organic layer was dried over anhydrous sodium sulfate, and the solvent was evaporated under reduced pressure. The obtained crude product was purified by HPLC preparative chromatography (column: Phenyl-Hexyl, eluent: ammonium hydrogencarbonate aqueous solution/acetonitrile, gradient mode) to give the title compound (0.15 g).
MS (m/z): 710.49 [M+H]⁺.

### (Step E) tert-Butyl (S)-(3-(isoquinolin-6-ylamino)-3-oxo-2-phenylpropyl) (2-((2-nitrophenyl) sulfonyl)-5,8,11,14-tetraoxa-2-azahexadecan-16-yl)carbamate

To a DCM solution (2.0 mL) of (S)-N-(isoquinolin-6-yl)-2-((2-nitrophenyl)sulfonyl)-19-phenyl-5,8,11,14-tetraoxa-2,17-diazaicosan-20-amide (0.15 g, 0.21 mmol) were added TEA (0.070 mL, 0.53 mmol) and di-tert-butyl dicarbonate (0.060 mL, 0.25 mmol) under ice-cooled. The mixture was stirred at room temperature for 2 hr, and the reaction solution was diluted with water, and subjected to extraction with DCM. The organic layer was dried over anhydrous sodium sulfate, and the solvent was evaporated under reduced pressure to give the title compound (0.30 g).
MS (m/z): 810.62 [M+H]⁺.

### (Step F) tert-Butyl (S)-(5,8,11,14-tetraoxa-2-azahexadecan-16-yl) (3-(isoquinolin-6-ylamino)-3-oxo-2-phenylpropyl)carbamate

To a DMF solution (5.0 mL) of tert-butyl (S)-(3-(isoquinolin-6-ylamino)-3-oxo-2-phenylpropyl) (2-((2-nitrophenyl)sulfonyl)-5,8,11,14-tetraoxa-2-azahexadecane-16-yl)carbamate (0.30 g, 0.37 mmol) were added thiophenol (0.050 mL, 0.56 mmol) and potassium carbonate (0.076 g, 0.56 mmol) under ice-cooled. The mixture was stirred at room temperature for 2 hr, and the reaction solution was diluted with ethyl acetate, and washed with water. The organic layer was washed with 1M hydrochloric acid, and the aqueous layer was alkalified with aqueous sodium hydroxide solution, and subjected to extraction with ethyl acetate. The obtained organic layer was dried over anhydrous sodium sulfate, and the solvent was evaporated under reduced pressure to give the title compound (0.10 g).
MS (m/z): 625.61 [M+H]⁺.

### (Step G) tert-Butyl (2R,5S)-4-(2-(6-(4-fluorobenzyl)-3,3-dimethyl-2,3-dihydro-1H-pyrrolo[3,2-b]pyridin-1-yl)-2-oxoethyl)-5-(17-((S)-3-(isoquinolin-6-ylamino)-3-oxo-2-phenylpropyl)-2,20,20-trimethyl-18-oxo-5,8,11,14,19-pentaoxa-2,17-diazahenicosyl)-2-methylpiperazine-1-carboxylate

To an acetonitrile solution (2.0 mL) of tert-butyl (S)-(5,8,11,14-tetraoxa-2-azahexadecane-16-yl)(3-(isoquinolin-6-ylamino)-3-oxo-2-phenylpropyl)carbamate (0.10 g, 0.16 mmol) were added tert-butyl (2R,5R)-5-(chloromethyl)-4-(2-(6-(4-fluorobenzyl)-3,3-dimethyl-2,3-dihydro-1H-pyrrolo[3,2-b]pyridin-1-yl)-2-oxoethyl)-2-methylpiperazine-1-carboxylate (the compound of Example 1, Step J, 0.090 g, 0.17 mmol), potassium carbonate (0.066 g, 0.48 mmol) and potassium iodide (catalytic amount) at room temperature. The mixture was stirred at 70°C for 16 hr, and the reaction solution was diluted with water, and subjected to extraction with ethyl acetate. The organic layer was dried over anhydrous sodium sulfate, and the solvent was evaporated under reduced pressure. The obtained crude product was purified by HPLC preparative chromatography (column: Phenyl-Hexyl, eluent: formic acid aqueous solution/acetonitrile, gradient mode) to give the title compound (0.020 g).
¹H NMR (400 MHz, CDCl₃) δ 1.19 (d, J = 6.8 Hz, 3H), 1.27 (d, J = 16.0 Hz, 10H), 1.38 (d, J = 4.4 Hz, 9H), 1.44 (d, J = 7.6 Hz, 9H), 2.18 (brs, 2H), 2.42 (brs, 4H), 2.75 (d, J = 9.6 Hz, 2H), 3.26 (brs, 1H), 3.29-3.31 (m, 6H), 3.38 (brs, 2H), 3.49 (s, 3H), 3.54 (brs, 2H), 3.61-3.65 (m, 2H), 3.77-3.79 (m, 2H), 3.89-3.92 (m, 6H), 3.99 (brs, 3H), 4.24-4.27 (m, 3H), 6.93-6.99 (m, 2H), 7.12-7.16 (m, 2H), 7.31-7.36 (m, 2H), 7.46 (d, J = 6.8 Hz, 2H), 7.56-7.58 (m, 2H), 7.85 (d, J = 8.8 Hz, 1H), 8.07 (s, 1H), 8.21 (s, 1H), 8.43 (d, J = 6.0 Hz, 2H), 9.10 (s, 1H). MS (m/z): 1133.77 [M+H]⁺.

### (Step H) (S)-1-((2R,5R)-1-(2-(6-(4-Fluorobenzyl)-3,3-dimethyl-2,3-dihydro-1H-pyrrolo[3,2-b]pyridin-1-yl)-2-oxoethyl)-5-methylpiperazin-2-yl)-N-(isoquinolin-6-yl)-2-methyl-19-phenyl-5,8,11,14-tetraoxa-2,17-diazaicosan-20-amide

To a DCM solution (1.0 mL) of tert-butyl (2R,5S)-4-(2-(6-(4-fluorobenzyl)-3,3-dimethyl-2,3-dihydro-1H-pyrrolo[3,2-b]pyridin-1-yl)-2-oxoethyl)-5-(17-((S)-3-(isoquinolin-6-ylamino)-3-oxo-2-phenylpropyl)-2,20,20-trimethyl-18-oxo-5,8,11,14,19-pentaoxa-2,17-diazahenicosyl)-2-methylpiperazine-1-carboxylate (0.020 g, 0.018 mmol) was added TFA (1.0 mL) under ice-cooled, and the mixture was stirred at room temperature for 2 hr. The reaction solution was concentrated, and the residue was diluted with water, and subjected to extraction with methanol/DCM (10/90). The organic layer was dried over anhydrous sodium sulfate, and the solvent was evaporated under reduced pressure to give the title compound (0.012 g).
¹H NMR (400 MHz, DMSO-d₆) δ 0.85 (d, J = 6.0 Hz, 3H), 1.23-1.26 (m, 8H), 1.97 (dd, J = 12.4, 3.2 Hz, 1H), 2.08 (s, 3H), 2.24-2.29 (m, 4H), 2.33 (t, J = 1.6 Hz, 1H), 2.66-2.67 (m, 3H), 2.69-2.84 (m, 3H), 3.27-3.28 (m, 5H), 3.34-3.51 (m, 14H), 3.79-3.99 (m, 6H), 7.10 (t, J = 8.8 Hz, 2H), 7.23-7.28 (m, 3H), 7.33 (t, J = 7.2 Hz, 2H), 7.40 (d, J = 7.2 Hz, 2H), 7.66 (d, J = 1.6 Hz, 1H), 7.69 (d, J = 5.6 Hz, 1H), 8.03 (d, J = 8.8 Hz, 2H), 8.07 (s, 1H), 8.39 (d, J = 6.0 Hz, 2H), 9.14 (s, 1H), 10.64 (s, 1H). MS (m/z): 933.94 [M+H]⁺; retention time/analysis method: 2.78 min/B.

The example compounds produced according to the above-mentioned production method or the method shown in Example 1, 8 or 18, or a method similar thereto, are described below. For each compound, the compound name, structural formula, MS (m/z) (actual measured value), retention time (unit: minutes), and the analytical method are shown.

### Example 2

### 4-((R)-1-((2R,5R)-1-(2-(6-(4-Fluorobenzyl)-3,3-dimethyl-2,3-dihydro-1H-pyrrolo[3,2-b]pyridin-1-yl)-2-oxoethyl)-5-methylpiperazin-2-yl)-2-methyl-5,8-dioxa-2,11-diazatridecan-12-yl)-N-(pyridin-4-yl)benzamide

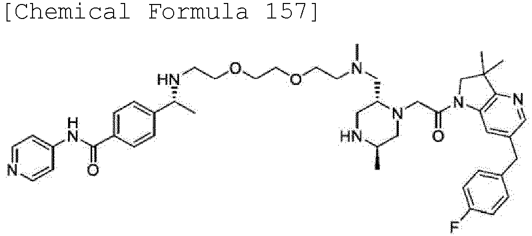

MS (m/z):795.72 [M+H]⁺; retention time/analysis method: 9.72 min/C

### Example 3

### 4-((R)-1-((2R,5R)-1-(2-(6-(4-Fluorobenzyl)-3,3-dimethyl-2,3-dihydro-1H-pyrrolo[3,2-b]pyridin-1-yl)-2-oxoethyl)-5-methylpiperazin-2-yl)-2-methyl-5,8,11-trioxa-2,14-diazahexadecan-15-yl)-N-(pyridin-4-yl)benzamide

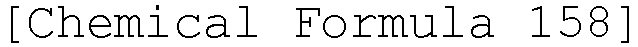

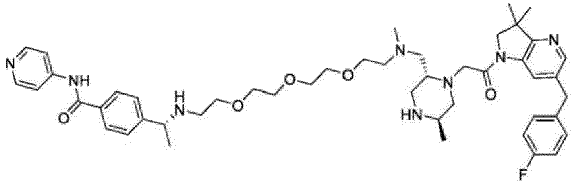

MS (m/z):839.68 [M+H]⁺; retention time/analysis method: 9.68 min/C

### Example 4

### 4-((R)-1-((2R,5R)-1-(2-(6-(4-Fluorobenzyl)-3,3-dimethyl-2,3-dihydro-1H-pyrrolo[3,2-b]pyridin-1-yl)-2-oxoethyl)-5-methylpiperazin-2-yl)-2-methyl-5,8,11,14-tetraoxa-2,17-diazanonadecan-18-yl)-N-(pyridin-4-yl)benzamide

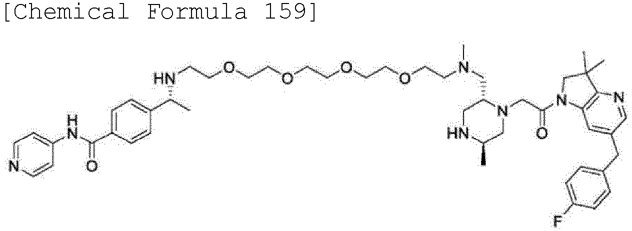

MS (m/z):883.90 [M+H]⁺; retention time/analysis method: 3.30 min/D

### Example 5

### 4-((R)-1-((2R,5R)-1-(2-(6-(4-Fluorobenzyl)-3,3-dimethyl-2,3-dihydro-1H-pyrrolo[3,2-b]pyridin-1-yl)-2-oxoethyl)-5-methylpiperazin-2-yl)-2-methyl-5,8,11,14,17-pentaoxa-2,20-diazadocosan-21-yl)-N-(pyridin-4-yl)benzamide

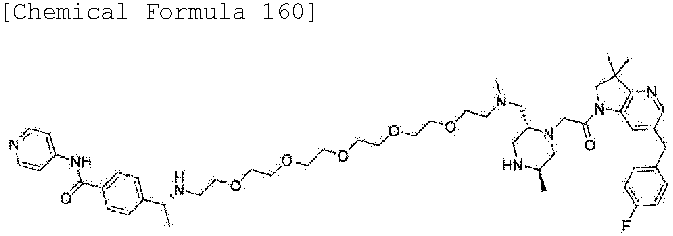

MS (m/z) :927.81 [M+H]⁺; retention time/analysis method: 9.66 min/C

### Example 6

### 4-((R)-1-((2R,5R)-1-(2-(6-(4-Fluorobenzyl)-3,3-dimethyl-2,3-dihydro-1H-pyrrolo[3,2-b]pyridin-1-yl)-2-oxoethyl)-5-methylpiperazin-2-yl)-2-methyl-5,8,11,14,17,20-hexaoxa-2,23-diazapentacosan-24-yl)-N-(pyridin-4-yl)benzamide

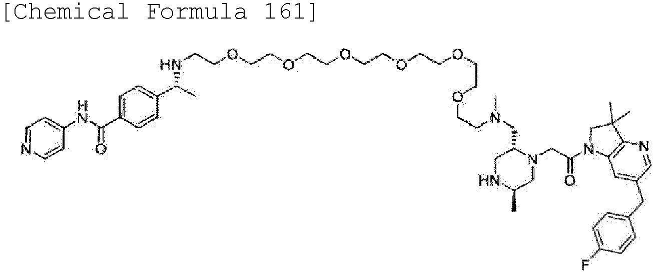

MS (m/z):971.77 [M+H]⁺; retention time/analysis method: 9.41 min/C

### Example 7

### 4-((R)-1-((2R,5R)-1-(2-(6-(4-Fluorobenzyl)-3,3-dimethyl-2,3-dihydro-1H-pyrrolo[3,2-b]pyridin-1-yl)-2-oxoethyl)-5-methylpiperazin-2-yl)-2-methyl-5,8,11,14,17,20,23-heptaoxa-2,26-diazaoctacosan-27-yl)-N-(pyridin-4-yl)benzamide

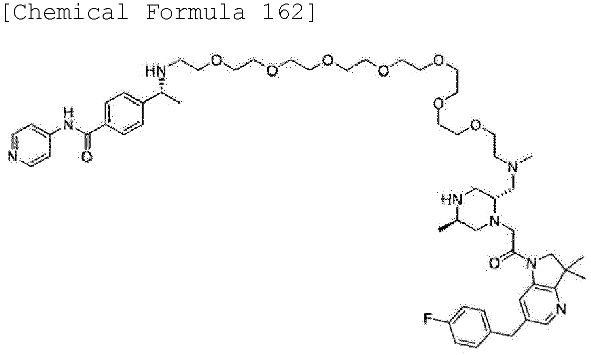

MS (m/z):1013.73 [M-H]⁻; retention time/analysis method: 11.30 min/E

### Example 9

### (R)-12-(3-Fluorobenzyl)-1-((2R,5R)-1-(2-(6-(4-fluorobenzyl)-3, 3-dimethyl-2,3-dihydro-1H-pyrrolo[3,2-b]pyridin-1-yl)-2-oxoethyl)-5-methylpiperazin-2-yl)-2-methyl-N-(4-(pyridin-4-yl)phenyl)-5,8-dioxa-2,11-diazatridecan-13-amide

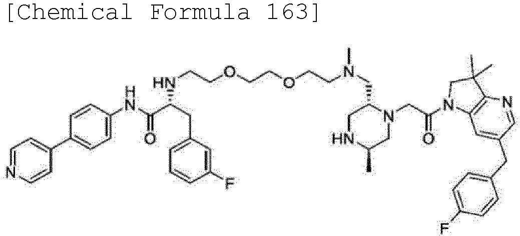

MS (m/z):887.56 [M-H]⁻; retention time/analysis method: 13.50 min/E

### Example 10

### (R)-15-(3-Fluorobenzyl)-1-((2R,5R)-1-(2-(6-(4-fluorobenzyl)-3,3-dimethyl-2,3-dihydro-1H-pyrrolo[3,2-b]pyridin-1-yl)-2-oxoethyl)-5-methylpiperazin-2-yl)-2-methyl-N-(4-(pyridin-4-yl)phenyl)-5,8,11-trioxa-2,14-diazahexadecan-16-amide

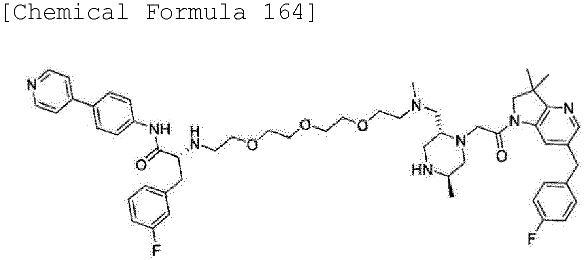

MS (m/z):933.70 [M+H]⁺; retention time/analysis method: 3.32 min/D

### Example 11

### (R)-18-(3-Fluorobenzyl)-1-((2R,5R)-1-(2-(6-(4-fluorobenzyl)-3,3-dimethyl-2,3-dihydro-1H-pyrrolo[3,2-b]pyridin-1-yl)-2-oxoethyl)-5-methylpiperazin-2-yl)-2-methyl-N-(4-(pyridin-4-yl)phenyl)-5,8,11,14-tetraoxa-2,17-diazanonadecan-19-amide

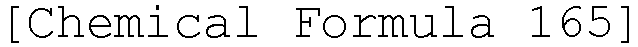

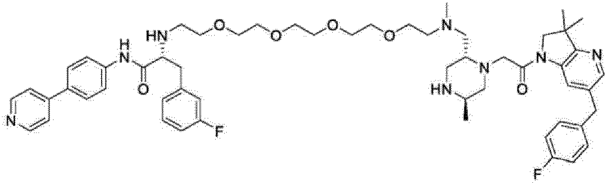

MS (m/z):975.76 [M-H]⁻; retention time/analysis method: 11.18 min/C

### Example 12

### (R)-21-(3-Fluorobenzyl)-1-((2R,5R)-1-(2-(6-(4-fluorobenzyl)-3,3-dimethyl-2,3-dihydro-1H-pyrrolo[3,2-b]pyridin-1-yl)-2-oxoethyl)-5-methylpiperazin-2-yl)-2-methyl-N-(4-(pyridin-4-yl)phenyl)-5,8,11,14,17-pentaoxa-2,20-diazadocosan-22-amide

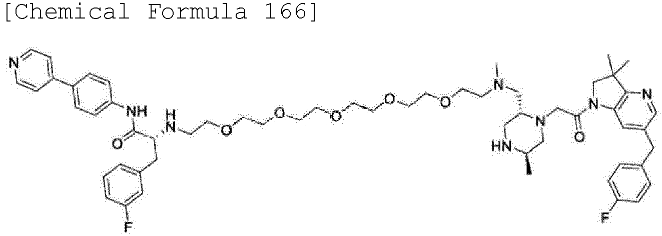

MS (m/z):1021.29 [M+H]⁺; retention time/analysis method: 10.87 min/C

### Example 13

### (R)-24-(3-Fluorobenzyl)-1-((2R,5R)-1-(2-(6-(4-fluorobenzyl)-3,3-dimethyl-2,3-dihydro-1H-pyrrolo[3,2-b]pyridin-1-yl)-2-oxoethyl)-5-methylpiperazin-2-yl)-2-methyl-N-(4-(pyridin-4-yl)phenyl)-5,8,11,14,17,20-hexaoxa-2,23-diazapentacosan-25-amide

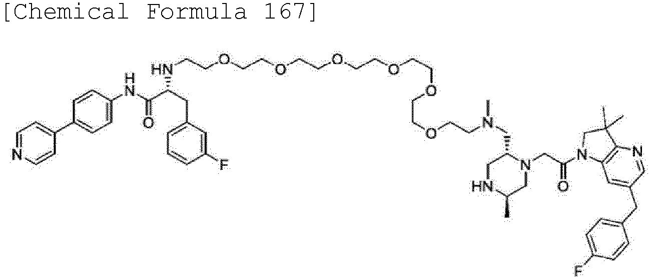

MS (m/z):1065.81 [M+H]⁺; retention time/analysis method: 10.86 min/C

### Example 14

### (R)-27-(3-Fluorobenzyl)-1-((2R,5R)-1-(2-(6-(4-fluorobenzyl)-3,3-dimethyl-2,3-dihydro-1H-pyrrolo[3,2-b]pyridin-1-yl)-2-oxoethyl)-5-methylpiperazin-2-yl)-2-methyl-N-(4-(pyridin-4-yl)phenyl)-5,8,11,14,17,20,23-heptaoxa-2,26-diazaoctacosan-28-amide

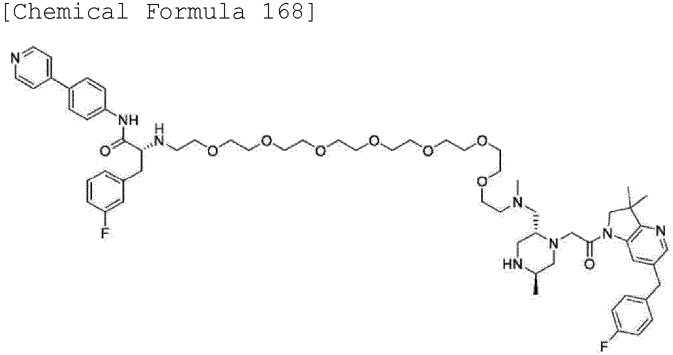

MS (m/z):1109.86 [M+H]⁺; retention time/analysis method: 10.63 min/C

### Example 15

### (S)-3-((2-(2-((((2R,5R)-1-(2-(6-(4-Fluorobenzyl)-3,3-dimethyl-2,3-dihydro-1H-pyrrolo[3,2-b]pyridin-1-yl)-2-oxoethyl)-5-methylpiperazin-2-yl)methyl)(methyl)amino)ethoxy)ethyl)amino)-N-(isoquinolin-6-yl)-2-phenylpropanamide

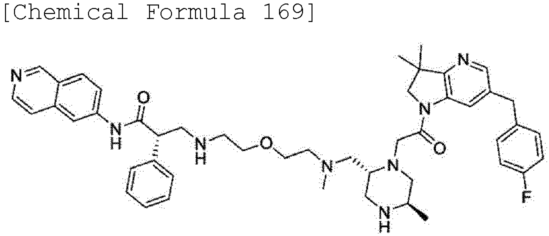

MS (m/z) :801.72 [M+H]⁺; retention time/analysis method: 3.79 min/B

### Example 16

### (S)-1-((2R,5R)-1-(2-(6-(4-Fluorobenzyl)-3,3-dimethyl-2,3-dihydro-1H-pyrrolo[3,2-b]pyridin-1-yl)-2-oxoethyl)-5-methylpiperazin-2-yl)-N-(isoquinolin-6-yl)-2-methyl-13-phenyl-5,8-dioxa-2,11-diazatetradecan-14-amide

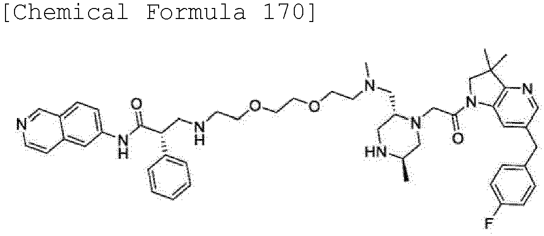

MS (m/z):845.70 [M+H]⁺; retention time/analysis method: 2.92 min/B

### Example 17

### (S)-1-((2R,5R)-1-(2-(6-(4-Fluorobenzyl)-3,3-dimethyl-2,3-dihydro-1H-pyrrolo[3,2-b]pyridin-1-yl)-2-oxoethyl)-5-methylpiperazin-2-yl)-N-(isoquinolin-6-yl)-2-methyl-16-phenyl-5,8,11-trioxa-2,14-diazaheptadecan-17-amide

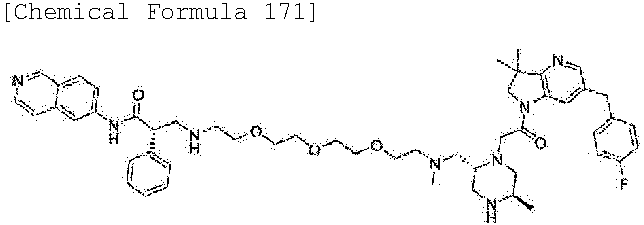

MS (m/z):889.67 [M+H]⁺; retention time/analysis method: 2.74 min/B

### Example 19

### (S)-1-((2R,5R)-1-(2-(6-(4-Fluorobenzyl)-3,3-dimethyl-2,3-dihydro-1H-pyrrolo[3,2-b]pyridin-1-yl)-2-oxoethyl)-5-methylpiperazin-2-yl)-N-(isoquinolin-6-yl)-2-methyl-22-phenyl-5,8,11,14,17-pentaoxa-2,20-diazatricosan-23-amide

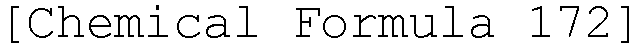

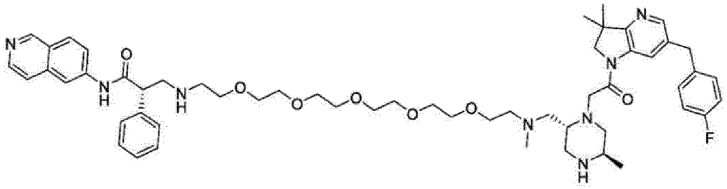

MS (m/z):977.84 [M+H]⁺; retention time/analysis method: 2.82 min/B

### Example 20

### (S)-1-((2R,5R)-1-(2-(6-(4-Fluorobenzyl)-3,3-dimethyl-2,3-dihydro-1H-pyrrolo[3,2-b]pyridin-1-yl)-2-oxoethyl)-5-methylpiperazin-2-yl)-N-(isoquinolin-6-yl)-2-methyl-25-phenyl-5,8,11,14,17,20-hexaoxa-2,23-diazahexacosan-26-amide

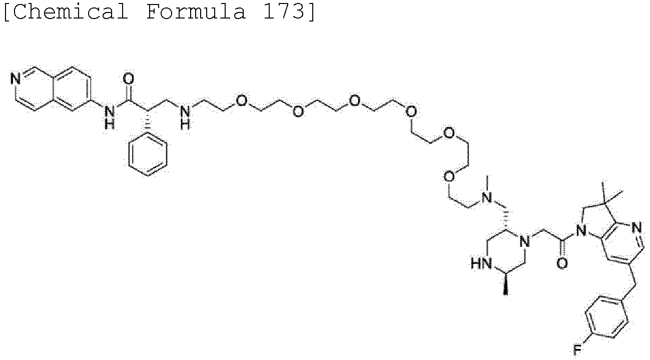

MS (m/z):1021.85 [M+H]⁺; retention time/analysis method: 3.05 min/B

### Example 21

### (S)-1-((2R,5R)-1-(2-(6-(4-Fluorobenzyl)-3,3-dimethyl-2,3-dihydro-1H-pyrrolo[3,2-b]pyridin-1-yl)-2-oxoethyl)-5-methylpiperazin-2-yl)-N-(isoquinolin-6-yl)-2-methyl-28-phenyl-5,8,11,14,17,20,23-heptaoxa-2,26-diazanonacosan-29-amide

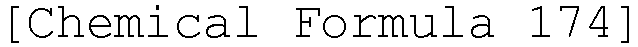

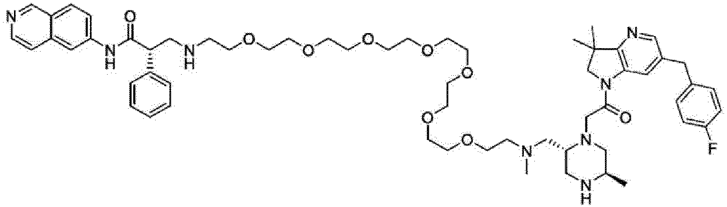

MS (m/z):1065.70 [M+H]⁺; retention time/analysis method: 3.63 min/D

### Example 22

### 4-((R)-1-((2-(2-(((6-(4-Fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)methyl)(methyl)amino)ethoxy)ethyl)amino)ethyl)-N-(pyridin-4-yl)benzamide

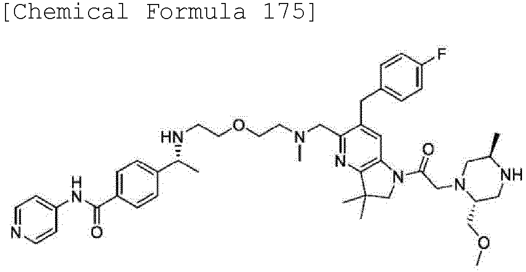

### (Step A) 5-(((tert-Butyldimethylsilyl)oxy)methyl)-6-(4-fluorobenzyl)-3,3-dimethyl-2,3-dihydro-1H-pyrrolo[3,2-b]pyridine

To a DCM solution (0.80 L) of (6-(4-fluorobenzyl)-3,3-dimethyl-2,3-dihydro-1H-pyrrolo[3,2-b]pyridin-5-yl)methanol (the compound of Reference Example 4, 25 g, 87 mmol) were added imidazole (18 g, 0.26 mol) and tert-butyldimethylsilyl chloride (26 g, 0.17 mol) at 0°C, and the reaction solution was stirred at room temperature for 5 hr. The reaction solution was diluted with DCM and water, and the organic layer was dried over anhydrous sodium sulfate. The solvent was evaporated under reduced pressure, and the obtained crude product was purified by column chromatography (silica gel, petroleum ether/ethyl acetate) to give the title compound (34 g).
¹H NMR (400 MHz, DMSO-d₆) δ 0.03 (s, 6H), 0.87 (s, 9H), 1.24 (s, 6H), 3.25-3.26 (m, 2H), 3.99 (s, 2H), 4.72 (s, 2H), 5.70 (brs, 1H), 6.42 (s, 1H), 7.08-7.13 (m, 2H), 7.19-7.27 (m, 2H); MS (m/z): 401.39 [M+H]⁺.

### (Step B) 1-(5-(((tert-Butyldimethylsilyl)oxy)methyl)-6-(4-fluorobenzyl)-3,3-dimethyl-2,3-dihydro-1H-pyrrolo[3,2-b]pyridin-1-yl)-2-chloroethan-1-one

To a THF solution (40 mL) of 5-(((tert-butyldimethylsilyl)oxy)methyl)-6-(4-fluorobenzyl)-3,3-dimethyl-2,3-dihydro-1H-pyrrolo[3,2-b]pyridine (1.5 g, 3.8 mmol) were added TEA (0.75 g, 7.4 mmol) and chloroacetyl chloride (0.5 g, 4.5 mmol), and the mixture was stirred at room temperature for 4 hr. The reaction solution was diluted with ethyl acetate, and washed with water, and the organic layer was concentrated under reduced pressure to give the title compound (1.8 g).
MS (m/z): 477.47 [M+H]⁺.

### (Step C) tert-Butyl (2R,5R)-4-(2-(5-(((tert-butyldimethylsilyl)oxy)methyl)-6-(4-fluorobenzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate

To an acetonitrile solution (17 mL) of 1-(5-(((tert-butyldimethylsilyl)oxy)methyl)-6-(4-fluorobenzyl)-3,3-dimethyl-2,3-dihydro-1H-pyrrolo[3,2-b]pyridin-1-yl)-2-chloroethan-1-one (1.7 g, 3.7 mmol) were added tert-butyl (2R,5R)-5-(methoxymethyl)-2-methylpiperazine-1-carboxylate (the compound of Reference Example 2, 0.94 g, 4.1 mmol), potassium carbonate (2.1 g, 15 mmol) and potassium iodide (0.062 g, 0.373 mmol) at room temperature. The mixture was stirred at 70°C for 18 hr, and the reaction solution was diluted with ethyl acetate, and washed with water. The organic layer was concentrated under reduced pressure to give the title compound (2.1 g).
MS (m/z): 685.64 [M+H]⁺.

### (Step D) tert-Butyl (2R,5R)-4-(2-(6-(4-fluorobenzyl)-5-(hydroxymethyl)-3,3-dimethyl-2,3-dihydro-1H-pyrrolo[3,2-b]pyridin-1-yl)-2-oxoethyl)-5-(methoxymethyl)-2-methylpiperazine-1-carboxylate

To a THF solution (21 mL) of tert-butyl (2R,5R)-4-(2-(5-(((tert-butyldimethylsilyl)oxy)methyl)-6-(4-fluorobenzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate (2.1 g, 3.1 mmol) was added TBAF (1M THF solution, 6.1 mL, 6.1 mmol), and the mixture was stirred at room temperature for 18 hr. The reaction solution was diluted with ethyl acetate, and washed with water. The organic layer was concentrated under reduced pressure, and the residue was purified by column chromatography (silica gel, petroleum ether/ethyl acetate) to give the title compound (1.3 g).
¹H NMR (400 MHz, DMSO-d₆) δ 1.09 (d, J = 6.40 Hz, 3H), 1.29 (s, 6H), 1.39 (s, 9H), 2.53-2.58 (m, 1H), 2.68 (d, J = 6.8 Hz, 1H), 3.13-3.24 (m, 3H), 3.26 (s, 3H), 3.44-3.47 (m, 2H), 3.59-3.63 (m, 1H), 3.80-3.83 (m, 1H), 3.93-4.05 (m, 5H), 4.55 (d, J = 5.2 Hz, 2H), 5.12 (t, J = 5.62 Hz, 1H), 7.11 (t, J = 8.80 Hz, 2H), 7.20-7.24 (m, 2H), 8.03 (s, 1H); MS (m/z): 572.02 [M+H]⁺.

### (Step E) tert-Butyl (2R,5R)-4-(2-(5-(chloromethyl)-6-(4-fluorobenzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate

To a DCM solution (20 mL) of tert-butyl (2R,5R)-4-(2-(6-(4-fluorobenzyl)-5-(hydroxymethyl)-3,3-dimethyl-2,3-dihydro-1H-pyrrolo[3,2-b]pyridin-1-yl)-2-oxoethyl)-5-(methoxymethyl)-2-methylpiperazine-1-carboxylate (1.3 g, 2.3 mmol) were added TEA (2.1 g, 21 mmol) and methanesulfonyl chloride (1.2 g, 10 mmol). The mixture was stirred at room temperature for 18 hr, and the reaction mixture was washed with water. The organic layer was concentrated under reduced pressure, and the obtained crude product was purified by column chromatography (silica gel, petroleum ether/ethyl acetate) to give the title compound (0.65 g).
¹H NMR (400 MHz, DMSO-d₆) δ 1.09 (d, J = 6.40 Hz, 3H), 1.29 (d, J = 2.0 Hz, 6H), 1.39 (s, 9H), 2.67 (brs, 1H), 2.91 (brs, 1H), 3.19 (t, J = 7.2 Hz, 2H), 3.24 (s, 3H), 3.44 (brs, 2H), 3.61-3.69 (m, 2H), 3.81 (d, J = 12.8 Hz, 1H), 3.95-4.02 (m, 3H), 4.09 (s, 2H), 4.82 (s, 2H), 7.14 (t, J = 9.20 Hz, 2H), 7.21-7.25 (m, 2H), 8.03 (s, 1H); MS (m/z): 589.57 [M+H]⁺.

### (Step F) tert-Butyl (2R,5R)-4-(2-(6-(4-fluorobenzyl)-3,3-dimethyl-5-(2,11,11-trimethyl-9-oxo-8-((R)-1-(4-(pyridin-4-ylcarbamoyl)phenyl)ethyl)-5,10-dioxa-2,8-diazadodecyl)-2,3-dihydro-1H-pyrrolo[3,2-b]pyridin-1-yl)-2-oxoethyl)-5-(methoxymethyl)-2-methylpiperazine-1-carboxylate

To an acetonitrile solution (2.0 mL) of tert-butyl (R)-(2-(2-(methylamino)ethoxy)ethyl) (1-(4-(pyridin-4-ylcarbamoyl) phenyl) ethyl) carbamate (the compound of Example 1, Step G, 0.12 g, 0.27 mmol) were added tert-butyl (2R,5R)-4-(2-(5-(chloromethyl)-6-(4-fluorobenzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate (0.17 g, 0.28 mmol), potassium carbonate (0.11 g, 0.81 mmol) and potassium iodide (catalytic amount) at room temperature. The mixture was stirred at 70°C for 16 hr, and the reaction solution was diluted with ethyl acetate, and washed with water. The organic layer was dried over anhydrous sodium sulfate, and the solvent was evaporated under reduced pressure. The obtained crude product was purified by HPLC preparative chromatography (column: X-SELECT CSH C18, eluent: 10 mM ammonium hydrogencarbonate aqueous solution/acetonitrile, gradient mode) to give the title compound (0.050 g).
MS (m/z): 996.00 [M+H]⁺.

### (Step G) 4-((R)-1-((2-(2-(((6-(4-fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)methyl)(methyl)amino)ethoxy)ethyl)amino)ethyl)-N-(pyridin-4-yl)benzamide

To a DCM solution (1.0 mL) of tert-butyl (2R,5R)-4-(2-(6-(4-fluorobenzyl)-3,3-dimethyl-5-(2,11,11-trimethyl-9-oxo-8-((R)-1-(4-(pyridin-4-ylcarbamoyl)phenyl)ethyl)-5,10-dioxa-2,8-diazadodecyl)-2,3-dihydro-1H-pyrrolo[3,2-b]pyridin-1-yl)-2-oxoethyl)-5-(methoxymethyl)-2-methylpiperazine-1-carboxylate (0.050 g, 0.050 mmol) was added TFA (1.0 mL) under ice-cooled. The mixture was stirred at room temperature for 3 hr, and the reaction solution was alkalified with aqueous sodium hydrogencarbonate solution, and subjected to extraction with DCM. The organic layer was dried over anhydrous sodium sulfate, and the solvent was evaporated under reduced pressure to give the title compound (0.040 g).
¹H NMR (400 MHz, DMSO-d₆) δ 0.86 (d, J = 6.4 Hz, 3H), 1.21 (d, J = 6.8 Hz, 3H), 1.23-1.27 (m, 6H), 2.09-2.12 (m, 2H), 2.14 (s, 4H), 2.32-2.39 (m, 3H), 2.46-2.49 (m, 1H), 2.54-2.56 (m, 2H), 2.60-2.64 (m, 1H), 2.66-2.67 (m, 1H), 2.79 (d, J = 8.8 Hz, 1H), 3.13 (s, 3H), 3.19-3.22 (m, 2H), 3.34-3.40 (m, 3H), 3.43 (t, J = 5.6 Hz, 2H), 3.59 (s, 2H), 3.63 (s, 1H), 3.74 (d, J = 6.4 Hz, 1H), 3.84 (d, J =10.4 Hz, 1H), 4.03 (d, J = 10.4 Hz, 1H), 4.09 (s, 2H), 7.09 (t, J = 9.2 Hz, 2H), 7.21 (dd, J = 8.8, 6.0 Hz, 2H), 7.45 (d, J = 8.0 Hz, 2H), 7.77 (dd, J = 4.8, 1.6 Hz, 2H), 7.87 (d, J = 8.4 Hz, 2H), 7.97 (s, 1H), 8.46 (dd, J = 4.8, 1.6 Hz, 2H), 10.49 (s, 1H); MS (m/z): 795.71 [M+H]; retention time/analysis method: 2.90 min/D.

### Example 29

### (R)-2-((2-(2-(((6-(4-Fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)methyl)(methyl)amino)ethoxy)ethyl)amino)-3-(3-fluorophenyl)-N-(4-(pyridin-4-yl)phenyl)propanamide

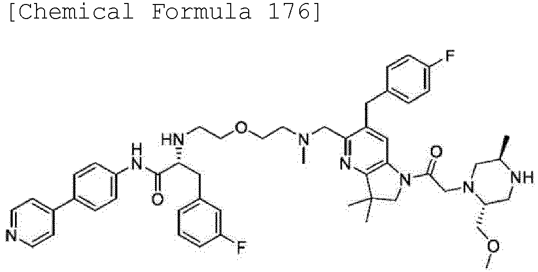

### (Step A) tert-Butyl (2R,5R)-4-(2-(6-(4-fluorobenzyl)-5-(8-((R)-3-(3-fluorophenyl)-1-oxo-1-((4-(pyridin-4-yl)phenyl) amino)propan-2-yl)-2,11,11-trimethyl-9-oxo-5,10-dioxa-2,8-diazadodecyl)-3,3-dimethyl-2,3-dihydro-1H-pyrrolo[3,2-b]pyridin-1-yl)-2-oxoethyl)-5-(methoxymethyl)-2-methylpiperazine-1-carboxylate

To an acetonitrile solution (1.0 mL) of tert-butyl (R)-(3-(3-fluorophenyl)-1-oxo-1-((4-(pyridin-4-yl)phenyl)amino)propan-2-yl)(2-(2-(methylamino)ethoxy)ethyl)carbamate (the compound of Example 8, Step D, 0.10 g, 0.19 mmol) were added tert-butyl (2R,5R)-4-(2-(5-(chloromethyl)-6-(4-fluorobenzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate (the compound of Example 22, Step E, 0.12 g, 0.20 mmol), potassium carbonate (0.078 g, 0.56 mmol) and potassium iodide (catalytic amount) at room temperature. The mixture was stirred at 70°C for 24 hr, and the reaction solution was diluted with ethyl acetate, and washed with water. The organic layer was dried over anhydrous sodium sulfate, and the solvent was evaporated under reduced pressure. The obtained crude product was purified by HPLC preparative chromatography (column: X-SELECT CSH C18, eluent: 10 mM ammonium hydrogencarbonate aqueous solution/acetonitrile, gradient mode) to give the title compound (0.060 g).
MS (m/z): 1089.74 [M+H]⁺.

### (Step B) (R)-2-((2-(2-(((6-(4-Fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)methyl) (methyl)amino) ethoxy)ethyl)amino)-3-(3-fluorophenyl)-N-(4-(pyridin-4-yl)phenyl)propanamide

To a DCM solution (1.0 mL) of tert-butyl (2R,5R)-4-(2-(6-(4-fluorobenzyl)-5-(8-((R)-3-(3-fluorophenyl)-1-oxo-1-((4-(pyridin-4-yl)phenyl)amino)propan-2-yl)-2,11,11-trimethyl-9-oxo-5,10-dioxa-2,8-diazadodecyl)-3,3-dimethyl-2,3-dihydro-1H-pyrrolo[3,2-b]pyridin-1-yl)-2-oxoethyl)-5-(methoxymethyl)-2-methylpiperazine-1-carboxylate (0.060 g, 0.055 mmol) was added TFA (0.5 mL) under ice-cooled, and the mixture was stirred at room temperature for 3 hr. The reaction solution was alkalified with aqueous sodium hydrogencarbonate solution, and subjected to extraction with DCM. The organic layer was dried over anhydrous sodium sulfate, and the solvent was evaporated under reduced pressure to give the title compound (0.045 g).
¹H NMR (400 MHz, DMSO-d₆) δ 0.86 (d, J = 6.4 Hz, 3H), 1.25 (d, J = 7.2 Hz, 7H), 2.15 (s, 5H), 2.32-2.40 (m, 1H), 2.61-2.67 (m, 7H), 2.79-2.85 (m, 2H), 2.92-2.96 (m, 1H), 3.13 (s, 3H), 3.19-3.22 (m, 2H), 3.34-3.43 (m, 5H), 3.47 (brs, 1H), 3.57 (s, 2H), 3.65 (d, J = 16 Hz, 1H), 3.83 (d, J = 10.8 Hz, 1H), 4.03 (d, J = 10.4 Hz, 1H), 4.07 (s, 1H), 6.96-7.01 (m, 1H), 7.05-7.09 (m, 4H), 7.17-7.20 (m, 2H), 7.24-7.29 (m, 1H), 7.66 (dd, J = 4.4, 1.6 Hz, 2H), 7.73 (q, J = 8.8 Hz, 4H), 7.96 (s, 1H), 8.59 (dd, J = 4.8, 1.6 Hz, 2H), 10.03 (s, 1H), one proton missing; MS (m/z): 889.76 [M+H]⁺; retention time/analysis method: 9.86 min/G.

### Example 38

### (S)-1-(6-(4-Fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)-N-(isoquinolin-6-yl)-2-methyl-16-phenyl-5,8,11-trioxa-2,14-diazaheptadecan-17-amide

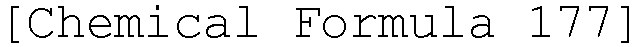

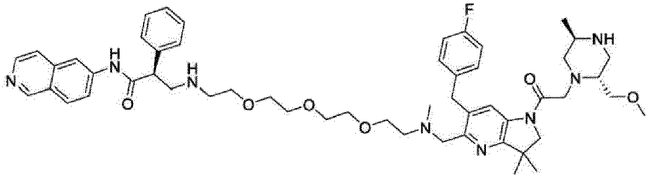

### (Step A) 2-(2-(2-(2-Hydroxyethoxy)ethoxy)ethoxy)ethyl 4-methylbenzenesulfonate

To a THF solution (0.13 L) of 2,2'-((oxybis(ethane-2,1-diyl))bis(oxy))bis(ethan-1-ol) (25 g, 0.13 mol) was added an aqueous solution (30 mL) of sodium hydroxide (7.8 g, 0.20 mol) under ice-cooled, and the mixture was stirred for 5 min. Then, a THF solution (0.3 L) of p-toluenesulfonyl chloride (25 g, 0.13 mol) was added dropwise thereto, and the reaction mixture was stirred at room temperature for 4 hr. The reaction solution was diluted with ethyl acetate, and washed with water. The organic layer was dried over anhydrous sodium sulfate, and the solvent was evaporated under reduced pressure. The obtained crude product was purified by column chromatography (silica gel, methanol/DCM) to give the title compound (24 g). ¹H NMR (400 MHz, CDCl₃) δ 2.45 (s, 3H), 3.59-3.73 (m, 15H), 4.17 (t, J = 4.8 Hz, 2H), 7.34 (d, J = 8.4 Hz, 2H), 7.80 (d, J = 8.4 Hz, 2H); MS (m/z): 349.39 [M+H]⁺.

### (Step B) N-(2-(2-(2-(2-Hydroxyethoxy)ethoxy)ethoxy)ethyl)-N-methyl-2-nitrobenzenesulfonamide

To an acetonitrile solution (80 mL) of N-methyl-2-nitrobenzenesulfonamide (4.0 g, 19 mmol) were added successively 2-(2-(2-(2-hydroxyethoxy)ethoxy)ethoxy)ethyl 4-methylbenzenesulfonate (7.0 g, 20 mmol), potassium carbonate (7.6 g, 55 mmol) and potassium iodide (0.30 g, 1.8 mmol). The reaction solution was stirred at 70°C for 16 hr, diluted with ethyl acetate, and washed with water. The organic layer was dried over anhydrous sodium sulfate, and the solvent was evaporated under reduced pressure. The obtained crude product was purified by column chromatography (silica gel, methanol/DCM) to give the title compound (6.4 g).
¹H NMR (400 MHz, DMSO-d₆) δ 2.90 (s, 3H), 3.36-3.42 (m, 4H), 3.46-3.50 (m, 10H), 3.56 (t, J = 5.6 Hz, 2H), 4.56 (t, J = 5.6 Hz, 1H), 7.82-7.91 (m, 2H), 7.97-8.02 (m, 2H). MS (m/z): 393.14 [M+H]⁺.

### (Step C) 2-((2-Nitrophenyl)sulfonyl)-5,8,11-trioxa-2-azatridecan-13-yl methanesulfonate

To a DCM solution (10 mL) of N-(2-(2-(2-(2-hydroxyethoxy)ethoxy)ethoxy)ethyl)-N-methyl-2-nitrobenzenesulfonamide (1.2 g, 3.1 mmol) were added TEA (2.6 mL, 18 mmol) and methanesulfonyl chloride (1.0 g, 9.2 mmol) under ice-cooled. The mixture was stirred at room temperature for 2 hr, and the reaction solution was diluted with DCM, and washed with water. The organic layer was dried over anhydrous sodium sulfate, and the solvent was evaporated under reduced pressure. The obtained crude product was purified by column chromatography (silica gel, methanol/DCM) to give the title compound (0.60 g).
MS (m/z): 471.2 [M+H]⁺.

### (Step D) (S)-N-(Isoquinolin-6-yl)-2-((2-nitrophenyl)sulfonyl)-16-phenyl-5,8,11-trioxa-2,14-diazaheptadecan-17-amide

To an acetonitrile solution (10 mL) of (S)-3-amino-N-(isoquinolin-6-yl)-2-phenylpropanamide dihydrochloride (the compound of Reference Example 7, 1.0 g, 2.8 mmol) were added successively 2-((2-nitrophenyl)sulfonyl)-5,8,11-trioxa-2-azatridecane-13-yl methanesulfonate (1.7 g, 3.6 mmol), potassium carbonate (1.9 g, 14 mmol) and potassium iodide (0.045 g, 0.28 mmol). The reaction mixture was stirred at 70°C for 16 hr, and diluted with a methanol/DCM mixed solvent (10:90) and water. The organic layer was dried over anhydrous sodium sulfate, and the solvent was evaporated under reduced pressure. The obtained crude product was purified by HPLC preparative chromatography (column: X-SELECT CSH C18, eluent: 0.1% formic acid aqueous solution/acetonitrile, gradient mode) to give the title compound (0.40 g).
¹H NMR (400 MHz, DMSO-d₆) δ 1.23 (s, 1H), 2.69-2.73 (m, 2H), 2.79-2.84 (m, 1H), 2.87 (s, 3H), 3.26-3.29 (m, 1H), 3.34-3.39 (m, 2H), 3.41-3.45 (m, 6H), 3.51 (t, J = 4.0 Hz, 6H), 3.93 (q, J = 4.8 Hz, 1H), 7.25 (t, J = 7.2 Hz, 2H), 7.33 (t, J = 7.2 Hz, 2H), 7.40 (d, J = 7.2 Hz, 1H), 7.68 (dd, J = 15.2, 6.0 Hz, 2H), 7.81-7.89 (m, 2H), 7.95-8.04 (m, 3H), 8.39 (d, J = 6.0 Hz, 2H), 9.15 (s, 1H), 10.64 (s, 1H); MS (m/z): 666.43 [M+H]⁺.

### (Step E) tert-Butyl (S)-(3-(isoquinolin-6-ylamino)-3-oxo-2-phenylpropyl) (2-((2-nitrophenyl)sulfonyl)-5,8,11-trioxa-2-azatridecan-13-yl) carbamate

To a DCM solution (10 mL) of (S)-N-(isoquinolin-6-yl)-2-((2-nitrophenyl)sulfonyl)-16-phenyl-5,8,11-trioxa-2,14-diazaheptadecane-17-amide (0.35 g, 0.53 mmol) were added TEA (0.11 g, 1.1 mmol) and di-tert-butyl dicarbonate (0.14 g, 0.63 mmol) under ice-cooled. The mixture was stirred at room temperature for 5 hr, and the reaction solution was diluted with water, and subjected to extraction with DCM. The organic layer was dried over anhydrous sodium sulfate, and the solvent was evaporated under reduced pressure to give the title compound (0.45 g).
MS (m/z): 766.59 [M+H]⁺.

### (Step F) tert-Butyl (S)-(3-(isoquinolin-6-ylamino)-3-oxo-2-phenylpropyl) (5,8,11-trioxa-2-azatride-can-13-yl)carbamate

To a DMF solution (5.0 mL) of tert-butyl (S)-(3-(isoquinolin-6-ylamino)-3-oxo-2-phenylpropyl) (2-((2-nitrophenyl)sulfonyl)-5,8,11-trioxa-2-azatridecane-13-yl)carbamate (0.45 g, 0.59 mmol) were added thiophenol (0.10 mL, 0.88 mmol) and potassium carbonate (0.12 g, 0.88 mmol) under ice-cooled. The mixture was stirred at room temperature for 4 hr, and the reaction solution was diluted with ethyl acetate and ice water, and the aqueous layer was separated. The organic layer was washed with 1M hydrochloric acid, and the washing was alkalified with aqueous sodium hydroxide solution, and subjected to extraction with a methanol/DCM mixed solvent (10:90). The organic layer was dried over anhydrous sodium sulfate, and the solvent was evaporated under reduced pressure to give the title compound (0.22 g).
MS (m/z): 581.62 [M+H]⁺.

### (Step F) tert-Butyl (2R,5R)-4-(2-(6-(4-fluorobenzyl)-5-(14-((S)-3-(isoquinolin-6-ylamino)-3-oxo-2-phenylpropyl)-2,17,17-trimethyl-15-oxo-5,8,11,16-tetraoxa-2,14-diazaoctadecyl)-3,3-dimethyl-2,3-dihydro-1H-pyrrolo[3,2-b]pyridin-1-yl)-2-oxoethyl)-5-(methoxymethyl)-2-methylpiperazine-1-carboxylate

To an acetonitrile solution (3.0 mL) of tert-butyl (S)-(3-(isoquinolin-6-ylamino)-3-oxo-2-phenylpropyl) (5,8,11-trioxa-2-azatridecane-13-yl)carbamate (0.11 g, 0.19 mmol) were added tert-butyl (2R,5R)-4-(2-(5-(chloromethyl)-6-(4-fluorobenzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate (the compound of Example 22, Step E, 0.11 g, 0.19 mmol), potassium carbonate (0.078 g, 0.56 mmol) and potassium iodide (3.0 mg, 0.019 mmol) at room temperature. The reaction solution was stirred at 70°C for 16 hr, and diluted with a methanol/DCM mixed solvent (10:90) and water. The organic layer was dried over anhydrous sodium sulfate, and the solvent was evaporated under reduced pressure. The obtained crude product was purified by HPLC preparative chromatography (column: XS PHENYL HEXYL(REPACKED), eluent: 10 mM ammonium hydrogencarbonate aqueous solution/acetonitrile, gradient mode) to give the title compound (0.090 g).
MS (m/z): 1134.54 [M+H]⁺.

### (Step G) (S)-1-(6-(4-Fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)-N-(isoquinolin-6-yl)-2-methyl-16-phenyl-5,8,11-trioxa-2,14-diazaheptadecan-17-amide

To a DCM solution (2.0 mL) of tert-butyl (2R,5R)-4-(2-(6-(4-fluorobenzyl)-5-(14-((S)-3-(isoquinolin-6-ylamino)-3-oxo-2-phenylpropyl)-2,17,17-trimethyl-15-oxo-5,8,11,16-tetraoxa-2,14-diazaoctadecyl)-3,3-dimethyl-2,3-dihydro-1H-pyrrolo[3,2-b]pyridin-1-yl)-2-oxoethyl)-5-(methoxymethyl)-2-methylpiperazine-1-carboxylate (0.090 g, 0.0079 mmol) was added TFA (1.0 mL) under ice-cooled, and the mixture was stirred at room temperature for 16 hr. The reaction solution was concentrated, and the residue was alkalified with aqueous sodium hydrogencarbonate solution, and subjected to extraction with a methanol/DCM mixed solvent (10:90). The organic layer was dried over anhydrous sodium sulfate, and the solvent was evaporated under reduced pressure to give the title compound (0.063 g).
¹H NMR (400 MHz, DMSO-d₆) δ 0.85 (d, J = 6.0 Hz, 3H), 1.22-1.27 (m, 8H), 1.68 (brs, 1H), 1.87 (brs, 1H), 2.12 (s, 4H), 2.33-2.39 (m, 1H), 2.55-2.61 (m, 2H), 2.63-2.64 (m, 1H), 2.67-2.70 (m, 2H), 2.77-2.83 (m, 2H), 3.13 (s, 3H), 3.18-3.28 (m, 3H), 3.34-3.43 (m, 13H), 3.57 (s, 2H), 3.65 (d, J = 16.0 Hz, 1H), 3.84 (d, J = 10.4 Hz, 1H), 3.93 (dd, J = 9.2, 5.2 Hz, 1H), 4.02-4.05 (m, 3H), 7.07-7.12 (m, 2H), 7.19-7.26 (m, 3H), 7.31-7.34 (m, 2H), 7.39-7.41 (m, 2H), 7.66-7.69 (m, 2H), 7.96 (s, 1H), 8.02 (d, J = 8.8 Hz, 1H), 8.38-8.39 (m, 2H), 9.13 (s, 1H), 10.64 (s, 1H); MS (m/z): 933.69 [M+H]⁺; retention time/analysis method: 2.61 min/B.

The example compounds produced according to the above-mentioned production method or the method shown in Example 22, 29 or 38, or a method similar thereto, are described below. For each compound, the compound name, structural formula, MS (m/z) (actual measured value), retention time/analysis method (unit: minutes), and the analytical method are shown.

### Example 23

### 4-((R)-1-(6-(4-Fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)-2-methyl-5,8-dioxa-2,11-diazatridecan-12-yl)-N-(pyridin-4-yl)benzamide

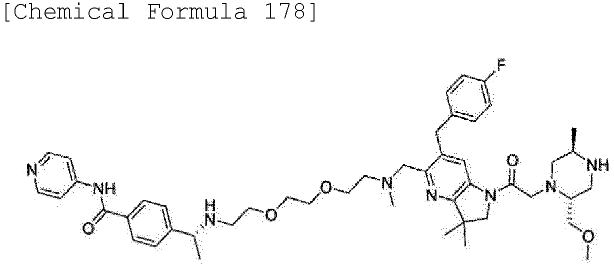

MS (m/z):839.69 [M+H]⁺; retention time/analysis method: 9.65 min/C

### Example 24

### 4-((R)-1-(6-(4-Fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)-2-methyl-5,8,11-trioxa-2,14-diazahexadecan-15-yl)-N-(pyridin-4-yl)benzamide

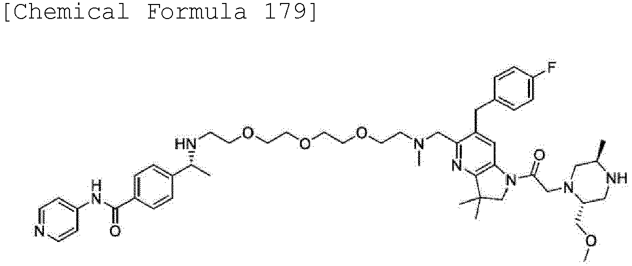

MS (m/z):883.70 [M+H]⁺; retention time/analysis method: 9.61 min/C

### Example 25

### 4-((R)-1-(6-(4-Fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)-2-methyl-5,8,11,14-tetraoxa-2,17-diazanonadecan-18-yl)-N-(pyridin-4-yl)benzamide

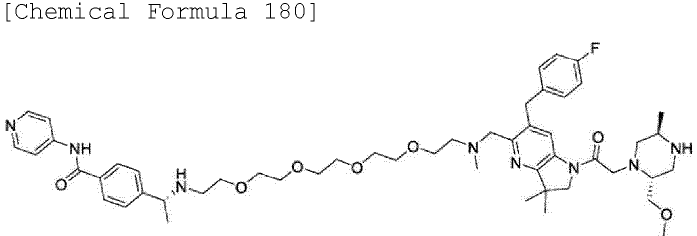

MS (m/z):927.78 [M+H]⁺; retention time/analysis method: 3.06 min/D

### Example 26

### 4-((R)-1-(6-(4-Fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)-2-methyl-5,8,11,14,17-pentaoxa-2,20-diazadocosan-21-yl)-N-(pyridin-4-yl)benzamide

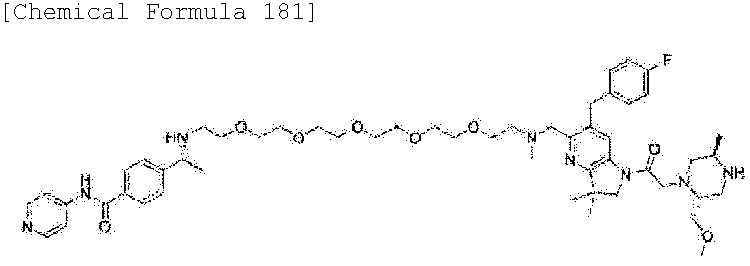

MS (m/z):971.87 [M+H]⁺; retention time/analysis method: 9.56 min/C

### Example 27

### 4-((R)-1-(6-(4-Fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)-2-methyl-5,8,11,14,17,20-hexaoxa-2,23-diazapentacosan-24-yl)-N-(pyridin-4-yl)benzamide

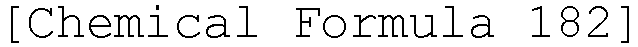

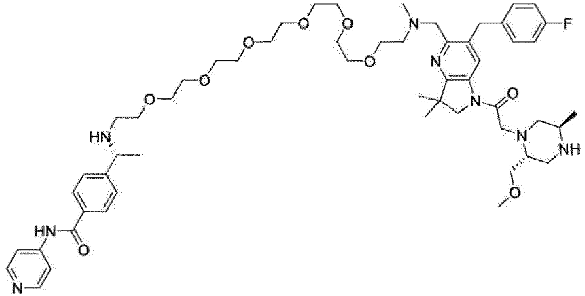

MS (m/z):1015.79 [M+H]⁺; retention time/analysis method: 9.47 min/C

### Example 28

### 4-((R)-1-(6-(4-Fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)-2-methyl-5,8,11,14,17,20,23-heptaoxa-2,26-diazaoctacosan-27-yl)-N-(pyridin-4-yl)benzamide

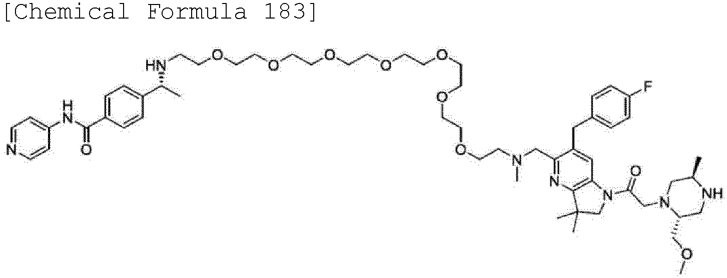

MS (m/z):1059.76 [M+H]⁺; retention time/analysis method: 2.45 min/B

### Example 30

### (R)-12-(3-Fluorobenzyl)-1-(6-(4-fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)-2-methyl-N-(4-(pyridin-4-yl)phenyl)-5,8-dioxa-2,11-diazatridecan-13-amide

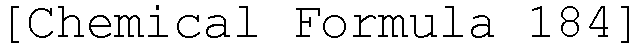

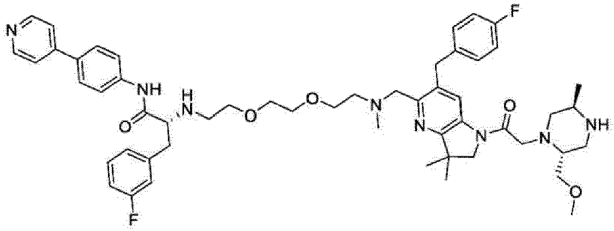

MS (m/z):933.72 [M+H]⁺; retention time/analysis method: 11.28 min/C

### Example 31

### (R)-15-(3-Fluorobenzyl)-1-(6-(4-fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)-2-methyl-N-(4-(pyridin-4-yl)phenyl)-5,8,11-trioxa-2,14-diazahexadecan-16-amide

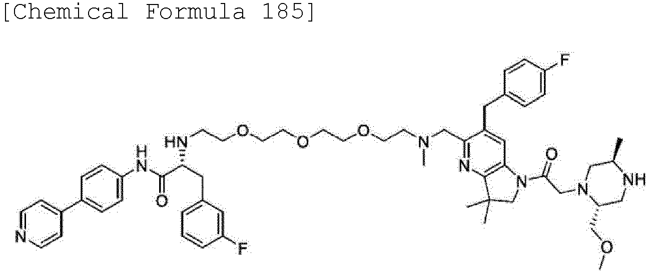

MS (m/z):977.6 [M+H]⁺; retention time/analysis method: 3.31 min/D

### Example 32

### (R)-18-(3-Fluorobenzyl)-1-(6-(4-fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)-2-methyl-N-(4-(pyridin-4-yl)phenyl)-5,8,11,14-tetraoxa-2,17-diazanonadecan-19-amide

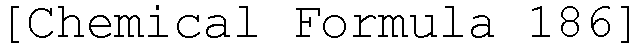

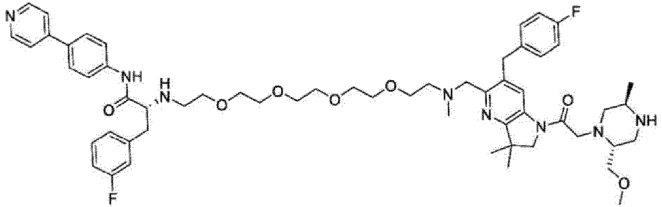

MS (m/z):1019.82 [M-H]⁻; retention time/analysis method: 11.23 min/C

### Example 33

### (R)-21-(3-Fluorobenzyl)-1-(6-(4-fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)-2-methyl-N-(4-(pyridin-4-yl)phenyl)-5,8,11,14,17-pentaoxa-2,20-diazadocosan-22-amide

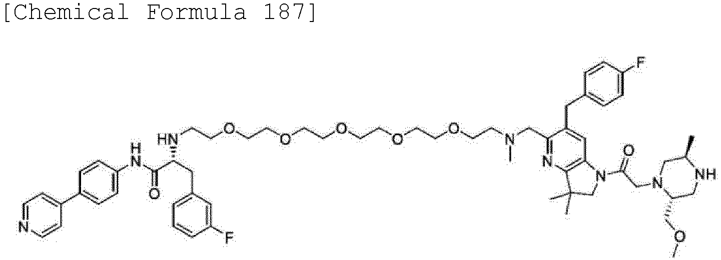

MS (m/z):1065.77 [M+H]⁺; retention time/analysis method: 10.87 min/C

### Example 34

### (R)-24-(3-Fluorobenzyl)-1-(6-(4-fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)-2-methyl-N-(4-(pyridin-4-yl)phenyl)-5,8,11,14,17,20-hexaoxa-2,23-diazapentacosan-25-amide

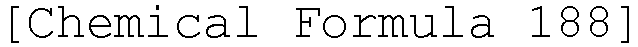

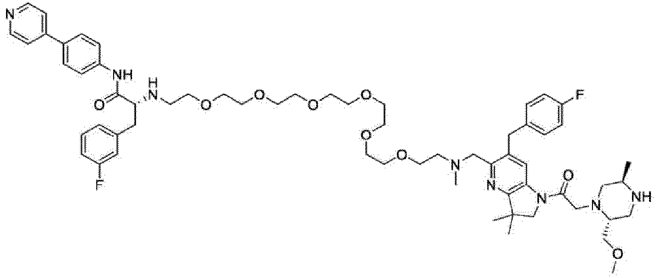

MS (m/z):1109.91 [M+H]⁺; retention time/analysis method: 10.80 min/C

### Example 35

### (R)-27-(3-Fluorobenzyl)-1-(6-(4-fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)-2-methyl-N-(4-(pyridin-4-yl)phenyl)-5,8,11,14,17,20,23-heptaoxa-2,26-diazaoctacosan-28-amide

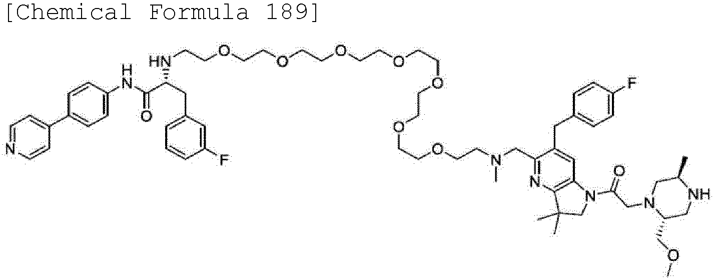

MS (m/z):1153.75 [M+H]⁺; retention time/analysis method: 10.51 min/C

### Example 36

### (S)-3-((2-(2-(((6-(4-Fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)methyl)(methyl)amino)ethoxy)ethyl)amino)-N-(isoquinolin-6-yl)-2-phenylpropanamide

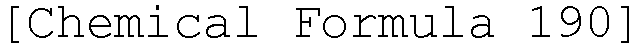

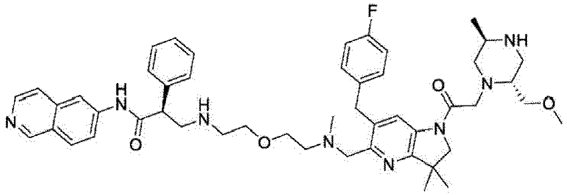

MS (m/z):845.70 [M+H]⁺; retention time/analysis method: 3.54 min/B

### Example 37

### (S)-1-(6-(4-Fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)-N-(isoquinolin-6-yl)-2-methyl-13-phenyl-5,8-dioxa-2,11-diazatetradecan-14-amide

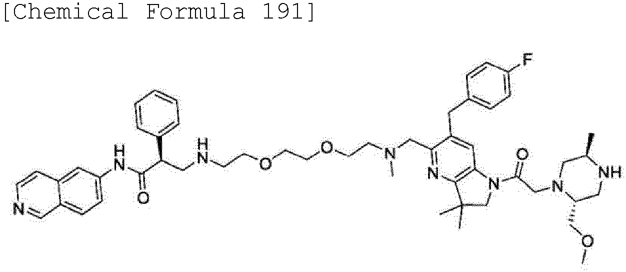

MS (m/z):889.76 [M+H]⁺; retention time/analysis method: 3.69 min/F

### Example 39

### (S)-1-(6-(4-Fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)-N-(isoquinolin-6-yl)-2-methyl-19-phenyl-5,8,11,14-tetraoxa-2,17-diazaicosan-20-amide

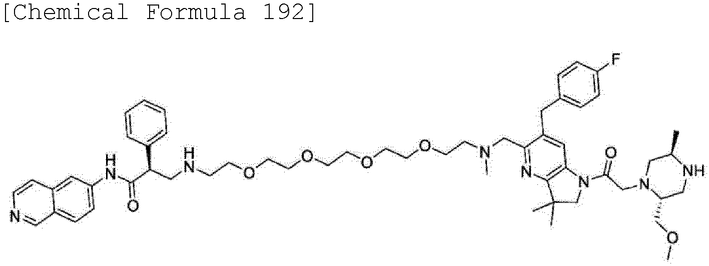

MS (m/z):977.79 [M+H]⁺; retention time/analysis method: 3.66 min/B

### Example 40

### (S)-1-(6-(4-Fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)-N-(isoquinolin-6-yl)-2-methyl-22-phenyl-5,8,11,14,17-pentaoxa-2,20-diazatricosan-23-amide

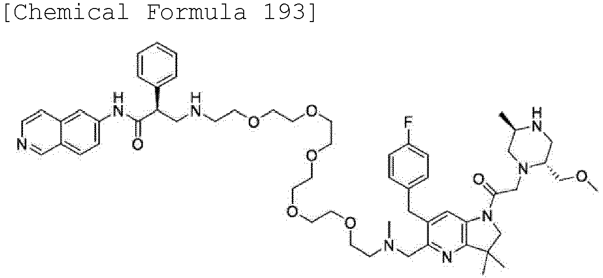

MS (m/z):1021.85 [M+H]⁺; retention time/analysis method: 3.01 min/B

### Example 41

### (S)-1-(6-(4-Fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)-N-(isoquinolin-6-yl)-2-methyl-25-phenyl-5,8,11,14,17,20-hexaoxa-2,23-diazahexacosan-26-amide

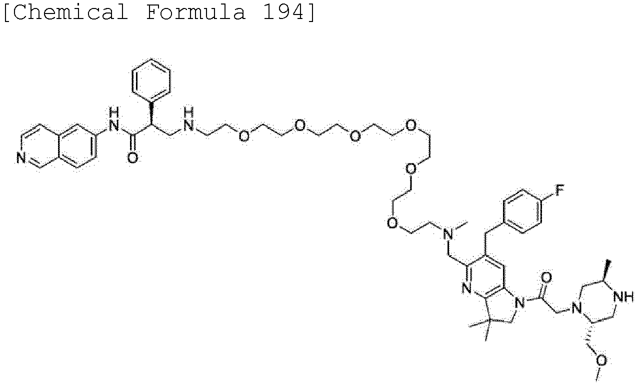

MS (m/z) :1065.91 [M+H]⁺; retention time/analysis method: 2.55 min/B

### Example 42

### (S)-1-(6-(4-Fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)-N-(isoquinolin-6-yl)-2-methyl-28-phenyl-5,8,11,14,17,20,23-heptaoxa-2,26-diazanonacosan-29-amide

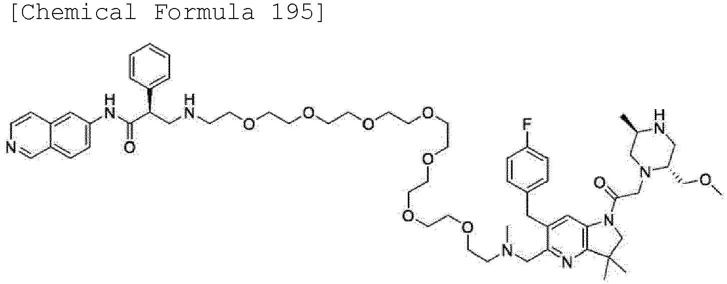

MS (m/z):1109.86 [M+H]⁺; retention time/analysis method: 2.67 min/B

### Example 48

### 4-((R)-23-(3-((1-(2-((2R,5R)-2-(Methoxymethyl)-5-methylpiperazin-1-yl)acetyl)-3,3-dimethyl-2,3-dihydro-1H-pyrrolo[3,2-b]pyridin-6-yl)methyl)phenoxy)-6,9,12,15,18,21-hexaoxa-3-azatricosan-2-yl)-N-(pyridin-4-yl)benzamide

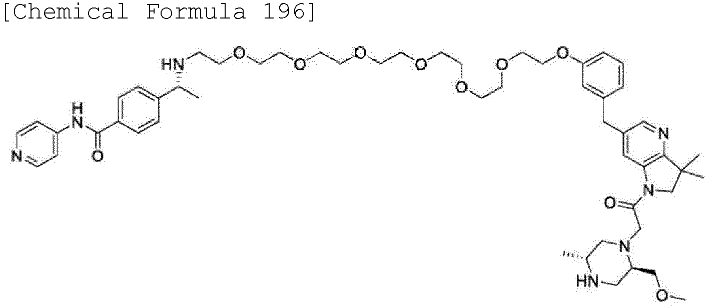

### (Step A) Ethyl 3-(benzyloxy)benzoate

To a DME solution (1.0 L) of ethyl 3-hydroxybenzoate (50 g, 0.0.30 mol) were added benzyl bromide (46 mL, 0.39 mol), potassium carbonate (83 g, 0.60 mol) and potassium iodide (2.0 g) at room temperature, and the mixture was stirred at 85°C for 24 hr. The reaction solution was filtered through Celite, and the Celite was washed with ethyl acetate. The filtrate was concentrated under reduced pressure to give the title compound (80 g) as a crude product.
¹H NMR (400 MHz, CDCl₃) δ 1.39 (t, J = 7.2 Hz, 3H), 4.37 (q, J = 7.2 Hz, 2H), 5.11 (s, 2H), 7.15-7.18 (m, 1H), 7.29-7.42 (m, 4H), 7.44-7.46 (m, 2H), 7.64-7.67 (m, 2H); MS (m/z): 257.26 [M+H]⁺.

### (Step B) (3-(Benzyloxy)phenyl)methanol

To a THF solution (0.80 L) of ethyl 3-(benzyloxy)benzoate (crude product, 80 g, 0.30 mol) was added dropwise LAH (1M THF solution, 0.16 L, 0.16 mmol) at 0°C over 10 min. The reaction solution was stirred at room temperature for 2 hr, and then water (6.0 mL) was added, followed by 5% aqueous sodium hydroxide solution (18 mL). The mixture was filtered through Celite, and the Celite was washed with ethyl acetate. The filtrate was dried over anhydrous sodium sulfate, and the solvent was concentrated under reduced pressure, and the obtained crude product was purified by column chromatography (silica gel, petroleum ether/ethyl acetate) to give the title compound (63 g).
¹H NMR (400 MHz, DMSO-d6) δ 4.46 (d, J = 5.6 Hz, 2H), 5.09 (s, 2H), 5.17 (t, J = 6.0 Hz, 1H), 6.85-6.90 (m, 2H), 6.97 (brs, 1H), 7.26 (t, J = 8.0 Hz, 1H), 7.31-7.34 (m, 1H), 7.37-7.41 (m, 2H), 7.44-7.46 (m, 2H).

### (Step C) 1-(Benzyloxy)-3-(chloromethyl)benzene

To a DCM solution (0.53 L) of (3-(benzyloxy)phenyl)methanol (53 g, 0.25 mol) was added dropwisethionyl chloride (20 mL,0.27 mol) at 0°C, and the mixture was stirred at room temperature for 6 hr. The reaction solution was neutralized with 10% aqueous sodium hydrogencarbonate solution, and subjected to extraction with DCM. The organic layer was washed with saturated brine, and dried over anhydrous sodium sulfate, and the solvent was evaporated under reduced pressure to give the title compound (53 g).

### (Step D) 2-(3-(Benzyloxy)benzyl)-4,4,5,5-tetramethyl-1,3,2-dioxaborolane

To a 1,4-dioxane solution (0.15 L) of 1-(benzyloxy)-3-(chloromethyl)benzene (10 g, 43 mmol) were added bis(pinacolato)diboron (13 g, 52 mmol), potassium acetate (13 g, 0.13 mmol) and 1,1'-bis(diphenylphosphino)ferrocene]palladium(II) dichloride dichloromethane adduct (3.5 g, 4.3 mmol), and the reaction solution was stirred under nitrogen atmosphere at 100°C for 6 hr. The reaction mixture was filtered through Celite, and the Celite was washed with ethyl acetate. The filtrate was concentrated under reduced pressure. The obtained crude product was purified by column chromatography (silica gel, petroleum ether/ethyl acetate) to give the title compound (18 g).
¹H NMR (400 MHz, CDCl₃) δ 1.23-1.26 (m, 12H), 2.27 (s, 2H), 5.07 (s, 2H), 6.74-6.83 (m, 3H), 7.15 (t, J = 8.0 Hz, 1H), 7.29-7.33 (m, 1H), 7.36-7.39 (m, 2H), 7.42-7.45 (m, 2H); MS (m/z): 325.27 [M+H]⁺.

### (Step E) tert-Butyl 6-(3-(benzyloxy)benzyl)-3,3-dimethyl-2,3-dihydro-1H-pyrrolo[3,2-b]pyridine-1-carboxylate

To a 1,4-dioxane/water mixed solution (80 mL:20 mL) of tert-butyl 6-bromo-3,3-dimethyl-2,3-dihydro-1H-pyrrolo[3,2-c]pyridine-1-carboxylate (the compound of Reference Example 3, Step D, 10 g, 31 mmol) and 2-(2-(benzyloxy)benzyl)-4,4,5,5-tetramethyl-1,3,2-dioxaborolane (12 g, 37 mmol) were added tripotassium phosphate (19 g, 92 mmol) and 1,1'-bis(diphenylphosphino)ferrocene]palladium(II) dichloride dichloromethane adduct (2.5 g, 3.1 mmol), and the mixture was stirred under nitrogen atmosphere at 100°C for 16 hr. The reaction solution was filtered through Celite, and the Celite was washed with ethyl acetate. The filtrate was concentrated under reduced pressure, and the residue was purified by column chromatography (silica gel, petroleum ether/ethyl acetate) to give the title compound (9.0 g).
¹H NMR (400 MHz, DMSO-d6) δ 1.25 (s, 6H), 1.46 (s, 9H), 3.68 (s, 2H), 3.88 (s, 2H), 5.06 (s, 2H), 6.76-6.87 (m, 2H), 6.91 (brs, 1H), 7.22 (t, J = 4.0 Hz, 1H), 7.29-7.39 (m, 3H), 7.40-7.44 (m, 2H), 7.76 (brs, 1H), 8.04 (d, J = 1.6 Hz, 1H); MS (m/z): 445.33 [M+H]⁺.

### (Step F) 6-(3-(Benzyloxy)benzyl)-3,3-dimethyl-2,3-dihydro-1H-pyrrolo[3,2-b]pyridine

To a 1,4-dioxane solution (50 mL) of tert-butyl 6-(3-(benzyloxy)benzyl)-3,3-dimethyl-2,3-dihydro-1H-pyrrolo[3,2-b]pyridine-1-carboxylate (5.0 g, 11 mmol) was added 4M hydrochloric acid/1,4-dioxane solution (50 mL) under ice-cooled, and the mixture was stirred at room temperature for 16 hr. The reaction solution was concentrated under reduced pressure, saturated aqueous sodium hydrogencarbonate solution was added, and the mixture was subjected to extraction with ethyl acetate. The organic layer was dried over anhydrous sodium sulfate, and the solvent was evaporated under reduced pressure to give the title compound (3.6 g).
¹H NMR (400 MHz, DMSO-d₆) δ 1.18 (s, 6H), 3.20 (s, 2H), 3.74 (s, 2H), 5.06 (s, 2H), 5.63 (s, 1H), 6.55 (d, J = 1.6 Hz, 1H), 6.79-6.89 (m, 3H), 7.17-7.21 (m, 1H), 7.32-7.45 (m, 5H), 7.62 (d, J = 2 Hz, 1H); MS (m/z): 345.36 [M+H]⁺.

### (Step G) 1-(6-(3-(Benzyloxy)benzyl)-3,3-dimethyl-2,3-dihydro-1H-pyrrolo[3,2-b]pyridin-1-yl)-2-chloroethan-1-one

To a THF solution (40 mL) of 6-(3-(benzyloxy)benzyl)-3,3-dimethyl-2,3-dihydro-1H-pyrrolo[3,2-b]pyridine (4.1 g, 12 mmol) were added TEA (5.0 mL, 36 mmol) and chloroacetyl chloride (1.4 mL, 18 mmol) under ice-cooled, and the mixture was stirred at room temperature for 1 hr. The reaction solution was diluted with water, and subjected to extraction with ethyl acetate.
The organic layer was dried over anhydrous sodium sulfate, and the solvent was evaporated under reduced pressure to give the title compound (4.4 g).
MS (m/z): 421.43 [M+H]⁺.

### (Step H) tert-Butyl (2R,5R)-4-(2-(6-(3-(benzyloxy)benzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate

To an acetonitrile solution (35 mL) of 1-(6-(3-(benzyloxy)benzyl)-3,3-dimethyl-2,3-dihydro-1H-pyrrolo[3,2-b]pyridin-1-yl)-2-chloroethan-1-one (6.0 g, 14 mmol) were added tert-butyl (2R,5R)-5-(methoxymethyl)-2-methylpiperazine-1-carboxylate (the compound of Reference Example 2, 3.5 g, 14 mmol), potassium carbonate (5.9 g, 43 mmol) and potassium iodide (catalytic amount). The mixture was stirred at 70°C for 16 hr, and the reaction solution was diluted with water, and subjected to extraction with ethyl acetate. The organic layer was dried over anhydrous sodium sulfate, and the solvent was evaporated under reduced pressure. The obtained crude product was purified by column chromatography (silica gel, petroleum ether/ethyl acetate) to give the title compound (5.0 g).
MS (m/z): 630.07 [M+H]⁺.

### (Step I) tert-Butyl (2R,5R)-4-(2-(6-(3-hydroxybenzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate

To a methanol solution (0.10 L) of tert-butyl (2R,5R)-4-(2-(6-(3-(benzyloxy)benzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate (5.0 g, 8.0 mmol) was added 10% palladium on carbon (1.0 g), and the mixture was stirred under hydrogen atmosphere (60 psi) at room temperature for 24 hr. The reaction solution was filtered through Celite, and the Celite was washed with a methanol/DCM mixed solvent (10:90). The filtrate was concentrated under reduced pressure, and the residue was purified by column chromatography (silica gel, petroleum ether/ethyl acetate) to give the title compound (4.0 g).
1H NMR (400 MHz, DMSO-d6) δ 1.09 (d, J = 6.4 Hz, 3H), 1.28 (s, 6H), 1.40 (s, 9H), 2.30-2.31 (m, 1H), 2.60-2.61 (m, 2H), 2.88-2.92 (m, 1H), 3.15-3.25 (m, 4H), 3.40-3.55 (m, 2H), 3.56-3.68 (m, 1H), 3.75-3.90 (m, 3H), 3.91-4.10 (m, 3H), 6.57-6.66 (m, 2H), 7.05-7.25 (m, 2H), 8.08 (m, 2H), 9.30 (s, 1H); MS (m/z): 539.92 [M+H]⁺.

### (Step J) 20-Hydroxy-3,6,9,12,15,18-hexaoxaicosyl 4-methylbenzenesulfonate

To a THF solution (0.20 L) of 3,6,9,12,15,18-hexaoxaicosane-1,20-diol (10 g, 31 mmol) was added an aqueous solution (10 mL) of sodium hydroxide (1.8 g, 46 mmol) under ice-cooled, and then a THF solution (0.10 L) of p-toluenesulfonyl chloride (5.8 g, 31 mmol) was added dropwise. The reaction solution was stirred at room temperature for 6 hr, and the solvent was evaporated under reduced pressure. The residue was diluted with ethyl acetate, and washed with water. The organic layer was dried over anhydrous sodium sulfate, and the solvent was evaporated. The obtained crude product was purified by column chromatography (silica gel, methanol/DCM) to give the title compound (4.5 g). ¹H NMR (400 MHz, DMSO-d₆) δ 2.42 (s, 3H), 3.48-3.54 (m, 24 Hz), 3.57 (t, J = 4.8 Hz, 2H), 4.11 (t, J = 4.4 Hz, 2H), 4.55-4.57 (m, 1H), 7.48 (d, J = 8.0 Hz, 2H), 7.78 (d, J = 8.0 Hz, 2H). MS (m/z): 481.50 [M+H]⁺.

### (Step K) 20-(Tosyloxy)-3,6,9,12,15,18-hexaoxaicosyl benzoate

To a DCM solution (80 mL) of 20-hydroxy-3,6,9,12,15,18-hexaoxaicosyl 4-methylbenzenesulfonate (7.3 g, 15 mmol) were added TEA (5.3 mL, 38 mmol) and benzoyl chloride (2.1 mL, 18 mmol) under ice-cooled, and the mixture was stirred at room temperature for 16 hr. The reaction solution was diluted with DCM and water, the organic layer was dried over anhydrous sodium sulfate, and the solvent was evaporated under reduced pressure. The crude product was purified by column chromatography (silica gel, methanol/DCM) to give the title compound (7.0 g).
¹H NMR (400 MHz, CDCl₃) δ 2.44 (s, 3H), 3.57-3.60 (m, 4H), 3.61-3.71 (m, 18H), 3.82-3.85 (m, 2H), 4.14-4.17 (m, 2H), 4.48 (t, J = 4.8 Hz, 2H), 7.34 (d, J = 8.0 Hz, 2H), 7.42-7.46 (m, 2H), 7.54-7.58 (m, 1H), 7.79 (d, J = 8.4 Hz, 2H), 8.04-8.07 (m, 2H). MS (m/z): 585.84 [M+H]⁺.

### (Step L) (S)-2-(4-(Pyridin-4-ylcarbamoyl)phenyl)-6,9,12,15,18,21-hexaoxa-3-azatricosan-23-yl benzoate

To an acetonitrile solution (40 mL) of (R)-4-(1-aminoethyl)-N-(pyridin-4-yl)benzamide dihydrochloride (the compound of Reference Example 5, 0.79 g, 2.5 mmol) were added 20-(tosyloxy)-3,6,9,12,15,18-hexaoxaicosyl benzoate (1.7 g, 2.8 mmol), potassium carbonate (1.0 g, 7.5 mmol) and potassium iodide (catalytic amount) at room temperature. The mixture was stirred at 80°C for 48 hr, and the reaction solution was diluted with a methanol/DCM mixed solvent (10:90), and washed with water. The organic layer was dried over anhydrous sodium sulfate, and the solvent was evaporated. The residue was purified by HPLC preparative chromatography (column: Inertsil ODS, eluent: 10 mM ammonium hydrogencarbonate aqueous solution/acetonitrile, gradient mode) to give the title compound (0.030 g).
MS (m/z): 654.43 [M+H]⁺.

### (Step M) (S)-2,2-Dimethyl-4-oxo-5-(1-(4-(pyridin-4-ylcarbamoyl)phenyl)ethyl)-3,8,11,14,17,20,23-heptaoxa-5-azapentacosan-25-yl benzoate

To a DCM solution (5.0 mL) of (S)-2-(4-(pyridin-4-ylcarbamoyl)phenyl)-6,9,12,15,18,21-hexaoxa-3-azatricosan-23-yl benzoate (0.30 g, 0.46 mmol) were added TEA (0.16 mL, 1.2 mmol) and di-tert-butyl dicarbonate (0.12 mL, 0.55 mmol) under ice-cooled, and the mixture was stirred at room temperature for 18 hr. The reaction solution was diluted with DCM and water, the organic layer was dried over anhydrous sodium sulfate, and the solvent was evaporated to give the title compound (0.34 g).
MS (m/z): 754.58 [M+H]⁺.

### (Step N) tert-Butyl (R)-(20-hydroxy-3,6,9,12,15,18-hexaoxaicosyl) (1-(4-(pyridin-4-ylcarbamoyl) phenyl) ethyl) carbamate

To a THF/methanol/water mixed solvent (5.0 mL:5.0 mL:5.0 mL) of (S)-2,2-dimethyl-4-oxo-5-(1-(4-(pyridin-4-ylcarbamoyl)phenyl)ethyl)-3,8,11,14,17,20,23-heptaoxa-5-azapentacosan-25-yl benzoate (0.42 g, 0.56 mmol) was added lithium hydroxide monohydrate (0.070 g, 1.7 mmol), and the mixture was stirred at room temperature for 18 hr. The reaction solution was concentrated under reduced pressure, and the residue was diluted with water, and subjected to extraction with a methanol/DCM mixed solvent (10:90). The organic layer was dried over anhydrous sodium sulfate, and the solvent was evaporated under reduced pressure to give the title compound (0.24 g).
MS (m/z): 649.36 [M]⁺.

### (Step O) (S)-2,2-Dimethyl-4-oxo-5-(1-(4-(pyridin-4-ylcarbamoyl)phenyl)ethyl)-3,8,11,14,17,20,23-heptaoxa-5-azapentacosan-25-yl methanesulfonate

To a DCM solution (1.0 mL) of tert-butyl (R)-(20-hydroxy-3,6,9,12,15,18-hexaoxaicosyl)(1-(4-(pyridin-4-ylcarbamoyl) phenyl) ethyl) carbamate (0.090 g, 0.14 mmol) were added TEA (0.030 g, 0.35 mmol) and methanesulfonyl chloride (0.020 g, 0.21 mmol) under ice-cooled, and the mixture was stirred at room temperature for 2 hr. To the reaction solution was added water to quench the reaction, and the mixture was subjected to extraction with DCM. The organic layer was dried over anhydrous sodium sulfate, and the solvent was evaporated to give the title compound (0.090 g).
MS (m/z): 728.70 [M+H]⁺.

### (Step P) tert-Butyl (2R,5R)-4-(2-(6-(3-((2,2-dimethyl-4-oxo-5-((S)-1-(4-(pyridin-4-ylcarbamoyl)phenyl)ethyl)-3,8,11,14,17,20,23-heptaoxa-5-azapentacosan-25-yl)oxy)benzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate

To an acetonitrile solution (3.0 mL) of tert-butyl (2R,5R)-4-(2-(6-(3-hydroxybenzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate (0.090 g, 0.17 mmol) were added (S)-2,2-dimethyl-4-oxo-5-(1-(4-(pyridin-4-ylcarbamoyl)phenyl)ethyl)-3,8,11,14,17,20,23-heptaoxa-5-azapentacosan-25-yl methanesulfonate (0.15 g, 0.20 mmol), potassium carbonate (0.070 g, 0.51 mmol) and potassium iodide (catalytic amount), and the mixture was stirred at 70°C for 48 hr. The reaction solution was diluted with water, and subjected to extraction with a methanol/DCM mixed solvent (10:90). The organic layer was dried over anhydrous sodium sulfate, and the solvent was evaporated under reduced pressure. The obtained residue was purified by HPLC preparative chromatography (column: Inertsil ODS, eluent: 10 mM ammonium hydrogencarbonate aqueous solution/acetonitrile, gradient mode) to give the title compound (0.035 g).
MS (m/z): 1171.14 [M+H]⁺.

### (Step Q) 4-((R)-23-(3-((1-(2-((2R,5R)-2-(Methoxymethyl)-5-methylpiperazin-1-yl)acetyl)-3,3-dimethyl-2,3-dihydro-1H-pyrrolo[3,2-b]pyridin-6-yl)methyl)phenoxy)-6,9,12,15,18,21-hexaoxa-3-azatricosan-2-yl)-N-(pyridin-4-yl)benzamide

To a DCM solution (2.0 mL) of tert-butyl (2R,5R)-4-(2-(6-(3-((2,2-dimethyl-4-oxo-5-((S)-1-(4-(pyridin-4-ylcarbamoyl)phenyl)ethyl)-3,8,11,14,17,20,23-heptaoxa-5-azapentacosan-25-yl)oxy)benzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate (0.035 g, 0.030 mmol) was added TFA (0.40 mL) under ice-cooled, and the mixture was stirred at room temperature for 4 hr. The reaction solution was concentrated under reduced pressure, and the residue was dissolved in methanol, and the solution was filtered through SCX column. The column was washed with methanol (2.0 mL), and the adsorbed components were eluted by passing 7M ammonia/methanol solution (5.0 mL). The fraction obtained by ammonia/methanol treatment was concentrated under reduced pressure to give the title compound (0.028 g).
¹H NMR (400 MHz, DMSO-d₆) δ 1.01-1.03 (m, 3H), 1.30-1.40 (m, 10H), 2.20-2.35 (m, 1H), 2.50-2.70 (m, 4H), 2.80-2.95 (m, 3H), 3.35-3.40 (m, 1H), 3.41-3.45 (m, 1H), 3.48-3.62 (m, 24H), 3.65-3.70 (m, 3H), 3.72-3.82 (m, 3H), 3.85-3.98 (m, 5H), 3.99-4.10 (m, 3H), 6.75-6.79 (m, 3H), 7.15-7.19 (m, 1H), 7.50-7.52 (m, 2H), 7.84-8.07 (m, 5H), 8.23-8.43 (m, 3H); MS (m/z):970.72 [M+H]⁺; retention time/analysis method: 8.85 min/C.

### Example 73

### (R)-2-(3-Fluorobenzyl)-14-(2-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)-N-(4-(pyridin-4-yl)phenyl)-6,9,12-trioxa-3-azatetradecanamide

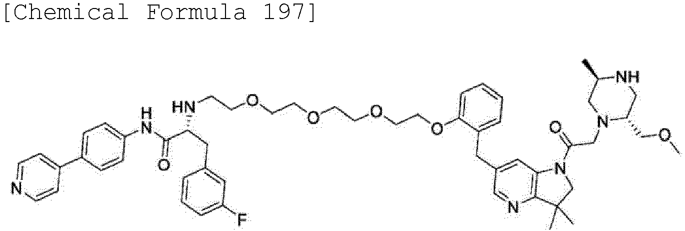

### (Step A) tert-Butyl (2R,5R)-4-(2-(6-(2-hydroxybenzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate

The title compound was obtained using ethyl 2-hydroxybenzoate as a starting raw material, according to the method described in Steps A to I of Example 48.
¹H NMR (400 MHz, CDCl₃) δ 1.21 (d, J = 6.4 Hz, 3H), 1.39 (s, 6H), 1.46 (s, 9H), 2.63-2.66 (m, 1H), 2.8-2.81 (m, 1H), 2.99 (d, J = 3.6 Hz, 1H), 3.26-3.40 (m, 5H), 3.44 (s, 1H), 3.49 (s, 1H), 3.59-3.64 (m, 2H), 3.86 (d, J = 10.0 Hz, 1H), 3.92-3.99 (m, 3H), 4.19 (d, J = 5.6 Hz, 1H), 6.77 (dd, J = 8.0, 0.8 Hz, 1H), 6.83-6.86 (m, 1H), 7.06-7.14 (m, 2H), 8.19 (d, J = 1.6 Hz, 1H), 8.35 (d, J = 1.6 Hz, 1H), one proton missing; MS (m/z): 540.04 [M+H]⁺.

### (Step B) 2-(2-(2-(2-(Tosyloxy)ethoxy)ethoxy)ethoxy)ethyl benzoate

To a DCM solution (50 mL) of 2-(2-(2-(2-hydroxyethoxy)ethoxy)ethoxy)ethyl 4-methylbenzenesulfonate (5.0 g, 14 mmol) were added TEA (4.8 mL, 36 mmol) and benzoyl chloride (1.8 mL, 16 mmol) under ice-cooled, and the mixture was stirred at room temperature for 8 hr. The reaction solution was diluted with DCM, and washed with water. The organic layer was dried over anhydrous sodium sulfate, and the solvent was evaporated under reduced pressure. The obtained crude product was purified by column chromatography (silica gel, methanol/DCM) to give the title compound (5.0 g).
¹H NMR (400 MHz, DMSO-d₆) δ 2.41 (s, 3H), 3.32-3.45 (m, 4H), 3.49-3.59 (m, 6H), 3.73-3.75 (m, 2H), 4.08-4.10 (m, 2H), 4.37-4.39 (m, 2H), 7.46-7.55 (m, 4H), 7.64-7.66 (m, 1H), 7.77 (d, J = 8.4 Hz, 2H), 7.95-7.97 (m, 2H). MS (m/z): 453.27 [M+H]⁺.

### (Step C) tert-Butyl (2S,5S)-4-(2-(3,3-dimethyl-6-(2-((1-oxo-1-phenyl-2,5,8,11-tetraoxatridecan-13-yl)oxy)benzyl)-2,3-dihydro-1H-pyrrolo[3,2-b]pyridin-1-yl)-2-oxoethyl)-5-(methoxymethyl)-2-methylpiperazine-1-carboxylate

To an acetonitrile solution (20 mL) of tert-butyl (2R,5R)-4-(2-(6-(2-hydroxybenzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate (0.75 g, 1.4 mmol) were added 2-(2-(2-(2-(tosyloxy)ethoxy)ethoxy)ethoxy)ethyl benzoate (0.69 g, 1.5 mmol), potassium carbonate (0.58 g, 4.2 mmol) and potassium iodide (catalytic amount), and the mixture was stirred at 75°C for 36 hr. The reaction solution was diluted with a methanol/DCM mixed solvent (10:90), and washed with water. The organic layer was dried over anhydrous sodium sulfate, and the solvent was evaporated under reduced pressure. The obtained crude product was purified by column chromatography (silica gel, methanol/DCM) to give the title compound (1.1 g).
MS (m/z): 819.76 [M+H]⁺.

### (Step D) tert-Butyl (2R,5R)-4-(2-(6-(2-(2-(2-(2-(2-hydroxyethoxy)ethoxy)ethoxy)ethoxy)benzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate

To a THF/water mixed solution (5 mL:5 mL) of tert-butyl (2S,5S)-4-(2-(3,3-dimethyl-6-(2-((1-oxo-1-phenyl-2,5,8,11-tetraoxatridecane-13-yl)oxy)benzyl)-2,3-dihydro-1H-pyrrolo[3,2-b]pyridin-1-yl)-2-oxoethyl)-5-(methoxymethyl)-2-methylpiperazine-1-carboxylate (1.1 g, 1.3 mmol) was added lithium hydroxide monohydrate (0.17 g, 4.0 mmol) under ice-cooled, and the mixture was stirred at room temperature for 18 hr. The reaction solution was concentrated under reduced pressure, and the residue was diluted with water, and subjected to extraction with methanol/DCM (10:90). The organic layer was dried over anhydrous sodium sulfate, and the solvent was evaporated under reduced pressure to give the title compound (0.40 g).
¹H NMR (400 MHz, DMSO-d₆) δ 1.09-1.11 (m, 3H), 1.24-1.27 (m, 6H), 1.40 (s, 9H), 2.55-2.60 (m, 1H), 2.62-2.70 (m, 1H), 2.85-2.95 (m, 1H), 3.10-3.20 (m, 5H), 3.40-3.60 (m, 13H), 3.62-3.70 (m, 3H), 3.75-3.82 (m, 4H), 3.90-4.20 (m, 5H), 4.50-4.60 (m, 1H), 6.89-6.97 (m, 2H), 7.18-7.21 (m, 2H), 8.10-8.16 (m, 2H); MS (m/z): 715.76 [M+H]⁺.

### (Step E) tert-Butyl (2S,5S)-4-(2-(3,3-dimethyl-6-(2-(2-(2-(2-(2-((methylsulfonyl)oxy)ethoxy)ethoxy)ethoxy)ethoxy)benzyl)-2,3-dihydro-1H-pyrrolo[3,2-b]pyridin-1-yl)-2-oxoethyl)-5-(methoxymethyl)-2-methylpiperazine-1-carboxylate

To a DCM solution (1.0 mL) of tert-butyl (2R,5R)-4-(2-(6-(2-(2-(2-(2-(2-hydroxyethoxy)ethoxy)ethoxy)ethoxy)benzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate (0.050 g, 0.070mmol) were added TEA (0.029 mL, 0.21 mmol) and methanesulfonyl chloride (0.010 mL, 0.17 mmol) under ice-cooled, and the mixture was stirred at room temperature for 2 hr. The reaction solution was diluted with water, and subjected to extraction with DCM. The organic layer was washed with aqueous sodium hydrogencarbonate solution, and dried over anhydrous sodium sulfate. The solvent was evaporated under reduced pressure to give the title compound (0.055 g).

### (Step F) tert-Butyl (2R,5R)-4-(2-(6-(2-(((R)-2-(3-fluorobenzyl)-1-oxo-1-((4-(pyridin-4-yl)phenyl)amino)-6,9,12-trioxa-3-azatetradecan-14-yl)oxy)benzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate

To an acetonitrile solution (5.0 mL) of tert-butyl (2S,5S)-4-(2-(3,3-dimethyl-6-(2-(2-(2-(2-(2-((methylsulfonyl)oxy)ethoxy)ethoxy)ethoxy)ethoxy)benzyl)-2,3-dihydro-1H-pyrrolo[3,2-b]pyridin-1-yl)-2-oxoethyl)-5-(methoxymethyl)-2-methylpiperazine-1-carboxylate (0.28 g, 0.35 mmol) were added (R)-2-amino-3-(3-fluorophenyl)-N-(4-(pyridin-4-yl)phenyl)propanamide dihydrochloride (the compound of Reference Example 6, 0.28 g, 0.69 mmol), potassium carbonate (0.57 g, 4.2 mmol) and potassium iodide (catalytic amount), and the mixture stirred at 75°C for 36 hr. The reaction solution was diluted with a methanol/DCM mixed solvent (10:90), and washed with water. The organic layer was dried over anhydrous sodium sulfate, and the solvent was evaporated to give a crude product. This was purified by HPLC preparative chromatography (column: Inertsil ODS, eluent: 10 mM ammonium hydrogencarbonate aqueous solution/acetonitrile, gradient mode) to give the title compound (0.028 g).
MS (m/z): 1033.04 [M+H]⁺.

### (Step G) (R)-2-(3-Fluorobenzyl)-14-(2-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)-N-(4-(pyridin-4-yl)phenyl)-6,9,12-trioxa-3-azatetradecanamide

To a DCM solution (1.0 mL) of tert-butyl (2R,5R)-4-(2-(6-(2-(((R)-2-(3-fluorobenzyl)-1-oxo-1-((4-(pyridin-4-yl)phenyl) amino)-6,9,12-trioxa-3-azatetradecane-14-yl)oxy)benzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate (0.031 g, 0.030 mmol) was added TFA (0.20 mL) under ice-cooled, and the mixture was stirred at room temperature for 4 hr. The reaction solution was concentrated under reduced pressure, and the residue was dissolved in methanol, and the solution was filtered through SCX column. The column was washed with methanol, and the adsorbed components were eluted by passing 7M ammonia/methanol solution. The obtained ammonia/methanol fraction was concentrated under reduced pressure to give the title compound (0.018 g).
¹H NMR (400 MHz, DMSO-d₆) δ 0.93-0.94 (m, 3H), 1.23-1.25 (m, 7H), 2.57-2.73 (m, 3H), 2.85-3.00 (m, 5H), 3.10-3.30 (m, 4H), 3.40-3.70 (m, 16H), 3.72-3.90 (m, 6H), 3.98-4.10 (m, 3H), 6.85-6.94 (m, 2H), 6.99-7.02 (m, 1H), 7.06-7.12 (m, 2H), 7.14-7.19 (m, 2H), 7.27-7.29 (m, 1H), 7.65-7.78 (m, 6H), 8.09-8.11 (m, 2H), 8.56-8.59 (m, 2H), 10.05 (s, 1H). MS (m/z):932.95 [M+H]⁺; retention time/analysis method: 9.64 min/I.

### Example 80

### (S)-N-(Isoquinolin-6-yl)-1-(2-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)-14-phenyl-3,6,9-trioxa-12-azapentadecan-15-amide

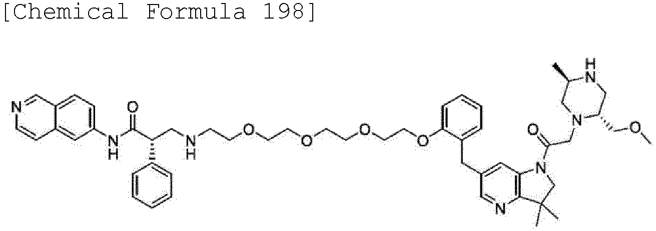

### (Step A) (S)-15-(Isoquinolin-6-ylamino)-15-oxo-14-phenyl-3,6,9-trioxa-12-azapentadecyl benzoate

To an acetonitrile solution (30 mL) of (S)-3-amino-N-(isoquinolin-6-yl)-2-phenylpropanamide dihydrochloride (the compound of Reference Example 7, 0.80 g, 2.4 mmol) were added 2-(2-(2-(2-(tosyloxy)ethoxy)ethoxy)ethoxy)ethyl benzoate (1.2 g, 2.7 mmol), potassium carbonate (1.0 g, 7.3 mmol) and potassium iodide (catalytic amount), and the mixture was stirred at 70°C for 18 hr. The reaction solution was diluted with water, and subjected to extraction with ethyl acetate. The organic layer was dried over anhydrous sodium sulfate, and the solvent was evaporated under reduced pressure. The obtained crude product was purified by column chromatography (silica gel, methanol/DCM) to give the title compound (0.59 g). ¹H NMR (400 MHz, DMSO-d₆) δ 2.35-2.40 (m, 1H), 2.60-2.85 (m, 3H), 3.25-3.30 (m, 1H), 3.40-3.60 (m, 9H), 3.65-3.75 (m, 2H), 3.85-3.90 (m, 1H), 4.35-4.36 (m, 2H), 7.24-7.27 (m, 1H), 7.32-7.35 (m, 2H), 7.39-7.41 (m, 2H), 7.50-7.53 (m, 2H), 7.63-7.70 (m, 3H), 7.94-8.04 (m, 3H), 8.39-8.40 (m, 2H), 9.14 (s, 1H), 10.60 (s, 1H). MS (m/z): 572.40 [M+H]⁺.

### (Step B) (R)-5-(3-(Isoquinolin-6-ylamino)-3-oxo-2-phenylpropyl)-2,2-dimethyl-4-oxo-3,8,11,14-tetraoxa-5-azahexadecan-16-yl benzoate

To a DCM solution (10 mL) of (S)-15-(isoquinolin-6-ylamino)-15-oxo-14-phenyl-3,6,9-trioxa-12-azapentadecyl benzoate (0.59 g, 1.0 mmol) were added TEA (0.36 mL, 2.6 mmol) and di-tert-butyl dicarbonate (0.28 mL, 1.2 mmol) under ice-cooled, and the mixture was stirred at room temperature for 16 hr. The reaction solution was diluted with DCM, and washed with water. The organic layer was dried over anhydrous sodium sulfate, and the solvent was evaporated under reduced pressure to give the title compound (0.60 g).
MS (m/z): 673.02 [M+H]⁺.

### (Step C) tert-Butyl (S)-(2-(2-(2-(2-hydroxyethoxy) ethoxy) ethoxy) ethyl) (3-(isoquinolin-6-ylamino)-3-oxo-2-phenylpropyl)carbamate

To a THF/methanol/water mixed solution (5 mL:5 mL:5 mL) of (R)-5-(3-(isoquinolin-6-ylamino)-3-oxo-2-phenylpropyl)-2,2-dimethyl-4-oxo-3,8,11,14-tetraoxa-5-azahexadecane-16-yl benzoate (0.70 g, 1.0 mmol) was added lithium hydroxide monohydrate (0.13 g, 3.1 mmol), and the mixture was stirred at room temperature for 18 hr. The reaction solution was concentrated under reduced pressure, and the residue was diluted with water, and subjected to extraction with a methanol/DCM mixed solvent (10:90). The organic layer was dried over anhydrous sodium sulfate, and the solvent was evaporated. The obtained crude product was purified by column chromatography (silica gel, methanol/DCM) to give the title compound (0.50 g).
MS (m/z): 568.97 [M+H]⁺.

### (Step D) (S)-5-(3-(Isoquinolin-6-ylamino)-3-oxo-2-phenylpropyl)-2,2-dimethyl-4-oxo-3,8,11,14-tetraoxa-5-azahexadecan-16-yl methanesulfonate

To a DCM solution (5.0 mL) of tert-butyl (S)-(2-(2-(2-(2-hydroxyethoxy) ethoxy) ethoxy) ethyl) (3-(isoquinolin-6-ylamino)-3-oxo-2-phenylpropyl)carbamate (0.20 g, 0.35 mmol) were added TFA (0.12 mL, 0.88 mmol) and methanesulfonyl chloride (0.080 mL, 0.53 mmol) under ice-cooled, and the mixture was stirred at room temperature for 2 hr. The reaction solution was diluted with water, and subjected to extraction with DCM. The organic layer was dried over anhydrous sodium sulfate, and the solvent was evaporated under reduced pressure to give the title compound (0.25 g).
MS (m/z): 646.41 [M+H]⁺.

### (Step E) tert-Butyl (2R,5R)-4-(2-(6-(2-((5-((S)-3-(isoquinolin-6-ylamino)-3-oxo-2-phenylpropyl)-2,2-dimethyl-4-oxo-3,8,11,14-tetraoxa-5-azahexadecan-16-yl)oxy)benzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate

To a DMF solution (5.0 mL) of tert-butyl (2R,5R)-4-(2-(6-(2-hydroxybenzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate (the compound of Example 73, Step A, 0.18 g, 0.33 mmol) were added (S)-5-(3-(isoquinolin-6-ylamino)-3-oxo-2-phenylpropyl)-2,2-dimethyl-4-oxo-3,8,11,14-tetraoxa-5-azahexadecan-16-yl methanesulfonate (0.23 g, 0.37 mmol), potassium carbonate (0.14 g, 1.0 mmol) and potassium iodide (catalytic amount), and the mixture was stirred at 70°C for 18 hr. The reaction solution was diluted with water, and subjected to extraction with ethyl acetate. The organic layer was dried over anhydrous sodium sulfate, and the solvent was evaporated under reduced pressure. The obtained crude product was purified by HPLC preparative chromatography (column: HICHROME, eluent: 10 mM ammonium hydrogencarbonate aqueous solution/acetonitrile, gradient mode) to give the title compound (0.045 g).
MS (m/z): 1088.94 [M+H]⁺.

### (Step F) (S)-N-(Isoquinolin-6-yl)-1-(2-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)-14-phenyl-3,6,9-trioxa-12-azapentadecan-15-amide

To a DCM solution (1.0 mL) of tert-butyl (2R,5R)-4-(2-(6-(2-((5-((S)-3-(isoquinolin-6-ylamino)-3-oxo-2-phenylpropyl)-2,2-dimethyl-4-oxo-3,8,11,14-tetraoxa-5-azahexadecane-16-yl)oxy)benzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate (0.050 g, 0.050 mmol) was added TFA (0.50 mL) under ice-cooled, and the mixture was stirred at room temperature for 4 hr. The reaction solution was concentrated under reduced pressure, and the residue was dissolved in methanol. The solution was filtered through SCX column, and the column was washed with methanol (2 mL). Then, the adsorbed components were eluted by passing 7M ammonia/methanol solution (5 mL). The obtained filtrate was concentrated under reduced pressure to give the title compound (0.039 g).
¹H NMR (400 MHz, DMSO-d₆) δ 0.79-0.92 (m, 1H), 1.27-1.37 (m, 11H), 1.64-1.68 (m, 1H), 2.21-2.25 (m, 1H), 2.85-3.0 (m, 2H), 3.05-3.15 (m, 1H), 3.18-3.20 (m, 4H), 3.32-3.35 (m, 1H), 3.38-3.45 (m, 1H), 3.48-3.52 (m, 2H), 3.58-3.62 (m, 1H), 3.63-3.70 (m, 8H), 3.71-3.80 (m, 5H), 3.82-3.98 (m, 5H), 4.00-4.20 (m, 2H), 4.35-4.45 (m, 1H), 6.80-6.90 (m, 2H), 7.10-7.20 (m, 2H), 7.30-7.50 (m, 5H), 8.0-8.10 (m, 2H), 8.21-8.42 (m, 4H), 8.74-8.75 (m, 1H), 9.50 (s, 1H), one proton missing; MS (m/z):888.74 [M+H]⁺; retention time/analysis method: 9.97 min/C.

The example compounds produced according to the above-mentioned production method or the method shown in Example 48, 73 or 80, or a method similar thereto, are described below. For each compound, the compound name, structural formula, MS (m/z) (actual measured value), retention time/analysis method (unit: minutes), and the analytical method are shown.

### Example 43

### 4-((R)-1-((2-(2-(3-((1-(2-((2R,5R)-2-(Methoxymethyl)-5-methylpiperazin-1-yl)acetyl)-3,3-dimethyl-2,3-dihydro-1H-pyrrolo[3,2-b]pyridin-6-yl)methyl)phenoxy)ethoxy)ethyl)amino)ethyl)-N-(pyridin-4-yl)benzamide

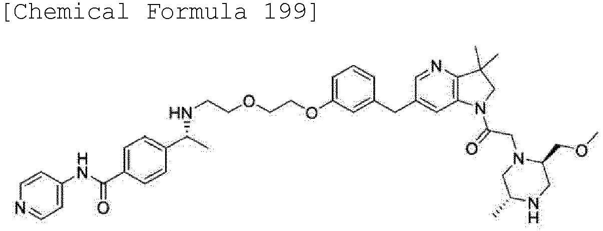

MS (m/z):750.64 [M+H]⁺; retention time/analysis method: 8.07 min/I

### Example 44

### 4-((R)-1-((2-(2-(2-(3-((1-(2-((2R,5R)-2-(Methoxymethyl)-5-methylpiperazin-1-yl)acetyl)-3,3-dimethyl-2,3-dihydro-1H-pyrrolo[3,2-b]pyridin-6-yl)methyl)phenoxy)ethoxy)ethoxy)ethyl)amino)ethyl)-N-(pyridin-4-yl)benzamide

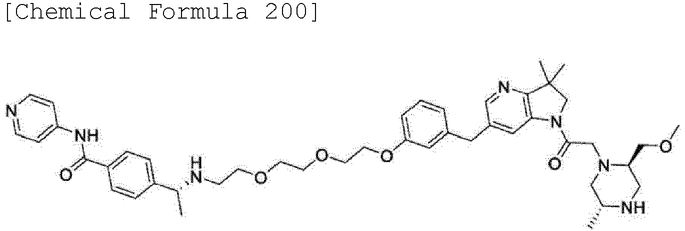

MS (m/z):794.57 [M+H]⁺; retention time/analysis method: 8.91 min/C

### Example 45

### 4-((R)-14-(3-((1-(2-((2R,5R)-2-(Methoxymethyl)-5-methylpiperazin-1-yl)acetyl)-3,3-dimethyl-2,3-dihydro-1H-pyrrolo[3,2-b]pyridin-6-yl)methyl)phenoxy)-6,9,12-trioxa-3-azatetradecan-2-yl)-N-(pyridin-4-yl)benzamide

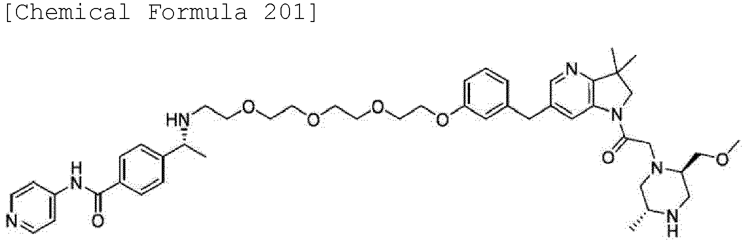

MS (m/z):838.59 [M+H]⁺; retention time/analysis method: 8.88 min/C

### Example 46

### 4-((R)-17-(3-((1-(2-((2R,5R)-2-(Methoxymethyl)-5-methylpiperazin-1-yl)acetyl)-3,3-dimethyl-2,3-dihydro-1H-pyrrolo[3,2-b]pyridin-6-yl)methyl)phenoxy)-6,9,12,15-tetraoxa-3-azaheptadecan-2-yl)-N-(pyridin-4-yl)benzamide

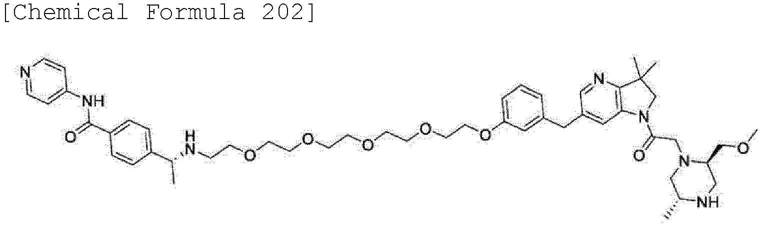

MS (m/z):882.61 [M+H]⁺; retention time/analysis method: 8.88 min/C

### Example 47

### 4-((R)-20-(3-((1-(2-((2R,5R)-2-(Methoxymethyl)-5-methylpiperazin-1-yl)acetyl)-3,3-dimethyl-2,3-dihydro-1H-pyrrolo[3,2-b]pyridin-6-yl)methyl)phenoxy)-6,9,12,15,18-pentaoxa-3-azaicosan-2-yl)-N-(pyridin-4-yl)benzamide

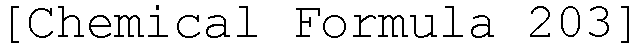

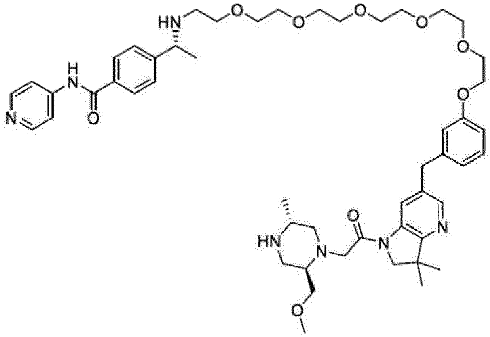

MS (m/z):926.62 [M+H]⁺; retention time/analysis method: 8.88 min/C

### Example 49

### 4-((R)-26-(3-((1-(2-((2R,5R)-2-(Methoxymethyl)-5-methylpiperazin-1-yl)acetyl)-3,3-dimethyl-2,3-dihydro-1H-pyrrolo[3,2-b]pyridin-6-yl)methyl)phenoxy)-6,9,12,15,18,21,24-heptaoxa-3-azahexacosan-2-yl)-N-(pyridin-4-yl)benzamide

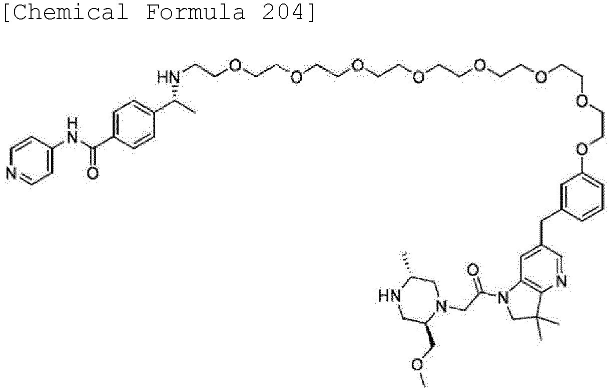

MS (m/z):1014.71 [M+H]⁺; retention time/analysis method: 8.76 min/C

### Example 50

### (R)-3-(3-Fluorophenyl)-2-((2-(2-(3-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy) ethoxy)ethyl)amino)-N-(4-(pyridin-4-yl)phenyl)propanamide

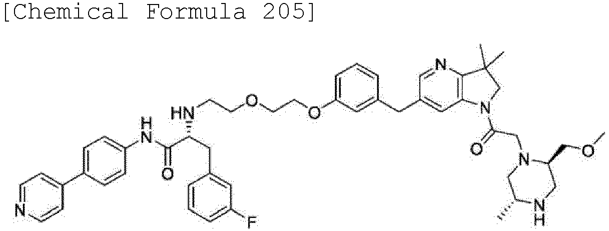

MS (m/z):844.41 [M+H]⁺; retention time/analysis method: 9.64 min/I

### Example 51

### (R)-3-(3-Fluorophenyl)-2-((2-(2-(2-(3-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)ethoxy)ethoxy)ethyl)amino)-N-(4-(pyridin-4-yl)phenyl)propenamide

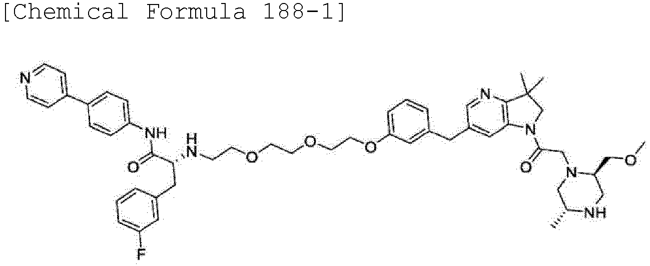

MS (m/z):888.70 [M+H]⁺; retention time/analysis method: 10.17 min/C

### Example 52

### (R)-2-(3-Fluorobenzyl)-14-(3-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)-N-(4-(pyridin-4-yl)phenyl)-6,9,12-trioxa-3-azatetradecanamide

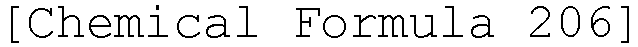

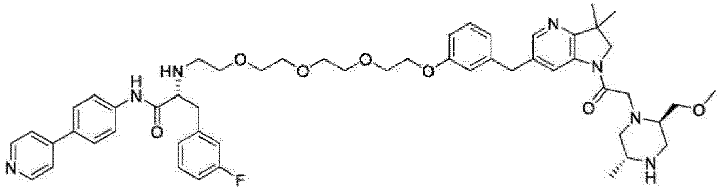

MS (m/z):932.79 [M+H]⁺; retention time/analysis method: 5.10 min/L

### Example 53

### (R)-2-(3-Fluorobenzyl)-17-(3-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)-N-(4-(pyridin-4-yl)phenyl)-6,9,12,15-tetraoxa-3-azaheptadecanamide

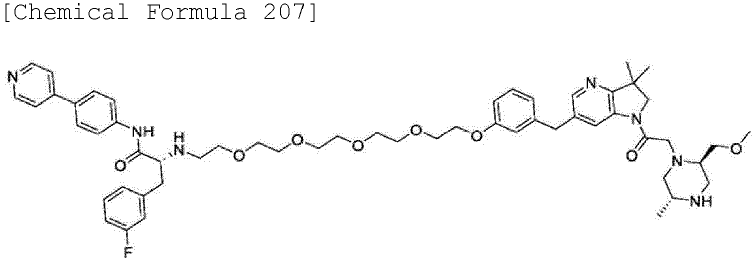

MS (m/z):977.05 [M+H]⁺; retention time/analysis method: 5.09 min/L

### Example 54

### (R)-2-(3-Fluorobenzyl)-20-(3-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)-N-(4-(pyridin-4-yl)phenyl)-6,9,12,15,18-pentaoxa-3-azaicosanamide

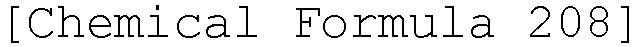

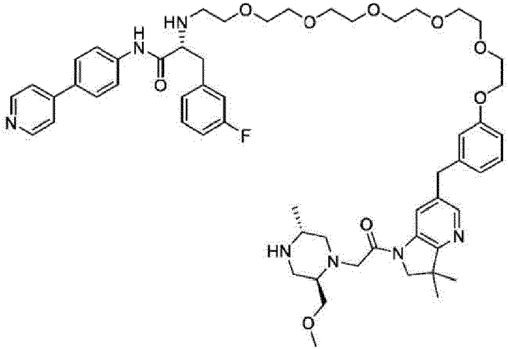

MS (m/z):1020.99 [M+H]⁺; retention time/analysis method: 5.04 min/K

### Example 55

### (R)-2-(3-Fluorobenzyl)-23-(3-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)-N-(4-(pyridin-4-yl)phenyl)-6,9,12,15,18,21-hexaoxa-3-azatricosanamide

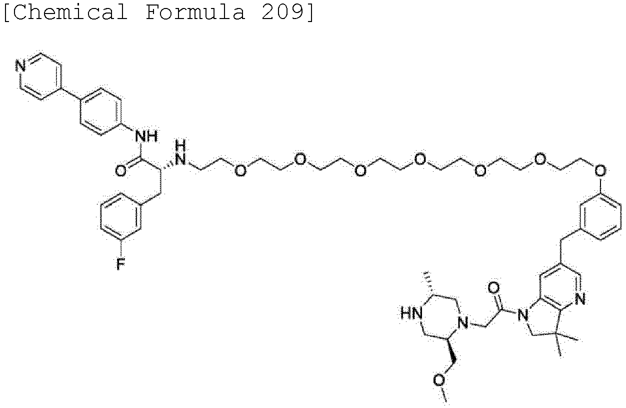

MS (m/z):1064.72 [M+H]⁺; retention time/analysis method: 5.07 min/L

### Example 56

### (R)-2-(3-Fluorobenzyl)-26-(3-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)-N-(4-(pyridin-4-yl)phenyl)-6,9,12,15,18,21,24-heptaoxa-3-azahexacosanamide

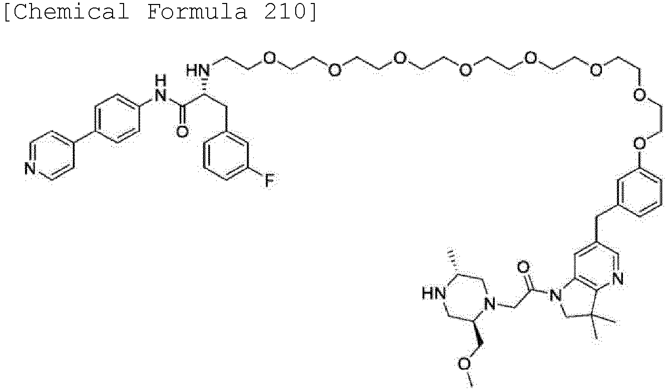

MS (m/z):1108.77 [M+H]⁺; retention time/analysis method: 5.04 min/L

### Example 57

### (S)-N-(Isoquinolin-6-yl)-3-((2-(2-(3-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)ethoxy)ethyl)amino)-2-phenylpropanamide

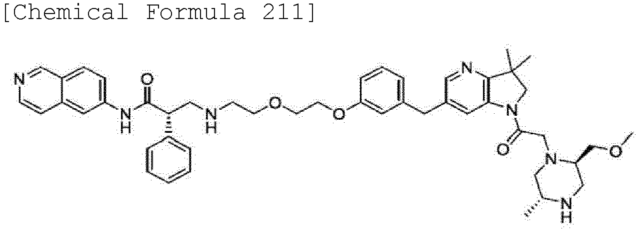

MS (m/z):800.58 [M+H]⁺; retention time/analysis method: 4.81 min/K

### Example 58

### (S)-N-(Isoquinolin-6-yl)-3-((2-(2-(2-(3-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)ethoxy)ethoxy)ethyl)amino)-2-phenylpropanamide

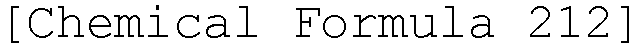

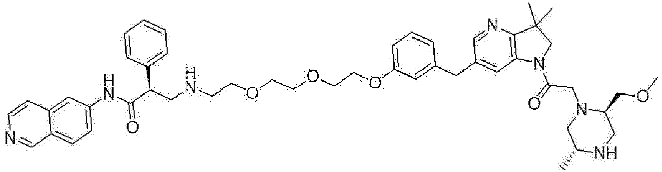

MS (m/z):844.60 [M+H]⁺; retention time/analysis method: 9.72 min/C

### Example 59

### (S)-N-(Isoquinolin-6-yl)-1-(3-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)-14-phenyl-3,6,9-trioxa-12-azapentadecan-15-amide

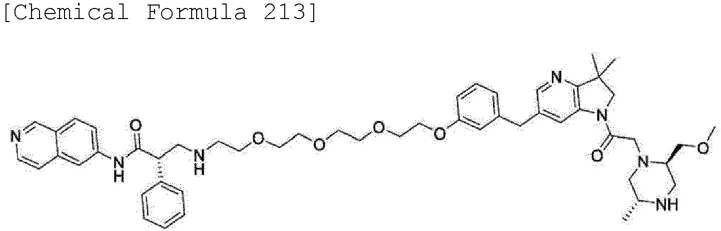

MS (m/z):888.62 [M+H]⁺; retention time/analysis method: 9.81 min/C

### Example 60

### (S)-N-(Isoquinolin-6-yl)-1-(3-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)-17-phenyl-3,6,9,12-tetraoxa-15-azaoctadecan-18-amide

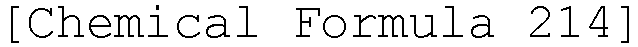

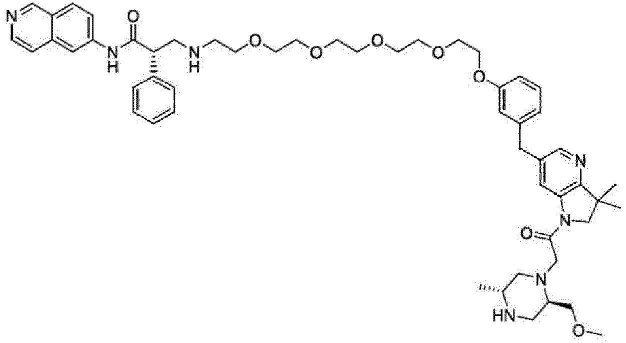

MS (m/z):932.71 [M+H]⁺; retention time/analysis method: 9.60 min/C

### Example 61

### (S)-N-(Isoquinolin-6-yl)-1-(3-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)-20-phenyl-3,6,9,12,15-pentaoxa-18-azahenicosan-21-amide

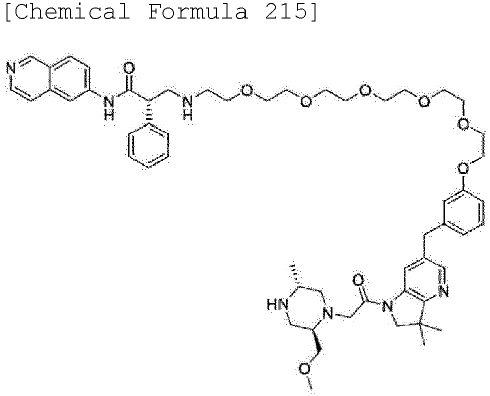

MS (m/z):976.60 [M+H]⁺; retention time/analysis method: 9.61 min/C

### Example 62

### (S)-N-(Isoquinolin-6-yl)-1-(3-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)-23-phenyl-3,6,9,12,15,18-hexaoxa-21-azatetracosan-24-amide

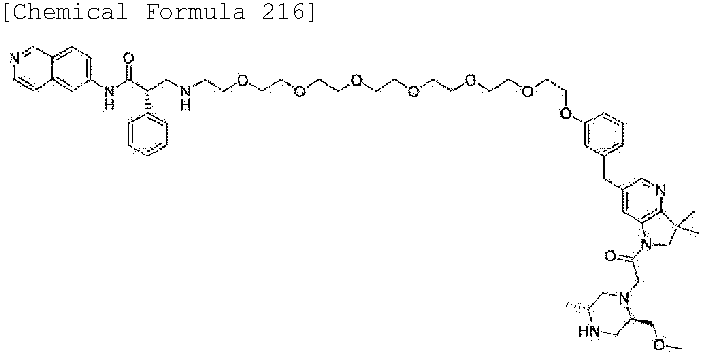

MS (m/z):1020.87 [M+H]⁺; retention time/analysis method: 9.73 min/C

### Example 63

### (S)-N-(Isoquinolin-6-yl)-1-(3-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)-26-phenyl-3,6,9,12,15,18,21-heptaoxa-24-azaheptacosan-27-amide

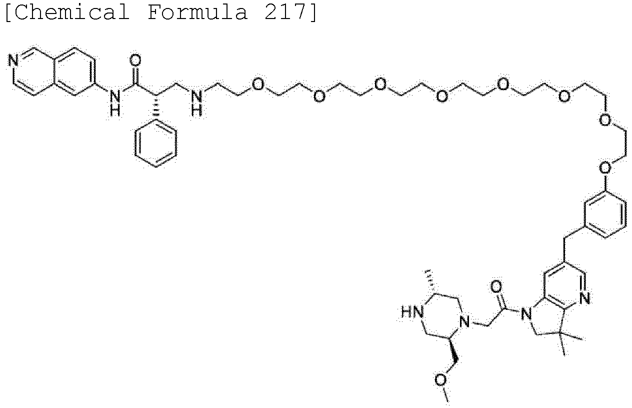

MS (m/z):1064.93 [M+H]⁺; retention time/analysis method: 9.68 min/C

### Example 64

### 4-((R)-1-((2-(2-(2-((1-(2-((2R,5R)-2-(Methoxymethyl)-5-methylpiperazin-1-yl)acetyl)-3,3-dimethyl-2,3-dihydro-1H-pyrrolo[3,2-b]pyridin-6-yl)methyl)phenoxy)ethoxy)ethyl) amino)ethyl)-N-(pyridin-4-yl)benzamide

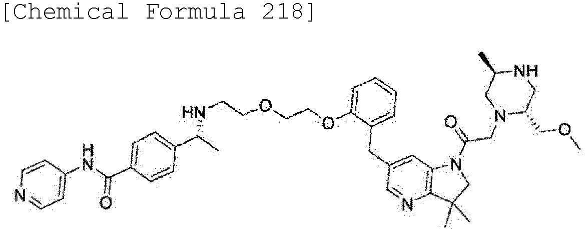

MS (m/z):750.55 [M+H]⁺; retention time/analysis method: 1.85 min/J

### Example 65

### 4-((R)-1-((2-(2-(2-(2-((1-(2-((2R,5R)-2-(Methoxymethyl)-5-methylpiperazin-1-yl)acetyl)-3,3-dimethyl-2,3-dihydro-1H-pyrrolo[3,2-b]pyridin-6-yl)methyl)phenoxy)ethoxy)ethoxy)ethyl)amino)ethyl)-N-(pyridin-4-yl)benzamide

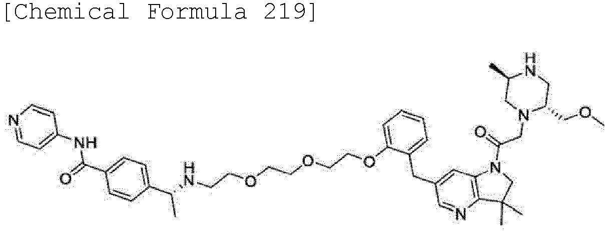

MS (m/z):792.56 [M+H]⁺; retention time/analysis method: 10.48 min/E

### Example 66

### 4-((R)-14-(2-((1-(2-((2R,5R)-2-(Methoxymethyl)-5-methylpiperazin-1-yl)acetyl)-3,3-dimethyl-2,3-dihydro-1H-pyrrolo[3,2-b]pyridin-6-yl)methyl)phenoxy)-6,9,12-trioxa-3-azatetradecan-2-yl)-N-(pyridin-4-yl)benzamide

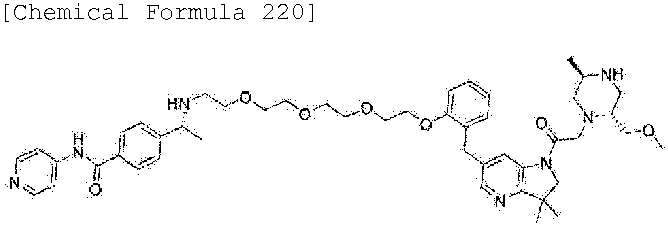

MS (m/z):838.59 [M+H]⁺; retention time/analysis method: 9.03 min/C

### Example 67

### 4-((R)-17-(2-((1-(2-((2R,5R)-2-(Methoxymethyl)-5-methylpiperazin-1-yl)acetyl)-3,3-dimethyl-2,3-dihydro-1H-pyrrolo[3,2-b]pyridin-6-yl)methyl)phenoxy)-6,9,12,15-tetraoxa-3-azaheptadecan-2-yl)-N-(pyridin-4-yl)benzamide

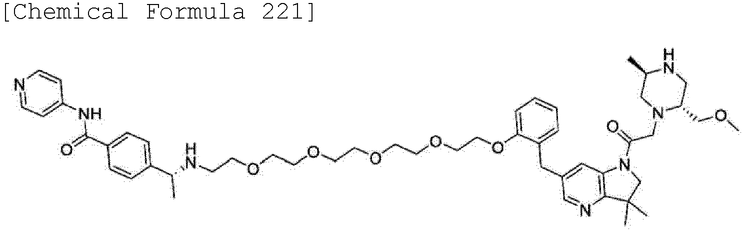

MS (m/z):882.61 [M+H]⁺; retention time/analysis method: 8.97 min/C

### Example 68

### 4-((R)-20-(2-((1-(2-((2R,5R)-2-(Methoxymethyl)-5-methylpiperazin-1-yl)acetyl)-3,3-dimethyl-2,3-dihydro-1H-pyrrolo[3,2-b]pyridin-6-yl)methyl)phenoxy)-6,9,12,15,18-pentaoxa-3-azaicosan-2-yl)-N-(pyridin-4-yl)benzamide

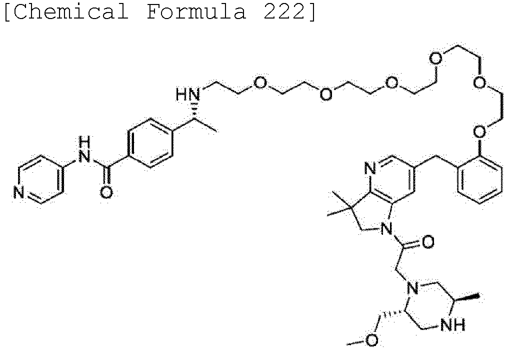

MS (m/z) :926.62 [M+H]⁺; retention time/analysis method: 8.97 min/C

### Example 69

### 4-((R)-23-(2-((1-(2-((2R,5R)-2-(Methoxymethyl)-5-methylpiperazin-1-yl)acetyl)-3,3-dimethyl-2,3-dihydro-1H-pyrrolo[3,2-b]pyridin-6-yl)methyl)phenoxy)-6,9,12,15,18,21-hexaoxa-3-azatricosan-2-yl)-N-(pyridin-4-yl)benzamide

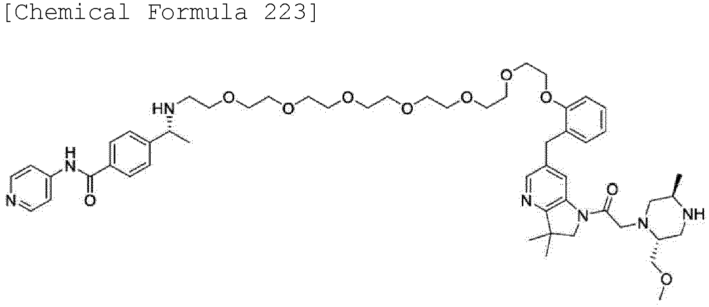

MS (m/z):970.72 [M+H]⁺; retention time/analysis method: 8.95 min/C

### Example 70

### 4-((R)-26-(2-((1-(2-((2R,5R)-2-(Methoxymethyl)-5-methylpiperazin-1-yl)acetyl)-3,3-dimethyl-2,3-dihydro-1H-pyrrolo[3,2-b]pyridin-6-yl)methyl)phenoxy)-6,9,12,15,18,21,24-heptaoxa-3-azahexacosan-2-yl)-N-(pyridin-4-yl)benzamide

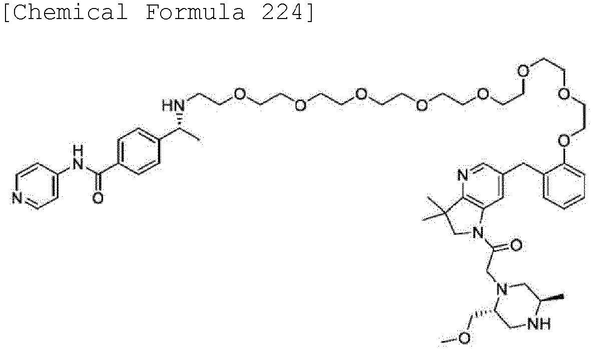

MS (m/z):1014.78 [M+H]⁺; retention time/analysis method: 8.87 min/C

### Example 71

### (R)-3-(3-Fluorophenyl)-2-((2-(2-(2-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)ethoxy)ethyl)amino)-N-(4-(pyridin-4-yl)phenyl)propanamide

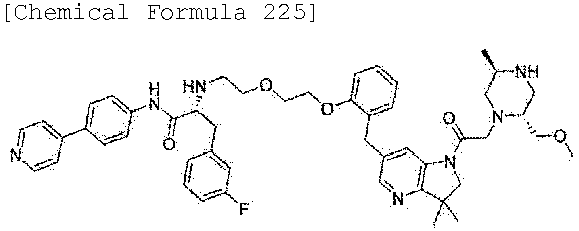

MS (m/z):844.55 [M+H]⁺; retention time/analysis method: 5.23 min/K

### Example 72

### (R)-3-(3-Fluorophenyl)-2-((2-(2-(2-(2-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)ethoxy)ethoxy)ethyl)amino)-N-(4-(pyridin-4-yl)phenyl)propanamide

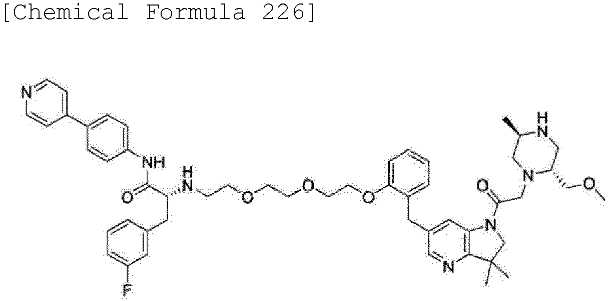

MS (m/z):888.60 [M+H]⁺; retention time/analysis method: 10.32 min/C

### Example 74

### (R)-2-(3-Fluorobenzyl)-17-(2-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)-N-(4-(pyridin-4-yl)phenyl)-6,9,12,15-tetraoxa-3-azaheptadecanamide

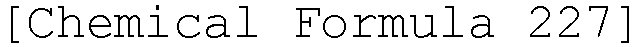

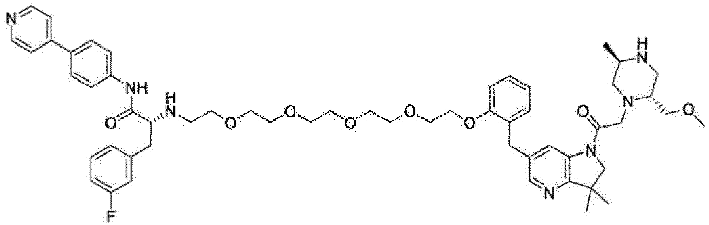

MS (m/z):976.60 [M+H]⁺; retention time/analysis method: 5.11 min/K

### Example 75

### (R)-2-(3-Fluorobenzyl)-20-(2-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)-N-(4-(pyridin-4-yl)phenyl)-6,9,12,15,18-pentaoxa-3-azaicosanamide

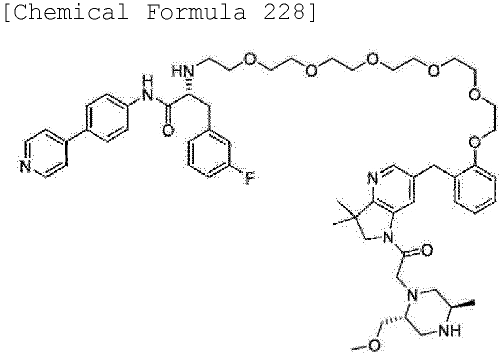

MS (m/z):1020.62 [M+H]⁺; retention time/analysis method: 5.12 min/L

### Example 76

### (R)-2-(3-Fluorobenzyl)-23-(2-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)-N-(4-(pyridin-4-yl)phenyl)-6,9,12,15,18,21-hexaoxa-3-azatricosanamide

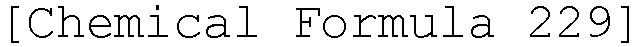

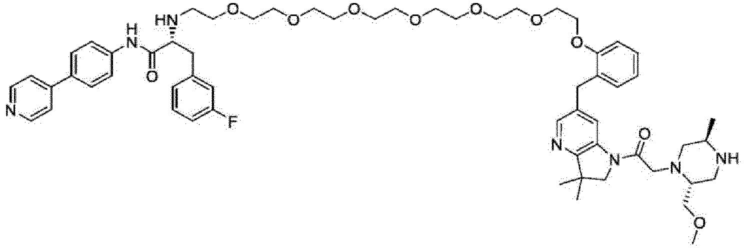

MS (m/z):1064.76 [M+H]⁺; retention time/analysis method: 9.72 min/I

### Example 77

### (R)-2-(3-Fluorobenzyl)-26-(2-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)-N-(4-(pyridin-4-yl)phenyl)-6,9,12,15,18,21,24-heptaoxa-3-azahexacosanamide

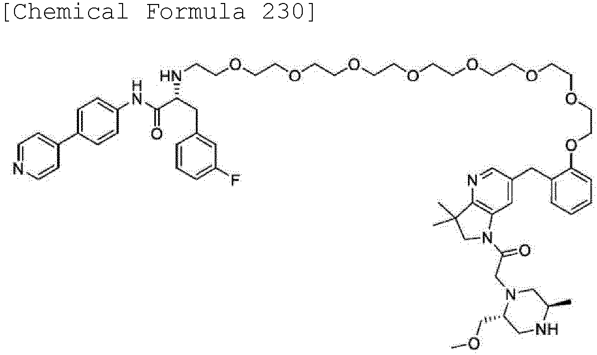

MS (m/z):1108.84 [M+H]⁺; retention time/analysis method: 5.13 min/L

### Example 78

### (S)-N-(Isoquinolin-6-yl)-3-((2-(2-(2-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)ethoxy)ethyl)amino)-2-phenylpropanamide

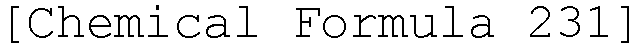

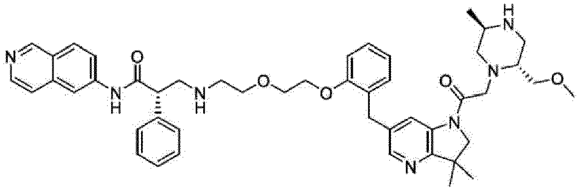

MS (m/z):800.62 [M+H]⁺; retention time/analysis method: 2.72 min/H

### Example 79

### (S)-N-(Isoquinolin-6-yl)-3-((2-(2-(2-(2-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl) phenoxy)ethoxy)ethoxy)ethyl)amino)-2-phenylpropanamide

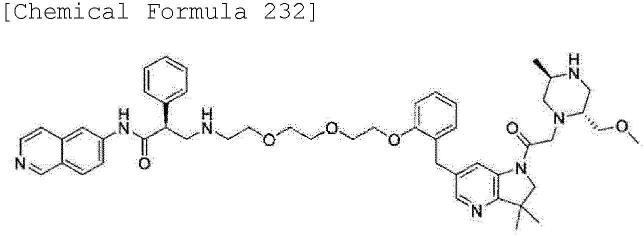

MS (m/z):844.60 [M+H]⁺; retention time/analysis method: 9.80 min/C

### Example 81

### (S)-N-(Isoquinolin-6-yl)-1-(2-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)-17-phenyl-3,6,9,12-tetraoxa-15-azaoctadecan-18-amide

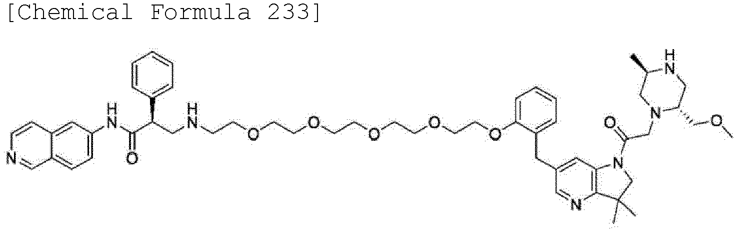

MS (m/z) :932.75 [M+H]⁺; retention time/analysis method: 8.85 min/I

### Example 82

### (S)-N-(Isoquinolin-6-yl)-1-(2-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)-20-phenyl-3,6,9,12,15-pentaoxa-18-azahenicosan-21-amide

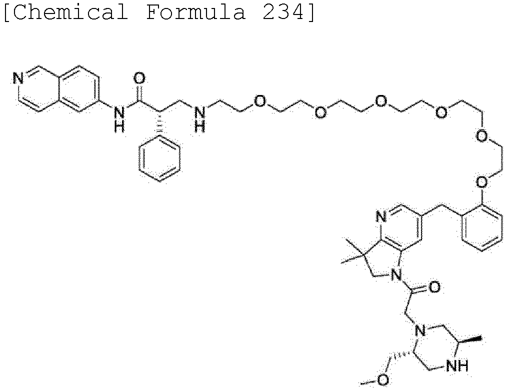

MS (m/z):976.60 [M+H]⁺; retention time/analysis method: 9.72 min/C

### Example 83

### (S)-N-(Isoquinolin-6-yl)-1-(2-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)-23-phenyl-3,6,9,12,15,18-hexaoxa-21-azatetracosan-24-amide

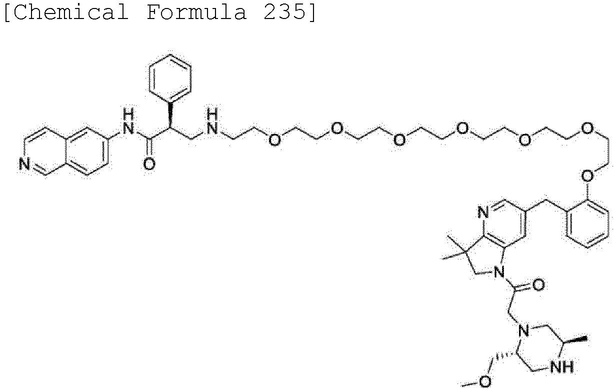

MS (m/z):1020.62 [M+H]⁺; retention time/analysis method: 9.70 min/C

### Example 84

### (S)-N-(Isoquinolin-6-yl)-1-(2-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)-26-phenyl-3,6,9,12,15,18,21-heptaoxa-24-azaheptacosan-27-amide

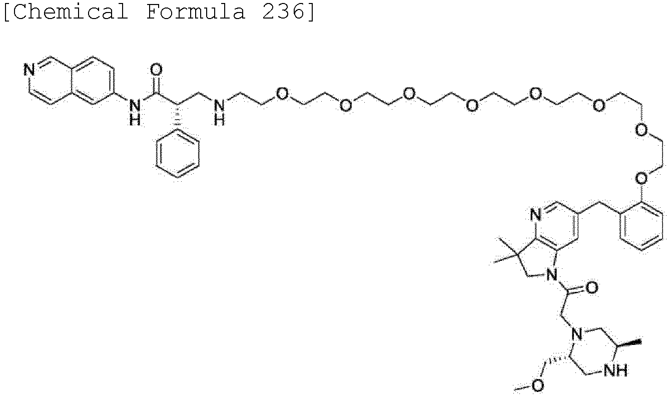

MS (m/z):1064.72 [M+H]⁺; retention time/analysis method: 8.67 min/I

### Example 85

### 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(2-(2-((((2R,5R)-1-(2-(6-(4-fluorobenzyl)-3,3-dimethyl-2,3-dihydro-1H-pyrrolo[3,2-b]pyridin-1-yl)-2-oxoethyl)-5-methylpiperazin-2-yl)methyl) (methyl)amino)ethoxy)ethyl)acetamide

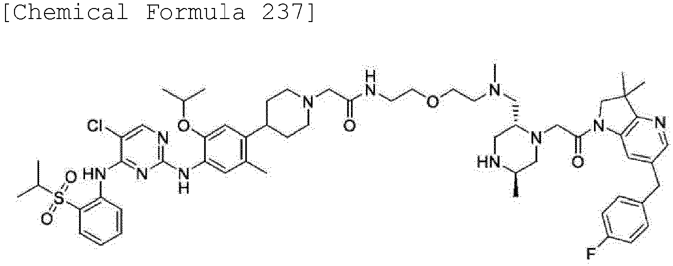

### (Step A) N-(2-(2-Azidoethoxy)ethyl)-N-methyl-2-nitrobenzenesulfonamide

To a DMF solution (30 mL) of 2-(2-((N-methyl-2-nitrophenyl) sulfonamido) ethoxy) ethyl methanesulfonate (the compound of Example 1, Step D, 3.0 g, 7.9 mmol) was added sodium azide (1.0 g, 16 mmol), and the mixture was stirred at 60°C for 16 hr. The reaction mixture was diluted with a methanol/DCM mixed solvent (10:90), and washed with water. The organic layer was dried over anhydrous sodium sulfate, and the solvent was evaporated under reduced pressure to give the title compound (2.1 g).
¹H NMR (400 MHz, CDCl₃) δ 3.02 (s, 3H), 3.35 (t, J = 4.8 Hz, 2H), 3.48 (t, J = 5.2 Hz, 2H), 3.62 (t, J = 4.8 Hz, 2H), 3.69 (t, J = 5.2 Hz, 2H), 7.62-7.64 (m, 1H), 7.68-7.71 (m, 2H), 8.01-8.03 (m, 1H); MS (m/z): 330.18 [M+H]⁺.

### (Step B) N-(2-(2-Aminoethoxy)ethyl)-N-methyl-2-nitrobenzenesulfonamide

To a THF/water mixed solution (18 mL:2.0 mL) of N-(2-(2-azidoethoxy)ethyl)-N-methyl-2-nitrobenzenesulfonamide (2.1 g, 6.4 mmol) was added triphenylphosphine (2.5 g, 9.6 mmol), and the mixture was stirred at 60°C for 4 hr. The reaction mixture was concentrated under reduced pressure, and the obtained residue was purified by column chromatography (neutral alumina, DCM/methanol) to give the title compound (1.6 g).
¹H NMR (400 MHz, DMSO-d₆) δ 1.44 (brs, 2H), 2.59 (t, J = 6.0 Hz, 2H), 2.89 (d, J = 6.8 Hz, 3H), 3.32 (t, J = 5.6 Hz, 2H), 3.38 (t, J = 5.6 Hz, 2H), 3.52-3.56 (m, 2H), 7.82-7.91 (m, 2H), 7.97-8.02 (m, 2H); MS (m/z): 304.27 [M+H]⁺.

### (Step C) 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(2-(2-((N-methyl-2-nitrophenyl)sulfonamido)ethoxy)ethyl)acetamide

To a DMF solution (6.0 mL) of 2-(4-(4-((5-chloro-4-((2-(isopropylsulfonyl)phenyl)amino)pyrimidin-2-yl)amino)-5-isopropoxy-2-methylphenyl)piperidin-1-yl)acetic acid (the compound of Reference Example 8, 0.40 g, 0.65 mmol) were added DIPEA (0.17 mL, 0.98 mmol) and HATU (0.29 g, 0.78 mmol), and then, N-(2-(2-aminoethoxy)ethyl)-N-methyl-2-nitrobenzenesulfonamide (0.24 g, 0.78 mmol) was added, and the mixture was stirred at room temperature for 16 hr. The reaction mixture was diluted with water, and subjected to extraction with a methanol/DCM mixed solvent (10:90). The organic layer was dried over anhydrous sodium sulfate, and the solvent was evaporated under reduced pressure. The obtained crude product was purified by column chromatography (silica gel, DCM/methanol) to give the title compound (0.70 g).
MS (m/z): 902.22 [M+H]⁺.

### (Step D) 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(2-(2-(methylamino) ethoxy) ethyl) acetamide

To a DMF solution (7.0 mL) of 2-(4-(4-((5-chloro-4-((2-(isopropylsulfonyl)phenyl)amino)pyrimidin-2-yl)amino)-5-isopropoxy-2-methylphenyl)piperidin-1-yl)-N-(2-(2-((N-methyl-2-nitrophenyl)sulfonamido)ethoxy)ethyl)acetamide (0.70 g, 0.77 mmol) were added potassium carbonate (0.16 g, 1.2 mmol) and thiophenol (0.12 mL, 1.7 mmol) at room temperature, and the mixture was stirred for 2 hr. To the reaction mixture was added ice water, and the mixture was subjected to extraction with a methanol/DCM mixed solvent (10:90). The organic layer was washed with 2M hydrochloric acid, and the obtained aqueous layer was alkalified with 10% aqueous sodium hydroxide solution, and subjected to extraction with a methanol/DCM mixed solvent (10:90). The organic layer was dried over anhydrous sodium sulfate, and the solvent was evaporated under reduced pressure to give the title compound (0.45 g).
MS (m/z): 717.06 [M+H]⁺.

### (Step E) tert-Butyl (2R,5S)-5-(((2-(2-(2-(4-(4-((5-chloro-4-((2-(isopropylsulfonyl)phenyl)amino)pyrimidin-2-yl)amino)-5-isopropoxy-2-methylphenyl)piperidin-1-yl)acetamido)ethoxy)ethyl)(methyl)amino)methyl)-4-(2-(6-(4-fluorobenzyl)-3,3-dimethyl-2,3-dihydro-1H-pyrrolo[3,2-b]pyridin-1-yl)-2-oxoethyl)-2-methylpiperazine-1-carboxylate

To an acetonitrile solution (1.0 mL) of tert-butyl (2R,5R)-5-(chloromethyl)-4-(2-(6-(4-fluorobenzyl)-3,3-dimethyl-2,3-dihydro-1H-pyrrolo[3,2-b]pyridin-1-yl)-2-oxoethyl)-2-methylpiperazine-1-carboxylate (the compound of Example 1, Step J, 0.10 g, 0.18 mmol) were added 2-(4-(4-((5-chloro-4-((2-(isopropylsulfonyl)phenyl)amino)pyrimidin-2-yl)amino)-5-isopropoxy-2-methylphenyl)piperidin-1-yl)-N-(2-(2-(methylamino)ethoxy)ethyl)acetamide (0.19 g, 0.27 mmol), potassium carbonate (0.080 g, 0.55 mmol) and potassium iodide (catalytic amount) at room temperature. The reaction mixture was stirred at 70°C for 18 hr, diluted with water, and subjected to extraction with a methanol/DCM mixed solvent (10:90). The organic layer was dried over anhydrous sodium sulfate, and the solvent was evaporated under reduced pressure. The obtained crude product was purified by HPLC preparative chromatography (column: HICHROME, eluent: 0.1% aqueous TFA solution/acetonitrile, gradient mode) to give the title compound (0.12 g).
MS (m/z): 1225.94 [M+H]⁺.

### (Step F) 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(2-(2-((((2R,5R)-1-(2-(6-(4-fluorobenzyl)-3,3-dimethyl-2,3-dihydro-1H-pyrrolo[3,2-b]pyridin-1-yl)-2-oxoethyl)-5-methylpiperazin-2-yl)methyl) (methyl)amino)ethoxy)ethyl)acetamide

To a DCM solution (1.0 mL) of tert-butyl (2R,5S)-5-(((2-(2-(2-(4-(4-((5-chloro-4-((2-(isopropylsulfonyl)phenyl)amino)pyrimidin-2-yl)amino)-5-isopropoxy-2-methylphenyl)piperidin-1-yl)acetamido)ethoxy)ethyl)(methyl)amino)methyl)-4-(2-(6-(4-fluorobenzyl)-3,3-dimethyl-2,3-dihydro-1H-pyrrolo[3,2-b]pyridin-1-yl)-2-oxoethyl)-2-methylpiperazine-1-carboxylate (0.12 g, 0.050 mmol) was added TFA (1.0 mL) at 0°C, and the mixture was stirred at room temperature for 4 hr. The reaction mixture was concentrated under reduced pressure, and diluted with methanol (2.5 mL), and the obtained solution was filtered through SCX. Then, this SCX was washed with 7M ammonia/methanol solution (3.0 mL), and the eluate was concentrated under reduced pressure to give the title compound (0.078 g).
MS (m/z):1124.64 [M(³⁵Cl)+H]⁺,1126.65 [M(³⁷Cl)+H]⁺; retention time/analysis method: 4.38 min/B.

### Example 91

### 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(2-(2-(((6-(4-fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)methyl) (methyl)amino)ethoxy)ethyl)acetamide

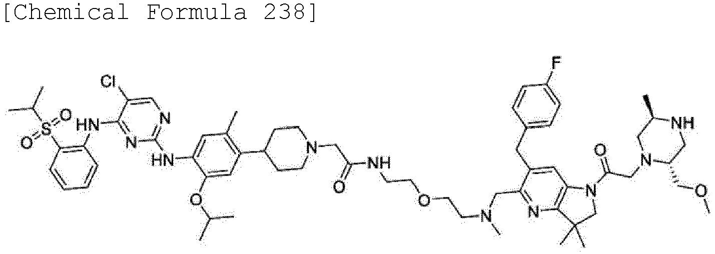

### (Step A) tert-Butyl (2R,5R)-4-(2-(5-(((2-(2-(2-(4-(4-((5-chloro-4-((2-(isopropylsulfonyl)phenyl)amino)pyrimidin-2-yl)amino)-5-isopropoxy-2-methylphenyl)piperidin-1-yl)acetamido)ethoxy)ethyl)(methyl)amino)methyl)-6-(4-fluorobenzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate

To an acetonitrile solution (1.0 mL) of tert-butyl (2R,5R)-4-(2-(5-(chloromethyl)-6-(4-fluorobenzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate (the compound of Example 22, Step E, 0.10 g, 0.17 mmol) were added 2-(4-(4-((5-chloro-4-((2-(isopropylsulfonyl)phenyl)amino)pyrimidin-2-yl)amino)-5-isopropoxy-2-methylphenyl)piperidin-1-yl)-N-(2-(2-(methylamino)ethoxy)ethyl)acetamide (the compound of Example 85, Step D, 0.18 g, 0.26 mmol), potassium carbonate (0.070 g, 0.51 mmol) and potassium iodide (0.010 g) at room temperature. The reaction mixture was stirred at 70°C for 18 hr, diluted with water, and subjected to extraction with a methanol/DCM mixed solvent (10:90). The organic layer was dried over anhydrous sodium sulfate, and the solvent was evaporated under reduced pressure. The obtained crude product was purified by HPLC preparative chromatography (column: X-Bridge, eluent: 10 mM ammonium hydrogencarbonate aqueous solution/acetonitrile, gradient mode) to give the title compound (0.12 g).
MS (m/z): 1267.88 [M-H]⁻.

### (Step B) 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(2-(2-(((6-(4-fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)methyl) (methyl)amino)ethoxy)ethyl)acetamide

To a DCM solution (5.0 mL) of tert-butyl (2R,5R)-4-(2-(5-(((2-(2-(2-(4-(4-((5-chloro-4-((2-(isopropylsulfonyl)phenyl)amino)pyrimidin-2-yl)amino)-5-isopropoxy-2-methylphenyl)piperidin-1-yl)acetamido)ethoxy)ethyl)(methyl)amino)methyl)-6-(4-fluorobenzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate (0.12 g, 0.090 mmol) was added TFA (1.0 mL), and the mixture was stirred at room temperature for 4 hr. The reaction mixture was concentrated under reduced pressure, the residue was diluted with methanol (2.0 mL), and the obtained solution was filtered through SCX. Then, this SCX was washed with 7M ammonia/methanol solution (3.0 mL), and the eluate was concentrated under reduced pressure to give the title compound (0.080 g).
MS (m/z):1168.76 [M(³⁵Cl)+H]⁺,1171.75 [M(³⁷Cl)+H]⁺; retention time/analysis method: 4.07 min/B

### Example 97

### 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(2-(2-(2-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy) ethoxy) ethyl) acetamide

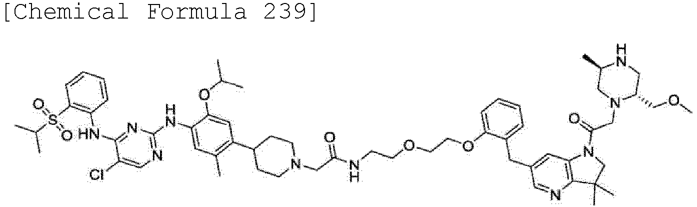

### (Step A) 2-(2-Azidoethoxy)ethan-1-ol

To a DMF solution (50 mL) of 2-(2-hydroxyethoxy)ethyl 4-methylbenzenesulfonate (the compound of Example 1, Step B, 5.0 g, 19 mmol) was added sodium azide (2.5 g, 39 mmol), and the mixture was stirred at 60°C for 16 hr. To the reaction mixture was added water, and the mixture was subjected to extraction with ethyl acetate. The organic layer was dried over anhydrous sodium sulfate, and the solvent was evaporated under reduced pressure to give the title compound (1.8 g).
¹H NMR (400 MHz, CDCl₃) δ 3.39 (t, J = 5.2 Hz, 2H), 3.60-3.63 (m, 4H), 3.73 (t, J = 4.0 Hz, 2H), one proton missing; MS (m/z): 133.01 [M+H]⁺.

### (Step B) 2-(2-Aminoethoxy)ethan-1-ol

To a methanol solution (30 mL) of 2-(2-azidoethoxy)ethan-1-ol (1.8 g, 14 mmol) was added palladium on carbon (catalytic amount), and the mixture was stirred under hydrogen atmosphere at room temperature for 18 hr. The reaction mixture was filtered through Celite, and then the Celite washed with methanol. The filtrate was concentrated under reduced pressure to give the title compound (1.0 g).
¹H NMR (400 MHz, CDCl₃) δ 2.09 (brs, 2H), 2.84-2.96 (m, 2H), 3.56 (t, J = 5.2 Hz, 2H), 3.59-3.64 (m, 2H), 3.70-3.76 (m, 2H), one proton missing; MS (m/z): 106.20 [M+H]⁺.

### (Step C) 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(2-(2-hydroxyethoxy) ethyl) acetamide

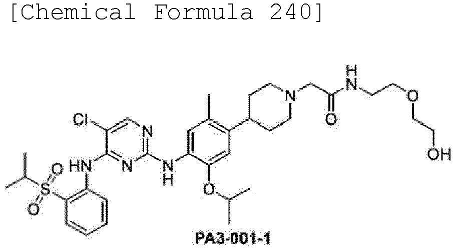

To a DMF solution (5.0 mL) of 2-(4-(4-((5-chloro-4-((2-(isopropylsulfonyl)phenyl)amino)pyrimidin-2-yl)amino)-5-isopropoxy-2-methylphenyl)piperidin-1-yl)acetic acid (the compound of Reference Example 8, 0.50 g, 0.81 mmol) were added DIPEA (0.20 mL, 1.2 mmol), HATU (0.37 g, 0.98 mmol) and 2-(2-aminoethoxy)ethan-1-ol (0.10 g, 0.98 mmol). The reaction mixture was stirred at room temperature for 16 hr, water was added, and the mixture was subjected to extraction with a methanol/DCM mixed solvent (10:90). The organic layer was dried over anhydrous sodium sulfate, and the solvent was evaporated under reduced pressure. The obtained crude product was purified by column chromatography (silica gel, DCM/methanol) to give the title compound (0.60 g).
MS (m/z): 704.04 [M+H]⁺.

### (Step D) 2-(2-(2-(4-(4-((5-Chloro-4-((2-(isopropylsulfonyl)phenyl)amino)pyrimidin-2-yl)amino)-5-isopropoxy-2-methylphenyl)piperidin-1-yl)acetamido)ethoxy)ethyl methanesulfonate

To a DCM solution (5.0 mL) of 2-(4-(4-((5-chloro-4-((2-(isopropylsulfonyl)phenyl)amino)pyrimidin-2-yl)amino)-5-isopropoxy-2-methylphenyl)piperidin-1-yl)-N-(2-(2-hydroxyethoxy)ethyl)acetamide (0.20 g, 0.28 mmol) were added TEA (0.072 g, 0.71 mmol) and methanesulfonyl chloride (0.050 g, 0.43 mmol) under ice-cooled, and the mixture was stirred at room temperature for 2 hr. The reaction mixture was diluted with DCM and water. The organic layer was washed with saturated aqueous sodium hydrogencarbonate solution, and dried over anhydrous sodium sulfate, and the solvent was evaporated under reduced pressure to give the title compound (0.25 g).
MS (m/z): 782.11 [M+H]⁺.

### (Step E) tert-Butyl (2R,5R)-4-(2-(6-(2-(2-(2-(2-(4-(4-((5-chloro-4-((2-(isopropylsulfonyl)phenyl)amino)pyrimidin-2-yl)amino)-5-isopropoxy-2-methylphenyl)piperidin-1-yl)acetamido)ethoxy)ethoxy)benzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate

To a DMF solution (2.0 mL) of tert-butyl (2R,5R)-4-(2-(6-(2-hydroxybenzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate (the compound of Example 73, Step A, 0.10 g, 0.19 mmol) were added 2-(2-(2-(4-(4-((5-chloro-4-((2-(isopropylsulfonyl)phenyl)amino)pyrimidin-2-yl)amino)-5-isopropoxy-2-methylphenyl)piperidin-1-yl)acetamido)ethoxy)ethyl methanesulfonate (0.22 g, 0.28 mmol) and cesium carbonate (0.18 g, 0.56 mmol), and the mixture was stirred at 70°C for 16 hr. The reaction mixture was diluted with water, and subjected to extraction with a methanol/DCM mixed solvent (10:90). The organic layer was dried over anhydrous sodium sulfate, and the solvent was evaporated under reduced pressure. The obtained crude product was purified by HPLC preparative chromatography (column: HICHROME, eluent: 10 mM ammonium hydrogencarbonate aqueous solution/acetonitrile, gradient mode) to give the title compound (0.030 g).
MS (m/z): 1224.05 [M+H]⁺.

### (Step F) 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(2-(2-(2-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy) ethoxy) ethyl) acetamide

To a DCM solution (1.0 mL) of tert-butyl (2R,5R)-4-(2-(6-(2-(2-(2-(2-(4-(4-((5-chloro-4-((2-(isopropylsulfonyl)phenyl)amino)pyrimidin-2-yl)amino)-5-isopropoxy-2-methylphenyl)piperidin-1-yl)acetamido)ethoxy)ethoxy)benzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate (0.030 g, 0.025 mmol) was added TFA (0.30 mL) under ice-cooled, and the mixture was stirred at room temperature for 4 hr. The reaction mixture was concentrated under reduced pressure, and diluted with methanol (2.0 mL), and the obtained solution was filtered through SCX. Then, this SCX was washed with 7M ammonia/methanol solution (2.0 mL), and the eluate was concentrated under reduced pressure to give the title compound (0.022 g).
MS (m/z):1123.76 [M(³⁵Cl)+H]⁺,1125.74 [M(³⁷Cl)+H]⁺; retention time/analysis method: 4.40 min/B

The example compounds produced according to the above-mentioned production method or the method shown in Example 85, 91 or 97, or a method similar thereto, are described below. For each compound, the compound name, structural formula, MS (m/z) (actual measured value), retention time/analysis method (unit: minutes), and the analytical method are shown.

### Example 86

### 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(2-(2-(2-((((2R,5R)-1-(2-(6-(4-fluorobenzyl)-3,3-dimethyl-2,3-dihydro-1H-pyrrolo[3,2-b]pyridin-1-yl)-2-oxoethyl)-5-methylpiperazin-2-yl)methyl) (methyl) amino) ethoxy) ethoxy) ethyl) acetamide

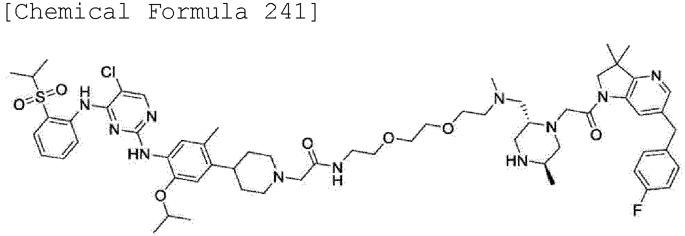

MS (m/z) :1168.81 [M(³⁵Cl)+H]⁺,1170.78 [M(³Cl)+H]⁺; retention time/analysis method: 4.36 min/B

### Example 87

### 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(1-((2R,5R)-1-(2-(6-(4-fluorobenzyl)-3,3-dimethyl-2,3-dihydro-1H-pyrrolo[3,2-b]pyridin-1-yl)-2-oxoethyl)-5-methylpiperazin-2-yl)-2-methyl-5, 8, 11-trioxa-2-azatridecan-13-yl) acetamide

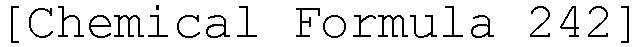

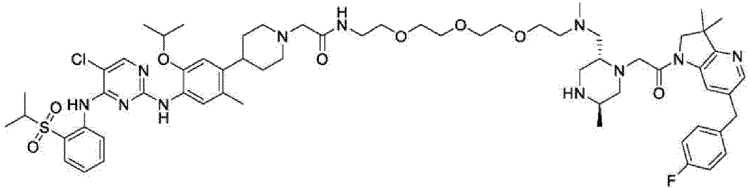

MS (m/z):1212.87 [M(³⁵Cl)+H]⁺,1214.85 [M(³⁷Cl)+H]⁺; retention time/analysis method: 4.07 min/B

### Example 88

### 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(1-((2R,5R)-1-(2-(6-(4-fluorobenzyl)-3,3-dimethyl-2,3-dihydro-1H-pyrrolo[3,2-b]pyridin-1-yl)-2-oxoethyl)-5-methylpiperazin-2-yl)-2-methyl-5,8,11,14-tetraoxa-2-azahexadecan-16-yl)acetamide

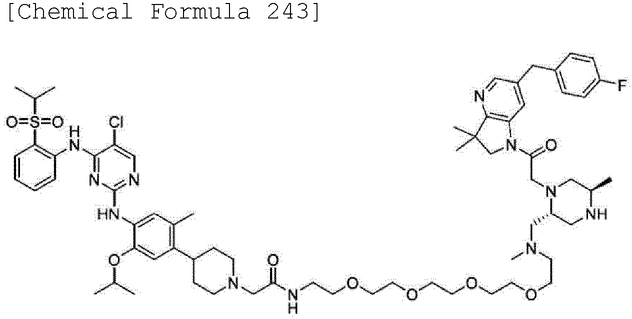

MS (m/z) :1256.93 [M(³⁵Cl)+H]⁺,1258.19 [M(³⁷Cl)+H]⁺; retention time/analysis method: 4.38 min/B

### Example 89

### 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(1-((2R,5R)-1-(2-(6-(4-fluorobenzyl)-3,3-dimethyl-2,3-dihydro-1H-pyrrolo[3,2-b]pyridin-1-yl)-2-oxoethyl)-5-methylpiperazin-2-yl)-2-methyl-5,8,11,14,17-pentaoxa-2-azanonadecan-19-yl)acetamide

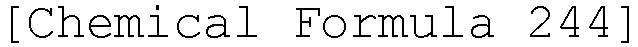

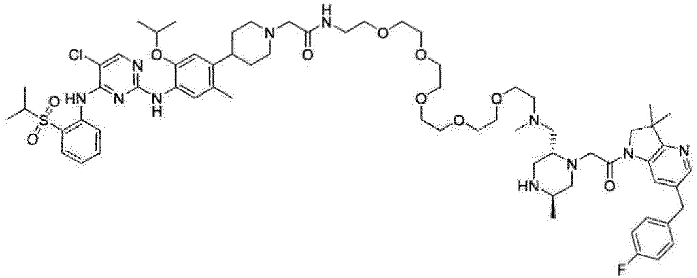

MS (m/z):1300.85 [M(³⁵Cl)+H]⁺,1302.86 [M(³⁷Cl)+H]⁺; retention time/analysis method: 4.35 min/B

### Example 90

### 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(1-((2R,5R)-1-(2-(6-(4-fluorobenzyl)-3,3-dimethyl-2,3-dihydro-1H-pyrrolo[3,2-b]pyridin-1-yl)-2-oxoethyl)-5-methylpiperazin-2-yl)-2-methyl-5,8,11,14,17,20-hexaoxa-2-azadocosan-22-yl)acetamide

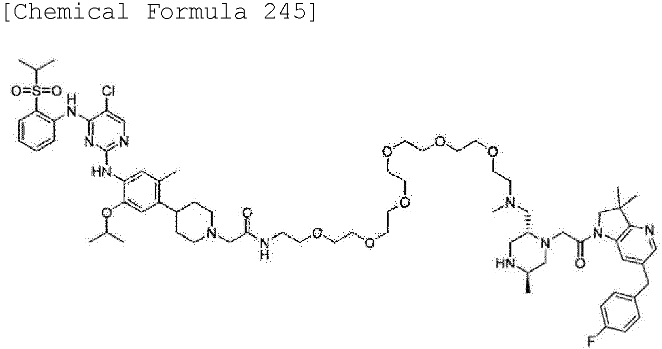

MS (m/z) :1345.08 [M(³⁵Cl)+H]⁺,1347.22 [M(³⁷Cl)+H]⁺; retention time/analysis method: 4.36 min/B

### Example 92

### 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(2-(2-(2-(((6-(4-fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)methyl) (methyl) amino) ethoxy) ethoxy) ethyl) acetamide

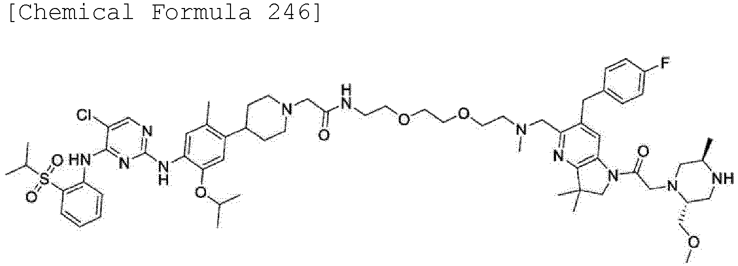

MS (m/z):1212.87 [M(³⁵Cl)+H]⁺,1214.88 [M(³⁷Cl)+H]⁺; retention time/analysis method: 2.93 min/J

### Example 93

### 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(1-(6-(4-fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)-2-methyl-5,8,11-trioxa-2-azatridecan-13-yl)acetamide

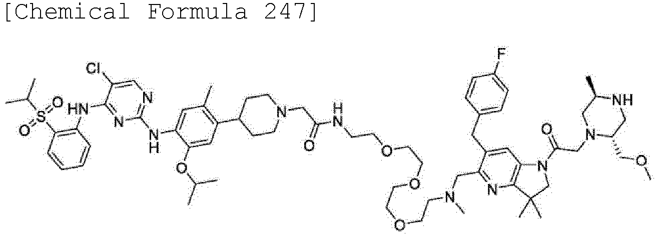

MS (m/z) :1256.96 [M(³⁵Cl)+H]⁺,1258.90 [M(³⁷Cl)+H]⁺; retention time/analysis method: 12.05 min/I

### Example 94

### 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(1-(6-(4-fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)-2-methyl-5,8,11,14-tetraoxa-2-azahexadecan-16-yl)acetamide

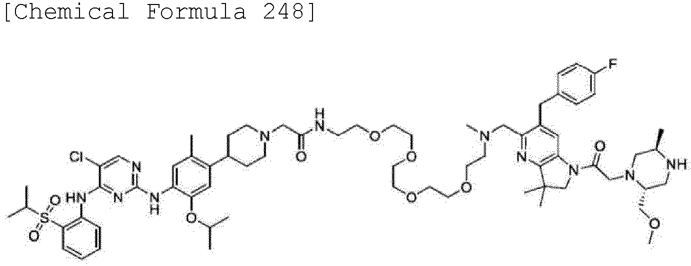

MS (m/z) :1301.01 [M(³⁵Cl)+H]⁺,1303.02 [M(³⁷Cl)+H]⁺; retention time/analysis method: 11.09 min/I

### Example 95

### 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(1-(6-(4-fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)-2-methyl-5,8,11,14,17-pentaoxa-2-azanonadecan-19-yl)acetamide

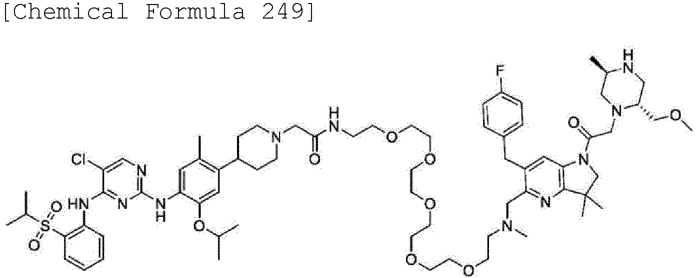

MS (m/z) :1345.16 [M(³⁵Cl)+H]⁺,1346.21 [M(³⁷Cl)+H]⁺; retention time/analysis method: 2.87 min/J

### Example 96

### 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(1-(6-(4-fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)-2-methyl-5,8,11,14,17,20-hexaoxa-2-azadocosan-22-yl) acetamide

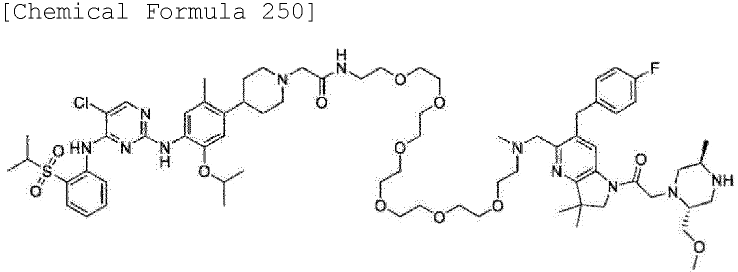

MS (m/z):1388.98 [M(³⁵Cl)+H]⁺,⁺1390.04 [M(³⁷Cl)+H]⁺; retention time/analysis method: 4.18 min/B

### Example 98

### 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(2-(2-(2-(2-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy) ethoxy) ethoxy) ethyl) acetamide

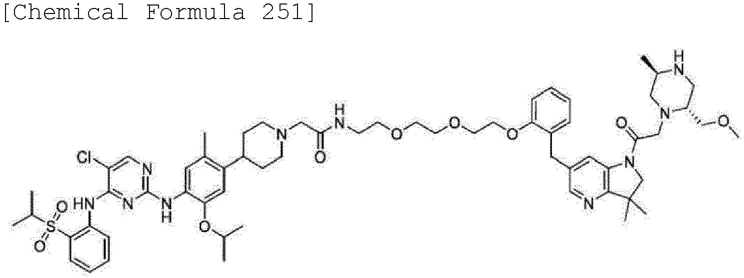

MS (m/z) :1167.84 [M(³⁵Cl)+H]⁺,1169.78 [M(³⁷Cl)+H]⁺; retention time/analysis method: 12.53 min/I

### Example 99

### 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(2-(2-(2-(2-(2-((1-(2-((2R,5R)-2-(methoxymethyl)-5-methylpiperazin-1-yl)acetyl)-3, 3-dimethyl-2,3-dihydro-1H-pyrrolo[3,2-b]pyridin-6-yl)methyl)phenoxy) ethoxy) ethoxy) ethoxy) ethyl) acetamide

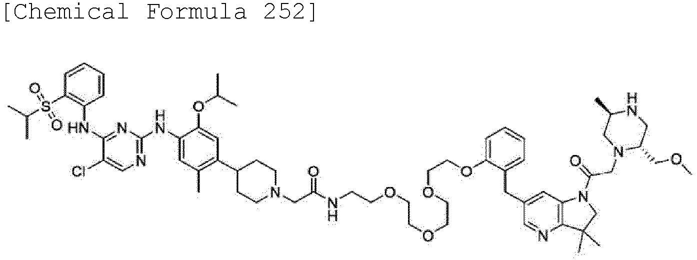

MS (m/z):1211.78[M(³⁵Cl)+H]⁺,1213.75 [M(³⁷Cl)+H]⁺; retention time/analysis method: 4.40 min/B

### Example 100

### 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(14-(2-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)-3,6,9,12-tetraoxatetradecyl)acetamide

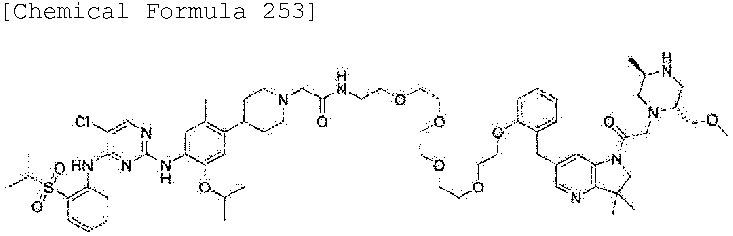

MS (m/z) :1255.75 [M(³⁵Cl)+H]⁺,1157.80 [M(³⁷Cl)+H]⁺; retention time/analysis method: 4.46 min/B

### Example 101

### 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(17-(2-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)-3,6,9,12,15-pentaoxaheptadecyl)acetamide

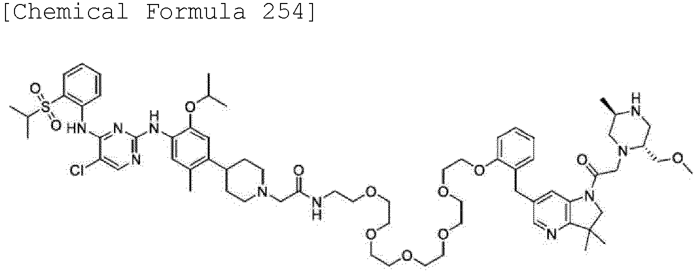

MS (m/z) :1300.17 [M(³⁵Cl)+H]⁺,1302.11 [M(³⁷Cl)+H]⁺; retention time/analysis method: 6.30 min/J

### Example 102

### 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(20-(2-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy) -3, 6, 9, 12, 15, 18-hexaoxaicosyl) acetamide

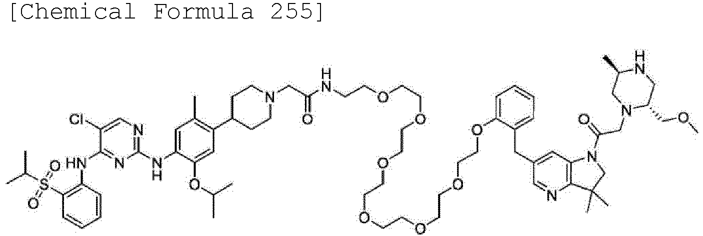

MS (m/z):1343.86 [M(³⁵Cl)+H]⁺,1345.92 [M(³⁷Cl)+H]⁺; retention time/analysis method: 4.45 min/B

### Example 103

### 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(2-(2-(3-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy) ethoxy) ethyl) acetamide

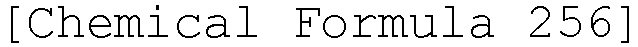

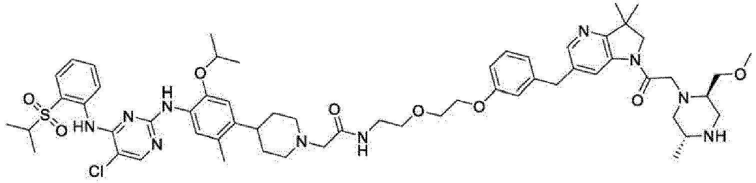

MS (m/z) :1123.44 [M(³⁵Cl)+H]⁺,1125.44 [M(³⁷Cl)+H]⁺; retention time/analysis method: 3.23 min/J

### Example 104

### 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(2-(2-(2-(3-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy) ethoxy) ethoxy) ethyl) acetamide

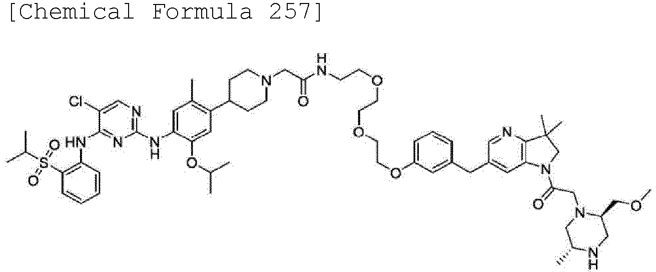

MS (m/z):1167.81 [M(³⁵Cl)+H]⁺,1169.82 [M(³⁷Cl)+H]⁺; retention time/analysis method: 4.41 min/J

### Example 105

### 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(2-(2-(2-(2-(3-((1-(2-((2R,5R)-2-(methoxymethyl)-5-methylpiperazin-1-yl)acetyl)-3, 3-dimethyl-2,3-dihydro-1H-pyrrolo[3,2-b]pyridin-6-yl)methyl)phenoxy) ethoxy) ethoxy) ethoxy) ethyl) acetamide

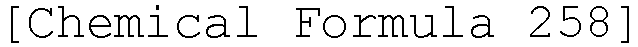

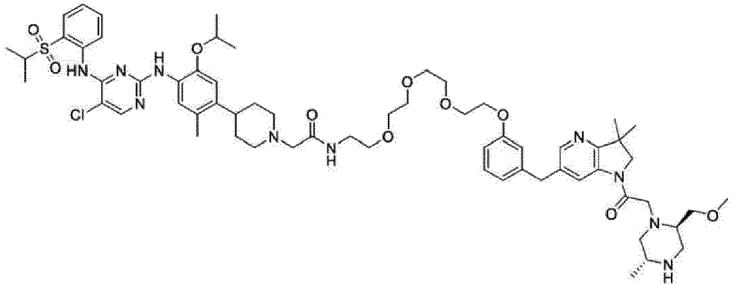

MS (m/z):1211.81 [M(³⁵Cl)+H]⁺,1213.75 [M(³⁷Cl)+H]⁺; retention time/analysis method: 4.44 min/B

### Example 106

### 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(14-(3-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)-3,6,9,12-tetraoxatetradecyl)acetamide

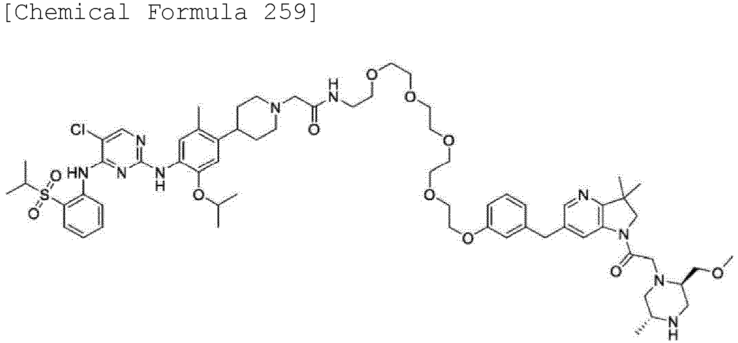

MS (m/z) :1255.90 [M(³⁵Cl)+H]⁺,1257.91 [M(³⁷Cl)+H]⁺; retention time/analysis method: 4.46 min/B

### Example 107

### 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(17-(3-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy)-3,6,9,12,15-pentaoxaheptadecyl)acetamide

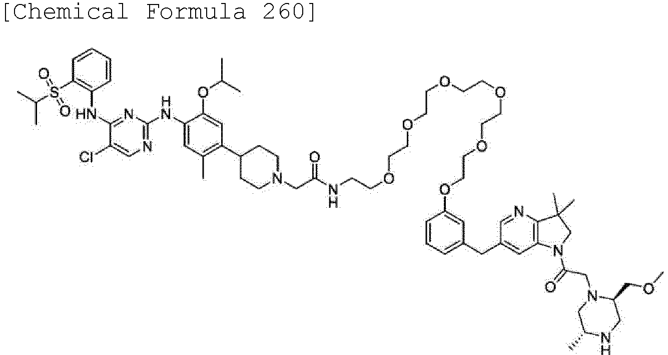

MS (m/z) :1299.82 [M(³⁵Cl)+H]⁺,1301.84 [M(³⁷Cl)+H]⁺; retention time/analysis method: 4.53 min/B

### Example 108

### 2-(4-(4-((5-Chloro-4-((2-(isopropylsulfonyl)phenyl)amino)pyrimidin-2-yl)amino)-5-isopropoxy-2-methylphenyl)piperidin-1-yl)-N-(20-(3-((1-(2-((2R,5R)-2-(methoxymethyl)-5-methylpiperazin-1-yl)acetyl)-3,3-dimethyl-2,3-dihydro-1H-pyrrolo[3,2-b]pyridin-6-yl)methyl)phenoxy) -3, 6, 9, 12, 15, 18-hexaoxaicosyl) acetamide

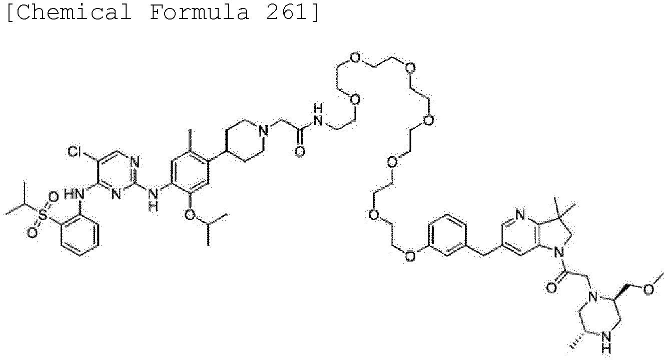

MS (m/z):1343.99[M(³⁵Cl)+H]⁺,1345.96 [M(³⁷Cl)+H]⁺; retention time/analysis method: 4.39 min/B

### Example 109

### 2-(2-((((2R,5R)-1-(2-(6-(4-Fluorobenzyl)-3,3-dimethyl-2,3-dihydro-1H-pyrrolo[3,2-b]pyridin-1-yl)-2-oxoethyl)-5-methylpiperazin-2-yl)methyl)(methyl)amino)ethoxy)-N-(4-((4-((3-(methylsulfonyl)benzyl)amino)-5-(trifluoromethyl)pyrimidin-2-yl)amino) phenyl) acetamide

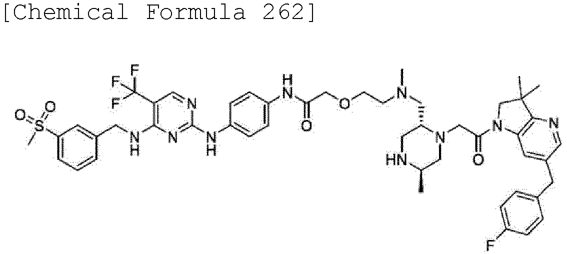

### (Step A) N-(2-Hydroxyethyl)-N-methyl-2-nitrobenzenesulfonamide

To a 1,4-dioxane solution (30 mL) of 2-(methylamino)ethanol (2.0 g, 27 mmol) was added 2-nitrobenzenesulfonyl chloride (3.0 g, 14 mmol), and the mixture was stirred at 80°C for 5 hr. To the reaction mixture was added saturated aqueous sodium hydrogencarbonate solution, and the mixture was subjected to extraction with ethyl acetate. The organic layer was dried over anhydrous sodium sulfate, and the solvent was evaporated under reduced pressure. The obtained crude product was purified by column chromatography (silica gel, petroleum ether/ethyl acetate) to give the title compound (3.0 g).
¹H NMR (400 MHz, CDCl₃) δ 2.06 (brs, 1H), 3.00 (s, 3H), 3.42 (t, J = 5.2 Hz, 2H), 3.79-3.81 (m, 2H), 7.62-7.65 (m, 1H), 7.70-7.73 (m, 2H), 8.02-8.04 (m, 1H); MS (m/z): 261.13 [M+H]⁺.

### (Step B) tert-Butyl 2-(2-((N-methyl-2-nitrophenyl)sulfonamido)ethoxy)acetate

To a DCM solution (10 mL) of N-(2-hydroxyethyl)-N-methyl-2-nitrobenzenesulfonamide (1.0 g, 3.8 mmol) were added 35% aqueous sodium hydroxide solution (10 mL), tetrabutylammonium chloride (1.1 g, 3.8 mmol) and tert-butyl 2-bromoacetate (0.90 g, 4.6 mmol) at room temperature. The reaction mixture was diluted with water, and subjected to extraction with DCM. The organic layer was dried over anhydrous sodium sulfate, and the solvent was evaporated under reduced pressure. The obtained crude product was purified by column chromatography (silica gel, petroleum ether/ethyl acetate) to give the title compound (1.0 g).
¹H NMR (400 MHz, CDCl₃) δ 1.47 (s, 9H), 3.04 (s, 3H), 3.49-3.51 (m, 2H), 3.72-3.73 (m, 2H), 3.94 (s, 2H), 7.61-7.63 (m, 1H), 7.67-7.70 (m, 2H), 8.02-8.04 (m, 1H); MS (m/z): 397.26 [M+Na]⁺.

### (Step C) 2-(2-((N-Methyl-2-nitrophenyl) sulfonamido) ethoxy) acetic acid

To a DCM solution (1.0 mL) of tert-butyl 2-(2-((N-methyl-2-nitrophenyl)sulfonamido)ethoxy)acetate (0.10 g, 0.27 mmol) was added TFA (0.50 mL), and the mixture was stirred at room temperature for 6 hr, and the reaction mixture was concentrated under reduced pressure to give the title compound (0.080 g).
¹H NMR (400 MHz, DMSO-d₆) δ 2.90 (s, 3H), 3.41 (t, J = 5.2 Hz, 2H), 3.63 (t, J = 5.4 Hz, 2H), 4.00 (s, 2H), 7.84-7.89 (m, 2H), 7.96-8.03 (m, 2H), 12.73 (brs, 1H); MS (m/z): 319.40 [M+H]⁺.

### (Step D) 2-(2-((N-Methyl-2-nitrophenyl)sulfonamido)ethoxy)-N-(4-((4-((3-(methylsulfonyl)benzyl)amino)-5-(trifluoromethyl)pyrimidin-2-yl)amino)phenyl)acetamide

To a DMF solution (2.0 mL) of 2-(2-((N-methyl-2-nitrophenyl)sulfonamido)ethoxy)acetic acid (0.10 g, 0.31 mmol) were added DIPEA (0.13 mL, 7.9 mmol) and HATU (0.13 g, 3.5 mmol) under ice-cooled, and the mixture was stirred for 15 min, and then N²-(4-aminophenyl)-N⁴-(3-(methylsulfonyl)benzyl)-5-(trifluoromethyl)pyrimidine-2,4-diamine (the compound of Reference Example 9, 0.11 g, 0.31 mmol) was added. The mixture was stirred at room temperature for 18 hr, to reaction mixture was added water, and the mixture was subjected to extraction with ethyl acetate. The organic layer was dried over anhydrous sodium sulfate, and the solvent was evaporated under reduced pressure. The obtained crude product was purified by column chromatography (silica gel, petroleum ether/ethyl acetate) to give the title compound (0.17 g).
¹H NMR (400 MHz, DMSO-d₆) δ 2.89-2.94 (m, 3H), 3.10-3.15 (m, 3H), 3.48-3.51 (m, 2H), 3.68-3.72 (m, 2H), 4.00-4.10 (m, 2H), 4.73-4.74 (m, 2H), 7.40-7.50 (m, 4H), 7.58-7.70 (m, 2H), 7.78-7.90 (m, 5H), 7.95-8.10 (m, 2H), 8.22 (brs, 1H), 9.45-9.55 (m, 2H); MS (m/z): 738.56 [M+H]⁺.

### (Step E) 2-(2-(Methylamino)ethoxy)-N-(4-((4-((3-(methylsulfonyl)benzyl)amino)-5-(trifluoromethyl)pyrimidin-2-yl)amino)phenyl)acetamide

To a DMF solution (2.0 mL) of 2-(2-((N-methyl-2-nitrophenyl)sulfonamido)ethoxy)-N-(4-((4-((3-(methylsulfonyl)benzyl)amino)-5-(trifluoromethyl)pyrimidin-2-yl)amino)phenyl)acetamide (0.17 g, 0.23 mmol) were added thiophenol (0.040 mL, 0.35 mmol) and potassium carbonate (0.048 g, 0.35 mmol), and the mixture was stirred at room temperature for 2 hr. To the reaction mixture was added 1M hydrochloric acid, and the mixture was washed with a methanol/DCM mixed solution (10:90). The aqueous layer was alkalified with 1M aqueous sodium hydroxide solution, and subjected to extraction with a methanol/DCM mixed solution (10:90). The obtained organic layer was dried over anhydrous sodium sulfate, and the solvent was evaporated under reduced pressure to give the title compound (0.12 g).
MS (m/z): 553.76 [M+H]⁺.

### (Step F) tert-Butyl (2R,5S)-4-(2-(6-(4-fluorobenzyl)-3,3-dimethyl-2,3-dihydro-1H-pyrrolo[3,2-b]pyridin-1-yl)-2-oxoethyl)-2-methyl-5-((methyl(2-(2-((4-((4-((3-(methylsulfonyl)benzyl)amino)-5-(trifluoromethyl)pyrimidin-2-yl)amino)phenyl)amino)-2-oxoethoxy)ethyl)amino)methyl)piperazine-1-carboxylate

To an acetonitrile solution (2.0 mL) of 2-(2-(methylamino)ethoxy)-N-(4-((4-((3-(methylsulfonyl)benzyl)amino)-5-(trifluoromethyl)pyrimidin-2-yl)amino)phenyl)acetamide (0.12 g, 0.22 mmol) were added tert-butyl (2R,5R)-5-(chloromethyl)-4-(2-(6-(4-fluorobenzyl)-3,3-dimethyl-2,3-dihydro-1H-pyrrolo[3,2-b]pyridin-1-yl)-2-oxoethyl)-2-methylpiperazine-1-carboxylate (the compound of Example 1, Step J, 0.12 g, 0.22 mmol), potassium carbonate (0.10 g, 0.66 mmol) and potassium iodide (catalytic amount), and the mixture was stirred at 70°C for 12 hr. The reaction mixture was diluted with a methanol/DCM mixed solution (10:90), and washed with water. The organic layer was dried over anhydrous sodium sulfate, and the solvent was evaporated under reduced pressure. The obtained crude product was purified by HPLC preparative chromatography (column: X-SELECT CSH C18, eluent: 0.1% aqueous TFA solution/acetonitrile, gradient mode) to give the title compound (0.070 g).
MS (m/z): 1061.91 [M+H] ⁺.

### (Step G) 2-(2-((((2R,5R)-1-(2-(6-(4-Fluorobenzyl)-3,3-dimethyl-2,3-dihydro-1H-pyrrolo[3,2-b]pyridin-1-yl)-2-oxoethyl)-5-methylpiperazin-2-yl)methyl) (methyl)amino)ethoxy)-N-(4-((4-((3-(methylsulfonyl)benzyl)amino)-5-(trifluoromethyl)pyrimidin-2-yl)amino)phenyl)acetamide

To a DCM solution (1.0 mL) of tert-butyl (2R,5S)-4-(2-(6-(4-fluorobenzyl)-3,3-dimethyl-2,3-dihydro-1H-pyrrolo[3,2-b]pyridin-1-yl)-2-oxoethyl)-2-methyl-5-((methyl(2-(2-((4-((4-((3-(methylsulfonyl)benzyl)amino)-5-(trifluoromethyl)pyrimidin-2-yl)amino)phenyl)amino)-2-oxoethyl)ethyl)amino)methyl)piperazine-1-carboxylate (0.074 g, 0.070 mmol) was added TFA (0.5 mL) under ice-cooled, and the mixture was stirred at room temperature for 4 hr. The reaction mixture was concentrated under reduced pressure, and the residue was dissolved in methanol (2.5 mL), and the solution was filtered through SCX. Then, this SCX was washed with 7M ammonia/methanol solution (3.0 mL), and the eluate was concentrated under reduced pressure to give the title compound (0.045 g).
¹H NMR (400 MHz, DMSO-d₆) δ 0.88-0.90 (m, 3H), 1.24-1.25 (m, 6H), 2.06-2.15 (m, 4H), 2.30-2.35 (m, 2H), 2.54-2.59 (m, 2H), 2.65-2.68 (m, 3H), 2.89-2.95 (m, 2H), 3.05-3.15 (m, 3H), 3.18-3.25 (m, 1H), 3.35-3.40 (m, 1H), 3.48-3.55 (m, 2H), 3.81-3.99 (m, 7H), 4.72-4.74 (m, 2H), 7.08-7.12 (m, 2H), 7.23-7.26 (m, 2H), 7.43-7.62 (m, 6H), 7.77-7.88 (m, 3H), 8.06-8.20 (m, 3H), 9.47-9.55 (m, 2H); MS (m/z): 961.56 [M+H]⁺; retention time/analysis method: 9.91 min/I.

### Example 115

### 2-(2-(4-((6-(4-Fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)methyl)piperazin-1-yl)ethoxy)-N-(4-((4-((3-(methylsulfonyl)benzyl)amino)-5-(trifluoromethyl)pyrimidin-2-yl)amino)phenyl)acetamide

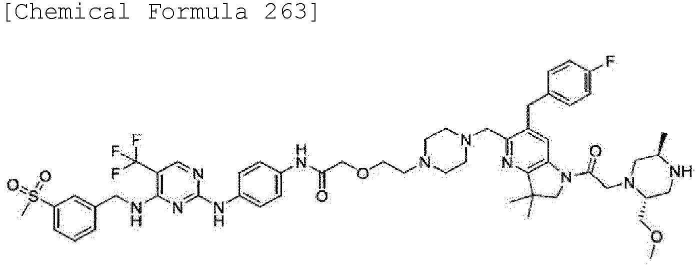

### (Step A) tert-Butyl (2R,5R)-4-(2-(6-(4-fluorobenzyl)-3,3-dimethyl-5-(piperazin-1-ylmethyl)-2,3-dihydro-1H-pyrrolo[3,2-b]pyridin-1-yl)-2-oxoethyl)-5-(methoxymethyl)-2-methylpiperazine-1-carboxylate

To an acetonitrile solution (2.0 mL) of tert-butyl (2R,5R)-4-(2-(5-(chloromethyl)-6-(4-fluorobenzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate (the compound of Example 22, Step E, 0.10 g, 0.17 mmol) were added piperazine (0.070 g, 0.85 mmol), potassium carbonate (0.070 g, 0.51 mmol) and potassium iodide (2.0 mg), and the mixture was stirred at 70°C for 16 hr. The reaction mixture was diluted with a methanol/DCM mixed solvent (10:90), and washed with water. The organic layer was dried over anhydrous sodium sulfate, and the solvent was evaporated under reduced pressure to give the title compound (0.10 g).
MS (m/z): 640.07 [M+H]⁺.

### (Step B) 2-((tert-Butyldiphenylsilyl)oxy)ethan-1-ol

To a THF solution (0.10 L) of tert-butylchlorodiphenylsilane (10 g, 37 mmol) were added imidazole (2.5 g, 37 mmol) and ethylene glycol (12 mL,0.22 mol) under ice-cooled, and the mixture was stirred at room temperature for 10 hr. The reaction mixture was concentrated, diluted with water, and subjected to extraction with ethyl acetate. The organic layer was dried over anhydrous sodium sulfate, and the solvent was evaporated under reduced pressure. The obtained crude product was purified by column chromatography (silica gel, petroleum ether/ethyl acetate) to give the title compound (8.0 g).
¹H NMR (400 MHz, CDCl₃) δ 1.07 (s, 9H), 2.11 (brs, 1H), 3.67-3.69 (m, 2H), 3.76-3.78 (m, 2H), 7.34-7.44 (m, 6H), 7.66-7.68 (m, 4H).

### (Step C) tert-Butyl 2-(2-((tert-butyldiphenylsilyl)oxy)ethoxy) acetate

To a DCM solution (40 mL) of 2-((tert-butyldiphenylsilyl)oxy)ethan-1-ol (4.0 g, 13 mmol) were added 35% aqueous sodium hydroxide solution (40 mL), tetrabutylammonium chloride (3.7 g, 13 mmol) and tert-butyl 2-bromoacetate (2.8 mL, 19 mmol) under ice-cooled, and the mixture was stirred at room temperature for 10 hr. To the reaction mixture was added water, and the mixture was subjected to extraction with DCM. The organic layer was dried over anhydrous sodium sulfate, and the solvent was evaporated under reduced pressure. The obtained crude product was purified by column chromatography (silica gel, petroleum ether/ethyl acetate) to give the title compound (2.3 g).
¹H NMR (400 MHz, CDCl₃) δ 1.05 (s, 9H), 1.47 (s, 9H), 3.67 (t, J = 5.2 Hz, 2H), 3.84 (t, J = 5.4 Hz, 2H), 4.04 (s, 2H), 7.36-7.42 (m, 6H), 7.67-7.69 (m, 4H).

### (Step D) 2-(2-((tert-Butyldiphenylsilyl)oxy)ethoxy)acetic acid

To a toluene solution (10 mL) of (tert-butyl 2-(2-((tert-butyldiphenylsilyl)oxy)ethoxy)acetate (0.25 g, 1.1 mmol) was added silica gel (230-400 mesh ,0.25g), and the mixture was stirred at 110°C for 16 hr. The reaction mixture was filtered through Celite, and the filtrate was concentrated under reduced pressure. The obtained crude product was purified by column chromatography (silica gel, DCM/methanol) to give the title compound (0.16 g).
¹H NMR (400 MHz, DMSO-d₆) δ 0.99 (s, 9H), 3.60-3.62 (m, 2H), 3.75-3.78 (m, 2H), 4.03 (s, 2H), 7.43-7.46 (m, 6H), 7.63-7.66 (m, 4H), one proton missing; MS (m/z): 359.51 [M+H]⁺.

### (Step E) 2-(2-((tert-Butyldiphenylsilyl)oxy)ethoxy)-N-(4-((4-((3-(methylsulfonyl)benzyl)amino)-5-(trifluoromethyl)pyrimidin-2-yl)amino)phenyl)acetamide

To a DMF solution (2.0 mL) of 2-(2-((tert-butyldiphenylsilyl)oxy)ethoxy)acetic acid (0.16 g, 0.45 mmol) were added DIPEA (0.12 mL, 0.95 mmol) and HATU (0.16 g, 0.42 mmol) under ice-cooled, and the mixture was stirred for 15 min. Then, N²-(4-aminophenyl)-N⁴-(3-(methylsulfonyl)benzyl)-5-(trifluoromethyl)pyrimidine-2,4-diamine (the compound of Reference Example 9, 0.12 g, 0.38 mmol) was added, and the mixture was stirred at room temperature for 16 hr. The reaction mixture was diluted with water, and subjected to extraction with a methanol/DCM mixed solvent (10:90). The organic layer was dried over anhydrous sodium sulfate, and the solvent was evaporated under reduced pressure. The obtained crude product was purified by column chromatography (silica gel, DCM/methanol) to give the title compound (0.25 g). MS (m/z): 779.13 [M+H]⁺.

### (Step F) 2-(2-Hydroxyethoxy)-N-(4-((4-((3-(methylsulfonyl)benzyl)amino)-5-(trifluoromethyl)pyrimidin-2-yl)amino)phenyl)acetamide

To a THF solution (4.0 mL) of 2-(2-((tert-butyldiphenylsilyl)oxy)ethoxy)-N-(4-((4-((3-(methylsulfonyl)benzyl)amino)-5-(trifluoromethyl)pyrimidin-2-yl)amino)phenyl)acetamide (0.40 g, 0.51 mmol) was added TBAF (1.0M THF solution, 1.0 mL, 1.0 mmol) under ice-cooled, and the mixture was stirred at room temperature for 2 hr. To the reaction mixture was added water, and the mixture was subjected to extraction with ethyl acetate. The organic layer was dried over anhydrous sodium sulfate, and the solvent was evaporated under reduced pressure. The obtained crude product was purified by column chromatography (silica gel, DCM/methanol) to give the title compound (0.27 g).
MS (m/z): 540.74 [M+H]⁺.

### (Step G) 2-(2-((4-((4-((3-(Methylsulfonyl)benzyl)amino)-5-(trifluoromethyl)pyrimidin-2-yl)amino)phenyl)amino)-2-oxoethoxy) ethyl methanesulfonate

To a THF solution (2 mL) of 2-(2-hydroxyethoxy)-N-(4-((4-((3-(methylsulfonyl)benzyl)amino)-5-(trifluoromethyl)pyrimidin-2-yl)amino)phenyl)acetamide (0.081 g, 0.15 mmol) were added TEA (0.060 mL, 0.44 mmol) and methanesulfonyl chloride (0.020 mL, 0.22mmol) at 0°C, and then the mixture was stirred at room temperature for 16 hr. To the reaction mixture was added water, the mixture was subjected to extraction with ethyl acetate, and the organic layer was dried over anhydrous sodium sulfate. The solvent was evaporated under reduced pressure to give the title compound (0.090 g).
MS (m/z): 618.82 [M+H]⁺.

### (Step H) tert-Butyl (2R,5R)-4-(2-(6-(4-fluorobenzyl)-3,3-dimethyl-5-((4-(2-(2-((4-((4-((3-(methylsulfonyl)benzyl)amino)-5-(trifluoromethyl)pyrimidin-2-yl)amino)phenyl)amino)-2-oxoethoxy)ethyl)piperazin-1-yl)methyl)-2,3-dihydro-1H-pyrrolo[3,2-b]pyridin-1-yl)-2-oxoethyl)-5-(methoxymethyl)-2-methylpiperazine-1-carboxylate

To a DMF solution (2.0 mL) of 2-(2-((4-((4-((3-(methylsulfonyl)benzyl)amino)-5-(trifluoromethyl)pyrimidin-2-yl)amino)phenyl)amino)-2-oxoethyl)ethyl methanesulfonate (0.090 g, 0.15 mmol) were added tert-butyl (2R,5R)-4-(2-(6-(4-fluorobenzyl)-3,3-dimethyl-5-(piperazin-1-ylmethyl)-2,3-dihydro-1H-pyrrolo[3,2-b]pyridin-1-yl)-2-oxoethyl)-5-(methoxymethyl)-2-methylpiperazine-1-carboxylate (0.096 g, 0.15 mmol), potassium carbonate (0.061 g, 0.44 mmol) and potassium iodide (2.0 mg) at room temperature, and the mixture was stirred at 75°C for 16 hr. To the reaction mixture was added water, and the mixture was subjected to extraction with a methanol/DCM mixed solvent (10:90). The organic layer was dried over anhydrous sodium sulfate, and the solvent was evaporated under reduced pressure. The obtained crude product was purified by HPLC preparative chromatography (column: X-SELECT CSH C18, eluent: 10 mM ammonium hydrogencarbonate aqueous solution/acetonitrile, gradient mode) to give the title compound (0.023 g).
MS (m/z): 1161.09 [M+H]⁺.

### (Step I) 2-(2-(4-((6-(4-Fluorobenzyl)-1-(2-((2R,5R)-2-(methoxymethyl)-5-methylpiperazin-1-yl)acetyl )-3,3-dimethyl-2,3-dihydro-1H-pyrrolo[3,2-b]pyridin-5-yl)methyl)piperazin-1-yl)ethoxy)-N-(4-((4-((3-(methylsulfonyl)benzyl)amino)-5-(trifluoromethyl)pyrimidin-2-yl)amino)phenyl)acetamide

To a DCM solution (1.0 mL) of tert-butyl (2R,5R)-4-(2-(6-(4-fluorobenzyl)-3,3-dimethyl-5-((4-(2-(2-((4-((4-((3-(methylsulfonyl)benzyl)amino)-5-(trifluoromethyl)pyrimidin-2-yl)amino)phenyl)amino)-2-oxoethoxy)ethyl)piperazin-1-yl)methyl)-2,3-dihydro-1H-pyrrolo[3,2-b]pyridin-1-yl)-2-oxoethyl)-5-(methoxymethyl)-2-methylpiperazine-1-carboxylate (0.023 g, 0.022 mmol) was added TFA (0.15 mL), and the mixture was stirred at room temperature for 6 hr. The reaction mixture was concentrated under reduced pressure, and the residue was dissolved in methanol (2.5 mL), and the solution was filtered through SCX. Then, this SCX was washed with 7M ammonia/methanol solution (3.0 mL), and the eluate was concentrated under reduced pressure to give the title compound (0.018 g).
MS (m/z): 1060.68 [M+H]⁺; retention time/analysis method: 3.15 min/J.

### Example 119

### 2-(4-(2-(2-((1-(2-((2R,5R)-2-(Methoxymethyl)-5-methylpiperazin-1-yl)acetyl)-3,3-dimethyl-2,3-dihydro-1H-pyrrolo[3,2-b]pyridin-6-yl)methyl)phenoxy)ethyl)piperazin-1-yl)-N-(4-((4-((3-(methylsulfonyl)benzyl)amino)-5-(trifluoromethyl)pyrimidin-2-yl)amino)phenyl)acetamide

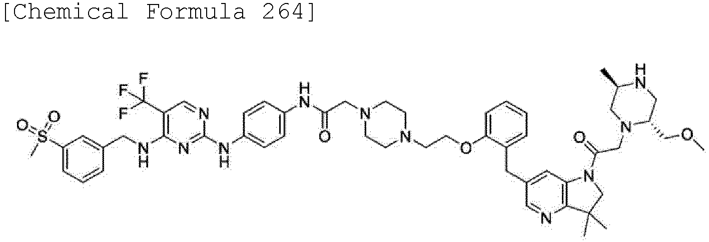

### (Step A) tert-Butyl (2R,5R)-4-(2-(6-(2-(2-bromoethoxy)benzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate

To an acetonitrile solution (1.0 mL) of tert-butyl (2R,5R)-4-(2-(6-(2-hydroxybenzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate (the compound of Example 73, Step A, 0.050 g, 0.090 mmol) was added potassium carbonate (0.065 g, 0.46 mmol), and then 1,2-dibromoethane (0.17 g, 0.92 mmol) was added at room temperature. The mixture was stirred at 70°C for 48 hr, and the reaction mixture was diluted with ethyl acetate, and washed with water. The organic layer was dried over anhydrous sodium sulfate, and the solvent was evaporated under reduced pressure to give the title compound (0.050 g) as a crude product.
MS (m/z): 645.84 [M+H]⁺.

### (Step B) tert-Butyl (2R,5R)-4-(2-(3,3-dimethyl-6-(2-(2-(piperazin-1-yl)ethoxy)benzyl)-2,3-dihydro-1H-pyrrolo[3,2-b]pyridin-1-yl)-2-oxoethyl)-5-(methoxymethyl)-2-methylpiperazine-1-carboxylate

To an acetonitrile solution (2.0 mL) of tert-butyl (2R,5R)-4-(2-(6-(2-(2-bromoethoxy)benzyl)-3,3-dimethyl-2,3-dihydro-1H-pyrrolo[3,2-b]pyridin-1-yl)-2-oxoethyl)-5-(methoxymethyl)-2-methylpiperazine-1-carboxylate (0.20 g, 0.31 mmol) were added piperazine (0.14 g, 1.5 mmol), potassium carbonate (0.13 g, 0.93 mmol) and potassium iodide (3.0 mg) at room temperature, and the mixture was stirred at 70°C for 16 hr. The reaction mixture was diluted with ethyl acetate, and washed with water. The organic layer was dried over anhydrous sodium sulfate, and the solvent was evaporated under reduced pressure. The obtained crude product was purified by column chromatography (silica gel, DCM/methanol) to give the title compound (0.10 g).
MS (m/z): 652.09 [M+H]⁺.

### (Step C) 2-Chloro-N-(4-((4-((3-(methylsulfonyl)benzyl)amino)-5-(trifluoromethyl)pyrimidin-2-yl)amino)phenyl)acetamide

To a DCM solution (2.0 mL) of N²-(4-aminophenyl)-N⁴-(3-(methylsulfonyl)benzyl)-5-(trifluoromethyl)pyrimidine-2,4-diamine (the compound of Reference Example 9, 0.10 g, 0.29 mmol) were added TEA (0.10 mL, 0.68 mmol) and chloroacetyl chloride (0.030 g, 0.35 mmol) at 0°C, and the mixture was stirred at room temperature for 4 hr. The reaction mixture was diluted with DCM, and washed with water. The organic layer was dried over anhydrous sodium sulfate, and the solvent was evaporated under reduced pressure to give the title compound (0.12 g).
MS (m/z): 514.61 [M+H]⁺.

### (Step D) tert-Butyl (2R,5R)-4-(2-(3,3-dimethyl-6-(2-(2-(4-(2-((4-((4-((3-(methylsulfonyl)benzyl)amino)-5-(trifluoromethyl)pyrimidin-2-yl)amino)phenyl)amino)-2-oxoethyl)piperazin-1-yl)ethoxy)benzyl)-2,3-dihydro-1H-pyrrolo[3,2-b]pyridin-1-yl)-2-oxoethyl)-5-(methoxymethyl)-2-methylpiperazine-1-carboxylate

To an acetonitrile solution (2.0 mL) of 2-chloro-N-(4-((4-((3-(methylsulfonyl)benzyl)amino)-5-(trifluoromethyl)pyrimidin-2-yl)amino)phenyl)acetamide (0.10 g, 0.19 mmol) were added tert-butyl (2R,5R)-4-(2-(3,3-dimethyl-6-(2-(2-(piperazin-1-yl)ethoxy)benzyl)-2,3-dihydro-1H-pyrrolo[3,2-b]pyridin-1-yl)-2-oxoethyl)-5-(methoxymethyl)-2-methylpiperazine-1-carboxylate (0.14 g, 0.21 mmol), potassium carbonate (0.080 g, 0.58 mmol) and potassium iodide (2.0 mg), and the mixture was stirred at 70°C for 16 hr. The reaction mixture was diluted with ethyl acetate, and washed with water. The organic layer was dried over anhydrous sodium sulfate, and the solvent was evaporated under reduced pressure. The obtained crude product was purified by HPLC preparative chromatography (column: X-SELECT CSH C18, eluent: 10 mM ammonium hydrogencarbonate aqueous solution/acetonitrile, gradient mode) to give the title compound (0.030 g). MS (m/z): 1129.73 [M+H]⁺.

### (Step E) 2-(4-(2-(2-((1-(2-((2R,5R)-2-(Methoxymethyl)-5-methylpiperazin-1-yl)acetyl)-3,3-dimethyl-2,3-dihydro-1H-pyrrolo[3,2-b]pyridin-6-yl)methyl)phenoxy)ethyl)piperazin-1-yl)-N-(4-((4-((3-(methylsulfonyl)benzyl)amino)-5-(trifluoromethyl)pyrimidin-2-yl)amino)phenyl)acetamide

To a DCM solution (1.0 mL) of tert-butyl (2R,5R)-4-(2-(3,3-dimethyl-6-(2-(2-(4-(2-((4-((4-((3-(methylsulfonyl)benzyl)amino)-5-(trifluoromethyl)pyrimidin-2-yl)amino)phenoxy)amino)-2-oxoethyl)piperazin-1-yl)ethoxy)benzyl)-2,3-dihydro-1H-pyrrolo[3,2-b]pyridin-1-yl)-2-oxoethyl)-5-(methoxymethyl)-2-methylpiperazine-1-carboxylate (0.030 g, 0.029 mmol) was added TFA (0.5 mL) under ice-cooled, and the mixture was stirred at room temperature for 5 hr. The reaction mixture was concentrated under reduced pressure, and the residue was dissolved in methanol (2.0 mL), and the solution was filtered through SCX. Then, this SCX was washed with 7M ammonia/methanol solution (3.0 mL), and the eluate was concentrated under reduced pressure to give the title compound (0.020 g).
¹H NMR (400 MHz, DMSO-d₆) δ 0.88-0.89 (m, 3H), 1.23-1.25 (m, 6H), 2.20-2.30 (m, 1H), 2.32-2.42 (m, 1H), 2.50-2.70 (m, 11H), 2.71-2.80 (m, 2H), 2.81-2.90 (m, 1H), 3.00-3.10 (m, 2H), 3.11-3.20 (m, 6H), 3.22-3.30 (m, 2H), 3.35-3.45 (m, 1H), 3.60-3.70 (m, 1H), 3.80-3.90 (m, 3H), 3.95-4.15 (m, 3H), 4.70-4.80 (m, 2H), 6.87-6.91 (m, 1H), 6.97-6.99 (m, 1H), 7.17-7.23 (m, 2H), 7.40-7.50 (m, 4H), 7.52-7.65 (m, 2H), 7.75-7.90 (m, 3H), 8.00-8.25 (m, 3H), 9.50-9.55 (m, 2H),one proton missing; MS (m/z): 1028.46 [M+H]⁺; retention time/analysis method: 3.09 min/B.

The example compounds produced according to the above-mentioned production method or the method shown in Example 109, 115 or 119, or a method similar thereto, are described below. For each compound, the compound name, structural formula, MS (m/z) (actual measured value), retention time/analysis method (unit: minutes), and the analytical method are shown.

### Example 110

### 2-(2-(2-((((2R,5R)-1-(2-(6-(4-fluorobenzyl)-3,3-dimethyl-2,3-dihydro-1H-pyrrolo[3,2-b]pyridin-1-yl)-2-oxoethyl)-5-methylpiperazin-2-yl)methyl) (methyl)amino)ethoxy)ethoxy)-N-(4-((4-((3-(methylsulfonyl)benzyl)amino)-5-(trifluoromethyl)pyrimidin-2-yl)amino)phenyl)acetamide

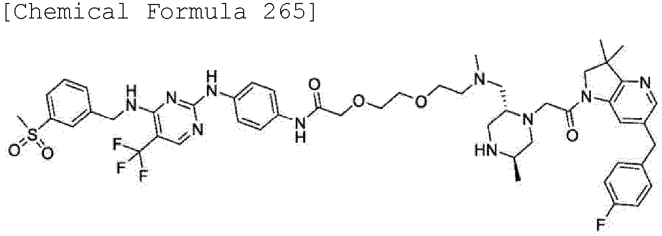

MS (m/z): 1005.65 [M+H]⁺; retention time/analysis method: 10.72 min/C

### Example 111

### 1-((2R,5R)-1-(2-(6-(4-Fluorobenzyl)-3,3-dimethyl-2,3-dihydro-1H-pyrrolo[3,2-b]pyridin-1-yl)-2-oxoethyl)-5-methylpiperazin-2-yl)-2-methyl-N-(4-((4-((3-(methylsulfonyl)benzyl)amino)-5-(trifluoromethyl)pyrimidin-2-yl)amino)phenyl)-5,8,11-trioxa-2-azatridecan-13-amide

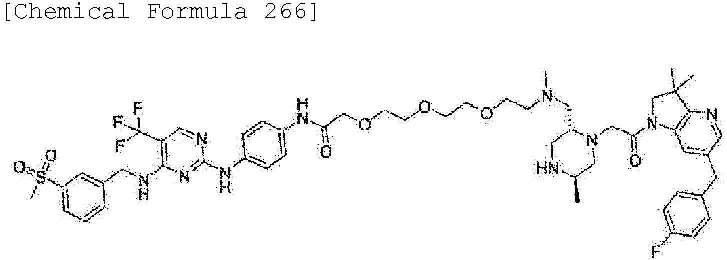

MS (m/z): 1049.65 [M+H]⁺; retention time/analysis method: 10.71 min/C

### Example 112

### 1-((2R,5R)-1-(2-(6-(4-Fluorobenzyl)-3,3-dimethyl-2,3-dihydro-1H-pyrrolo[3,2-b]pyridin-1-yl)-2-oxoethyl)-5-methylpiperazin-2-yl)-2-methyl-N-(4-((4-((3-(methylsulfonyl)benzyl)amino)-5-(trifluoromethyl)pyrimidin-2-yl)amino)phenyl)-5,8,11,14-tetraoxa-2-azahexadecan-16-amide

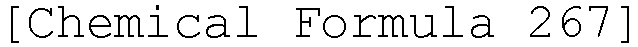

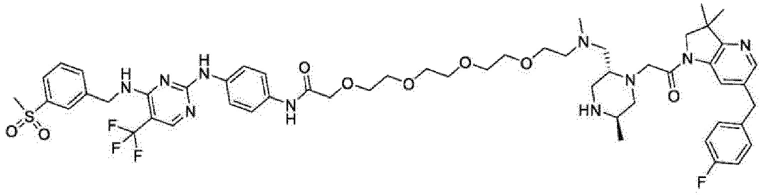

MS (m/z): 1093.70 [M+H]⁺; retention time/analysis method: 2.78 min/J

### Example 113

### 1-((2R,5R)-1-(2-(6-(4-Fluorobenzyl)-3,3-dimethyl-2,3-dihydro-1H-pyrrolo[3,2-b]pyridin-1-yl)-2-oxoethyl)-5-methylpiperazin-2-yl)-2-methyl-N-(4-((4-((3-(methylsulfonyl)benzyl)amino)-5-(trifluoromethyl)pyrimidin-2-yl)amino)phenyl)-5,8,11,14,17-pentaoxa-2-azanonadecan-19-amide

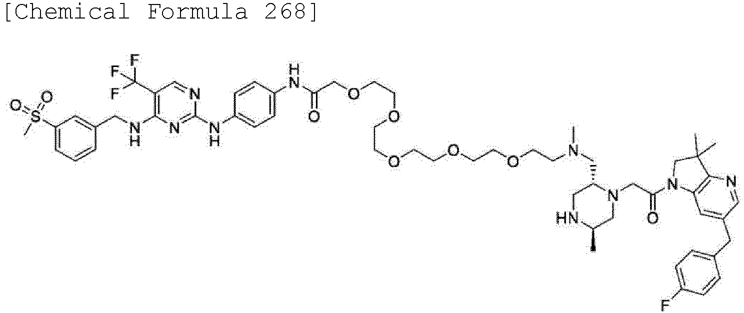

MS (m/z): 1137.71 [M+H]⁺; retention time/analysis method: 10.69 min/C

### Example 114

### 2-(4-((6-(4-Fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)methyl)piperazin-1-yl)-N-(4-((4-((3-(methylsulfonyl)benzyl)amino)-5-(trifluoromethyl)pyrimidin-2-yl)amino)phenyl)acetamide

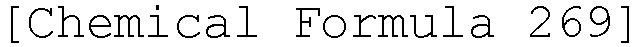

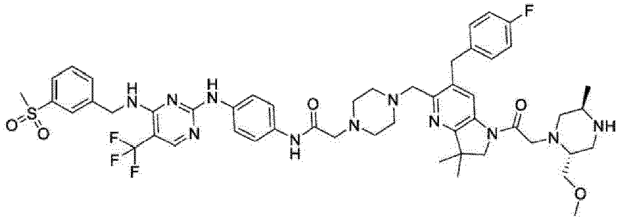

MS (m/z): 1020.62 [M+H]⁺; retention time/analysis method: 3.21 min/B

### Example 116

### 2-(2-(2-(4-((6-(4-Fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)methyl)piperazin-1-yl)ethoxy)ethoxy)-N-(4-((4-((3-(methylsulfonyl)benzyl)amino)-5-(trifluoromethyl)pyrimidin-2-yl)amino)phenyl)acetamide

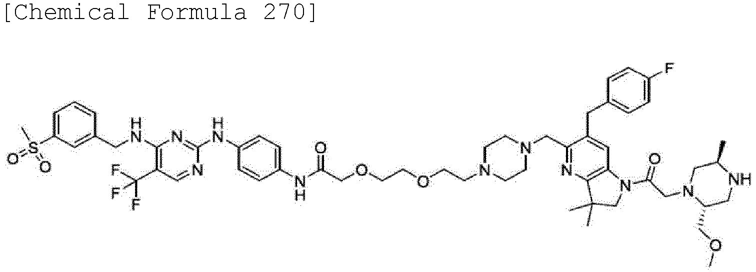

MS (m/z): 1104.72 [M+H]⁺; retention time/analysis method: 3.11 min/J

### Example 117

### 2-(2-(2-(2-(4-((6-(4-Fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)methyl)piperazin-1-yl)ethoxy)ethoxy)ethoxy)-N-(4-((4-((3-(methylsulfonyl)benzyl)amino)-5-(trifluoromethyl)pyrimidin-2-yl)amino)phenyl)acetamide

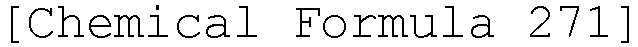

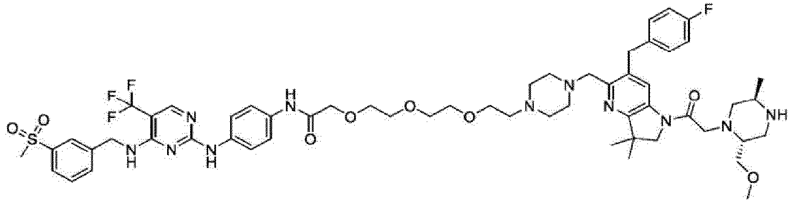

MS (m/z): 1148.71 [M+H]⁺; retention time/analysis method: 3.18 min/J

### Example 118

### 14-(4-((6-(4-Fluorobenzyl)-1-(2-((2R,5R)-2-(methoxymethyl)-5-methylpiperazin-1-yl)acetyl)-3,3-dimethyl-2,3-dihydro-1H-pyrrolo[3,2-b]pyridin-5-yl)methyl)piperazin-1-yl)-N-(4-((4-((3-(methylsulfonyl)benzyl)amino)-5-(trifluoromethyl)pyrimidin-2-yl)amino)phenyl)-3,6,9,12-tetraoxatetradecanamide

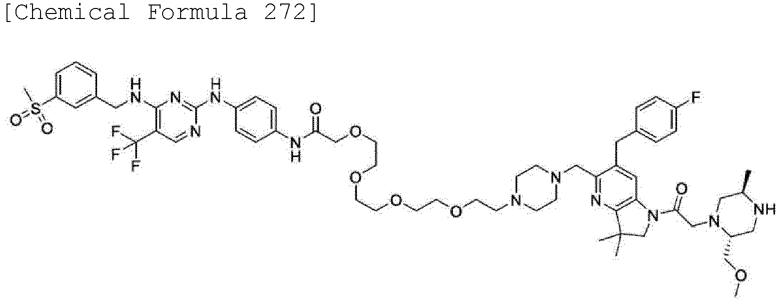

MS (m/z): 1192.45 [M+H]⁺; retention time/analysis method: 9.99 min/C

### Example 120

### 2-(2-(4-(2-(2-((1-(2-((2R,5R)-2-(Methoxymethyl)-5-methylpiperazin-1-yl)acetyl)-3,3-dimethyl-2,3-dihydro-1H-pyrrolo[3,2-b]pyridin-6-yl)methyl)phenoxy)ethyl)piperazin-1-yl)ethoxy)-N-(4-((4-((3-(methylsulfonyl)benzyl)amino)-5-(trifluoromethyl)pyrimidin-2-yl)amino)phenyl)acetamide

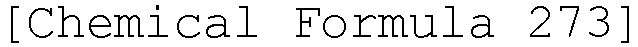

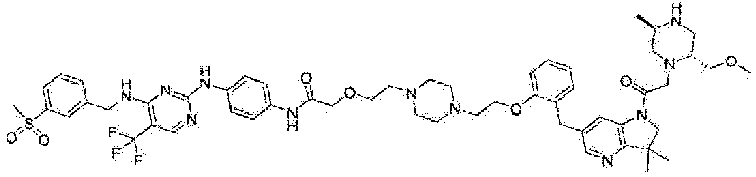

MS (m/z): 1072.72 [M+H]⁺; retention time/analysis method: 9.86 min/C

### Example 121

### 2-(2-(2-(4-(2-(2-((1-(2-((2R,5R)-2-(Methoxymethyl)-5-methylpiperazin-1-yl)acetyl)-3,3-dimethyl-2,3-dihydro-1H-pyrrolo[3,2-b]pyridin-6-yl)methyl)phenoxy)ethyl)piperazin-1-yl)ethoxy)ethoxy)-N-(4-((4-((3-(methylsulfonyl)benzyl)amino)-5-(trifluoromethyl)pyrimidin-2-yl)amino)phenyl)acetamide

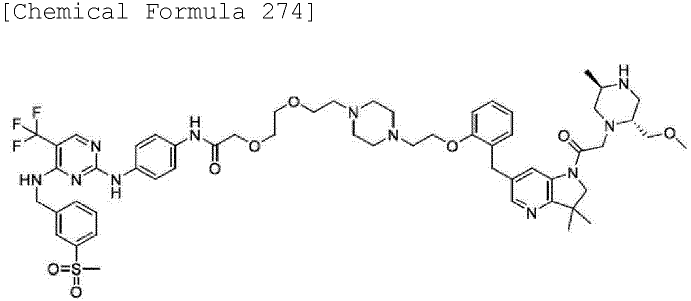

MS (m/z): 1116.73 [M+H]⁺; retention time/analysis method: 3.12 min/J

### Example 122

### 2-(2-(2-(2-(4-(2-(2-((1-(2-((2R,5R)-2-(Methoxymethyl)-5-methylpiperazin-1-yl)acetyl)-3,3-dimethyl-2,3-dihydro-1H-pyrrolo[3,2-b]pyridin-6-yl)methyl)phenoxy)ethyl)piperazin-1-yl)ethoxy)ethoxy)ethoxy)-N-(4-((4-((3-(methylsulfonyl)benzyl)amino)-5-(trifluoromethyl)pyrimidin-2-yl)amino)phenyl)acetamide

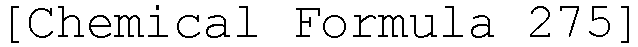

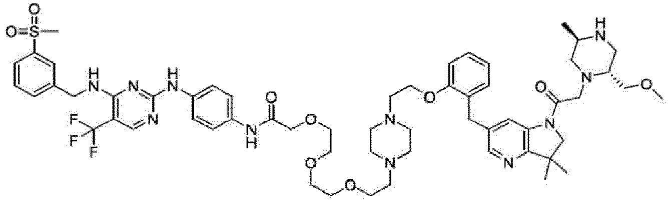

MS (m/z): 1160.70 [M+H]⁺; retention time/analysis method: 3.15 min/J

### Example 123

### 14-(4-(2-(2-((1-(2-((2R,5R)-2-(Methoxymethyl)-5-methylpiperazin-1-yl)acetyl)-3,3-dimethyl-2,3-dihydro-1H-pyrrolo[3,2-b]pyridin-6-yl)methyl)phenoxy)ethyl)piperazin-1-yl)-N-(4-((4-((3-(methylsulfonyl)benzyl)amino)-5-(trifluoromethyl)pyrimidin-2-yl)amino)phenyl)-3,6,9,12-tetraoxatetradecanamide

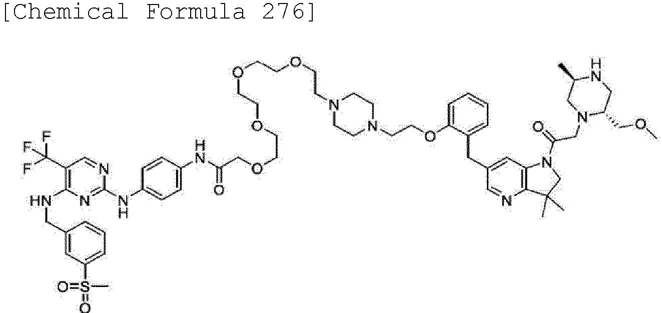

MS (m/z): 1204.82 [M+H]⁺; retention time/analysis method: 3.20 min/J

### Example 124

### 2-(4-(2-(3-((1-(2-((2R,5R)-2-(Methoxymethyl)-5-methylpiperazin-1-yl)acetyl)-3,3-dimethyl-2,3-dihydro-1H-pyrrolo[3,2-b]pyridin-6-yl)methyl)phenoxy)ethyl)piperazin-1-yl)-N-(4-((4-((3-(methylsulfonyl)benzyl)amino)-5-(trifluoromethyl)pyrimidin-2-yl)amino)phenyl)acetamide

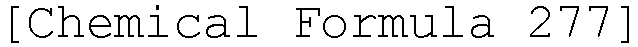

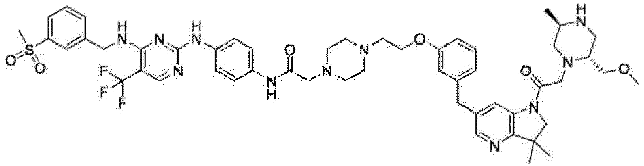

MS (m/z): 1028.68 [M+H]⁺; retention time/analysis method: 3.07 min/J

### Example 125

### 2-(2-(4-(2-(3-((1-(2-((2R,5R)-2-(Methoxymethyl)-5-methylpiperazin-1-yl)acetyl)-3,3-dimethyl-2,3-dihydro-1H-pyrrolo[3,2-b]pyridin-6-yl)methyl)phenoxy)ethyl)piperazin-1-yl)ethoxy)-N-(4-((4-((3-(methylsulfonyl)benzyl)amino)-5-(trifluoromethyl)pyrimidin-2-yl)amino)phenyl)acetamide

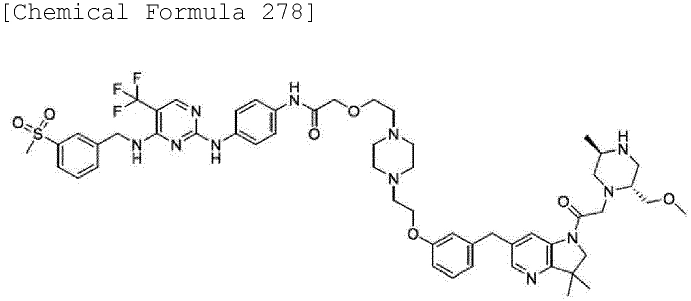

MS (m/z): 1072.63 [M+H]⁺; retention time/analysis method: 3.12 min/J

### Example 126

### 2-(2-(2-(4-(2-(3-((1-(2-((2R,5R)-2-(Methoxymethyl)-5-methylpiperazin-1-yl)acetyl)-3,3-dimethyl-2,3-dihydro-1H-pyrrolo[3,2-b]pyridin-6-yl)methyl)phenoxy)ethyl)piperazin-1-yl)ethoxy)ethoxy)-N-(4-((4-((3-(methylsulfonyl)benzyl)amino)-5-(trifluoromethyl) pyrimidin-2-yl)amino)phenyl)acetamide

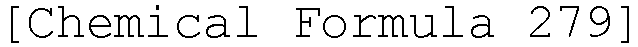

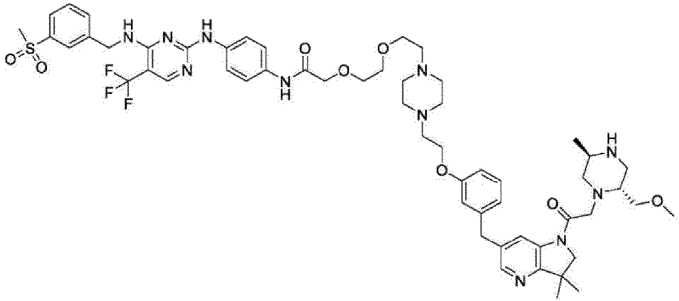

MS (m/z): 1116.73 [M+H]⁺; retention time/analysis method: 3.08 min/J

### Example 127

### 2-(2-(2-(2-(4-(2-(3-((1-(2-((2R,5R)-2-(Methoxymethyl)-5-methylpiperazin-1-yl)acetyl)-3,3-dimethyl-2,3-dihydro-1H-pyrrolo[3,2-b]pyridin-6-yl)methyl)phenoxy)ethyl)piperazin-1-yl)ethoxy)ethoxy)ethoxy)-N-(4-((4-((3-(methylsulfonyl)benzyl)amino)-5-(trifluoromethyl)pyrimidin-2-yl)amino)phenyl)acetamide

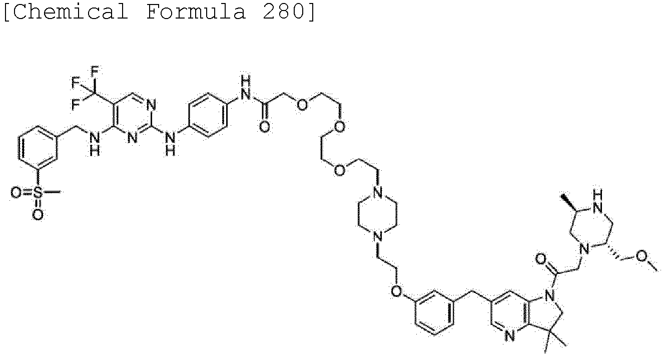

MS (m/z): 1160.65 [M+H]⁺; retention time/analysis method: 3.14 min/J

### Example 128

### 14-(4-(2-(3-((1-(2-((2R,5R)-2-(Methoxymethyl)-5-methylpiperazin-1-yl)acetyl)-3,3-dimethyl-2,3-dihydro-1H-pyrrolo[3,2-b]pyridin-6-yl)methyl)phenoxy)ethyl)piperazin-1-yl)-N-(4-((4-((3-(methylsulfonyl)benzyl)amino)-5-(trifluoromethyl)pyrimidin-2-yl)amino)phenyl)-3,6,9,12-tetraoxatetradecanamide

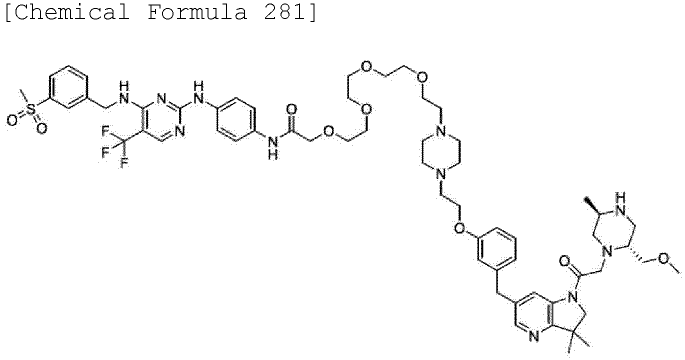

MS (m/z): 1204.82 [M+H]⁺; retention time/analysis method: 3.17 min/J

### Test Example 1

### [Measurement of ROCK2 Kinase Inhibitory Activity]

The ROCK2 kinase inhibitory activity was measured using a mobility shift assay (MSA) provided by Carna Biosciences, Inc.

### (Preparation of Test Substance Solution)

The test compound was dissolved in DMSO and further diluted with DMSO to prepare a solution having a concentration 100 times the evaluation concentration. The solution was further diluted 25 times with an assay buffer (20 mmol/L HEPES, 0.01% Triton (registered trademark) X-100, 1 mmol/L DTT, pH 7.5) to obtain a test compound solution.

### (Preparation of ROCK2 Kinase)

The human ROCK2 kinase was expressed by fusing the catalytic domain (1 to 553 amino acids of Accession No. NP_004841.2) with GST at the N-terminus using the baculovirus expression system. GST-ROCK2 was purified by glutathione sepharose chromatography.

### (Test Preparation and Test Method)

A 4-fold concentration substrate (LIMKtide, 1000 nmol/L) /ATP (5 µmol/L)/metal solution (Mg, 5 mmol/L) was prepared with kit buffer (20 mmol/L HEPES, 0.01% Triton (registered trademark) X-100, 5 mmol/L DTT, pH 7.5). A 2-fold concentration kinase solution was prepared with assay buffer. 5 µL of a 4-fold concentration test compound solution, 5 µL of a 4-fold concentration substrate/ATP/metal solution, and 10 µL of a 2-fold concentration kinase solution were mixed in a well of a polypropylene 384 well plate, and the mixture was reacted at room temperature for 1 hour. 70 µL of termination buffer (127 mmol/L HEPES, 0.01% Triton (registered trademark) X-100, 26.7 mmol/L EDTA-2Na, 1% DMSO, pH 7.5) was added to stop the reaction. The substrate peptide and the phosphorylated peptide in the reaction solution were separated and quantified using a LabChip (trademark) system (Perkin Elmer).

### (Method for Evaluating Inhibitory Activity and Data Analysis)

The kinase reaction was evaluated by the product ratio (P/(P + S)) calculated from the substrate peptide peak height (S) and the phosphorylated peptide peak height (P).

The average signal of the control well including all the reaction components was set to 0% suppression, the average signal of the background well (no enzyme added) was set to 100% suppression, and the inhibition rate was calculated from the average signal of the test compound test well.

The ROCK2 inhibition rate and the evaluation concentration of the test compound are shown in Table 4-1, and the IC50 value of the inhibitory activity is shown in Table 4-2. The ROCK2 inhibition rate in Table 4-1 is represented by A ≥ 75%, 75% > B ≥ 50%, 50% > C ≥ 25%, and D < 25%, and the IC50 value in Table 4-2 is represented by A<0.03 µM, 0.03 µM ≤ B < 0.3 µM, 0.3 µM ≤ C < 3 µM, and 3 µM ≤ D < 10 µM.

**[Table 4-1]**

| Test compound | *Inhibition rate | **Evaluation concentration |
|---|---|---|
| Example 3 | A | 10 µM |
| Example 9 | C | 1 µM |
| Example 16 | B | 0.03 µM |
| Example 24 | A | 10 µM |
| Example 31 | C | 1 µM |
| Example 36 | B | 0.03 µM |
| Example 48 | A | 10 µM |
| Example 56 | D | 1 µM |
| Example 80 | C | 0.03 µM |

| | | |
|---|---|---|
| *Inhibition rate: ROCK2 inhibition rate **Evaluation concentration: concentration of test compound | | |

**[Table 4-2]**

| Test compound | *Inhibitory activity | Test compound | *Inhibitory activity |
|---|---|---|---|
| Ref.Example 10 | D | Ref.Example 13 | B |
| Ref.Example 11 | D | Ref.Example 14 | A |
| Ref.Example 12 | C | Ref.Example 15 | A |

| | | | |
|---|---|---|---|
| *Inhibitory activity: IC50 value | | | |

From the above results, it was shown that the compound of the present invention has excellent ROCK2 kinase inhibitory activity.

### Test Example 2

### [Measurement of ALK Kinase Inhibitory Activity]

The ALK kinase inhibitory activity was measured using a mobility shift assay (MSA) provided by Carna Biosciences, Inc.

### (Preparation of Test Substance Solution)

The test compound was dissolved in DMSO to prepare a 1 µM solution. The solution was further diluted 25 times with an assay buffer (20 mmol/L HEPES, 0.01%Triton (registered trademark) X-100, 1 mmol/L DTT, pH 7.5) to obtain a test compound solution.

### (Preparation of ALK Kinase and Test Preparation)

The human ALK kinase was expressed by fusing the cytoplasmic domain (1058 to 1620 amino acids of Accession No. BAG 10812.1) with GST at the N-terminus using the baculovirus expression system. The GST-ALK was purified by glutathione sepharose chromatography. A 4-fold concentration substrate (Srctide, 1000 nmol/L)/ATP (50 µmol/L)/metal solution (Mg, 5 mmol/L) was prepared with kit buffer (20 mmol/L HEPES, 0.01% Triton (registered trademark) X-100, 5 mmol/L DTT, pH 7.5). A 2-fold concentration kinase solution was prepared with assay buffer.

### (Test Method and Method for Evaluating Inhibitory Activity and Data Analysis)

In the same manner as described in Test Example 1, tests and evaluation of inhibitory activity and data analysis were performed.

The ALK kinase inhibition rate when the concentration of the test compound is 10 nM is shown in Table 5 below as A ≥ 75%, 75% > B ≥ 50%, 50% > C ≥ 25%, and D < 25%.

**[Table 5]**

| Test compound | *Inhibition rate | Test compound | *Inhibition rate |
|---|---|---|---|
| Example 85 | B | Example 95 | A |
| Example 89 | B | Example 100 | A |
| Example 92 | A | Example 107 | A |

| | | | |
|---|---|---|---|
| *Inhibition rate: inhibition rate at 10 nM | | | |

From the above results, it was shown that the compound of the present invention has excellent ALK kinase inhibitory activity.

### Test Example 3

### [Measurement of FAK Kinase Inhibitory Activity]

The FAK kinase inhibitory activity was measured using a mobility shift assay (MSA) provided by Carna Biosciences, Inc.

### (Preparation of Test Substance Solution)

The test compound was dissolved in DMSO to prepare a 3 µM solution. The solution was further diluted 25 times with an assay buffer (20 mmol/L HEPES, 0.01%Triton (registered trademark) X-100, 1 mmol/L DTT, pH 7.5) to obtain a test compound solution.

### (Preparation of FAK Kinase and Test Preparation)

Incomplete human FAK kinase was expressed by fusing 376 to 1052 amino acids of Accession No. NP_722560.1 with GST at the N-terminus using the baculovirus expression system. The GST-FAK was purified by glutathione sepharose chromatography. A 4-fold concentration substrate (Blk/Lyntide, 1000 nmol/L)/ATP (25 µmol/L)/metal solution (Mg, 5 mmol/L) was prepared with kit buffer (20 mmol/L HEPES, 0.01% Triton (registered trademark) X-100, 5 mmol/L DTT, pH 7.5). A 2-fold concentration kinase solution was prepared with assay buffer.

### (Test Method and Method for Evaluating Inhibitory Activity and Data Analysis)

In the same manner as described in Test Example 1, tests and evaluation of inhibitory activity and data analysis were performed. The reaction time was set to 5 hours. The FAK kinase inhibition rate when the concentration of the test compound is 30 nM is shown in Table 6 below as A ≥ 75%, 75% > B ≥ 50%, 50% > C ≥ 25%, and D < 25%.

**[Table 6]**

| Test compound | *Inhibition rate | Test compound | *Inhibition rate |
|---|---|---|---|
| Example 109 | A | Example 116 | B |
| Example 111 | A | Example 122 | B |
| Example 115 | A | Example 128 | B |

| | | | |
|---|---|---|---|
| *Inhibition rate: inhibition rate at 30 nM | | | |

From the above results, it was shown that the compound of the present invention has excellent FAK kinase inhibitory activity.

### Test Example 4

### [Measurement of Degradation Inducing Activity of ROCK2 Protein]

In vitro degradation inducing activity of ROCK2 protein of compounds of Examples was evaluated by Western Blotting (WB).

### (Preparation of Test Substance Solution)

By a test compound, 10 mM DMSO solution was prepared, which was used as a 10 µM test compound solution. Further, the solution was diluted 10-fold with DMSO to prepare a 1 mM solution, which was used as a 1 µM test compound solution.

### (Test Method)

HUVEC cells (ATCC: CRL-1730, Lot: 70041472) were cultured in F-12K medium supplemented with 10% FBS, 0.1 mg/mL heparin, 30 mg/mL Endothelial Cell Growth Supplement (ECGS, Corning), and 1% Penicillin/Streptomycin. The HUVEC cells seeded in a 24 well plate at 50,000 cells/well/1 mL were cultured for 24 hours, and then 1 µL of DMSO or a test compound solution was added thereto and treatment was performed for 24 hours. Cells were harvested and lysed for 20 minutes on ice using lysis buffer (Cell Signaling Technology, Cat#9803) supplemented with protease inhibitor cocktail (Sigma, Cat#P8340; Roche, Cat#P11873580001), and phosphatase inhibitor cocktail (Sigma, Cat#P5726, P0044). The lysate was sonicated (15 seconds, amplitude 30%, 1 cycle) and then centrifuged at 12500 rpm for 15 minutes at 4°C. The protein concentration was determined by BCA assay kit (Thermo Fisher Scientific, Cat#23225).

### (Method for Evaluating Degradation Inducing Activity and Data Analysis)

The western blotting was performed using Simple Western JESS (protein simple). A cell lysis sample (protein amount; about 1.06 µg) was loaded on a plate of Simple Western JESS, and the expression level of ROCK2 protein was detected using a 12-230 kDa Wes Separation Module (Protein simple, Cat#SM-WO04), an Anti-Rabbit Detection Module for Wes (Protein simple, Cat#DM-001), and a Total Protein Detection Module for Wes (Protein simple, Cat#DM-TP01). As the primary antibody, a material obtained by diluting ROCK2 (D1B1) Rabbit mAb (Cell Signaling Technology, Cat#9029) at 1:10 was used, and as the secondary antibody, a material obtained by diluting HRP-labeled anti-rabbit IgG antibody (Cell Signaling Technology, Cat#7074S) at 1:100 was used. The expression level of the target protein was standardized using the total protein amount loaded as a reference. The standardized expression level data of the control (DMSO) was taken as 100%, and the degradation rate (%) was calculated from the average standardized expression level data of the test compound. All analysis steps, such as data detection and analysis, are fully automated.

With respect to the ROCK2 protein degradation rate of the test compound, the protein degradation rates (%) at the evaluation concentrations were set to A ≥ 75%, 75% > B ≥ 50%, 50% > C ≥ 25%, and D < 25%, and are shown in Table 7.

**[Table 7]**

| Test compound | *Degradation rate | Test compound | *Degradation rate | Test compound | *Degradation rate |
|---|---|---|---|---|---|
| Example 1 | A/C | Example 29 | A/B | Example 57 | A/C |
| Example 2 | B/D | Example 30 | D/D | Example 58 | A/B |
| Example 3 | A/B | Example 31 | B/B | Example 59 | A/A |
| Example 4 | A/A | Example 32 | A/D | Example 60 | A/C |
| Example 5 | D/C | Example 33 | A/D | Example 61 | A/B |
| Example 6 | A/B | Example 34 | A/D | Example 62 | A/C |
| Example 7 | B/B | Example 35 | A/B | Example 63 | B/D |
| Example 8 | A/C | Example 36 | A/C | Example 64 | B/D |
| Example 9 | A/D | Example 37 | A/D | Example 65 | B/D |
| Example 10 | A/C | Example 38 | A/D | Example 68 | A/A |
| Example 11 | A/B | Example 39 | A/C | Example 67 | B/D |
| Example 12 | C/D | Example 40 | B/C | Example 68 | B/A |
| Example 13 | D/D | Example 41 | A/B | Example 69 | A/A |
| Example 14 | A/C | Example 42 | A/B | Example 70 | A/A |
| Example 15 | A/D | Example 43 | A/C | Example 71 | D/C |
| Example 16 | A/C | Example 44 | A/B | Example 72 | A/D |
| Example 17 | A/C | Example 45 | B/D | Example 73 | A/D |
| Example 18 | A/A | Example 46 | C/C | Example 74 | B/D |
| Example 19 | A/C | Example 47 | A/B | Example 75 | B/D |
| Example 20 | A/B | Example 48 | B/B | Example 76 | B/D |
| Example 21 | B/C | Example 49 | B/A | Example 77 | A/D |
| Example 22 | A/D | Example 50 | A/C | Example 78 | A/D |
| Example 23 | A/A | Example 51 | A/B | Example 79 | A/A |
| Example 24 | B/A | Example 52 | B/C | Example 80 | A/B |
| Example 25 | A/A | Example 53 | B/D | Example 81 | C/B |
| Example 26 | A/B | Example 54 | C/A | Example 82 | A/C |
| Example 27 | D/D | Example 55 | B/D | Example 83 | A/B |
| Example 28 | D/B | Example 56 | B/B | Example 84 | C/B |

| | | | | | |
|---|---|---|---|---|---|
| *Degradation rate: degradation rate at 10 µM/degradation rate at 1 µM | | | | | |

From the above results, it was shown that the compound of the present invention has not only an ROCK2 kinase inhibitory activity but also an excellent ROCK2 protein degradation inducing activity.

### Test Example 5

### [Measurement of Degradation Inducing Activity of ALK Protein]

In vitro degradation inducing activity of ALK protein of compounds of Examples was evaluated by Western Blotting (WB).

### (Preparation of Test Substance Solution)

By a test compound, 10 mM DMSO solution was prepared, which was used as a 10 µM test compound solution. Further, the solution was diluted 10-fold with DMSO to prepare a 1 mM solution, which was used as a 1 µM test compound solution.

### (Test Method)

SU-DHL-1 cells (ATCC: CRL-2955, Lot: 70051509) were cultured in RPM1640 medium supplemented with 10% FBS, and 1% Penicillin/Streptomycin. The SU-DHL-1 cells seeded in a 24 well plate at 200,000 cells/well/1 mL were cultured for 24 hours, and then 1 µL of DMSO or a test compound solution was added thereto and treatment was performed for 24 hours. Cells were harvested and lysed for 20 minutes on ice using RIPA lysis buffer (Sigma, Cat#R0278) supplemented with protease inhibitor cocktail (Sigma, Cat#P8340; Roche, Cat#P11873580001), and phosphatase inhibitor cocktail (Sigma, Cat#P5726, P0044). The lysate was sonicated (15 seconds, amplitude 30%, 1 cycle) and then centrifuged at 12500 rpm for 15 minutes at 4°C. The protein concentration was determined by BCA assay kit (Thermo Fisher Scientific, Cat#23225).

### (Method for Evaluating Degradation Inducing Activity and Data Analysis)

The western blotting was performed using Simple Western JESS (protein simple). A cell lysis sample (protein amount; about 0.6 µg) was loaded on a plate of Simple Western JESS, and the expression level of ALK protein was detected using a 12-230 kDa Wes Separation Module (Protein simple, Cat#SM-WO04), an Anti-Rabbit Detection Module for Wes (Protein simple, Cat#DM-001), and a Total Protein Detection Module for Wes (Protein simple, Cat#DM-TP01). As the primary antibody, a material obtained by diluting ALK (D5F3) XP (registered trademark) Rabbit mAb (Cell Signaling Technology, Cat#3633) at 1:75 was used, and as the secondary antibody, a material obtained by diluting HRP-labeled anti-rabbit IgG antibody (Cell Signaling Technology, Cat#7074S) at 1:250 was used. The expression level of the target protein was standardized using the total protein amount loaded as a reference. The standardized expression level data of the control (DMSO) was taken as 100%, and the degradation rate (%) was calculated from the average standardized expression level data of the test compounds (compounds of Examples 85 to 108). All analysis steps, such as data detection and analysis, are fully automated.

All of the compounds of Examples 85 to 108 showed an ALK protein degradation rate of 75% or more at 10 µM. Therefore, it was confirmed that the compound of the present invention had not only an ALK kinase inhibitory activity but also an ALK protein degradation inducing activity.

### Test Example 6

### [Measurement of Degradation Inducing Activity of FAK Protein]

In vitro degradation inducing activity of FAK protein of compounds of Examples was evaluated by Western Blotting (WB).

### (Preparation of Test Substance Solution)

By a test compound, 10 mM DMSO solution was prepared, which was used as a 10 µM test compound solution. Further, the solution was diluted 10-fold with DMSO to prepare a 1 mM solution, which was used as a 1 µM test compound solution.

### (Test Method)

PA-1 cells (ATCC: CRL-1572, Lot: 70055709) were cultured in Eagle's Minimum Essential Medium supplemented with 10% FBS and 1% Penicillin/Streptomycin. The PA-1 cells seeded in a 24 well plate at 200,000 cells/well/1 mL were cultured for 24 hours, and then 1 µL of DMSO or a test compound solution was added thereto and treatment was performed for 24 hours. Cells were harvested and lysed for 20 minutes on ice using RIPA lysis buffer (Sigma, Cat#R0278) supplemented with protease inhibitor cocktail (Sigma, Cat#P8340; Roche, Cat#P11873580001), and phosphatase inhibitor cocktail (Sigma, Cat#P5726, P0044). The lysate was sonicated (15 seconds, amplitude 30%, 1 cycle) and then centrifuged at 12500 rpm for 15 minutes at 4°C. The protein concentration was determined by BCA assay kit (Thermo Fisher Scientific, Cat#23225).

### (Method for Evaluating Degradation Inducing Activity and Data Analysis)

The western blotting was performed using Simple Western JESS (protein simple). A cell lysis sample (protein amount; about 2 µg) was loaded on a plate of Simple Western JESS, and the expression level of ALK protein was detected using a 12-230 kDa Wes Separation Module (Protein simple, Cat#SM-WO04), an Anti-Rabbit Detection Module for Wes (Protein simple, Cat#DM-001), and a Total Protein Detection Module for Wes (Protein simple, Cat#DM-TP01). As the primary antibody, a material obtained by diluting FAK Rabbit mAb (Cell Signaling Technology, Cat#3285) at 1:100 was used, and as the secondary antibody, a material obtained by diluting HRP-labeled anti-rabbit IgG antibody (Cell Signaling Technology, Cat#7074S) at 1:250 was used. The expression level of the target protein was standardized using the total protein amount loaded as a reference. The standardized expression level data of the control (DMSO) was taken as 100%, and the degradation rate (%) was calculated from the average standardized expression level data of the test compounds (compounds of Examples 109 to 128). All analysis steps, such as data detection and analysis, are fully automated.

All of the compounds of Examples 110, 111, 112, 114, 115, 119, and 124 showed a FAK protein degradation rate of 75% or more at 10 µM. It was confirmed that other compounds degraded FAK protein at 10 µM. Therefore, it was confirmed that the compound of the present invention had not only an FAK kinase inhibitory activity but also an FAK protein degradation inducing activity.

### (Example A: Formulation Example)

The compounds of the present disclosure may be provided in appropriate dosage forms. Examples thereof will be described below.

Examples thereof include tablets, capsules, powders, granules, liquids, suspensions, injections, patches, and poultices. In addition, these formulations can be produced by a known method including appropriately mixing additives used as normal pharmaceutical additives.

### (Example B: Animal Experiment)

Candidate compounds of the present disclosure or a placebo were administered to Mice orally, by eye drop, intraperitoneally, intravenously, intravitreally or intracamerally for up to 28 days.

Subsequently, a comparison between the placebo and the compound can be performed to evaluate the efficacy of the compound of the present disclosure in a mouse body.

### (Example C: Clinical Trial)

The compounds of the present disclosure can be used orally or instilled by eye and can provide such therapeutic or prophylactic embodiments.

Therefore, the effect can be confirmed by selecting a patient who has developed a disease and administering the compound. The prophylactic effect can also be tested as well.

### (Note)

As disclosed above, the present disclosure is exemplified by the use of its preferred embodiments. However, it is understood that the scope of the present disclosure should be interpreted based solely on the Claims. It is also understood that any patent, any patent application, and any other references cited herein should be incorporated herein by reference in the same manner as the contents are specifically described herein.

### [Industrial Applicability]

The technology provided in this disclosure can be used in the manufacturing industry for targeted proteolytic agents using IAP ligands.

## Claims

1. A compound represented by Formula (A) Z-L-X or an isomer thereof, or a salt thereof:
wherein
Z is a ligand moiety capable of specifically binding to an intracellular protein,
L is a linker moiety or a single bond, and
X is the following formula 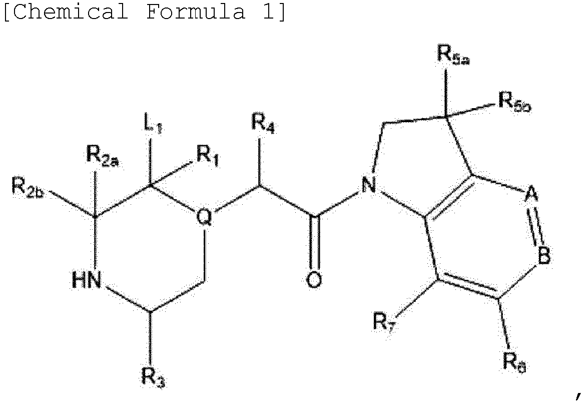 wherein
A is N or CR₈,
B is N, CR₉, or CL₂,
Q is N or CR₁₀,
R₁, and L₁ not used for bonding to L, are each independently selected from hydrogen, C₁₋₆ alkyl, C₂₋₆ alkenyl, C₂₋₆ alkynyl, - C(=O)NH_{(2-q)}(C₁₋₆alkyl)_{q}, - (CH₂)ₛ- (3- to 12-membered heterocyclyl), -(CH₂)ₛ-C₃₋₁₂ carbocyclyl, -C(=O)-(3- to 12-membered heterocyclyl), and -C(=O)-C₃₋₁₂ carbocyclyl, or R₁, and L₁ not used for bonding to L, together with the carbon atom to which they are attached, can join to form a 3-to 10-membered saturated carbocyclyl or heterocyclyl group, wherein the C₁₋₆ alkyl, C₂₋₆ alkenyl, C₂₋₆ alkynyl, heterocyclyl and carbocyclyl groups are optionally substituted with one or more R^{b} groups;
R₂ₐ and R_{2b} are each independently selected from hydrogen, C₁₋₆ alkyl, C₂₋₆ alkenyl, C₂₋₆ alkynyl, -C (=O) NH_{(2-q)}(C₁₋₆ alkyl)_{q}, - (CH₂)ₛ- (3- to 12-membered heterocyclyl), and - (CH₂)ₛ-C₃₋₁₂ carbocyclyl,
or R₂ₐ and R_{2b}, together with the carbon atom to which they are attached, can join to form a 3- to 10-membered saturated carbocyclyl or heterocyclyl group, wherein the C₁₋₆ alkyl, C₂₋₆ alkenyl, C₂₋₆ alkynyl, heterocyclyl and carbocyclyl groups are optionally substituted with one or more R^{b} groups;
R³ is selected from C₁₋₄ alkyl, C₂₋₄ alkenyl and - (CH₂)ₛ-C₃₋₈ cycloalkyl, wherein the C₁₋₄ alkyl, C₂₋₄ alkenyl, and C₃₋₈ cycloalkyl are optionally substituted with one or more R^{a} groups;
R² is selected from halogen, -OH and -O-C₁₋₆ alkyl;
R₄ is selected from hydrogen, C₁₋₆ alkyl, C₂₋₆ alkenyl, C₂₋₆ alkynyl, - (CH₂)ₛ-C₃₋₈ cycloalkyl and - (CH₂)ₛ-C₃₋₈ cycloalkenyl, wherein the C₁₋₆ alkyl, C₂₋₆ alkenyl, C₂₋₆ alkynyl, C₃₋₈ cycloalkyl and C₃₋₈ cycloalkenyl are optionally substituted with one or more R² groups;
R₅ₐ and R_{5b} are each independently selected from hydrogen, C₁₋₈ alkyl, C₂₋₈ alkenyl, C₂₋₈ alkynyl, -Y-C₃₋₁₂ carbocyclyl, -W-(3- to 12-membered heterocyclyl), -(CR^{x}R^{y})ₛ-O-R^{z}, -O-(CR^{x}R^{y})ₙ-OR^{z}, - (CH₂)ₛ-CN, -S(O)_{q}-R^{×}, -C(=O)R^{x}, -C(=S)R^{x}, -C(=N)R^{x}, - (CRₓR_{y})ₛ-C(=O) OR^{z}, - (CR^{×}R^{y})ₛ-O-C(=O)-R^{z}, - (CR^{×}R^{y})ₛ-C (=O) NR^{x}R^{y}, - (CH₂)ₛ-NR^{×}C (=O)R^{y}, - (CH₂)ₛ-OC (=O)NR^{×}R^{y}, - (CH₂)ₛ-NR^{×}C(=O) OR^{y}, - (CH₂)ₛ-NR^{x}R^{y}, -NR^{×}- (CH₂)ₛ-R^{z}, - (CR^{×}R^{y}) ₛ-C(=S) NR^{z}, - (CR^{×}R^{y}) ₛ-C(=N) NR^{z}, - (CH₂)ₛ-O-C(=O) -C₁₋₄ alkyl-NR^{×}R^{y}, - (CH₂)ₛ-NR^{x}- (CH₂)ₙ-O-C (=O) -R^{z}, - (CH₂)ₛ-NR^{×}- (CH₂)ₑ-SO₂-R^{y}, - (CH₂)ₛ-NH-SO₂-NR^{×}R^{y} and - (CH₂)ₛ-SO₂NR^{×}R^{y} groups,
or R₅ₐ and R_{5b}, together with the carbon atom to which they are attached, can join to form a 3- to 6-membered carbocyclyl or 4-to 6-membered heterocyclyl group,
wherein the C₁₋₈ alkyl, C₂₋₈ alkenyl and C₂₋₈ alkynyl groups are optionally substituted with one or more R^{b} groups, and wherein the carbocyclyl group and heterocyclyl group are optionally substituted with one or more R^{b} groups;
R₆ is selected from hydrogen, halogen, C₁₋₈ alkyl, C₂₋₈ alkenyl, C₂₋₈ alkynyl, -Y-C₃₋₁₂ carbocyclyl, -W- (3- to 12-membered heterocyclyl), -(CR^{×}R^{y})ₛ-O-R^{z}, -O-(CR^{×}R^{y})ₙ-OR^{z}, =O, =S, nitro, Si(R^{×})₄, - (CH₂)ₛ-CN, -S(O)_{q} (CR^{×}R^{y}) ₛ-R^{z}, -C(=O)R^{×}, -C(=S)R^{×}, - C(=N)R^{x}, - (CR^{x}R^{y})ₛ-C(=O)OR^{z}, -(CR^{x}R^{y})ₛ-O-C(=O) -R^{z}, -(CR^{x}R^{y})ₛ-C(=O)NR^{×}R^{y}, - (CH₂)ₛ-NR^{×}C(=O)R^{y}, - (CH₂)ₛ-OC(=O)NR^{×}R^{y}, -(CH₂)ₛ-NR^{x}C(=O)OR^{y}, - (CH₂)ₛ-NR^{×}R^{y}, -NR^{x}- (CH₂)ₛ-R^{z}, - (CR^{x}R^{y})ₛ-C(=S)NR^{z}, - (CR^{x}R^{y})ₛ-C(=N)NR^{×}, -S(O)(=NR^{x})R^{y}, - (CH₂)ₛ-O-C(=O)-C₁₋₄ alkyl-NR^{×}R^{y}, -(CH₂)ₛ-NR^{x}-(CH₂)ₙ-O-C(=O)-R^{z}, - (CH₂)ₛ-NR^{×}- (CH₂)ₑ-SO₂-R^{y}, - (CH₂)ₛ-NH-SO₂-NR^{×}R^{Y} and - (CH₂)ₛ-SO₂NR^{×}R^{y} groups, -P(=O)(R^{x})₂, -Y-C₅₋₁₀ aryl, -Y-(5- to 10-membered heteroaryl), an optionally fused 5-to 10-membered carbocyclyl group, an optionally fused 5- to 10-membered heterocyclyl group, and 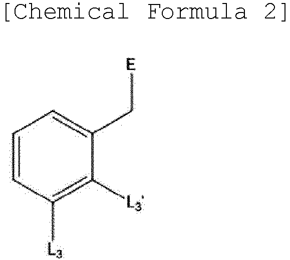
wherein E indicates a bonding point,
L₃ and L₃', together with the carbon atoms to which they are attached, can join to form a 3- to 6-membered carbocyclyl or 4-to 6-membered heterocyclyl group connected to a linker, wherein the C₁₋₈ alkyl, C₂₋₈ alkenyl and C₂₋₈ alkynyl groups, carbocyclyl group and heterocyclyl group, and 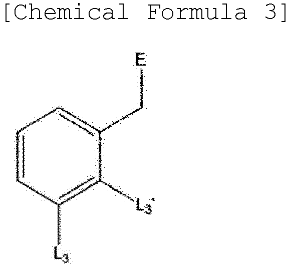
wherein E indicates a bonding point, are optionally substituted with one or more R^{b} groups;
R₇, R₈ and R₉ are each independently selected from hydrogen, halogen, C₁₋₈ alkyl, C₂₋₈ alkenyl, C₂₋₈ alkynyl, -Y-C₃₋₁₂ carbocyclyl, -W-(3-to 12-membered heterocyclyl), -(CR^{×}R^{y})ₛ-OR^{z}, -O-(CR^{×}R^{y})ₙ-OR^{z}, =O, =S, nitro, Si(R^{x})₄, - (CH₂)ₛ-CN, -S(O)_{q}-(CR^{x}R^{y})ₛ-R^{z}, -C(=O)R^{x}, -C(=S)R^{x}, -C(=N)R^{×}, -(CR^{×}R^{y})ₛ-C(=O)OR^{z}, - (CR^{×}R^{y}) ₛ-O-C(=O) -R^{z}, - (CR^{×}R^{y}) ₛ-C(=O)NR^{x}R^{y}, - (CH₂)ₛ-NR^{x}C (=O)R^{y}, - (CH₂)ₛ-OC(=O)NR^{×}R^{y}, - (CH₂)ₛ-NR^{x}C(=O)OR^{y}, -(CH₂)ₛ-NR^{×}R^{y}, -NR^{x}-(CH₂)ₛ-R^{z}, -(CR^{x}R^{y})ₛ-C(=S)NR^{z}, -(CR^{×}R^{y})ₛ-C(=N)NR^{×}, -S(O)(=NR^{x})R^{y}, -(CH₂)ₛ-O-C(=O)-C₁₋₄ alkyl-NR^{×}R^{y}, - (CH₂)ₛ-NR^{x}-(CH₂)ₙ-O-C(=O) -R^{z}, - (CH₂)ₛ-NR^{x}-(CH₂)ₑ-SO₂-R^{y}, - (CH₂)ₛ-NH-SO₂-NR^{x}R^{y} and - (CH₂)ₛ-SO₂NR^{×}R^{y} groups, -P(=O)(R^{x})₂ and -Y-C₅₋₁₀ aryl, wherein the C₁₋₈ alkyl, C₂₋₈ alkenyl and C₂₋₈ alkynyl groups are optionally substituted with one or more R^{b} groups, and wherein the carbocyclyl and heterocyclyl groups are optionally substituted with one or more (e.g., 1, 2 or 3) R^{b} group;
each R^{b} is independently selected from halogen, C₁₋₆ alkyl, C₂₋₆ alkenyl, C₂₋₆ alkynyl, - (CH₂)ₛ-C₃₋₈ cycloalkyl, - (CH₂)ₛ-C₃₋₈ cycloalkenyl, -(CH₂)ₛ-phenyl, -(CH₂)ₛ-(5- to 10-membered heteroaryl), -(CH₂)ₑ-(4- to 7-membered saturated heterocyclyl), - (CR^{×}R^{y})ₛ-O-R^{z}, -O- (CR^{×}R^{y})ₙ-OR^{z}, haloC₁₋₆ alkyl, haloC₁₋₆ alkoxy, hydroxy, hydroxyC₁₋₆ alkyl, =O, =S, nitro, Si(Rx)₄, -(CH₂)ₛ-CN, - S(O)_{q}-R^{×}, -C(=O)R^{x}, -(CR^{x}R^{y})ₛ-C(=O)OR^{z}, -(CR^{x}R^{y})ₛ-O-C(=O) -R^{z}, - (CR^{x}R^{y})ₛ-C(=O)NR^{x}R^{y}, -(CH₂)ₛ-NR^{×}C(=O)R^{y}, -(CH₂)ₛ-OC (=O)NR^{×}R^{y}, - (CH₂)ₛ-NR^{×}C(=O)OR^{y}- (CH₂)ₛ-NR^{×}R^{y}, -NR^{x}-(CH₂)ₛ-R^{z}, - (CH₂)ₛ-O-C(=O)-C₁-₄ alkyl-NR^{×}R^{y}, - (CH₂)ₛ-NR^{×}- (CH₂)ₙ-O-C (=O) -R^{z}, - (CH₂)ₛ-NR^{×}- (CH₂)ₛ-SO₂-R^{y}, - (CH₂)ₛ-NH-SO₂-NR^{×}R^{y} and -(CH₂)ₛ-SO₂NR^{x}R^{y} groups and - P(=O)(R^{x})₂, wherein the C₁₋₆ alkyl, C₂₋₆ alkenyl, C₂₋₆ alkynyl, C₃₋₈ cycloalkyl, C₃₋₈ cycloalkenyl and heterocyclyl groups, phenyl and heteroaryl are optionally substituted with one or more R^{×} groups;
R^{x}, R^{y} and R^{z} are each independently selected from halogen, hydrogen, C₁₋₆ alkyl, C₂₋₆ alkenyl, C₂₋₆ alkynyl, - (CH₂)ₛ-C₃₋₈ cycloalkyl, -(CH₂)ₛ-C₃₋₈ cycloalkenyl, -(CH₂)ₛ-phenyl, -(CH₂)ₛ- (4-to 7-membered saturated heterocyclyl), hydroxyC₁₋₆ alkyl optionally substituted with one or more halo, -C(=O)OC₁₋₆ alkyl, hydroxy, C₁₋₆ alkoxy, haloC₁₋₆ alkyl, - (CH₂)ₙ-O-C₁₋₆ alkyl, - C(=O)-(CH₂)ₙ-C₁₋₆ alkoxy, -C(=O)-C₁₋₆ alkyl, -(CH₂)ₛ-CN, C₁₋₆ alkyl-N(H)_{2-q} (C₁₋₆ alkyl)_{q}, -N(H)_{2-q}(C₁₋₆alkyl)_{q}, -C(=O)-N(H)_{2-q}(C₁-₆alkyl)_{q}, -(CH₂)ₑ-NH-SO₂-N(H)_{2-q}(C₁₋₈ alkyl)_{q}, - (CH₂)ₛ-N(C₁₋₄ alkyl)-SO₂-N(H)_{2-q}(C₁₋₆alkyl)_{q} and - (CH₂)ₛ-O-C(=O) -C₁₋₄ alkyl-N(H)_{2-q}(C₁₋₈ alkyl)_{q}, and when attached to nitrogen, carbon, silicon or phosphorus atom, R^{x} and R^{y}, together with the atom(s) to which they are attached, may join to form a 3- to 7-membered ring optionally containing one or two additional heteroatoms selected from O, N, S and oxidized forms of N and S;
Y and W are each independently selected from a bond, -(CR^{x}R^{y})ₘ-, -C(=CR^{×})-, -C(=O)-, -NR^{x}, -C(=O)NR^{x}-, -NR^{x}C(=O)-, -(CR^{x}R^{y})_{q}-O-, - O-(CR^{x}R^{y})_{q}-, -S(O)₂-NH, NH-S(O)₂- and -S(O)_{q}-;
R₁₀ is selected from hydrogen, C₁₋₆ alkyl, C₂₋₆ alkenyl, C₂₋₆ alkynyl, -C(=O)NH_{(2-q)}(C₁₋₈ alkyl)_{q}, -(CH₂)ₛ-(3- to 12-membered heterocyclyl), -(CH₂)ₛ-C₃₋₁₂ carbocyclyl, -C(=O)-(3-to 12-membered heterocyclyl), and -C(=O)-C₃₋₁₂ carbocyclyl, wherein the C₁₋₆ alkyl, C₂₋₆ alkenyl, C₂₋₆ alkynyl, heterocyclyl and carbocyclyl groups are optionally substituted with one or more R^{b} groups;
s independently represents an integer of 0 to 4;
n independently represents an integer of 1 to 4;
q independently represents an integer of 0 to 2;
m represents an integer of 1 to 2, and
x is bonded to L at any one of L₁ to L₃ and L₃' .

2. The compound or the isomer thereof or the salt thereof of claim 1, wherein the intracellular protein in Z is a serin/threonine kinase, a receptor tyrosine kinase, a non-receptor tyrosine kinase, or a non-kinase.

3. The compound or the isomer thereof or the salt thereof of claim 1, which has an inhibitory activity or binding activity against the intracellular protein in Z.

4. The compound or the isomer thereof or the salt thereof of any one of claims 1 to 3, wherein the intracellular protein in Z is a serin/threonine kinase, and the serin/threonine kinase is ROCK2.

5. The compound or the isomer thereof or the salt thereof of any one of claims 1 to 3, wherein the intracellular protein in Z is a receptor tyrosine kinase, and the receptor tyrosine kinase is ALK.

6. The compound or the isomer thereof or the salt thereof of any one of claims 1 to 3, wherein the intracellular protein in Z is a non-receptor tyrosine kinase, and the non-receptor tyrosine kinase is FAK.

7. The compound or the isomer thereof or the salt thereof of any one of claims 1 to 6, wherein X is connected to L at either L₁, L₂, L₃ or L₃' .

8. The compound or the isomer thereof or the salt thereof of any one of claims 1 to 7, wherein Q is N.

9. The compound or the isomer thereof or the salt thereof of any one of claims 1 to 8, wherein Q is CH.

10. The compound or the isomer thereof or the salt thereof of any one of claims 1 to 9, wherein A is N.

11. The compound or the isomer thereof or the salt thereof of any one of claims 1 to 10, wherein A is CH.

12. The compound or the isomer thereof or the salt thereof of any one of claims 1 to 11, wherein B is N.

13. The compound or the isomer thereof or the salt thereof of any one of claims 1 to 12, wherein B is CH.

14. The compound or the isomer thereof or the salt thereof of any one of claims 1 to 13, wherein
R₁, and L₁ not used for bonding to L, are each independently selected from hydrogen, C₁₋₆ alkyl, C₂₋₆ alkenyl, C₂₋₆ alkynyl, - C(=O)NH_{(2-q)}(C₁₋₆ alkyl)_{q}, -(CH₂)ₛ-(3- to 7-membered heterocyclyl), -(CH₂)ₛ-C₃₋₇ carbocyclyl, -C(=O)-(3- to 7-membered heterocyclyl), and -C(=O)-C₃₋₇ carbocyclyl, or R₁, and L₁ not used for bonding to L, together with the carbon atom to which they are attached, can join to form a 3- to 6-membered saturated carbocyclyl or 4- to 6-membered heterocyclyl group, wherein the C₁₋₆ alkyl, C₂₋₆ alkenyl, C₂₋₆ alkynyl, heterocyclyl and carbocyclyl groups are optionally substituted with one or more R^{b} groups,
each R^{b} is independently selected from halogen, C₁₋₄ alkyl, C₃₋₄ alkynyl, -(CH₂)_{q}-C₃₋₆ cycloalkyl, - (CH₂)_{q}-phenyl, -(CH₂)_{q}-(5- to 6-membered heteroaryl), - (CH₂)_{q}-O-C₁₋₄ alkyl, haloC₁₋₄ alkyl, haloC₁₋₄ alkoxy, hydroxy, hydroxyC₁₋₄ alkyl, =O, -S(O)_{q}-C₁₋₄ alkyl, -C(=O)C₁₋₄ alkyl, -(CH₂)_{q}-C(=O)NR^{×}R^{y}, - (CH₂)_{q}-NR^{x}C (=O) C₁₋₄ alkyl, -(CH₂)ₛ-NR^{×}R^{y}, -NR^{×}-(CH₂)_{q}-C₁₋₄ alkyl, - (CH₂)_{q}-NR^{x}-(CH₂)_{q}-SO₂-C₁₋₄ alkyl, and - (CH₂)_{q}-SO₂NR^{x}R^{y} groups, wherein the cycloalkyl, phenyl and heteroaryl groups are optionally substituted with one or more R^{x} groups, and
R^{x} and R^{y} are each independently selected from halogen, hydrogen, C₁₋₄ alkyl, and C₁₋₄ alkoxy, and when attached to nitrogen or carbon, R^{x} and R^{y}, together with the atom(s) to which they are attached, may join to form a 3- to 7-membered ring optionally containing one additional heteroatom selected from O and N.

15. The compound or the isomer thereof or the salt thereof of any one of claims 1 to 14, wherein
R₁, and L₁ not used for bonding to L, are each independently selected from hydrogen, C₁₋₆ alkyl, C₂₋₆ alkenyl, C₂₋₆ alkynyl, - C(=O)NH_{(2-q)}(C₁₋₆ alkyl)_{q}, -(CH₂)ₛ-(3- to 7-membered heterocyclyl), -(CH₂)ₛ-C₃₋₇ carbocyclyl, -C(=O)-(3-to 7-membered heterocyclyl), and -C(=O)-C₃₋₇ carbocyclyl, wherein the C₁₋₆ alkyl, C₂₋₆ alkenyl, C₂₋₆ alkynyl, heterocyclyl and carbocyclyl groups are optionally substituted with one or more R^{b} groups,
each R^{b} is independently selected from fluorine, methyl, C₃₋₆ cycloalkyl, phenyl, 5- to 6-membered heteroaryl, -O-C₁₋₂ alkyl, fluoromethyl, fluoromethoxy, hydroxy, =O, -S(O)₂-C₁₋₄ alkyl, - C(=O)C₁₋₄ alkyl, -C(=O)NR^{x}R^{y}, -NR^{x}C(=O)C₁₋₄ alkyl, and -NR^{x}R^{y} groups, wherein the cycloalkyl, phenyl and heteroaryl groups are optionally substituted with one or more R^{x} groups, and
R^{x} and R^{y} are each independently selected from chlorine, fluorine, hydrogen, C₁₋₄ alkyl, and C₁₋₄ alkoxy, and when attached to nitrogen or carbon, R^{×} and R^{y}, together with the atom(s) to which they are attached, may join to form a 3- to 7-membered ring optionally containing one additional heteroatom selected from O and N.

16. The compound or the isomer thereof or the salt thereof of any one of claims 1 to 15, wherein R₂ₐ and R_{2b} are each independently selected from hydrogen, C₁₋₈ alkyl, C₂₋₃ alkenyl and C₂₋₃ alkynyl, or R₂ₐ and R_{2b}, together with the carbon atom to which they are attached, can join to form a 3- to 4-membered saturated carbocyclyl group.

17. The compound or the isomer thereof or the salt thereof of any one of claims 1 to 16, wherein R₂ₐ and R_{2b} are hydrogen.

18. The compound or the isomer thereof or the salt thereof of any one of claims 1 to 17, wherein R³ is C₁₋₄ alkyl, wherein the C₁₋₄ alkyl is optionally substituted with one or more R^{a} groups.

19. The compound or the isomer thereof or the salt thereof of any one of claims 1 to 18, wherein R³ is methyl, wherein the methyl is optionally substituted with one or more fluorine.

20. The compound or the isomer thereof or the salt thereof of any one of claims 1 to 19, wherein R₄ is hydrogen.

21. The compound or the isomer thereof or the salt thereof of any one of claims 1 to 20, wherein
R₅ₐ and R_{5b} are each independently selected from hydrogen, C₁₋₈ alkyl, C₂₋₈ alkenyl, C₂₋₈ alkynyl, -Y-C₃₋₆ carbocyclyl, -W-(3- to 6-membered heterocyclyl), -(CR^{x}R^{y})ₛ-O-R^{z}, -O- (CR^{×}R^{y}) ₙ-OR^{z}, - (CH₂)ₛ-CN and -C(=O)R^{x}, or R₅ₐ and R_{5b}, together with the carbon atom to which they are attached, can join to form a 3- to 5-membered carbocyclyl or 5- to 6-membered heterocyclyl group, wherein the C₁₋₈ alkyl, C₂₋₈ alkenyl and C₂₋₈ alkynyl groups are optionally substituted with one or more R^{b} groups, and
each R^{b} is independently selected from fluorine, C₁₋₄ alkyl, C₃₋₄ alkynyl, -C₃₋₆ cycloalkyl, -O-C₁₋₄ alkyl, fluoroC₁₋₄ alkyl, haloC₁₋₄ alkoxy, hydroxy, hydroxyC₁₋₄ alkyl, -C(=O)NR^{x}R^{y}, - NR^{x}C(=O)C₁₋₄ alkyl, and -NR^{x}R^{y} groups.

22. The compound or the isomer thereof or the salt thereof of any one of claims 1 to 21, wherein R₅ₐ and R_{5b} are each independently hydrogen or methyl, or R₅ₐ and R_{5b}, together with the carbon atom to which they are attached, can join to form a cyclopropyl group.

23. The compound or the isomer thereof or the salt thereof of any one of claims 1 to 22, wherein
R₆ is selected from hydrogen, halogen, C₁₋₄ alkyl, C₂₋₅ alkenyl, C₂₋₅ alkynyl, -(CR^{×}R^{y})-C₃₋₇ carbocyclyl, -(CR^{x}R^{y})-(3- to 7-membered heterocyclyl), -(CR^{x}R^{y})-O-R^{z}, -O-(CR^{x}R^{y})ₙ-OR^{z}, -(CH₂)ₛ-CN, -S(O)₂-(CH₂)ₛ-R^{z}, -C(=O)R^{x}, -(CH₂)ₛ-NR^{x}R^{y}, -NR^{×}- (CH₂)ₛ-R^{z}, - (CH₂)ₙ-C₅₋₁₀ aryl, -(CH₂)ₙ-(5- to 10-membered heteroaryl), an optionally fused 5- to 7-membered carbocyclyl group, an optionally fused 5- to 7-membered heterocyclyl group, and 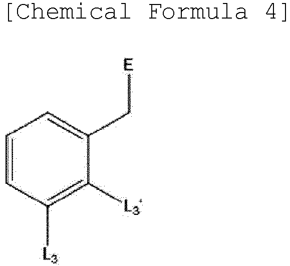
wherein E indicates a bonding point,
L₃ and L₃', together with the carbon atoms to which they are attached, can join to form a 3- to 6-membered carbocyclyl or 4-to 6-membered heterocyclyl group that can be connected to a linker, wherein the C₁₋₄ alkyl, C₂₋₅ alkenyl and C₂₋₅ alkynyl groups, carbocyclyl group, heterocyclyl group, aryl group,
heteroaryl group, and 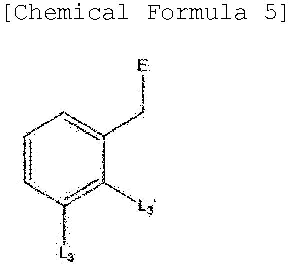
wherein E indicates a bonding point, are optionally substituted with one or more R^{b} groups,
each R^{b} is independently selected from halogen, C₁₋₄ alkyl, C₃₋₄ alkynyl, -(CH₂)_{q}-C₃₋₆ cycloalkyl, -(CH₂)_{q}-phenyl, -(CH₂)_{q}-(5- to 6-membered heteroaryl), - (CH₂)_{q}-O-C₁₋₄ alkyl, haloC₁₋₄ alkyl, haloC₁₋₄ alkoxy, hydroxy, hydroxyC₁₋₄ alkyl, =O, -S(O)_{q}-C₁₋₄ alkyl, -C(=O)C₁₋₄ alkyl, -(CH₂)_{q}-C(=O)NR^{x}R^{y}, -(CH₂)_{q}-NR^{x}C(=O)C₁₋₄ alkyl, -(CH₂)ₛ-NR^{×}R^{y}, -NR^{x}-(CH₂)_{q}-C₁₋₄ alkyl, -(CH₂)_{q}-NR^{x}-(CH₂)_{q}-SO₂-C₁₋₄ alkyl, and -(CH₂)_{q}-SO₂NR^{×}R^{y} groups, wherein the cycloalkyl, phenyl and heteroaryl groups are optionally substituted with one or more R^{x} groups,
the CR^{x}R^{y} is CH₂ or CF₂, and the other R^{x}, R^{y} and R^{z} are each independently selected from hydrogen, C₁₋₄ alkyl, C₃₋₄ alkynyl, fluoroC₁₋₄ alkyl, and C₁₋₄ alkoxy, and
the R^{x} group as a substituent of R^{b} is selected from halogen, hydrogen, C₁₋₄ alkyl, and C₁₋₄ alkoxy.

24. The compound or the isomer thereof or the salt thereof of any one of claims 1 to 23, wherein
R₆ is selected from -(CR^{×}R^{y})-H, -(CR^{x}R^{y})-F, -(CR^{x}R^{y})-C₁₋₂ alkyl, - (CR^{×}R^{y}) -O-phenyl, -S(O)₂-CH₂-R^{z}, -C(=O)C₁₋₄ alkyl, -C(=O) -phenyl, -C(=O)-(5-6-membered heteroaryl), -CH₂-phenyl, -CH₂-(5-6-membered heteroaryl), and 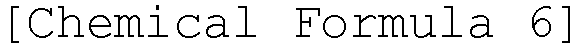 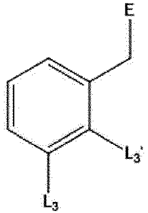
wherein E indicates a bonding point,
L₃ and L₃', together with the carbon atoms to which they are attached, can join to form a 3- to 6-membered carbocyclyl or 4-to 6-membered heterocyclyl group that can be connected to a linker, wherein the C₁₋₂ alkyl, C₁₋₄ alkyl and phenyl groups, heteroaryl group, and 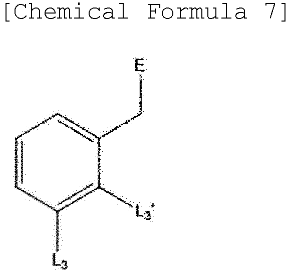
wherein E indicates a bonding point, are optionally substituted with one or more C₁₋₈ alkyl groups, fluorine, chlorine, or -O-C₁₋₈ alkyl groups, and
the CR^{x}R^{y} is CH₂ or CF₂, and the other R^{x}, R^{y} and R^{z} are each independently selected from hydrogen, C₁₋₄ alkyl, C₃₋₄ alkynyl, fluoroC₁₋₄ alkyl, and C₁₋₄ alkoxy.

25. The compound or the isomer thereof or the salt thereof of any one of claims 1 to 24, wherein
R₇ is selected from hydrogen, halogen, C₁₋₈ alkyl, C₂₋₃ alkenyl, C₂₋₃ alkynyl and C₃₋₄ carbocyclyl, wherein the C₁₋₈ alkyl, C₂₋₃ alkenyl, C₂₋₃ alkynyl and C₃₋₄ carbocyclyl are optionally substituted with one or more R^{b} groups,
each R^{b} is independently selected from fluorine, -O-C₁₋₄ alkyl, fluoroC₁₋₄ alkoxy, hydroxy, and -NR^{x}R^{y} groups, and
R^{x} and R^{y} are each independently selected from hydrogen, and C₁-₄ alkyl, and when attached to nitrogen, R^{x} and R^{y}, together with the atom(s) to which they are attached, may join to form a 4-to 6-membered ring optionally containing one additional heteroatom selected from O and N.

26. The compound or the isomer thereof or the salt thereof of any one of claims 1 to 25, wherein R₇ is hydrogen or fluorine.

27. The compound or the isomer thereof or the salt thereof of any one of claims 1 to 26, wherein R₈ is hydrogen.

28. The compound of any one of claims 1 to 27, wherein
R₉ is selected from hydrogen, halogen, C₁₋₄ alkyl, C₂₋₄ alkenyl, C₂₋₄ alkynyl, -Y-C₃₋₆ carbocyclyl, -W-(4- to 6-membered heterocyclyl), -(CR^{x}R^{y})ₛ-O-R^{z}, -O-(CR^{x}R^{y})ₙ-OR^{z}, =O, -(CH₂)ₛ-CN, - C(=O)R^{×}, -(CR^{x}R^{y})ₛ-C(=O)NR^{x}R^{y}, -(CH₂)ₛ-NR^{x}C(=O)R^{y}, -(CH₂)ₛ-NR^{×}R^{y}, - (CH₂)ₛ-O-C(=O)-C₁₋₄ alkyl-NR^{×}R^{y}, - (CH₂)ₛ-NR^{x}-(CH₂)ₛ-SO₂-R^{y}, -(CH₂)ₛ-NH-SO₂-NR^{×}R^{y}, -(CH₂)ₛ-SO₂NR^{x}R^{y}, and -Y-C₅₋₁₀ aryl, wherein the C₁₋₄ alkyl, C₂₋₄ alkenyl, C₂₋₄ alkynyl, carbocyclyl and heterocyclyl groups are optionally substituted with one or more R^{b} groups, each R^{b} is independently selected from fluorine, C₁₋₄ alkyl, C₃₋₄ alkynyl, C₃₋₆ cycloalkyl, phenyl, 5- to 6-membered heteroaryl, - O-C₁₋₄ alkyl, fluoroC₁₋₄ alkyl, fluoroC₁₋₄ alkoxy, hydroxy, hydroxyC₁₋₄ alkyl, =O, -C(=O)C₁₋₄ alkyl, -C(=O)NR^{x}R^{y}, -NR^{x}C(=O)C₁₋₄ alkyl, and -NR^{x}R^{y} groups, wherein the cycloalkyl, phenyl and heteroaryl groups are optionally substituted with one or more R^{x} groups, and
R^{x}, R^{y} and R^{z} are each independently selected from chlorine, fluorine, hydrogen, C₁₋₄ alkyl, and C₁₋₄ alkoxy, and when attached to nitrogen or carbon, R^{×} and R^{y}, together with the atom(s) to which they are attached, may join to form a 3- to 7-membered ring optionally containing one additional heteroatom selected from O and N.

29. The compound of any one of claims 1 to 28, wherein
R₉ is selected from hydrogen, C₁₋₄ alkyl, C₂₋₄ alkenyl, C₂₋₄ alkynyl, -CH₂-C₃₋₆ carbocyclyl, -CH₂-(4- to 6-membered heterocyclyl), -CH₂-O-R^{z}-C(=O)R^{x}, and -CH₂-NR^{x}R^{y}, wherein the C₁₋₄ alkyl, C₂₋₄ alkenyl, C₂₋₄ alkynyl, carbocyclyl and heterocyclyl groups are optionally substituted with one or more R^{b} groups, each R^{b} is independently selected from fluorine, C₁₋₄ alkyl, C₃₋₄ alkynyl, phenyl, 5- to 6-membered heteroaryl, -O-C₁₋₄ alkyl, fluoroC₁₋₄ alkyl, fluoroC₁₋₄ alkoxy, hydroxy, and -NR^{x}R^{y} groups, wherein the phenyl and heteroaryl groups are optionally substituted with one or more R^{x} groups, and
R^{x}, R^{y} and R^{z} are each independently selected from chlorine, fluorine, hydrogen, C₁₋₄ alkyl, and C₁₋₄ alkoxy, and when attached to nitrogen or carbon, R^{x} and R^{y}, together with the atom(s) to which they are attached, may join to form a 3- to 7-membered ring optionally containing one additional heteroatom selected from O and N.

30. The compound of any one of claims 1 to 29, wherein R₁₀ is hydrogen or methyl.

31. The compound of any one of claims 1 to 30, which is represented by the following structure:

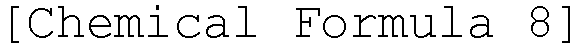

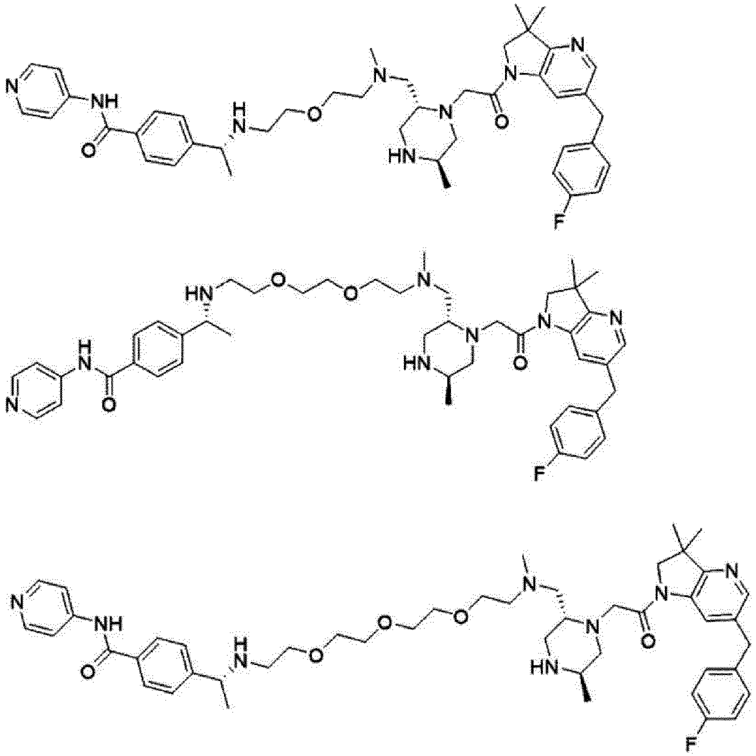

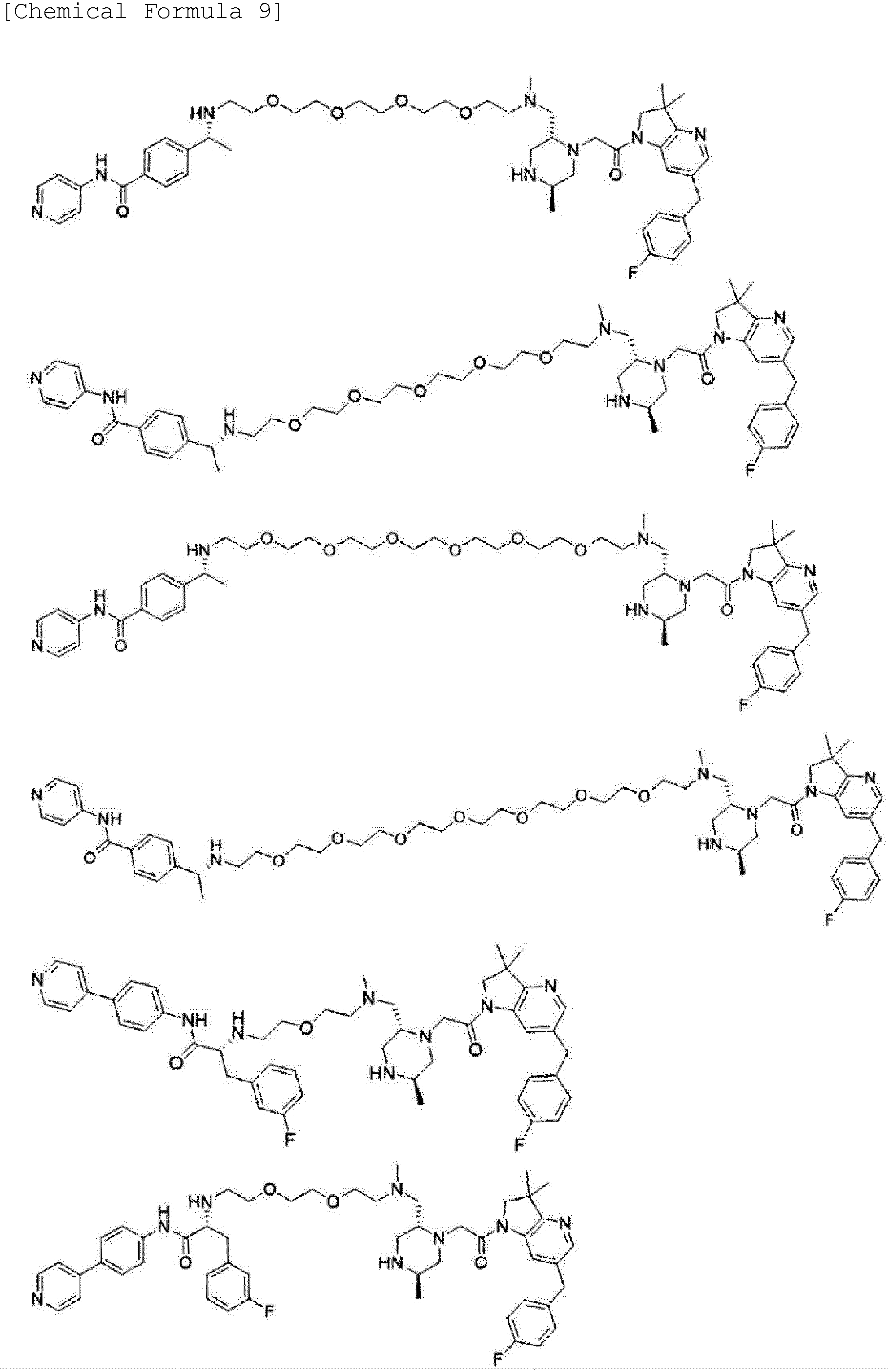

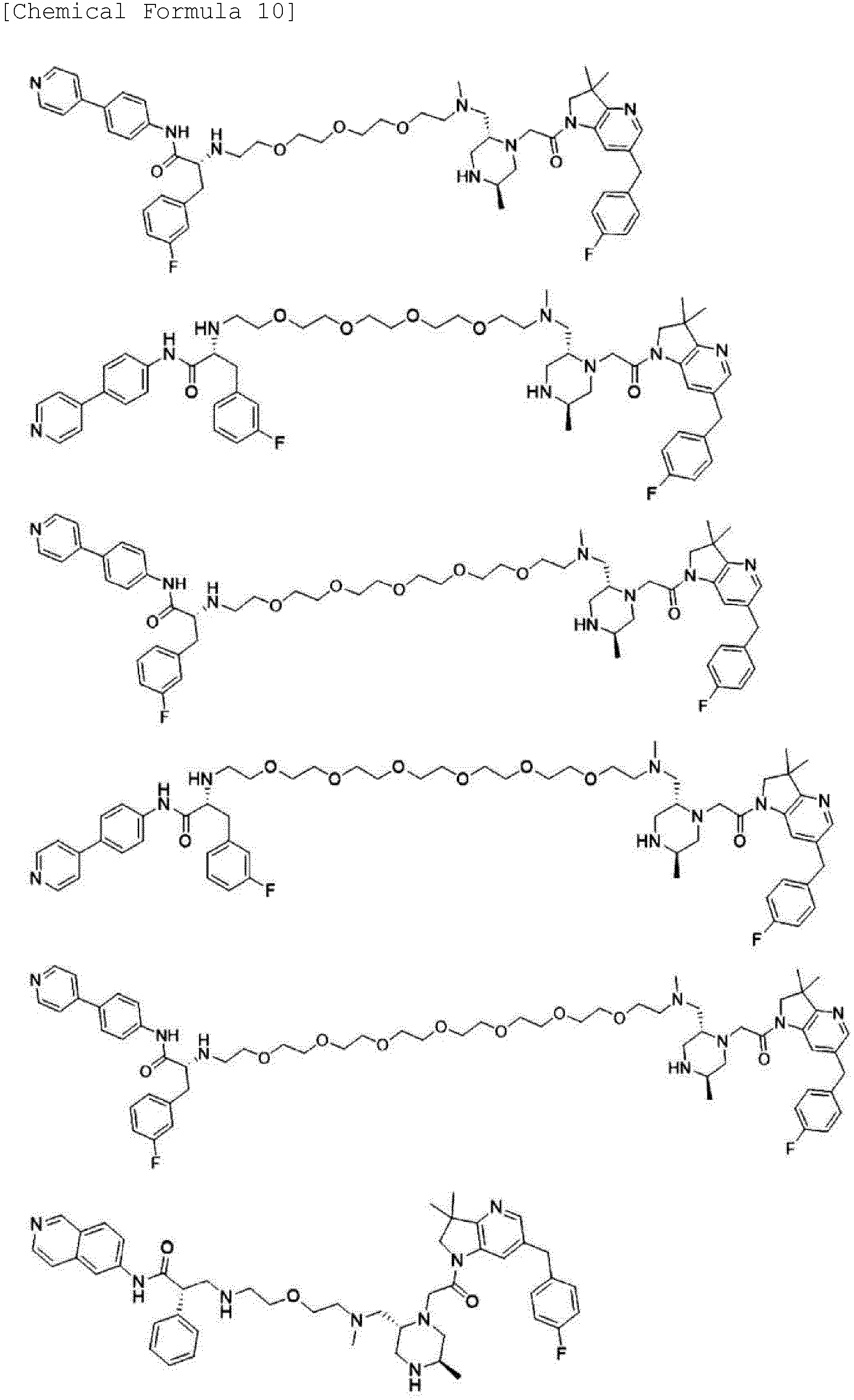

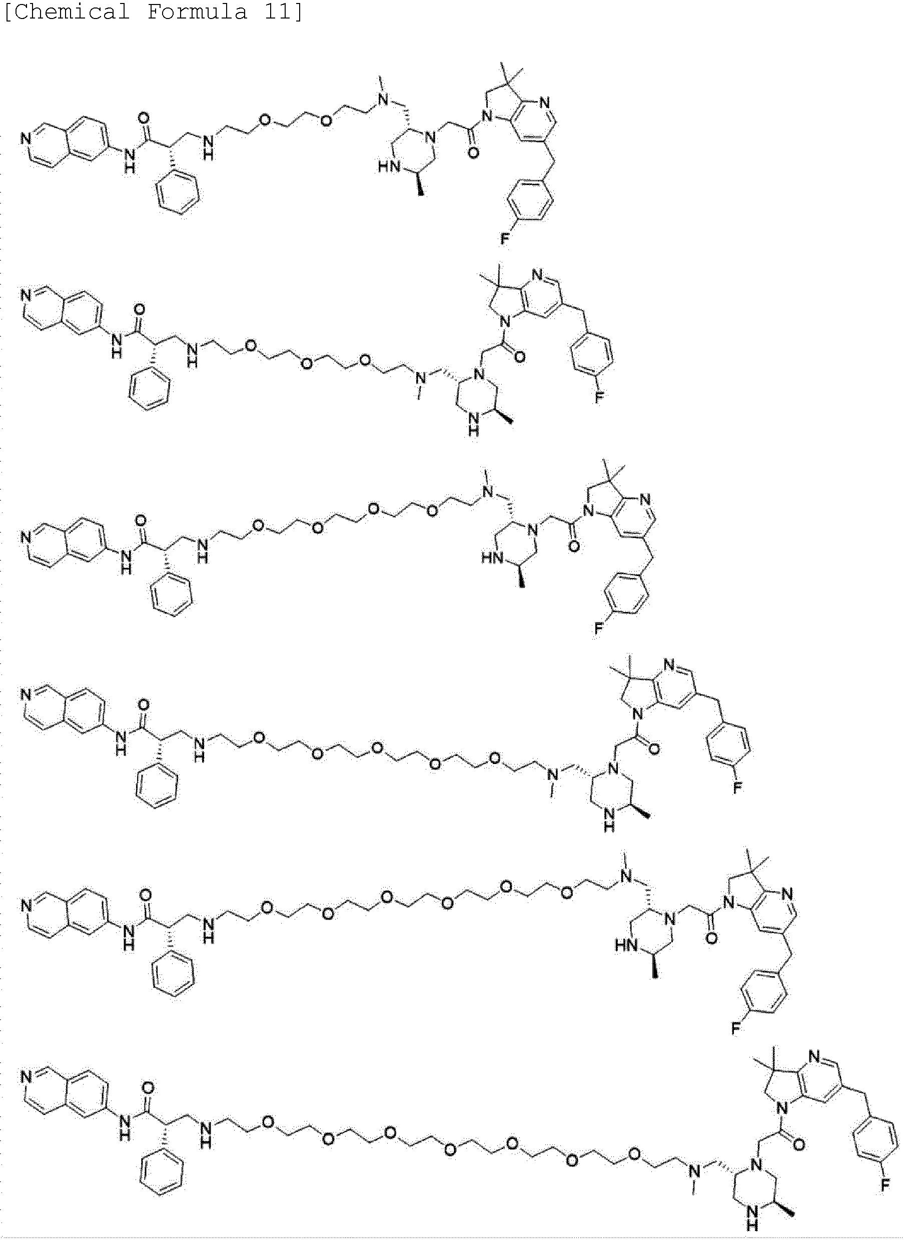

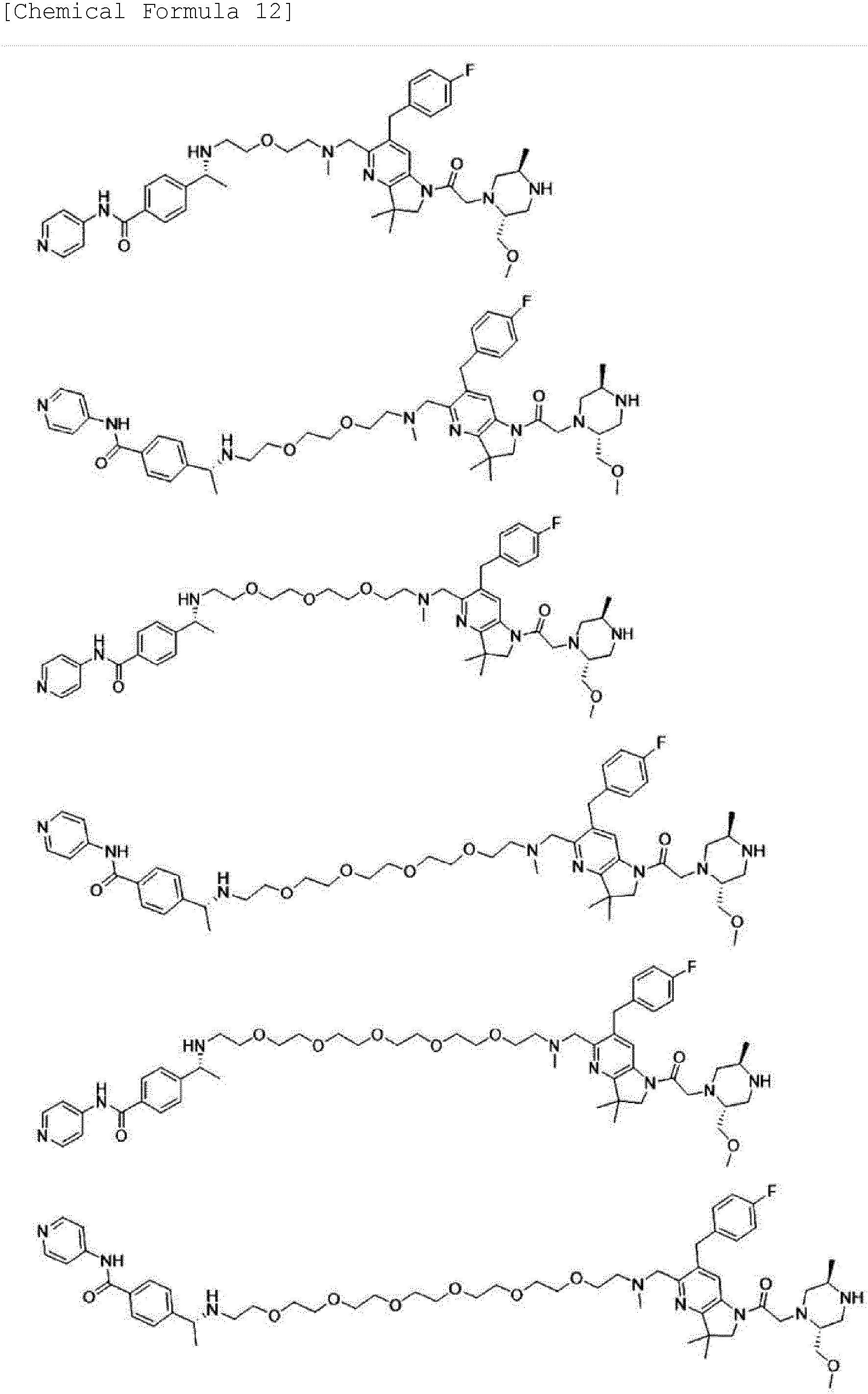

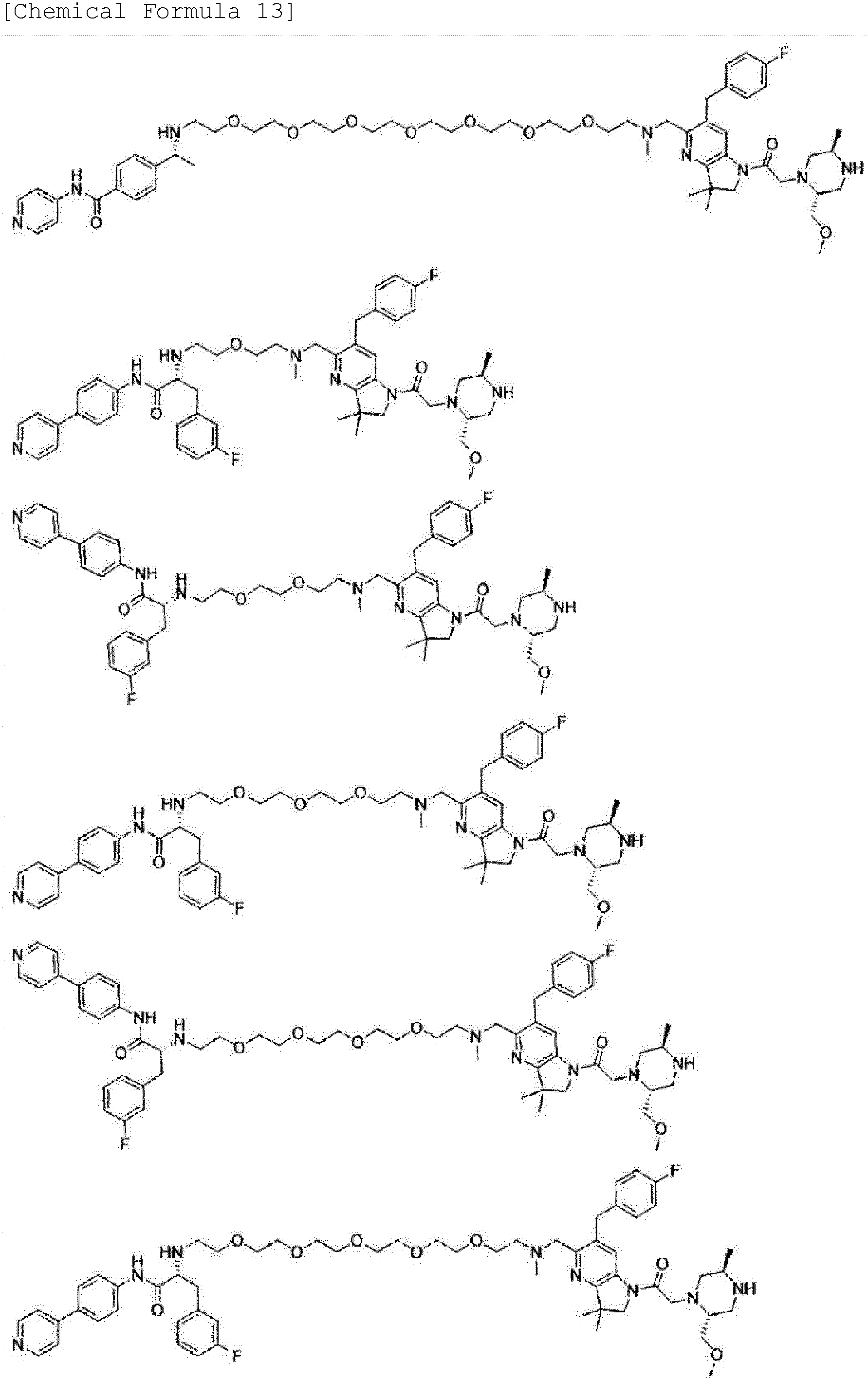

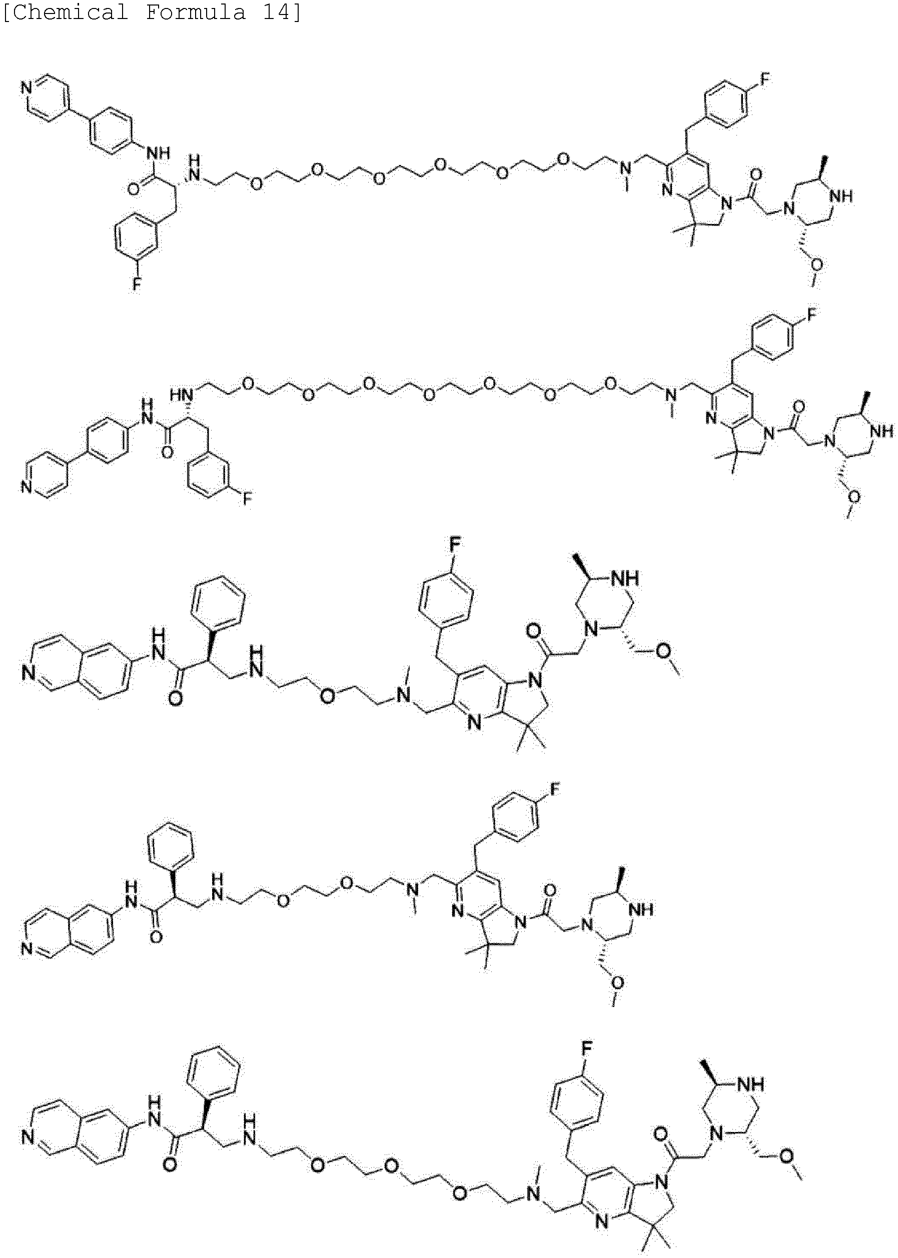

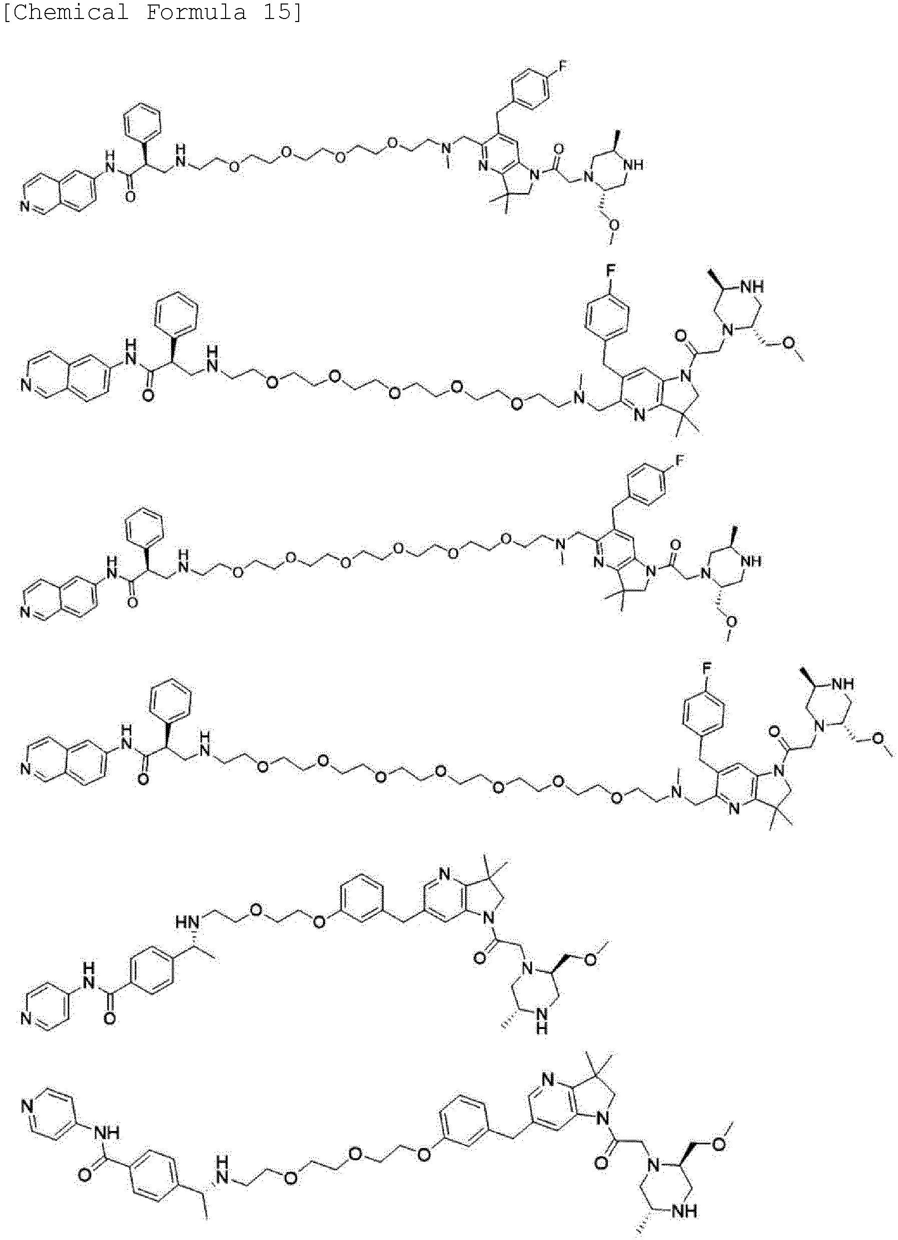

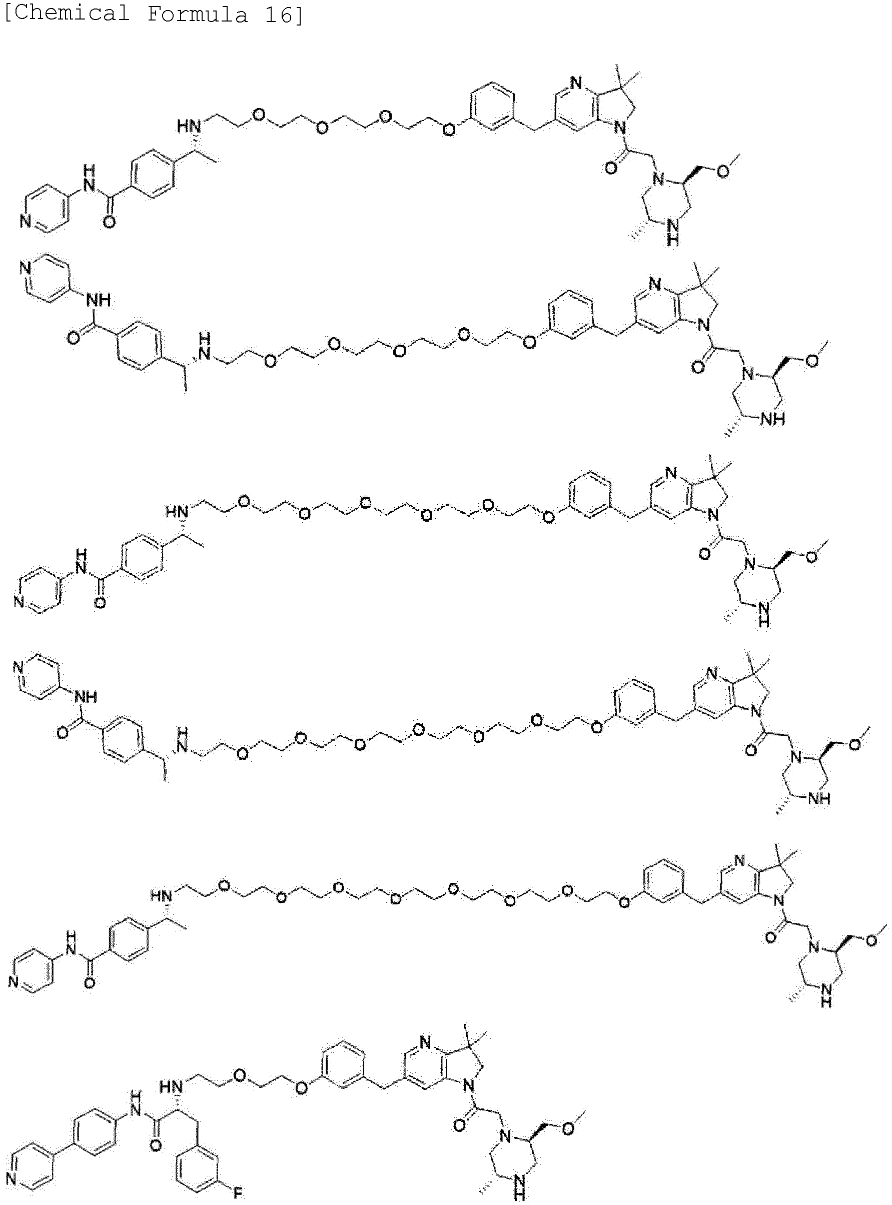

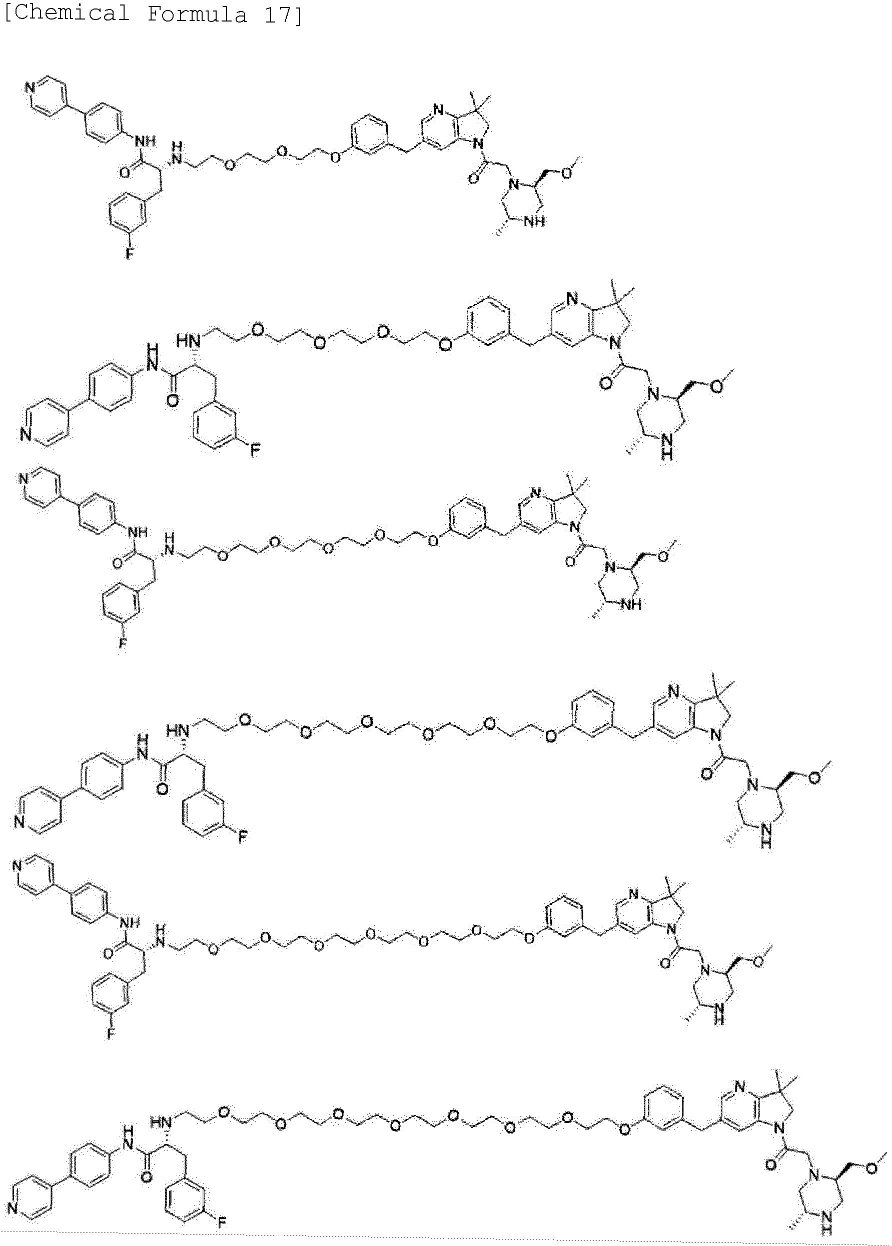

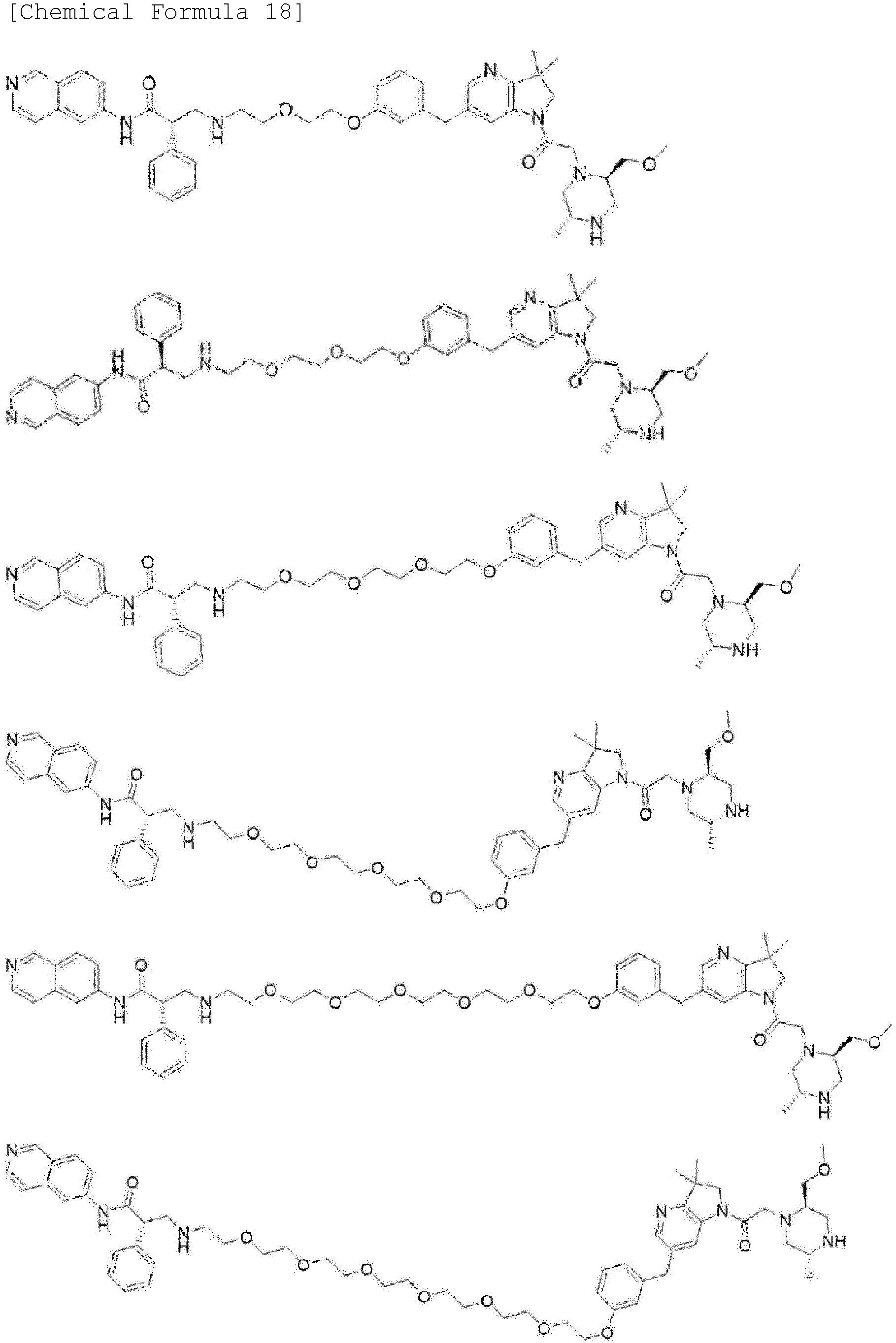

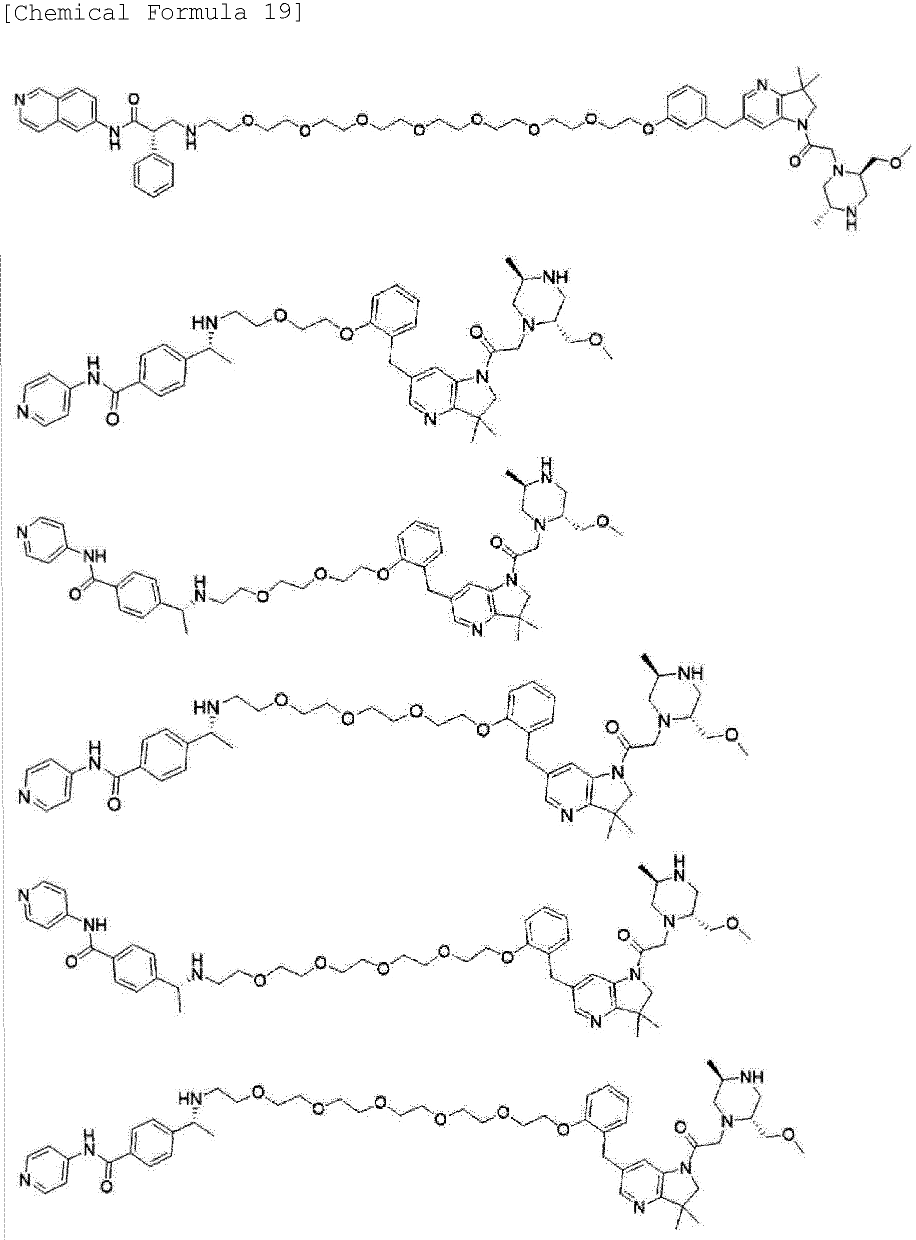

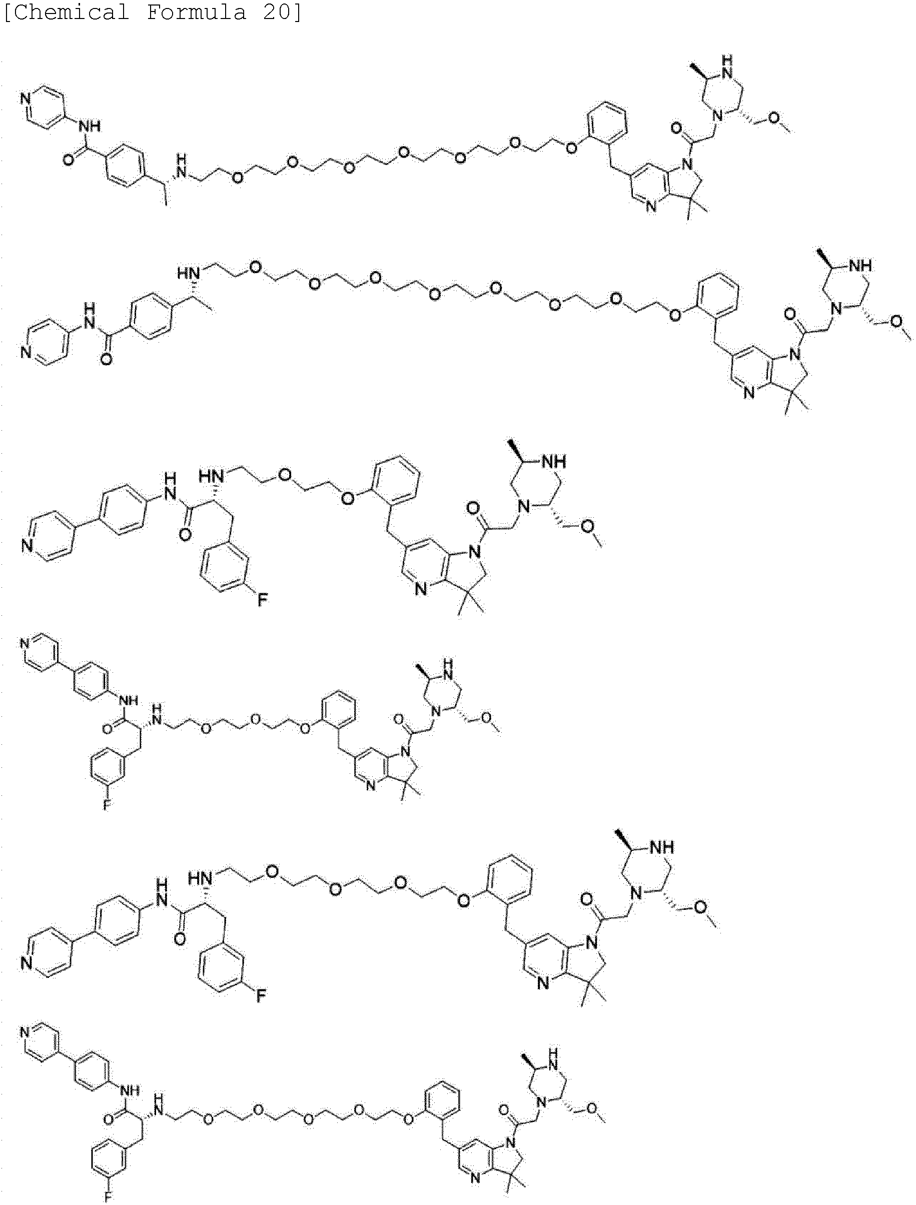

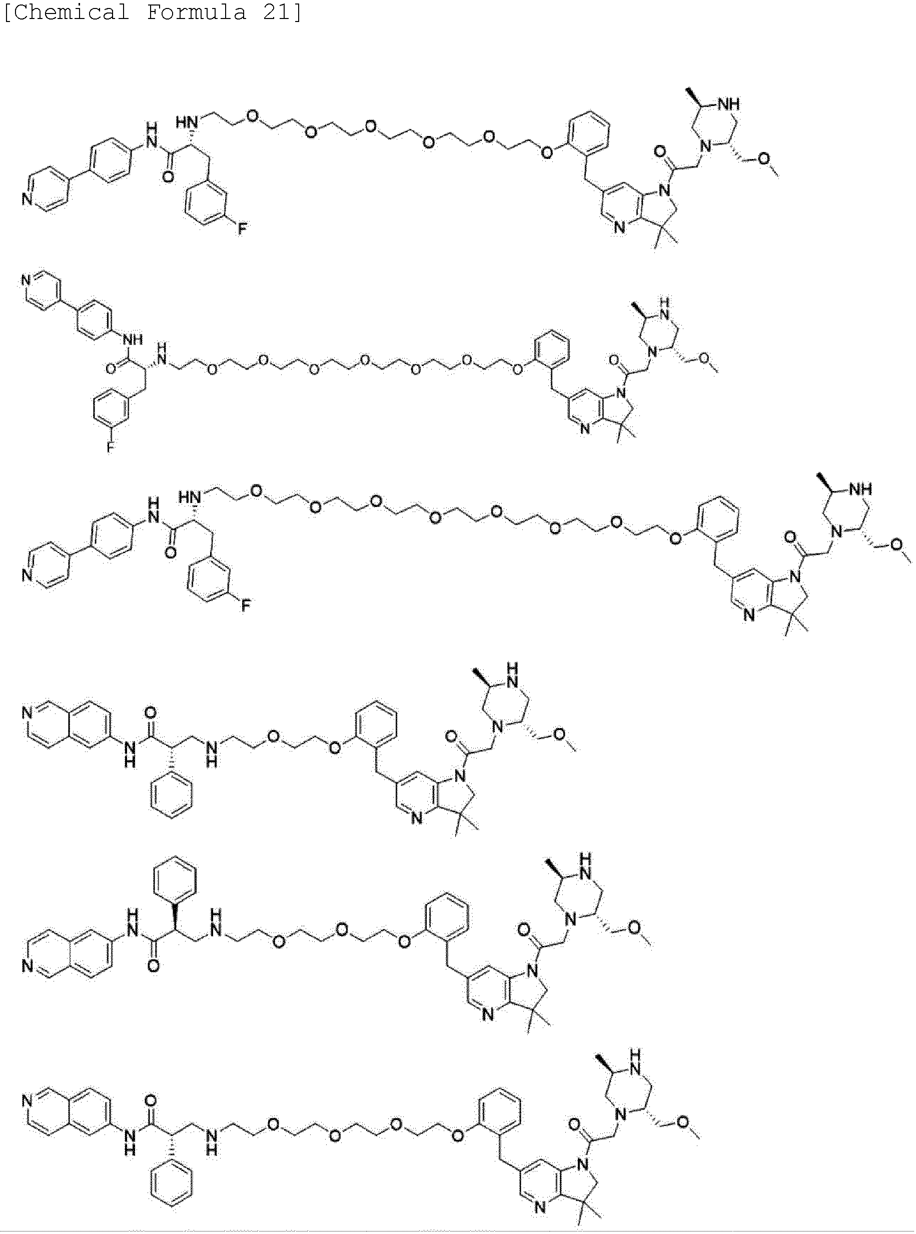

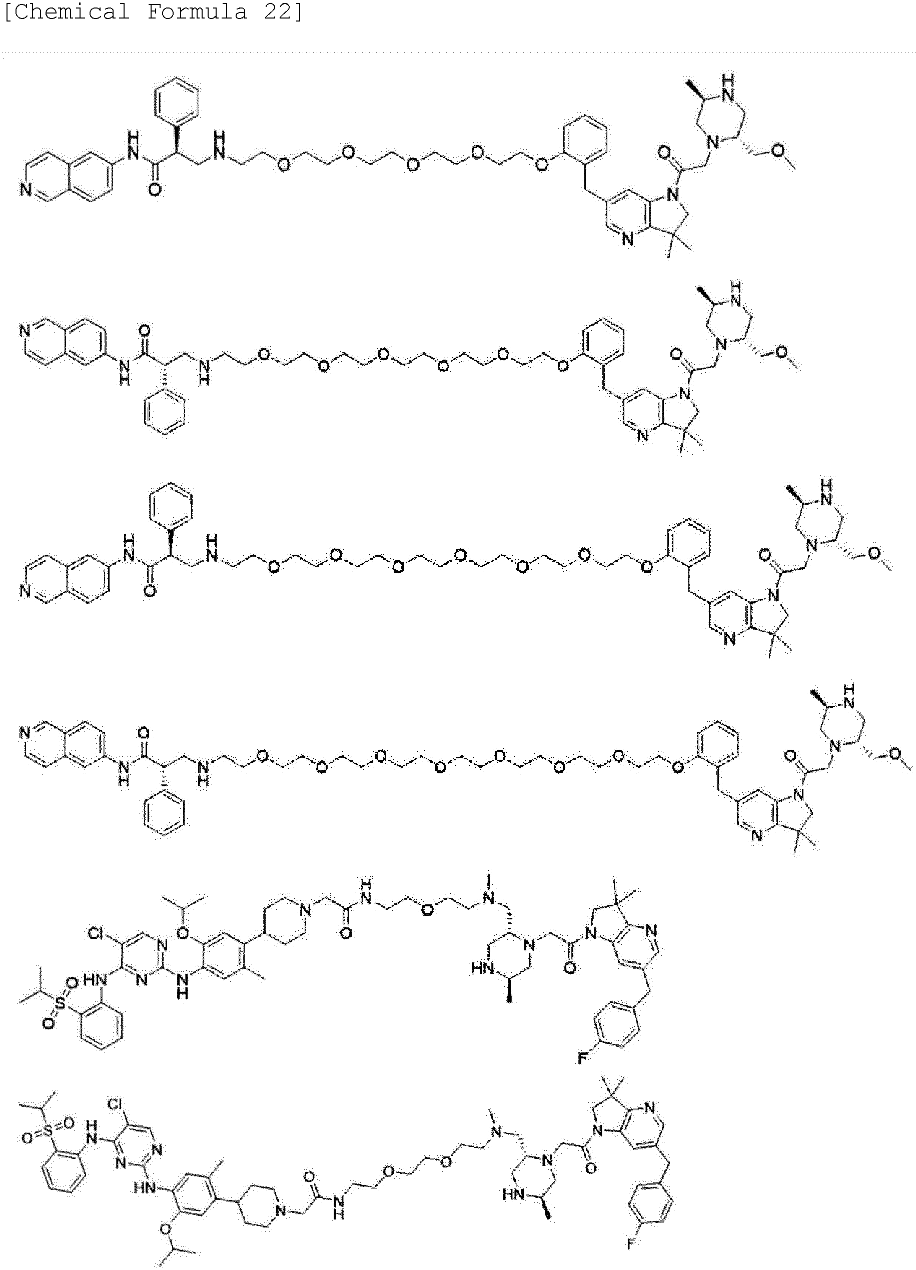

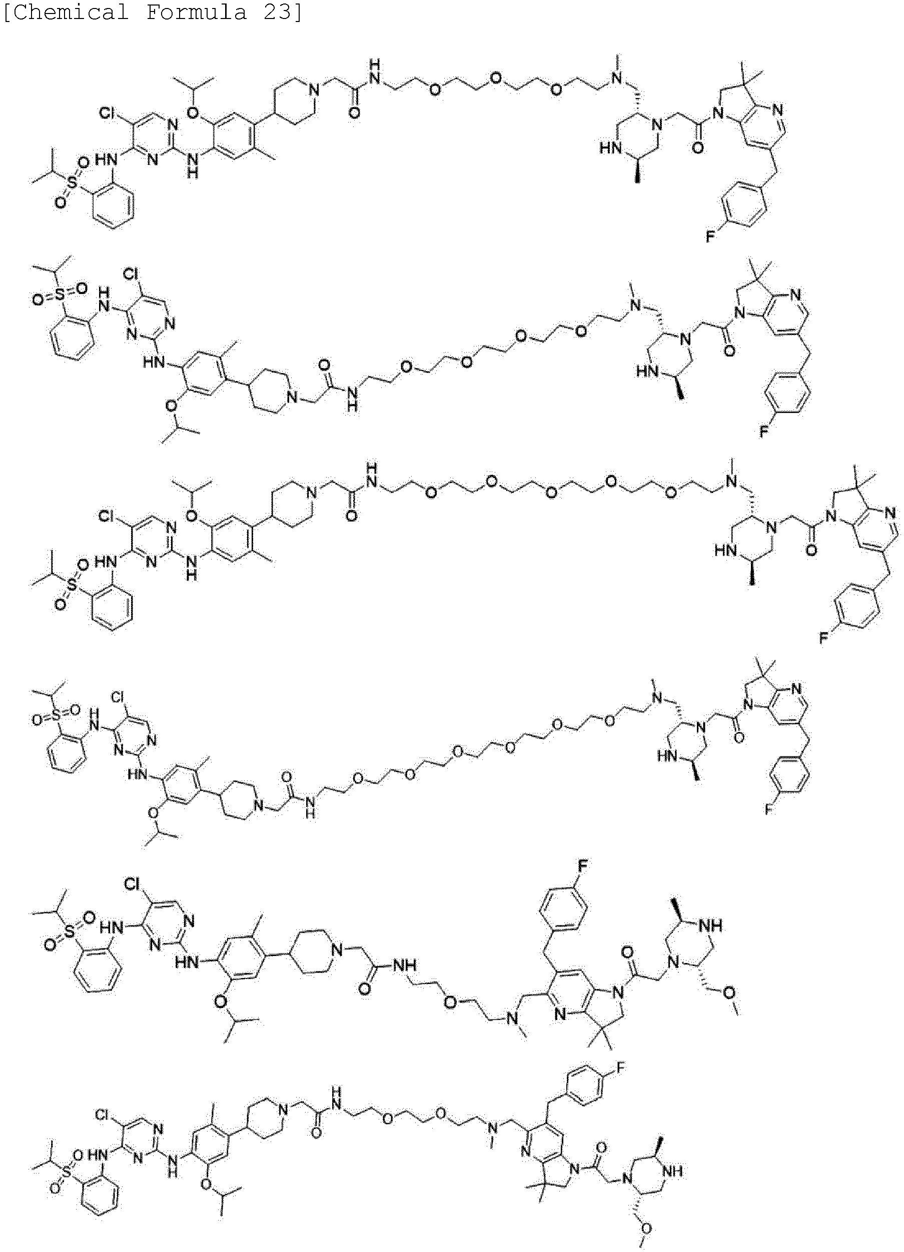

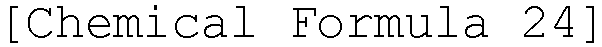

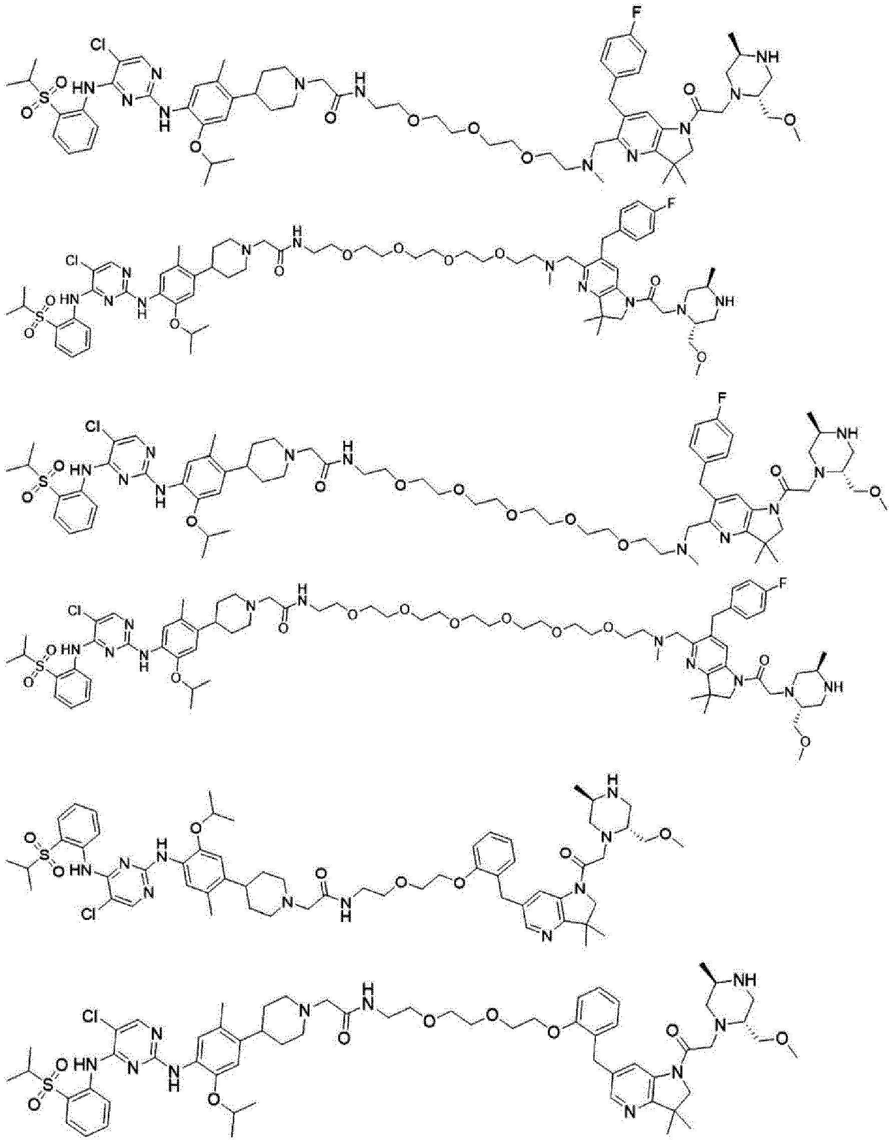

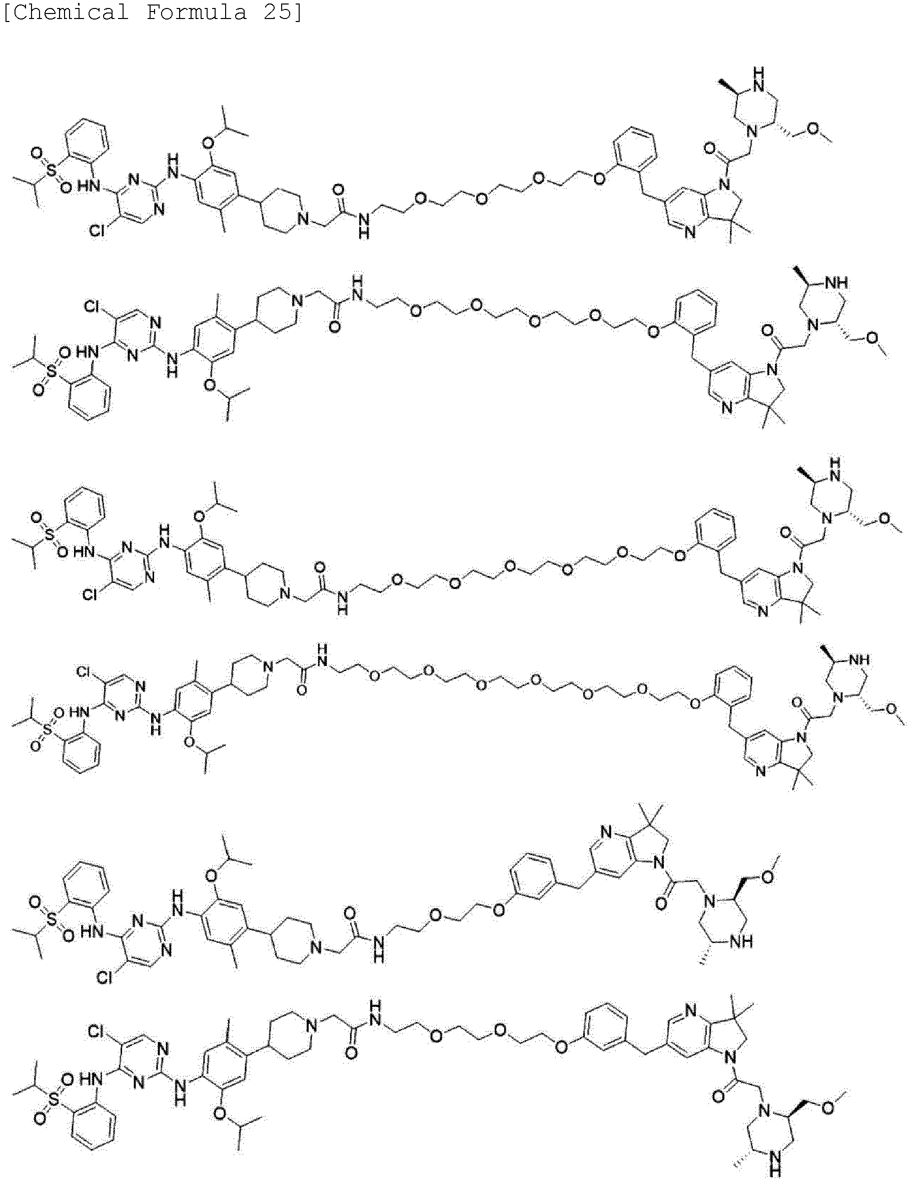

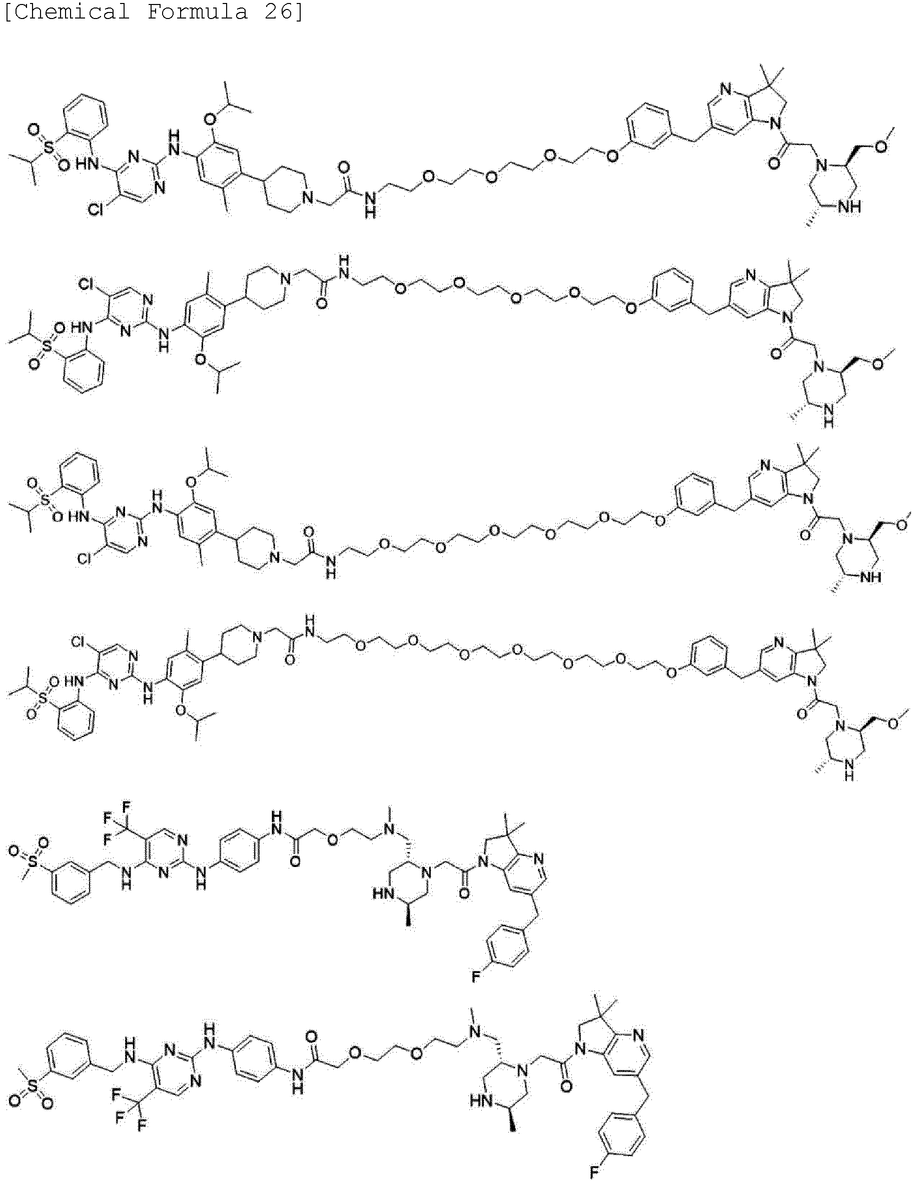

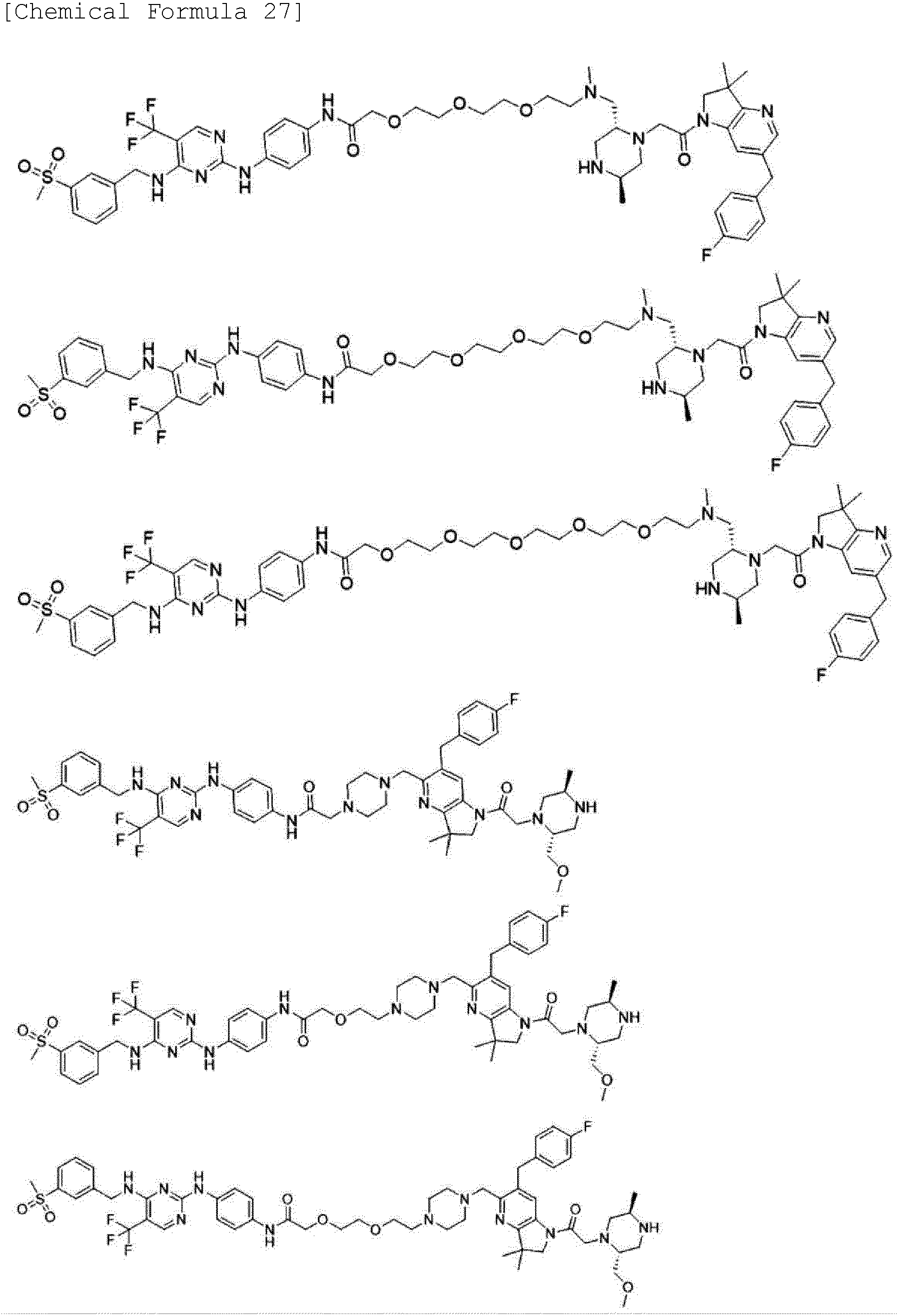

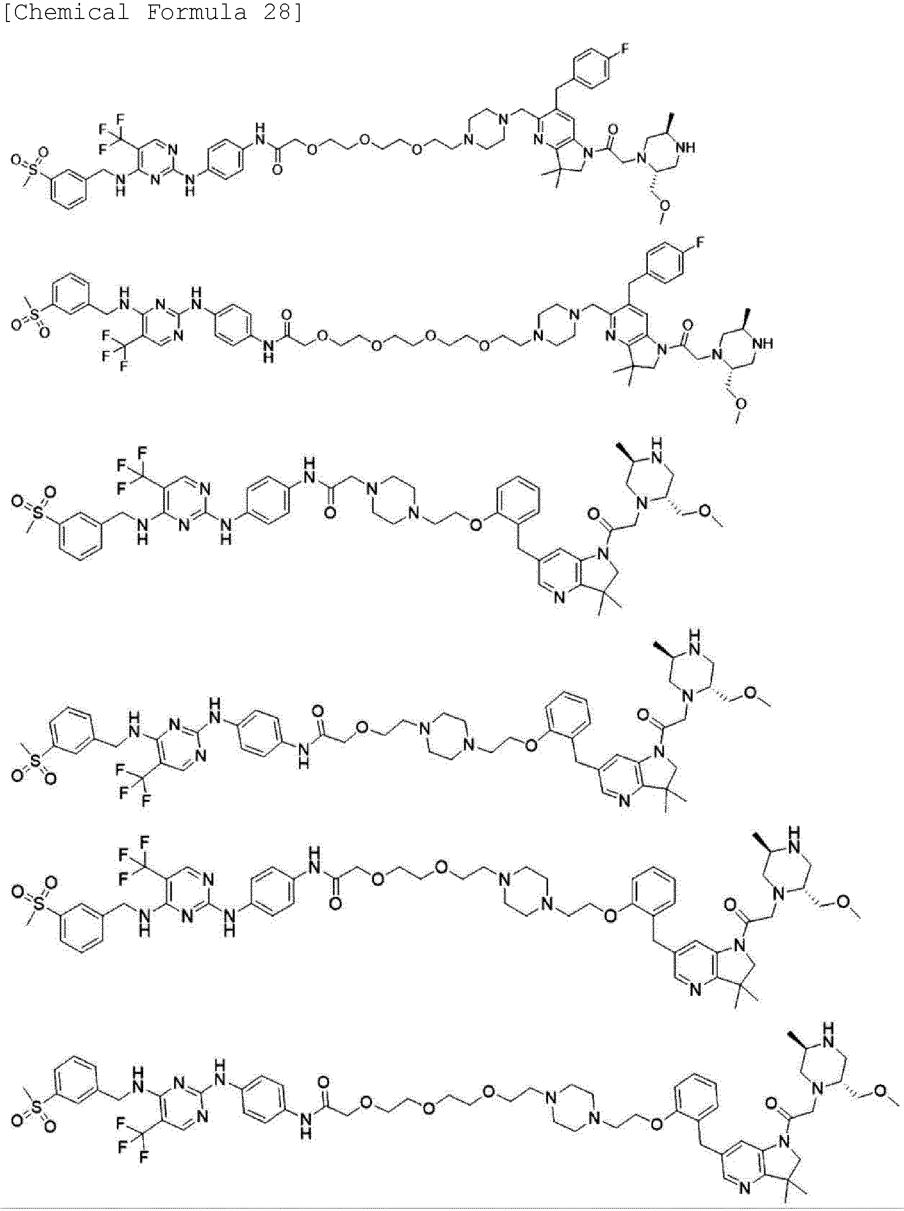

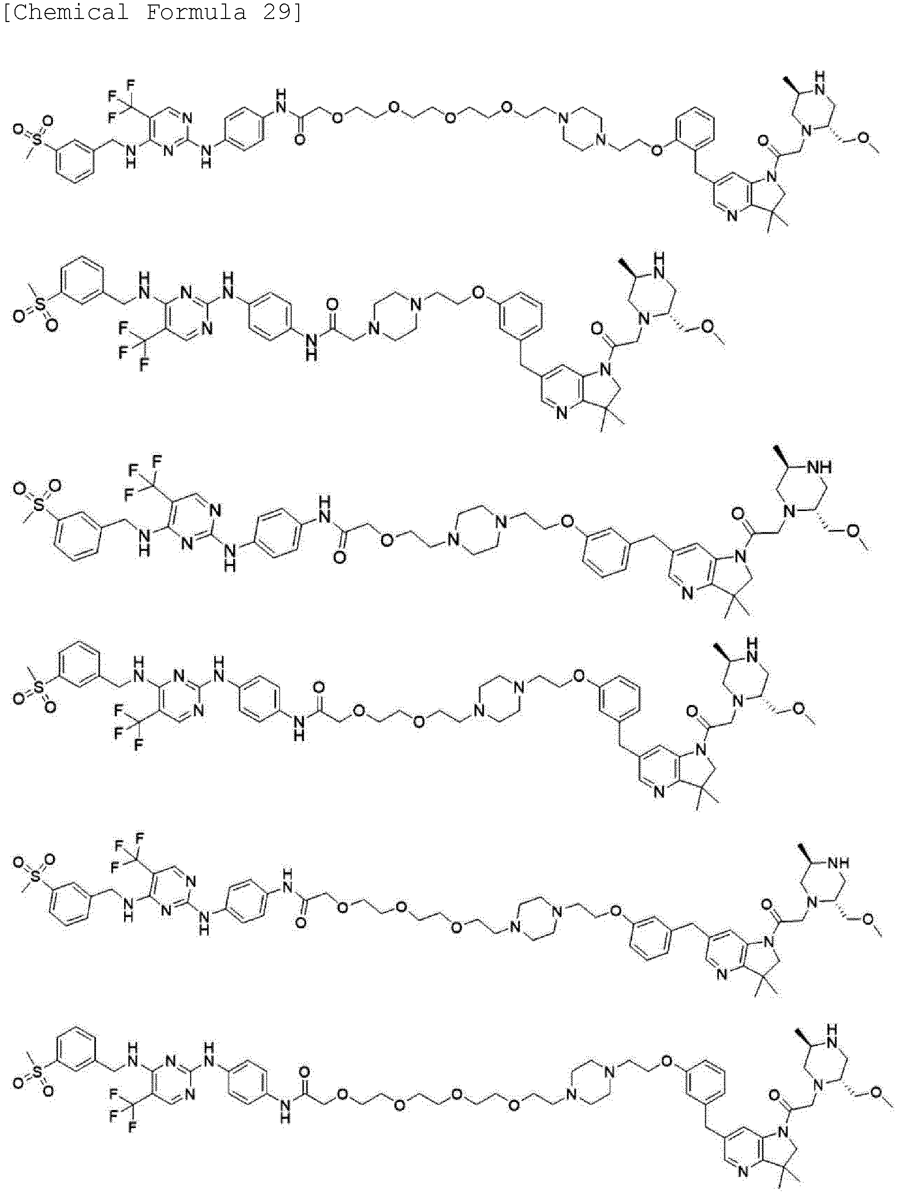

32. A pharmaceutical composition comprising the compound or the isomer thereof or the salt thereof of any one of claims 1 to 31.

33. A target protein modulator comprising the compound or the isomer thereof or the salt thereof of any one of claims 1 to 31.
